# EUROPEAN PATENT APPLICATION

(11) **EP 1 884 513 A1**
(43) Date of publication of application: **06.02.2008**
(21) Application number: 06756652.1
(22) Date of filing: 22.05.2006
(51) Int. Cl.: C07D 231/40

(54) **PYRAZOLE COMPOUND AND THERAPEUTIC AGENT FOR DIABETES COMPRISING THE SAME**

(30) Priority: 23.05.2005 JP 2005148847; 26.05.2005 US 685037 P; 20.12.2005 JP 2005367286; 03.01.2006 US 755820 P
(71) Applicant: Japan Tobacco, Inc., Tokyo 105-8422 (JP)
(72) Inventor: TAKAGI, Masaki c/o Central Pharmaceutical Research, Takatsuki-shi, Osaka 5691125 (JP); NAKAMURA, T. c/o Central Pharmaceutical Research, Takatsuki-shi, Osaka 5691125 (JP); MATSUDA, I. c/o Central Pharmaceutical Research, Takatsuki-shi, Osaka 5691125 (JP); FUKUDA, K. c/o Central Pharmaceutical Research, Takatsuki-shi, Osaka 5691125 (JP); OZAWA, Koichi c/o Central Pharmaceutical Research, Takatsuki-shi, Osaka 5691125 (JP); UEDA, Nobuhisa c/o Central Pharmaceutical Research, Takatsuki-shi, Osaka 5691125 (JP); SAKATA, Kaoru c/o Central Pharmaceutical Research, Takatsuki-shi, Osaka 5691125 (JP); NOMURA, Y. c/o Central Pharmaceutical Research, Takatsuki-shi, Osaka 5691125 (JP)
(74) Representative: Vossius & Partner
(86) International application number: PCT/JP2006/310603
(87) International publication number: WO 2006/126695

(57) **Abstract**

The present invention provides a pyrazole compound that has liver glycogen phosphorylase inhibitory activity and is useful as a therapeutic or prophylactic agent for diabetes, the pyrazole compound represented by the following general formula (I): 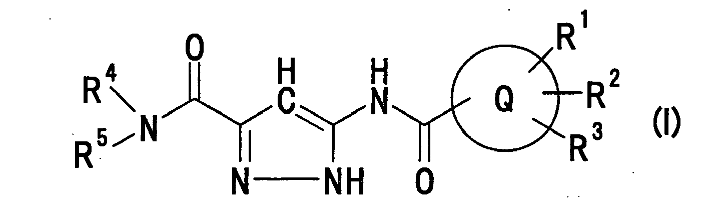 wherein Ring Q represents an aryl or heteroaromatic group, R¹ represents a hydrogen atom, a halogen atom, a C₁₋₆ alkyl group or a C₁₋₆ alkoxy group, R² represents a halogen atom, a C₁₋₆ alkyl group, a C₁₋₆ alkoxy group or an azido group, R³ represents a halogen atom, a hydroxyl group, a C₁₋₆ alkyl group, a halo C₁₋₆ alkyl group, a C₁₋₆ alkoxy group, an azido group, an amino group, an acylamino group or a C₁₋₆ alkylsulfonylamino group, R⁴ and R⁵ are identical with or different from each other and represent a hydrogen atom, a substituted or unsubstituted C₁₋₆ alkyl group, a C₃₋₈ cycloalkyl group, a substituted or unsubstituted saturated heterocyclic group, a substituted or unsubstituted aryl group, a C₇₋₁₄ aralkyl group, a heteroaromatic group, or the like, or a pharmacologically acceptable salt thereof.

## Description

### Technical Field

The present invention relates to a pharmaceutical composition for treating or preventing diabetes comprising a pyrazole compound having liver glycogen phosphorylase inhibitory activity and a pharmaceutically acceptable carrier, a novel pyrazole compound having such liver glycogen phosphorylase inhibitory activity, and a pharmaceutical composition comprising a combination of the pharmaceutical composition for treating or preventing diabetes and another agent (for example, an antihyperlipidemic agent, a therapeutic or prophylactic agent for obesity, a therapeutic agent for diabetes, a therapeutic agent for diabetic complications, or an antihypertensive agent).

### Background Art

Diabetes is a chronic disease resulting from abnormal metabolism of glucose, lipids and amino acids due to a loss of insulin action. If untreated, diabetes may lead to hyperglycemia and glycosuria.

Diabetes is classified into type I diabetes mellitus (insulin-dependent diabetes mellitus; IDDM) characterized by insulin hyposecretion resulting from destruction of islet cells caused by production of autoantibodies mainly to the pancreatic islets, and type II diabetes mellitus (non-insulin-dependent diabetes mellitus; NIDDM) resulting from the disorder of the insulin secretory function of the pancreas and insulin resistance in peripheral tissues such as the muscular tissue, and in the liver. Insulin-dependent diabetes mellitus may lead to ketonemia and acidosis due to the loss of insulin secreting capacity, and if untreated, may result in diabetic coma. Neither dietary therapy nor treatment with an oral hypoglycemic agent is effective, and only treatment with insulin is effective. On the other hand, non-insulin dependent diabetes mellitus (NIDDM) presents only a small degree of ketonemia and acidosis although the insulin action is reduced from normal, and treatment with insulin is not always required. About 90 to 95% of diabetics have non-insulin-dependent diabetes mellitus.

Diabetes is a disease that can lead to complications such as neuropathy, retinopathy and microvascular disorders in the kidney as a result of long-term hyperglycemia, and further to fatal complications such as coronary artery disease and strokes.

Hypoglycemic agents currently used for treating hyperglycemia include insulin preparations, sulfonylurea agents (for example, glibenclamide, tolbutamide), biguanides (for example, metformin), insulin resistance ameliorating agents (for example, pioglitazone), and α-glucosidase inhibitors (for example, acarbose).

Insulin preparations, which are used for insulin-dependent diabetes, can surely lower blood glucose levels, but they must be administered via injections and can lead to hypoglycemia.

Sulfonylurea agents stimulates pancreatic beta cells to promote endogenous insulin secretion from pancreatic beta cells, but the timing and amount of insulin secretion depends on the timing and dose of drug administration rather than blood glucose levels. Consequently the side effect of hypoglycemia may often result from prolonged drug action. In addition, digestive symptoms such as anorexia may occur. It is contraindicated for patients with serious ketosis or liver or renal disfunction.

Biguanides do not stimulate pancreatic beta cells, and do not induce hypoglycemia in healthy people or diabetics by single administration. Possible modes of action include increased glucose use by anaerobic glycolysis, suppression of gluconeogenesis, and suppression of intestinal absorption of glucose. A likely side effect is comparatively serious lactic acidosis.

Thiazolidine derivatives, which are included among insulin resistance ameliorating agents (insulin sensitizers), enhance insulin action instead of stimulating insulin secretion, and are capable of activating insulin receptor kinase, promoting glucose uptake by peripheral tissues, and ameliorating increased liver glucose production. Known side effects include digestive symptoms and edema, and also include decreases in red blood cell count, hematocrit and hemoglobin and an increase in LDH.

α-glucosidase inhibitors prevent the elevation in blood glucose levels after meals by delaying digestion/absorption of carbohydrate in digestive tracts, but they can cause problematic side effects such as distension, borborygmus and diarrhea. (see, for example, Non-Patent Document 1)

These drugs thus have limited use because of side effects and unresponsive patients, and there is a need for a hypoglycemic agent with a new mechanism of action.

Recently, glucose release from the liver was reported to increase during fasting in NIDDM patients compared to normal people. The report suggests that the phenomenon that glucose is released from the liver can be a target for drug therapy for NIDDM.

According to recent findings in diabetic conditions, pancreatic beta cell disfunction and increased glucose release from the liver may be involved in the onset and progress of diabetes to a great extent (see, for example, Non-Patent Document 2). Glucose release from the liver is expressed as the sum of two pathways, i.e. , degradation of liver glycogen and gluconeogenesis. In diabetic conditions, the liver glycogen degradation system is enhanced (see for example, Non-Patent Documents 3 and 4). In addition, by suppressing the degradation of liver glycogen, blood glucose levels decreased in diabetic animals or normal people subjected to glucagon stimulation (see for example, Non-Patent Document 5). These findings suggest that the enhanced degradation of liver glycogen is involved in diabetic conditions.

On the other hand, it is known that this glycogenolysis is catalyzed by liver glycogen phosphorylase and the degradation of glycogen (n glucose units) by phosphorolysis results in glucose 1-phosphate (G-1-P) and glycogen (n-1 glucose units).

Antidiabetics of a new mechanism have therefore been developed that have the effect to inhibit liver glycogen phosphorylase which is significantly involved in this glycogenolysis. However, antidiabetics with satisfactory activity have not yet been found.

In addition, several liver glycogen phosphorylase inhibitors have been reported to have various effects as well as being an effective therapeutic or prophylactic drug for diabetes.

For example, a compound having liver glycogen phosphorylase inhibitory activity is useful for the treatment of "diabetes, insulin resistance, diabetic neuropathy, diabetic nephropathy, diabetic retinopathy, cataract, hyperglycemia, hypercholesterolemia, hypertension, hyperinsulinemia, hyperlipidemia, atherosclerosis, tissue ischemia and myocardial ischemia". (See for example, Patent Document 1)

In addition, a compound having liver glycogen phosphorylase inhibitory activity is effective as an appetite control agent and a therapeutic agent for obesity, and is useful for the treatment or prevention of diseases for which decreasing blood lipid levels is effective, such as dyslipidemia, hypertriglyceridemia, hyperlipidemia, hyperlipoproteinemia, cardiovascular disease and hypertension. (See for example, Patent Document 2)

In addition, a liver glycogen phosphorylase inhibitor is effective at treating and preventing diseases related to glucose metabolism, such as diabetes, and especially non-insulin-dependent diabetes mellitus (NIDDM) including prolonged complications (for example, retinopathy, neuropathy, nephropathy and micro- and macro-vascular disease). (See for example, Patent Document 3)

In addition, three isoforms of glycogen phosphorylase, i.e., liver isoform, muscle isoform and brain isoform, have been identified in humans, and these isoforms are products of three different genes, having 80-83% amino acid homology. Glycogen phosphorylase is also present in bacteria, and a compound having liver glycogen phosphorylase inhibitory activity provides means of treating or preventing infections, such as bacterial, fungal, parasitic or viral infections, and is effective at treating and preventing these infections. (See for example, Patent Document 4)

Therefore, as well as being useful for the treatment and preventing diabetes, a liver glycogen phosphorylase inhibitor is expected to be useful as a therapeutic agent for insulin resistance, diabetic neuropathy, diabetic nephropathy, diabetic retinopathy, cataract, hypercholesterolemia, hypertension, hyperinsulinemia, hyperlipidemia, atherosclerosis, tissue ischemia and myocardial ischemia, as a therapeutic agent for appetite control and obesity, and a therapeutic agent for infections such as bacterial, fungal, parasitic or viral infection.

The compounds below are known to be liver glycogen phosphorylase inhibitors or compounds that may have a structure relatively similar to that of the compound of the present invention.

WO96/39384 (National Publication of International Patent Application No. 1998-511687) discloses indole compounds having glycogen phosphorylase inhibitory activity. (see Patent Document 5)

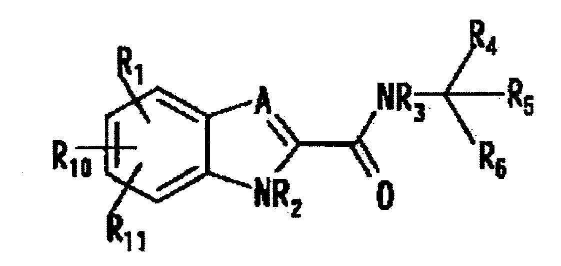

In the formula, R₄ is a phenylalkyl group or the like, R₅ is a hydrogen atom or the like, R₆ is an alkoxycarbonyl group or the like, R₂ is a hydrogen atom, R₁, R₁₀ and R₁₁ are independently a hydrogen atom, a halogen atom or the like, R₃ is a hydrogen atom or the like, A is -N= or the like.

For example, the following compound is disclosed as a specific compound.

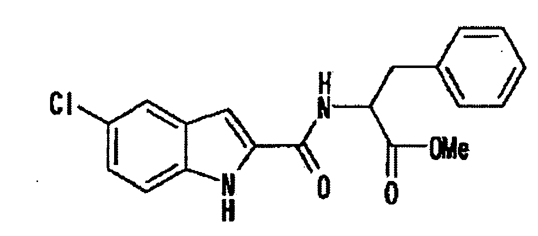

WO01/94300A1 discloses acylphenylurea derivatives represented by the following general formula (I) that are effective for the treatment of type II diabetes, and a preparation method thereof. (see Patent Document 6)

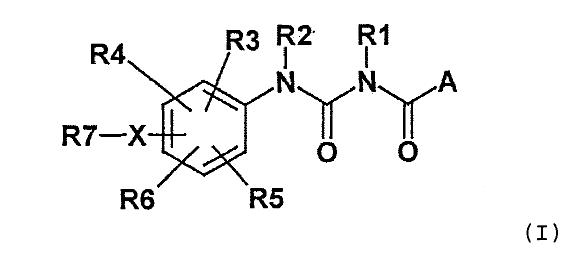

In the formula, A represents a phenyl group or a naphthyl group that may be substituted with various substituents, R1 and R2 represent a hydrogen atom, an alkyl group or the like, R3, R4, R5 and R6 represent a hydrogen atom, a halogen atom or the like, X represents an oxygen atom or sulfur atom, and R7 represents an alkyl group or the like substituted with a carboxy, phenyl or heterocyclic group.

WO02/096864A1 discloses carboxamido-substituted phenylurea derivatives represented by the following general formula (I) that are effective for the treatment of type II diabetes, and a preparation method thereof. (see Patent Document 7)

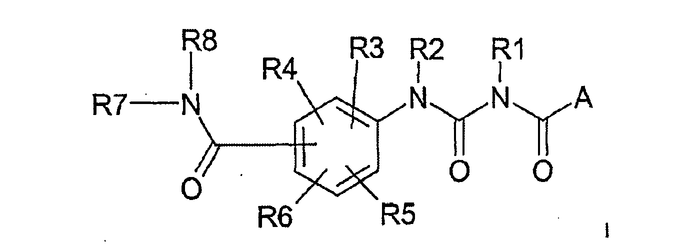

In the formula, A represents a phenyl group or a naphthyl group that may be substituted with various substituents, R1 and R2 represent a hydrogen atom, a halogen atom or the like, R3, R4, R5 and R6 represent a hydrogen atom, a halogen atom or the like, R7 represents a hydrogen atom, an alkyl group or the like, R8 represents a hydrogen atom, a substituted or unsubstituted alkyl group or the like, and R9 represents a halogen atom.

WO03/015774A1 discloses the use of the derivatives represented by the following general formula (I) for the treatment of diseases caused by glucokinase (GLK), such as type II diabetes. (see Patent Document 8)

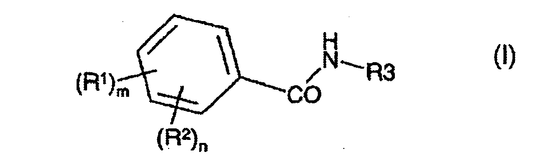

In the formula, m is 0, 1 or 2, n is 0,1, 2, 3 or 4, n + m is >0, R¹ is independently OH, - (CH₂)₁₋₄OH, -CH₃₋ₐFₐ, halo, C₁₋₆ alkyl group, phenyl, a heterocyclic ring or the like that may be substituted with a C₁₋₆ alkyl group, R² is -X-Y, wherein X is a linker selected from -O-Z-, -O-Z-O-Z-, -C(O)O-Z-, -OC(O)Z-, -S-Z-, a direct bond, or the like, Z is a direct bond, a C₂₋₆ alkenylene group, or the like, Y is aryl-Z¹-, heteroaryl-Z¹-, C₁₋₆ alkyl group, or the like, and R³ is a heterocyclic ring that may be substituted with R⁷.

The reference discloses the compounds below in Example GG 2 and 5 as specific examples of compounds having a pyrazole backbone for R³.

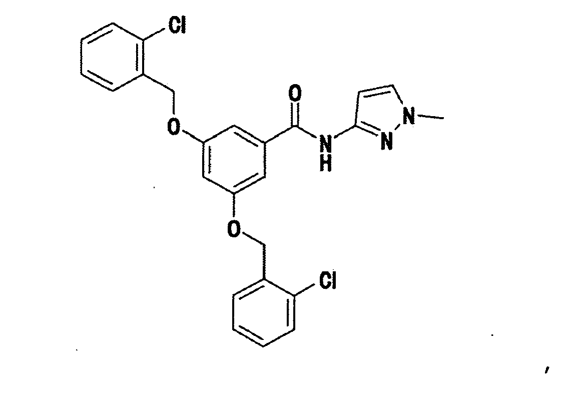

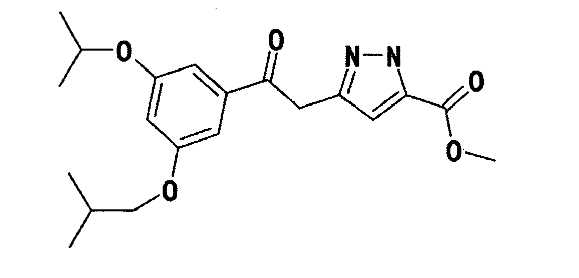

The reference also discloses in its specification proposed compounds represented by the following general formula (Ib) as a matter invention.

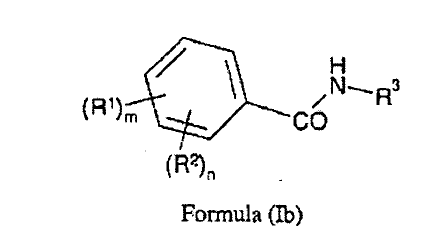

In the formula, R¹ is a hydroxyl group, a hydroxyalkyl group, a fluorinated alkyl group, a halogen atom, an alkoxy group, an alkyl group or the like, R² is -X-Y, wherein X is -O-Z- or the like, Y is -Zl-Aryl or the like, and R² does not include a halogen atom or an alkyl group. m is 0, 1 or 2, n is 1, 2 or 3, and m+n is 2 or 3.

m is 2 at the maximum as is clear from the above, and the phenyl group is never substituted with three halogen atoms at the same time.

WO03/084922A1 discloses acyl-4-carboxyphenylurea derivatives represented by the following general formula that are useful as antidiabetics. (see Patent Document 9)

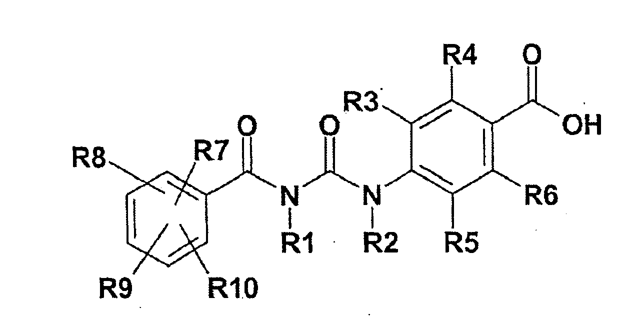

In the formula, R7, R8, R9 and R10 are identical with or different from each other and represent a hydrogen atom, a halogen atom, an alkoxy group or the like, R1 and R2 represent a hydrogen atom, an alkyl group or the like, R3 represents a hydrogen atom, a halogen atom, a phenoxy group or the like, R4 represents a hydrogen atom, a halogen atom, a nitro group, a phenoxy group or the like, R5 represents a hydrogen atom, a halogen atom, a phenoxy group, an alkyl group, or the like, and R6 represents a hydrogen atom, a halogen atom, a phenoxy group, an alkyl group or the like.

WO03/084923A1 discloses acyl-3-carboxyphenylurea derivatives represented by the following general formula (I) that are useful as antidiabetics. (see Patent Document 10)

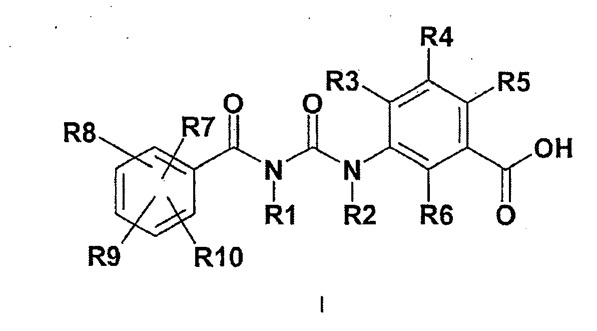

In the formula, R7, R8, R9 and R10 are identical with or different from each other and represent a hydrogen atom, a halogen atom, an alkoxy group or the like, R1 and R2 represent a hydrogen atom, an alkyl group or the like, R3 represents a hydrogen atom, a halogen atom, a phenoxy group or the like, R4 represents a hydrogen atom, a halogen atom, a nitro group, a phenoxy group or the like, R5 represents a hydrogen atom, a halogen atom, a phenoxy group, an alkyl group or the like, and R6 represents a hydrogen atom, a halogen atom, a phenoxy group, an alkyl group or the like.

WO03/104188A1 discloses N-benzoyl ureidocinnamate derivatives represented by the following general formula (I) that are useful as antidiabetics. (see Patent Document 11)

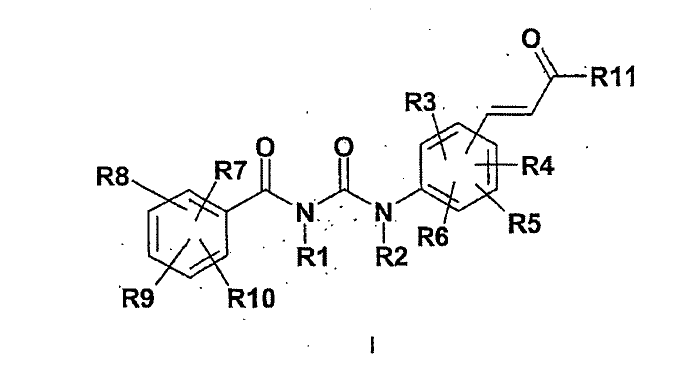

In the formula, R7, R8, R9 and R10 are identical with or different from each other and represent a hydrogen atom, a halogen atom, an alkoxy group or the like, R1 and R2 represent a hydrogen atom, an alkyl group or the like, R3, R4, R5 and R6 represent a hydrogen atom, a halogen atom, an alkyl group or the like, and R11 represents -OR12 or -N(R18) (R19).

WO2004/007455A1 discloses heterocyclic aryl-substituted benzoylurea derivatives represented by the following general formula (I) that are useful as therapeutic agents for type II diabetes. (see Patent Document 12)

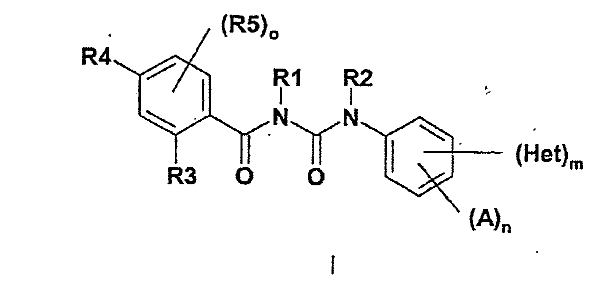

In the formula, R1 and R2 represent a hydrogen atom, an alkyl group, an amino group or the like, R3 and R4 represent a halogen atom, a hydroxyl group, an alkyl group, an alkoxy group or the like, R5 represents a hydrogen atom, a halogen atom, a hydroxyl group, an alkyl group or the like, A represents a hydrogen atom, a halogen atom, an alkyl group, an alkyl carbonyl group or the like, and Het represents a heterocyclic group such as triazole, tetrazole or pyrazole.

W02004/033416A2 discloses carboxyalkoxy-substituted acylcarboxyurea derivatives represented by the following general formula (I) that are useful as therapeutic agents for type II diabetes. (see Patent Document 13)

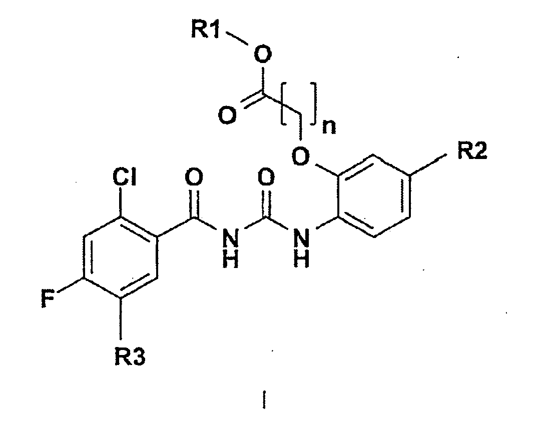

In the formula, R1 represents a hydrogen atom, an alkyl group, a phenyl group or the like, R2 represents a hydrogen atom, an alkyl group, an alkoxy group or the like, R3 represents a hydrogen atom, a halogen atom, an alkoxy group or the like, and n represents an integer from 1 to 8.

WO2004/065356A1 discloses carbonylamino-substituted acylphenylurea derivatives represented by the following general formula (I) that are useful as therapeutic agents for type II diabetes. (see Patent Document 14)

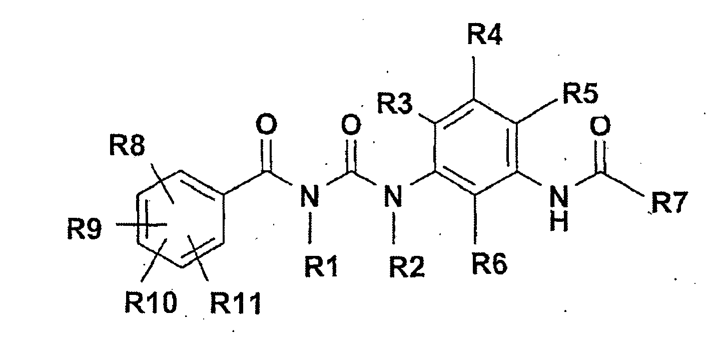

In the formula, R8, R9, R10 and R11 represent a hydrogen atom, a halogen atom, a hydroxy group, a nitro group, an alkoxy group or the like, R1 and R2 represent a hydrogen atom, a substituted or unsubstituted alkyl group, an alkoxy group or the like, R3, R4, R5 and R6 represent a hydrogen atom, a halogen atom, a nitro group, a phenoxy group, a hydroxy group or the like, and R7 represents a hydrogen atom, an alkyl group, a cycloalkyl group or the like.

Pyrazole compounds having an amide substituent have also been reported.

For example, W02004/098589A1 discloses derivatives of the pyrazole compound 4-bromo-5-(2-chloro-benzoylamino)-1H-pyrazole-3-carboxylic acid amide represented by the following general formula (I) or (II) .

However, these compounds relate to bradykinin B₁ receptor antagonists for treating inflammatory disease. (see Patent Document 15)

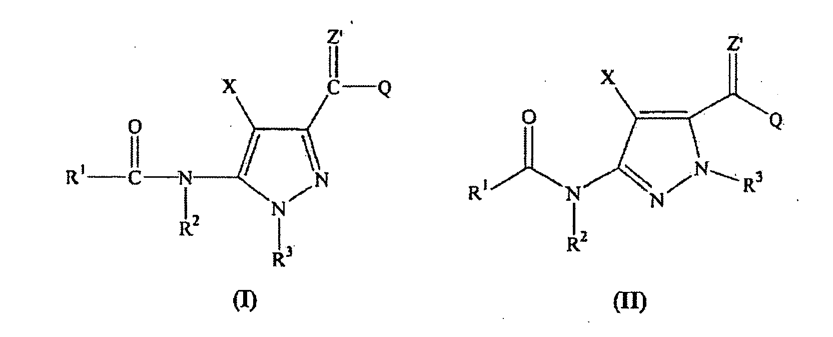

In the formulas, Z' represents 0, S or NH, Q represents N(R⁴) (R⁵), OH, an alkyl group or the like, R¹ represents a hydrogen atom, an alkyl group, a substituted alkyl group, an aryl group, a substituted aryl group, a heterocyclic group, a substituted heterocyclic group or the like, R² and R³ represent a hydrogen atom, an alkyl group, a substituted alkyl group, an aryl group or the like, R⁴ and R⁵ represent a hydrogen atom, an alkyl group, a substituted alkyl group, an alkoxy group, a cycloalkyl group, a substituted cycloalkyl group, a heterocyclic group, a substituted heterocyclic group or the like, and X represents a hydrogen atom, a halogen atom, an alkyl group or the like.

In addition, in Page 333 of the patent specification, many substituents including 2-chlorophenyl, 2-fluorophenyl, 2-hydroxyphenyl, 2-nitrophenyl, etc., and more specifically 2,4-dichlorophenyl and 3,4,5-trifluorophenyl are disclosed as specific examples of R¹. According to the description in Page 17 of the patent specification, however, a preferable R¹ group is 2-chlorophenyl, 2-fluorophenyl, 2-(quinolin-8-yl)phen-1-yl, or 2-[(3-methylphen-1-ylsulfanyl)methyl]phen-1-yl, and 2-chloro-4,5-difluorophenyl group, 4,5-difluoro-2-methylphenyl group, 2,4-dichloro-5-fluorophenyl group are not specifically described or even suggested.

In the document above, the following compounds are disclosed as examples of derivatives. However, specific compounds including the examples described therein are mainly characterized by having a 2-chloro-phenyl group as R¹ and a pyrazole ring having a substituent consisting of a halogen atom or an alkyl group, as is clear from its title "4-bromo-5-(2-chloro-benzoylamino)-1H-pyrazole-3-carboxylic acid amide derivatives." In addition, their uses are related to the bradykinin B₁ receptor useful for treating inflammation that is different from diabetes.

### Example 2

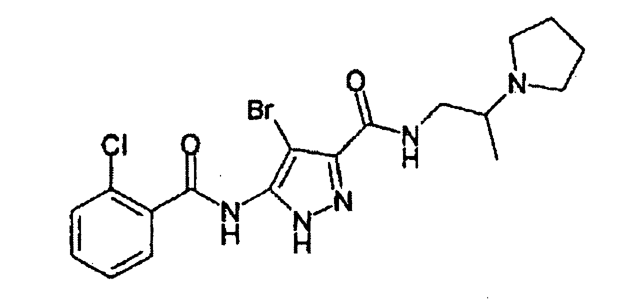

### Example 6

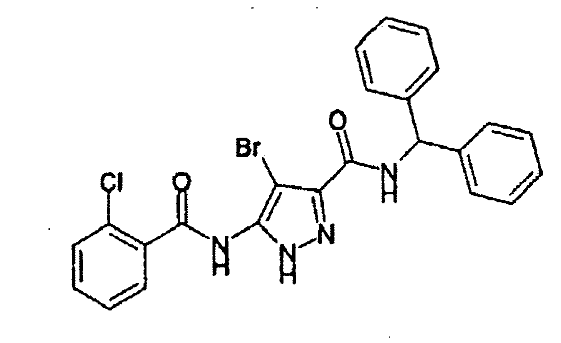

### Example 7

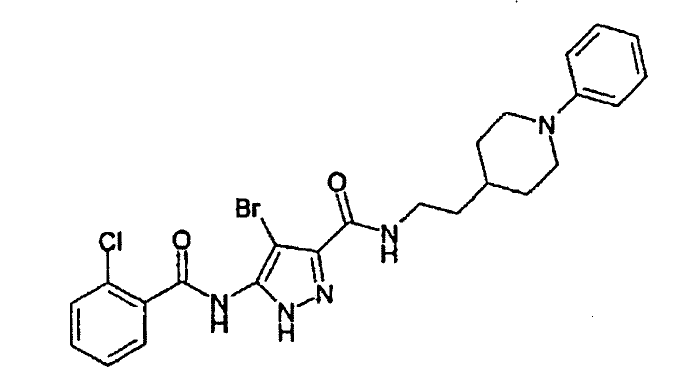

### Example 11

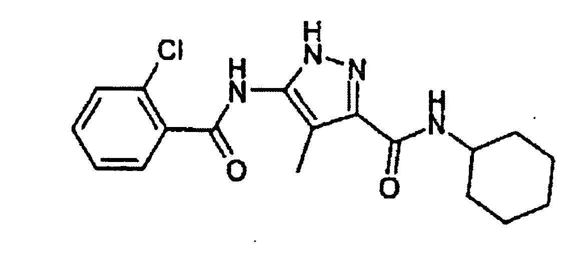

### Example 16

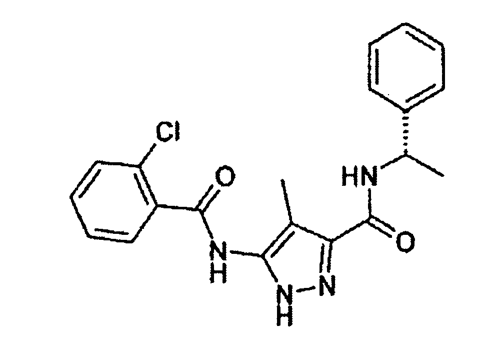

### Example 20

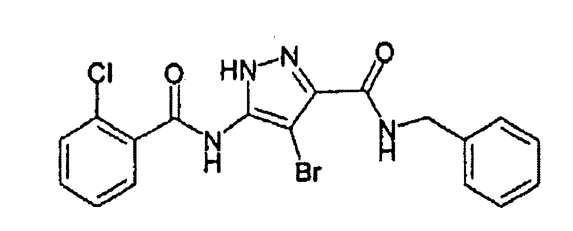

### Example 33

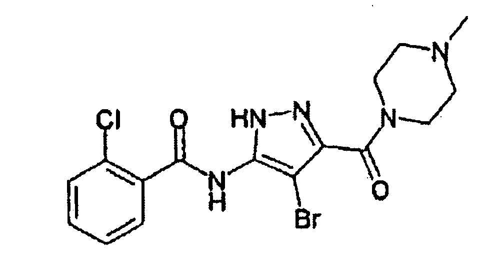

### Example 34

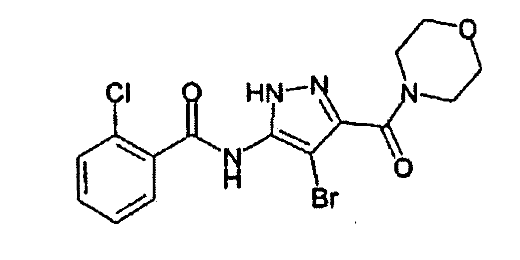

### Example 79

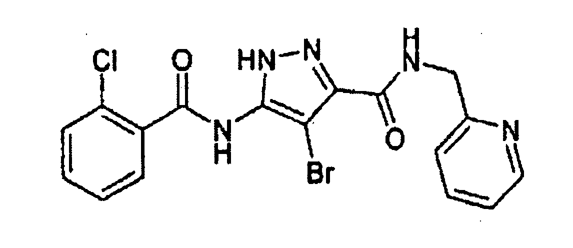

### Example 93

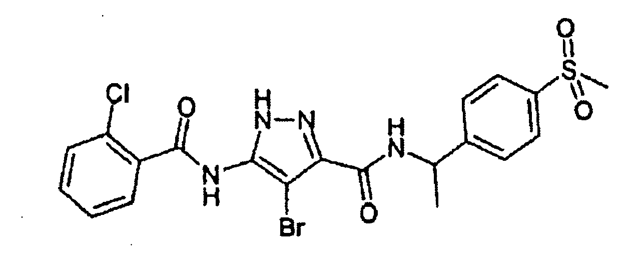

### Example 121

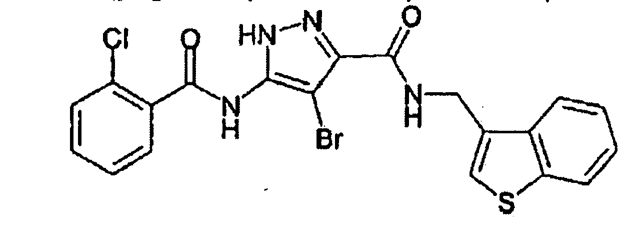

WO2004/098590A1 discloses pyrazole compounds represented by the following general formula (I) or (II) that are useful as bradykinin B₁ receptor antagonists for treating inflammatory disease. (See Patent Document 16). These, however, relate in particular to specific pyrazole compounds, i.e., 4-bromo-5-(2-chloro-benzoylamino)-1H-pyrazole compounds, as is clear from its title "4-bromo-5-(2-chloro-benzoylamino)-1H-pyrazole-3-carboxylic acid (1-(aminocarbonyl)eth-1-yl)amide derivatives."

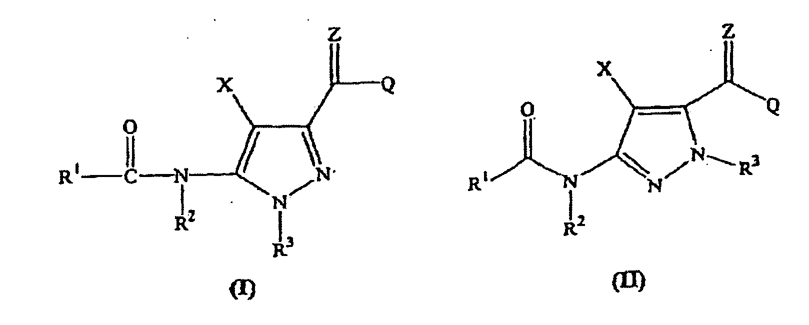

In the formulas, Z represents O, S or NH, and Q represents the following general formula.

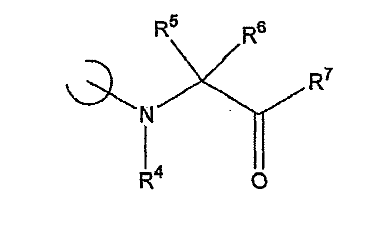

R¹ represents a hydrogen atom, a substituted or unsubstituted alkyl group, a substituted or unsubstituted aryl group or the like, R² and R⁴ represent a hydrogen atom, a substituted or unsubstituted alkyl group, R³ represents a hydrogen atom, a substituted or unsubstituted alkyl group, a substituted or unsubstituted aryl group or the like, R⁵ and R⁶ represent a hydrogen atom, an amino acid side chain or the like, and R⁷ represents -NR_{b}R_{c}, -OR_{b} or the like.

The following compounds are also disclosed as examples of the derivatives described above.

### Example 1

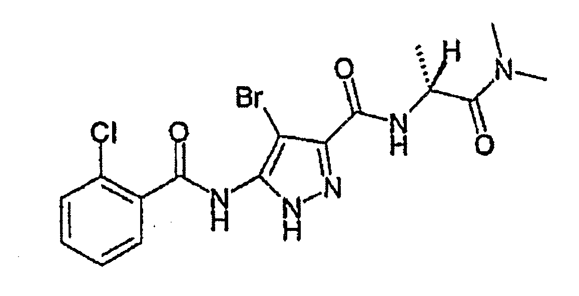

WO2004/099155A2 discloses substituted pyrazole derivatives represented by the following general formula (I) or (II) that are useful as bradykinin B₁ receptor antagonists for treating inflammatory disease. (see Patent Document 17) 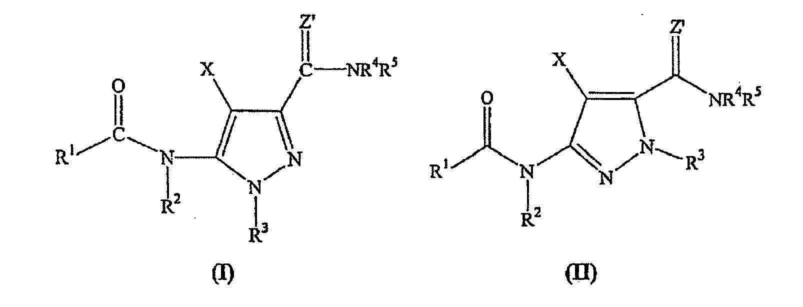 wherein Z' represents O, S or NH, R¹ represents a hydrogen atom, an alkyl group, a substituted alkyl group, an aryl group, a substituted aryl group or the like, R² represents a hydrogen atom, an alkyl group, a substituted alkyl group or the like, R³ represents a hydrogen atom, an alkyl group, a substituted alkyl group, an aryl group, a substituted aryl group or the like, R⁴ represents an aryl group, a substituted aryl group, a heteroaryl group or a substituted heteroaryl group, R⁵ represents a hydrogen atom, an alkyl group or a substituted alkyl group, and X represents a hydrogen atom, a substituted or unsubstituted alkyl group or the like.

The following compounds are disclosed as examples of the derivatives described above.

### Example 2

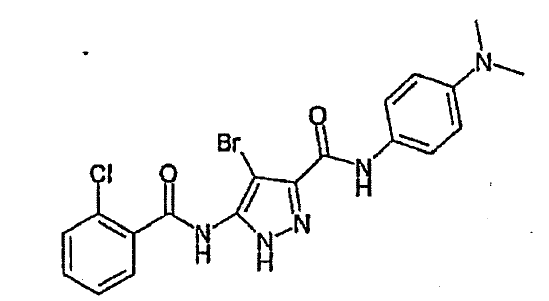

### Example 6

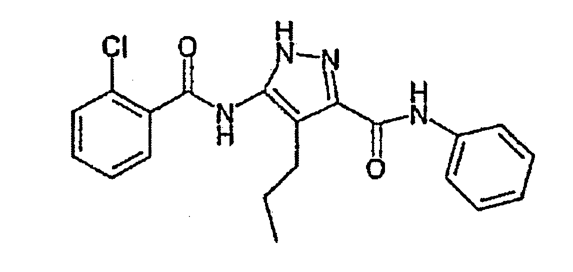

### Example 7

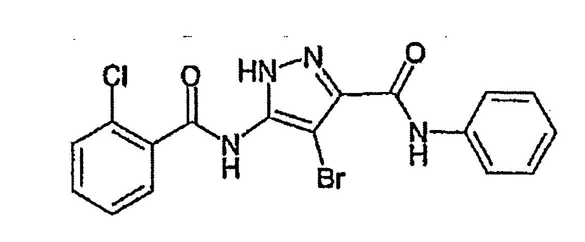

### Example 14

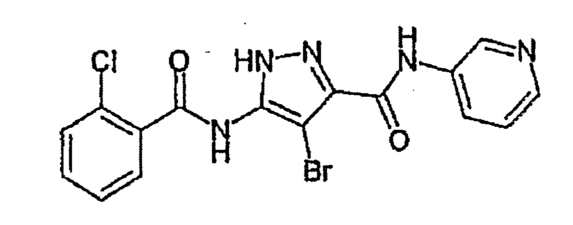

WO2004/072060A1 discloses substituted 3-(benzoylureido)-thiophene derivatives that are useful for treating and preventing type II diabetes.(see Patent Document 18)

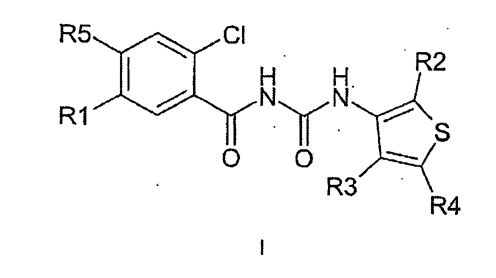

In the formula, R5 represents F, C1 or Br, R1 represents a hydrogen atom, F, Cl or Br, R2 represents a hydrogen atom, F, Cl, Br, an alkyl group or the like, R3 represents a hydrogen atom, an alkyl group or the like, and R4 represents a hydrogen atom, an alkyl group or the like.

WO2004/078743A1 discloses substituted benzoylureidopyridyl-piperizine and-pyrrolidine carboxylic acid derivatives that are useful for treating and preventing type II diabetes. (see Patent Document 19)

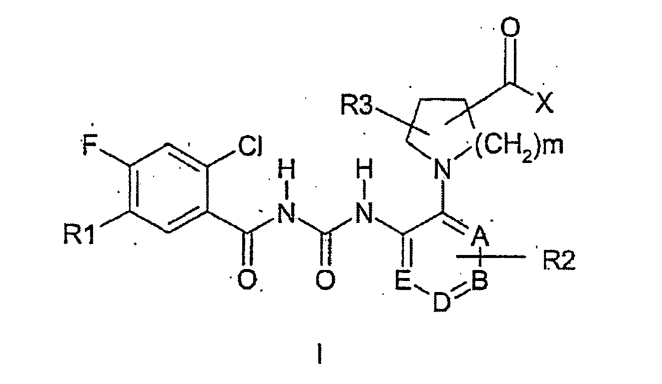

In the formula, R1 and R2 represent a hydrogen atom, F or Cl, X represents OH, NH₂, an alkoxy group or the like, A, B, D and E represent CH or N, and m represents 0, 1 or 2.
[Non-Patent Document 1] JOSLIN' S DIABETES MELLITUS 13Th Edition, pp.521-522
[Non-Patent Document 2] Withers D.J., Endocrinology 141; pp.1917-1921, 2000
[Non-Patent Document 3] Tayek J.A., Am. J. Physiol. 270; pp.E709-E717, 1996
[Non-Patent Document 4] Diraison F., Diabetologa 41; pp.212-220, 1998
[Non-Patent Document 5] William H. Martin, Proc. Natl. Acad. Sci. USA 95; pp.1776-1781, 1998, Judith L., Abstracts from the ADA 61st Scientific Session
[Patent Document 1] Japanese Patent Laid-Open No. 2004-002311
[Patent Document 2] National Publication of International Patent Application No. 2002-536410
[Patent Document 3] National Publication of International Patent Application No. 2002-515064
[Patent Document 4] Japanese Patent Laid-Open No. 2001-247565
[Patent Document 5] WO96/39384 (National Publication of International Patent Application No. 1998-511687)
[Patent Document 6] WO01/94300A1
[Patent Document 7] WO02/096864A1
[Patent Document 8] WO03/015774A1
[Patent Document 9] WO03/084922A1
[Patent Document 10] WO03/084923A1
[Patent Document 11] WO03/104188A1
[Patent Document 12] WO2004/007455A1
[Patent Document 13] WO2004/033416A2
[Patent Document 14] WO2004/065356A1
[Patent Document 15] WO2004/098589A1
[Patent Document 16] WO2004/098590A1
[Patent Document 17] WO2004/099155A2
[Patent Document 18] WO2004/072060A1
[Patent Document 19] WO2004/078743A1

### Disclosure of the Invention

### Problems to be Solved by the Invention

Many antidiabetics currently used are used for pancreas (to promote insulin secretion), periphery (to ameliorate insulin resistance), small intestines (to inhibit glucose absorption) and insulin itself. In recent years, however, there has been a need for the development of an antidiabetic based on a new mode of action, and the development of an antidiabetic that acts against inhibiting liver glycogen phosphorylase has been attracting attention. However, no drug has been developed that has satisfactory activity. No drug has been developed either that meets the requirements in terms of oral absorption and metabolic stability.

Therefore, there has been the need of developing a liver glycogen phosphorylase inhibitor that has potent activity and fewer side effects, and is superior in oral absorbability and metabolic stability compared to conventional antidiabetics.

It is an object of the present invention to provide a liver glycogen phosphorylase inhibitor that has potent activity and fewer side effects, and is superior in oral absorbability and metabolic stability compared to conventional antidiabetics.

It is another object of the invention to provide a therapeutic agent for insulin resistance, diabetic neuropathy, diabetic nephropathy, diabetic retinopathy, cataract, hypercholesterolemia, hypertension, hyperinsulinemia, hyperlipidemia, atherosclerosis, tissue ischemia and myocardial ischemia, a therapeutic agent for appetite control and obesity, and a therapeutic agent for infections such as bacterial, fungal, parasitic or viral infection, wherein the therapeutic agent has a liver glycogen phosphorylase inhibitor as an active ingredient.

### Means for Solving the Problems

In light of the problems described above, intensive studies have been conducted to discover a useful antidiabetic with liver glycogen phosphorylase inhibitory activity, and as a result the pyrazole compounds represented by the following general formula (I) have been found to possess a potent liver glycogen phosphorylase inhibitory activity.

The following items 1 to 57 describe the invention more specifically.
1. A pharmaceutical composition for treating or preventing diabetes, comprising a pyrazole compound represented by the following general formula (I): 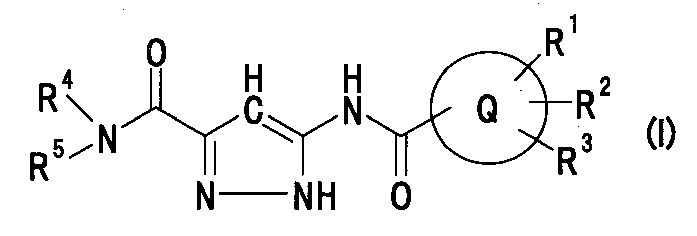 wherein Ring Q represents an aryl group or a heteroaromatic group;
   R¹ represents a hydrogen atom, a halogen atom, a C₁₋₆ alkyl group or a C₁₋₆ alkoxy group;
   R² represents a halogen atom, a C₁₋₆ alkyl group, a C₁₋₆ alkoxy group or an azido group;
   R³ represents a halogen atom, a hydroxyl group, a C₁₋₆ alkyl group, a halo C₁₋₆ alkyl group, a C₁₋₆ alkoxy group, an azido group, an amino group, an acylamino group or a C₁₋₆ alkylsulfonylamino group;
   R⁴ and R⁵ are identical with or different from each other, and represent
      (1) a hydrogen atom;
      (2) a C₁₋₆ alkyl group that may be substituted with one or more substituents selected from Group A below:
         [Group A]
         A1. a hydroxyl group,
         A2. a C₁₋₆ alkoxy group,
         A3. -N(R⁴¹) (R^{41'})
            wherein R⁴¹ and R^{41'} are identical with or different from each other and represent a hydrogen atom or a C₁₋₆ alkyl group, or R⁴¹ and R^{41'} may, together with the adjacent nitrogen atom, forma saturated 5- or 6-memberedmonocyclic heterocyclic ring,
         A4. an aryl group,
         A5. a heteroaromatic group,
         A6. -CO-N (R⁴¹¹) (R^{411'})
            wherein R⁴¹¹ and R^{411'} are identical with or different from each other and represent a hydrogen atom or a C₁₋₆ alkyl group, or R⁴¹¹ and R^{411'} may, together with the adjacent nitrogen atom, form a saturated 5- or 6-memberedmonocyclic heterocyclic ring,
         A7. a carboxy group,
            and,
         A8. a C₁₋₆ alkoxycarbonyl group,
      (3) a C₂₋₆ alkenyl group;
      (4) a C₂₋₆ alkynyl group;
      (5) a C₃₋₈ cycloalkyl group;
         wherein the cycloalkyl group may be substituted with a hydroxyl group, a carboxy group or a C₁₋₆ alkoxycarbonyl group or may be condensed with a pyridine ring,
      (6) a C₃₋₈ cycloalkyl C₁₋₆ alkyl group;
         wherein the cycloalkyl group of the C₃₋₈ cycloalkyl C₁₋₆ alkyl group may be substituted with a hydroxyl group, a carboxy group or a C₁₋₆ alkoxycarbonyl group or may be condensed with a pyridine ring,
      (7) a saturated 5- or 6-memberedmonocyclic heterocyclic group that may be substituted with one or more substituents selected from Group C below:
         [Group C]
         C1. a C₁₋₆ alkyl group,
         C2. an acyl group,
         C3. a C₁₋₆ alkylsulfonyl group,
         C4. a carboxy group,
         C5. a C₁₋₆ alkoxycarbonyl group,
            and,
         C6. -CO-(Alk)n-COOR⁵²,
            wherein R⁵² is a hydrogen atom or a C₁₋₆ alkyl group, Alk is a C₁₋₄ alkylene group, and n is 0 or an integer from 1 to 3,
      (8) an aryl group that may be substituted with one or more substituents selected from Group D below:
         [Group D]
         D1. a hydroxyl group,
         D2. a C₁₋₆ alkoxy group,
         D3. a cyano group,
         D4. a C₁₋₆ alkyl group,
            wherein the C₁₋₆ alkyl group may be substituted with one or more substituents selected from the group consisting of a hydroxyl group, a carboxy group and a C₁₋₆ alkoxycarbonyl group,
         D5 . -N (R⁵³) (R^{53'}),
            wherein R⁵³ and R^{53'} are identical with or different from each other and represent a hydrogen atom, a C₁₋₆ alkyl group or a C₁₋₆ alkylsulfonyl group,
         D6. -CO-N(R ⁵³¹)(R^{531'}),
            wherein R⁵³¹ and R^{531'} are identical with or different from each other and represent a hydrogen atom, a C₁₋₆ alkyl group or a C₁₋₆ alkylsulfonyl group,
         D7. -COOR⁵⁴,
            wherein R⁵⁴ is a hydrogen atom, a C₁₋₆ alkyl group, a C₁₋₆ alkylcarbonyloxy C₁₋₆ alkyl group or a C₃₋₈ cycloalkyloxycarbonyloxy C₁₋₆ alkyl group,
         D8. -C(NH(OH))=N-R⁵⁵,
            wherein R⁵⁵ i's a hydrogen atom or a C₁₋₆ alkylsulfonyl group,
         D9. a saturated 5- or 6-membered monocyclic heterocyclic group,
            and,
         D10. a 5- or 6-membered monocyclic heteroaromatic group that may be substituted with an oxo or thioxo group,
      (9) a 5- or 6-membered monocyclic heteroaromatic group or a 5- or 6-membered condensed heteroaromatic group with a benzene ring, wherein the 5- or 6-membered heteroaromatic groups may be substituted with one or more substituents selected from the group consisting of a carboxy group and a C₁₋₆ alkoxycarbonyl group;
      (10) a C₇₋₁₄ aralkyl group;
         wherein the alkyl moiety of the C₇₋₁₄ aralkyl group may be substituted with one or two substituents selected from Group E below, and the aryl moiety may be substituted with one or more substituents selected from Group F below:
         [Group E]
         E1. a C₁₋₆ alkyl group that may be substituted with a hydroxyl group,
         E2. a cyano group,
         E3. a carboxy group,
         E4. a C₁₋₆ alkoxycarbonyl group,
            and,
         E5. a phenyl group,
         [Group F]
         F1. a C₁₋₆ alkyl group,
         F2. a halogen atom,
         F3. a cyano group,
         F4. a hydroxyl group,
         F5. a C₁₋₆ alkoxy group that may be substituted with one or more substituents selected from the group consisting of a carboxy group and a C₁₋₆ alkoxycarbonyl group,
         F6. a halo C₁₋₆ alkyl group,
         F7. a carboxy group,
         F8. a C₁₋₆ alkoxycarbonyl group,
         F9. -CO-N(R^{56a}) (R^{56a'})
            wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f below:
            [Group f]
            f1. an amino group,
            f2. a mono C₁₋₆ alkylamino group,
            f3. a di C₁₋₆ alkylamino group,
            f4. a carboxy group,
            f5. a C₁₋₆ alkoxycarbonyl group,
            f6. a hydroxyl group,
               and,
            f7. a saturated 5- or 6-membered monocyclic heterocyclic group having at least one nitrogen atom,
         F10. -N(R^{56b}) (R^{56b'}),
            wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
         F11. -N=CR⁵⁷(-N(R⁵⁸)(R^{58'})),
            wherein R⁵⁷ is a hydrogen atom or a C₁₋₆ alkyl group, R⁵⁸ and R^{58'} are identical with or different from each other and represent a hydrogen atom or a C₁₋₆ alkyl group,
         F12. a 5- or 6-membered monocyclic heteroaromatic group, and,
         F13. a methylenedioxy group or an ethylenedioxy group, or,
      (11) a C₁₋₆ alkyl group substituted with one or more identical or different substituents selected from the group consisting of a 5- or 6-membered monocyclic heteroaromatic group and a 5- or 6-membered condensed heteroaromatic group with a benzene ring;
         wherein the heteroaromatic group or the condensed heteroaromatic group may be substituted with one or more substituents selected from Group G below:
         [Group G]
         G1. a C₁₋₆ alkyl group that may be substituted with one or more substituents selected from Group g below:
            [Group g]
            g1. a halogen atom,
            g2. an amino group,
            g3. a mono C₁₋₆ alkylamino group,
            g4. a di C₁₋₆ alkylamino group,
            g5. a C₁₋₆ alkoxycarbonylamino group,
               and,
            g6. a hydroxyimino group,
         G2. a halogen atom,
         G3. a C₁₋₆ alkoxy group that may be substituted with a halogen atom,
         G4. an aryloxy group,
         G5. a cyano group,
         G6. -N (R^{59a}) (R^{59a'}),
            wherein R^{59a} and R^{59a'} are identical with or different from each other and represent a hydrogen atom or a C₁₋₆ alkyl group, or R^{59a} and R^{59a'} may, together with the adjacent nitrogen atom, form a saturated 5- or 6-memberedmonocyclic heterocyclic ring, and the saturated heterocyclic ring may be substituted with one or more identical or different substituents selected from the group consisting of a hydroxyl group, an amino group, a mono C₁₋₆ alkylamino group and °a di C₁₋₆ alkylamino group,
         G7. -CO-N(R^{59b}) (R^{59b'})
            wherein R^{59b} and R^{59b}' are identical with or different from each other and represent a hydrogen atom, a saturated 5- or 6-membered monocyclic heterocyclic group or a C₁₋₆ alkyl group that may be substituted with the heterocyclic group,
         G8. an aryl group,
            and,
         G9. a 5- or 6-membered monocyclic heteroaromatic group that may be substituted with an oxo or thioxo group, or
   R⁴ and R⁵ may, together with the adjacent nitrogen atom, forma saturated 5- or 6-memberedmonocyclic heterocyclic ring, wherein a part of the saturated heterocyclic ring may have a double bond, and the saturated heterocyclic ring may be condensed with a benzene ring to form a condensed ring, the saturated heterocyclic ring which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof, and a pharmaceutically acceptable carrier.
2. The pharmaceutical composition for treating or preventing diabetes according to the above-described 1, comprising a pyrazole compound represented by the following general formula (I): 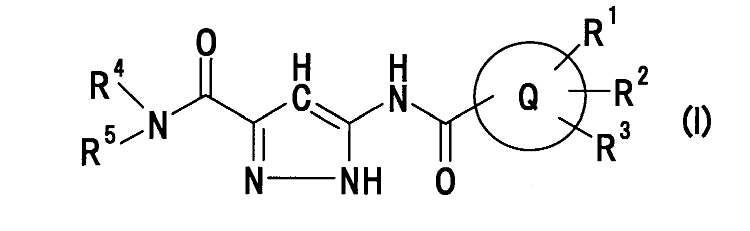 wherein Ring Q represents an aryl group or a heteroaromatic group;
   R¹ represents a hydrogen atom, a halogen atom, a C₁₋₆ alkyl group or a C₁₋₆ alkoxy group;
   R² represents a halogen atom, a C₁₋₆ alkyl group, a C₁₋₆ alkoxy group or an azido group;
   R³ represents a halogen atom, a hydroxyl group, a C₁₋₄ alkyl group, a halo C₁₋₆ alkyl group, a C₁₋₆ alkoxy group, an azido group, an amino group, an acylamino group or a C₁₋₆ alkylsulfonylamino group;
   R⁴ represents
      (1) a hydrogen atom;
      (2) a C₁₋₆ alkyl group that may be substituted with one or more substituents selected from Group A below:
         [Group A]
         A1. a hydroxyl group,
         A2. a C₁₋₆ alkoxy group,
         A3. -N(R⁴¹) (R^{41'})
            wherein R⁴¹ and R^{41'} are identical with or different from each other and represent a hydrogen atom or a C₁₋₆ alkyl group, or R⁴¹ and R^{41'} may, together with the adjacent nitrogen atom, forma saturated 5- or 6-memberedmonocyclic heterocyclic ring,
         A4. an aryl group,
         A5. a heteroaromatic group,
         A6. -CO-N(R⁴¹¹) (R^{411'})
            wherein R⁴¹¹ and R^{411'} are identical with or different from each other and represent a hydrogen atom or a C₁₋₆ alkyl group, or R⁴¹¹ and R^{411'} may, together with the adjacent nitrogen atom, forma saturated 5- or 6-memberedmonocyclic heterocyclic ring,
         A7. a carboxy group,
            and,
         A8. a C₁₋₆ alkoxycarbonyl group,
      (3) a C₂₋₆ alkenyl group;
      (4) a C₂₋₆ alkynyl group;
      (5) a C₃₋₈ cycloalkyl group;
      (6) a C₃₋₈ cycloalkyl C₁₋₆ alkyl group;
         or,
      (7) an aryl group; and
   R⁵ represents
      (1) a C₁₋₆ alkyl group that may be substituted with one or more substituents selected from Group B below:
         [Group B]
         B1. a hydroxyl group,
         B2. a C₁₋₆ alkoxy group,
         B3 . -N(R⁵¹) (R^{51'})
            wherein R⁵¹ and R^{51'} are identical with or different from each other and represent a hydrogen atom or a C₁₋₆ alkyl group, or R⁵¹ and R^{51'} may, together with the adjacent nitrogen atom, form a saturated 5- or 6-memberedmonocyclic heterocyclic ring, and,
         B4. -CO-N(R⁵¹¹) (R^{511'})
            wherein R⁵¹¹ and R^{511'} are identical with or different from each other and represent a hydrogen atom or a C₁₋₆ alkyl group, or R⁵¹¹ and R^{511'} may, together with the adjacent nitrogen atom, forma saturated 5- or 6-memberedmonocyclicheterocyclic ring,
      (2) a C₃₋₈ cycloalkyl group;
         wherein the cycloalkyl group may be substituted with a hydroxyl group, a carboxy group or a C₁₋₆ alkoxycarbonyl group or may be condensed with a pyridine ring,
      (3) a C₃₋₈ cycloalkyl C₁₋₆ alkyl group;
         wherein the cycloalkyl group of the C₃₋₈ cycloalkyl C₁₋₆ alkyl group may be substituted with a hydroxyl group, a carboxy group or a C₁₋₆ alkoxycarbonyl group or maybe condensed with a pyridine ring,
      (4) a saturated 5- or 6-membered monocyclic heterocyclic group that may be substituted with one or more substituents selected from Group C below:
         [Group C]
         C1. a C₁₋₆ alkyl group,
         C2. an acyl group,
         C3. a C₁₋₆ alkylsulfonyl group,
         C4. a carboxy group,
         C5. a C₁₋₆ alkoxycarbonyl group,
            and,
         C6 . -CO-(Alk)n-COOR⁵²,
            wherein R⁵² is a hydrogen atom or a C₁₋₆ alkyl group, Alk is a C₁₋₄ alkylene group, and n is 0 or an integer from 1 to 3,
      (5) an aryl group that may be substituted with one or more substituents selected from Group D below:
         [Group D]
         D1. a hydroxyl group,
         D2. a C₁₋₆ alkoxy group,
         D3. a cyano group,
         D4. a C₁₋₆ alkyl group,
            wherein the C₁₋₆ alkyl group may be substituted with one or more substituents selected from the group consisting of a hydroxyl group, a carboxy group and a C₁₋₆ alkoxycarbonyl group,
         D5. -N(R⁵³) (R^{53'}),
            wherein R⁵³ and R^{53'} are identical with or different from each other and represent a hydrogen atom, a C₁₋₆ alkyl group or a C₁₋₆ alkylsulfonyl group,
         D6. -CO-N (R⁵³¹) (R^{531'})
            wherein R⁵³¹ and R^{531'} are identical with or different from each other and represent a hydrogen atom, a C₁₋₆ alkyl group or a C₁₋₆ alkylsulfonyl group,
         D7. -COOR⁵⁴,
            wherein R⁵⁴ is a hydrogen atom, a C₁₋₆ alkyl group, a C₁₋₆ alkylcarbonyloxy C₁₋₆ alkyl group or a C₃₋₈ cycloalkyloxycarbonyloxy C₁₋₆ alkyl group,
         D8. -C(NH(OH))=N-R⁵⁵,
            wherein R⁵⁵ is a hydrogen atom or a C₁₋₆ alkylsulfonyl group,
         D9. a saturated 5- or 6-membered monocyclic heterocyclic group, and
         D10. a 5- or 6-membered monocyclic heteroaromatic group that may be substituted with an oxo or thioxo group,
      (6) a 5- or 6-membered monocyclic heteroaromatic group or a 5- or 6-membered condensed heteroaromatic group with a benzene ring, wherein the 5- or 6-membered heteroaromatic groups may be substituted with one or more substituents selected from the group consisting of a carboxy group and a C₁₋₆ alkoxycarbonyl group;
      (7) a C₇₋₁₄ aralkyl group;
         wherein the alkyl moiety of the C₇₋₁₄ aralkyl group may be substituted with one or two substituents selected from Group E below, and the aryl moiety may be substituted with one or more substituents selected from Group F below:
         [Group E]
         E1. a C₁₋₆ alkyl group that may be substituted with a hydroxyl group,
         E2. a cyano group,
         E3. a carboxy group,
         E4. a C₁₋₆ alkoxycarbonyl group,
            and,
         E5. a phenyl group,
         [Group F]
         F1. a C₁₋₆ alkyl group,
         F2. a halogen atom,
         F3. a cyano group,
         F4. a hydroxyl group,
         F5. a C₁₋₆ alkoxy group that may be substituted with one or more substituents selected from the group consisting of a carboxy group and a C₁₋₆ alkoxycarbonyl group,
         F6. a halo C₁₋₆ alkyl group,
         F7. a carboxy group,
         F8. a C₁₋₆ alkoxycarbonyl group,
         F9. -CO-N(R^{56a}) (R^{56a'})
            wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f below:
            [Group f]
            f1. an amino group,
            f2. a mono C₁₋₆ alkylamino group,
            f3. a di C₁₋₆ alkylamino group,
            f4. a carboxy group,
            f5. a C₁₋₆ alkoxycarbonyl group,
            f6. a hydroxyl group,
               and,
            f7. a saturated 5- or 6-membered monocyclic heterocyclic group having at least one nitrogen atom,
         F10. -N(R^{56b}) (R^{56b'}),
            wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
         F11 . -N=CR⁵⁷ (-N (R⁵⁸) (R^{58'})),
            wherein R⁵⁷ is a hydrogen atom or a C₁₋₆ alkyl group, R⁵⁸ and R^{58'} are identical with or different from each other and represent a hydrogen atom or a C₁₋₆ alkyl group,
         F12. a 5- or 6-membered monocyclic heteroaromatic group, and,
         F13. a methylenedioxy group or an ethylenedioxy group, or,
      (8) a C₁₋₆ alkyl group substituted with a 5- or 6-membered monocyclic heteroaromatic group or a C₁₋₆ alkyl group substituted with a 5- or 6-membered condensed heteroaromatic group with a benzene ring;
         wherein the heteroaromatic group may be substituted with one or more substituents selected from Group G below:
         [Group G]
         G1. a C₁₋₆ alkyl group that may be substituted with one or more substituents selected from Group g below:
            [Group g]
            g1. a halogen atom,
            g2. an amino group,
            g3. a mono C₁₋₆ alkylamino group,
            g4. a di C₁₋₆ alkylamino group,
            g5. a C₁₋₆ alkoxycarbonylamino group,
               and
            g6. a hydroxyimino group,
         G2. a halogen atom,
         G3. a C₁₋₆ alkoxy group that may be substituted with a halogen atom,
         G4. an aryloxy group,
         G5. a cyano group,
         G6. -N(R^{59a}) (R^{59a'}),
            wherein R^{59a} and R^{59a}' are identical with or different from each other and represent a hydrogen atom or a C₁₋₆ alkyl group, or R^{59a} and R^{59a'} may, together with the adjacent nitrogen atom, forma saturated 5- or 6-memberedmonocyclicheterocyclic ring, and the saturated heterocyclic ring may be substituted with one or more identical or different substituents selected from the group consisting of a hydroxyl group, an amino group, a mono C₁₋₆ alkylamino group and a di C₁₋₆ alkylamino group,
         G7. -CO-N(R^{59b}) (R^{59b'})
            wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
         G8. an aryl group,
            and,
         G9. a 5- or 6-membered monocyclic heteroaromatic group that may be substituted with an oxo or thioxo group,
            or
   R⁴ and R⁵ may, together with the adjacent nitrogen atom, forma saturated 5- or 6-memberedmonocyclic heterocyclic ring, wherein a part of the saturated heterocyclic ring may have a double bond, and the saturated heterocyclic ring may be condensed with a benzene ring to form a condensed ring, and the saturated heterocyclic ring which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof, and a pharmaceutically acceptable carrier.
3. The pharmaceutical composition for treating or preventing diabetes according to the above-described 1, wherein the pyrazole compound is a pyrazole compound represented by the following general formula (II'): 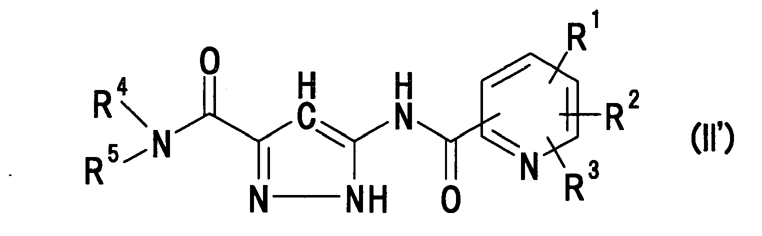 wherein R¹, R², R³, R⁴ and R⁵ are as defined in the above-described 1.
4. The pharmaceutical composition for treating or preventing diabetes according to the above-described 1, wherein the pyrazole compound is a pyrazole compound represented by the following general formula (II): 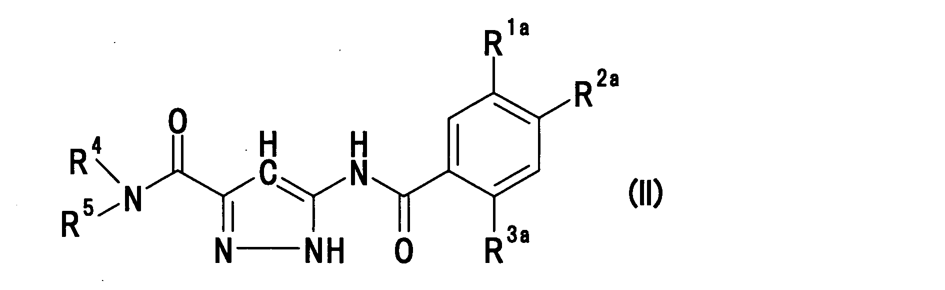 wherein R⁴ and R⁵ are as defined in the above-described 1, R^{1a} represents a halogen atom or a hydrogen atom, R^{2a} a halogen atom, and R^{3a} a halogen atom or a C₁₋₆ alkyl group.
5. The pharmaceutical composition for treating or preventing diabetes according to the above-described 4, wherein R^{1a} is a hydrogen or fluorine atom, R^{2a} is a fluorine or chlorine atom, and R^{3a} is a chlorine atom or a C₁₋₆ alkyl group.
6. The pharmaceutical composition for treating or preventing diabetes according to the above-described 5, wherein the pyrazole compound is a pyrazole compound represented by the following general formula (III): 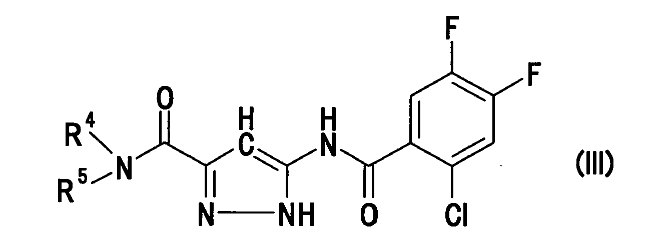 wherein R⁴ and R⁵ are as defined above.
7. The pharmaceutical composition for treating or preventing diabetes according to any of the above-described 2 to 6, wherein R⁴ is a group selected from the following group:
   (1) a hydrogen atom;
   (2) a C₁₋₆ alkyl group that may be substituted with one or more substituents selected from Group A' below:
      [Group A']
      A'1. a hydroxyl group,
      A'2. a C₁₋₄ alkoxy group,
      A'3. -N(R⁴¹) (R^{41'})
         wherein R⁴¹ and R^{41'} are identical with or different from each other and represent a hydrogen atom or a C₁₋₄ alkyl group, or R⁴¹ and R^{41'} may, together with the adjacent nitrogen atom, forma saturated 5- or 6-memberedmonocyclic heterocyclic ring,
      A'4. a phenyl group,
      A'5 . a 5- or 6-membered monocyclic heteroaromatic group,
      A'6. -CO-N(R⁴¹¹) (R^{411'})
         wherein _{R}⁴¹¹ and _{R}⁴¹¹ ' are identical with or different from each other and represent a hydrogen atom or a C₁₋₄ alkyl group, or R⁴¹¹ and R^{411'} may, together with the adjacent nitrogen atom, form a saturated 5- or 6-membered monocyclic heterocyclic ring,
      A'7. a carboxy group, and
      A'8. a C₁₋₄ alkoxycarbonyl group,
   (3) a C₂₋₆ alkenyl group;
   (4) a C₂₋₆ alkynyl group;
   (5) a C₃₋₈ cycloalkyl group;
      and,
   (6) a C₃₋₈ cycloalkyl C₁₋₄ alkyl group.
8. The pharmaceutical composition for treating or preventing diabetes according to any of the above-described 2 to 7, wherein R⁵ is a C₁₋₆ alkyl group.
9. The pharmaceutical composition for treating or preventing diabetes according to any of the above-described 2 to 7, wherein R⁵ is a C₃₋₈ cycloalkyl group that may be substituted with one or more identical or different substituents selected from the group consisting of a hydroxyl group, a carboxy group and a C₁₋₆ alkoxycarbonyl group.
10. The pharmaceutical composition for treating or preventing diabetes according to any of the above-described 2 to 7, wherein R⁵ is a C₃₋₈ cycloalkyl C₁₋₆ alkyl group.
11. The pharmaceutical composition for treating or preventing diabetes according to any of the above-described 2 to 7, wherein R⁵ is a saturated 5- or 6-membered monocyclic heterocyclic group that may be substituted with one or more substituents selected from the group consisting of an acyl group and -CO-(Alk)n-COOR⁵², wherein R⁵² is a hydrogen atom or a C₁₋₆ alkyl group, Alk is a C₁₋₄ alkylene group, and n is 0 or an integer from 1 to 3.
12. The pharmaceutical composition for treating or preventing diabetes according to any of the above-described 2 to 7, wherein R⁵ is a phenyl group that may be substituted with one or more substituents selected from the following Group D':
   [Group D']
   D'1. a C₁₋₄ alkyl group that may be substituted with a carboxy group,
   D'2. -CO-N(R⁵³) (R^{53'}),
      wherein R⁵³ and R^{53'} are identical with or different from each other and represent a hydrogen atom, a C₁₋₄ alkyl group or a C₁₋₄ alkylsulfonyl group,
   D'3. -COOR⁵⁴,
      wherein _{R}⁵⁴ is a hydrogen atom, a C₁₋₄ alkyl group, a C₁₋₄ alkylcarbonyloxy C₁₋₄ alkyl group or a C₃₋₈ cycloalkyloxycarbonyloxy C₁₋₄ alkyl group,
      and,
   D'4. a 5- or 6-membered monocyclic heteroaromatic group that may be substituted with an oxo or thioxo group.
13. The pharmaceutical composition for treating or preventing diabetes according to any of the above-described 2 to 7, wherein R⁵ is a C₇₋₁₄ aralkyl group and the aryl moiety of the C₇₋₁₄ aralkyl group may be substituted with one or more substituents selected from the following Group F':
   [Group F']
   F'1. a C₁₋₆ alkyl group,
   F'2. a halogen atom,
   F'3. a cyano group,
   F'4. a hydroxyl group,
   F'5. a C₁₋₆ alkoxy group that may be substituted with one or more substituents selected from the group consisting of a carboxy group and a C₁₋₆ alkoxycarbonyl group,
   F'6. a halo C₁₋₆ alkyl group,
   F'7. a carboxy group,
   F'8. a C₁₋₆ alkoxycarbonyl group,
   F'9. -CO-N (R^{56a}) (R^{56a'}),
      wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from the following Group f':
      [Group f']
      f'1. an amino group,
      f'2. a mono C₁₋₆ alkylamino group,
      f'3. a di C₁₋₆ alkylamino group,
      f'4. a carboxy group,
      f'5. a C₁₋₆ alkoxycarbonyl group,
      f'6. a hydroxyl group,
         and,
      f'7. a saturated 5- or 6-membered monocyclic heterocyclic group having at least one nitrogen atom,
   F'10. -N(R^{56b}) (R^{56b'})
      wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
   F'11. -N=CR⁵⁷ (-N (R⁵⁸) (R^{58'})),
      wherein R⁵⁷ is a hydrogen atom or a C₁₋₄ alkyl group, R⁵⁸ and R^{58'} are identical with or different from each other and represent a hydrogen atom or a C₁₋₄ alkyl group,
   F'12. a 5- or 6-membered monocyclic heteroaromatic group, and,
   F'13. a methylenedioxy group or an ethylenedioxy group.
14. The pharmaceutical composition for treating or preventing diabetes according to any of the above-described 2 to 7, wherein R⁵ is a C₁₋₆ alkyl group substituted with a 5- or 6-membered monocyclic heteroaromatic group and the heteroaromatic group may be substituted with one or more substituents selected from the following Group G':
   [Group G']
   G'1. a C₁₋₆ alkyl group that may be substituted with one or more substituents selected from Group g' below:
      [Group g']
      g'1. a halogen atom,
      g'2. an amino group,
      g'3. a mono C₁₋₆ alkylamino group,
      g'4. a di C₁₋₆ alkylamino group,
      g'5. a C₁₋₆ alkoxycarbonylamino group,
         and,
      g'6. a hydroxyimino group,
   G'2. a halogen atom,
   G'3. a C₁₋₆ alkoxy group that may be substituted with a halogen atom,
   G'4. an aryloxy group,
   G'5. a cyano group,
   G'6. -N(R^{59a}) (R^{59a'}),
      wherein R^{59a} and R^{59a'} are identical with or different from each other and represent a hydrogen atom or a C₁₋₆ alkyl group, or R^{59a} and R^{59a'} may, together with the adjacent nitrogen atom, forma saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
   G'7. -CO-N(R^{59b}) (R^{59b'})
      wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
   G'8. an aryl group,
      and,
   G'9. a 5- or 6-membered monocyclic heteroaromatic group that may be substituted with an oxo or thioxo group.
15. The pharmaceutical composition for treating or preventing diabetes according to any of the above-described 2 to 6, wherein R⁵ and R⁴, together with the adjacent nitrogen atom, form a saturated 5- or 6-memberedmonocyclic heterocyclic group,
   wherein a part of the saturated heterocyclic group may have a double bond, and the saturated heterocyclic group may be condensed with a benzene ring to form a condensed ring, and the saturated heterocyclic group which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group.
16. The pharmaceutical composition for treating or preventing diabetes according to the above-described 2, wherein the pyrazole compound is a pyrazole compound represented by the following general formula (IV): 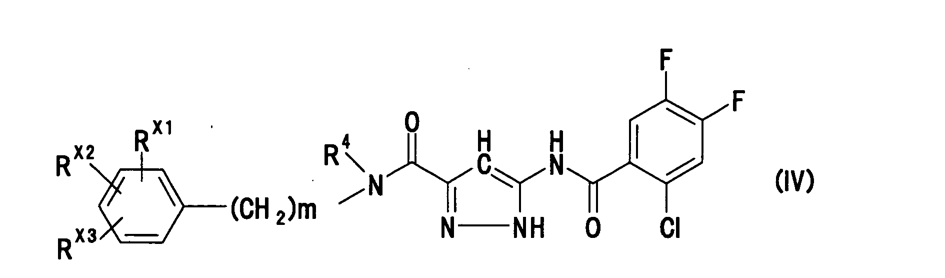 wherein R⁴ is as described in 2, R^{X1}, R^{X2} and R^{X3} are identical with or different from each other and represent a hydrogen atom or a substituent selected from the following Group F'', and m is 0 or an integer from 1 to 2,
   [Group F'']
   F''1. a C₁₋₆ alkyl group,
   F"2. a halogen atom,
   F''3. a cyano group,
   F"4. a hydroxyl group,
   F''5. a C₁₋₄ alkoxy group that may be substituted with one or more substituents selected from the group consisting of a carboxy group and a C₁₋₄ alkoxycarbonyl group,
   F''6. a halo C₁₋₆ alkyl group,
   F''7. a carboxy group,
   F''8. a C₁₋₆ alkoxycarbonyl group,
   F''9. -CO-N(R^{56a}) (R^{56a'})
      wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f'' below:
      [Group f'']
      f''1. an amino group,
      f''2. a mono C₁₋₆ alkylamino group,
      f''3. a di C₁₋₆ alkylamino group,
      f''4. a carboxy group,
      f''5. a C₁₋₆ alkoxycarbonyl group,
      f''6. a hydroxyl group,
         and,
      f''7. a saturated 5- or 6-membered monocyclic heterocyclic group having at least one nitrogen atom,
   F''10. -N (R^{56b}) (R^{56b'})
      wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
   F''11. -N=CR⁵⁷(-N(R⁵⁸) (R^{58'})),
      wherein R⁵⁷ is a hydrogen atom or a C₁₋₆ alkyl group, R⁵⁸ and R^{58'} are identical with or different from each other and represent a hydrogen atom or a C₁₋₆ alkyl group,
   F''12. a 5- or 6-membered monocyclic heteroaromatic group,
      and,
   F''13. a methylenedioxy group or an ethylenedioxy group.
17. The pharmaceutical composition for treating or preventing diabetes according to the above-described 2, wherein the pyrazole compound is a pyrazole compound represented by the following general formula (V): 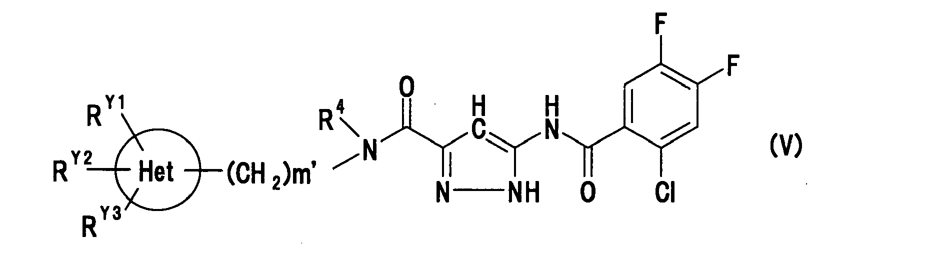 wherein R⁴ is as defined in the above-described 2, the ring Het represents a heteroaromatic group of a 5- or 6-membered monocyclic ring, R^{Y1}, R^{Y2} and R^{Y3} are identical with or different from each other and represent a hydrogen atom or a substituent selected from the following Group G', and m' is 0 or an integer from 1 to 2,
   [Group G']
   G'1. a C₁₋₆ alkyl group that may be substituted with one or more substituents selected from Group g' below:
      [Group g']
      g'1. a halogen atom,
      g'2. an amino group,
      g'3. a mono C₁₋₆ alkylamino group,
      g'4. a di C₁₋₆ alkylamino group,
      g'5. a C₁₋₆ alkoxycarbonylamino group,
         and,
      g'6. a hydroxyimino group,
   G'2. a halogen atom,
   G'3. a C₁₋₆ alkoxy group that may be substituted with a halogen atom,
   G'4. an aryloxy group,
   G'5. a cyano group,
   G'6. -N(R^{59a}) (R^{59a'}),
      wherein R^{59a} and R^{59a'} are identical with or different from each other and represent a hydrogen atom or a C₁₋₆ alkyl group, or R^{59a} and R^{59a'} may, together with the adjacent nitrogen atom, form a saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
   G'7. -CO-N(R^{59b}) (R^{59b'})
      wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
   G'8. an aryl group,
      and,
   G'9. a 5- or 6-membered monocyclic heteroaromatic group that may be substituted with an oxo or thioxo group.
18. The pharmaceutical composition for treating or preventing diabetes according to the above-described 17, wherein the ring Het is a pyridine ring, a triazole ring or an oxazole ring.
19. The pharmaceutical composition for treating or preventing diabetes according to the above-described 18, wherein the pyrazole compound is a pyrazole compound represented by the following general formula (VI): 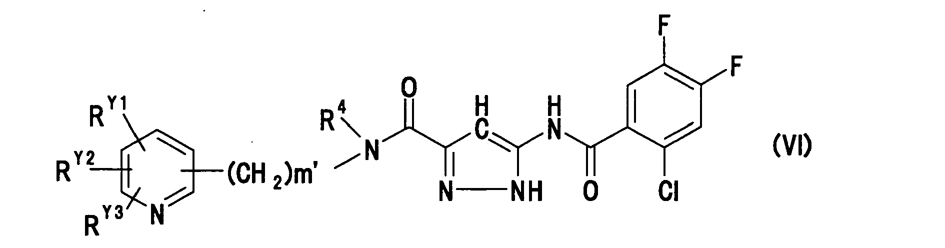 wherein R⁴, R^{Y1}, R^{Y2}, R^{Y3} and m' are as described above.
20. The pharmaceutical composition for treating or preventing diabetes according to the above-described 2, wherein the pyrazole compound or a pharmacologically acceptable salt thereof is selected from the following group:
   5-(2-Chloro-4,5-difluoro-benzoylamino)-1H-pyrazole-3-carboxylic acid (pyridin-3-ylmethyl)-amide,
   5-(2-Chloro-4,5-difluoro-benzoylamino)-1H-pyrazole-3-carboxylic acid (pyridin-3-ylmethyl)-amide dihydrochloride,
   5-(2-Chloro-4,5-difluoro-benzoylamino)-1H-pyrazole-3-carboxylic acid (pyridin-3-ylmethyl)-amide hydrochloride,
   5-(2-Chloro-4,5-difluoro-benzoylamino)-1H-pyrazole-3-carboxylic acid (4-methyl-oxazol-5-ylmethyl)-amide,
   5-(2-Chloro-4,5-difluoro-benzoylamino)-1H-pyrazole-3-carboxylic acid (4-methyl-oxazol-5-ylmethyl)-amide,
   5-(2-Chloro-4,5-difluoro-benzoylamino)-1H-pyrazole-3-carboxylic acid
   (4-methyl-oxazol-5-ylmethyl)amide-p-toluenesulfonate, 5-(2-Chloro-4,5-difluoro-benzoylamino)-1H-pyrazole-3-carboxylic acid
   (4-methyl-oxazol-5-ylmethyl)amide-L-(+)-tartrate,
   5-(2-Chloro-4,5-difluoro-benzoylamino)-1H-pyrazole-3-carboxylic acid (2,4-dimethyl-oxazol-5-ylmethyl)-amide p-toluenesulfonate,
   5-(2-Chloro-4,5-difluoro-benzoylamino)-1H-pyrazole-3-carboxylic acid (2,4-dimethyl-oxazol-5-ylmethyl)-amide,
   5-(2-Chloro-4,5-difluoro-benzoylamino)-1H-pyrazole-3-carboxylic acid (2,4-dimethyl-pyridin-3-ylmethyl)amide,
   5-(2-Chloro-4,5-difluoro-benzoylamino)-1H-pyrazole-3-carboxylic acid diethylamide,
   5-(2-Chloro-4,5-difluoro-benzoylamino)-1H-pyrazole-3-carboxylic acid ethyl-(pyridin-4-ylmethyl)-amide,
   5-(2-Chloro-4,5-difluoro-benzoylamino)-1H-pyrazole-3-carboxylic acid (2-methoxy-ethyl)-methyl-amide,
   5-(2-Chloro-4,5-difluoro-benzoylamino)-1H-pyrazole-3-carboxylic acid ethyl-(2-methoxy-ethyl)-amide,
   5- (2-Chloro-4,5-difluoro-benzoylamino) -1H-pyrazole-3-carboxylic acid 2-fluoro-benzylamide,
   5-(2-Chloro-4,5-difluoro-benzoylamino)-1H-pyrazole-3-carboxylic acid 3-fluoro-benzylamide,
   5-(2-Chloro-4,5-difluoro-benzoylamino)-1H-pyrazole-3-carboxylic acid 2-dimethylamino-benzylamide,
   5-(2-Chloro-4,5-difluoro-benzoylamino)-1H-pyrazole-3-carboxylic acid
   (4-ethoxycarbonyl-cyclohexyl)-propyl-amide,
   5-(2-Chloro-4,5-difluoro-benzoylamino)-1H-pyrazole-3-carboxylic acid (6-methoxy-pyridin-3-ylmethyl)-amide,
   5-(2-Chloro-4,5-difluoro-benzoylamino)-1H-pyrazole-3-carboxylic acid 2,3-dimethoxy-benzylamide,
   5-(2-Chloro-4,5-difluoro-benzoylamino)-1H-pyrazole-3-carboxylic acid 2,4-dimethoxy-benzylamide,
   5-(2-Chloro-4,5-difluoro-benzoylamino)-1H-pyrazole-3-carboxylic acid 2,6-dimethoxy-benzylamide,
   5-(2-Chloro-4,5-difluoro-benzoylamino)-1H-pyrazole-3-carboxylic acid 3,5-dimethoxy-benzylamide,
   5-(2-Chloro-4,5-difluoro-benzoylamino)-1H-pyrazole-3-carboxylic acid 3,4-dimethoxy-benzylamide,
   5-(2-Chloro-4,5-difluoro-benzoylamino)-1H-pyrazole-3-carboxylic acid 2,3-difluoro-benzylamide,
   5-(2-Chloro-4,5-difluoro-benzoylamino)-1H-pyrazole-3-carboxylic acid 2,4-difluoro-benzylamide,
   5-(2-Chloro-4,5-difluoro-benzoylamino)-1H-pyrazole-3-carboxylic acid 2,5-difluoro-benzylamide,
   5- (2-Chloro-4,5-difluoro-benzoylamino) -1H-pyrazole-3-carboxylic acid 2,6-difluoro-benzylamide,
   5-(2-Chloro-4,5-difluoro-benzoylamino)-1H-pyrazole-3-carboxylic acid 3,4-difluoro-benzylamide,
   5-(2-Chloro-4,5-difluoro-benzoylamino)-1H-pyrazole-3-carboxylic acid 3,5-difluoro-benzylamide,
   5-(2-Chloro-4,5-difluoro-benzoylamino)-1H-pyrazole-3-carboxylic acid (2-isopropoxy-pyridin-3-ylmethyl)-amide,
   5-(2-Chloro-4,5-difluoro-benzoylamino)-1H-pyrazole-3-carboxylic acid (2-phenoxy-pyridin-3-ylmethyl)-amide,
   5-(2-Chloro-4,5-difluoro-benzoylamino)-1H-pyrazole-3-carboxylic acid (3,5-difluoro-pyridin-2-ylmethyl)-amide,
   5-(2-Chloro-4,5-difluoro-benzoylamino)-1H-pyrazole-3-carboxylic acid 2-trifluoromethyl-benzylamide,
   5-(2-Chloro-4,5-difluoro-benzoylamino)-1H-pyrazole-3-carboxylic acid 3-trifluoromethyl-benzylamide
   5-(2-Chloro-4,5-difluoro-benzoylamino)-1H-pyrazole-3-carboxylic acid 4-trifluoromethyl-benzylamide,
   5-(2-Chloro-4,5-difluoro-benzoylamino)-1H-pyrazole-3-carboxylic acid 4-tert-butyl-benzylamide,
   5-(2-Chloro-4,5-difluoro-benzoylamino)-1H-pyrazole-3-carboxylic acid (6-isopropoxy-pyridin-3-ylmethyl)-amide,
   5-(2-Chloro-4,5-difluoro-benzoylamino)-1H-pyrazole-3-carboxylic acid
   [2-(2,2,2-trifluoro-ethoxy)-pyridin-3-ylmethyl]-amide, 5-(2-Chloro-4,5-difluoro-benzoylamino)-1H-pyrazole-3-carboxylic acid
   4-(2-dimethylamino-ethylcarbamoyl)-benzylamide,
   5-(2-Chloro-4,5-difluoro-benzoylamino)-1H-pyrazole-3-carboxylic acid 4-ethyl-benzylamide,
   5-(2-Chloro-4,5-difluoro-benzoylamino)-1H-pyrazole-3-carboxylic acid 4-isopropyl-benzylamide,
   5-(2-Chloro-4,5-difluoro-benzoylamino)-1H-pyrazole-3-carboxylic acid 2-chloro-6-fluoro-benzylamide,
   5-(2-Chloro-4,5-difluoro-benzoylamino)-1H-pyrazole-3-carboxylic acid (6-ethoxy-pyridin-3-ylmethyl)-amide dihydrochloride,
   5-(2-Chloro-4,5-difluoro-benzoylamino)-1H-pyrazole-3-carboxylic acid (thiazol-4-ylmethyl)-amide,
   5-(2-Chloro-4,5-difluoro-benzoylamino)-1H-pyrazole-3-carboxylic acid
   [6-(2,2,2-trifluoro-ethoxy)-pyridin-3-ylmethyl]-amide dihydrochloride,
   5-(2-Chloro-4,5-difluoro-benzoylamino)-1H-pyrazole-3-carboxylic acid 2,6-dimethyl-benzylamide,
   5-(2-Chloro-4,5-difluoro-benzoylamino)-1H-pyrazole-3-carboxylic acid
   [2-(pyridin-3-yl)-thiazol-4-ylmethyl]-amide dihydrochloride,
   5-(2-Chloro-4,5-difluoro-benzoylamino)-1H-pyrazole-3-carboxylic acid (1H-benzoimidazol-2-ylmethyl)-amide dihydrochloride,
   [4-({[5-(2-Chloro-4,5-difluoro-benzoylamino)-1H-pyrazole-3-carbonyl]-amino}-methyl)-benzoylamino]-acetic acid methyl ester,
   3-[4-({[5-(2-Chloro-4,5-difluoro-benzoylamino)-1H-pyrazole-3-carbonyl]-amino}-methyl)-benzoylamino]-propioni c acid methyl ester
   5-(2-Chloro-4,5-difluoro-benzoylamino)-1H-pyrazole-3-carboxylic acid (2-methyl-thiazol-4-ylmethyl)-amide dihydrochloride,
   5-(2-Chloro-4,5-difluoro-benzoylamino)-1H-pyrazole-3-carboxylic acid (3,5-dimethyl-isoxazol-4-ylmethyl)-amide,
   5-(2-Chloro-4,5-difluoro-benzoylamino)-1H-pyrazole-3-carboxylic acid
   4-[2-bis-(2-acetoxyethyl)-amino-ethylcarbamoyl]-benzylamide,
   5-(2-Chloro-4,5-difluoro-benzoylamino)-1H-pyrazole-3-carboxylic acid4-(2-hydroxy-ethylcarbamoyl)-benzylamide,
   5-(2-Chloro-4,5-difluoro-benzoylamino)-1H-pyrazole-3-carboxylic acid
   4-{2-[(2-acetoxyethyl)-(2-hyroxyethyl)-amino]-ethylcarbamoyl}-benzylamide,
   5-(2-Chloro-4,5-difluoro-benzoylamino)-1H-pyrazole-3-carboxylic acid
   (1-methyl-1H-benzoimidazol-2-ylmethyl)-amide hydrochloride,
   5-(2-Chloro-4,5-difluoro-benzoylamino)-1H-pyrazole-3-carboxylic acid (thiazol-2-ylmethyl)-amide dihydrochloride,
   5-(2-Chloro-4,5-difluoro-benzoylamino)-1H-pyrazole-3-carboxylic acid (benzothiazol-2-ylmethyl)-amide dihydrochloride,
   5-(2-Chloro-4,5-difluoro-benzoylamino)-1H-pyrazole-3-carboxylic acid (2,5-dimethyl-thiazol-4-ylmethyl)-amide dihydrochloride,
   5-(2-Chloro-4,5-difluoro-benzoylamino)-1H-pyrazole-3-carboxylic acid [2-(morpholin-4-yl)-thiazol-4-ylmethyl]-amide dihydrochloride,
   5-(2-Chloro-4,5-difluoro-benzoylamino)-1H-pyrazole-3-carboxylic acid (1,3,5-trimethyl-1H-pyrazol-4-ylmethyl)-amide,
   5-(2-Chloro-4,5-difluoro-benzoylamino)-1H-pyrazole-3-carboxylic acid (2-chloro-6-methyl-pyridin-3-ylmethyl)-amide dihydrochloride,
   5-(2-Chloro-4,5-difluoro-benzoylamino)-1H-pyrazole-3-carboxylic acid (2-dimethylamino-thiazol-4-ylmethyl)-amide dihydrochloride,
   5-(2-Chloro-4,5-difluoro-benzoylamino)-1H-pyrazole-3-carboxylic acid (1-methyl-1H-pyrrol-2-ylmethyl)-amide,
   5-(2-Chloro-4,5-difluoro-benzoylamino)-1H-pyrazole-3-carboxylic acid (3,4-dimethoxy-pyridin-2-ylmethyl)-amide dihydrochloride,
   5-(2-Chloro-4,5-difluoro-benzoylamino)-1H-pyrazole-3-carboxylic acid (5-tert-butyl-thiazol-2-ylmethyl)-amide dihydrochloride,
   5-(2-Chloro-4,5-difluoro-benzoylamino)-1H-pyrazole-3-carboxylic acid (5-methyl-2-phenyl-2H-[1,2,3]triazol-4-ylmethyl)-amide,
   5-(2-Chloro-4,5-difluoro-benzoylamino)-1H-pyrazole-3-carboxylic acid [4-(tert-butoxycarbonyl-amino)-methyl-pyridin-2-ylmethyl]-amide,
   5-(2-Chloro-4,5-difluoro-benzoylamino)-1H-pyrazole-3-carboxylic acid [4-methyl-2-(morpholin-4-yl)-thiazol-5-ylmethyl]-amide dihydrochloride,
   5-(2-Chloro-4,5-difluoro-benzoylamino)-1H-pyrazole-3-carboxylic acid (pyridin-3-ylmethyl)-amide 3/2hydrochloride hemihydrate,
   5-(2-Chloro-4,5-difluoro-benzoylamino)-1H-pyrazole-3-carboxylic acid 2-amino-benzylamide,
   5-(2-Chloro-4,5-difluoro-benzoylamino)-1H-pyrazole-3-carboxylic acid 2-methoxy-benzylamide,
   5-(2-Chloro-4,5-difluoro-benzoylamino)-1H-pyrazole-3-carboxylic acid 3-methoxy-benzylamide,
   5-(2-Chloro-4,5-difluoro-benzoylamino)-1H-pyrazole-3-carboxylic acid 4-methoxy-benzylamide,
   5-(2-Chloro-4,5-difluoro-benzoylamino)-1H-pyrazole-3-carboxylic acid 4-methyl-benzylamide,
   5-(2-Chloro-4,5-difluoro-benzoylamino)-1H-pyrazole-3-carboxylic acid 4-fluoro-benzylamide,
   5-(2-Chloro-4,5-difluoro-benzoylamino)-1H-pyrazole-3-carboxylic acid 4-chloro-benzylamide,
   5-(2-Chloro-4,5-difluoro-benzoylamino)-1H-pyrazole-3-carboxylic acid 4-ethoxycarbonyl-benzylamide,
   5-(2-Chloro-4,5-difluoro-benzoylamino)-1H-pyrazole-3-carboxylic acid 4-carboxy-benzylamide,
   5-(2-Chloro-4,5-difluoro-benzoylamino)-1H-pyrazole-3-carboxylic acid dibenzylamide,
   5-(2-Chloro-4,5-difluoro-benzoylamino)-1H-pyrazole-3-carboxylic acid (cyano-phenyl-methyl)-amide,
   5-(2-Chloro-4,5-difluoro-benzoylamino)-1H-pyrazole-3-carboxylic acid cyclohexylmethyl-amide,
   5-(2-Chloro-4,5-difluoro-benzoylamino)-1H-pyrazole-3-carboxylic acid (2-hydroxy-ethyl)-phenyl-amide,
   5-(2-Chloro-4,5-difluoro-benzoylamino)-1H-pyrazole-3-carboxylic acid cyclohexyl-methyl-amide,
   5-(2-Chloro-4,5-difluoro-benzoylamino)-1H-pyrazole-3-carboxylic acid cyclohexylamide,
   5-(2-Chloro-4,5-difluoro-benzoylamino)-1H-pyrazole-3-carboxylic acid cyclohexyl-ethyl-amide,
   5-(2-Chloro-4,5-difluoro-benzoylamino)-1H-pyrazole-3-carboxylic acid allyl-cyclohexyl-amide,
   5-(2-Chloro-4,5-difluoro-benzoylamino)-1H-pyrazole-3-carboxylic acid cyclohexyl-(pyridin-2-ylmethyl)-amide,
   5-(2-Chloro-4,5-difluoro-benzoylamino)-1H-pyrazole-3-carboxylic acid methyl-(1-methyl-piperidin-4-yl)-amide,
   5-(2-Chloro-4,5-difluoro-benzoylamino)-1H-pyrazole-3-carboxylic acid [(3,4-methylenedioxyphenyl)-methyl]-amide hydrochloride,
   5-(2-Chloro-4,5-difluoro-benzoylamino)-1H-pyrazole-3-carboxylic acid benzyl-butyl-amide,
   5-(2-Chloro-4,5-difluoro-benzoylamino)-1H-pyrazole-3-carboxylic acid benzyl-(4-hydroxy-butyl)-amide hydrochloride,
   5-(2-Chloro-4,5-difluoro-benzoylamino)-1H-pyrazole-3-carboxylic acid butyl-ethyl-amide,
   5-(2-Chloro-4,5-difluoro-benzoylamino)-1H-pyrazole-3-carboxylic acid cyclohexyl-propyl-amide,
   5-(2-Chloro-4,5-difluoro-benzoylamino)-1H-pyrazole-3-carboxylic acid butyl-cyclohexyl-amide,
   5-(2-Chloro-4,5-difluoro-benzoylamino)-1H-pyrazole-3-carboxylic acid cyclohexyl-(2-methoxy-ethyl)-amide,
   5-(2-Chloro-4,5-difluoro-benzoylamino)-1H-pyrazole-3-carboxylic acid cyclohexyl-(pyridin-3-ylmethyl)-amide,
   5-(2-Chloro-4,5-difluoro-benzoylamino)-1H-pyrazole-3-carboxylic acid [2-(morpholin-4-yl)-phenyl]-amide,
   5-(2-Chloro-4,5-difluoro-benzoylamino)-1H-pyrazole-3-carboxylic acid dibutylamide,
   5-(2-Chloro-4,5-difluoro-benzoylamino)-1H-pyrazole-3-carboxylic acid bis-(2-methoxy-ethyl)-amide,
   5-(2-Chloro-4,5-difluoro-benzoylamino)-1H-pyrazole-3-carboxylic acid (2-methoxy-ethyl)-propyl-amide,
   5-(2-Chloro-4,5-difluoro-benzoylamino)-1H-pyrazole-3-carboxylic acid butyl-(tetrahydro-pyran-4-yl)-amide,
   5-(2-Chloro-4,5-difluoro-benzoylamino)-1H-pyrazole-3-carboxylic acid (2-methoxy-ethyl)-(tetrahydro-pyran-4-yl)-amide,
   5-(2-Chloro-4,5-difluoro-benzoylamino)-1H-pyrazole-3-carboxylic acid cyclopentyl-(2-methoxy-ethyl)-amide,
   5-(2-Chloro-4,5-difluoro-benzoylamino)-1H-pyrazole-3-carboxylic acid cyclohexyl-(3-methoxy-propyl)-amide,
   5-(2-Chloro-4,5-difluoro-benzoylamino)-1H-pyrazole-3-carboxylic acid cyclohexyl-(2-ethoxy-ethyl)-amide,
   5-(2-Chloro-4,5-difluoro-benzoylamino)-1H-pyrazole-3-carboxylic acid cyclohexyl-(2-isopropoxy-ethyl)-amide,
   5-(2-Chloro-4,5-difluoro-benzoylamino)-1H-pyrazole-3-carboxylic acid cyclohexyl-(2-propoxy-ethyl)-amide,
   5-(2-Chloro-4,5-difluoro-benzoylamino)-1H-pyrazole-3-carboxylic acid (1-ethyl-propyl)-(2-methoxy-ethyl)-amide,
   5-(2-Chloro-4,5-difluoro-benzoylamino)-1H-pyrazole-3-carboxylic acid butyl- (1-tert-butoxycarbonyl-piperidin-4-yl) -amide,
   5-(2-Chloro-4,5-difluoro-benzoylamino)-1H-pyrazole-3-carboxylic acid butyl-(tetrahydro-thiopyran-4-yl)-amide,
   5-(2-Chloro-4,5-difluoro-benzoylamino)-1H-pyrazole-3-carboxylic acid (2-methoxy-ethyl)-amide,
   5-(2-Chloro-4,5-difluoro-benzoylamino)-1H-pyrazole-3-carboxylic acid butyl-(pyridin-3-ylmethyl)-amide,
   5-(2-Chloro-4,5-difluoro-benzoylamino)-1H-pyrazole-3-carboxylic acid benzyl-(2-methoxy-ethyl)-amide,
   5-(2-Chloro-4,5-difluoro-benzoylamino)-1H-pyrazole-3-carboxylic acid butyl-(piperidin-4-yl)-amide,
   5-(2-Chloro-4,5-difluoro-benzoylamino)-1H-pyrazole-3-carboxylic acid (1-acetyl-piperidin-4-yl)-butyl-amide,
   5-(2-Chloro-4,5-difluoro-benzoylamino)-1H-pyrazole-3-carboxylic acid butyl-(1-methanesulfonyl-piperidin-4-yl)-amide,
   5-(2-Chloro-4,5-difluoro-benzoylamino)-1H-pyrazole-3-carboxylic acid butyl-(1-ethoxalyl-piperidin-4-yl)-amide,
   5-(2-Chloro-4,5-difluoro-benzoylamino)-1H-pyrazole-3-carboxylic acid butyl-(1-oxalo-piperidin-4-yl)-amide,
   5-(2-Chloro-4,5-difluoro-benzoylamino)-1H-pyrazole-3-carboxylic acid butyl-(1,1-dioxo-hexahydro-1λ⁶ -thiopyran-4-yl)-amide,
   5- (2-Chloro-4,5-difluoro-benzoylamino) -1H-pyrazole-3-carboxylic acid butyl-(1-oxo-hexahydro-1λ⁴-thiopyran-4-yl)-amide,
   5-(2-Chloro-4,5-difluoro-benzoylamino)-1H-pyrazole-3-carboxylic acid (6-methoxy-pyridin-3-ylmethyl)-propyl-amide,
   5-(2-Chloro-4,5-difluoro-benzoylamino)-1H-pyrazole-3-carboxylic acid propyl-(pyridin-3-ylmethyl)-amide,
   5-(2-Chloro-4,5-difluoro-benzoylamino)-1H-pyrazole-3-carboxylic acid 4-diethylcarbamoyl-benzylamide,
   5-(2-Chloro-4,5-difluoro-benzoylamino)-1H-pyrazole-3-carboxylic acid 3-diethylcarbamoyl-benzylamide,
   5- (2-Chloro-4,5-difluoro-benzoylamino) -1H-pyrazole-3-carboxylic acid 4-ethylcarbamoyl-benzylamide,
   5-(2-Chloro-4,5-difluoro-benzoylamino)-1H-pyrazole-3-carboxylic acid 3-ethylcarbamoyl-benzylamide,
   5-(2-Chloro-4,5-difluoro-benzoylamino)-1H-pyrazole-3-carboxylic acid 4-carbamoyl-benzylamide,
   5-(2-Chloro-4,5-difluoro-benzoylamino)-1H-pyrazole-3-carboxylic acid (1-oxalo-piperidin-4-yl)-propyl-amide,
   5-(2-Chloro-4,5-difluoro-benzoylamino)-1H-pyrazole-3-carboxylic acid (1-carboxyacetyl-piperidin-4-yl)-propyl-amide,
   5-(2-Chloro-4,5-difluoro-benzoylamino)-1H-pyrazole-3-carboxylic acid [1-(2-carboxypropionyl)-piperidin-4-yl]-propyl-amide,
   5-(2-Chloro-4,5-difluoro-benzoylamino)-1H-pyrazole-3-carboxylic acid cyclopropyl-(6-methoxy-pyridin-3-ylmethyl)-amide
   5-(2-Chloro-4,5-difluoro-benzoylamino)-1H-pyrazole-3-carboxylic acid cyclobutyl-(6-methoxy-pyridin-3-ylmethyl)-amide,
   5-(2-Chloro-4,5-difluoro-benzoylamino)-1H-pyrazole-3-carboxylic acid cyclopropylmethyl-(6-methoxy-pyridin-3-ylmethyl)-amide
   5-(2-Chloro-4,5-difluoro-benzoylamino)-1H-pyrazole-3-carboxylic acid (6-dimethylamino-pyridin-3-ylmethyl)-amide,
   5-(2-Chloro-4,5-difluoro-benzoylamino)-1H-pyrazole-3-carboxylic acid (6-dimethylamino-pyridin-3-ylmethyl)-propyl-amide,
   5-(2-Chloro-4,5-difluoro-benzoylamino)-1H-pyrazole-3-carboxylic acid benzyl-(2-carboxy-ethyl)-amide,
   5-(2-Chloro-4,5-difluoro-benzoylamino)-1H-pyrazole-3-carboxylic acid benzyl-(2-ethoxycarbonyl-ethyl)-amide,
   5-(2-Chloro-4,5-difluoro-benzoylamino)-1H-pyrazole-3-carboxylic acid (6-chloro-pyridin-3-ylmethyl)-propyl-amide,
   5-(2-Chloro-4,5-difluoro-benzoylamino)-1H-pyrazole-3-carboxylic acid 2,6-dichloro-benzylamide,
   5-(2-Chloro-4,5-difluoro-benzoylamino)-1H-pyrazole-3-carboxylic acid (3,5-dichloro-pyridin-4-ylmethyl)-amide,
   5-(2-Chloro-4,5-difluoro-benzoylamino)-1H-pyrazole-3-carboxylic acid (3,5-dichloro-pyridin-4-ylmethyl)-propyl-amide,
   5-(2-Chloro-4,5-difluoro-benzoylamino)-1H-pyrazole-3-carboxylic acid [6-(1H-pyrazol-1-yl)-pyridin-3-ylmethyl]-amide dihydrochloride,
   5-(2-Chloro-4,5-difluoro-benzoylamino)-1H-pyrazole-3-carboxylic acid [6-(4-hydroxy-piperidin-1-yl)-pyridin-3-ylmethyl]-amid e dihydrochloride,
   5- (2-Chloro-4,5-difluoro-benzoylamino) -1H-pyrazole-3-carboxylic acid (2-pyridin-2-yl-ethyl)-amide,
   5-(2-Chloro-4,5-difluoro-benzoylamino)-1H-pyrazole-3-carboxylic acid (2-pyridin-3-yl-ethyl)-amide,
   5-(2-Chloro-4,5-difluoro-benzoylamino)-1H-pyrazole-3-carboxylic acid (2-pyridin-4-yl-ethyl)-amide,
   5-(2-Chloro-4,5-difluoro-benzoylamino)-1H-pyrazole-3-carboxylic acid (4-[1,2,3]thiadiazol-4-yl-benzyl)-amide,
   5-(2-Chloro-4,5-difluoro-benzoylamino)-1H-pyrazole-3-carboxylic acid (5-methyl-pyrazin-2-ylmethyl)-amide,
   5-(2-Chloro-4,5-difluoro-benzoylamino)-1H-pyrazole-3-carboxylic acid pyrazin-2-ylamide,
   5-(2-Chloro-4,5-difluoro-benzoylamino)-1H-pyrazole-3-carboxylic acid quinolin-5-ylamide,
   5-(2-Chloro-4,5-difluoro-benzoylamino)-1H-pyrazole-3-carboxylic acid quinolin-8-ylamide,
   5-(2-Chloro-4,5-difluoro-benzoylamino)-1H-pyrazole-3-carboxylic acid isoquinolin-5-ylamide,
   5- (2-Chloro-4,5-difluoro-benzoylamino) -1H-pyrazole-3-carboxylic acid [3-ethoxy-5-(1-ethoxycarbonyl-1-methyl-ethyl)-pyridin-2-yl]-amide,
   5-(2-Chloro-4,5-difluoro-benzoylamino)-1H-pyrazole-3-carboxylic acid [3-ethoxy-5-(1-ethoxycarbonyl-1-hydroxy-ethyl)-pyridin -2-yl]-amide,
   5-(2-Chloro-4,5-difluoro-benzoylamino)-1H-pyrazole-3-carboxylic acid
   [5-(1-carboxy-1-methyl-ethyl)-3-ethoxy-pyridin-2-yl]-amide,
   5-(2-Chloro-4,5-difluoro-benzoylamino)-1H-pyrazole-3-carboxylic acid
   [5-(1-carboxy-1-hydroxy-ethyl)-3-ethoxy-pyridin-2-yl]-amide,
   5-(2-Chloro-4,5-difluoro-benzoylamino)-1H-pyrazole-3-carboxylic acid (pyrazin-2-ylmethyl)-amide,
   5-(2-Chloro-4,5-difluoro-benzoylamino)-1H-pyrazole-3-carboxylic acid [6-(thiomorpholin-4-yl)-pyridin-3-ylmethyl]-amide,
   5-(2-Chloro-4,5-difluoro-benzoylamino)-1H-pyrazole-3-carboxylic acid [6-(1,1-dioxo-1λ⁶-thiomorpholin-4-yl)-pyridin-3-ylmethyl]-amide,
   5-(2-Chloro-4,5-difluoro-benzoylamino)-1H-pyrazole-3-carboxylic acid (4,5-dibromo-thiophen-2-ylmethyl)-amide,
   5-(2-Chloro-4,5-difluoro-benzoylamino)-1H-pyrazole-3-carboxylic acid (5-chloro-thiophen-2-ylmethyl)-amide,
   5-(2-Chloro-4,5-difluoro-benzoylamino)-1H-pyrazole-3-carboxylic acid (4-e thyl-2-methyl-oxazol-5-ylmethyl)-amide,
   5-(2-Chloro-4,5-difluoro-benzoylamino)-1H-pyrazole-3-carboxylic acid (2-e thyl-4-methyl-oxazol-5-ylmethyl)-amide dihydrochloride,
   5-(2-Chloro-4,5-difluoro-benzoylamino)-1H-pyrazole-3-carboxylic acid ([2,2']bithiophenyl-5-ylmethyl)-amide,
   5-(2-Chloro-4,5-difluoro-benzoylamino)-1H-pyrazole-3-carboxylic acid (3-methoxy-thiophen-2-ylmethyl)-amide,
   5-(2-Chloro-4,5-difluoro-benzoylamino)-1H-pyrazole-3-carboxylic acid (4,5-Dichloro-thiophen-2-ylmethyl)-amide,
   5-(2-Chloro-4,5-difluoro-benzoylamino)-1H-pyrazole-3-carboxylic acid (2,5-dimethyl-oxazol-4-ylmethyl)-amide,
   5-(2-Chloro-4,5-difluoro-benzoylamino)-1H-pyrazole-3-carboxylic acid (2-hexyl-4-methyl-oxazol-5-ylmethyl)-amide,
   5-(2-Chloro-4,5-difluoro-benzoylamino)-1H-pyrazole-3-carboxylic acid (4-methoxy-thiophen-3-ylmethyl)-amide,
   5-(2-Chloro-4,5-difluoro-benzoylamino)-1H-pyrazole-3-carboxylic acid (4-methyl-2-phenyl-oxazol-5-ylmethyl)-amide,
   5-(2-Chloro-4,5-difluoro-benzoylamino)-1H-pyrazole-3-carboxylic acid (2-methyl-4-phenyl-oxazol-5-ylmethyl)-amide,
   5-(2-Chloro-4,5-difluoro-benzoylamino)-1H-pyrazole-3-carboxylic acid (4-hexyl-2-methyl-oxazol-5-ylmethyl)-amide dihydrochloride,
   5-(2-Chloro-4,5-difluoro-benzoylamino)-1H-pyrazole-3-carboxylic acid (6-chloro-pyridin-3-ylmethyl)-amide,
   5-(2-Chloro-4,5-difluoro-benzoylamino)-1H-pyrazole-3-carboxylic acid (6-chloro-pyridin-3-ylmethyl)-amide dihydrochloride,
   5-(2-Chloro-4,5-difluoro-benzoylamino)-1H-pyrazole-3-carboxylic acid (4H-[1,2,4]triazol-3-ylmethyl)-amide,
   5-(2-Chloro-4,5-difluoro-benzoylamino)-1H-pyrazole-3-carboxylic acid (4H-[1,2,4]triazol-3-ylmethyl)-amide dihydrochloride,
   5-(2-Chloro-4,5-difluoro-benzoylamino)-1H-pyrazole-3-carboxylic acid(5-fluoro-4H-quinazoline-3-yl)-amide,
   5-(2-Chloro-4,5-difluoro-benzoylamino)-1H-pyrazole-3-carboxylic acid (5-fluoro-4H-quinazoline-3-yl)-amide dihydrochloride,
   5-(2-Chloro-4,5-difluoro-benzoylamino)-1H-pyrazole-3-carboxylic acid (4,6-dimethyl-pyridin-3-ylmethyl)-amide,
   5-(2-Chloro-4,5-difluoro-benzoylamino)-1H-pyrazole-3-carboxylic acid (4,6-dimethyl-pyridin-3-ylmethyl)-amide dihydrochloride,
   5-(2-Chloro-4,5-difluoro-benzoylamino)-1H-pyrazole-3-carboxylic acid (2-methoxy-6-methyl-pyridin-3-ylmethyl)-amide,
   5-(2-Chloro-4,5-difluoro-benzoylamino)-1H-pyrazole-3-carboxylic acid (4-methoxy-6-methyl-pyridin-3-ylmethyl)-amide,
   5-(2-Chloro-4,5-difluoro-benzoylamino)-1H-pyrazole-3-carboxylic acid (4-methoxy-6-methyl-pyridin-3-ylmethyl)-amide dihydrochloride,
   5-(2-Chloro-4,5-difluoro-benzoylamino)-1H-pyrazole-3-carboxylic acid (2-methoxy-4,6-dimethyl-pyridin-3-ylmethyl)-amide,
   5-(2-Chloro-4,5-difluoro-benzoylamino)-1H-pyrazole-3-carboxylic acid (2-methoxy-4,6-dimethyl-pyridin-3-ylmethyl)-amide dihydrochloride,
   5-(2-Chloro-4,5-difluoro-benzoylamino)-1H-pyrazole-3-carboxylic acid (3-methyl-pyridin-2-ylmethyl)-amide,
   5-(2-Chloro-4,5-difluoro-benzoylamino)-1H-pyrazole-3-carboxylic acid (5-methyl-pyridin-3-ylmethyl)-amide,
   5-(2-Chloro-4,5-difluoro-benzoylamino)-1H-pyrazole-3-carboxylic acid (6-methyl-pyridin-3-ylmethyl)-amide,
   5-(2-Chloro-4,5-difluoro-benzoylamino)-1H-pyrazole-3-carboxylic acid (6-methyl-pyridin-2-ylmethyl)-amide,
   5-(2,4-Dichloro-5-fluoro-benzoylamino)-1H-pyrazole-3-carboxylic acid (pyridin-3-ylmethyl)-amide dihydrochloride,
   5-(2-Chloro-4,5-difluoro-benzoylamino)-1H-pyrazole-3-carboxylic acid (6-fluoro-pyridin-3-ylmethyl)-amide dihydrochloride,
   5-(2-Chloro-4,5-difluoro-benzoylamino)-1H-pyrazole-3-carboxylic acid (3-carboxy-phenyl)-methyl-amide,
   5-(2-Chloro-4,5-difluoro-benzoylamino)-1H-pyrazole-3-carboxylic acid 3-methanesulfonylamino-benzylamide,
   5-(2-Chloro-4,5-difluoro-benzoylamino)-1H-pyrazole-3-carboxylic acid 4-methanesulfonylamino-benzylamide,
   5-(2-Chloro-4,5-difluoro-benzoylamino)-1H-pyrazole-3-carboxylic acid 3-acetylamino-benzylamide,
   5-(2-Chloro-4,5-difluoro-benzoylamino)-1H-pyrazole-3-carboxylic acid 4-acetylamino-benzylamide,
   5-(2-Chloro-4,5-difluoro-benzoylamino)-1H-pyrazole-3-carboxylic acid (2-phenyl-thiazol-4-ylmethyl)-amide,
   5-(2-Chloro-4,5-difluoro-benzoylamino)-1H-pyrazole-3-carboxylic acid ((R)-1-phenyl-ethyl)-amide,
   5-(2-Chloro-4,5-difluoro-benzoylamino)-1H-pyrazole-3-carboxylic acid ((S)-1-phenyl-ethyl)-amide,
   5-(2-Chloro-4,5-difluoro-benzoylamino)-1H-pyrazole-3-carboxylic acid (6-phenoxy-pyridin-3-ylmethyl)-amide dihydrochloride,
   5-(2-Methyl-4,5-difluoro-benzoylamino)-1H-pyrazole-3-carboxylic acid (6-chloro-pyridin-3-ylmethyl)-amide dihydrochloride,
   5-(2-Chloro-4,5-difluoro-benzoylamino)-1H-pyrazole-3-carboxylic acid (2-chloro-pyridin-3-ylmethyl)-amide dihydrochloride,
   5-(2-Methyl-4,5-difluoro-benzoylamino)-1H-pyrazole-3-carboxylic acid(6-chloro-pyridin-3-ylmethyl)-amide sodium salt,
   5-(2-Chloro-4,5-difluoro-benzoylamino)-1H-pyrazole-3-carboxylic acid [1-(pyridin-3-yl)-ethyl]-amide dihydrochloride ,
   5-(2-Chloro-4,5-difluoro-benzoylamino)-1H-pyrazole-3-carboxylic acid (2-fluoro-pyridin-3-ylmethyl)-amide dihydrochloride,
   5-(2-Chloro-4,5-difluoro-benzoylamino)-1H-pyrazole-3-carboxylic acid (2-methyl-pyridin-3-ylmethyl)-amide dihydrochloride,
   5-(2-Chloro-4,5-difluoro-benzoylamino)-1H-pyrazole-3-carboxylic acid (biphenyl-3-ylmethyl)-amide,
   5-(2-Chloro-4,5-difluoro-benzoylamino)-1H-pyrazole-3-carboxylic acid ((1R,2S)-2-hydroxy-indan-1-yl)-amide,
   5-(2-Chloro-4,5-difluoro-benzoylamino)-1H-pyrazole-3-carboxylic acid ((1S,2R)-2-hydroxy-indan-1-yl)-amide,
   5-(2-Chloro-4,5-difluoro-benzoylamino)-1H-pyrazole-3-carboxylic acid (6-phenyl-pyridin-3-ylmethyl)-amide dihydrochloride,
   5-(2-Chloro-4,5-difluoro-benzoylamino)-1H-pyrazole-3-carboxylic acid (6-methyl-2-pyridon-3-ylmethyl)-amide hydrochloride,
   5-(2-Chloro-4,5-difluoro-benzoylamino)-1H-pyrazole-3-carboxylic acid benzyl-methyl-amide,
   5-(2-Chloro-4,5-difluoro-benzoylamino)-1H-pyrazole-3-carboxylic acid benzylamide ,
   5-(2-Chloro-4,5-difluoro-benzoylamino)-1H-pyrazole-3-carboxylic acid phenylamide,
   5- (2-Chloro-4,5-difluoro-benzoylamino) -1H-pyrazole-3-carboxylic acid methyl-phenyl-amide,
   5-(2-Chloro-4,5-difluoro-benzoylamino)-1H-pyrazole-3-carboxylic acid (piperidin-1-yl)-amide,
   5-(2-Chloro-4,5-difluoro-benzoylamino)-1H-pyrazole-3-carboxylic acid (4-ethoxycarbonyl-piperidin-1-yl)-amide,
   5-(2-Chloro-4,5-difluoro-benzoylamino)-1H-pyrazole-3-carboxylic acid (4-methyl-piperazin-1-yl)-amide,
   5-(2-Chloro-4,5-difluoro-benzoylamino)-1H-pyrazole-3-carboxylic acid (4-carboxy-piperidin-1-yl)-amide,
   5-(2-Chloro-4,5-difluoro-benzoylamino)-1H-pyrazole-3-carboxylic acid phenethylamide,
   5-(2-Chloro-4,5-difluoro-benzoylamino)-1H-pyrazole-3-carboxylic acid methyl-phenethyl-amide,
   5-(2-Chloro-4,5-difluoro-benzoylamino)-1H-pyrazole-3-carboxylic acid ethyl-phenyl-amide,
   5-(2-Chloro-4,5-difluoro-benzoylamino)-1H-pyrazole-3-carboxylic acid (1,2,3,4-tetrahydroisoquinolin-2-yl)-amide,
   5-(2-Chloro-4,5-difluoro-benzoylamino)-1H-pyrazole-3-carboxylic acid ((S)-a-methoxycarbonyl-benzyl)-amide,
   5-(2-Chloro-4,5-difluoro-benzoylamino)-1H-pyrazole-3-carboxylic acid ((R)-α-methoxycarbonyl-benzyl)-amide,
   5-(2-Chloro-4,5-difluoro-benzoylamino)-1H-pyrazole-3-carboxylic acid (1,2,3,4-tetrahydroquinoline-1-yl)-amide,
   5-(2-Chloro-4,5-difluoro-benzoylamino)-1H-pyrazole-3-carboxylic acid phenyl-propyl-amide,
   5-(2-Chloro-4,5-difluoro-benzoylamino)-1H-pyrazole-3-carboxylic acid butyl-phenyl-amide,
   5-(2-Chloro-4,5-difluoro-benzoylamino)-1H-pyrazole-3-carboxylic acid pentyl-phenyl-amide,
   5-(2-Chloro-4,5-difluoro-benzoylamino)-1H-pyrazole-3-carboxylic acid benzhydryl-amide,
   5-(2-Chloro-4,5-difluoro-benzoylamino)-1H-pyrazole-3-carboxylic acid (pyridin-2-ylmethyl)-amide hydrochloride,
   5-(2-Chloro-4,5-difluoro-benzoylamino)-1H-pyrazole-3-carboxylic acid (pyridin-4-ylmethyl)-amide hydrochloride,
   5-(2-Chloro-4,5-difluoro-benzoylamino)-1H-pyrazole-3-carboxylic acid ((R)-2-hydroxy-1-phenyl-ethyl)-amide,
   5-(2-Chloro-4,5-difluoro-benzoylamino)-1H-pyrazole-3-carboxylic acid ((S)-2-hydroxy-1-phenyl-ethyl)-amide,
   5-(2-Chloro-4,5-difluoro-benzoylamino)-1H-pyrazole-3-carboxylic acid 4-dimethylamino-benzylamide,
   5-(2-Chloro-4,5-difluoro-benzoylamino)-1H-pyrazole-3-carboxylic acid (furan-2-ylmethyl)-amide,
   5-(2-Chloro-4,5-difluoro-benzoylamino)-1H-pyrazole-3-carboxylic acid (thiophen-2-ylmethyl)-amide,
   5-(2-Chloro-4,5-difluoro-benzoylamino)-1H-pyrazole-3-carboxylic acid (2-methoxy-phenyl)-amide,
   5- (2-Chloro-4,5-difluoro-benzoylamino) -1H-pyrazole-3-carboxylic acid (2-methoxy-phenyl)-methyl-amide,
   5-(2-Chloro-4,5-difluoro-benzoylamino)-1H-pyrazole-3-carboxylic acid4-dimethylamino-benzylamide hydrochloride,
   5-(2-Chloro-4,5-difluoro-benzoylamino)-1H-pyrazole-3-carboxylic acid 3-amino-benzylamide,
   5-(2-Chloro-4,5-difluoro-benzoylamino)-1H-pyrazole-3-carboxylic acid 4-amino-benzylamide,
   5-(2-Chloro-4,5-difluoro-benzoylamino)-1H-pyrazole-3-carboxylic acid 2-methyl-benzylamide,
   5-(2-Chloro-4,5-difluoro-benzoylamino)-1H-pyrazole-3-carboxylic acid 3-methyl-benzylamide,
   5-(2-Chloro-4,5-difluoro-benzoylamino)-1H-pyrazole-3-carboxylic acid 3-dimethylamino-benzylamide,
   5-(2-Chloro-4,5-difluoro-benzoylamino)-1H-pyrazole-3-carboxylic acid (2-methoxy-pyridin-3-ylmethyl)-amide,
   5-(2-Chloro-4,5-difluoro-benzoylamino)-1H-pyrazole-3-carboxylic acid 2-chloro-benzylamide,
   5-(2-Chloro-4,5-difluoro-benzoylamino)-1H-pyrazole-3-carboxylic acid 3-chloro-benzylamide,
   5-(2-Chloro-4,5-difluoro-benzoylamino)-1H-pyrazole-3-carboxylic acid 3-methoxycarbonyl-benzylamide,
   5-(2-Chloro-4,5-difluoro-benzoylamino)-1H-pyrazole-3-carboxylic acid 3-carboxy-benzylamide,
   5-(2-Chloro-4,5-difluoro-benzoylamino)-1H-pyrazole-3-carboxylic acid (6-trifluoromethyl-pyridin-3-ylmethyl)-amide,
   5-(2-Chloro-4,5-difluoro-benzoylamino)-1H-pyrazole-3-carboxylic acid (2-ethoxy-pyridin-3-ylmethyl)-amide,
   5-(2-Chloro-4,5-difluoro-benzoylamino)-1H-pyrazole-3-carboxylic acid (2-dimethylamino-ethyl)-amide,
   5-(2-Chloro-4,5-difluoro-benzoylamino)-1H-pyrazole-3-carboxylic acid ethyl-(2-dimethylamino-ethyl)-amide dihydrochloride,
   5-(2-Chloro-4,5-difluoro-benzoylamino)-1H-pyrazole-3-carboxylic acid cyclohexyl-(2-dimethylamino-ethyl)-amide,
   5-(2-Chloro-4,5-difluoro-benzoylamino)-1H-pyrazole-3-carboxylic acid (4-methoxy-pyridin-2-ylmethyl)-amide,
   5-(2-Chloro-4,5-difluoro-benzoylamino)-1H-pyrazole-3-carboxylic acid (2,6-dimethoxy-pyridin-3-ylmethyl)-amide,
   5-(2-Chloro-4,5-difluoro-benzoylamino)-1H-pyrazole-3-carboxylic acid 2-methoxycarbonylmethoxy-benzylamide,
   5-(2-Chloro-4,5-difluoro-benzoylamino)-1H-pyrazole-3-carboxylic acid 2-carboxymethoy-benzylamide,
   5-(4,5-Difluoro-2-methyl-benzoylamino)-1H-pyrazole-3-carboxylic acid (6-methoxy-pyridin-3-ylmethyl)-amide,
   5-(2,4-Dichloro-benzoylamino)-1H-pyrazole-3-carboxylicacid (6-methoxy-pyridin-3-ylmethyl)-amide,
   5-(2-Chloro-4,5-difluoro-benzoylamino)-1H-pyrazole-3-carboxylic acid (3,5-dimethoxy-pyridin-4-ylmethyl)-amide,
   5-(4,5-Difluoro-2-methyl-benzoylamino)-1H-pyrazole-3-carboxylic acid (2,4-dimethyl-oxazol-5-ylmethyl)-amide,
   5-(2,4-Dichloro-5-fluoro-benzoylamino)-1H-pyrazole-3-carboxylic acid (2,4-dimethyl-oxazol-5-ylmethyl)-amide,
   5-(2-Chloro-4,5-difluorobenzoylamino)-1H-pyrazole-3-carbocylic acid (4-methoxycarbonyl-phenyl)-methyl-amide,
   5-(2-Chloro-4,5-difluoro-benzoylamino)-1H-pyrazole-3-carboxylic acid (4-carboxyphenyl)-methyl-amide, 4-{[5-(2-Chloro-4,5-difluoro-benzoylamino)-1H-pyrazole -3-carbonyl]-methyl-amino}-benzoic acid sodium salt,
   5-(2-Chloro-4,5-difluoro-benzoylamino)-1H-pyrazole-3-carboxylic acid isopropyl-phenyl-amide,
   5-(2-Chloro-4,5-difluoro-benzoylamino)-1H-pyrazole-3-carboxylic acid (ethoxycarbonyl-methyl)-phenyl-amide,
   5- (2-Chloro-4,5-difluoro-benzoylamino) -1H-pyrazole-3-carboxylic acid (carboxy-methyl)-phenyl-amide,
   5-(2-Chloro-4-fluoro-benzoylamino)-1H-pyrazole-3-carboxylic acid benzylamide,
   5-(2,4-Dichloro-benzoylamino)-1H-pyrazole-3-carboxylic acid benzylamide,
   5-(2-Chloro-5-fluoro-benzoylamino)-1H-pyrazole-3-carboxylic acid benzylamide,
   5-(2-Chloro-4,5-difluoro-benzoylamino)-1H-pyrazole-3-carboxylic acid benzyl-phenyl-amide,
   5-(2-Chloro-4,5-difluoro-benzoylamino)-1H-pyrazole-3-carboxylic acid (1-methyl-1-phenyl-ethyl)-amide,
   5-(2-Chloro-4,5-difluoro-benzoylamino)-1H-pyrazole-3-carboxylic acid [2-oxo-2-(piperidin-1-yl)-ethyl]-phenyl-amide,
   5-(5-Fluoro-2-methyl-benzoylamino)-1H-pyrazole-3-carboxylic acid benzylamide,
   5-(5-Chloro-2-methyl-benzoylamino)-1H-pyrazole-3-carboxylic acid benzylamide,
   5-(4-Methoxy-2-methyl-benzoylamino)-1H-pyrazole-3-carboxylic acid benzylamide,
   5-(4-Chloro-2-methyl-benzoylamino)-1H-pyrazole-3-carboxylic acid benzylamide,
   5-(5-Fluoro-2-methoxy-benzoylamino)-1H-pyrazole-3-carboxylic acid benzylamide,
   5-(5-Chloro-2-methoxy-benzoylamino)-1H-pyrazole-3-carboxylic acid benzylamide,
   5-(2-Chloro-4,5-difluoro-benzoylamino)-1H-pyrazole-3-carboxylic acid (2-ethoxy-ethyl)-phenyl-amide,
   5-(2-Fluoro-5-methyl-benzoylamino)-1H-pyrazole-3-carboxylic acid benzylamide,
   5-(5-Chloro-2-fluoro-benzoylamino)-1H-pyrazole-3-carboxylic acid benzylamide,
   5-(3-Chloro-2,6-difluoro-benzoylamino)-1H-pyrazole-3-carboxylic acid benzylamide,
   5-(2-Chloro-3-fluoro-benzoylamino)-1H-pyrazole-3-carboxylic acid benzylamide,
   5-(2-Chloro-4,5-difluoro-benzoylamino)-1H-pyrazole-3-carboxylic acid ethylcarbamoylmethyl-phenyl-amide,
   5-(2-Chloro-4,5-difluoro-benzoylamino)-1H-pyrazole-3-carboxylic acid isobutyl-phenyl-amide,
   5-(2-Chloro-4,5-difluoro-benzoylamino)-1H-pyrazole-3-carboxylic acid (2-methoxycarbonyl-ethyl)-phenyl-amide,
   5-(2-Chloro-4,5-difluoro-benzoylamino)-1H-pyrazole-3-carboxylic acid (3-ethoxycarbonyl-propyl)-phenyl-amide,
   5-(2-Chloro-4,5-difluoro-benzoylamino)-1H-pyrazole-3-carboxylic acid (2-carboxy-ethyl)-phenyl-amide,
   5-(2-Chloro-4,5-difluoro-benzoylamino)-1H-pyrazole-3-carboxylic acid (3-carboxy-propyl)-phenyl-amide,
   5-(2-Hydroxy-4-methoxy-benzoylamino)-1H-pyrazole-3-carboxylic acid benzylamide,
   5-(2-Hydroxy-5-methoxy-benzoylamino)-1H-pyrazole-3-carboxylic acid benzylamide,
   5-(5-Fluoro-2-hydroxy-benzoylamino)-1H-pyrazole-3-carboxylic acid benzylamide,
   5-(4-Fluoro-2-trifluoromethyl-benzoylamino)-1H-pyrazole-3-carboxylic acid benzylamide,
   5-(5-Fluoro-2-trifluoromethyl-benzoylamino)-1H-pyrazole-3-carboxylic acid benzylamide,
   5-(2-Chloro-4,5-difluoro-benzoylamino)-1H-pyrazole-3-carboxylic acid (2-methoxy-ethyl)-phenyl-amide,
   5-(2,5-Dimethyl-benzoylamino)-1H-pyrazole-3-carboxylicacid benzylamide,
   5-(2-Chloro-5-methyl-benzoylamino)-1H-pyrazole-3-carboxylic acid benzylamide,
   5-(2-Chloro-pyridine-3-carbonylamino)-1H-pyrazole-3-carboxylic acid benzylamide,
   5-(4-Chloro-pyridine-2-carbonylamino)-1H-pyrazole-3-carboxylic acid benzylamide,
   5-(2,6-Dichloro-pyridine-3-carbonylamino)-1H-pyrazole-3-carboxylic acid benzylamide,
   5- (2-Chloro-4,5-difluoro-benzoylamino) -1H-pyrazole-3-carboxylic acid butyl-(4-ethoxycarbonyl-phenyl)-amide,
   5-(2-Chloro-4,5-difluoro-benzoylamino)-1H-pyrazole-3-carboxylic acid butyl-(4-carboxy-phenyl)-amide,
   5-(2-Methyl-pyridine-3-carbonylamino)-1H-pyrazole-3-carboxylic acid benzylamide,
   5-(2-Chloro-6-methyl-pyridine-3-carbonylamino)-1H-pyrazole-3-carboxylic acid benzylamide,
   5-(3-Chloro-pyridine-4-carbonylamino)-1H-pyrazole-3-carboxylic acid benzylamide,
   5-(2-Chloro-4,5-difluoro-benzoylamino)-1H-pyrazole-3-carboxylic acid butyl-(4-ethoxycarbonylmethyl-phenyl)-amide,
   5-(2-Chloro-4,5-difluoro-benzoylamino)-1H-pyrazole-3-carboxylic acid butyl-(4-carboxymethyl-phenyl)-amide,
   5-(2-Chloro-4,5-difluoro-benzoylamino)-1H-pyrazole-3-carboxylic acid (4-methoxycarbonyl-phenyl)-(2-methoxy-ethyl)-amide,
   5-(2-Chloro-4,5-difluoro-benzoylamino)-1H-pyrazole-3-carboxylic acid (4-carboxy-phenyl)-(2-methoxy-ethyl)-amide,
   5-(2-Chloro-4,5-difluoro-benzoylamino)-1H-pyrazole-3-carboxylic acid (6-methoxy-pyridin-3-ylmethyl)-amide dihydrochloride,
   5-(2-Methyl-4,5-difluoro-benzoylamino)-1H-pyrazole-3-carboxylic acid (6-methoxy-pyridin-3-ylmethyl)-amide dihydrochloride,
   5-(2-Chloro-4,5-difluoro-benzoylamino)-1H-pyrazole-3-carboxylic acid (thiazol-4-ylmethyl)-amide dihydrochloride,
   5-(2-Chloro-4,5-difluoro-benzoylamino)-1H-pyrazole-3-carboxylic acid 4-[2-(morpholin-4-yl)-ethylcarbamoyl]-benzylamide dihydrochloride,
   5-(2-Chloro-4,5-difluoro-benzoylamino)-1H-pyrazole-3-carboxylic acid 4-(morpholin-4-ylcarbamoyl)-benzylamide dihydrochloride, [4-({[5-(2-Chloro-4,5-difluoro-benzoylamino)-1H-pyrazole-3-carbonyl]-amino}-methyl)-benzoylamino]-acetic acid sodium salt, 3-[4-({[5-(2-Chloro-4,5-difluoro-benzoylamino)-1H-pyrazole-3-carbonyl]-amino}-methyl)-benzoylamino]-propioni c acid sodium salt,
   5-(2-Chloro-4,5-difluoro-benzoylamino)-1H-pyrazole-3-carboxylic acid (3,5-dimethyl-isoxazol-4-ylmethyl)-amide hydrochloride,
   5-(2-Chloro-4,5-difluoro-benzoylamino)-1H-pyrazole-3-carboxylic acid (1,3,5-trimethyl-1H-pyrazol-4-ylmethyl)-amide dihydrochloride,
   5-(2-Chloro-4,5-difluoro-benzoylamino)-1H-pyrazole-3-carboxylic acid (3,5-dimethyl-isoxazol-4-ylmethyl)-amide methanesulfonate,
   5-(2-Chloro-4,5-difluoro-benzoylamino)-1H-pyrazole-3-carboxylic acid 3-[2-(morpholin-4-yl)-ethylcarbamoyl]-benzylamide dihydrochloride,
   5-(2-Chloro-4,5-difluoro-benzoylamino)-1H-pyrazole-3-carboxylic acid 3-(morpholin-4-ylcarbamoyl)-benzylamide dihydrochloride,
   5-(2-Chloro-4,5-difluoro-benzoylamino)-1H-pyrazole-3-carboxylic acid {2-[2-(morpholin-4-yl)-ethylcarbamoyl]-pyridin-4-ylmet hyl}-amide trihydrochloride,
   5-(2-Chloro-4,5-difluoro-benzoylamino)-1H-pyrazole-3-carboxylic acid [2-(morpholin-4-ylcarbamoyl)-pyridin-4-ylmethyl]-amide trihydrochloride,
   5-(2-Chloro-4,5-difluoro-benzoylamino)-1H-pyrazole-3-carboxylic acid (6-methoxy-pyridin-3-ylmethyl)-amide methanesulfonate,
   5-(2-Chloro-4,5-difluoro-benzoylamino)-1H-pyrazole-3-carboxylic acid (6-methoxy-pyridin-3-ylmethyl)-amide hemisulfate
   5-(2-Chloro-4,5-difluoro-benzoylamino)-1H-pyrazole-3-carboxylic acid (3,5-dimethoxy-pyridin-4-ylmethyl)-amide dihydrochloride,
   5-(2-Chloro-4,5-difluoro-benzoylamino)-1H-pyrazole-3-carboxylic acid (4-aminomethyl-pyridin-2-ylmethyl)-amide trihydrochloride,
   5-(2-Chloro-4,5-difluoro-benzoylamino)-1H-pyrazole-3-carboxylic acid (6-chloro-pyridin-3-ylmethyl)-amide methanesulfonate,
   5-(2-Chloro-4,5-difluoro-benzoylamino)-1H-pyrazole-3-carboxylic acid (6-chloro-pyridin-3-ylmethyl)-amide dimethanesulfonate,
   5-(2-Chloro-4,5-difluoro-benzoylamino)-1H-pyrazole-3-carboxylic acid (2,4-dimethyl-pyridin-3-ylmethyl)-amide hydrochloride,
   5-(2-Chloro-4,5-difluoro-benzoylamino)-1H-pyrazole-3-carboxylic acid (pyridin-3-ylmethyl)-amide hydrochloride,
   5-(2-Chloro-4,5-difluoro-benzoylamino)-1H-pyrazole-3-carboxylic acid (6-methoxy-pyridin-3-ylmethyl)-propyl-amide dihydrochloride,
   5-(2-Chloro-4,5-difluoro-benzoylamino)-1H-pyrazole-3-carboxylic acid [6-(piperidin-1-yl)-pyridin-3-ylmethyl]-amide dihydrochloride,
   5-(2-Chloro-4,5-difluoro-benzoylamino)-1H-pyrazole-3-carboxylic acid [6-(morpholin-4-yl)-pyridin-3-ylmethyl]-amide dihydrochloride,
   5- (2-Chloro-4,5-difluoro-benzoylamino) -1H-pyrazole-3-carboxylic acid (3,5-dichloro-pyridin-4-ylmethyl)-amide hydrochloride,
   5-(2-Chloro-4,5-difluoro-benzoylamino)-1H-pyrazole-3-carboxylic acid (6-dimethylamino-pyridin-3-ylmethyl)-amide dihydrochloride,
   5-(2-Chloro-4,5-difluoro-benzoylamino)-1H-pyrazole-3-carboxylic acid (6-dimethylamino-pyridin-3-ylmethyl)-propyl-amide dihydrochloride,
   5-(2-Chloro-4,5-difluoro-benzoylamino)-1H-pyrazole-3-carboxylic acid (6-diethylamino-pyridin-3-ylmethyl)-amide dihydrochloride,
   5-(2-Chloro-4,5-difluoro-benzoylamino)-1H-pyrazole-3-carboxylic acid (6-chloro-2-dimethylamino-pyridin-3-ylmethyl)-amide dihydrochloride,
   5-(2-Chloro-4,5-difluoro-benzoylamino)-1H-pyrazole-3-carboxylic acid (4-[1,2,3]thiadiazol-4-yl-benzyl)-amide hydrochloride,
   5-(2-Chloro-4,5-difluoro-benzoylamino)-1H-pyrazole-3-carboxylic acid (4-methyl-thiazol-5-ylmethyl)-amide hydrochloride,
   5-(2-Chloro-4,5-difluoro-benzoylamino)-1H-pyrazole-3-carboxylic acid (2,4-dimethyl-thiazol-5-ylmethyl)-amide hydrochloride,
   5-(2-Chloro-4,5-difluoro-benzoylamino)-1H-pyrazole-3-carboxylic acid [6-(4-hydroxy-piperidin-1-yl)-pyridin-3-ylmethyl]-amid e dihydrochloride,
   5-(2-Chloro-4,5-difluoro-benzoylamino)-1H-pyrazole-3-carboxylic acid 5-methyl-pyrazin-2-ylamide dihydrochloride,
   5-(2-Chloro-4,5-difluoro-benzoylamino)-1H-pyrazole-3-carboxylic acid quinolin-5-ylamide dihydrochloride,
   5-(2-Chloro-4,5-difluoro-benzoylamino)-1H-pyrazole-3-carboxylic acid quinolin-8-ylamide dihydrochloride,
   5-(2-Chloro-4,5-difluoro-benzoylamino)-1H-pyrazole-3-carboxylic acid isoquinolin-5-ylamide dihydrochloride,
   5-(2-Chloro-4,5-difluoro-benzoylamino)-1H-pyrazole-3-carboxylic acid [6-(4-methylpiperazin-1-yl)-pyridin-3-ylmethyl]-amide dihydrochloride,
   5-(2-Chloro-4,5-difluoro-benzoylamino)-1H-pyrazole-3-carboxylic acid [6-(4-dimethylamino-piperidin-1-yl)-pyridin-3-ylmethyl ]-amide dihydrochloride,
   5-(2-Chloro-4,5-difluoro-benzoylamino)-1H-pyrazole-3-carboxylic acid [6-(pyrrolidin-1-yl)-pyridin-3-ylmethyl] -amide dihydrochloride,
   5-(2-Chloro-4,5-difluoro-benzoylamino)-1H-pyrazole-3-carboxylic acid (pyrazin-2-ylmethyl)-amide dihydrochloride,
   5-(2-Chloro-4,5-difluoro-benzoylamino)-1H-pyrazole-3-carboxylic acid [6-(thiomorpholin-4-yl)-pyridin-3-ylmethyl]-amide dihydrochloride,
   5-(2-Chloro-4,5-difluoro-benzoylamino)-1H-pyrazole-3-carboxylic acid [6-(1,1-dioxo-116-thiomorpholin-4-yl)-pyridin-3-ylmeth yl]-amide dihydrochloride,
   5-(2-Chloro-4,5-difluoro-benzoylamino)-1H-pyrazole-3-carboxylic acid carbamoylmethyl-amide,
   5-(2,4-Dichloro-5-fluoro-benzoylamino)-1H-pyrazole-3-carboxylic acid (4-carbamoyl-phenyl)-methyl-amide,
   5-(2,4-Dichloro-5-fluoro-benzoylamino)-1H-pyrazole-3-carboxylic acid methyl-(4-methylcarbamoyl-phenyl)-amide,
   5-(2,4-Dichloro-5-fluoro-benzoylamino)-1H-pyrazole-3-carboxylic acid (4-dimethylcarbamoyl-phenyl)-methyl-amide,
   5-(2-Chloro-4,5-difluoro-benzoylamino)-1H-pyrazole-3-carboxylic acid (4-methanesulfonylaminocarbonyl-phenyl)-methyl-amide,
   5-(2-Chloro-4,5-difluoro-benzoylamino)-1H-pyrazole-3-carboxylic acid (4-methanesulfonylaminocarbonyl-phenyl)-methyl-amide sodium salt,
   5-(2-Chloro-4,5-difluoro-benzoylamino)-1H-pyrazole-3-carboxylic acid (4-cyano-phenyl)-methyl-amide,
   5-(2-Chloro-4,5-difluoro-benzoylamino)-1H-pyrazole-3-carboxylic acid methyl-[4-(4H-[1,2,4]triazol-3-yl)-phenyl]-amide hydrochloride,
   5-(4-Azido-2-chloro-5-fluoro-benzoylamino)-1H-pyrazole -3-carboxylic acid (4-cyano-phenyl)-methyl-amide,
   5-(4-Azido-2-chloro-5-fluoro-benzoylamino)-1H-pyrazole -3-carboxylic acid methyl-[4-(1H-tetrazol-5-yl)-phenyl]-amide,
   5-(2-Chloro-4,5-difluoro-benzoylamino)-1H-pyrazole-3-carboxylic acid [4-(N-hydroxycarbamimidoyl)-phenyl]-methyl-amide,
   5-(2-Chloro-4,5-difluoro-benzoylamino)-1H-pyrazole-3-carboxylic acid methyl-[4-(5-oxo-2,5-dihydro-[1,2,4]oxadiazol-3-yl)-phenyl]-amide,
   5-(2-Chloro-4,5-difluoro-benzoylamino)-1H-pyrazole-3-carboxylic acid {4-[(2,2-dimethyl-propionyloxy)-methoxycarbonyl]-phenyl}-methyl-amide,
   5-(2-Chloro-4,5-difluoro-benzoylamino)-1H-pyrazole-3-carboxylic acid 4-[N-(methoxy-thiocarbonyloxy)-carbamimidoyl]-methyl-amide,
   5-(2-Chloro-4,5-difluoro-benzoylamino)-1H-pyrazole-3-carboxylic acid methyl-[4-(5-thioxo-4,5-dihydro-[1,2,4]oxadiazol-3-yl) -phenyl]-amide,
   5-(2-Chloro-4,5-difluoro-benzoylamino)-1H-pyrazole-3-carboxylic acid methyl-[4-(5-oxo-4,5-dihydro-[1,2,4]thiadiazol-3-yl)-phenyl]-amide,
   5- (2-Chloro-4,5-difluoro-benzoylamino) -1H-pyrazole-3-carboxylic acid {4-[1-(cyclohexyloxy-carbonyloxy)-ethoxycarbonyl]-phenyl}-methyl-amide,
   5-(2-Chloro-4,5-difluoro-benzoylamino)-1H-pyrazole-3-carboxylic acid (4-ethoxycarbonyl-thiazol-2-yl)-ethyl-amide,
   5-(2-Chloro-4,5-difluoro-benzoylamino)-1H-pyrazole-3-carboxylic acid 2-methanesulfonylamino-benzylamide,
   5-(2-Chloro-4,5-difluoro-benzoylamino)-1H-pyrazole-3-carboxylic acid 2-acetylamino-benzylamide,
   5-(2-Chloro-4,5-difluoro-benzoylamino)-1H-pyrazole-3-carboxylic acid 2-(dimethylamino-methyleneamino)-benzylamide hydrochloride,
   5-(2-Chloro-4,5-difluoro-benzoylamino)-1H-pyrazole-3-carboxylic acid 3-(dimethylamino-methyleneamino)-benzylamide hydrochloride,
   5-(2-Chloro-4,5-difluoro-benzoylamino)-1H-pyrazole-3-carboxylic acid 4-(dimethylamino-methyleneamino)-benzylamide hydrochloride,
   5-(2-Chloro-4,5-difluoro-benzoylamino)-1H-pyrazole-3-carboxylic acid 2-[1-(dimethylamino)ethylideneamino]-benzylamide dihydrochloride,
   5-(2-Chloro-4,5-difluoro-benzoylamino)-1H-pyrazole-3-carboxylic acid 3-[1-(dimethylamino)ethylideneamino]-benzylamide dihydrochloride,
   5-(2-Chloro-4,5-difluoro-benzoylamino)-1H-pyrazole-3-carboxylic acid 4-[1-(dimethylamino)ethylideneamino]-benzylamide dihydrochloride,
   5-(2-Chloro-4,5-difluoro-benzoylamino)-1H-pyrazole-3-carboxylic acid 2-(diethylamino-methyleneamino)-benzylamide dihydrochloride,
   5-(2-Chloro-4,5-difluoro-benzoylamino)-1H-pyrazole-3-carboxylic acid 3-(diethylamino-methyleneamino)-benzylamide dihydrochloride,
   5-(2-Chloro-4,5-difluoro-benzoylamino)-1H-pyrazole-3-carboxylic acid 4-(diethylamino-methyleneamino)-benzylamide dihydrochloride,
   5-(2-Chloro-4,5-difluoro-benzoylamino)-1H-pyrazole-3-carboxylic acid 2-acetimidoylamino-benzylamide dihydrochloride,
   5-(2-Chloro-4,5-difluoro-benzoylamino)-1H-pyrazole-3-carboxylic acid 3-acetimidoylamino-benzylamide dihydrochloride,
   5-(2-Chloro-4,5-difluoro-benzoylamino)-1H-pyrazole-3-carboxylic acid 4-acetimidoylamino-benzylamide dihydrochloride,
   5-(2-Chloro-4,5-difluoro-benzoylamino)-1H-pyrazole-3-carboxylic acid 4-cyano-benzylamide,
   5-(2-Chloro-4,5-difluoro-benzoylamino)-1H-pyrazole-3-carboxylic acid 3-[(2-fluoro-benzimidoyl)-amino]-benzylamide hyrdoiodide,
   5-(2-Chloro-4,5-difluoro-benzoylamino)-1H-pyrazole-3-carboxylic acid 4-[(2-fluoro-benzimidoyl)-amino]-benzylamide hyrdoiodide,
   5-(2-Chloro-4,5-difluoro-benzoylamino)-1H-pyrazole-3-carboxylic acid 3-(3,3-dimethyl-ureido)-benzylamide,
   5-(2-Chloro-4,5-difluoro-benzoylamino)-1H-pyrazole-3-carboxylic acid 4-(3,3-dimethyl-ureido)-benzylamide,
   5-(2-Chloro-4,5-difluoro-benzoylamino)-1H-pyrazole-3-carboxylic acid 2-(toluene-4-sulfonylamino)-benzylamide,
   5-(2-Chloro-4,5-difluoro-benzoylamino)-1H-pyrazole-3-carboxylic acid 2-amino-6-fluoro-benzylamide hydrochloride,
   5-(2-Chloro-4,5-difluoro-benzoylamino)-1H-pyrazole-3-carboxylic acid 2-amino-4,5-difluoro-benzylamide hydrochloride,
   5-(2-Chloro-4,5-difluoro-benzoylamino)-1H-pyrazole-3-carboxylic acid (indan-1-yl)-amide,
   5-(2-Chloro-4,5-difluoro-benzoylamino)-1H-pyrazole-3-carboxylic acid (indan-2-yl)-amide,
   5-(2-Chloro-4,5-difluoro-benzoylamino)-1H-pyrazole-3-carboxylic (4H-quinazoline-3-yl)-amide,
   5-(2-Chloro-4,5-difluoro-benzoylamino)-1H-pyrazole-3-carboxylic (4H-quinazoline-3-yl)-amide dihydrochloride,
   5-(2-Chloro-4,5-difluoro-benzoylamino)-1H-pyrazole-3-carboxylic acid (7-fluoro-4H-quinazoline-3-yl)-amide dihydrochloride,
   5-(2-Chloro-4,5-difluoro-benzoylamino)-1H-pyrazole-3-carboxylic acid (6-fluoro-4H-quinazoline-3-yl)-amide hydrochloride,
   5-(2-Chloro-4,5-difluoro-benzoylamino)-1H-pyrazole-3-carboxylic acid (2-ethoxy-2-methyl-1,4-dihydro-2H-quinazoline-3-yl)-amide,
   5-(2-Chloro-4,5-difluoro-benzoylamino)-1H-pyrazole-3-carboxylic acid (2-cyano-pyridin-4-ylmethyl)-amide,
   5-(2-Chloro-4,5-difluoro-benzoylamino)-1H-pyrazole-3-carboxylic acid [2-(N-hydroxycarbamimidoyl)-pyridin-4-ylmethyl]-amide,
   5-(2-Chloro-4,5-difluoro-benzoylamino)-1H-pyrazole-3-carboxylic acid [2-(5-oxo-4,5-dihydro-[1,2,4]oxadiazol-3-yl)-pyridin-4 -ylmethyl]-amide, 4-{[5-(4,5-difluoro-2-methyl-benzoylamino)-1H-pyrazole -3-carbonyl]-methyl-amino}-benzoic acid sodium salt,
   5-(4,5-Difluoro-2-methyl-benzoylamino)-1H-pyrazole-3-carboxylic acid methyl-[4-(1H-tetrazol-5-yl)-phenyl]-amide,
   5-(2,4-Dichloro-benzoylamino)-1H-pyrazole-3-carboxylic acid (pyridin-3-ylmethyl)-amide,
   5-(2,4-Dichloro-5-fluoro-benzoylamino)-1H-pyrazole-3-carboxylic acid (pyridin-3-ylmethyl)-amide,
   5-(2,6-Dichloro-5-fluoro-pyridine-3-carbonylamino)-1H-pyrazole-3-carboxylic acid (pyridin-3-ylmethyl)-amide,
   5-[(3-Chloro-benzo[b]thiophene-2-carbonyl)-amino]-1H-pyrazole-3-carboxylic acid (pyridin-3-ylmethyl)-amide,
   5-[(3-Chloro-thiophene-2-carbonyl)-amino]-1H-pyrazole-3-carboxylic acid (pyridin-3-ylmethyl)-amide,
   5-Benzoylamino-1H-pyrazole-3-carboxylic acid (pyridin-3-ylmethyl)-amide,
   5-Phenylacetylamino-1H-pyrazole-3-carboxylic acid (pyridin-3-ylmethyl)-amide,
   5-(2-Methyl-4,5-difluoro-benzoylamino)-1H-pyrazole-3-carboxylic acid (pyridin-3-ylmethyl)-amide dihydrochloride,
   5-(2-Amino-4,5-difluoro-benzoylamino)-1H-pyrazole-3-carboxylic acid (pyridin-3-ylmethyl)-amide,
   5-(4,5-Difluoro-2-methanesulfonylamino-benzoylamino)-1H-pyrazole-3-carboxylic acid (pyridin-3-ylmethyl)-amide,
   5-(2-Acetylamino-4,5-difluoro-benzoylamino)-1H-pyrazole-3-carboxylic acid (pyridin-3-ylmethyl)-amide,
   5-[(3,4,5-Trichloro-thiophene-2-carbonyl)-amino]-1H-pyrazole-3-carboxylic acid (pyridin-3-ylmethyl)-amide,
   5-(4,5-Difluoro-2-methyl-benzoylamino)-1H-pyrazole-3-carboxylic acid (2-cyano-pyridin-4-ylmethyl)-amide,
   5- (4,5-Difluoro-2-methyl-benzoylamino) -1H-pyrazole-3-carboxylic acid [2-(1H-tetrazol-5-yl)-pyridin-4-ylmethyl]-amide, and 1H-Pyrazole-3,5-dicarboxylic acid 3-benzylamide 5-[(2,4-dichloro-phenyl)-amide].
21. A medicine comprising a combination of a pharmaceutical composition for treating or preventing diabetes according to any of the above-described 1 to 20 and another therapeutic or prophylactic agent for diabetes.
22. The medicine according to the above-described 21, wherein the other therapeutic or prophylactic agent for diabetes is an insulin preparation (injection), a low molecular insulin oral agent, a sulfonylurea receptor agonist (SU agent), a non-sulfonylureainsulinotropic agent,a potassium-dependent ATP (KATP) channel opening agent, an α glucosidase inhibitor, an α amylase inhibitor, an insulin sensitizer, a low molecular tGLP-1 receptor agonist, a tGLP-1 peptide analogue, a dipeptidyl peptidase IV (DPP-IV) inhibitor, a glucagon receptor antagonist, a glucocorticoid receptor antagonist, a biguanide, an SGLUT inhibitor, a fructose-1,6-bisphosphatase (FBPase) inhibitor, a glycogen synthase kinase 3 (GSK-3) inhibitor, a phosphoenolpyruvate carboxykinase (PEPCK) inhibitor, a protein tyrosine phosphatase 1B (PTPase1B) inhibitor, an SH2 domain containing inositol phosphatase (SHIP2) inhibitor, a glycogen phosphorylase (GP) inhibitor, a glucokinase activator, a GPR40 receptor agonist, apyruvatedehydrogenasekinase (PDHK) inhibitor, aglutamine: fructose-6-phosphate aminotransferase (GFAT) inhibitor, an antioxidant; a nitric monoxide scavenger, a carnitine palmitoyltransferase 1 (CPT-1) inhibitor, a growth hormone-release factor (GHRF), a triacylglycerol lipase (hormone-sensitive lipase) inhibitor, a PPAR γ receptor agonist, a PPAR γ receptor antagonist, a PPAR α/γ receptor agonist, an AMP activation protein kinase (AMPK) activator, an adiponectin receptor agonist or a β₃ adrenoceptor agonist .
23. The medicine according to the above-described 21 or 22, wherein the other therapeutic or prophylactic agent for diabetes is insulin, tolbutamide, glyclopyramide, acetohexamide, chlorpropamide, glybuzole, glibenclamide, gliclazide, glimepiride, mitiglinide, repaglinide, nateglinide, voglibose, acarbose, miglitol, rosiglitazone maleate,metformin hydrochloride,pioglitazone hydrochloride or buformin hydrochloride.
24. A medicine comprising a combination of a pharmaceutical composition for treating or preventing diabetes according to any of the above-described 1 to 20 and another therapeutic or prophylactic agent for diabetic complications.
25. The medicine according to the above-described 24, wherein the other therapeutic or prophylactic agent for diabetic complications is a protein kinase β (PKC β) inhibitor, an angiotensin II receptor antagonist, an aldose reductase inhibitor, an angiotensin converting enzyme (ACE) inhibitor, an advanced glycation end product (AGE) production inhibitor, a neuropathy therapeutic agent or a diabetic nephropathy therapeutic agent.
26. The medicine according to the above-described 24 or 25, wherein the other therapeutic or prophylactic agent for diabetic complications is epalrestat, mexiletine hydrochloride or imidapril hydrochloride.
27. A medicine comprising a combination of a pharmaceutical composition for treating or preventing diabetes according to any of the above-described 1 to 20 and another therapeutic or prophylactic agent for hyperlipidemia.
28. The medicine according to the above-described 27, wherein the other therapeutic or prophylactic agent for hyperlipidemia is a fibrate (PPARα receptor agonist), a PPARδ receptor agonist, a microsome triglyceride transfer protein (MTP) inhibitor, a cholesteryl ester transferprotein (CETP) inhibitor, a statin (HMG-CoA reductase inhibitor), an anion exchanger, probucol, anicotinicdrug, aplantsterol, an apolipoprotein-A1 (Apo-A1) inducer, a lipoprotein lipase (LPL) activator, an endodermis lipase inhibitor, ezetimibu, an IBAT inhibitor, a squalene synthesis enzyme inhibitor, an ACAT inhibitor, an LXR receptor agonist, an FXR receptor agonist, an FXR receptor antagonist or an adenosine A1 agonist.
29. The medicine according to the above-described 27 or 28, wherein the other therapeutic or prophylactic agent for hyperlipidemia is clofibrate, bezafibrate, fenofibrate, lovastatin, simvastatin, pravastatin, fluvastatin, atorvastatin, pitavastatin, rosuvastatin, cerivastatin, cholestyramine, colestyramine, tocopherol nicotinate, nicomol, niceritrol, soysterol or gamma orizanol.
30. A medicine comprising a combination of a pharmaceutical composition for treating or preventing diabetes according to any of the above-described 1 to 20 and another therapeutic or prophylactic agent for obesity.
31. The medicine according to the above-described 30, wherein the other therapeutic or prophylactic agent for obesity is a leptin preparation, a pancreatic lipase inhibitor, a noradrenaline-serotonin reuptake inhibitor, a cannabinoid receptor antagonist, a monoamine reuptake inhibitor, a diacylglycerol acyltransferase (DGAT) inhibitor, a glucose-dependent insulinotropic polypeptide (GIP) receptor antagonist, a leptin receptor agonist, a bombesin receptor subtype 3 (BRS-3) agonist, aperilipininhibitor, anacetyl-COA carboxylase 1 (ACC1) inhibitor, an acetyl-CoA carboxylase 2 (ACC2) inhibitor, a fatty acid synthase inhibitor, an sn-1-acyl-glycerol-3-phosphate acyltransferase (AGPAT) inhibitor, a pancreas phospholipase A2 (pPLA2) inhibitor, a melanocortin (MC) receptor agonist, a neuropeptide Y5 (NPY5) receptor antagonist, an uncoupling protein (UCP) inducer or activator, a carnitine palmitoyltransferase 1 (CPT-1) activator, a CCK1 (CCKA) agonist, a ciliaryneurotrophic factor (CNTF), a CRF2 agonist, a neuropeptide Y2 (NPY2) receptor antagonist, a neuropeptide Y4 (NPY4) receptor antagonist, a thyroid hormone receptor β agonist, a growth hormone, an ATP citrate lyase inhibitor, a 5-HT6 antagonist or a 5-HT2C agonist.
32. The medicine according to the above-described 30 or 31, wherein the other therapeutic orprophylactic agent for obesity is leptin, orlistat, sibutramine, rimonabant or mazindol.
33. A medicine comprising a combination of a pharmaceutical composition for treating or preventing diabetes according to any of the above-described 1 to 20 with another therapeutic or prophylactic agent for hypertension.
34. The medicine according to the above-described 33, wherein the other therapeutic or prophylactic agent for hypertension is a thiazide diuretic, a similar thiazide diuretic, a loop diuretic, a K retaining diuretic, a β blocker, an α, β blocker, an α blocker, a central sympathetic nerve depressant, a peripheral sympathetic nerve depressant (rauwolfia preparation), a Ca antagonist (benzothiazepin), a Ca antagonist (dihydropyridine), a vasodilator, an angiotensin converting enzyme (ACE) inhibitor, an angiotensin II receptor antagonist, a nitric acid preparation, an endothelin ETA receptor antagonist, an endothelin-converting enzyme inhibitor; a neprilysin inhibitor, a prostaglandin; a prostanoid FP agonist, a renin inhibitor, an NOS3 expression enhancer; a prostacyclin analogue, a phosphodiesterase V (PDE5A) inhibitor, a prostacyclin analogue or an aldosterone antagonist.
35. The medicine according to the above-described 33 or 34, wherein the other therapeutic or prophylactic agent for hypertension is hydrochlorothiazide, trichlormethiazide, bentylhydrochlorothiazide, meticrane, indapamide, tripamide, chlortalidone, mefruside, furosemide, spironolactone, triamteren, atenolol, bisoprolol fumarate, betaxolol hydrochloride, bevantololhydrochloride, metoprolol tartrate, acebutolol hydrochloride, celiprolol hydrochloride, nipradilol, tilisolol hydrochloride, nadolol, propranolol hydrochloride, indenolol hydrochloride, carteolol hydrochloride, pindolol, pindolol sustained-release tablet, bunitrolol hydrochloride, penbutolol sulfate, bopindolol malonate, amosulalol hydrochloride, arotinolol hydrochloride, carvedilol, labetalol hydrochloride, urapidil, terazosin hydrochloride, doxazosin mesilate, bunazosin hydrochloride, prazosin hydrochloride, phentolamine mesilate, clonidine hydrochloride, guanfacine hydrochloride, guanabenz acetate, methyldopa, reserpine, rescinnamine, amlodipine besilate, aranidipine, efonidipine hydrochloride, cilnidipine, nicardipine hydrochloride, nisoldipine, nitrendipine, nifedipine, sustained-release nifedipine, nilvadipine, barnidipine hydrochloride, felodipine, benidipine hydrochloride, manidipine hydrochloride, azelnidipine, diltiazem hydrochloride, hydrazine monohydrochloride, todoralazine hydrochloride, budralazine, cadralazine, captopril, enalapril maleate, alacepril, delapril hydrochloride, cilazapril, lisinopril, benazepril hydrochloride, imidapril hydrochloride, tamocapril hydrochloride, quinapril hydrochloride, trandolapril, perindopril erbumine, candesartan cilexetil, valsartan, telmisartan, olmesartan medoxomil, sodium nitroprusside or nitroglycerine.
36. A pyrazole compound represented by the following general formula (I): 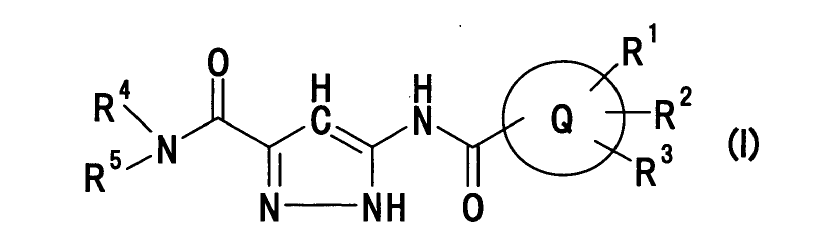 wherein,
   Ring Q represents an aryl group or a heteroaromatic group;
   R¹ represents a hydrogen atom, a halogen atom, a C₁₋₆ alkyl group or a C₁₋₆ alkoxy group;
   R² represents a halogen atom, a C₁₋₆ alkyl group, a C₁₋₆ alkoxy group or an azido group;
   R³ represents a halogen atom, a hydroxyl group, a C₁₋₆ alkyl group, a halo C₁₋₆ alkyl group, a C₁₋₆ alkoxy group, an azido group, an amino group, an acylamino group or a C₁₋₆ alkylsulfonylamino group; and
   R⁴ and R⁵ are identical with or different from each other, and represent
      (1) a hydrogen atom;
      (2) a C₁₋₆ alkyl group that may be substituted with one or more substituents selected from Group A below:
         [Group A]
         A1. a hydroxyl group,
         A2. a C₁₋₆ alkoxy group,
         A3. -N(R⁴¹) (R^{41'})
            wherein R⁴¹ and R⁴¹' are identical with or different from each other and represent a hydrogen atom or a C₁₋₆ alkyl group, or R⁴¹ and R⁴¹' may, together with the adjacent nitrogen atom, forma saturated 5- or 6-memberedmonocyclicheterocyclic ring,
         A4. an aryl group,
         A5. a heteroaromatic group,
         A6. -CO-N(R⁴¹¹) (R^{411'})
            wherein R⁴¹¹ and _{R}⁴¹¹ ' are identical with or different from each other and represent a hydrogen atom or a C₁₋₆ alkyl group, or R⁴¹¹ and R^{411'} may, together with the adjacent nitrogen atom, form a saturated 5- or 6-memberedmonocyclic heterocyclic ring,
         A7. a carboxy group,
            and,
         A8. a C₁₋₆ alkoxycarbonyl group,
      (3) a C₂₋₆ alkenyl group;
      (4) a C₂₋₆ alkynyl group;
      (5) a C₃₋₈ cycloalkyl group;
         wherein the cycloalkyl group may be substituted with a hydroxyl group, a carboxy group or a C₁₋₆ alkoxycarbonyl group or may be condensed with a pyridine ring,
      (6) a C₃₋₈ cycloalkyl C₁₋₆ alkyl group;
         wherein the cycloalkyl group of the C₃₋₈ cycloalkyl C₁₋₆ alkyl group may be substituted with a hydroxyl group, a carboxy group or a C₁₋₆ alkoxycarbonyl group or may be condensed with a pyridine ring,
      (7) a saturated 5- or 6-memberedmonocyclic heterocyclic group that may be substituted with one or more substituents selected from Group C below:
         [Group C]
         C1. a C₁₋₆ alkyl group,
         C2. an acyl group,
         C3. a C₁₋₆ alkylsulfonyl group,
         C4. a carboxy group,
         C5. a C₁₋₆ alkoxycarbonyl group,
            and,
         C6. -CO-(Alk)n-COOR⁵²,
            wherein R⁵² is a hydrogen atom or a C₁₋₆ alkyl group, Alk is a C₁₋₄ alkylene group, and n is 0 or an integer from 1 to 3,
      (8) an aryl group that may be substituted with one or more substituents selected from Group D below:
         [Group D]
         D1. a hydroxyl group,
         D2. a C₁₋₆ alkoxy group,
         D3. a cyano group,
         D4. a C₁₋₆ alkyl group,
            wherein the C₁₋₆ alkyl group may be substituted with one or more substituents selected from the group consisting of a hydroxyl group, a carboxy group and a C₁₋₆ alkoxycarbonyl group,
         D5. -N(R⁵³) (R^{53'}),
            wherein R⁵³ and R^{53'} are identical with or different from each other and represent a hydrogen atom, a C₁₋₆ alkyl group or a C₁₋₆ alkylsulfonyl group,
         D6. -CO-N(R ⁵³¹)(R^{531'})
            wherein R⁵³¹ and R^{531'} are identical with or different from each other and represent a hydrogen atom, a C₁₋₆ alkyl group or a C₁₋₆ alkylsulfonyl group,
         D7. -COOR⁵⁴,
            wherein R⁵⁴ is a hydrogen atom, a C₁₋₆ alkyl group, a C₁₋₆ alkylcarbonyloxy C₁₋₆ alkyl group or a C₃₋₈ cycloalkyloxycarbonyloxy C₁₋₆ alkyl group,
         D8. -C (NH (OH))=N-R⁵⁵,
            whereinR⁵⁵ is a hydrogen atom or a C₁₋₆ alkylsulfonyl group,
         D9. a saturated 5- or 6-membered monocyclic heterocyclic group,
            and,
         D10. a 5- or 6-membered monocyclic heteroaromatic group that may be substituted with an oxo or thioxo group,
      (9) a 5- or 6-membered monocyclic heteroaromatic group or a 5- or 6-membered condensed heteroaromatic group with a benzene ring, wherein the 5- or 6-membered heteroaromatic groups may be substituted with one or more substituents selected from the group consisting of a carboxy group and a C₁₋₆ alkoxycarbonyl group;
      (10) a C₇₋₁₄ aralkyl group;
         wherein the alkyl moiety of the C₇₋₁₄ aralkyl group may be substituted with one or two substituents selected from Group E below, and the aryl moiety may be substituted with one or more substituents selected from Group F below:
         [Group E]
         E1. a C₁₋₆ alkyl group that may be substituted with a hydroxyl group,
         E2. a cyano group,
         E3. a carboxy group,
         E4. a C₁₋₆ alkoxycarbonyl group,
            and,
         E5. a phenyl group,
         [Group F]
         F1. a C₁₋₆ alkyl group,
         F2. a halogen atom,
         F3. a cyano group,
         F4. a hydroxyl group,
         F5. a C₁₋₆ alkoxy group that may be substituted with one or more substituents selected from the group consisting of a carboxy group and a C₁₋₆ alkoxycarbonyl group,
         F6. a halo C₁₋₆ alkyl group,
         F7. a carboxy group,
         F8. a C₁₋₆ alkoxycarbonyl group,
         F9. -CO-N(R ^{56a}) (R^{56a'}),
            wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f below:
            [Group f]
            f1. an amino group,
            f2. a mono C₁₋₆ alkylamino group,
            f3. a di C₁₋₆ alkylamino group,
            f4. a carboxy group,
            f5. a C₁₋₆ alkoxycarbonyl group,
            f6. a hydroxyl group,
               and,
            f7. a saturated 5- or 6-membered monocyclic heterocyclic group having at least one nitrogen atom,
         F10. -N (R^{56b})(R^{56b'})
            wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
         F11. -N=CR⁵⁷ (-N(R⁵⁸) (R^{58'})),
            wherein R⁵⁷ is a hydrogen atom or a C₁₋₆ alkyl group, R⁵⁸ and R^{58'} are identical with or different from each other and represent a hydrogen atom or a C₁₋₆ alkyl group,
         F12. a 5- or 6-membered monocyclic heteroaromatic group,
            and,
         F13. a methylenedioxy group or an ethylenedioxy group, or,
      (11) a C₁₋₆ alkyl group substituted with one or more substituents selected from the group consisting of a 5- or 6-membered monocyclic heteroaromatic group and a 5- or 6-membered condensed heteroaromatic group with a benzene ring;
         wherein the heteroaromatic groups maybe substituted with one or more substituents selected from Group G below:
         [Group G]
         G1. a C₁₋₆ alkyl group that may be substituted with one or more substituents selected from Group g below:
            [Group g]
            g1. a halogen atom,
            g2. an amino group,
            g3. a mono C₁₋₆ alkylamino group,
            g4. a di C₁₋₆ alkylamino group,
            g5. a C₁₋₆ alkoxycarbonylamino group,
               and,
            g6. a hydroxyimino group,
         G2. a halogen atom,
         G3. a C₁₋₆ alkoxy group that may be substituted with a halogen atom,
         G4. an aryloxy group,
         G5. a cyano group,
         G6. -N (R^{59a}) (R^{59a'}),
            wherein R^{59a} and R^{59a'} are identical with or different from each other and represent a hydrogen atom or a C₁₋₆ alkyl group, or R^{59a} and R^{59a'} may, together with the adjacent nitrogen atom, forma saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
         G7. -CO-N (R^{59b}) (R^{59b'})
            wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
         G8. an aryl group,
            and,
         G9. a 5- or 6-membered monocyclic heteroaromatic group that may be substituted with an oxo or thioxo group,
   R⁴ and R⁵ may, together with the adjacent nitrogen atom, form a saturated monocyclic 5- or 6-membered heterocyclic ring, wherein a part of the saturated heterocyclic ring may have a double bond, and the saturated heterocyclic ring may be condensed with a benzene ring to form a condensed ring, the saturated heterocyclic ring which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof.
37. The pyrazole compound according to the above-described 36 represented by the following general formula (I): 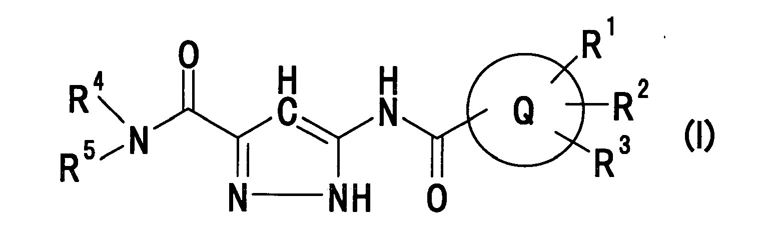 wherein Ring Q represents an aryl group or a heteroaromatic group;
   R¹ represents a hydrogen atom, a halogen atom, a C₁₋₆ alkyl group or a C₁₋₆ alkoxy group;
   R² represents a halogen atom, a C₁₋₆ alkyl group, a C₁₋₆ alkoxy group or an azido group;
   R³ represents a halogen atom, a hydroxyl group, a C₁₋₄ alkyl group, a halo C₁₋₆ alkyl group, a C₁₋₆ alkoxy group, an azido group, an amino group, an acylamino group or a C₁₋₆ alkylsulfonylamino group;
   R⁴ represents
      (1) a hydrogen atom;
      (2) a C₁₋₆ alkyl group that may be substituted with one or more substituents selected from Group A below:
         [Group A]
         A1. a hydroxyl group,
         A2. a C₁₋₆ alkoxy group,
         A3. -N(R⁴¹) (R^{41'}),
            wherein R⁴¹ and R^{41'} are identical with or different from each other and represent a hydrogen atom or a C₁₋₆ alkyl group, or R⁴¹ and R^{41'} may, together with the adjacent nitrogen atom, forma saturated 5- or 6-memberedmonocyclic heterocyclic ring,
         A4. an aryl group,
         A5. a heteroaromatic group,
         A6. -CO-N (R⁴¹¹) (R^{411'}),
            wherein R⁴¹¹ and R^{411'} are identical with or different from each other and represent a hydrogen atom or a C₁₋₆ alkyl group, or R⁴¹¹ and R^{411'} may, together with the adjacent nitrogen atom, forma saturated 5- or 6-memberedmonocyclic heterocyclic ring,
         A7. a carboxy group,
            and,
         A8. a C₁₋₆ alkoxycarbonyl group,
      (3) a C₂₋₆ alkenyl group;
      (4) a C₂₋₆ alkynyl group;
      (5) a C₃₋₈ cycloalkyl group;
      (6) a C₃₋₈ cycloalkyl C₁₋₆ alkyl group;
         or,
      (7) an aryl group; and
   R⁵ represents
      (1) a C₁₋₆ alkyl group that may be substituted with one or more substituents selected from Group B below:
         [Group B]
         B1. a hydroxyl group,
         B2. a C₁₋₆ alkoxy group,
         B3. -N (R⁵¹) (R^{51'}),
            wherein R⁵¹ and R^{51'} are identical with or different from each other and represent a hydrogen atom or a C₁₋₆ alkyl group, or R⁵¹ and R^{51'} may, together with the adjacent nitrogen atom, forma saturated 5- or 6-memberedmonocyclic heterocyclic ring, and,
         B4. -CO-N(R⁵¹¹) (R^{511'}),
            wherein R⁵¹¹ and R^{511'} are identical with or different from each other and represent a hydrogen atom or a C₁₋₆ alkyl group, or R⁵¹¹ and R^{511'} may, together with the adjacent nitrogen atom, forma saturated 5- or 6-memberedmonocyclic heterocyclic ring,
      (2) a C₃₋₈ cycloalkyl group;
         wherein the cycloalkyl group may be substituted with a hydroxyl group, a carboxy group or a C₁₋₆ alkoxycarbonyl group or may be condensed with a pyridine ring,
      (3) a C₃₋₈ cycloalkyl C₁₋₆ alkyl group;
         wherein the cycloalkyl group of the C₃₋₈ cycloalkyl C₁₋₆ alkyl group may be substituted with a hydroxyl group, a carboxy group or a C₁₋₆ alkoxycarbonyl group or may be condensed with a pyridine ring,
      (4) a saturated 5- or 6-membered monocyclic heterocyclic group that may be substituted with one or more substituents selected from Group C below:
         [Group C]
         C1. a C₁₋₆ alkyl group,
         C2. an acyl group,
         C3. a C₁₋₆ alkylsulfonyl group,
         C4. a carboxy group,
         C5. a C₁₋₆ alkoxycarbonyl group,
            and,
         C6. -co- (Alk) n-COOR⁵²,
            wherein R⁵² is a hydrogen atom or a C₁₋₆ alkyl group, Alk is a C₁₋₄ alkylene group, and n is 0 or an integer from 1 to 3,
      (5) an aryl group that may be substituted with one or more substituents selected from Group D below:
         [Group D]
         D1. a hydroxyl group,
         D2. a C₁₋₆ alkoxy group,
         D3. a cyano group,
         D4. a C₁₋₆ alkyl group,
            wherein the C₁₋₆ alkyl group may be substituted with one or more substituents selected from the group consisting of a hydroxyl group, a carboxy group and a C₁₋₆ alkoxycarbonyl group,
         D5. -N(R⁵³) (R^{53'}),
            wherein R⁵³ and R^{53'} are identical with or different from each other and represent a hydrogen atom, a C₁₋₆ alkyl group or a C₁₋₆ alkylsulfonyl group,
         D6. -CO-N(R⁵³¹) (R^{531'}),
            wherein R⁵³¹ and R^{531'} are identical with or different from each other and represent a hydrogen atom, a C₁₋₆ alkyl group or a C₁₋₆ alkylsulfonyl group,
         D7. -COOR⁵⁴,
            wherein R⁵⁴ is a hydrogen atom, a C₁₋₆ alkyl group, a C₁₋₆ alkylcarbonyloxy C₁₋₆ alkyl group or a C₃₋₈ cycloalkyloxycarbonyloxy C₁₋₆ alkyl group,
         D8. -C(NH(OH))=N-R⁵⁵,
            wherein R⁵⁵ is a hydrogen atom or a C₁₋₆ alkylsulfonyl group,
         D9. a saturated 5- or 6-membered monocyclic heterocyclic group,
            and,
         D10. a 5- or 6-membered monocyclic heteroaromatic group that may be substituted with an oxo or thioxo group,
      (6) a 5- or 6-membered monocyclic heteroaromatic group or a 5- or 6-membered condensed heteroaromatic group with a benzene ring, wherein the 5- or 6-membered heteroaromatic groups may be substituted with one or more substituents selected from the group consisting of a carboxy group and a C₁₋₆ alkoxycarbonyl group;
      (7) a C₇₋₁₄ aralkyl group;
         wherein the alkyl moiety of the C₇₋₁₄ aralkyl group may be substituted with one or two substituents selected from Group E below, and the aryl moiety may be substituted with one or more substituents selected from Group F below:
         [Group E]
         E1. a C₁₋₆ alkyl group that may be substituted with a hydroxyl group,
         E2. a cyano group,
         E3. a carboxy group,
         E4. a C₁₋₆ alkoxycarbonyl group,
            and,
         E5. a phenyl group,
         [Group F]
         F1. a C₁₋₆ alkyl group,
         F2. a halogen atom,
         F3. a cyano group,
         F4. a hydroxyl group,
         F5. a C₁₋₆ alkoxy group that may be substituted with one or more substituents selected from the group consisting of a carboxy group and a C₁₋₆ alkoxycarbonyl group,
         F6. a halo C₁₋₆ alkyl group,
         F7. a carboxy group,
         F8. a C₁₋₆ alkoxycarbonyl group,
         F9. -CO-N (R^{56a}) (R^{56a'}),
            wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f below:
            [Group f]
            f1. an amino group,
            f2. a mono C₁₋₆ alkylamino group,
            f3. a di C₁₋₆ alkylamino group,
            f4. a carboxy group,
            f5. a C₁₋₆ alkoxycarbonyl group,
            f6. a hydroxyl group,
               and,
            f7. a saturated 5- or 6-membered monocyclic heterocyclic group having at least one nitrogen atom,
         F10. -N (R^{56b}) (R^{56b'}),
            wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
         F11. -N=CR⁵⁷(-N(R⁵⁸) (R^{58'})),
            wherein R⁵⁷ is a hydrogen atom or a C₁₋₆ alkyl group, R⁵⁸ and R^{58'} are identical with or different from each other and represent a hydrogen atom or a C₁₋₆ alkyl group,
         F12. a 5- or 6-membered monocyclic heteroaromatic group, and,
         F13. a methylenedioxy group or an ethylenedioxy group, or,
      (8) a C₁₋₆ alkyl group substituted with a 5- or 6-membered monocyclic heteroaromatic group or a C₁₋₆ alkyl group substituted with a 5- or 6-membered condensed heteroaromatic group with a benzene ring;
         wherein the heteroaromatic group may be substituted with one or more substituents selected from Group G below:
         [Group G]
         G1. a C₁₋₆ alkyl group that may be substituted with one or more substituents selected from Group g below:
            [Group g]
            g1. a halogen atom,
            g2. an amino group,
            g3. a mono C₁₋₆ alkylamino group,
            g4. a di C₁₋₆ alkylamino group,
            g5. a C₁₋₆ alkoxycarbonylamino group,
               and,
            g6. a hydroxyimino group,
         G2. a halogen atom,
         G3. a C₁₋₆ alkoxy group that may be substituted with a halogen atom,
         G4. an aryloxy group,
         G5. a cyano group,
         G6. -N(R^{59a}) (R^{59a'}),
            wherein R^{59a} and R^{59a'} are identical with or different from each other and represent a hydrogen atom or a C₁₋₆ alkyl group, or R^{59a} and R^{59a'} may, together with the adjacent nitrogen atom, form a saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
         G7. -CO-N(R^{59b}) (R^{59b'})
            wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
         G8. an aryl group,
            and,
         G9. a 5- or 6-membered monocyclic heteroaromatic group that may be substituted with an oxo or thioxo group,
   R⁴ and R⁵ may, together with the adjacent nitrogen atom, forma saturated 5- or 6-memberedmonocyclic heterocyclic ring, wherein a part of the saturated heterocyclic ring may have a double bond, and the saturated heterocyclic ring may be condensed with a benzene ring to form a condensed ring, or the saturated heterocyclic ring which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof.
38. The pyrazole compound according to the above-described 36 represented by the following general formula (II'): 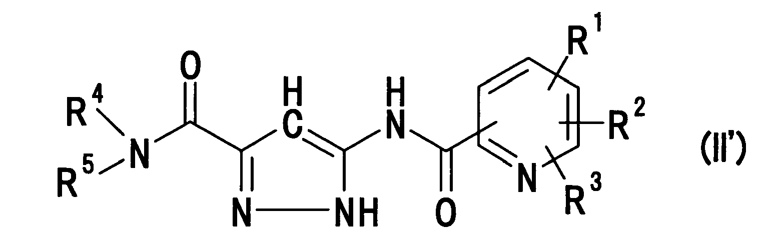 wherein R¹, R², R³, R⁴ and R⁵ are as defined in the above-described 36,
   or a pharmacologically acceptable salt thereof.
39. The pyrazole compound according to the above-described 36 represented by the following general formula (II): 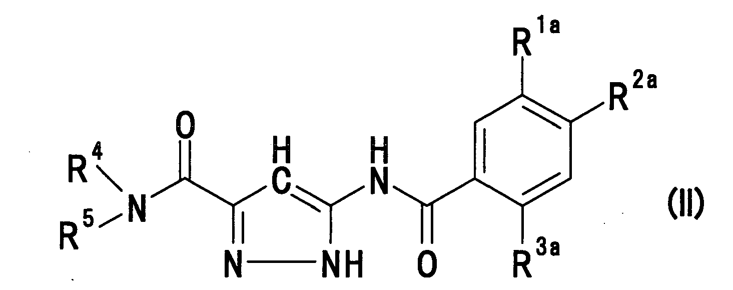 wherein R⁴ and R⁵ are as defined in the above-described 36, R^{1a} represents a halogen atom or a hydrogen atom, R^{2a} a halogen atom, and R^{3a} a halogen atom or a C₁₋₆ alkyl group, or a pharmacologically acceptable salt thereof.
40. The pyrazole compound according to the above-described 39, wherein R^{1a} is a hydrogen or fluorine atom, R^{2a} is a fluorine or chlorine atom, and R^{3a} is a chlorine atom or a C₁₋₆ alkyl group, or a pharmacologically acceptable salt thereof.
41. The pyrazole compound according to the above-described 40, wherein the pyrazole compound is a pyrazole compound represented by the following general formula (III): 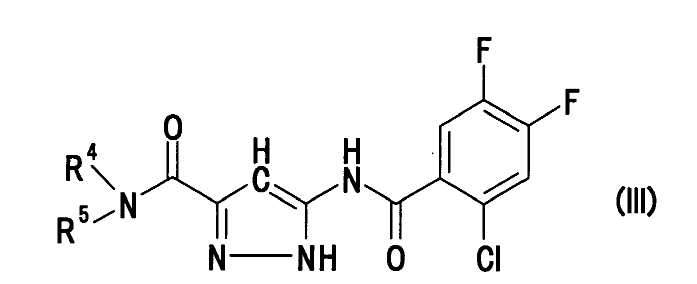 wherein R⁴ and R⁵ are as defined in the above-described 37, or a pharmacologically acceptable salt thereof.
42. The pyrazole compound according to any of the above-described 37 to 41, wherein R⁴ is a group selected from the following group:
   (1) a hydrogen atom;
   (2) a C₁₋₆ alkyl group that may be substituted with one or more substituents selected from Group A' below:
      [Group A']
      A'1. a hydroxyl group,
      A'2. a C₁₋₄ alkoxy group,
      A'3. -N(R⁴¹) (R^{41'}),
         wherein _{R}⁴¹ and R⁴¹' are identical with or different from each other and represent a hydrogen atom or a C₁₋₄ alkyl group, or R⁴¹ and R⁴¹' may, together with the adjacent nitrogen atom, forma saturated 5- or 6-memberedmonocyclic heterocyclic ring,
      A'4. a phenyl group,
      A'5. a 5- or 6-membered monocyclic heteroaromatic group,
      A'6. -CO-N(R ⁴¹¹) (R^{411'}),
         wherein R⁴¹¹ and R^{411'} are identical with or different from each other and represent a hydrogen atom or a C₁₋₄ alkyl group, or R⁴¹¹ and R^{411'} may, together with the adjacent nitrogen atom, forma saturated 5- or 6-memberedmonocyclicheterocyclic ring,
      A'7. a carboxy group,
         and,
      A'8. a C₁₋₄ alkoxycarbonyl group,
   (3) a C₂₋₆ alkenyl group;
   (4) a C₂₋₆ alkynyl group;
   (5) a C₃₋₈ cycloalkyl group;
      and,
   (6) a C₃₋₈ cycloalkyl C₁₋₄ alkyl group;
      or a pharmacologically acceptable salt thereof.
43. The pyrazole compound according to any of the above-described 37 to 42, wherein R⁵ is a C₁₋₆ alkyl group, or a pharmacologically acceptable salt thereof.
44. The pyrazole compound according to any of the above-described 37 to 42, wherein R⁵ is a C₃₋₈ cycloalkyl group that may be substituted with one or more substituents selected from the group consisting of a hydroxyl group, a carboxy group or a C₁₋₆ alkoxycarbonyl group, or a pharmacologically acceptable salt thereof.
45. The pyrazole compound according to any of the above-described 37 to 42, wherein R⁵ is a C₃₋₈ cycloalkyl C₁₋₆ alkyl group, or a pharmacologically acceptable salt thereof.
46. The pyrazole compound according to any of the above-described 37 to 42, wherein R⁵ is a saturated 5- or 6-membered monocyclic heterocyclic group that may be substituted with one or more substituents selected from the group consisting of an acyl group and -CO-(Alk)n-COOR⁵², wherein R⁵² is a hydrogen atom or a C₁₋₆ alkyl group, Alk is a C₁₋₄ alkylene group, and n is 0 or an integer from 1 to 3, or a pharmacologically acceptable salt thereof.
47. The pyrazole compound according to any of the above-described 37 to 42, wherein R⁵ is a phenyl group that may be substituted with one or more substituents selected from the following Group D':
   [Group D']
   D'1 a C₁₋₄ alkyl group that may be substituted with a carboxy group,
   D'2. -CO-N(R⁵³) (R^{53'}),
      wherein R⁵³ and R^{53'} are identical with or different from each other and represent a hydrogen atom, a C₁₋₄ alkyl group or a C₁₋₄ alkylsulfonyl group,
   D'3. -COOR⁵⁴,
      wherein R⁵⁴ is a hydrogen atom, a C₁₋₄ alkyl group, a C₁₋₄ alkylcarbonyloxy C₁₋₄ alkyl group or a C₃₋₈ cycloalkyloxycarbonyloxy C₁₋₄ alkyl group,
      and,
   D'4. a 5- or 6-membered monocyclic heteroaromatic group that may be substituted with an oxo or thioxo group, or a pharmacologically acceptable salt thereof.
48. The pyrazole compound according to any of the above-described 37 to 42, wherein R⁵ is a C₇₋₁₄ aralkyl group and the aryl moiety of the C₇₋₁₄ aralkyl group may be substituted with one or more substituents selected from the following Group F':
   [Group F']
   F'1. a C₁₋₆ alkyl group,
   F'2. a halogen atom,
   F'3. a cyano group,
   F'4. a hydroxyl group,
   F'5. a C₁₋₆ alkoxy group that may be substituted with one or more substituents selected from the group consisting of a carboxy group and a C₁₋₆ alkoxycarbonyl group,
   F'6. a halo C₁₋₆ alkyl group,
   F'7. a carboxy group,
   F'8. a C₁₋₆ alkoxycarbonyl group,
   F'9. -CO-N (R^{56a}) (R^{56a'})
      wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f' below:
      [Group f']
      f'1. an amino group,
      f'2. a mono C₁₋₆ alkylamino group,
      f'3. a di C₁₋₆ alkylamino group,
      f'4. a carboxy group,
      f'5. a C₁₋₆ alkoxycarbonyl group,
      f'6. a hydroxyl group,
         and,
      f'7. a saturated 5- or 6-membered monocyclic heterocyclic group having at least one nitrogen atom,
   F'10. -N (R^{56b}) (R^{56b'}),
      wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
   F'11. -N=CR⁵⁷(-N(R⁵⁸) (R^{58'})),
      wherein R⁵⁷ is a hydrogen atom or a C₁₋₄ alkyl group, R⁵⁸ and R^{58'} are identical with or different from each other and represent a hydrogen atom or a C₁₋₄ alkyl group,
   F'12. a 5- or 6-memberedmonocyclic heteroaromatic group,
      and,
   F'13. a methylenedioxy group or an ethylenedioxy group, or a pharmacologically acceptable salt thereof.
49. The pyrazole compound according to any of the above-described 37 to 42, wherein R⁵ is a C₁₋₆ alkyl group substituted with a 5- or 6-membered monocyclic heteroaromatic group and the heteroaromatic group may be substituted with one or more substituents selected from the following Group G':
   [Group G']
   G'1. a C₁₋₆ alkyl group that may be substituted with one or more substituents selected from Group g' below:
      [Group g']
      g'1. a halogen atom,
      g'2. an amino group,
      g'3. a mono C₁₋₆ alkylamino group,
      g'4. a di C₁₋₆ alkylamino group,
      g'5. a C₁₋₆ alkoxycarbonylamino group,
         and,
      g'6. a hydroxyimino group,
   G'2. a halogen atom,
   G'3. a C₁₋₆ alkoxy group that may be substituted with a halogen atom,
   G'4. an aryloxy group,
   G'5. a cyano group,
   G'6. -N(R^{59a}) (R^{59a'}),
      wherein R^{59a} and R^{59a'} are identical with or different from each other and represent a hydrogen atom or a C₁₋₆ alkyl group, or R^{59a} and R^{59a'} may, together with the adjacent nitrogen atom, form a saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
   G'7. -CO-N(R^{59b}) (R^{59b'})
      wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
   G'8. an aryl group,
      and,
   G'9. a 5- or 6-membered monocyclic heteroaromatic group that may be substituted with an oxo or thioxo group, or a pharmacologically acceptable salt thereof.
50. The pyrazole compound according to any of the above-described 37 to 41, wherein R⁵ and R⁴, together with the adjacent nitrogen atom, form a saturated 5- or 6-membered monocyclic heterocyclic ring, and the saturated heterocyclic group may have a double bond in part and may be condensed with a benzene ring to form a condensed ring, and the saturated heterocyclic group which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof.
51. The pyrazole compound according to the above-described 37, wherein the pyrazole compound is a pyrazole compound represented by the following general formula (IV): 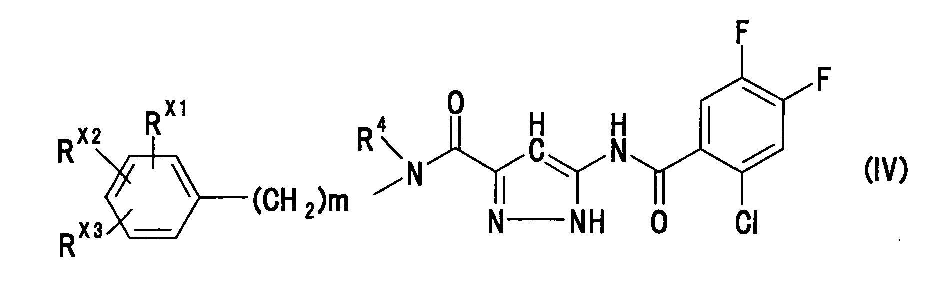 wherein R⁴ is as described in 37, R^{X1}, R^{X2} and R^{X3} are identical with or different from each other and represent a hydrogen atom or a substituent selected from the following Group F'', and m is 0 or an integer from 1 to 2,
   [Group F'']
   F''1. a C₁₋₆ alkyl group,
   F''2. a halogen atom,
   F''3. a cyano group,
   F''4. a hydroxyl group,
   F''5. a C₁₋₄ alkoxy group that may be substituted with one or more substituents selected from the group consisting of a carboxy group and a C₁₋₄ alkoxycarbonyl group,
   F''6. a halo C₁₋₆ alkyl group,
   F''7. a carboxy group,
   F''8. a C₁₋₆ alkoxycarbonyl group,
   F''9. -CO-N (R^{56a}) (R^{56a'})
      wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f'' below:
      [Group f'']
      f''1. an amino group,
      f''2. a mono C₁₋₆ alkylamino group,
      f''3. a di C₁₋₆ alkylamino group,
      f''4. a carboxy group,
      f''5. a C₁₋₆ alkoxycarbonyl group,
      f''6. a hydroxyl group,
         and,
      f''7. a saturated 5- or 6-membered monocyclic heterocyclic group having at least one nitrogen atom,
   F''10. -N(R^{56b}) (R^{56b'}),
      wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
   F''11. -N=CR⁵⁷ (-N (R⁵⁸) (R^{58'})),
      wherein R⁵⁷ is a hydrogen atom or a C₁₋₆ alkyl group, R⁵⁸ and R^{58'} are identical with or different from each other and represent a hydrogen atom or a C₁₋₆ alkyl group,
   F''12. a 5- or 6-membered monocyclic heteroaromatic group,
      and,
   F''13. a methylenedioxy group or an ethylenedioxy group, or a pharmacologically acceptable salt thereof.
52. The pyrazole compound according to the above-described 37, wherein the pyrazole compound is a pyrazole compound represented by the following general formula (V): 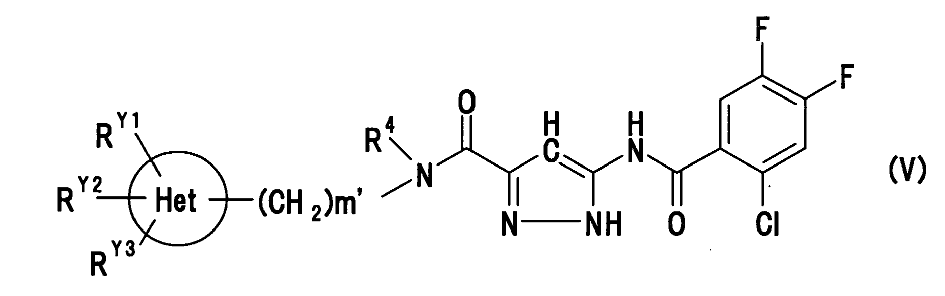 wherein R⁴ is as defined above, the ring Het represents a 5-or 6-membered monocyclic heteroaromatic group, R^{Y1}, R^{Y2} and R^{Y3} are identical with or different from each other and represent a hydrogen atom or a substituent selected from the following Group G', and m' is 0 or an integer from 1 to 2,
   [Group G']
   G'1. a C₁₋₆ alkyl group that may be substituted with one or more substituents selected from Group g' below:
      [Group g']
      g'1. a halogen atom,
      g'2. an amino group,
      g'3. a mono C₁₋₆ alkylamino group,
      g'4. a di C₁₋₆ alkylamino group,
      g'5. a C₁₋₆ alkoxycarbonylamino group,
         and,
      g'6. a hydroxyimino group,
   G'2. a halogen atom,
   G'3. a C₁₋₆ alkoxy group that may be substituted with a halogen atom,
   G'4. an aryloxy group,
   G'5. a cyano group,
   G'6. -N(R⁵⁹a) (R^{59a'}),
      wherein R^{59a} and R^{59a'} are identical with or different from each other and represent a hydrogen atom or a C₁₋₆ alkyl group, or R^{59a} and R^{59a'} may, together with the adjacent nitrogen atom, forma saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
   G'7. -CO-N(R^{59b}) (R^{59b'})
      wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
   G'8. an aryl group,
      and,
   G'9. a 5- or 6-membered monocyclic heteroaromatic group that may be substituted with an oxo or thioxo group, or a pharmacologically acceptable salt thereof.
53. The pyrazole compound according to the above-described 52, wherein the ring Het is a pyridine ring, a triazole ring or an oxazole ring, or a pharmacologically acceptable salt thereof.
54. The pyrazole compound according to the above-described 53, wherein the pyrazole compound is a pyrazole compound represented by the following general formula (VI), 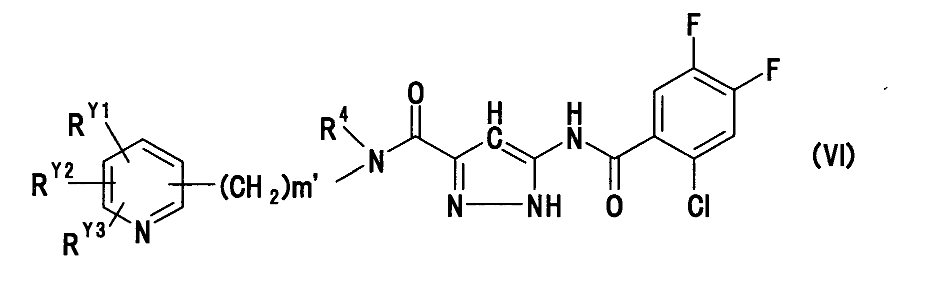 wherein R⁴, R^{Y1}, R^{Y2}, R^{Y3} and m' are as defined above, or a pharmacologically acceptable salt thereof.
55. The pyrazole compound or a pharmacologically acceptable salt thereof according to the above-described 37, wherein the pyrazole compound or a pharmacologically acceptable salt thereof is selected from the following group:
   5-(2-Chloro-4,5-difluoro-benzoylamino)-1H-pyrazole-3-carboxylic acid (pyridin-3-ylmethyl)-amide,
   5-(2-Chloro-4,5-difluoro-benzoylamino)-1H-pyrazole-3-carboxylic acid (pyridin-3-ylmethyl)-amide dihydrochloride,
   5-(2-Chloro-4,5-difluoro-benzoylamino)-1H-pyrazole-3-carboxylic acid (pyridin-3-ylmethyl) -amide hydrochloride,
   5-(2-Chloro-4,5-difluoro-benzoylamino)-1H-pyrazole-3-carboxylic acid (4-methyl-oxazol-5-ylmethyl)-amide,
   5-(2-Chloro-4,5-difluoro-benzoylamino)-1H-pyrazole-3-carboxylic acid (4-methyl-oxazol-5-ylmethyl)-amide,
   5-(2-Chloro-4,5-difluoro-benzoylamino)-1H-pyrazole-3-carboxylic acid (4-methyl-oxazol-5-ylmethyl)amide-p-toluenesulfonate,
   5-(2-Chloro-4,5-difluoro-benzoylamino)-1H-pyrazole-3-carboxylic acid (4-methyl-oxazol-5-ylmethyl)amide-L-(+)-tartrate,
   5-(2-Chloro-4,5-difluoro-benzoylamino)-1H-pyrazole-3-carboxylic acid (2,4-dimethyl-oxazol-5-ylmethyl)-amide p-toluenesulfonate,
   5-(2-Chloro-4,5-difluoro-benzoylamino)-1H-pyrazole-3-carboxylic acid (2,4-dimethyl-oxazol-5-ylmethyl)-amide,
   5-(2-Chloro-4,5-difluoro-benzoylamino)-1H-pyrazole-3-carboxylic acid (2,4-dimethyl-pyridin-3-ylmethyl)amide,
   5-(2-Chloro-4,5-difluoro-benzoylamino)-1H-pyrazole-3-carboxylic acid diethylamide,
   5-(2-Chloro-4,5-difluoro-benzoylamino)-1H-pyrazole-3-carboxylic acid ethyl-(pyridin-4-ylmethyl)-amide,
   5-(2-Chloro-4,5-difluoro-benzoylamino)-1H-pyrazole-3-carboxylic acid (2-methoxy-ethyl)-methyl-amide,
   5-(2-Chloro-4,5-difluoro-benzoylamino)-1H-pyrazole-3-carboxylic acid ethyl-(2-methoxy-ethyl)-amide,
   5-(2-Chloro-4,5-difluoro-benzoylamino)-1H-pyrazole-3-carboxylic acid 2-fluoro-benzylamide,
   5-(2-Chloro-4,5-difluoro-benzoylamino)-1H-pyrazole-3-carboxylic acid 3-fluoro-benzylamide,
   5-(2-Chloro-4,5-difluoro-benzoylamino)-1H-pyrazole-3-carboxylic acid 2-dimethylamino-benzylamide,
   5-(2-Chloro-4,5-difluoro-benzoylamino)-1H-pyrazole-3-carboxylic acid (4-ethoxycarbonyl-cyclohexyl)-propyl-amide,
   5-(2-Chloro-4,5-difluoro-benzoylamino)-1H-pyrazole-3-carboxylic acid (6-methoxy-pyridin-3-ylmethyl)-amide,
   5-(2-Chloro-4,5-difluoro-benzoylamino)-1H-pyrazole-3-carboxylic acid 2,3-dimethoxy-benzylamide,
   5-(2-Chloro-4,5-difluoro-benzoylamino)-1H-pyrazole-3-carboxylic acid 2,4-dimethoxy-benzylamide,
   5-(2-Chloro-4,5-difluoro-benzoylamino)-1H-pyrazole-3-carboxylic acid 2,6-dimethoxy-benzylamide,
   5-(2-Chloro-4,5-difluoro-benzoylamino)-1H-pyrazole-3-carboxylic acid 3,5-dimethoxy-benzylamide,
   5-(2-Chloro-4,5-difluoro-benzoylamino)-1H-pyrazole-3-carboxylic acid 3,4-dimethoxy-benzylamide,
   5-(2-Chloro-4,5-difluoro-benzoylamino)-1H-pyrazole-3-carboxylic acid 2,3-difluoro-benzylamide,
   5-(2-Chloro-4,5-difluoro-benzoylamino)-1H-pyrazole-3-carboxylic acid 2,4-difluoro-benzylamide,
   5-(2-Chloro-4,5-difluoro-benzoylamino)-1H-pyrazole-3-carboxylic acid 2,5-difluoro-benzylamide,
   5-(2-Chloro-4,5-difluoro-benzoylamino)-1H-pyrazole-3-carboxylic acid 2,6-difluoro-benzylamide,
   5-(2-Chloro-4,5-difluoro-benzoylamino)-1H-pyrazole-3-carboxylic acid 3,4-difluoro-benzylamide,
   5-(2-Chloro-4,5-difluoro-benzoylamino)-1H-pyrazole-3-carboxylic acid 3,5-difluoro-benzylamide,
   5-(2-Chloro-4,5-difluoro-benzoylamino)-1H-pyrazole-3-carboxylic acid (2-isopropoxy-pyridin-3-ylmethyl)-amide,
   5-(2-Chloro-4,5-difluoro-benzoylamino)-1H-pyrazole-3-carboxylic acid (2-phenoxy-pyridin-3-ylmethyl)-amide,
   5-(2-Chloro-4,5-difluoro-benzoylamino)-1H-pyrazole-3-carboxylic acid (3,5-difluoro-pyridin-2-ylmethyl)-amide,
   5-(2-Chloro-4,5-difluoro-benzoylamino)-1H-pyrazole-3-carboxylic acid 2-trifluoromethyl-benzylamide,
   5-(2-Chloro-4,5-difluoro-benzoylamino)-1H-pyrazole-3-carboxylic acid 3-trifluoromethyl-benzylamide
   5-(2-Chloro-4,5-difluoro-benzoylamino)-1H-pyrazole-3-carboxylic acid 4-trifluoromethyl-benzylamide,
   5-(2-Chloro-4,5-difluoro-benzoylamino)-1H-pyrazole-3-carboxylic acid 4-tert-butyl-benzylamide,
   5-(2-Chloro-4,5-difluoro-benzoylamino)-1H-pyrazole-3-carboxylic acid (6-isopropoxy-pyridin-3-ylmethyl)-amide,
   5-(2-Chloro-4,5-difluoro-benzoylamino)-1H-pyrazole-3-carboxylic acid [2-(2,2,2-trifluoro-ethoxy)-pyridin-3-ylmethyl]-amide,
   5-(2-Chloro-4,5-difluoro-benzoylamino)-1H-pyrazole-3-carboxylic acid 4-(2-dimethylamino-ethylcarbamoyl)-benzylamide,
   5-(2-Chloro-4,5-difluoro-benzoylamino)-1H-pyrazole-3-carboxylic acid 4-ethyl-benzylamide,
   5-(2-Chloro-4,5-difluoro-benzoylamino)-1H-pyrazole-3-carboxylic acid 4-isopropyl-benzylamide,
   5-(2-Chloro-4,5-difluoro-benzoylamino)-1H-pyrazole-3-carboxylic acid 2-chloro-6-fluoro-benzylamide,
   5-(2-Chloro-4,5-difluoro-benzoylamino)-1H-pyrazole-3-carboxylic acid (6-ethoxy-pyridin-3-ylmethyl)-amide dihydrochloride,
   5-(2-Chloro-4,5-difluoro-benzoylamino)-1H-pyrazole-3-carboxylic acid (thiazol-4-ylmethyl)-amide,
   5-(2-Chloro-4,5-difluoro-benzoylamino)-1H-pyrazole-3-carboxylic acid [6-(2,2,2-trifluoro-ethoxy)-pyridin-3-ylmethyl]-amide dihydrochloride,
   5-(2-Chloro-4,5-difluoro-benzoylamino)-1H-pyrazole-3-carboxylic acid 2,6-dimethyl-benzylamide,
   5-(2-Chloro-4,5-difluoro-benzoylamino)-1H-pyrazole-3-carboxylic acid [2-(pyridin-3-yl)-thiazol-4-ylmethyl]-amide dihydrochloride,
   5-(2-Chloro-4,5-difluoro-benzoylamino)-1H-pyrazole-3-carboxylic acid (1H-benzoimidazol-2-ylmethyl)-amide dihydrochloride, [4-({[5-(2-Chloro-4,5-difluoro-benzoylamino)-1H-pyrazole-3-carbonyl]-amino}-methyl)-benzoylamino]-acetic acid methyl ester, 3-[4-({[5-(2-Chloro-4,5-difluoro-benzoylamino)-1H-pyrazole-3-carbonyl]-amino}-methyl)-benzoylamino]-propioni c acid methyl ester
   5-(2-Chloro-4,5-difluoro-benzoylamino)-1H-pyrazole-3-carboxylic acid (2-methyl-thiazol-4-ylmethyl)-amide dihydrochloride,
   5-(2-Chloro-4,5-difluoro-benzoylamino)-1H-pyrazole-3-carboxylic acid (3,5-dimethyl-isoxazol-4-ylmethyl)-amide,
   5-(2-Chloro-4,5-difluoro-benzoylamino)-1H-pyrazole-3-carboxylic acid 4-[2-bis-(2-acetoxyethyl)-amino-ethylcarbamoyl]-benzyl amide,
   5-(2-Chloro-4,5-difluoro-benzoylamino)-1H-pyrazole-3-carboxylic acid4-(2-hydroxy-ethylcarbamoyl)-benzylamide,
   5-(2-Chloro-4,5-difluoro-benzoylamino)-1H-pyrazole-3-carboxylic acid 4-{2-[(2-acetoxyethyl)-(2-hyroxyethyl)-amino]-ethylcarbamoyl}-benzylamide,
   5-(2-Chloro-4,5-difluoro-benzoylamino)-1H-pyrazole-3-carboxylic acid (1-methyl-1H-benzoimidazol-2-ylmethyl)-amide hydrochloride,
   5-(2-Chloro-4,5-difluoro-benzoylamino)-1H-pyrazole-3-carboxylic acid (thiazol-2-ylmethyl)-amide dihydrochloride,
   5-(2-Chloro-4,5-difluoro-benzoylamino)-1H-pyrazole-3-carboxylic acid (benzothiazol-2-ylmethyl)-amide dihydrochloride,
   5-(2-Chloro-4,5-difluoro-benzoylamino)-1H-pyrazole-3-carboxylic acid (2,5-dimethyl-thiazol-4-ylmethyl)-amide dihydrochloride,
   5-(2-Chloro-4,5-difluoro-benzoylamino)-1H-pyrazole-3-carboxylic acid [2-(morpholin-4-yl)-thiazol-4-ylmethyl]-amide dihydrochloride,
   5-(2-Chloro-4,5-difluoro-benzoylamino)-1H-pyrazole-3-carboxylic acid (1,3,5-trimethyl-1H-pyrazol-4-ylmethyl)-amide,
   5-(2-Chloro-4,5-difluoro-benzoylamino)-1H-pyrazole-3-carboxylic acid (2-chloro-6-methyl-pyridin-3-ylmethyl)-amide dihydrochloride,
   5-(2-Chloro-4,5-difluoro-benzoylamino)-1H-pyrazole-3-carboxylic acid (2-dimethylamino-thiazol-4-ylmethyl)-amide dihydrochloride,
   5-(2-Chloro-4,5-difluoro-benzoylamino)-1H-pyrazole-3-carboxylic acid (1-methyl-1H-pyrrol-2-ylmethyl)-amide,
   5-(2-Chloro-4,5-difluoro-benzoylamino)-1H-pyrazole-3-carboxylic acid (3,4-dimethoxy-pyridin-2-ylmethyl)-amide dihydrochloride,
   5-(2-Chloro-4,5-difluoro-benzoylamino)-1H-pyrazole-3-carboxylic acid (5-tert-butyl-thiazol-2-ylmethyl)-amide dihydrochloride,
   5-(2-Chloro-4,5-difluoro-benzoylamino)-1H-pyrazole-3-carboxylic acid (5-methyl-2-phenyl-2H-[1,2,3]triazol-4-ylmethyl)-amide
   5-(2-Chloro-4,5-difluoro-benzoylamino)-1H-pyrazole-3-carboxylic acid [4-(tert-butoxycarbonyl-amino)-methyl-pyridin-2-ylmethyl]-amide,
   5-(2-Chloro-4,5-difluoro-benzoylamino)-1H-pyrazole-3-carboxylic acid [4-methyl-2-(morpholin-4-yl)-thiazol-5-ylmethyl]-amide dihydrochloride,
   5-(2-Chloro-4,5-difluoro-benzoylamino)-1H-pyrazole-3-carboxylic acid (pyridin-3-ylmethyl)-amide 3/2hydrochloride hemihydrate,
   5-(2-Chloro-4,5-difluoro-benzoylamino)-1H-pyrazole-3-carboxylic acid 2-amino-benzylamide,
   5-(2-Chloro-4,5-difluoro-benzoylamino)-1H-pyrazole-3-carboxylic acid 2-methoxy-benzylamide,
   5-(2-Chloro-4,5-difluoro-benzoylamino)-1H-pyrazole-3-carboxylic acid 3-methoxy-benzylamide,
   5-(2-Chloro-4,5-difluoro-benzoylamino)-1H-pyrazole-3-carboxylic acid 4-methoxy-benzylamide,
   5-(2-Chloro-4,5-difluoro-benzoylamino)-1H-pyrazole-3-carboxylic acid 4-methyl-benzylamide,
   5-(2-Chloro-4,5-difluoro-benzoylamino)-1H-pyrazole-3-carboxylic acid 4-fluoro-benzylamide,
   5-(2-Chloro-4,5-difluoro-benzoylamino)-1H-pyrazole-3-carboxylic acid 4-chloro-benzylamide,
   5- (2-Chloro-4,5-difluoro-benzoylamino) -1H-pyrazole-3-carboxylic acid 4-ethoxycarbonyl-benzylamide,
   5- (2-Chloro-4,5-difluoro-benzoylamino) -1H-pyrazole-3-carboxylic acid 4-carboxy-benzylamide,
   5-(2-Chloro-4,5-difluoro-benzoylamino)-1H-pyrazole-3-carboxylic acid dibenzylamide,
   5-(2-Chloro-4,5-difluoro-benzoylamino)-1H-pyrazole-3-carboxylic acid (cyano-phenyl-methyl)-amide,
   5-(2-Chloro-4,5-difluoro-benzoylamino)-1H-pyrazole-3-carboxylic acid cyclohexylmethyl-amide,
   5-(2-Chloro-4,5-difluoro-benzoylamino)-1H-pyrazole-3-carboxylic acid (2-hydroxy-ethyl)-phenyl-amide,
   5-(2-Chloro-4,5-difluoro-benzoylamino)-1H-pyrazole-3-carboxylic acid cyclohexyl-methyl-amide,
   5-(2-Chloro-4,5-difluoro-benzoylamino)-1H-pyrazole-3-carboxylic acid cyclohexylamide,
   5-(2-Chloro-4,5-difluoro-benzoylamino)-1H-pyrazole-3-carboxylic acid cyclohexyl-ethyl-amide,
   5-(2-Chloro-4,5-difluoro-benzoylamino)-1H-pyrazole-3-carboxylic acid allyl-cyclohexyl-amide,
   5-(2-Chloro-4,5-difluoro-benzoylamino)-1H-pyrazole-3-carboxylic acid cyclohexyl-(pyridin-2-ylmethyl)-amide,
   5-(2-Chloro-4,5-difluoro-benzoylamino)-1H-pyrazole-3-carboxylic acid methyl-(1-methyl-piperidin-4-yl)-amide,
   5-(2-Chloro-4,5-difluoro-benzoylamino)-1H-pyrazole-3-carboxylic acid [(3,4-methylenedioxyphenyl)-methyl]-amide hydrochloride,
   5-(2-Chloro-4,5-difluoro-benzoylamino)-1H-pyrazole-3-carboxylic acid benzyl-butyl-amide,
   5-(2-Chloro-4,5-difluoro-benzoylamino)-1H-pyrazole-3-carboxylic acid benzyl-(4-hydroxy-butyl)-amide hydrochloride,
   5-(2-Chloro-4,5-difluoro-benzoylamino)-1H-pyrazole-3-carboxylic acid butyl-ethyl-amide,
   5-(2-Chloro-4,5-difluoro-benzoylamino)-1H-pyrazole-3-carboxylic acid cyclohexyl-propyl-amide,
   5-(2-Chloro-4,5-difluoro-benzoylamino)-1H-pyrazole-3-carboxylic acid butyl-cyclohexyl-amide,
   5-(2-Chloro-4,5-difluoro-benzoylamino)-1H-pyrazole-3-carboxylic acid cyclohexyl-(2-methoxy-ethyl)-amide,
   5-(2-Chloro-4,5-difluoro-benzoylamino)-1H-pyrazole-3-carboxylic acid cyclohexyl-(pyridin-3-ylmethyl)-amide,
   5-(2-Chloro-4,5-difluoro-benzoylamino)-1H-pyrazole-3-carboxylic acid [2-(morpholin-4-yl)-phenyl]-amide,
   5-(2-Chloro-4,5-difluoro-benzoylamino)-1H-pyrazole-3-carboxylic acid dibutylamide,
   5-(2-Chloro-4,5-difluoro-benzoylamino)-1H-pyrazole-3-carboxylic acid bis-(2-methoxy-ethyl)-amide,
   5-(2-Chloro-4,5-difluoro-benzoylamino)-1H-pyrazole-3-carboxylic acid (2-methoxy-ethyl)-propyl-amide,
   5-(2-Chloro-4,5-difluoro-benzoylamino)-1H-pyrazole-3-carboxylic acid butyl-(tetrahydro-pyran-4-yl)-amide,
   5-(2-Chloro-4,5-difluoro-benzoylamino)-1H-pyrazole-3-carboxylic acid (2-methoxy-ethyl)-(tetrahydro-pyran-4-yl)-amide,
   5-(2-Chloro-4,5-difluoro-benzoylamino)-1H-pyrazole-3-carboxylic acid cyclopentyl-(2-methoxy-ethyl)-amide,
   5-(2-Chloro-4,5-difluoro-benzoylamino)-1H-pyrazole-3-carboxylic acid cyclohexyl-(3-methoxy-propyl)-amide,
   5- (2-Chloro-4,5-difluoro-benzoylamino) -1H-pyrazole-3-carboxylic acid cyclohexyl-(2-ethoxy-ethyl)-amide,
   5-(2-Chloro-4,5-difluoro-benzoylamino)-1H-pyrazole-3-carboxylic acid cyclohexyl-(2-isopropoxy-ethyl)-amide,
   5-(2-Chloro-4,5-difluoro-benzoylamino)-1H-pyrazole-3-carboxylic acid cyclohexyl-(2-propoxy-ethyl)-amide,
   5-(2-Chloro-4,5-difluoro-benzoylamino)-1H-pyrazole-3-carboxylic acid (1-ethyl-propyl)-(2-methoxy-ethyl)-amide,
   5-(2-Chloro-4,5-difluoro-benzoylamino)-1H-pyrazole-3-carboxylic acid butyl-(1-tert-butoxycarbonyl-piperidin-4-yl)-amide,
   5-(2-Chloro-4,5-difluoro-benzoylamino)-1H-pyrazole-3-carboxylic acid butyl-(tetrahydro-thiopyran-4-yl)-amide,
   5-(2-Chloro-4,5-difluoro-benzoylamino)-1H-pyrazole-3-carboxylic acid (2-methoxy-ethyl)-amide,
   5-(2-Chloro-4,5-difluoro-benzoylamino)-1H-pyrazole-3-carboxylic acid butyl-(pyridin-3-ylmethyl)-amide,
   5-(2-Chloro-4,5-difluoro-benzoylamino)-1H-pyrazole-3-carboxylic acid benzyl-(2-methoxy-ethyl)-amide,
   5-(2-Chloro-4,5-difluoro-benzoylamino)-1H-pyrazole-3-carboxylic acid butyl-(piperidin-4-yl)-amide,
   5-(2-Chloro-4,5-difluoro-benzoylamino)-1H-pyrazole-3-carboxylic acid (1-acetyl-piperidin-4-yl)-butyl-amide,
   5-(2-Chloro-4,5-difluoro-benzoylamino)-1H-pyrazole-3-carboxylic acid butyl-(1-methanesulfonyl-piperidin-4-yl)-amide,
   5-(2-Chloro-4,5-difluoro-benzoylamino)-1H-pyrazole-3-carboxylic acid butyl-(1-ethoxalyl-piperidin-4-yl)-amide,
   5-(2-Chloro-4,5-difluoro-benzoylamino)-1H-pyrazole-3-carboxylic acid butyl-(1-oxalo-piperidin-4-yl)-amide,
   5-(2-Chloro-4,5-difluoro-benzoylamino)-1H-pyrazole-3-carboxylic acid butyl-(1,1-dioxo-hexahydro-1λ⁶ -thiopyran-4-yl)-amide,
   5-(2-Chloro-4,5-difluoro-benzoylamino)-1H-pyrazole-3-carboxylic acid butyl-(1-oxo-hexahydro-1λ⁴-thiopyran-4-yl)-amide,
   5-(2-Chloro-4,5-difluoro-benzoylamino)-1H-pyrazole-3-carboxylic acid (6-methoxy-pyridin-3-ylmethyl)-propyl-amide,
   5-(2-Chloro-4,5-difluoro-benzoylamino)-1H-pyrazole-3-carboxylic acid propyl-(pyridin-3-ylmethyl)-amide,
   5-(2-Chloro-4,5-difluoro-benzoylamino)-1H-pyrazole-3-carboxylic acid 4-diethylcarbamoyl-benzylamide,
   5-(2-Chloro-4,5-difluoro-benzoylamino)-1H-pyrazole-3-carboxylic acid 3-diethylcarbamoyl-benzylamide,
   5-(2-Chloro-4,5-difluoro-benzoylamino)-1H-pyrazole-3-carboxylic acid 4-ethylcarbamoyl-benzylamide,
   5-(2-Chloro-4,5-difluoro-benzoylamino)-1H-pyrazole-3-carboxylic acid 3-ethylcarbamoyl-benzylamide,
   5-(2-Chloro-4,5-difluoro-benzoylamino)-1H-pyrazole-3-carboxylic acid 4-carbamoyl-benzylamide,
   5- (2-Chloro-4,5-difluoro-benzoylamino) -1H-pyrazole-3-carboxylic acid (1-oxalo-piperidin-4-yl)-propyl-amide,
   5-(2-Chloro-4,5-difluoro-benzoylamino)-1H-pyrazole-3-carboxylic acid (1-carboxyacetyl-piperidin-4-yl)-propyl-amide,
   5-(2-Chloro-4,5-difluoro-benzoylamino)-1H-pyrazole-3-carboxylic acid [1-(2-carboxypropionyl)-piperidin-4-yl]-propyl-amide,
   5-(2-Chloro-4,5-difluoro-benzoylamino)-1H-pyrazole-3-carboxylic acid cyclopropyl-(6-methoxy-pyridin-3-ylmethyl)-amide
   5-(2-Chloro-4,5-difluoro-benzoylamino)-1H-pyrazole-3-carboxylic acid cyclobutyl-(6-methoxy-pyridin-3-ylmethyl)-amide,
   5-(2-Chloro-4,5-difluoro-benzoylamino)-1H-pyrazole-3-carboxylic acid cyclopropylmethyl-(6-methoxy-pyridin-3-ylmethyl)-amide
   5-(2-Chloro-4,5-difluoro-benzoylamino)-1H-pyrazole-3-carboxylic acid (6-dimethylamino-pyridin-3-ylmethyl)-amide,
   5-(2-Chloro-4,5-difluoro-benzoylamino)-1H-pyrazole-3-carboxylic acid (6-dimethylamino-pyridin-3-ylmethyl)-propyl-amide,
   5-(2-Chloro-4,5-difluoro-benzoylamino)-1H-pyrazole-3-carboxylic acid benzyl-(2-carboxy-ethyl)-amide,
   5-(2-Chloro-4,5-difluoro-benzoylamino)-1H-pyrazole-3-carboxylic acid benzyl-(2-ethoxycarbonyl-ethyl)-amide,
   5-(2-Chloro-4,5-difluoro-benzoylamino)-1H-pyrazole-3-carboxylic acid (6-chloro-pyridin-3-ylmethyl)-propyl-amide,
   5-(2-Chloro-4,5-difluoro-benzoylamino)-1H-pyrazole-3-carboxylic acid 2,6-dichloro-benzylamide,
   5-(2-Chloro-4,5-difluoro-benzoylamino)-1H-pyrazole-3-carboxylic acid (3,5-dichloro-pyridin-4-ylmethyl)-amide,
   5-(2-Chloro-4,5-difluoro-benzoylamino)-1H-pyrazole-3-carboxylic acid (3,5-dichloro-pyridin-4-ylmethyl)-propyl-amide,
   5-(2-Chloro-4,5-difluoro-benzoylamino)-1H-pyrazole-3-carboxylic acid [6-(1H-pyrazol-1-yl)-pyridin-3-ylmethyl]-amide dihydrochloride,
   5- (2-Chloro-4,5-difluoro-benzoylamino) -1H-pyrazole-3-carboxylic acid [6-(4-hydroxy-piperidin-1-yl)-pyridin-3-ylmethyl]-amide dihydrochloride,
   5- (2-Chloro-4,5-difluoro-benzoylamino) -1H-pyrazole-3-carboxylic acid (2-pyridin-2-yl-ethyl)-amide,
   5- (2-Chloro-4,5-difluoro-benzoylamino) -1H-pyrazole-3-carboxylic acid (2-pyridin-3-yl-ethyl)-amide,
   5-(2-Chloro-4,5-difluoro-benzoylamino)-1H-pyrazole-3-carboxylic acid (2-pyridin-4-yl-ethyl)-amide,
   5-(2-Chloro-4,5-difluoro-benzoylamino)-1H-pyrazole-3-carboxylic acid (4-[1,2,3]thiadiazol-4-yl-benzyl)-amide,
   5-(2-Chloro-4,5-difluoro-benzoylamino)-1H-pyrazole-3-carboxylic acid (5-methyl-pyrazin-2-ylmethyl)-amide,
   5-(2-Chloro-4,5-difluoro-benzoylamino)-1H-pyrazole-3-carboxylic acid pyrazin-2-ylamide,
   5-(2-Chloro-4,5-difluoro-benzoylamino)-1H-pyrazole-3-carboxylic acid quinolin-5-ylamide,
   5-(2-Chloro-4,5-difluoro-benzoylamino)-1H-pyrazole-3-carboxylic acid quinolin-8-ylamide,
   5-(2-Chloro-4,5-difluoro-benzoylamino)-1H-pyrazole-3-carboxylic acid isoquinolin-5-ylamide,
   5-(2-Chloro-4,5-difluoro-benzoylamino)-1H-pyrazole-3-carboxylic acid [3-ethoxy-5-(1-ethoxycarbonyl-1-methyl-ethyl)-pyridin-2-yl]-amide,
   5-(2-Chloro-4,5-difluoro-benzoylamino)-1H-pyrazole-3-carboxylic acid [3-ethoxy-5-(1-ethoxycarbonyl-1-hydroxy-ethyl)-pyridin-2-yl]-amide,
   5-(2-Chloro-4,5-difluoro-benzoylamino)-1H-pyrazole-3-carboxylic acid
   [5-(1-carboxy-1-methyl-ethyl)-3-ethoxy-pyridin-2-yl]-amide,
   5-(2-Chloro-4,5-difluoro-benzoylamino)-1H-pyrazole-3-carboxylic acid
   [5-(1-carboxy-1-hydroxy-ethyl)-3-ethoxy-pyridin-2-yl]-amide,
   5-(2-Chloro-4,5-difluoro-benzoylamino)-1H-pyrazole-3-carboxylic acid (pyrazin-2-ylmethyl)-amide,
   5-(2-Chloro-4,5-difluoro-benzoylamino)-1H-pyrazole-3-carboxylic acid [6-(thiomorpholin-4-yl)-pyridin-3-ylmethyl]-amide,
   5-(2-Chloro-4,5-difluoro-benzoylamino)-1H-pyrazole-3-carboxylic acid [6-(1,1-dioxo-1λ⁶-thiomorpholin-4-yl)-pyridin-3-ylmethy 1]-amide,
   5-(2-Chloro-4,5-difluoro-benzoylamino)-1H-pyrazole-3-carboxylic acid (4,5-dibromo-thiophen-2-ylmethyl)-amide,
   5-(2-Chloro-4,5-difluoro-benzoylamino)-1H-pyrazole-3-carboxylic acid (5-chloro-thiophen-2-ylmethyl)-amide,
   5-(2-Chloro-4,5-difluoro-benzoylamino)-1H-pyrazole-3-carboxylic acid (4-e thyl-2-methyl-oxazol-5-ylmethyl)-amide,
   5-(2-Chloro-4,5-difluoro-benzoylamino)-1H-pyrazole-3-carboxylic acid (2-e thyl-4-methyl-oxazol-5-ylmethyl)-amide dihydrochloride,
   5-(2-Chloro-4,5-difluoro-benzoylamino)-1H-pyrazole-3-carboxylic acid ([2,2']bithiophenyl-5-ylmethyl)-amide,
   5-(2-Chloro-4,5-difluoro-benzoylamino)-1H-pyrazole-3-carboxylic acid (3-methoxy-thiophen-2-ylmethyl)-amide,
   5-(2-Chloro-4,5-difluoro-benzoylamino)-1H-pyrazole-3-carboxylic acid (4,5-Dichloro-thiophen-2-ylmethyl)-amide,
   5-(2-Chloro-4,5-difluoro-benzoylamino)-1H-pyrazole-3-carboxylic acid (2,5-dimethyl-oxazol-4-ylmethyl)-amide,
   5-(2-Chloro-4,5-difluoro-benzoylamino)-1H-pyrazole-3-carboxylic acid (2-hexyl-4-methyl-oxazol-5-ylmethyl)-amide,
   5-(2-Chloro-4,5-difluoro-benzoylamino)-1H-pyrazole-3-carboxylic acid (4-methoxy-thiophen-3-ylmethyl)-amide,
   5-(2-Chloro-4,5-difluoro-benzoylamino)-1H-pyrazole-3-carboxylic acid (4-methyl-2-phenyl-oxazol-5-ylmethyl)-amide,
   5-(2-Chloro-4,5-difluoro-benzoylamino)-1H-pyrazole-3-carboxylic acid (2-methyl-4-phenyl-oxazol-5-ylmethyl)-amide,
   5-(2-Chloro-4,5-difluoro-benzoylamino)-1H-pyrazole-3-carboxylic acid (4-hexyl-2-methyl-oxazol-5-ylmethyl)-amide dihydrochloride,
   5-(2-Chloro-4,5-difluoro-benzoylamino)-1H-pyrazole-3-carboxylic acid (6-chloro-pyridin-3-ylmethyl)-amide,
   5-(2-Chloro-4,5-difluoro-benzoylamino)-1H-pyrazole-3-carboxylic acid (6-chloro-pyridin-3-ylmethyl)-amide dihydrochloride,
   5-(2-Chloro-4,5-difluoro-benzoylamino)-1H-pyrazole-3-carboxylic acid (4H-[1,2,4]triazol-3-ylmethyl)-amide,
   5-(2-Chloro-4,5-difluoro-benzoylamino)-1H-pyrazole-3-carboxylic acid (4H-[1,2,4]triazol-3-ylmethyl)-amide dihydrochloride,
   5-(2-Chloro-4,5-difluoro-benzoylamino)-1H-pyrazole-3-carboxylic acid (5-fluoro-4H-quinazoline-3-yl) -amide,
   5-(2-Chloro-4,5-difluoro-benzoylamino)-1H-pyrazole-3-carboxylic acid (5-fluoro-4H-quinazoline-3-yl)-amide dihydrochloride,
   5-(2-Chloro-4,5-difluoro-benzoylamino)-1H-pyrazole-3-carboxylic acid (4,6-dimethyl-pyridin-3-ylmethyl)-amide,
   5-(2-Chloro-4,5-difluoro-benzoylamino)-1H-pyrazole-3-carboxylic acid (4,6-dimethyl-pyridin-3-ylmethyl)-amide dihydrochloride,
   5-(2-Chloro-4,5-difluoro-benzoylamino)-1H-pyrazole-3-carboxylic acid (2-methoxy-6-methyl-pyridin-3-ylmethyl)-amide,
   5-(2-Chloro-4,5-difluoro-benzoylamino)-1H-pyrazole-3-carboxylic acid (4-methoxy-6-methyl-pyridin-3-ylmethyl)-amide,
   5-(2-Chloro-4,5-difluoro-benzoylamino)-1H-pyrazole-3-carboxylic acid (4-methoxy-6-methyl-pyridin-3-ylmethyl)-amide dihydrochloride,
   5-(2-Chloro-4,5-difluoro-benzoylamino)-1H-pyrazole-3-carboxylic acid (2-methoxy-4,6-dimethyl-pyridin-3-ylmethyl)-amide,
   5-(2-Chloro-4,5-difluoro-benzoylamino)-1H-pyrazole-3-carboxylic acid (2-methoxy-4,6-dimethyl-pyridin-3-ylmethyl)-amide dihydrochloride,
   5-(2-Chloro-4,5-difluoro-benzoylamino)-1H-pyrazole-3-carboxylic acid (3-methyl-pyridin-2-ylmethyl)-amide,
   5-(2-Chloro-4,5-difluoro-benzoylamino)-1H-pyrazole-3-carboxylic acid.(5-methyl-pyridin-3-ylmethyl)-amide,
   5-(2-Chloro-4,5-difluoro-benzoylamino)-1H-pyrazole-3-carboxylic acid (6-methyl-pyridin-3-ylmethyl)-amide,
   5-(2-Chloro-4,5-difluoro-benzoylamino)-1H-pyrazole-3-carboxylic acid (6-methyl-pyridin-2-ylmethyl)-amide,
   5-(2,4-Dichloro-5-fluoro-benzoylamino)-1H-pyrazole-3-carboxylic acid (pyridin-3-ylmethyl)-amide dihydrochloride,
   5-(2-Chloro-4,5-difluoro-benzoylamino)-1H-pyrazole-3-carboxylic acid (6-fluoro-pyridin-3-ylmethyl)-amide dihydrochloride,
   5-(2-Chloro-4,5-difluoro-benzoylamino)-1H-pyrazole-3-carboxylic acid (3-carboxy-phenyl)-methyl-amide,
   5-(2-Chloro-4,5-difluoro-benzoylamino)-1H-pyrazole-3-carboxylic acid 3-methanesulfonylamino-benzylamide,
   5-(2-Chloro-4,5-difluoro-benzoylamino)-1H-pyrazole-3-carboxylic acid 4-methanesulfonylamino-benzylamide,
   5-(2-Chloro-4,5-difluoro-benzoylamino)-1H-pyrazole-3-carboxylic acid 3-acetylamino-benzylamide,
   5-(2-Chloro-4,5-difluoro-benzoylamino)-1H-pyrazole-3-carboxylic acid 4-acetylamino-benzylamide,
   5-(2-Chloro-4,5-difluoro-benzoylamino)-1H-pyrazole-3-carboxylic acid (2-phenyl-thiazol-4-ylmethyl)-amide,
   5- (2-Chloro-4,5-difluoro-benzoylamino) -1H-pyrazole-3-carboxylic acid ((R)-1-phenyl-ethyl)-amide,
   5-(2-Chloro-4,5-difluoro-benzoylamino)-1H-pyrazole-3-carboxylic acid ((S)-1-phenyl-ethyl)-amide,
   5-(2-Chloro-4,5-difluoro-benzoylamino)-1H-pyrazole-3-carboxylic acid (6-phenoxy-pyridin-3-ylmethyl)-amide dihydrochloride,
   5-(2-Methyl-4,5-difluoro-benzoylamino)-1H-pyrazole-3-carboxylic acid (6-chloro-pyridin-3-ylmethyl)-amide dihydrochloride,
   5-(2-Chloro-4,5-difluoro-benzoylamino)-1H-pyrazole-3-carboxylic acid (2-chloro-pyridin-3-ylmethyl)-amide dihydrochloride,
   5-(2-Methyl-4,5-difluoro-benzoylamino)-1H-pyrazole-3-carboxylic acid (6-chloro-pyridin-3-ylmethyl)-amide sodium salt,
   5-(2-Chloro-4,5-difluoro-benzoylamino)-1H-pyrazole-3-carboxylic acid [1-(pyridin-3-yl)-ethyl]-amide dihydrochloride ,
   5-(2-Chloro-4,5-difluoro-benzoylamino)-1H-pyrazole-3-carboxylic acid (2-fluoro-pyridin-3-ylmethyl)-amide dihydrochloride,
   5-(2-Chloro-4,5-difluoro-benzoylamino)-1H-pyrazole-3-carboxylic acid (2-methyl-pyridin-3-ylmethyl)-amide dihydrochloride,
   5-(2-Chloro-4,5-difluoro-benzoylamino)-1H-pyrazole-3-carboxylic acid (biphenyl-3-ylmethyl)-amide,
   5-(2-Chloro-4,5-difluoro-benzoylamino)-1H-pyrazole-3-carboxylic acid ((1R,2S)-2-hydroxy-indan-1-yl)-amide,
   5-(2-Chloro-4,5-difluoro-benzoylamino)-1H-pyrazole-3-carboxylic acid ((1S,2R)-2-hydroxy-indan-1-yl)-amide,
   5-(2-Chloro-4,5-difluoro-benzoylamino)-1H-pyrazole-3-carboxylic acid (6-phenyl-pyridin-3-ylmethyl)-amide dihydrochloride,
   5-(2-Chloro-4,5-difluoro-benzoylamino)-1H-pyrazole-3-carboxylic acid (6-methyl-2-pyridon-3-ylmethyl)-amide hydrochloride,
   5-(2-Chloro-4,5-difluoro-benzoylamino)-1H-pyrazole-3-carboxylic acid benzyl-methyl-amide,
   5-(2-Chloro-4,5-difluoro-benzoylamino)-1H-pyrazole-3-carboxylic acid benzylamide ,
   5-(2-Chloro-4,5-difluoro-benzoylamino)-1H-pyrazole-3-carboxylic acid phenylamide,
   5-(2-Chloro-4,5-difluoro-benzoylamino)-1H-pyrazole-3-carboxylic acid methyl-phenyl-amide,
   5-(2-Chloro-4,5-dif.luoro-benzoylamino)-1H-pyrazole-3-carboxylic acid (piperidin-1-yl).-amide,
   5-(2-Chloro-4,5-difluoro-benzoylamino)-1H-pyrazole-3-carboxylic acid (4-ethoxycarbonyl-piperidin-1-yl)-amide,
   5-(2-Chloro-4,5-difluoro-benzoylamino)-1H-pyrazole-3-carboxylic acid (4-methyl-piperazin-1-yl)-amide,
   5-(2-Chloro-4,5-difluoro-benzoylamino)-1H-pyrazole-3-carboxylic acid (4-carboxy-piperidin-1-yl)-amide,
   5-(2-Chloro-4,5-difluoro-benzoylamino)-1H-pyrazole-3-carboxylic acid phenethylamide,
   5-(2-Chloro-4,5-difluoro-benzoylamino)-1H-pyrazole-3-carboxylic acid methyl-phenethyl-amide,
   5-(2-Chloro-4,5-difluoro-benzoylamino)-1H-pyrazole-3-carboxylic acid ethyl-phenyl-amide,
   5-(2-Chloro-4,5-difluoro-benzoylamino)-1H-pyrazole-3-carboxylic acid (1,2,3,4-tetrahydroisoquinolin-2-yl)-amide,
   5-(2-Chloro-4,5-difluoro-benzoylamino)-1H-pyrazole-3-carboxylic acid ((S)-α-methoxycarbonyl-benzyl)-amide,
   5-(2-Chloro-4,5-difluoro-benzoylamino)-1H-pyrazole-3-carboxylic acid ((R)-a-methoxycarbonyl-benzyl)-amide,
   5-(2-Chloro-4,5-difluoro-benzoylamino)-1H-pyrazole-3-carboxylic acid (1,2,3,4-tetrahydroquinoline-1-yl)-amide,
   5-(2-Chloro-4,5-difluoro-benzoylamino)-1H-pyrazole-3-carboxylic acid phenyl-propyl-amide,
   5-(2-Chloro-4,5-difluoro-benzoylamino)-1H-pyrazole-3-carboxylic acid butyl-phenyl-amide,
   5-(2-Chloro-4,5-difluoro-benzoylamino)-1H-pyrazole-3-carboxylic acid pentyl-phenyl-amide,
   5-(2-Chloro-4,5-difluoro-benzoylamino)-1H-pyrazole-3-carboxylic acid benzhydryl-amide,
   5-(2-Chloro-4,5-difluoro-benzoylamino)-1H-pyrazole-3-carboxylic acid (pyridin-2-ylmethyl) -amide hydrochloride,
   5-(2-Chloro-4,5-difluoro-benzoylamino)-1H-pyrazole-3-carboxylic acid (pyridin-4-ylmethyl)-amide hydrochloride,
   5-(2-Chloro-4,5-difluoro-benzoylamino)-1H-pyrazole-3-carboxylic acid ((R)-2-hydroxy-1-phenyl-ethyl)-amide,
   5-(2-Chloro-4,5-difluoro-benzoylamino)-1H-pyrazole-3-carboxylic acid ((S)-2-hydroxy-1-phenyl-ethyl)-amide,
   5-(2-Chloro-4,5-difluoro-benzoylamino)-1H-pyrazole-3-carboxylic acid 4-dimethylamino-benzylamide,
   5-(2-Chloro-4,5-difluoro-benzoylamino)-1H-pyrazole-3-carboxylic acid (furan-2-ylmethyl)-amide,
   5-(2-Chloro-4,5-difluoro-benzoylamino)-1H-pyrazole-3-carboxylic acid (thiophen-2-ylmethyl)-amide,
   5-(2-Chloro-4,5-difluoro-benzoylamino)-1H-pyrazole-3-carboxylic acid (2-methoxy-phenyl)-amide,
   5-(2-Chloro-4,5-difluoro-benzoylamino)-1H-pyrazole-3-carboxylic acid (2-methoxy-phenyl)-methyl-amide,
   5-(2-Chloro-4,5-difluoro-benzoylamino)-1H-pyrazole-3-carboxylic acid 4-dimethylamino-benzylamide hydrochloride,
   5- (2-Chloro-4,5-difluoro-benzoylamino) -1H-pyrazole-3-carboxylic acid 3-amino-benzylamide,
   5-(2-Chloro-4,5-difluoro-benzoylamino)-1H-pyrazole-3-carboxylic acid 4-amino-benzylamide,
   5-(2-Chloro-4,5-difluoro-benzoylamino)-1H-pyrazole-3-carboxylic acid 2-methyl-benzylamide,
   5-(2-Chloro-4,5-difluoro-benzoylamino)-1H-pyrazole-3-carboxylic acid 3-methyl-benzylamide,
   5-(2-Chloro-4,5-difluoro-benzoylamino)-1H-pyrazole-3-carboxylic acid 3-dimethylamino-benzylamide,
   5-(2-Chloro-4,5-difluoro-benzoylamino)-1H-pyrazole-3-carboxylic acid (2-methoxy-pyridin-3-ylmethyl)-amide,
   5-(2-Chloro-4,5-difluoro-benzoylamino)-1H-pyrazole-3-carboxylic acid 2-chloro-benzylamide,
   5- (2-Chloro-4,5-difluoro-benzoylamino) -1H-pyrazole-3-carboxylic acid 3-chloro-benzylamide,
   5-(2-Chloro-4,5-difluoro-benzoylamino)-1H-pyrazole-3-carboxylic acid 3-methoxycarbonyl-benzylamide,
   5-(2-Chloro-4,5-difluoro-benzoylamino)-1H-pyrazole-3-carboxylic acid 3-carboxy-benzylamide,
   5-(2-Chloro-4,5-difluoro-benzoylamino)-1H-pyrazole-3-carboxylic acid (6-trifluoromethyl-pyridin-3-ylmethyl)-amide,
   5-(2-Chloro-4,5-difluoro-benzoylamino)-1H-pyrazole-3-carboxylic acid (2-ethoxy-pyridin-3-ylmethyl)-amide,
   5-(2-Chloro-4,5-difluoro-benzoylamino)-1H-pyrazole-3-carboxylic acid (2-dimethylamino-ethyl)-amide,
   5-(2-Chloro-4,5-difluoro-benzoylamino)-1H-pyrazole-3-carboxylic acid ethyl-(2-dimethylamino-ethyl)-amide dihydrochloride,
   5-(2-Chloro-4,5-difluoro-benzoylamino)-1H-pyrazole-3-carboxylic acid cyclohexyl-(2-dimethylamino-ethyl)-amide,
   5-(2-Chloro-4,5-difluoro-benzoylamino)-1H-pyrazole-3-carboxylic acid (4-methoxy-pyridin-2-ylmethyl)-amide,
   5-(2-Chloro-4,5-difluoro-benzoylamino)-1H-pyrazole-3-carboxylic acid (2,6-dimethoxy-pyridin-3-ylmethyl)-amide,
   5-(2-Chloro-4,5-difluoro-benzoylamino)-1H-pyrazole-3-carboxylic acid 2-methoxycarbonylmethoxy-benzylamide,
   5-(2-Chloro-4,5-difluoro-benzoylamino)-1H-pyrazole-3-carboxylic acid 2-carboxymethoy-benzylamide,
   5-(4,5-Difluoro-2-methyl-benzoylamino)-1H-pyrazole-3-carboxylic acid (6-methoxy-pyridin-3-ylmethyl)-amide,
   5-(2,4-Dichloro-benzoylamino)-1H-pyrazole-3-carboxylic acid (6-methoxy-pyridin-3-ylmethyl)-amide,
   5-(2-Chloro-4,5-difluoro-benzoylamino)-1H-pyrazole-3-carboxylic acid (3,5-dimethoxy-pyridin-4-ylmethyl)-amide,
   5-(4,5-Difluoro-2-methyl-benzoylamino)-1H-pyrazole-3-carboxylic acid (2,4-dimethyl-oxazol-5-ylmethyl)-amide,
   5- (2,4-Dichloro-5-fluoro-benzoylamino) -1H-pyrazole-3-carboxylic acid (2,4-dimethyl-oxazol-5-ylmethyl)-amide,
   5-(2-Chloro-4,5-difluorobenzoylamino)-1H-pyrazole-3-carbocylic acid (4-methoxycarbonyl-phenyl)-methyl-amide,
   5-(2-Chloro-4,5-difluoro-benzoylamino)-1H-pyrazole-3-carboxylic acid (4-carboxyphenyl)-methyl-amide, 4-{[5-(2-Chloro-4,5-difluoro-benzoylamino)-1H-pyrazole -3-carbonyl]-methyl-amino}-benzoic acid sodium salt,
   5-(2-Chloro-4,5-difluoro-benzoylamino)-1H-pyrazole-3-carboxylic acid isopropyl-phenyl-amide,
   5-(2-Chloro-4,5-difluoro-benzoylamino)-1H-pyrazole-3-carboxylic acid (ethoxycarbonyl-methyl)-phenyl-amide,
   5-(2-Chloro-4,5-difluoro-benzoylamino)-1H-pyrazole-3-carboxylic acid (carboxy-methyl)-phenyl-amide,
   5-(2-Chloro-4-fluoro-benzoylamino)-1H-pyrazole-3-carboxylic acid benzylamide,
   5-(2,4-Dichloro-benzoylamino)-1H-pyrazole-3-carboxylic acid benzylamide,
   5-(2-Chloro-5-fluoro-benzoylamino)-1H-pyrazole-3-carboxylic acid benzylamide,
   5-(2-Chloro-4,5-difluoro-benzoylamino)-1H-pyrazole-3-carboxylic acid benzyl-phenyl-amide,
   5-(2-Chloro-4,5-difluoro-benzoylamino)-1H-pyrazole-3-carboxylic acid (1-methyl-1-phenyl-ethyl)-amide,
   5-(2-Chloro-4,5-difluoro-benzoylamino)-1H-pyrazole-3-carboxylic acid [2-oxo-2-(piperidin-1-yl)-ethyl]-phenyl-amide,
   5-(5-Fluoro-2-methyl-benzoylamino)-1H-pyrazole-3-carboxylic acid benzylamide,
   5-(5-Chloro-2-methyl-benzoylamino)-1H-pyrazole-3-carboxylic acid benzylamide,
   5-(4-Methoxy-2-methyl-benzoylamino)-1H-pyrazole-3-carboxylic acid benzylamide,
   5-(4-Chloro-2-methyl-benzoylamino)-1H-pyrazole-3-carboxylic acid benzylamide,
   5- (5-Fluoro-2-methoxy-benzoylamino) -1H-pyrazole-3-carboxylic acid benzylamide,
   5-(5-Chloro-2-methoxy-benzoylamino)-1H-pyrazole-3-carboxylic acid benzylamide,
   5-(2-Chloro-4,5-difluoro-benzoylamino)-1H-pyrazole-3-carboxylic acid (2-ethoxy-ethyl)-phenyl-amide,
   5-(2-Fluoro-5-methyl-benzoylamino)-1H-pyrazole-3-carboxylic acid benzylamide,
   5-(5-Chloro-2-fluoro-benzoylamino)-1H-pyrazole-3-carboxylic acid benzylamide,
   5-(3-Chloro-2,6-difluoro-benzoylamino)-1H-pyrazole-3-carboxylic acid benzylamide,
   5-(2-Chloro-3-fluoro-benzoylamino)-1H-pyrazole-3-carboxylic acid benzylamide,
   5-(2-Chloro-4,5-difluoro-benzoylamino)-1H-pyrazole-3-carboxylic acid ethylcarbamoylmethyl-phenyl-amide,
   5-(2-Chloro-4,5-difluoro-benzoylamino)-1H-pyrazole-3-carboxylic acid isobutyl-phenyl-amide,
   5-(2-Chloro-4,5-difluoro-benzoylamino)-1H-pyrazole-3-carboxylic acid (2-methoxycarbonyl-ethyl)-phenyl-amide,
   5-(2-Chloro-4,5-difluoro-benzoylamino)-1H-pyrazole-3-carboxylic acid (3-ethoxycarbonyl-propyl)-phenyl-amide,
   5- (2-Chloro-4,5-difluoro-benzoylamino) -1H-pyrazole-3-carboxylic acid (2-carboxy-ethyl)-phenyl-amide,
   5-(2-Chloro-4,5-difluoro-benzoylamino)-1H-pyrazole-3-carboxylic acid (3-carboxy-propyl)-phenyl-amide,
   5-(2-Hydroxy-4-methoxy-benzoylamino)-1H-pyrazole-3-carboxylic acid benzylamide,
   5-(2-Hydroxy-5-methoxy-benzoylamino)-1H-pyrazole-3-carboxylic acid benzylamide,
   5-(5-Fluoro-2-hydroxy-benzoylamino)-1H-pyrazole-3-carboxylic acid benzylamide,
   5-(4-Fluoro-2-trifluoromethyl-benzoylamino)-1H-pyrazole-3-carboxylic acid benzylamide,
   5-(5-Fluoro-2-trifluoromethyl-benzoylamino)-1H-pyrazole-3-carboxylic acid benzylamide,
   5-(2-Chloro-4,5-difluoro-benzoylamino)-1H-pyrazole-3-carboxylic acid (2-methoxy-ethyl)-phenyl-amide,
   5-(2,5-Dimethyl-benzoylamino)-1H-pyrazole-3-carboxylic acid benzylamide,
   5-(2-Chloro-5-methyl-benzoylamino)-1H-pyrazole-3-carboxylic acid benzylamide,
   5-(2-Chloro-pyridine-3-carbonylamino)-1H-pyrazole-3-carboxylic acid benzylamide,
   5-(4-Chloro-pyridine-2-carbonylamino)-1H-pyrazole-3-carboxylic acid benzylamide,
   5-(2,6-Dichloro-pyridine-3-carbonylamino)-1H-pyrazole-3-carboxylic acid benzylamide,
   5-(2-Chloro-4,5-difluoro-benzoylamino)-1H-pyrazole-3-carboxylic acid butyl-(4-ethoxycarbonyl-phenyl)-amide,
   5-(2-Chloro-4,5-difluoro-benzoylamino)-1H-pyrazole-3-carboxylic acid butyl-(4-carboxy-phenyl)-amide,
   5-(2-Methyl-pyridine-3-carbonylamino)-1H-pyrazole-3-carboxylic acid benzylamide,
   5-(2-Chloro-6-methyl-pyridine-3-carbonylamino)-1H-pyrazole-3-carboxylic acid benzylamide,
   5-(3-Chloro-pyridine-4-carbonylamino)-1H-pyrazole-3-carboxylic acid benzylamide,
   5-(2-Chloro-4,5-difluoro-benzoylamino)-1H-pyrazole-3-carboxylic acid butyl-(4-ethoxycarbonylmethyl-phenyl)-amide,
   5-(2-Chloro-4,5-difluoro-benzoylamino)-1H-pyrazole-3-carboxylic acid butyl-(4-carboxymethyl-phenyl)-amide,
   5-(2-Chloro-4,5-difluoro-benzoylamino)-1H-pyrazole-3-carboxylic acid (4-methoxycarbonyl-phenyl)-(2-methoxy-ethyl)-amide,
   5-(2-Chloro-4,5-difluoro-benzoylamino)-1H-pyrazole-3-carboxylic acid (4-carboxy-phenyl)-(2-methoxy-ethyl)-amide,
   5-(2-Chloro-4,5-difluoro-benzoylamino)-1H-pyrazole-3-carboxylic acid (6-methoxy-pyridin-3-ylmethyl)-amide dihydrochloride,
   5-(2-Methyl-4,5-difluoro-benzoylamino)-1H-pyrazole-3-carboxylic acid (6-methoxy-pyridin-3-ylmethyl)-amide dihydrochloride,
   5-(2-Chloro-4,5-difluoro-benzoylamino)-1H-pyrazole-3-carboxylic acid (thiazol-4-ylmethyl)-amide dihydrochloride,
   5-(2-Chloro-4,5-difluoro-benzoylamino)-1H-pyrazole-3-carboxylic acid 4-[2-(morpholin-4-yl)-ethylcarbamoyl]-benzylamide dihydrochloride,
   5-(2-Chloro-4,5-difluoro-benzoylamino)-1H-pyrazole-3-carboxylic acid 4-(morpholin-4-ylcarbamoyl)-benzylamide dihydrochloride, [4-({[5-(2-Chloro-4,5-difluoro-benzoylamino)-1H-pyrazole-3-carbonyl]-amino}-methyl)-benzoylamino]-acetic acid sodium salt, 3-[4-({[5-(2-Chloro-4,5-difluoro-benzoylamino)-1H-pyrazole-3-carbonyl]-amino}-methyl)-benzoylamino]-propionic acid sodium salt,
   5-(2-Chloro-4,5-difluoro-benzoylamino)-1H-pyrazole-3-carboxylic acid (3,5-dimethyl-isoxazol-4-ylmethyl)-amide hydrochloride,
   5-(2-Chloro-4,5-difluoro-benzoylamino)-1H-pyrazole-3-carboxylic acid (1,3,5-trimethyl-1H-pyrazol-4-ylmethyl)-amide dihydrochloride,
   5-(2=Chloro-4,5-difluoro-benzoylamino)-1H-pyrazole-3-carboxylic acid (3,5-dimethyl-isoxazol-4-ylmethyl)-amide methanesulfonate,
   5-(2-Chloro-4,5-difluoro-benzoylamino)-1H-pyrazole-3-carboxylic acid 3-[2-(morpholin-4-yl)-ethylcarbamoyl]-benzylamide dihydrochloride,
   5-(2-Chloro-4,5-difluoro-benzoylamino)-1H-pyrazole-3-carboxylic acid 3-(morpholin-4-ylcarbamoyl)-benzylamide dihydrochloride,
   5-(2-Chloro-4,5-difluoro-benzoylamino)-1H-pyrazole-3-carboxylic acid {2-[2-(morpholin-4-yl)-ethylcarbamoyl]-pyridin-4-ylmethyl}-amide trihydrochloride,
   5-(2-Chloro-4,5-difluoro-benzoylamino)-1H-pyrazole-3-carboxylic acid [2-(morpholin-4-ylcarbamoyl)-pyridin-4-ylmethyl]-amide trihydrochloride,
   5-(2-Chloro-4,5-difluoro-benzoylamino)-1H-pyrazole-3-carboxylic acid (6-methoxy-pyridin-3-ylmethyl)-amide methanesulfonate,
   5- (2-Chloro-4,5-difluoro-benzoylamino) -1H-pyrazole-3-carboxylic acid (6-methoxy-pyridin-3-ylmethyl)-amide hemisulfate
   5-(2-Chloro-4,5-difluoro-benzoylamino)-1H-pyrazole-3-carboxylic acid (3,5-dimethoxy-pyridin-4-ylmethyl)-amide dihydrochloride,
   5-(2-Chloro-4,5-difluoro-benzoylamino)-1H-pyrazole-3-carboxylic acid (4-aminomethyl-pyridin-2-ylmethyl)-amide trihydrochloride,
   5-(2-Chloro-4,5-difluoro-benzoylamino)-1H-pyrazole-3-carboxylic acid (6-chloro-pyridin-3-ylmethyl)-amide methanesulfonate,
   5-(2-Chloro-4,5-difluoro-benzoylamino)-1H-pyrazole-3-carboxylic acid (6-chloro-pyridin-3-ylmethyl)-amide dimethanesulfonate,
   5-(2-Chloro-4,5-difluoro-benzoylamino)-1H-pyrazole-3-carboxylic acid (2,4-dimethyl-pyridin-3-ylmethyl)-amide hydrochloride,
   5-(2-Chloro-4,5-difluoro-benzoylamino)-1H-pyrazole-3-carboxylic acid (pyridin-3-ylmethyl)-amide hydrochloride,
   5-(2-Chloro-4,5-difluoro-benzoylamino)-1H-pyrazole-3-carboxylic acid (6-methoxy-pyridin-3-ylmethyl)-propyl-amide dihydrochloride,
   5-(2-Chloro-4,5-difluoro-benzoylamino)-1H-pyrazole-3-carboxylic acid [6-(piperidin-1-yl)-pyridin-3-ylmethyl]-amide dihydrochloride,
   5-(2-Chloro-4,5-difluoro-benzoylamino)-1H-pyrazole-3-carboxylic acid [6-(morpholin-4-yl)-pyridin-3-ylmethyl]-amide dihydrochloride,
   5-(2-Chloro-4,5-difluoro-benzoylamino)-1H-pyrazole-3-carboxylic acid (3,5-dichloro-pyridin-4-ylmethyl)-amide hydrochloride,
   5-(2-Chloro-4,5-difluoro-benzoylamino)-1H-pyrazole-3-carboxylic acid (6-dimethylamino-pyridin-3-ylmethyl)-amide dihydrochloride,
   5-(2-Chloro-4,5-difluoro-benzoylamino)-1H-pyrazole-3-carboxylic acid (6-dimethylamino-pyridin-3-ylmethyl)-propyl-amide dihydrochloride,
   5-(2-Chloro-4,5-difluoro-benzoylamino)-1H-pyrazole-3-carboxylic acid (6-diethylamino-pyridin-3-ylmethyl)-amide dihydrochloride,
   5-(2-Chloro-4,5-difluoro-benzoylamino)-1H-pyrazole-3-carboxylic acid (6-chloro-2-dimethylamino-pyridin-3-ylmethyl)-amide dihydrochloride,
   5-(2-Chloro-4,5-difluoro-benzoylamino)-1H-pyrazole-3-carboxylic acid (4-[1,2,3]thiadiazol-4-yl-benzyl)-amide hydrochloride,
   5-(2-Chloro-4,5-difluoro-benzoylamino)-1H-pyrazole-3-carboxylic acid (4-methyl-thiazol-5-ylmethyl)-amide hydrochloride,
   5-(2-Chloro-4,5-difluoro-benzoylamino)-1H-pyrazole-3-carboxylic acid (2,4-dimethyl-thiazol-5-ylmethyl)-amide hydrochloride,
   5-(2-Chloro-4,5-difluoro-benzoylamino)-1H-pyrazole-3-carboxylic acid [6-(4-hydroxy-piperidin-1-yl)-pyridin-3-ylmethyl]-amid e dihydrochloride,
   5-(2-Chloro-4,5-difluoro-benzoylamino)-1H-pyrazole-3-carboxylic acid 5-methyl-pyrazin-2-ylamide dihydrochloride,
   5- (2-Chloro-4,5-difluoro-benzoylamino) -1H-pyrazole-3-carboxylic acid quinolin-5-ylamide dihydrochloride,
   5-(2-Chloro-4,5-difluoro-benzoylamino)-1H-pyrazole-3-carboxylic acid quinolin-8-ylamide dihydrochloride,
   5-(2-Chloro-4,5-difluoro-benzoylamino)-1H-pyrazole-3-carboxylic acid isoquinolin-5-ylamide dihydrochloride,
   5-(2-Chloro-4,5-difluoro-benzoylamino)-1H-pyrazole-3-carboxylic acid [6-(4-methylpiperazin-1-yl)-pyridin-3-ylmethyl]-amide dihydrochloride,
   5-(2-Chloro-4,5-difluoro-benzoylamino)-1H-pyrazole-3-carboxylic acid [6-(4-dimethylamino-piperidin-1-yl)-pyridin-3-ylmethyl ]-amide dihydrochloride,
   5-(2-Chloro-4,5-difluoro-benzoylamino)-1H-pyrazole-3-carboxylic acid [6-(pyrrolidin-1-yl)-pyridin-3-ylmethyl] -amide dihydrochloride,
   5-(2-Chloro-4,5-difluoro-benzoylamino)-1H-pyrazole-3-carboxylic acid (pyrazin-2-ylmethyl)-amide dihydrochloride,
   5-(2-Chloro-4,5-difluoro-benzoylamino)-1H-pyrazole-3-carboxylic acid [6-(thiomorpholin-4-yl)-pyridin-3-ylmethyl]-amide dihydrochloride,
   5-(2-Chloro-4,5-difluoro-benzoylamino)-1H-pyrazole-3-carboxylic acid [6-(1,1-dioxo-116-thiomorpholin-4-yl)-pyridin-3-ylmethyl]-amide dihydrochloride,
   5-(2-Chloro-4,5-difluoro-benzoylamino)-1H-pyrazole-3-carboxylic acid carbamoylmethyl-amide,
   5- (2,4-Dichloro-5-fluoro-benzoylamino) -1H-pyrazole-3-carboxylic acid (4-carbamoyl-phenyl)-methyl-amide,
   5-(2,4-Dichloro-5-fluoro-benzoylamino)-1H-pyrazole-3-carboxylic acid methyl-(4-methylcarbamoyl-phenyl)-amide,
   5-(2,4-Dichloro-5-fluoro-benzoylamino)-1H-pyrazole-3-carboxylic acid (4-dimethylcarbamoyl-phenyl)-methyl-amide,
   5-(2-Chloro-4,5-difluoro-benzoylamino)-1H-pyrazole-3-carboxylic acid (4-methanesulfonylaminocarbonyl-phenyl)-methyl-amide,
   5-(2-Chloro-4,5-difluoro-benzoylamino)-1H-pyrazole-3-carboxylic acid (4-methanesulfonylaminocarbonyl-phenyl)-methyl-amide sodium salt,
   5-(2-Chloro-4,5-difluoro-benzoylamino)-1H-pyrazole-3-carboxylic acid (4-cyano-phenyl)-methyl-amide,
   5-(2-Chloro-4,5-difluoro-benzoylamino)-1H-pyrazole-3-carboxylic acid methyl- [4- (4H- [1, 2, 4] triazol-3-yl) -phenyl] -amide hydrochloride,
   5-(4-Azido-2-chloro-5-fluoro-benzoylamino)-1H-pyrazole-3-carboxylic acid (4-cyano-phenyl)-methyl-amide,
   5-(4-Azido-2-chloro-5-fluoro-benzoylamino)-1H-pyrazole -3-carboxylic acid methyl-[4-(1H-tetrazol-5-yl)-phenyl]-amide,
   5-(2-Chloro-4,5-difluoro-benzoylamino)-1H-pyrazole-3-carboxylic acid [4-(N-hydroxycarbamimidoyl)-phenyl]-methyl-amide,
   5-(2-Chloro-4,5-difluoro-benzoylamino)-1H-pyrazole-3-carboxylic acid methyl-[4-(5-oxo-2,5-dihydro-[1,2,4]oxadiazol-3-yl)-phenyl]-amide,
   5-(2-Chloro-4,5-difluoro-benzoylamino)-1H-pyrazole-3-carboxylic acid {4-[(2,2-dimethyl-propionyloxy)-methoxycarbonyl]-phenyl}-methyl-amide,
   5-(2-Chloro-4,5-difluoro-benzoylamino)-1H-pyrazole-3-carboxylic acid 4-[N-(methoxy-thiocarbonyloxy)-carbamimidoyl]-methyl-amide,
   5-(2-Chloro-4,5-difluoro-benzoylamino)-1H-pyrazole-3-carboxylic acid methyl-[4-(5-thioxo-4,5-dihydro-[1,2,4]oxadiazol-3-yl) -phenyl]-amide,
   5-(2-Chloro-4,5-difluoro-benzoylamino)-1H-pyrazole-3-carboxylic acid methyl-[4-(5-oxo-4,5-dihydro-[1,2,4]thiadiazol-3-yl)-phenyl]-amide,
   5-(2-Chloro-4,5-difluoro-benzoylamino)-1H-pyrazole-3-carboxylic acid {4-[l-(cyclohexyloxy-carbonyloxy)-ethoxycarbonyl]-phenyl}-methyl-amide,
   5-(2-Chloro-4,5-difluoro-benzoylamino)-1H-pyrazole-3-carboxylic acid (4-ethoxycarbonyl-thiazol-2-yl)-ethyl-amide,
   5-(2-Chloro-4,5-difluoro-benzoylamino)-1H-pyrazole-3-carboxylic acid 2-methanesulfonylamino-benzylamide,
   5-(2-Chloro-4,5-difluoro-benzoylamino)-1H-pyrazole-3-carboxylic acid 2-acetylamino-benzylamide,
   5-(2-Chloro-4,5-difluoro-benzoylamino)-1H-pyrazole-3-carboxylic acid 2-(dimethylamino-methyleneamino)-benzylamide hydrochloride,
   5-(2-Chloro-4,5-difluoro-benzoylamino)-1H-pyrazole-3-carboxylic acid 3-(dimethylamino-methyleneamino)-benzylamide hydrochloride,
   5-(2-Chloro-4,5-difluoro-benzoylamino)-1H-pyrazole-3-carboxylic acid 4-(dimethylamino-methyleneamino)-benzylamide hydrochloride,
   5-(2-Chloro-4,5-difluoro-benzoylamino)-1H-pyrazole-3-carboxylic acid 2-[1-(dimethylamino)ethylideneamino]-benzylamide dihydrochloride,
   5-(2-Chloro-4,5-difluoro-benzoylamino)-1H-pyrazole-3-carboxylic acid 3-[1-(dimethylamino)ethylideneamino]-benzylamide dihydrochloride,
   5-(2-Chloro-4,5-difluoro-benzoylamino)-1H-pyrazole-3-carboxylic acid 4-[1-(dimethylamino)ethylideneamino]-benzylamide dihydrochloride,
   5-(2-Chloro-4,5-difluoro-benzoylamino)-1H-pyrazole-3-carboxylic acid 2-(diethylamino-methyleneamino)-benzylamide dihydrochloride,
   5-(2-Chloro-4,5-difluoro-benzoylamino)-1H-pyrazole-3-carboxylic acid 3-(diethylamino-methyleneamino)-benzylamide dihydrochloride,
   5-(2-Chloro-4,5-difluoro-benzoylamino)-1H-pyrazole-3-carboxylic acid 4-(diethylamino-methyleneamino)-benzylamide dihydrochloride,
   5-(2-Chloro-4,5-difluoro-benzoylamino)-1H-pyrazole-3-carboxylic acid 2-acetimidoylamino-benzylamide dihydrochloride,
   5-(2-Chloro-4,5-difluoro-benzoylamino)-1H-pyrazole-3-carboxylic acid 3-acetimidoylamino-benzylamide dihydrochloride,
   5-(2-Chloro-4,5-difluoro-benzoylamino)-1H-pyrazole-3-carboxylic acid 4-acetimidoylamino-benzylamide dihydrochloride,
   5-(2-Chloro-4,5-difluoro-benzoylamino)-1H-pyrazole-3-carboxylic acid 4-cyano-benzylamide,
   5-(2-Chloro-4,5-difluoro-benzoylamino)-1H-pyrazole-3-carboxylic acid 3-[(2-fluoro-benzimidoyl)-amino]-benzylamide hyrdoiodide,
   5-(2-Chloro-4,5-difluoro-benzoylamino)-1H-pyrazole-3-carboxylic acid 4-[(2-fluoro-benzimidoyl)-amino]-benzylamide hyrdoiodide,
   5-(2-Chloro-4,5-difluoro-benzoylamino)-1H-pyrazole-3-carboxylic acid 3-(3,3-dimethyl-ureido)-benzylamide,
   5-(2-Chloro-4,5-difluoro-benzoylamino)-1H-pyrazole-3-carboxylic acid 4-(3,3-dimethyl-ureido)-benzylamide,
   5-(2-Chloro-4,5-difluoro-benzoylamino)-1H-pyrazole-3-carboxylic acid 2-(toluene-4-sulfonylamino)-benzylamide,
   5-(2-Chloro-4,5-difluoro-benzoylamino)-1H-pyrazole-3-carboxylic acid 2-amino-6-fluoro-benzylamide hydrochloride,
   5-(2-Chloro-4,5-difluoro-benzoylamino)-1H-pyrazole-3-carboxylic acid 2-amino-4,5-difluoro-benzylamide hydrochloride,
   5-(2-Chloro-4,5-difluoro-benzoylamino)-1H-pyrazole-3-carboxylic acid (indan-1-yl)-amide,
   5-(2-Chloro-4,5-difluoro-benzoylamino)-1H-pyrazole-3-carboxylic acid (indan-2-yl)-amide,
   5-(2-Chloro-4,5-difluoro-benzoylamino)-1H-pyrazole-3-carboxylic (4H-quinazoline-3-yl)-amide,
   5-(2-Chloro-4,5-difluoro-benzoylamino)-1H-pyrazole-3-carboxylic (4H-quinazoline-3-yl)-amide dihydrochloride,
   5-(2-Chloro-4,5-difluoro-benzoylamino)-1H-pyrazole-3-carboxylic acid (7-fluoro-4H-quinazoline-3-yl)-amide dihydrochloride,
   5-(2-Chloro-4,5-difluoro-benzoylamino)-1H-pyrazole-3-carboxylic acid (6-fluoro-4H-quinazoline-3-yl)-amide hydrochloride,
   5-(2-Chloro-4,5-difluoro-benzoylamino)-1H-pyrazole-3-carboxylic acid (2-ethoxy-2-methyl-1,4-dihydro-2H-quinazoline-3-yl)-amide,
   5-(2-Chloro-4,5-difluoro-benzoylamino)-1H-pyrazole-3-carboxylic acid (2-cyano-pyridin-4-ylmethyl)-amide,
   5-(2-Chloro-4,5-difluoro-benzoylamino)-1H-pyrazole-3-carboxylic acid [2-(N-hydroxycarbamimidoyl)-pyridin-4-ylmethyl]-amide,
   5-(2-Chloro-4,5-difluoro-benzoylamino)-1H-pyrazole-3-carboxylic acid [2-(5-oxo-4,5-dihydro-[1,2,4]oxadiazol-3-yl)-pyridin-4 -ylmethyl]-amide, 4-{[5-(4,5-difluoro-2-methyl-benzoylamino)-1H-pyrazole -3-carbonyl]-methyl-amino}-benzoic acid sodium salt,
   5-(4,5-Difluoro-2-methyl-benzoylamino)-1H-pyrazole-3-carboxylic acid methyl-[4-(1H-tetrazol-5-yl)-phenyl]-amide, 5-(2,4-Dichloro-benzoylamino)-1H-pyrazole-3-carboxylic acid (pyridin-3-ylmethyl)-amide,
   5-(2,4-Dichloro-5-fluoro-benzoylamino)-1H-pyrazole-3-carboxylic acid (pyridin-3-ylmethyl)-amide,
   5- (2,6-Dichloro-5-fluoro-pyridine-3-carbonylamino) -1H-pyrazole-3-carboxylic acid (pyridin-3-ylmethyl)-amide,
   5-[(3-Chloro-benzo[b]thiophene-2-carbonyl)-amino]-1H-p yrazole-3-carboxylic acid (pyridin-3-ylmethyl)-amide,
   5-[(3-Chloro-thiophene-2-carbonyl)-amino]-1H-pyrazole-3-carboxylic acid (pyridin-3-ylmethyl)-amide,
   5-Benzoylamino-1H-pyrazole-3-carboxylic acid (pyridin-3-ylmethyl)-amide,
   5-Phenylacetylamino-1H-pyrazole-3-carboxylic acid (pyridin-3-ylmethyl)-amide,
   5-(2-Methyl-4,5-difluoro-benzoylamino)-1H-pyrazole-3-carboxylic acid (pyridin-3-ylmethyl)-amide dihydrochloride,
   5-(2-Amino-4,5-difluoro-benzoylamino)-1H-pyrazole-3-carboxylic acid (pyridin-3-ylmethyl)-amide,
   5-(4,5-Difluoro-2-methanesulfonylamino-benzoylamino)-1H-pyrazole-3-carboxylic acid (pyridin-3-ylmethyl)-amide,
   5-(2-Acetylamino-4,5-difluoro-benzoylamino)-1H-pyrazole-3-carboxylic acid (pyridin-3-ylmethyl)-amide,
   5-[(3,4,5-Trichloro-thiophene-2-carbonyl)-amino]-1H-pyrazole-3-carboxylic acid (pyridin-3-ylmethyl)-amide,
   5-(4,5-Difluoro-2-methyl-benzoylamino)-1H-pyrazole-3-carboxylic acid (2-cyano-pyridin-4-ylmethyl)-amide,
   5-(4,5-Difluoro-2-methyl-benzoylamino)-1H-pyrazole-3-carboxylic acid [2-(1H-tetrazol-5-yl)-pyridin-4-ylmethyl]-amide, and 1H-Pyrazole-3,5-dicarboxylic acid 3-benzylamide 5-[(2,4-dichloro-phenyl)-amide].
56. A therapeutic or prophylactic agent for insulin resistance, diabetic neuropathy, diabetic nephropathy, diabetic retinopathy, cataract, hypercholesterolemia, hypertension, hyperinsulinemia, hyperlipidemia, atherosclerosis, tissue ischemia, myocardial ischemia, obesity or bacterial, fungal, parasitic or viral infection, comprising a pyrazole compound according to any of the above-described 36 to 55 or a pharmacologically acceptable salt thereof as an active ingredient.
57. A medicine comprising a combination of a therapeutic or prophylactic agent according to the above-described 56 and another drug.

### Advantages of the Invention

The pyrazole compound of the present invention has a liver glycogen phosphorylase inhibitory activity with a potent activity and fewer adverse reactions and superior oral absorbability and metabolic stability compared to conventional antidiabetics, provides an extremely useful novel pharmaceutical composition for treating or preventing diabetes, and has the potential as a useful therapeutic agent for insulin resistance, diabetic neuropathy, diabetic nephropathy, diabetic retinopathy, cataract, hypercholesterolemia, hypertension, hyperinsulinemia, hyperlipidemia, atherosclerosis, tissue ischemia and myocardial ischemia, a therapeutic agent for appetite control and obesity, and a therapeutic agent for infections such as bacterial, fungal, parasitic or viral infection.

### Best Mode for Carrying Out the Invention

The pyrazole skeleton of the pyrazole compound of the present invention includes not only the following formula below: 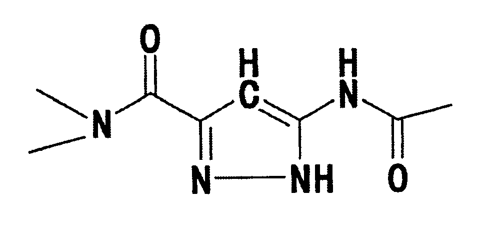 but also the following isomeric formula:

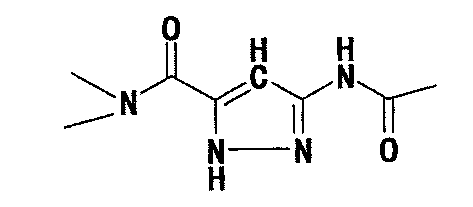

Therefore, the pyrazole compounds of the present invention are not limited to the pyrazole compounds represented by the general formula (I) described above, but include the pyrazole compounds represented by the following formula (I').

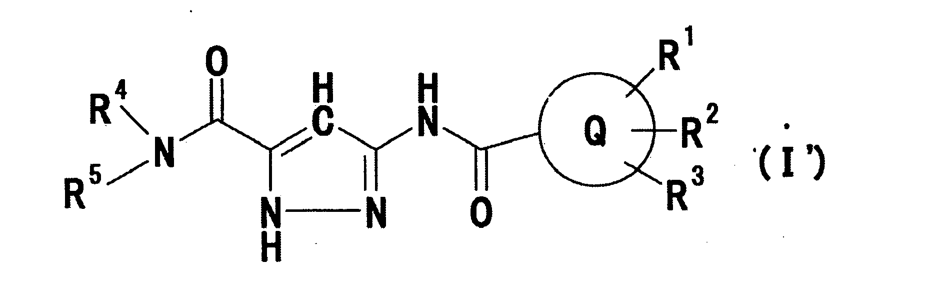

The substituents and moieties used herein are defined as follows.

A "halogen atom" is a fluorine, chlorine, bromine or iodine atom. A fluorine or chlorine atom is preferred.

A "C₁₋₆ alkyl group" represents a linear or branched alkyl group having 1 to 6 carbon atoms, and includes, for example, a methyl group, ethyl group, propyl group, isopropyl group, butyl group, isobutyl group, sec-butyl group, tert-butyl group, pentyl group, isopentyl group, 2-methylbutyl group, neopentyl group, 1-ethylpropyl group, hexyl group, isohexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, 1,3-dimethylbutyl group, 2, 3-dimethylbutyl group, 1-ethylbutyl group and 2-ethyl butyl group. A C₁₋₄ alkyl group is preferred.

A "C₁₋₄ alkyl group" represents a linear or branched alkyl group having 1 to 4 carbon atoms, and includes, for example, a methyl group, ethyl group, propyl group, isopropyl group, butyl group, isobutyl group, sec-butyl group and tert-butyl group. A methyl group is preferred.

A "C₂₋₆ alkenyl group" represents an optionally branched alkenyl group having 2 to 6 carbon atoms with one or more double bonds, and includes, for example, an ethenyl group (vinyl group), 1-propenyl group, 2-propenyl group, 1-butenyl group, 2-butenylgroup, 3-butenyl group,2-methyl-1-propenylgroup, 1-pentenyl group, 2-pentenyl group, 3-pentenyl group, 4-pentenyl group, 3-methyl-2-butenyl group, 1-hexenyl group, 3-hexenyl group, 2,4-hexadienyl group and 5-hexenyl group. Optionally branched C₂₋₄ alkenyl groups having 2 to 4 carbon atoms are preferred, such as an ethenyl group (vinyl group), 1-propenyl group, 2-propenyl group and 1-butenyl group. A vinyl group and propenyl group are more preferred.

A "C₂₋₆ alkynyl group" represents an optionally branched alkynyl group having 2 to 6 carbon atoms with one or more triple bonds, and includes, for example, an ethynyl group, 1-propynyl group, 2-propynyl group, 1-butynyl group, 2-butynyl group, 3-butynyl group, 1-pentynyl group, 2-pentynyl group, 3-pentynyl group, 4-pentynyl group, 1-hexynyl group, 2-hexynyl group, 3-hexynyl group, 4-hexynyl group and 5-hexynyl group. Preferred are optionally branched C₂₋₄ alkynyl groups having 2 to 4 carbon atoms, such as an ethynyl group, 1-propynyl group, 2-propynyl group, 1-butynyl group and 2-butynyl group.

A "C₁₋₆ alkylene group" represents an optionally branched alkylene group having 1 to 6 carbon atoms, and includes, for example, a methylene group, ethylene group, propylene group, butylene group, pentylene group, and hexylene group, and preferably optionally branched C₁₋₄ alkylene groups having 1 to 4 carbon atoms, such as a methylene group, ethylene group, propylene group, and butylene group.

A "halo C₁₋₆ alkyl group" represents a "C₁₋₆ alkyl group" as described above substituted with one or more, preferably 1 to 6, especially preferably 1 to 3 "halogen atoms" described above, and includes, for example, a trifluoromethyl group, trichloromethyl group, difluoromethyl group, dichloromethyl group, dibromomethyl group, fluoromethyl group, 2,2,2-trifluoroethyl group, 2,2,2-trichloroethyl group, 2-bromoethyl group, 2-chloroethyl group, 2-fluoroethyl group, 2-iodoethyl group, 3-chloropropyl group, 4-fluorobutyl group, 6-iodohexyl group and 2,2-dibromoethyl group, and preferably halo C₁₋₄ alkyl groups having 1 to 4 carbon atoms.

A "C₁₋₆ alkoxy group" represents an alkoxy group having 1 to 6 carbon atoms in which the alkyl moiety is an optionally branched "C₁₋₆ alkyl group" as defined above, and includes, for example, a methoxy group, ethoxy group, propoxy group, isopropoxy group, butoxy group, isobutoxy group, sec-butoxy group, tert-butoxy group, pentyloxy group, iso pentyloxy group, 2-methylbutoxy group, 1-ethylpropoxy group, 2-ethylpropoxy group, neo pentyloxy group, hexyloxy group, 4-methyl pentyloxy group, 3-methyl pentyloxy group, 2-methyl pentyloxy group, 3,3-dimethylbutoxy group, 2,2-dimethylbutoxy group, 1,1-dimethylbutoxy group, 1,2-dimethylbutoxy group, 1,3-dimethylbutoxy group and 2,3-dimethylbutoxy group. Preferred are C₁₋₄ alkoxy groups having 1 to 4 carbon atoms, such as a methoxy group, ethoxy group, propoxy group, isopropoxy group and butoxy group.

A "C₁₋₆ alkyl group substituted with a C₁₋₆ alkoxy group" or a "C₁₋₆ alkoxy C₁₋₆ alkyl group" represents an optionally branched "C₁₋₆ alkyl group" as described above that is substituted with an optionally branched "C₁₋₆ alkoxy group" as described above, and includes, for example, a methoxymethyl group, ethoxymethyl group, propoxymethyl group, isopropoxymethyl group, butoxymethyl group, isobutoxymethyl group, sec-butoxymethyl group, tert-butoxymethyl group, 2-methoxyethyl group, 2-ethoxyethyl group, 2-propoxyethyl group, 2-isopropoxyethyl group, 2-butoxyethyl group, 2-isobutoxyethyl group, 2-(sec-butoxy)ethyl group, 2-(tert-butoxy)ethyl group, 1-methoxyethyl group, 1-ethoxyethylgroup,1-propoxyethylgroup,1-isopropoxyethyl group, 1-butoxyethyl group, 1-isobutoxyethyl group, 1-(sec-butoxy)ethyl group, 1-(tert-butoxy)ethyl group and 3-isopropoxypropyl group. Preferred are C₁₋₄ alkoxy C₁₋₄ alkyl groups with the alkoxy and alkyl moieties having 1 to 4 carbon atoms, such as a methoxymethyl group, ethoxymethyl group, 1-methoxyethyl group, 2-methoxyethyl group and 2-butoxyethyl group.

A "C₁₋₆ alkoxycarbonyl group" represents an optionally branched alkoxy group having 1 to 6 carbon atoms as defined above which is bound to a carbonyl group, and includes, for example, a methoxycarbonyl group, ethoxycarbonyl group, propoxycarbonyl group, isopropoxycarbonyl group, butoxycarbonyl group, isobutoxycarbonyl group, sec-butoxycarbonyl group, tert-butoxycarbonyl group, pentyloxycarbonyl group, isopentyloxycarbonyl group, 2-methylbutoxycarbonyl group, neopentyloxycarbonyl group, 1-ethylpropoxycarbonyl group, hexyloxycarbonyl group, 4-methylpentyloxycarbonyl group, 3-methylpentyloxycarbonyl group, 2-methylpentyloxycarbonyl group, 1-methylpentyloxycarbonyl group, 3,3-dimethylbutoxycarbonyl group, 2,2-dimethylbutoxycarbonyl group, 1,1-dimethylbutoxycarbonyl group, 1,2-dimethylbutoxycarbonyl group, 1,3-dimethylbutoxycarbonyl group, 2,3-dimethylbutoxycarbonyl group and 2-ethylbutoxycarbonyl group. Preferred are (C₁₋₄ alkoxy)carbonyl groups in which the alkoxy moiety has 1 to 4 carbon atoms, such as a methoxycarbonylgroup, ethoxycarbonyl group, propoxycarbonyl group and isopropoxycarbonyl group.

A "C₁₋₆ alkoxycarbonyloxy group" represents an optionally branched "C₁₋₆ alkoxycarbonyl group" as defined above which is bound to an oxygen atom, and includes, for example, a methoxycarbonyloxy group, ethoxycarbonyloxy group, propoxycarbonyloxy group, isopropoxycarbonyloxy group, butoxycarbonyloxy group, isobutoxycarbonyloxy group, sec-butoxycarbonyloxy group, tert-butoxycarbonyloxy group, pentyloxycarbonyloxy group, isopentyloxycarbonyloxy group, 2-methylbutoxycarbonyloxy group, neopentyloxycarbonyloxy group, 1-ethylpropoxycarbonyloxy group,hexyloxycarbonyloxy group, 4-methylpentyloxycarbonyloxy group, 3-methylpentyloxycarbonyloxy group, 2-methylpentyloxycarbonyloxy group, 1-methylpentyloxycarbonyloxy group, 3,3-dimethylbutoxycarbonyloxy group, 2,2-dimethylbutoxycarbonyloxy group, 1,1-dimethylbutoxycarbonyloxy.group, 1,2-dimethylbutoxycarbonyloxy group, 1,3-dimethylbutoxycarbonyloxy group, 2,3-dimethylbutoxycarbonyloxy group or 2-ethylbutoxycarbonyloxy group. Preferred are (C₁₋₄ alkoxy)carbonyloxy groups in which the alkoxy moiety has 1 to 4 carbon atoms, such as a methoxycarbonyloxy group, ethoxycarbonyloxy group, propoxycarbonyloxy group, isopropoxycarbonyloxy group and butoxycarbonyloxy group.

"C₁₋₆ alkylcarbonyloxy group" represents an optionally branched "C₁₋₆ alkyl group" as defined above which is bound to a carbonyloxy group, and includes, for example, an acetoxy group, a propionyloxy group, a butyryloxy group, an isobutyryloxy group, a valeryloxy group, an isovaleryloxy group, a pivaloyloxy group and a hexanoyloxy group.

A "C₁₋₆ alkylcarbonyloxy C₁₋₆ alkyl group" represents a "C₁₋₆ alkyl group" substituted with the "C₁₋₆ alkylcarbonyloxy group" as described above and is preferably a C₁₋₄ alkyl group substituted with a C₁₋₄ alkylcarbonyloxy group.

A "C₁₋₆ alkoxy group which may be substituted with a C₁₋₆ alkoxycarbonyl group" represents an unsubstituted C₁₋₆ alkoxy group or a C₁₋₆ alkoxy group substituted with a C₁₋₆ alkoxycarbonyl group, and a C₁₋₆ alkoxy group substituted with a C₁₋₆ alkoxycarbonyl group represents a "C₁₋₆ alkoxy group" substituted with the "C₁₋₆ alkoxycarbonyl group" as defined above. Preferred are an unsubstituted C₁₋₄ alkoxy group and a C₁₋₄ alkoxy group substituted with a C₁₋₄ alkoxycarbonyl group.

A "methylenedioxy group" and an "ethylenedioxy group" represent -O-CH₂-O- and -O-C₂H₄-O-, respectively.

An "acyl group" represents an optionally branched C₂₋₇ aliphatic acyl group or an aromatic acyl group in which a saturated or unsaturated hydrocarbon group is bound to the carbonyl group. Aliphatic acyl groups include, for example, an acetyl group, propionyl group, butyryl group, isobutyryl group, valeryl group, isovaleryl group, pivaloyl group, hexanoyl group, acryloyl group, methacryloyl group and crotonoyl group. Aromatic acyl groups include arylcarbonyl groups such as benzoyl group, α-naphthoyl groupandβ-naphthoyl group, halogenated arylcarbonyl groups such as a 2-bromobenzoyl.group and 4-chlorobenzoyl group, C₁₋₆ alkylated arylcarbonyl groups such as a 2,4,6-trimethylbenzoyl group and 4-toluoyl group, C₁₋₆ alkoxylated arylcarbonyl groups such as a 4-anisoyl group, nitrated arylcarbonyl groups such as a 4-nitrobenzoyl group and 2-nitrobenzoyl group, C₁₋₆ alkoxycarbonylated arylcarbonyl groups such as a 2-(methoxycarbonyl) benzoyl group, and arylated arylcarbonyl groups such as a 4-phenylbenzoyl group.

A "C₁₋₆ alkylamino group" represents the following "mono C₁₋₆ alkylamino groups" or "di C₁₋₆ alkylamino groups." Preferred are C₁₋₄ alkylamino groups having 1 to 4 carbon atoms .

A "mono C₁₋₆ alkylamino group" represents an amino group substituted with the "C₁₋₆ alkyl group" defined above, and includes, for example, a methylamino group, ethylamino group, propylamino group, isopropylamino group, butylamino group, isobutylamino group, sec-butylamino group, tert-butylamino group, pentylamino group, isopentylamino group, 2-methylbutylamino group, neopentylamino group, 1-ethylpropylamino group, hexylamino group, isohexylamino group, 4-methylpentylaminogroup, 3-methylpentylaminogroup, 2-methylpentylamino group, 1-methylpentylamino group, 3,3-dimethylbutylamino group,2,2-dimethylbutylamino group, 1,1-dimethylbutylamino group, 1,2-dimethylbutylamino group, 1,3-dimethylbutylamino group, 2,3-dimethylbutylamino group and 2-ethylbutylamino group. Preferred are mono-C₁₋₄ alkylamino groups substituted with a C₁₋₄ alkyl group, such as a methylamino group, ethylamino group, propylamino group, isopropylamino group, butylamino group and isobutylamino group.

A "di C₁₋₆ alkylamino group" represents an amino group substituted with two identical or different "C₁₋₆ alkyl groups" as defined above, and includes, for example, a dimethylamino group, diethylamino group, N-ethyl-N-methylamino group, dipropylamino group, dibutylamino group, dipentylamino group and dihexylamino group. Preferred are di-C₁₋₄ alkylamino groups substituted with two C₁₋₄ alkyl groups, such as a dimethylamino group and diethylamino group.

A "acylamino group" represents an amino group substituted with an "acyl group" described above, and includes, for example, optionally branched lower aliphatic acylamino groups having 2 to 7 carbon atoms, such as an acetylamino group, propionylamino group, butyrylamino group, isobutyrylamino group, valerylamino group, isovalerylamino group, pivaloylamino group, hexanoylamino group, acryloylamino group, methacryloylamino group and crotonoylamino group, or aromatic acylamino groups such as a benzoylamino group.

A "C₁₋₆ alkoxycarbonylamino group" represents an amino group substituted with the "C₁₋₆ alkoxycarbonyl group" defined above, and includes, for example, a methoxycarbonylamino group, ethoxycarbonylamino group, propoxycarbonylamino group and isopropoxycarbonylamino group. Preferred are C₁₋₄ alkoxy carbonylamino groups, such as a methoxycarbonylamino group, ethoxycarbonylamino group and propoxycarbonylamino group.

A "carbamoyl group" represents, in a limited sense, -CONH₂, and, in a broad sense, -CONH₂, a "mono-C₁₋₆ alkylcarbamoyl group" , a "di-C₁₋₆ alkylcarbamoyl group" , "di-arylcarbamoyl group"or an "N-C₁₋₆ alkyl N-arylcarbamoyl group."

A "mono-C₁₋₆ alkylcarbamoyl group" represents a carbamoyl group binding a "C₁₋₆ alkyl group" as defined above, and includes, for example, a methylcarbamoyl group, ethylcarbamoyl group, propylcarbamoyl group, isopropylcarbamoyl group, butylcarbamoyl group, isobutylcarbamoyl group, sec-butylcarbamoyl group, tert-butylcarbamoyl group, pentylcarbamoyl group, isopentylcarbamoyl group, 2-methylbutylcarbamoyl group, neopentylcarbamoyl group, 1-ethylpropylcarbamoyl group and hexylcarbamoyl group, and preferably mono-C₁₋₄ alkylcarbamoyl group in which the alkyl group is an alkyl group having 1 to 4 carbon atoms, such as a methylcarbamoyl group, ethylcarbamoyl group, propylcarbamoyl group, isopropylcarbamoyl group and butylcarbamoyl group.

A "di-C₁₋₆ alkylcarbamoyl group" represents a carbamoyl group binding two identical or different "C₁₋₆ alkyl groups" as defined above, and includes, for example, a dimethylcarbamoyl group, diethylcarbamoyl group, N-ethyl N-methylcarbamoyl group, dipropylcarbamoyl group, dibutylcarbamoyl group, dipentylcarbamoyl group and dihexylcarbamoyl group, and preferably di-C₁₋₄ alkylcarbamoyl groups in which the alkyl group is an alkyl group having 1 to 4 carbon atoms, such as a dimethylcarbamoyl group and diethylcarbamoyl group.

A "N-C₁₋₆ alkyl-N-arylcarbamoyl group" represents a carbamoyl group binding on its nitrogen atom an "aryl group" and the "C₁₋₆ alkyl group" defined above, and includes, for example, an N-methyl-N-phenylcarbamoyl group, N-ethyl-N-phenylcarbamoyl group, N-phenyl-N-propylcarbamoyl group, N-isopropyl-N-phenylcarbamoyl group, N-butyl-N-phenylcarbamoyl group, N-pentyl-N-phenylcarbamoyl group, N-hexyl-N-phenylcarbamoyl group, N-methyl-N-naphthylcarbamoyl group, N-ethyl-N-naphthylcarbamoyl group, N-naphthyl-N-propylcarbamoyl group, N-isopropyl-N-naphthylcarbamoyl group, N-butyl-N-naphthylcarbamoyl group, N-naphthyl-N-pentylcarbamoyl and N-hexyl-N-naphthylcarbamoyl group, and preferably N-C₁₋₄ alkyl-N-phenylcarbamoyl groups in which the alkyl group is an alkyl group having 1 to 4 carbon atoms and the aryl group is a phenyl group, such as an N-methyl-N-phenylcarbamoyl group and N-ethyl-N-phenylcarbamoyl group.

A "C₁₋₆ alkylsulfonyl group" represents a sulfonyl group binding the "C₁₋₆ alkyl group" defined above, and includes, for example, a methylsulfonyl group, ethylsulfonyl group, propylsulfonylgroup, isopropylsulfonylgroup,butylsulfonyl group, isobutylsulfonyl group, sec-butylsulfonyl group, tert-butylsulfonyl group, pentylsulfonyl group, isopentylsulfonyl group, 2-methylbutylsulfonyl group, neopentylsulfonyl group, 1-ethylpropylsulfonyl group, hexylsulfonyl group, isohexylsulfonyl group, 4-methylpentylsulfonyl group, 3-methylpentylsulfonyl group, 2-methylpentylsulfonyl group, 1-methylpentylsulfonyl group, 3,3-dimethylbutylsulfonyl group, 2,2-dimethylbutylsulfonyl group, 1,1-dimethylbutylsulfonyl group, 1,2-dimethylbutylsulfonyl group, 1,3-dimethylbutylsulfonyl group, 2,3-dimethylbutylsulfonyl group and 2-ethylbutylsulfonyl group, and preferably C₁₋₄ alkylsulfonyl groups in which the alkyl moiety is an alkyl group having 1 to 4 carbon atoms, such as a methylsulfonyl group, ethylsulfonyl group, propylsulfonyl group, isopropylsulfonyl group and butylsulfonyl group. More preferred is a methylsulfonyl group.

A "C₁₋₆ alkylsulfonylamino group" represents an amino group binding the "C₁₋₆ alkylsulfonyl group" defined above, and includes, for example, a methylsulfonylamino group, ethylsulfonylamino group, propylsulfonylamino group, isopropylsulfonylamino group, butylsulfonylamino group, isobutylsulfonylamino group, sec-butylsulfonylamino group, tert-butylsulfonylamino group, pentylsulfonylamino group, isopentylsulfonylamino group, 2-methylbutylsulfonylamino group, neopentylsulfonylamino group, 1-ethylpropylsulfonylamino group, hexylsulfonylamino group, isohexylsulfonylamino group, 4-methylpentylsulfonylamino group, 3-methylpentylsulfonylamino group, 2-methylpentylsulfonylamino group, 1-methylpentylsulfonylamino group, 3,3-dimethylbutylsulfonylamino group, 2,2-dimethylbutylsulfonylamino group, 1,1-dimethylbutylsulfonylamino group, 1,2-dimethylbutylsulfonylamino group, 1,3-dimethylbutylsulfonylamino group, 2,3-dimethylbutylsulfonylamino group and 2-ethylbutylsulfonylamino group, and preferably C₁₋₄ alkylsulfonylamino groups in which the alkyl group is an alkyl group having 1 to 4 carbon atoms, such as a methylsulfonylamino group, ethylsulfonylamino group, propylsulfonylamino group, isopropylsulfonylamino group and butylsulfonylamino group.

A "C₃₋₈ cycloalkyl group" represents a saturated cycloalkyl group having 3 to 8, preferably 3 to 6 carbon atoms, or may also represent a C₃₋₈ cycloalkenyl group containing one or two double bonds within the ring. Examples of "saturated C₃₋₈ cycloalkyl groups" include a cyclopropyl group, cyclobutyl group, cyclopentyl group, cyclohexyl group, cycloheptyl group and cyclooctyl group . In addition, a "C₃₋₈ cycloalkenyl group" represents a cycloalkenyl group having 3 to 8, preferably 5 to 7 carbon atoms, and containing at least one, preferably one or two double bonds within the ring. Specific examples include cyclopropenyl group, cyclobutenyl group, cyclopentenyl group, cyclopentadienyl group, cyclohexenyl group, 2,4-cyclohexadien-1-yl group, 2,5-cyclohexadien-1-yl group, cycloheptenyl group and cyclooctenyl group.

A "C₃₋₈ cycloalkyl C₁₋₆ alkyl group" represents a "C₁₋₆ alkyl group" as described above substituted with the "C₃₋₈ cycloalkyl group" described above, and includes, for example, a cyclopropylmethyl group, cyclopropylethyl group, cyclopentylmethyl group, cyclopentylethyl group, cyclohexylmethyl group and cyclohexylethyl group. Preferred are "C₃₋₈ cycloalkyl C₁₋₄ alkyl groups" in which the alkyl group is an alkyl group having 1 to 4 carbon atoms, such as a cyclopropylmethyl group, cyclopropylethyl group, cyclopentylmethyl group, cyclopentylethyl group and cyclohexylmethyl group.

A "C₃₋₈ cycloalkyloxy group" represents a "C₃₋₈ cycloalkyl group" as defined above, bound to an oxygen atom, and includes, for example, a cyclopropyloxy group, cyclobutyloxy group, cyclopentyloxy group, cyclohexyloxy group, cycloheptyloxy group and cyclooctyloxy group. Preferred are C₃₋₆ cycloalkyloxy groups in which the cycloalkyl group is a cycloalkyl group having 3 to 6 carbon atoms, such as a cyclopropyloxy group, cyclobutyloxy group, cyclopentyloxy group and cyclohexyloxy group.

A "C₃₋₈ cycloalkyloxycarbonyloxy group" represents a "C₃₋₈ cycloalkyloxy group" as defined above bound to a carbonyloxy group, and includes, for example, a cyclopropyloxycarbonyloxy group, cyclobutyloxycarbonyloxy group, cyclopentyloxycarbonyloxy group, cyclohexyloxycarbonyloxy group, cycloheptyloxycarbonyloxy group and cyclooctyloxycarbonyloxy group. Preferred are C₃₋₆ cycloalkyloxycarbonyloxy groups in which the cycloalkyl group is a cycloalkyl group having 3 to 6 carbon atoms, such as a cyclopropyloxycarbonyloxy group, cyclobutyloxycarbonyloxy group, cyclopentyloxycarbonyloxy group, cyclohexyloxycarbonyloxy groups.

A "C₃₋₈ cycloalkyloxycarbonyloxy C₁₋₆ alkyl group" represents a "C₁₋₆ alkyl group" substituted with the "C₃₋₈ cycloalkyloxycarbonyloxy group" described above and preferably is a C₃₋₆ cycloalkyloxycarbonyloxy group in which the cycloalkyl group is a cycloalkyl group having 3 to 6 carbon atoms and a cycloalkyloxycarbonyloxyalkyl group in which the alkyl group is a "C₁₋₄ alkyl group."

An "aryl group" represents an aromatic hydrocarbon groups having 6 to 14 carbon atoms, and includes, for example, a phenyl group, naphthyl group, anthryl group, indenyl group, azulenyl group, fluorenyl group and phenanthryl group. The aryl group may be saturated partially depending on the case. Examples of partially saturated aryl groups include a dihydroindenyl group and tetrahydronaphthyl group. Preferred are C₆₋₁₀ aryl groups, more preferred is a phenyl or naphthyl group, and most preferred is a phenyl group.

A phenyl group is preferred as "Q" in formula (I).

An "aralkyl group" may be a "C₁₋₆ alkyl group" as defined above binding one or two aryl groups having 6 to 14 carbon atoms, such as a benzyl group, naphthylmethyl group, indenylmethyl group, phenanthrenylmethyl group, anthracenylmethyl group, diphenylmethyl group, phenethyl group, naphthylethyl group, phenylpropyl group, naphthylpropyl group, phenylbutyl group, naphthylbutyl group, phenylpentyl group, naphthylpentyl group and phenylhexyl group, and preferably is an aralkyl group in which the aryl group is a phenyl group and the alkyl group is a C₁₋₄ alkyl group, such as a benzyl group, naphthylmethyl group, diphenylmethyl group and phenethyl group, and more preferably a benzyl or phenethyl group, and particularly preferably a benzyl group.

An "aryloxy group" is an "aryl group" as defined above binding an oxygen atom, and includes, for example, a phenoxy group and a naphthoxy group. A phenoxy group is preferable.

An "arylsulfonyl group" represents a sulfonyl group binding the "aryl group" defined above, and includes, for example, C₆₋₁₄ arylsulfonyl groups such as a phenylsulfonyl group, indenylsulfonyl group, naphthylsulfonyl group, phenanthrenylsulfonyl group, anthracenylsulfonyl group and fluorenylsulfonyl group, preferably C₆₋₁₀ arylsulfonyl groups in which a C₆₋₁₀ aryl is bound to the sulfonyl group, more preferably a phenylsulfonyl or naphthylsulfonyl group, most preferably a phenylsulfonyl group.

An "arylsulfonyl group that may be substituted with a C₁₋₆ alkyl group" represents an "arylsulfonyl group" as defined above in which the aryl group is substituted with one or more, preferably 1 to 3 "C₁₋₆ alkyl groups" as defined above, preferably "C₁₋₄ alkyl groups," and includes, for example, a p-toluenesulfonyl group.

An"arylsulfonylaminogroup"represents an "arylsulfonyl group" as defined above bound to an amino group, and includes, for example, C₆₋₁₄ arylsulfonylamino groups, such as a phenylsulfonylamino group, indenylsulfonylamino group, naphthylsulfonylamino group, phenanthrenylsulfonylamino group, anthracenylsulfonylamino group and fluorenylsulfonylamino group, preferably C₆₋₁₀ arylsulfonylamino groups, more preferably a phenylsulfonylamino or naphthylsulfonylamino group, most preferably a phenylsulfonylamino group.

A "phenyl group that may be substituted with a halogen atom" represents a phenyl group substituted with one or more, preferably 1 to 3 identical or different halogen atoms selected from the group consisting of a fluorine atom, chlorine atom, bromine atom and iodine atom.

A "phenyl group that may be substituted with a halo C₁₋₆ alkyl group" represents a phenyl group substituted with one or more identical or different "halo C₁₋₆ alkyl groups" defined above.

A "heterocyclic group" represents a saturated, partially unsaturated or aromatic ring having, as ring-forming atoms other than carbon atoms, 1 to 4 identical or different heteroatoms selected from the group consisting of an oxygen atom, nitrogen atom and sulfur atom, and having 3 to 14, preferably 5 to 7 ring-forming atoms, and the ring may be a monocyclic or condensed ring.

A "saturated monocyclic heterocyclic group" represents a saturated or partially saturated heterocyclic group, and includes, for example, a pyrrolidinyl group, tetrahydrofuryl group, tetrahydrothienyl group, imidazolidinyl group, pyrazolidinyl group, 1,3-dioxolanyl group, 1,3-oxathiolanyl group, oxazolidinyl group, thiazolidinyl group, piperidyl group, piperazinyl group, tetrahydropyranyl group, tetrahydrothiopyranyl group, dioxanyl group, morpholinyl group, thiomorpholinyl group, 2-oxopyrrolidinyl group, 2-oxopiperidyl group, 4-oxopiperidyl group and 2,6-dioxopiperidyl group. Preferred are saturated 5- or 6-membered heterocyclic groups, such as a pyrrolidinyl group, piperidyl group and morpholinyl group.

A "saturated 5- or 6-membered monocyclic heterocyclic group" represents the "saturated monocyclic heterocyclic group" defined above that has a 5- or 6-membered ring. Examples of heterocyclic rings constituting these heterocyclic groups, specifically, saturated 5- or 6-membered monocyclic heterocyclic rings, include 5-membered heterocyclic rings, such as tetrahydrofuran,1,3-dioxolane,tetrahydrothiophene, pyrrolidine, pyrazolidine and imidazolidine, and 6-membered heterocyclic rings, such as tetrahydropyran, 1,4-dioxane, thiane, 1,4-dithiane, piperidine, piperazine and morpholine. The heterocyclic group may partially have a double bond.

A "5-memberedheterocyclic group having 1 to 4 heteroatoms selected from the group consisting of sulfur, oxygen and nitrogen atoms" represents, for example, 5-membered heteroaromatic groups, such as a furyl group, thienyl group, pyrrolyl group, pyrazolyl group, imidazolyl group, oxazolyl group, isoxazolyl group, thiazolylgroup, isothiazolyl group, oxadiazolyl group, triazolyl group, tetrazolyl group and thiadiazolyl group, and examples of saturated or partially saturated monocyclic heterocyclic groups include a tetrahydrofuran-2-yl group, tetrahydrofuran-3-yl group, imidazolidin-1-yl group, imidazolidin-2-yl group, imidazolidin-4-yl group, pyrrolidin-1-yl group, pyrrolidin-2-yl group, pyrrolidin-3-yl group, 1,3-oxazolidin-3-yl group, isothiazolidin-2-yl group, 1,3-thiazolidin-3-yl group, 1,2-pyrazolidin-2-yl group and 1,2-pyrazolidin-1-yl group.

A "saturated 5- or 6-membered monocyclic heterocyclic group, having at least one nitrogen atom" represents a saturated 5- or 6-membered monocyclic heterocyclic group, which has at least one nitrogen atom, and may have 1 to 3 heteroatoms selected from the group consisting of nitrogen, sulfur and oxygen atoms. Examples of heterocyclic rings constituting 5-membered heterocyclic groups include, for example, pyrrolidine, pyrazolidine, imidazolidine, oxazolidine and thiazolidine, and examples of heterocyclic rings constituting 6-membered heterocyclic groups include piperidine, piperazine, morpholine and oxadiazine. A part of the saturated heterocyclic ring may have a double bond.

Examples of a "monocyclic heteroaromatic group" include a pyridyl group, pyrazinyl group, pyrimidinyl group, pyridazinyl group, 1,3,5-triazinyl group, pyrrolyl group, pyrazolyl group, imidazolyl group, 1,2,4-triazolyl group, tetrazolyl group, thienyl group, furyl group, oxazolyl group, isoxazolyl group, thiazolyl group, isothiazolyl group and thiadiazolyl group. A 5- or 6-membered heteroaromatic group is preferable.

The "saturated heterocyclic ring" as used in "something may, together with the adjacent nitrogen atom, form a saturated heterocyclic ring" represents, for example, nitrogen-containing heterocyclic rings of a 5- to 7-membered ring that contain as ring-forming atoms other than carbon atoms, at least one nitrogen atom, and may further contain one or two heteroatoms selected from the group consisting of oxygen, sulfur and nitrogen atoms,including,for example,pyrrolidine, imidazolidine, pyrazolidine, piperidine, piperazine, morpholine and thiomorpholine.

"The saturated heterocyclic group may be condensed with a benzene ring to form a condensed ring" means that the "saturatedmonocyclic heterocyclic group" may forma condensed ring with a benzene ring, including, for example, indolinyl, isoindolinyl, 2,3-dihydrobenzofuranyl, benzothiazolinyland chromanyl.

A "heteroaromatic group" represents a heteroaromatic group having, as ring-forming atoms other than carbon atoms, 1 to 4 identical or different heteroatoms selected from the group consisting of oxygen, nitrogen and sulfur atoms, and having 3 to 14, preferably 5 to 7, more preferably 5 to 6 ring-forming atoms, or a condensed group thereof. The heteroaromatic group may be substituted with one or more oxo or thioxo groups. These heteroaromatic groups substituted with an oxo group or a thioxo group also include heterocyclic groups in which an oxo group or a thioxo group is present due to keto-enol tautomerization in a heteroaromatic group substituted with a hydroxyl group or a thiol group. Examples of the heteroaromatic group include, but not limited to, a pyridyl group, pyridazinyl group, imidazolyl group, pyrimidinyl group, pyrazolyl group, triazolyl group, pyrazinyl group, quinolyl group, isoquinolyl group, tetrazolyl group, furyl group, thienyl group, isoxazolyl group, thiazolylgroup,oxazolylgroup, isothiazolylgroup,pyrrolyl group, quinolinyl group, isoquinolinyl group, indolyl group, benzimidazolyl group, benzofuranyl group, cinnolinyl group, indazolyl group, indolizinyl group, phthalazinyl group, pyridazinylgroup, triazinyl group, isoindolyl group, purinyl group, oxadiazolyl group, thiazolyl group, thiadiazolyl group, furazanyl group, benzofurazanyl group, benzothiophenyl group, benzotriazolyl group, benzothiazolyl group, benzoxazolyl group, quinazolinyl group, quinoxalinyl group, naphthylizinyl group, dihydroquinolyl group, furopyridinyl group, pyrrolopyrimidinyl group and azaindolyl group.

A "5- or 6-membered heteroaromatic group" represents a heteroaromatic group having, as ring-forming atoms other than carbon atoms, 1 to 4 identical or different heteroatoms selected from the group consisting of an oxygen atom, nitrogen atom and sulfur atom, and having 5 to 6 ring-forming atoms, or a condensed group thereof. Examples include a pyrrolyl group, furylgroup, thienyl group, imidazolyl group, pyrazolyl group, oxazolyl group, isoxazolyl group, thiazolyl group, isothiazolyl group, 1,2,4-triazolyl group, 1,2,3-triazolyl group, tetrazolyl group, 1,3,4-oxadiazolyl group, 1,2,4-oxadiazolyl group, 1,3,4-thiadiazolyl group, 1,2,4-thiadiazolyl group, furazanyl group, pyridyl group, pyrimidinyl group, pyridazinyl group, pyrazinyl group, 1,3,5-triazinylgroup, imidazolinyl group, pyrazolinyl group, oxazolinyl group (2-oxazolinyl group, 3-oxazolinyl group, 4-oxazolinyl group), isooxazolinyl group, thiazolinyl group, isothiazolinyl group, pyranyl group, 2-oxopyranyl group and 2-oxo-2,5-dihydrofuranyl group. Preferred are a pyrrolyl group, furylgroup, thienyl group, imidazolyl group, pyrazolyl group, oxazolyl group, isoxazolyl group, thiazolyl group, isothiazolyl group, pyridyl group and 2-oxo-2,5-dihydrofuranyl group.

A "5- or 6-membered heteroaromatic group that may be substituted with an oxo or thioxo group" represents a 5- or 6-membered heteroaromatic group as defined above that may be substituted identical with or different from each other with one or more substituents selected from an oxo group (=O) or a thioxo group (= S). These 5- or 6-membered heteroaromatic groups substituted with an oxo group or a thioxo group also include 5- or 6-membered heterocyclic groups in which an oxo group or a thioxo group is present due to keto-enol tautomerization in a heteroaromatic group substituted with a hydroxyl group or a thiol group.

A "5- or 6-membered heteroaromatic group which may be substituted with a carboxy group or a C₁₋₆ alkoxycarbonyl group" represents a "5- or 6-membered heteroaromatic group" as defined above that may substituted with one or more carboxy groups or "C₁₋₆ alkoxycarbonyl groups" defined above.

"The heteroaromatic group maybe a monocyclic or condensed ring" means that the heteroaromatic group may be a monocyclic heteroaromatic group or a condensed group thereof with one or more aromatic carbocyclic groups such as benzene ring, or other heterocyclic groups.

A "monocyclic heteroaromatic group or a heteroaromatic group condensed with a benzene ring" represents a "monocyclic heteroaromatic group, " such as a thienyl group, furyl group, pyrrolyl group, imidazolyl group, pyrazolyl group, isothiazolyl group, isoxazolyl group, pyridyl group, pyrazinyl group, pyrimidinyl group, pyridazinyl group and oxazolyl group, or a heteroaromatic group condensed with a benzene ring, such as a benzofuranyl group, isobenzofuranyl group, benzo [b] thienyl group, indolyl group, isoindolyl group, 1H-indazolyl group, benzimidazolyl group, benzoxazolyl group, benzothiazolyl group, 1H-benzotriazolyl group, quinolyl group, isoquinolyl group, cinnolinyl group, quinazolinyl group, quinoxalinyl group and phthalazinyl group.

A "C₁₋₆ alkyl group substituted with a monocyclic heteroaromatic group or a heteroaromatic group condensed with a benzene ring" represents a "C₁₋₆ alkyl group" substituted with the "monocyclic heteroaromatic group or a heteroaromatic group condensed with a benzene ring" defined above. A "C₁₋₆ alkyl group" substituted with a "monocyclic heteroaromatic group" is preferred.

A " condensed heterocyclic group " includes an indolyl group (for example, 4-indolyl group, 7-indolyl group), isoindolyl group, 1,3-dihydro-1,3-dioxoisoindolyl group, benzofuranyl group (for example, 4-benzofuranyl group, 7-benzofuranyl group), indazolyl group, isobenzofuranyl group, benzothiophenylgroup (for example, 4-benzothiophenyl group, 7-benzothiophenyl group), benzoxazolyl group (for example, 4-benzoxazolyl group, 7-benzoxazolyl group), benzimidazolyl group (for example, 4-benzimidazolyl group, 7-benzimidazolyl group), benzothiazolyl group (for example, 4-benzothiazolyl group, 7-benzothiazolylgroup), indolizinyl group, quinolyl group, isoquinolyl group, 1,2-dihydro-2-oxoquinolyl group, quinazolinyl group, quinoxalinyl group, cinnolinyl group, phthalazinyl group, quinolizinyl group, purinyl group, pteridinyl group, indolinyl group, isoindolinyl group, 5,6,7,8-tetrahydroquinolyl group, 1,2,3,4-tetrahydroquinolyl group, 2-oxo-1,2,3,4-tetrahydroquinolyl group, benzo[1,3]dioxolyl group, 3,4-methylenedioxypyridyl group, 4,5-ethylenedioxypyrimidinyl group, chromenyl group, chromanyl group and isochromanyl group.

"Something may be substituted with one or more substituents" means that something may be substituted with one or more, preferably 1 to 6, particularly preferably 1 to 3 identical or different substituents. In addition, even if the number of substitutents is not specified, it means substantially that "something may be substituted with one or more substituents." The term "identical with or different from" means that, of a plurality of substituents, all substituents are the same, all substituents are different from each other, or that two substituents of three or more substituents are the same.

The following describes preferred groups in the general formula (I) described above, but the compounds of the present invention are not limited to these groups.

The "aryl group" in Ring Q is preferably a C₆₋₁₀ aryl group, more preferably a phenyl or naphthyl group, and most preferably a phenyl group.

The "heteroaromatic group" in Ring Q is preferably a "5-or 6-membered heteroaromatic group containing 1 to 4 heteroatoms selected from the group consisting of sulfur, oxygen and nitrogen atoms, " and more preferably a thienyl group or pyridyl group.

Ring Q is preferably a phenyl or pyridyl group, and particularly preferably a phenyl group.

R¹ is preferably a hydrogen atom, a halogen atom, a C₁₋₄ alkyl group or a C₁₋₄ alkoxy group, more preferably a hydrogen atom or a halogen atom, particularly preferably a hydrogen atom, a fluorine or chlorine atom, yet more preferably a hydrogen atom or a fluorine atom, and most preferably a fluorine atom.

R² is preferably a halogen atom, a C₁₋₄ alkyl group, a C₁₋₄ alkoxy group or an azido group, more preferably a halogen atom, particularly preferably a fluorine or chlorine atom, still more preferably a fluorine atom.

R³ is preferably a halogen atom, a hydroxyl group, a C₁₋₆ alkyl group, a halo C₁₋₄ alkyl group, a C₁₋₄ alkoxy group, an azido group, an amino group, an acylamino group or a C₁₋₆ alkylsulfonylamino group, more preferably a halogen atom or a C₁₋₄ alkyl group, particularly preferably a fluorine atom, a chlorine atom or a methyl group, still more preferably a chlorine atom or a methyl group.

Preferred substituents (R¹, R² and R³) of Ring Q are a hydrogen atom, a fluorine atom or a chlorine atom at the 5-position for R¹, more preferably a fluorine atom or a hydrogen atom, a fluorine atom or a chlorine atom at the 4-position for R², more preferably a fluorine atom, and a chlorine atom or a methyl group at the 2-position for R³, preferably a chlorine atom.

Ring Q substituted with the substituents R¹, R² and R³ is preferably as follows.

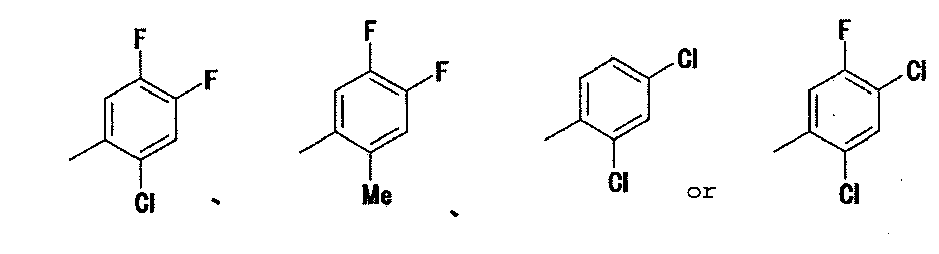

More preferred is 2-chloro-4,5-difluorophenyl group shown below.

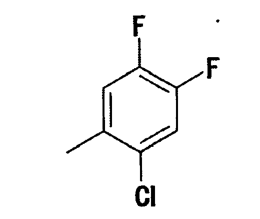

Preferably R⁴ is a group selected from 1' to 7' below.
1'. a hydrogen atom;
2'. a C₁₋₆ alkyl group or a C₁₋₆ alkyl group substituted with a substituent selected from Group A' below:
   [Group A']
   A'1. a hydroxyl group,
   A'2. a C₁₋₄ alkoxy group, such as a methoxy group, ethoxy group, propoxy group,isopropoxy group and butoxy group,particularly preferably a methoxy group, ethoxy group, propoxy group and isopropoxy group,
   A'3. -N(R⁴¹) (R^{41'}), wherein R⁴¹ and R^{41'} are preferably identical with or different from each other and represent a hydrogen atom or a C₁₋₄ alkyl group, or R⁴¹ and R^{41'} may, together with the adjacent nitrogen atom, form a saturated 5- or 6-membered monocyclic heterocyclic ring, and more preferred examples of -N (R⁴¹) (R^{41'}) include an amino group, methylamino group, ethylamino group, dimethylamino group, diethylamino group, piperidino group, morpholino group, pyrrolidino group and piperazino group, still more preferably an amino group, methylamino group, ethylamino group, dimethylamino group, diethylamino group, piperidino group, morpholino group, pyrrolidino group and piperazino group,
   A'4. a phenyl group or a naphthyl group (preferably a phenyl group),
   A'5. a 5- or 6-membered heteroaromatic group (preferably a pyridyl group),
   A'6. -CO-N(R⁴¹) (R^{41'}), such as -CONH₂, a mono-C₁₋₄ alkylcarbamoyl group, di-C₁₋₄ alkylcarbamoyl group, N-C₁₋₄ alkyl N-arylcarbamoyl group, dimethylcarbamoyl group, diethylcarbamoyl group, morpholinocarbonyl group, pyrrolidinocarbonyl group and piperazinocarbonyl group, preferably methylcarbamoyl group, ethylcarbamoyl group, dimethylcarbamoyl group or diethylcarbamoyl group,
   A'7. a carboxy group, and
   A'8. a C₁₋₄ alkoxycarbonyl group, such as a methoxycarbonyl group, ethoxycarbonyl group, propoxycarbonyl group, isopropoxycarbonyl group, butoxycarbonyl group and isobutoxycarbonyl group, preferably methoxycarbonyl group, ethoxycarbonyl group, propoxycarbonyl group and isopropoxycarbonyl group,
3'. a C₂₋₄ alkenyl group, such as an ethenyl (vinyl) group, 1-propenyl group, 2-propenyl group, 1-butenyl group, 2-butenyl group, 3-butenyl group and 2-methyl-1-propenyl group, preferably an ethenyl (vinyl) group, 1-propenyl group or 2-propenyl group;
4'. a C₂₋₄ alkynyl group, such as an ethynyl group, 1-propynyl group, 2-propynyl group and 1-butynyl group, preferably an ethynyl group, 1-propynyl group and 2-propynyl group;
5'. a C₃₋₆ cycloalkyl group, such as a cyclopropyl group, cyclobutyl group, cyclopentyl group and cyclohexyl group; 6'. a C₃₋₆ cycloalkyl C₁₋₄ alkyl group, preferably a cyclopropylmethyl group, cyclopropylethyl group, cyclobutylmethyl group, cyclobutylethyl group, cyclopentylmethyl group, cyclopentylethyl group, cyclohexylmethyl group and cyclohexylethyl group;
7'. an aryl group, for example, a phenyl group and a naphthyl group, preferably a phenyl group;

Preferably R⁵ is a group selected from 1' to 8' below.
1'. a C₁₋₆ alkyl group or a C₁₋₆ alkyl group substituted with a substituent selected from Group B' below, preferably a C₁₋₆ alkyl group;
   [Group B']
   B'1. a hydroxyl group,
   B'2. a C₁₋₄ alkoxy group, such as a methoxy group, ethoxy group, propoxy group, isopropoxy group, butoxy group and isobutoxy group,
   B'3. -N (R⁵¹) (R^{51'}), wherein R⁵¹ and R^{51'} are preferably identical with or different from each other and represent a hydrogen atom or a C₁₋₄ alkyl group, or R⁵¹ and R^{51'} may, together with the adjacent nitrogen atom, form a saturated heterocyclic ring, and examples include an amino group, methylamino group, ethylamino group, dimethylamino group, diethylamino group, N-ethyl-N-methylamino group,dipropylamino group,piperidino group, morpholino group, pyrrolidino group and piperazino group, preferably an amino group, methylamino group, ethylamino group, dimethylamino group and diethylamino group, and,
   B'4. -CO-N(R⁵¹) (R^{51'}), wherein R⁵¹ and R^{51'} are as described above. Examples include a methylcarbamoyl group, ethylcarbamoyl group, dimethylcarbamoyl group, diethylcarbamoyl group, morpholinocarbonyl group, pyrrolidinocarbonyl group and piperazinocarbonyl group, preferably a methylcarbamoyl group, ethylcarbamoyl group, dimethylcarbamoyl group and diethylcarbamoyl group,
2'. a C₃₋₈ cyclo alkyl group, such as a cyclopropyl group, cyclobutyl group, cyclopentyl group, cyclohexyl group, cycloheptyl group and cyclooctyl group, preferably a C₃₋₆ cycloalkyl group selected from the group consisting of a cyclopropyl group, cyclobutyl group, cyclopentyl group and cyclohexyl group,
   wherein the cycloalkyl group may be substituted with a hydroxyl group, a carboxy group or a C₁₋₄ alkoxycarbonyl group (preferably a methoxycarbonyl group, ethoxycarbonyl group, propoxycarbonyl group or isopropoxycarbonyl group), and may also be condensed with a pyridine ring to form the following group; 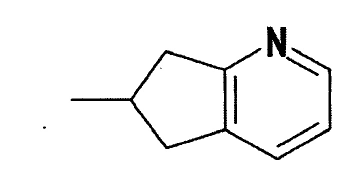
3'. a C₁₋₆ alkyl group substituted with a C₃₋₈ cycloalkyl group, preferably a C₁₋₄ alkyl group substituted with a C₃₋₆ cycloalkyl group, particularly preferably a C₁₋₂ alkyl group substituted with a C₃₋₆ cycloalkyl group, and specific examples include a cyclopropylmethyl group, cyclopropylethyl group, cyclobutylmethyl group, cyclobutylethyl group, cyclopentylmethyl group, cyclopentylethyl group, cyclohexylmethyl group and cyclohexylethyl group, wherein the cycloalkyl group of the C₃₋₈ cycloalkyl C₁₋₆ alkyl group may be substituted with a hydroxyl group, carboxy group or C₁₋₆ alkoxycarbonyl group, preferably a C₁₋₄ alkoxycarbonyl group, such as a methoxycarbonyl group, ethoxycarbonyl group, propoxycarbonyl group and isopropoxycarbonyl group;
4'. a saturated 5- or 6-membered monocyclic heterocyclic group that may be substituted with one or more substituents selected from Group C' below, wherein preferred examples of the saturated 5- or 6-membered monocyclic heterocyclic group include, for example, a pyrrolidinyl group, tetrahydrofuryl group, tetrahydrothienyl group, imidazolidinyl group, pyrazolidinyl group, 1,3-dioxolanyl group, 1,3-oxathiolanyl group, oxazolidinyl group, thiazolidinyl group, piperidyl group, piperazinyl group, tetrahydropyranyl group, tetrahydrothiopyranyl group, dioxanyl group, morpholinyl group, thiomorpholinyl group, 2-oxopyrrolidinyl group, 2-oxopiperidyl group, 4-oxopiperidyl group and 2,6-dioxopiperidyl group, preferably a pyrrolidinyl group, piperidyl group and morpholinyl group, most preferably a 6-membered saturated heterocyclic group shown below: 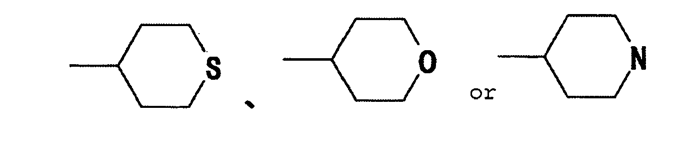 [Group C']
   C'1. a C₁₋₆ alkyl group, preferably C₁₋₄ alkyl groups, such as a methyl group, ethyl group, propyl group, isopropyl group and butyl group,
   C'2. an acyl group, for example, an acetyl group, propionyl group, butyryl group and benzoyl group, preferably an acetyl group, propionyl group and butyryl group,
   C'3. a C₁₋₆ alkylsulfonyl group, for example, a methylsulfonyl group, ethylsulfonyl group, propylsulfonyl group, isopropylsulfonyl group and butylsulfonyl group, preferably a methylsulfonyl group and ethylsulfonyl group,
   C'4. a carboxy group,
   C'5. a C₁₋₆ alkoxycarbonyl group, preferably C₁₋₄ alkoxycarbonyl groups, such as a methoxycarbonyl group, ethoxycarbonyl group, propoxycarbonyl group, isopropoxycarbonyl group, butoxycarbonyl group and isobutoxycarbonyl group,
      and
   C'6. -CO- (Alk) n-COOR⁵², wherein R⁵² is preferably a hydrogen atom or a C₁₋₄ alkyl group, Alk is a C₁₋₄ alkylene group, and n is 0 or an integer from 1 to 2,
5'. an aryl group, or an aryl group substituted with one or more, preferably 1 to 3 substituents selected from Group D' below, wherein the aryl group is, for example, a phenyl or naphthyl group, preferably a phenyl group;
   [Group D']
   D'1. a hydroxyl group,
   D'2. a C₁₋₆ alkoxy groups, preferably C₁₋₄ alkoxy groups, such as a methoxy group, ethoxy group, propoxy group, isopropoxy group, butoxy group and isobutoxy group,
   D'3. a cyano group,
   D'4. a C₁₋₆ alkyl group, preferably C₁₋₄ alkyl groups, such as a methyl group, ethyl group, propyl group, isopropyl group, butyl group, isobutyl group and sec-butyl group,
      wherein the C₁₋₆ alkyl group may be substituted with one or more substituents selected from the group consisting of a hydroxyl group, carboxy group and C₁₋₄ alkoxycarbonyl group (for example, a methoxycarbonyl group, ethoxycarbonyl group, propoxycarbonyl group and isopropoxycarbonyl group),
   D'5. -N(R⁵³) (R^{53'}), wherein R⁵³ and R^{53'} are preferably a hydrogen atom, a C₁₋₄ alkyl group or a C₁₋₄ alkylsulfonyl group, and specif ic examples may include an amino group, methylamino group, ethylamino group, dimethylamino group, diethylamino group, methylsulfonylamino group and ethylsulfonylamino group,
   D'6. -CO-N (R⁵³) (R^{53'}), wherein R⁵³ and R^{53'} are preferably a hydrogen atom, a C₁₋₄ alkyl group or a C₁₋₄ alkylsulfonyl group, and specific examples may include carbamoyl group, dimethylcarbamoyl group and diethylcarbamoyl group, preferably dimethylcarbamoyl group, diethylcarbamoyl group, dimethylcarbamoyl group, diethylcarbamoyl group and methylsulfonylcarbamoyl group,
   D'7. -COOR⁵⁴, wherein R⁵⁴ is preferably a hydrogen atom, a C₁₋₄ alkyl group (for example, a methyl group, ethyl group, propyl group, isopropyl group and butyl group), a C₁₋₄ alkylcarbonyloxy C₁₋₄ alkyl group (for example, a methylcarbonyloxymethyl group, ethylcarbonyloxymethyl group and propylcarbonyloxymethyl group) or a C₃₋₆ cycloalkyloxycarbonyloxy C₁₋₄ alkyl group (for example, a cyclohexyloxycarbonyloxymethyl group and cyclohexyloxycarbonyloxyethyl group), most preferably a hydrogen atom or a C₁₋₄ alkyl group,
   D'8. -C(NH(OH))=N-R⁵⁵, wherein R⁵⁵ is preferably a hydrogen atom or a C₁₋₄ alkylsulfonyl group, for example, a methylsulfonyl group,
   D'9. a saturated 5- or 6-memberedmonocyclic heterocyclic group, for example, a pyrrolidinyl group, tetrahydrofuryl group, tetrahydrothienyl group, imidazolidinyl group, pyrazolidinyl group, 1,3-dioxolanyl group, 1,3-oxathiolanyl group, oxazolidinyl group, thiazolidinyl group, piperidyl group, piperazinyl group, tetrahydropyranyl group, tetrahydrothiopyranyl group, dioxanyl group, morpholinyl group, thiomorpholinyl group, 2-oxopyrrolidinyl group, 2-oxopiperidyl group, 4-oxopiperidyl group and 2,6-dioxopiperidyl group, preferably a saturated 5- or 6-membered heterocyclic group having at least one nitrogen atom, such as a pyrrolidinyl group, piperidyl group and morpholinyl group, more preferably the 6-membered saturated heterocyclic groups shown below: 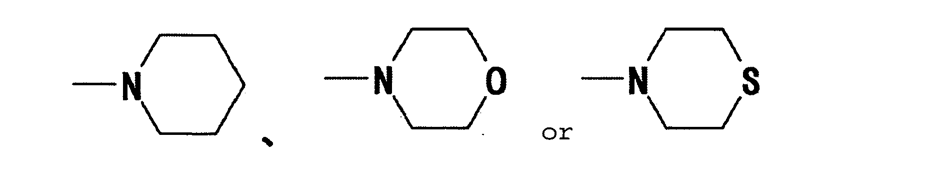 and,
   D'10. a 5- or 6-membered monocyclic heteroaromatic group that may be substituted with an oxo or thioxo group, for example, apyrrolylgroup, furylgroup, thienyl group, imidazolyl group, pyrazolyl group, oxazolyl group, isoxazolyl group, thiazolyl group, isothiazolyl group, 1,2,4-triazolyl group, 1,2,3-triazolyl group, tetrazolyl group, 1,3,4-oxadiazolyl group, 1,2,4-oxadiazolyl group, 1,3,4-thiadiazolyl group, 1,2,4-thiadiazolyl group, furazanyl group, pyridyl group, pyrimidinyl group, pyridazinyl group, pyrazinyl group, 1,3,5-triazinylgroup, imidazolinyl group, pyrazolinyl group, oxazolinyl group (2-oxazolinyl group, 3-oxazolinyl group, 4-oxazolinyl group), isooxazolinyl group, thiazolinyl group, isothiazolinyl group, pyranyl group, 2-oxopyranyl group and 2-oxo-2 , 5-dihydrofuranyl group, preferably a pyrrolyl group, furyl group, thienyl group, imidazolyl group, pyrazolyl group, oxazolyl group, isoxazolyl group, thiazolyl group, isothiazolyl group, pyridyl group and 2-oxo-2,5-dihydrofuranyl group, more preferably the 5-membered heterocyclic groups having at least two nitrogen atoms shown below: 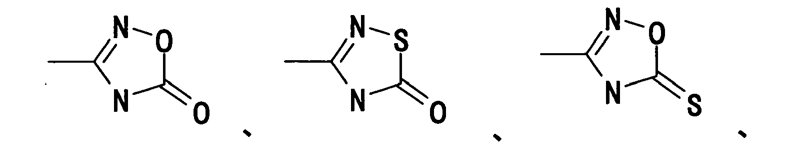 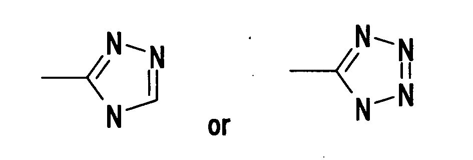 even more preferably the following 5-membered heterocyclic groups: 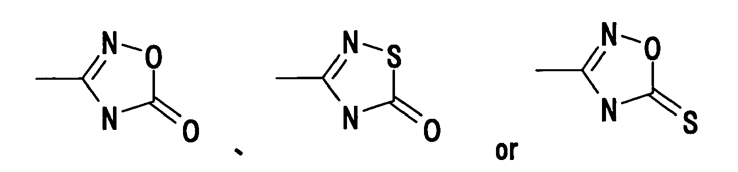 wherein particularly preferred substituents among those in D'1 to D'10 described above are those selected from the group consisting of D'4, D'6, D'7 and D'10, and an unsubstituted phenyl group is also preferred as R⁵,
6'. a 5- or 6-membered monocyclic heteroaromatic group or a condensed ring with a benzene ring that may be substituted with one or more substituents selected from the group consisting of a carboxy group oand a C₁₋₆ alkoxycarbonyl group, wherein the C₁₋₆ alkoxycarbonyl group is preferably a C₁₋₄ alkoxycarbonyl group, such as a methoxycarbonyl group, ethoxycarbonyl group, propoxycarbonyl group, isopropoxycarbonyl group and butoxycarbonyl group, and the heteroaromatic group is, for example, a pyrrolyl group, furyl group, thienyl group, imidazolyl group, pyrazolyl group, oxazolyl group, isoxazolyl group, thiazolyl group, isothiazolyl group, 1,2,4-triazolyl group, 1,2,3-triazolyl group, tetrazolyl group, 1,3,4-oxadiazolyl group, 1,2,4-oxadiazolyl group, 1,3,4-thiadiazolyl group, 1,2,4-thiadiazolyl group, furazanyl group, pyridyl group, pyrimidinyl group, pyridazinyl group, pyrazinyl group, 1,3,5-triazinylgroup, imidazolinyl group, pyrazolinyl group, oxazolinyl group (2-oxazolinyl group, 3-oxazolinyl group, 4-oxazolinyl group), isooxazolinyl group, thiazolinyl group, isothiazolinyl group, pyranyl group, 2-oxopyranyl group and 2-oxo-2,5-dihydrofuranyl group, preferably a pyrrolyl group, furyl group, thienyl group, imidazolyl group, pyrazolyl group, oxazolyl group, isoxazolyl group, thiazolyl group, isothiazolyl group, pyridyl group and 2-oxo-2 , 5-dihydrofuranyl group, preferably a pyrrolyl group, furyl group, thienyl group, imidazolyl group, pyrazolyl group, oxazolyl group, isoxazolyl group, thiazolyl group, isothiazolyl group, 1,2,4-triazolyl group, pyridyl group, pyrimidinyl group, pyridazinyl group, pyrazinyl group, thiazolinyl group, isothiazolinyl group and pyranyl group;
   particularly preferred 5- or 6-membered heteroaromatic groups are shown below: 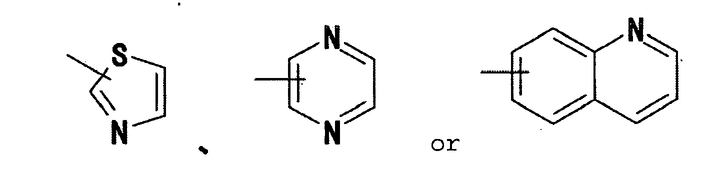
7'. a C₇₋₁₄ aralkyl group, for example, a C₁₋₄ alkyl group substituted with an aromatic hydrocarbon group, such as a benzyl group, naphthylmethyl group, indenylmethyl group, phenanthrenylmethyl group, anthracenylmethyl group, diphenylmethyl group, phenethyl group, naphthylethyl group, phenylpropyl group, naphthylpropyl group, phenylbutyl group and naphthylbutyl group, preferably a benzyl group, naphthylmethyl group, diphenylmethyl group and phenethyl group, more preferably a benzyl group or phenethyl group,
   wherein the alkyl moiety of the C₇₋₁₄ aralkyl group may be unsubstituted or substituted with one or two substituents selected from the following Group E', preferably the alkyl moiety is unsubstituted, or the aryl moiety of the C₇₋₁₄ aralkyl group may be substituted with one or more, preferably 1 to 3 substituents selected from the following Group F',
   [Group E']
   E'1. a C₁₋₄ alkyl group that may be substituted with a hydroxyl group, for example, a methyl group, ethyl group, propyl group, isopropyl group, butyl group, isobutyl group, hydroxymethyl group and hydroxyethyl group,
   E'2. a cyano group,
   E'3. a carboxy group,
   E'4. a C₁₋₆ alkoxycarbonyl group, preferably (C₁₋₄ alkoxy)carbonyl groups, such as a methoxycarbonyl group, ethoxycarbonyl group, propoxycarbonyl group, isopropoxycarbonyl group, butoxycarbonyl group, isobutoxycarbonyl group, sec-butoxycarbonyl group and tert-butoxycarbonyl group,
      and,
   E'5. a phenyl group,
   [Group F']
   F'1. a C₁₋₆ alkyl group, preferably C₁₋₄ alkyl groups, such as a methyl group, ethyl group, propyl group, isopropyl group, butyl group, isobutyl group and sec-butyl group,
   F'2. a halogen atom, for example, a fluorine atom, chlorine atom, bromine atom and iodine atom, preferably a fluorine or chlorine atom,
   F'3. a cyano group,
   F'4. a hydroxyl group,
   F'5. a C₁₋₆ alkoxy group, a C₁₋₆ alkoxy group substituted with a carboxy group or a C₁₋₆ alkoxy group substituted with a C₁₋₆ alkoxycarbonyl group, wherein the C₁₋₆ alkoxy group is preferably C₁₋₄ alkoxy groups, such as a methoxy group, ethoxy group, propoxy group, isopropoxy group, butoxy group and isobutoxy group, and the C₁₋₆ alkoxycarbonyl group in the C₁₋₆ alkoxy group substituted with a C₁₋₆ alkoxycarbonyl group is preferably C₁₋₄ alkoxycarbonyl groups, such as a methoxycarbonyl group, ethoxycarbonyl group, propoxycarbonyl group, isopropoxycarbonyl group and butoxycarbonyl group, F'6. a halo C₁₋₆ alkyl group, for example, a trifluoromethyl group, fluoromethyl group and 2,2,2- trifluoroethyl group, preferably halo C₁₋₄ alkyl groups such as a trifluoromethyl group,
   F'7. a carboxy group,
   F'8. a C₁₋₆ alkoxycarbonyl group, preferably C₁₋₄ alkoxycarbonyl groups, such as a methoxycarbonyl group, ethoxycarbonyl group, propoxycarbonyl group, isopropoxycarbonyl group, butoxycarbonyl group, isobutoxycarbonyl group and sec-butoxycarbonyl group,
   F'9. -CO-N(R^{56a}) (R^{56a'}), wherein R^{56a} and R^{56a'} are identical with or different from each other and represent a hydrogen atom, a saturated 5- or 6-membered monocyclic heterocyclic group having at least one nitrogen atom, such as a piperidino group, morpholino group, pyrrolidino group and piperazino group, or a C₁₋₆ alkyl group that may be substituted with one or more substituents selected from Group f' below, preferably the saturated heterocyclic group is the 6-membered saturated heterocyclic groups shown below: 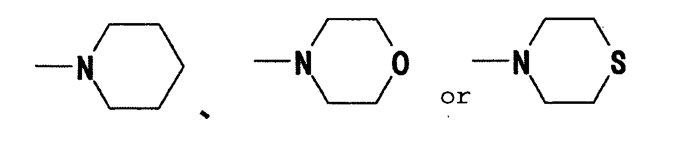 and, the C₁₋₆ alkyl group is preferably C₁₋₄ alkyl groups, such as a methyl group, ethyl group, propyl group, isopropyl group, butyl group, isobutyl group and sec-butyl group,
      [Group f']
      f'1. an amino group,
      f'2. a "mono C₁₋₆ alkylaminogroup", such as a methylamino group, ethylamino group, propylamino group, isopropylamino group, butylamino group, isobutylamino group, sec-butylamino group, tert-butylamino group, pentylamino group, isopentylamino group, 2-methylbutylamino group, neopentylamino group, 1-ethylpropylamino group, hexylamino group, isohexylamino group, 4-methylpentylamino group, 3-methylpentylamino group, 2-methylpentylamino group, 1-methylpentylamino group, 3,3-dimethylbutylamino group, 2,2-dimethylbutylaminogroup, 1,1-dimethylbutylamino group, 1,2-dimethylbutylamino group, 1,3-dimethylbutylamino group, 2,3-dimethylbutylamino group and 2-ethylbutylamino group, preferably a mono-C₁₋₄ alkylamino group,
      f'3. a di C₁₋₆ alkylamino group, such as a dimethylamino group, diethylamino group, N-ethyl-N-methylamino group, dipropylamino group, dibutylamino group, dipentylamino group and dihexylamino group, preferably a di-C₁₋₄ alkylamino group,
      f'4. a carboxy group,
      f'5. a C₁₋₆ alkoxycarbonyl group, preferably C₁₋₄ alkoxycarbonyl groups, such as a methoxycarbonyl group, ethoxycarbonyl group, propoxycarbonyl group, isopropoxycarbonyl group, butoxycarbonyl group and isobutoxycarbonyl group,
      f'6. a hydroxyl group,
         and,
      f'7. a saturated 5- or 6-membered monocyclic heterocyclic group having at least one nitrogen atom, such as a piperidino group, morpholino group, pyrrolidino group and piperazino group,
   F'10. -N(R^{56b}) (R^{56b'}), wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group, the C₁₋₆ alkyl group is preferably C₁₋₄ alkyl groups, such as a methyl group, ethyl group, propyl group, isopropyl group, butyl group, isobutyl group, sec-butyl group and tert-butyl group, the aralkyl group that may be substituted with one or more identical or different substituents selected from the group consisting of an imino group and a halogen atom is, for example, an (imino)(halogenated phenyl)methyl group, the arylsulfonyl group that may be substituted with a C₁₋₆ alkyl group is, for example, a toluenesulfonyl group, and the C₁₋₆ alkyl group in the mono C₁₋₆ alkylcarbamoyl group or the di C₁₋₆ alkylcarbamoyl group is preferably a C₁₋₄ alkyl group, F'11. -N=CR⁵⁷(-N(R⁵⁸) (R^{58'})), wherein R⁵⁷ is preferably a hydrogen atom or a C₁₋₄ alkyl group, R⁵⁸ and R^{58'} are preferably identical with or different from each other and represent a hydrogen atom or a C₁-₄ alkyl group,
   F'12. a 5- or 6-membered monocyclic heteroaromatic group, preferably the group represented by the following formula below: 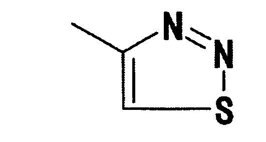 and,
   F'13. a methylenedioxy group bound to the two neighboring carbon atoms of the phenyl ring constituting a C₇₋₁₄ aralkyl group, such as a benzyl or phenethyl group,
      and,
8'. a C₁₋₆ alkyl group substituted with one or more identical or different substituents selected from the group consisting of a monocyclic 5- or 6-membered heteroaromatic group and a condensed 5- or 6-membered heteroaromatic group with a benzene ring, wherein the 5- or 6-membered heteroaromatic groups is, for example, a pyrrolyl group, furyl group, thienyl group, imidazolyl group, pyrazolyl group, oxazolyl group, isoxazolyl group, thiazolyl group, isothiazolyl group, 1,2,4-triazolyl group, 1,2,3-triazolyl group, tetrazolyl group, 1,3,4-oxadiazolyl group, 1,2,4-oxadiazolyl group, 1,3,4-thiadiazolyl group, 1,2,4-thiadiazolyl group, furazanyl group, pyridyl group, pyrimidinyl group, pyridazinyl group, pyrazinyl group, 1,3,5-triazinyl group, imidazolinyl group, pyrazolinyl group, oxazolinyl group (2-oxazolinyl group, 3-oxazolinyl group, 4-oxazolinyl group), isooxazolinyl group, thiazolinyl group, isothiazolinyl group, pyranyl group, 2-oxopyranyl group and 2-oxo-2,5-dihydrofuranyl group, preferably a pyrrolyl group, furyl group, thienyl group, imidazolyl group, pyrazolyl group, oxazolyl group, isoxazolyl group, thiazolyl group, isothiazolyl group, pyridyl group and 2-oxo-2,5-dihydrofuranyl group, wherein the condensed 5- or 6-membered heteroaromatic group with a benzene ring is, for example, a benzofuranyl group, isobenzofuranyl group, benzo[b]thienyl group, indolyl group, isoindolyl group, 1H-indazolyl group, benzimidazolyl group, benzoxazolyl group, benzothiazolyl group, 1H-benzotriazolyl group, quinolyl group, isoquinolyl group, cinnolyl group, quinazolyl group, quinoxalinyl group and phthalazinyl group, more preferably the C₁₋₆ alkyl moiety is a C₁₋₄ alkyl group, such as a methyl group, ethyl group and propyl group, and the heteroaromatic group is a heterocyclic group as shown below: 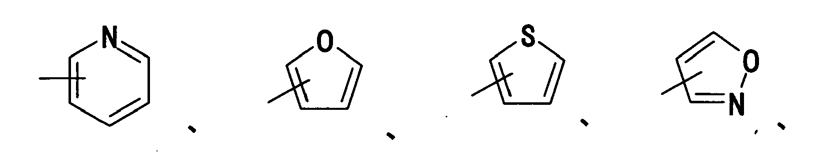 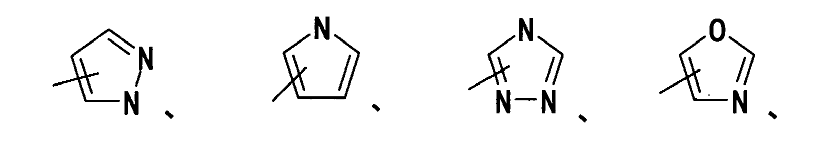 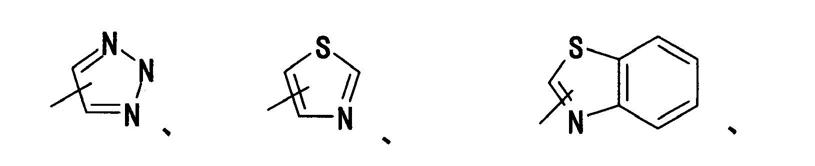 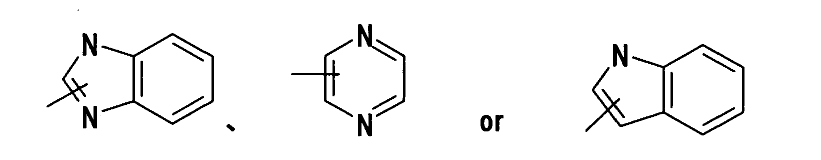 these heteroaromatic groups are particularly preferably, but not limited to, a pyridyl group, oxazolyl group and triazolyl group,
   the heteroaromatic group or the condensed heteroaromatic group may be substituted with one or more, preferably 1 to 3 substituents selected from Group G' below:
   [Group G']
   G'1. a C₁₋₆ alkyl group that may be substituted with one or more substituents selected from Group g' below, preferably a C₁₋₄ alkyl group;
      [Group g']
      g'1. a halogen atom, for example, a fluorine atom or chlorine atom, preferably a fluorine atom,
      g'2. an amino group,
      g'3. a mono C₁₋₆ alkylamino group, preferably a mono-C₁₋₄ alkylaminogroup, suchasamethylaminogroup, ethylaminogroup and propylamino group,
      g'4. a di C₁₋₆ alkylamino group, preferably a di-C₁₋₄ alkylamino group, such as a dimethylamino group and diethylamino group,
      g'5. a C₁₋₆ alkoxycarbonylamino group, for example, a methoxycarbonylamino group, ethoxycarbonylamino group, propoxycarbonyl amino group and isopropoxycarbonyl amino group, preferably a (C₁₋₄ alkoxy)carbonylamino group,
         and,
      g'6. a hydroxyimino group,
   G'2. a halogen atom, for example, a fluorine atom or chlorine atom, preferably a fluorine atom,
   G'3. a C₁₋₆ alkoxy group which may be substituted with a halogen atom, preferably, for example, a C₁₋₄ alkoxy group substituted with one or more fluorine or chlorine atoms, such as a trifluoroethoxy group,
   G'4. an aryloxy group, preferably a phenoxy group,
   G'5. a cyano group,
   G'6. -N (R^{59a}) (R^{59a'}),
      wherein R^{59a} and R^{59a'} are identical with or different from each other and represent a hydrogen atom or a C₁₋₄ alkyl group, or R^{59a} and R^{59a'} may, together with the adjacent nitrogen atom, forma saturated 5- or 6-memberedmonocyclic heterocyclic group, which is preferably pyrrolidino, imidazolidino, pyrazolidino, piperidino, piperazino, morpholinoorthiomorpholino, and the saturated heterocyclic group may be substituted with one or more substituents selected the group consisting of a hydroxyl group, an amino group, a mono C₁₋₄ alkylamino group and a di C₁₋₄ alkylamino group,
   G'7. -CO-N(R^{59b}) (R^{59b'})
      wherein _{R}^{59b} and _{R}^{59b}' are identical with or different from each other, and represent a hydrogen atom, a saturated 5- or 6-memberedmonocyclic heterocyclic group formed together with the adjacent nitrogen atom, such as pyrrolidinyl, imidazolidinyl, pyrazolidinyl, piperidinyl, piperazinyl, morpholinyl and thiomorpholinyl, or a C₁₋₄ alkyl group which may be substituted with the heterocyclic group,
   G'8. an aryl group, for example, preferably a phenyl group, and,
   G'9. a 5- or 6-membered monocyclic heteroaromatic group that may be substituted with an oxo or thioxo group, for example, a pyridyl group, pyridazinyl group, imidazolyl group, pyrimidinyl group, pyrazolyl group, triazolyl group, pyrazinyl group, tetrazolyl group, furyl group, thienyl group, isoxazolyl group, thiazolyl group, oxazolyl group, isothiazolyl group, pyrrolyl group, quinolinyl group, isoquinolinyl group, indolyl group, benzimidazolyl group, benzofuranyl group, cinnolinyl group, indazolyl group, indolizinyl group, phthalazinyl group, pyridazinyl group, triazinyl group, isoindolyl group, purinyl group, oxadiazolyl group, thiazolyl group, thiadiazolyl group, furazanyl group, benzofurazanyl group, benzothiophenyl group, benzotriazolyl group, benzothiazolyl group, benzoxazolyl group, quinazolinyl group, quinoxalinyl group, naphthylizinyl group, dihydroquinolyl group, benzofuranyl group, furopyridinyl group, pyrrolopyrimidinyl group and azaindolyl group, preferably the heteroaromatic groups shown below: 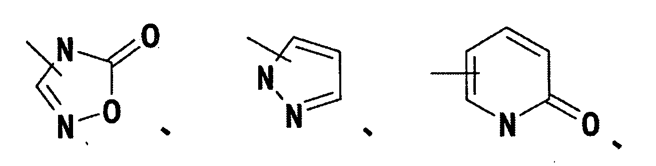 or 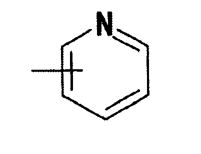 R⁴ and R⁵ may, together with the adjacent nitrogen atom, form a saturated 5- or 6-membered monocyclic heterocyclic ring, wherein the "saturated heterocyclic group" may have a double bond in part, and may be condensed with a benzene ring to form a condensed ring, for example, saturated heterocyclic groups such as pyrrolidino, imidazolidino, pyrazolidino, piperidino, piperazino, morpholino and thiomorpholino, or a 4H-quinazolino group prepared by condensation of the saturated heterocyclic group with a benzene ring, preferably the groups represented by the following formulas: 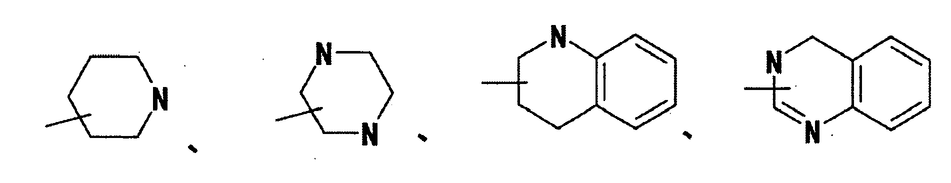 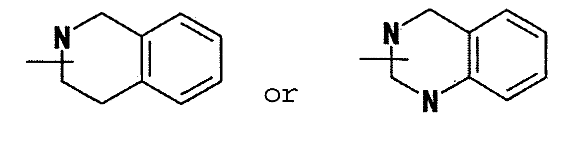 or the saturated heterocyclic group may be substituted with one or moure substituents selected from the group of a halogen atom, a C₁₋₄ alkyl group, a C₁₋₄ alkoxy group, a carboxy group and a C₁₋₄ alkoxycarbonyl group.

Preferred specific examples of the compounds (I) of the present invention are described later in examples. Particularly preferred pyrazole compounds include, but not limited to, the following.
5-(2-Chloro-4,5-difluoro-benzoylamino)-1H-pyrazole-3-carboxylic acid (pyridin-3-ylmethyl)-amide,
5-(2-Chloro-4,5-difluoro-benzoylamino)-1H-pyrazole-3-carboxylic acid (pyridin-3-ylmethyl)-amide dihydrochloride,
5-(2-Chloro-4,5-difluoro-benzoylamino)-1H-pyrazole-3-carboxylic acid (pyridin-3-ylmethyl) -amide hydrochloride,
5-(2-Chloro-4,5-difluoro-benzoylamino)-1H-pyrazole-3-carboxylic acid (4-methyl-oxazol-5-ylmethyl)-amide,
5-(2-Chloro-4,5-difluoro-benzoylamino)-1H-pyrazole-3-carboxylic acid (4-methyl-oxazol-5-ylmethyl)-amide,
5-(2-Chloro-4,5-difluoro-benzoylamino)-1H-pyrazole-3-carboxylic acid (4-methyl-oxazol-5-ylmethyl)amide-p-toluenesulfonate,
5-(2-Chloro-4,5-difluoro-benzoylamino)-1H-pyrazole-3-carboxylic acid (4-methyl-oxazol-5-ylmethyl)amide-L-(+)-tartrate,
5-(2-Chloro-4,5-difluoro-benzoylamino)-1H-pyrazole-3-carboxylic acid (2,4-dimethyl-oxazol-5-ylmethyl)-amide p-toluenesulfonate,
5-(2-Chloro-4,5-difluoro-benzoylamino)=1H-pyrazole-3-carboxylic acid (2,4-dimethyl-oxazol-5-ylmethyl)-amide,
5-(2-Chloro-4,5-difluoro-benzoylamino)-1H-pyrazole-3-carboxylic acid (2,4-dimethyl-pyridin-3-ylmethyl)amide,
5-(2-Chloro-4,5-difluoro-benzoylamino)-1H-pyrazole-3-carboxylic acid diethylamide,
5-(2-Chloro-4,5-difluoro-benzoylamino)-1H-pyrazole-3-carboxylic acid ethyl-(pyridin-4-ylmethyl)-amide,
5-(2-Chloro-4,5-difluoro-benzoylamino)-1H-pyrazole-3-carboxylic acid (2-methoxy-ethyl)-methyl-amide,
5-(2-Chloro-4,5-difluoro-benzoylamino)-1H-pyrazole-3-carboxylic acid ethyl-(2-methoxy-ethyl)-amide,
5-(2-Chloro-4,5-difluoro-benzoylamino)-1H-pyrazole-3-carboxylic acid 2-fluoro-benzylamide,
5-(2-Chloro-4,5-difluoro-benzoylamino)-1H-pyrazole-3-carboxylic acid 3-fluoro-benzylamide,
5-(2-Chloro-4,5-difluoro-benzoylamino)-1H-pyrazole-3-carboxylic acid 2-dimethylamino-benzylamide,
5-(2-Chloro-4,5-difluoro-benzoylamino)-1H-pyrazole-3-carboxylic acid (4-ethoxycarbonyl-cyclohexyl)-propyl-amide,
5-(2-Chloro-4,5-difluoro-benzoylamino)-1H-pyrazole-3-carboxylic acid (6-methoxy-pyridin-3-ylmethyl)-amide,
5-(2-Chloro-4,5-difluoro-benzoylamino)-1H-pyrazole-3-carboxylic acid 2,3-dimethoxy-benzylamide,
5-(2-Chloro-4,5-difluoro-benzoylamino)-1H-pyrazole-3-carboxylic acid 2,4-dimethoxy-benzylamide,
5-(2-Chloro-4,5-difluoro-benzoylamino)-1H-pyrazole-3-carboxylic acid 2,6-dimethoxy-benzylamide,
5-(2-Chloro-4,5-difluoro-benzoylamino)-1H-pyrazole-3-carboxylic acid 3,5-dimethoxy-benzylamide,
5-(2-Chloro-4,5-difluoro-benzoylamino)-1H-pyrazole-3-carboxylic acid 3,4-dimethoxy-benzylamide,
5-(2-Chloro-4,5-difluoro-benzoylamino)-1H-pyrazole-3-carboxylic acid 2,3-difluoro-benzylamide,
5-(2-Chloro-4,5-difluoro-benzoylamino)-1H-pyrazole-3-carboxylic acid 2,4-difluoro-benzylamide,
5-(2-Chloro-4,5-difluoro-benzoylamino)-1H-pyrazole-3-carboxylic acid 2,5-difluoro-benzylamide,
5- (2-Chloro-4,5-difluoro-benzoylamino) -1H-pyrazole-3-carboxylic acid 2,6-difluoro-benzylamide,
5-(2-Chloro-4,5-difluoro-benzoylamino)-1H-pyrazole-3-carboxylic acid 3,4-difluoro-benzylamide,
5-(2-Chloro-4,5-difluoro-benzoylamino)-1H-pyrazole-3-carboxylic acid 3,5-difluoro-benzylamide,
5-(2-Chloro-4,5-difluoro-benzoylamino)-1H-pyrazole-3-carboxylic acid (2-isopropoxy-pyridin-3-ylmethyl)-amide,
5-(2-Chloro-4,5-difluoro-benzoylamino)-1H-pyrazole-3-carboxylic acid (2-phenoxy-pyridin-3-ylmethyl)-amide,
5-(2-Chloro-4,5-difluoro-benzoylamino)-1H-pyrazole-3-carboxylic acid (3, 5-difluoro-pyridin-2-ylmethyl) -amide,
5-(2-Chloro-4,5-difluoro-benzoylamino)-1H-pyrazole-3-carboxylic acid 2-trifluoromethyl-benzylamide,
5-(2-Chloro-4,5-difluoro-benzoylamino)-1H-pyrazole-3-carboxylic acid 3-trifluoromethyl-benzylamide
5-(2-Chloro-4,5-difluoro-benzoylamino)-1H-pyrazole-3-carboxylic acid 4-trifluoromethyl-benzylamide,
5-(2-Chloro-4,5-difluoro-benzoylamino)-1H-pyrazole-3-carboxylic acid 4-tert-butyl-benzylamide,
5-(2-Chloro-4,5-difluoro-benzoylamino)-1H-pyrazole-3-carboxylic acid (6-isopropoxy-pyridin-3-ylmethyl)-amide,
5-(2-Chloro-4,5-difluoro-benzoylamino)-1H-pyrazole-3-carboxylic acid [2-(2,2,2-trifluoro-ethoxy)-pyridin-3-ylmethyl]-amide,
5-(2-Chloro-4,5-difluoro-benzoylamino)-1H-pyrazole-3-carboxylic acid 4-(2-dimethylamino-ethylcarbamoyl)-benzylamide,
5-(2-Chloro-4,5-difluoro-benzoylamino)-1H-pyrazole-3-carboxylic acid 4-ethyl-benzylamide,
5-(2-Chloro-4,5-difluoro-benzoylamino)-1H-pyrazole-3-carboxylic acid 4-isopropyl-benzylamide,
5-(2-Chloro-4,5-difluoro-benzoylamino)-1H-pyrazole-3-carboxylic acid 2-chloro-6-fluoro-benzylamide,
5-(2-Chloro-4,5-difluoro-benzoylamino)-1H-pyrazole-3-carboxylic acid (6-ethoxy-pyridin-3-ylmethyl)-amide dihydrochloride,
5-(2-Chloro-4,5-difluoro-benzoylamino)-1H-pyrazole-3-carboxylic acid (thiazol-4-ylmethyl)-amide,
5-(2-Chloro-4,5-difluoro-benzoylamino)-1H-pyrazole-3-carboxylic acid [6-(2,2,2-trifluoro-ethoxy)-pyridin-3-ylmethyl]-amide dihydrochloride,
5- (2-Chloro-4,5-difluoro-benzoylamino) -1H-pyrazole-3-carboxylic acid 2,6-dimethyl-benzylamide,
5-(2-Chloro-4,5-difluoro-benzoylamino)-1H-pyrazole-3-carboxylic acid [2-(pyridin-3-yl)-thiazol-4-ylmethyl]-amide dihydrochloride,
5-(2-Chloro-4,5-difluoro-benzoylamino)-1H-pyrazole-3-carboxylic acid (1H-benzoimidazol-2-ylmethyl)-amide dihydrochloride, [4-({[5-(2-Chloro-4,5-difluoro-benzoylamino)-1H-pyrazole-3-carbonyl]-amino}-methyl)-benzoylamino]-acetic acid methyl ester, 3-[4-({[5-(2-Chloro-4,5-difluoro-benzoylamino)-1H-pyrazole-3-carbonyl]-amino}-methyl)-benzoylamino]-propionic acid methyl ester
5-(2-Chloro-4,5-difluoro-benzoylamino)-1H-pyrazole-3-carboxylic acid (2-methyl-thiazol-4-ylmethyl)-amide dihydrochloride,
5-(2-Chloro-4,5-difluoro-benzoylamino)-1H-pyrazole-3-carboxylic acid (3,5-dimethyl-isoxazol-4-ylmethyl)-amide,
5-(2-Chloro-4,5-difluoro-benzoylamino)-1H-pyrazole-3-carboxylic acid 4-[2-bis-(2-acetoxyethyl)-amino-ethylcarbamoyl]-benzylamide,
5-(2-Chloro-4,5-difluoro-benzoylamino)-1H-pyrazole-3-carboxylic acid 4-(2-hydroxy-ethylcarbamoyl)-benzylamide,
5-(2-Chloro-4,5-difluoro-benzoylamino)-1H-pyrazole-3-carboxylic acid 4-{2-[(2-acetoxyethyl)-(2-hyroxyethyl)-amino]-ethylcarbamoyl}-benzylamide,
5-(2-Chloro-4,5-difluoro-benzoylamino)-1H-pyrazole-3-carboxylic acid (1-methyl-1H-benzoimidazol-2-ylmethyl)-amide hydrochloride,
5-(2-Chloro-4,5-difluoro-benzoylamino)-1H-pyrazole-3-carboxylic acid (thiazol-2-ylmethyl)-amide dihydrochloride,
5-(2-Chloro-4,5-difluoro-benzoylamino)-1H-pyrazole-3-carboxylic acid (benzothiazol-2-ylmethyl)-amide dihydrochloride,
5-(2-Chloro-4,5-difluoro-benzoylamino)-1H-pyrazole-3-carboxylic acid (2,5-dimethyl-thiazol-4-ylmethyl)-amide dihydrochloride,
5- (2-Chloro-4,5-difluoro-benzoylamino) -1H-pyrazole-3-carboxylic acid [2-(morpholin-4-yl)-thiazol-4-ylmethyl]-amide dihydrochloride,
5-(2-Chloro-4,5-difluoro-benzoylamino)-1H-pyrazole-3-carboxylic acid (1,3,5-trimethyl-1H-pyrazol-4-ylmethyl)-amide,
5-(2-Chloro-4,5-difluoro-benzoylamino)-1H-pyrazole-3-carboxylic acid (2-chloro-6-methyl-pyridin-3-ylmethyl)-amide dihydrochloride,
5-(2-Chloro-4,5-difluoro-benzoylamino)-1H-pyrazole-3-carboxylic acid (2-dimethylamino-thiazol-4-ylmethyl)-amide dihydrochloride,
5-(2-Chloro-4,5-difluoro-benzoylamino)-1H-pyrazole-3-carboxylic acid (1-methyl-1H-pyrrol-2-ylmethyl)-amide,
5-(2-Chloro-4,5-difluoro-benzoylamino)-1H-pyrazole-3-carboxylic acid (3,4-dimethoxy-pyridin-2-ylmethyl)-amide dihydrochloride,
5-(2-Chloro-4,5-difluoro-benzoylamino)-1H-pyrazole-3-carboxylic acid (5-tert-butyl-thiazol-2-ylmethyl)-amide dihydrochloride,
5-(2-Chloro-4,5-difluoro-benzoylamino)-1H-pyrazole-3-carboxylic acid (5-methyl-2-phenyl-2H-[1,2,3]triazol-4-ylmethyl)-amide,
5-(2-Chloro-4,5-difluoro-benzoylamino)-1H-pyrazole-3-carboxylic acid [4-(tert-butoxycarbonyl-amino)-methyl-pyridin-2-ylmethyl]-amide,
5-(2-Chloro-4,5-difluoro-benzoylamino)-1H-pyrazole-3-carboxylic acid [4-methyl-2-(morpholin-4-yl)-thiazol-5-ylmethyl]-amide dihydrochloride,
5-(2-Chloro-4,5-difluoro-benzoylamino)-1H-pyrazole-3-carboxylic acid (pyridin-3-ylmethyl)-amide 3/2hydrochloride hemihydrate,
5-(2-Chloro-4,5-difluoro-benzoylamino)-1H-pyrazole-3-carboxylic acid 2-amino-benzylamide,
5-(2-Chloro-4,5-difluoro-benzoylamino)-1H-pyrazole-3-carboxylic acid 2-methoxy-benzylamide,
5-(2-Chloro-4,5-difluoro-benzoylamino)-1H-pyrazole-3-carboxylic acid 3-methoxy-benzylamide,
5-(2-Chloro-4,5-difluoro-benzoylamino)-1H-pyrazole-3-carboxylic acid 4-methoxy-benzylamide,
5-(2-Chloro-4,5-difluoro-benzoylamino)-1H-pyrazole-3-carboxylic acid 4-methyl-benzylamide,
5-(2-Chloro-4,5-difluoro-benzoylamino)-1H-pyrazole-3-carboxylic acid 4-fluoro-benzylamide,
5-(2-Chloro-4,5-difluoro-benzoylamino)-1H-pyrazole-3-carboxylic acid 4-chloro-benzylamide,
5-(2-Chloro-4,5-difluoro-benzoylamino)-1H-pyrazole-3-carboxylic acid 4-ethoxycarbonyl-benzylamide,
5-(2-Chloro-4,5-difluoro-benzoylamino)-1H-pyrazole-3-carboxylic acid 4-carboxy-benzylamide,
5-(2-Chloro-4,5-difluoro-benzoylamino)-1H-pyrazole-3-carboxylic acid dibenzylamide,
5-(2-Chloro-4,5-difluoro-benzoylamino)-1H-pyrazole-3-carboxylic acid (cyano-phenyl-methyl)-amide,
5-(2-Chloro-4,5-difluoro-benzoylamino)-1H-pyrazole-3-carboxylic acid cyclohexylmethyl-amide,
5-(2-Chloro-4,5-difluoro-benzoylamino)-1H-pyrazole-3-carboxylic acid (2-hydroxy-ethyl)-phenyl-amide,
5-(2-Chloro-4,5-difluoro-benzoylamino)-1H-pyrazole-3-carboxylic acid cyclohexyl-methyl-amide,
5-(2-Chloro-4,5-difluoro-benzoylamino)-1H-pyrazole-3-carboxylic acid cyclohexylamide,
5-(2-Chloro-4,5-difluoro-benzoylamino)-1H-pyrazole-3-carboxylic acid cyclohexyl-ethyl-amide,
5-(2-Chloro-4,5-difluoro-benzoylamino)-1H-pyrazole-3-carboxylic acid allyl-cyclohexyl-amide,
5-(2-Chloro-4,5-difluoro-benzoylamino)-1H-pyrazole-3-carboxylic acid cyclohexyl-(pyridin-2-ylmethyl)-amide,
5-(2-Chloro-4,5-difluoro-benzoylamino)-1H-pyrazole-3-carboxylic acid methyl-(1-methyl-piperidin-4-yl)-amide,
5-(2-Chloro-4,5-difluoro-benzoylamino)-1H-pyrazole-3-carboxylic acid [(3,4-methylenedioxyphenyl)-methyl]-amide hydrochloride,
5-(2-Chloro-4,5-difluoro-benzoylamino)-1H-pyrazole-3-carboxylic acid benzyl-butyl-amide,
5-(2-Chloro-4,5-difluoro-benzoylamino)-1H-pyrazole-3-carboxylic acid benzyl-(4-hydroxy-butyl)-amide hydrochloride,
5-(2-Chloro-4,5-difluoro-benzoylamino)-1H-pyrazole-3-carboxylic acid butyl-ethyl-amide,
5-(2-Chloro-4,5-difluoro-benzoylamino)-1H-pyrazole-3-carboxylic acid cyclohexyl-propyl-amide,
5-(2-Chloro-4,5-difluoro-benzoylamino)-1H-pyrazole-3-carboxylic acid butyl-cyclohexyl-amide,
5-(2-Chloro-4,5-difluoro-benzoylamino)-1H-pyrazole-3-carboxylic acid cyclohexyl-(2-methoxy-ethyl)-amide,
5-(2-Chloro-4,5-difluoro-benzoylamino)-1H-pyrazole-3-carboxylic acid cyclohexyl-(pyridin-3-ylmethyl)-amide,
5-(2-Chloro-4,5-difluoro-benzoylamino)-1H-pyrazole-3-carboxylic acid [2-(morpholin-4-yl)-phenyl]-amide,
5-(2-Chloro-4,5-difluoro-benzoylamino)-1H-pyrazole-3-carboxylic acid dibutylamide,
5-(2-Chloro-4,5-difluoro-benzoylamino)-1H-pyrazole-3-carboxylic acid bis-(2-methoxy-ethyl)-amide,
5-(2-Chloro-4,5-difluoro-benzoylamino)-1H-pyrazole-3-carboxylic acid (2-methoxy-ethyl)-propyl-amide,
5-(2-Chloro-4,5-difluoro-benzoylamino)-1H-pyrazole-3-carboxylic acid butyl-(tetrahydro-pyran-4-yl)-amide,
5-(2-Chloro-4,5-difluoro-benzoylamino)-1H-pyrazole-3-carboxylic acid (2-methoxy-ethyl)-(tetrahydro-pyran-4-yl)-amide,
5-(2-Chloro-4,5-difluoro-benzoylamino)-1H-pyrazole-3-carboxylic acid cyclopentyl-(2-methoxy-ethyl)-amide,
5-(2-Chloro-4,5-difluoro-benzoylamino)-1H-pyrazole-3-carboxylic acid cyclohexyl-(3-methoxy-propyl)-amide,
5-(2-Chloro-4,5-difluoro-benzoylamino)-1H-pyrazole-3-carboxylic acid cyclohexyl-(2-ethoxy-ethyl)-amide,
5- (2-Chloro-4,5-difluoro-benzoylamino) -1H-pyrazole-3-carboxylic acid cyclohexyl-(2-isopropoxy-ethyl)-amide,
5-(2-Chloro-4,5-difluoro-benzoylamino)-1H-pyrazole-3-carboxylic acid cyclohexyl-(2-propoxy-ethyl)-amide,
5-(2-Chloro-4,5-difluoro-benzoylamino)-1H-pyrazole-3-carboxylic acid (1-ethyl-propyl)-(2-methoxy-ethyl)-amide,
5-(2-Chloro-4,5-difluoro-benzoylamino)-1H-pyrazole-3-carboxylic acid butyl-(1-tert-butoxycarbonyl-piperidin-4-yl)-amide,
5-(2-Chloro-4,5-difluoro-benzoylamino)-1H-pyrazole-3-carboxylic acid butyl-(tetrahydro-thiopyran-4-yl)-amide,
5-(2-Chloro-4,5-difluoro-benzoylamino)-1H-pyrazole-3-carboxylic acid (2-methoxy-ethyl)-amide,
5-(2-Chloro-4,5-difluoro-benzoylamino)-1H-pyrazole-3-carboxylic acid butyl-(pyridin-3-ylmethyl)-amide,
5-(2-Chloro-4,5-difluoro-benzoylamino)-1H-pyrazole-3-carboxylic acid benzyl-(2-methoxy-ethyl)-amide,
5-(2-Chloro-4,5-difluoro-benzoylamino)-1H-pyrazole-3-carboxylic acid butyl-(piperidin-4-yl)-amide,
5-(2-Chloro-4,5-difluoro-benzoylamino)-1H-pyrazole-3-carboxylic acid (1-acetyl-piperidin-4-yl)-butyl-amide,
5-(2-Chloro-4,5-difluoro-benzoylamino)-1H-pyrazole-3-carboxylic acid butyl-(1-methanesulfonyl-piperidin-4-yl)-amide,
5-(2-Chloro-4,5-difluoro-benzoylamino)-1H-pyrazole-3-carboxylic acid butyl-(1-ethoxalyl-piperidin-4-yl)-amide,
5-(2-Chloro-4,5-difluoro-benzoylamino)-1H-pyrazole-3-carboxylic acid butyl-(1-oxalo-piperidin-4-yl)-amide,
5-(2-Chloro-4,5-difluoro-benzoylamino)-1H-pyrazole-3-carboxylic acid butyl-(1,1-dioxo-hexahydro-1λ⁶ -thiopyran-4-yl)-amide,
5-(2-Chloro-4,5-difluoro-benzoylamino)-1H-pyrazole-3-carboxylic acid butyl-(1-oxo-hexahydro-1λ⁴-thiopyran-4-yl)-amide,
5-(2-Chloro-4,5-difluoro-benzoylamino)-1H-pyrazole-3-carboxylic acid (6-methoxy-pyridin-3-ylmethyl)-propyl-amide,
5-(2-Chloro-4,5-difluoro-benzoylamino)-1H-pyrazole-3-carboxylic acid propyl-(pyridin-3-ylmethyl)-amide,
5-(2-Chloro-4,5-difluoro-benzoylamino)-1H-pyrazole-3-carboxylic acid 4-diethylcarbamoyl-benzylamide,
5-(2-Chloro-4,5-difluoro-benzoylamino)-1H-pyrazole-3-carboxylic acid 3-diethylcarbamoyl-benzylamide,
5-(2-Chloro-4,5-difluoro-benzoylamino)-1H-pyrazole-3-carboxylic acid 4-ethylcarbamoyl-benzylamide,
5-(2-Chloro-4,5-difluoro-benzoylamino)-1H-pyrazole-3-carboxylic acid 3-ethylcarbamoyl-benzylamide,
5-(2-Chloro-4,5-difluoro-benzoylamino)-1H-pyrazole-3-carboxylic acid 4-carbamoyl-benzylamide,
5-(2-Chloro-4,5-difluoro-benzoylamino)-1H-pyrazole-3-carboxylic acid (1-oxalo-piperidin-4-yl)-propyl-amide,
5-(2-Chloro-4,5-difluoro-benzoylamino)-1H-pyrazole-3-carboxylic acid (1-carboxyacetyl-piperidin-4-yl)-propyl-amide,
5-(2-Chloro-4,5-difluoro-benzoylamino)-1H-pyrazole-3-carboxylic acid [1-(2-carboxypropionyl)-piperidin-4-yl]-propyl-amide,
5-(2-Chloro-4,5-difluoro-benzoylamino)-1H-pyrazole-3-carboxylic acid cyclopropyl-(6-methoxy-pyridin-3-ylmethyl)-amide
5-(2-Chloro-4,5-difluoro-benzoylamino)-1H-pyrazole-3-carboxylic acid cyclobutyl-(6-methoxy-pyridin-3-ylmethyl)-amide,
5-(2-Chloro-4,5-difluoro-benzoylamino)-1H-pyrazole-3-carboxylic acid cyclopropylmethyl-(6-methoxy-pyridin-3-ylmethyl)-amide,
5-(2-Chloro-4,5-difluoro-benzoylamino)-1H-pyrazole-3-carboxylic acid (6-dimethylamino-pyridin-3-ylmethyl)-amide,
5-(2-Chloro-4,5-difluoro-benzoylamino)-1H-pyrazole-3-carboxylic acid (6-dimethylamino-pyridin-3-ylmethyl)-propyl-amide,
5-(2-Chloro-4,5-difluoro-benzoylamino)-1H-pyrazole-3-carboxylic acid benzyl-(2-carboxy-ethyl)-amide,
5-(2-Chloro-4,5-difluoro-benzoylamino)-1H-pyrazole-3-carboxylic acid benzyl-(2-ethoxycarbonyl-ethyl)-amide,
5-(2-Chloro-4,5-difluoro-benzoylamino)-1H-pyrazole-3-carboxylic acid (6-chloro-pyridin-3-ylmethyl)-propyl-amide,
5-(2-Chloro-4,5-difluoro-benzoylamino)-1H-pyrazole-3-carboxylic acid 2,6-dichloro-benzylamide,
5-(2-Chloro-4,5-difluoro-benzoylamino)-1H-pyrazole-3-carboxylic acid (3,5-dichloro-pyridin-4-ylmethyl)-amide,
5-(2-Chloro-4,5-difluoro-benzoylamino)-1H-pyrazole-3-carboxylic acid (3,5-dichloro-pyridin-4-ylmethyl)-propyl-amide,
5-(2-Chloro-4;5-difluoro-benzoylamino)-1H-pyrazole-3-carboxylic acid [6-(1H-pyrazol-1-yl)-pyridin-3-ylmethyl]-amide dihydrochloride,
5-(2-Chloro-4,5-difluoro-benzoylamino)-1H-pyrazole-3-carboxylic acid [6-(4-hydroxy-piperidin-1-yl)-pyridin-3-ylmethyl]-amid e dihydrochloride,
5-(2-Chloro-4,5-difluoro-benzoylamino)-1H-pyrazole-3-carboxylic acid (2-pyridin-2-yl-ethyl)-amide,
5-(2-Chloro-4,5-difluoro-benzoylamino)-1H-pyrazole-3-carboxylic acid (2-pyridin-3-yl-ethyl)-amide,
5-(2-Chloro-4,5-difluoro-benzoylamino)-1H-pyrazole-3-carboxylic acid (2-pyridin-4-yl-ethyl)-amide,
5-(2-Chloro-4,5-difluoro-benzoylamino)-1H-pyrazole-3-carboxylic acid (4-[1,2,3]thiadiazol-4-yl-benzyl)-amide,
5-(2-Chloro-4,5-difluoro-benzoylamino)-1H-pyrazole-3-carboxylic acid (5-methyl-pyrazin-2-ylmethyl)-amide,
5-(2-Chloro-4,5-difluoro-benzoylamino)-1H-pyrazole-3-carboxylic acid pyrazin-2-ylamide,
5-(2-Chloro-4,5-difluoro-benzoylamino)-1H-pyrazole-3-carboxylic acid quinolin-5-ylamide,
5-(2-Chloro-4,5-difluoro-benzoylamino)-1H-pyrazole-3-carboxylic acid quinolin-8-ylamide,
5-(2-Chloro-4,5-difluoro-benzoylamino)-1H-pyrazole-3-carboxylic acid isoquinolin-5-ylamide,
5-(2-Chloro-4,5-difluoro-benzoylamino)-1H-pyrazole-3-carboxylic acid [3-ethoxy-5-(1-ethoxycarbonyl-1-methyl-ethyl)-pyridin-2-yl]-amide,
5-(2-Chloro-4,5-difluoro-benzoylamino)-1H-pyrazole-3-carboxylic acid [3-ethoxy-5-(1-ethoxycarbonyl-1-hydroxy-ethyl)-pyridin-2-yl]-amide,
5-(2-Chloro-4,5-difluoro-benzoylamino)-1H-pyrazole-3-carboxylic acid [5-(1-carboxy-1-methyl-ethyl)-3-ethoxy-pyridin-2-yl]-amide,
5-(2-Chloro-4,5-difluoro-benzoylamino)-1H-pyrazole-3-carboxylic acid
[5-(1-carboxy-1-hydroxy-ethyl)-3-ethoxy-pyridin-2-yl]-amide,
5-(2-Chloro-4,5-difluoro-benzoylamino)-1H-pyrazole-3-carboxylic acid (pyrazin-2-ylmethyl)-amide,
5- (2-Chloro-4,5-difluoro-benzoylamino) -1H-pyrazole-3-carboxylic acid [6-(thiomorpholin-4-yl)-pyridin-3-ylmethyl]-amide,
5-(2-Chloro-4,5-difluoro-benzoylamino)-1H-pyrazole-3-carboxylic acid [6-(1,1-dioxo-1λ⁶-thiomorpholin-4-yl)-pyridin-3-ylmethyl]-amide,
5-(2-Chloro-4,5-difluoro-benzoylamino)-1H-pyrazole-3-carboxylic acid (4,5-dibromo-thiophen-2-ylmethyl)-amide,
5-(2-Chloro-4,5-difluoro-benzoylamino)-1H-pyrazole-3-carboxylic acid (5-chloro-thiophen-2-ylmethyl)-amide,
5-(2-Chloro-4,5-difluoro-benzoylamino)-1H-pyrazole-3-carboxylic acid (4-e thyl-2-methyl-oxazol-5-ylmethyl)-amide,
5-(2-Chloro-4,5-difluoro-benzoylamino)-1H-pyrazole-3-carboxylic acid (2-e thyl-4-methyl-oxazol-5-ylmethyl)-amide dihydrochloride,
5-(2-Chloro-4,5-difluoro-benzoylamino)-1H-pyrazole-3-carboxylic acid ([2,2']bithiophenyl-5-ylmethyl)-amide,
5-(2-Chloro-4,5-difluoro-benzoylamino)-1H-pyrazole-3-carboxylic acid (3-methoxy-thiophen-2-ylmethyl)-amide,
5-(2-Chloro-4,5-difluoro-benzoylamino)-1H-pyrazole-3-carboxylic acid (4,5-Dichloro-thiophen-2-ylmethyl)-amide,
5-(2-Chloro-4,5-difluoro-benzoylamino)-1H-pyrazole-3-carboxylic acid (2,5-dimethyl-oxazol-4-ylmethyl)-amide,
5-(2-Chloro-4,5-difluoro-benzoylamino)-1H-pyrazole-3-carboxylic acid (2-hexyl-4-methyl-oxazol-5-ylmethyl)-amide,
5-(2-Chloro-4,5-difluoro-benzoylamino)-1H-pyrazole-3-carboxylic acid (4-methoxy-thiophen-3-ylmethyl)-amide,
5-(2-Chloro-4,5-difluoro-benzoylamino)-1H-pyrazole-3-carboxylic acid (4-methyl-2-phenyl-oxazol-5-ylmethyl)-amide,
5-(2-Chloro-4,5-difluoro-benzoylamino)-1H-pyrazole-3-carboxylic acid (2-methyl-4-phenyl-oxazol-5-ylmethyl)-amide,
5-(2-Chloro-4,5-difluoro-benzoylamino)-1H-pyrazole-3-carboxylic acid (4-hexyl-2-methyl-oxazol-5-ylmethyl)-amide dihydrochloride,
5-(2-Chloro-4,5-difluoro-benzoylamino)-1H-pyrazole-3-carboxylic acid (6-chloro-pyridin-3-ylmethyl)-amide,
5-(2-Chloro-4,5-difluoro-benzoylamino)-1H-pyrazole-3-carboxylic acid (6-chloro-pyridin-3-ylmethyl)-amide dihydrochloride,
5-(2-Chloro-4,5-difluoro-benzoylamino)-1H-pyrazole-3-carboxylic acid (4H-[1,2,4]triazol-3-ylmethyl)-amide,
5-(2-Chloro-4,5-difluoro-benzoylamino)-1H-pyrazole-3-carboxylic acid (4H-[1,2,4]triazol-3-ylmethyl)-amide dihydrochloride,
5-(2-Chloro-4,5-difluoro-benzoylamino)-1H-pyrazole-3-carboxylic acid (5-fluoro-4H-quinazoline-3-yl)-amide,
5-(2-Chloro-4,5-difluoro-benzoylamino)-1H-pyrazole-3-carboxylic acid (5-fluoro-4H-quinazoline-3-yl)-amide dihydrochloride,
5-(2-Chloro-4,5-difluoro-benzoylamino)-1H-pyrazole-3-carboxylic acid (4,6-dimethyl-pyridin-3-ylmethyl)-amide,
5-(2-Chloro-4,5-difluoro-benzoylamino)-1H-pyrazole-3-carboxylic acid (4,6-dimethyl-pyridin-3-ylmethyl)-amide dihydrochloride,
5-(2-Chloro-4,5-difluoro-benzoylamino)-1H-pyrazole-3-carboxylic acid (2-methoxy-6-methyl-pyridin-3-ylmethyl)-amide,
5-(2-Chloro-4,5-difluoro-benzoylamino)-1H-pyrazole-3-carboxylic acid (4-methoxy-6-methyl-pyridin-3-ylmethyl)-amide,
5-(2-Chloro-4,5-difluoro-benzoylamino)-1H-pyrazole-3-carboxylic acid (4-methoxy-6-methyl-pyridin-3-ylmethyl)-amide dihydrochloride,
5-(2-Chloro-4,5-difluoro-benzoylamino)-1H-pyrazole-3-carboxylic acid (2-methoxy-4,6-dimethyl-pyridin-3-ylmethyl)-amide,
5-(2-Chloro-4,5-difluoro-benzoylamino)-1H-pyrazole-3-carboxylic acid (2-methoxy-4,6-dimethyl-pyridin-3-ylmethyl)-amide dihydrochloride,
5-(2-Chloro-4,5-difluoro-benzoylamino)-1H-pyrazole-3-carboxylic acid (3-methyl-pyridin-2-ylmethyl)-amide,
5-(2-Chloro-4,5-difluoro-benzoylamino)-1H-pyrazole-3-carboxylic acid (5-methyl-pyridin-3-ylmethyl)-amide,
5-(2-Chloro-4,5-difluoro-benzoylamino)-1H-pyrazole-3-carboxylic acid (6-methyl-pyridin-3-ylmethyl)-amide,
5-(2-Chloro-4,5-difluoro-benzoylamino)-1H-pyrazole-3-carboxylic acid (6-methyl-pyridin-2-ylmethyl)-amide,
5-(2,4-Dichloro-5-fluoro-benzoylamino)-1H-pyrazole-3-carboxylic acid (pyridin-3-ylmethyl)-amide dihydrochloride,
5-(2-Chloro-4,5-difluoro-benzoylamino)-1H-pyrazole-3-carboxylic acid (6-fluoro-pyridin-3-ylmethyl)-amide dihydrochloride,
5-(2-Chloro-4,5-difluoro-benzoylamino)-1H-pyrazole-3-carboxylic acid (3-carboxy-phenyl)-methyl-amide,
5-(2-Chloro-4,5-difluoro-benzoylamino)-1H-pyrazole-3-carboxylic acid 3-methanesulfonylamino-benzylamide,
5-(2-Chloro-4,5-difluoro-benzoylamino)-1H-pyrazole-3-carboxylic acid 4-methanesulfonylamino-benzylamide,
5-(2-Chloro-4,5-difluoro-benzoylamino)-1H-pyrazole-3-carboxylic acid 3-acetylamino-benzylamide,
5-(2-Chloro-4,5-difluoro-benzoylamino)-1H-pyrazole-3-carboxylic acid 4-acetylamino-benzylamide,
5-(2-Chloro-4,5-difluoro-benzoylamino)-1H-pyrazole-3-carboxylic acid (2-phenyl-thiazol-4-ylmethyl)-amide,
5-(2-Chloro-4,5-difluoro-benzoylamino)-1H-pyrazole-3-carboxylic acid ((R)-1-phenyl-ethyl)-amide,
5-(2-Chloro-4,5-difluoro-benzoylamino)-1H-pyrazole-3-carboxylic acid ((S)-1-phenyl-ethyl)-amide,
5-(2-Chloro-4,5-difluoro-benzoylamino)-1H-pyrazole-3-carboxylic acid (6-phenoxy-pyridin-3-ylmethyl)-amide dihydrochloride,
5-(2-Methyl-4,5-difluoro-benzoylamino)-1H-pyrazole-3-carboxylic acid (6-chloro-pyridin-3-ylmethyl)-amide dihydrochloride,
5-(2-Chloro-4,5-difluoro-benzoylamino)-1H-pyrazole-3-carboxylic acid (2-chloro-pyridin-3-ylmethyl)-amide dihydrochloride,
5-(2-Methyl-4,5-difluoro-benzoylamino)-1H-pyrazole-3-carboxylic acid (6-chloro-pyridin-3-ylmethyl)-amide sodium salt,
5-(2-Chloro-4,5-difluoro-benzoylamino)-1H-pyrazole-3-carboxylic acid [1-(pyridin-3-yl)-ethyl]-amide dihydrochloride ,
5- (2-Chloro-4,5-difluoro-benzoylamino) -1H-pyrazole-3-carboxylic acid (2-fluoro-pyridin-3-ylmethyl)-amide dihydrochloride,
5-(2-Chloro-4,5-difluoro-benzoylamino)-1H-pyrazole-3-carboxylic acid (2-methyl-pyridin-3-ylmethyl)-amide dihydrochloride,
5-(2-Chloro-4,5-difluoro-benzoylamino)-1H-pyrazole-3-carboxylic acid (biphenyl-3-ylmethyl)-amide,
5-(2-Chloro-4,5-difluoro-benzoylamino)-1H-pyrazole-3-carboxylic acid ((1R,2S)-2-hydroxy-indan-1-yl)-amide,
5-(2-Chloro-4,5-difluoro-benzoylamino)-1H-pyrazole-3-carboxylic acid ((1S,2R)-2-hydroxy-indan-1-yl)-amide,
5-(2-Chloro-4,5-difluoro-benzoylamino)-1H-pyrazole-3-carboxylic acid (6-phenyl-pyridin-3-ylmethyl)-amide dihydrochloride,
5-(2-Chloro-4,5-difluoro-benzoylamino)-1H-pyrazole-3-carboxylic acid (6-methyl-2-pyridon-3-ylmethyl)-amide hydrochloride,
5-(2-Chloro-4,5-difluoro-benzoylamino)-1H-pyrazole-3-carboxylic acid benzyl-methyl-amide,
5-(2-Chloro-4,5-difluoro-benzoylamino)-1H-pyrazole-3-carboxylic acid benzylamide ,
5-(2-Chloro-4,5-difluoro-benzoylamino)-1H-pyrazole-3-carboxylic acid phenylamide,
5-(2-Chloro-4,5-difluoro-benzoylamino)-1H-pyrazole-3-carboxylic acid methyl-phenyl-amide,
5-(2-Chloro-4,5-difluoro-benzoylamino)-1H-pyrazole-3-carboxylic acid (piperidin-1-yl)-amide,
5-(2-Chloro-4,5-difluoro-benzoylamino)-1H-pyrazole-3-carboxylic acid (4-ethoxycarbonyl-piperidin-1-yl)-amide,
5-(2-Chloro-4,5-difluoro-benzoylamino)-1H-pyrazole-3-carboxylic acid (4-methyl-piperazin-1-yl)-amide,
5-(2-Chloro-4,5-difluoro-benzoylamino)-1H-pyrazole-3-carboxylic acid (4-carboxy-piperidin-1-yl)-amide,
5-(2-Chloro-4,5-difluoro-benzoylamino)-1H-pyrazole-3-carboxylic acid phenethylamide,
5-(2-Chloro-4,5-difluoro-benzoylamino)-1H-pyrazole-3-carboxylic acid methyl-phenethyl-amide,
5-(2-Chloro-4,5-difluoro-benzoylamino)-1H-pyrazole-3-carboxylic acid ethyl-phenyl-amide,
5-(2-Chloro-4,5-difluoro-benzoylamino)-1H-pyrazole-3-carboxylic acid (1,2,3,4-tetrahydroisoquinolin-2-yl)-amide,
5-(2-Chloro-4,5-difluoro-benzoylamino)-1H-pyrazole-3-carboxylic acid ((S)-α-methoxycarbonyl-benzyl)-amide,
5-(2-Chloro-4,5-difluoro-benzoylamino)-1H-pyrazole-3-carboxylic acid ((R)-α-methoxycarbonyl-benzyl)-amide,
5-(2-Chloro-4,5-difluoro-benzoylamino)-1H-pyrazole-3-carboxylic acid (1,2,3,4-tetrahydroquinoline-1-yl)-amide,
5-(2-Chloro-4,5-difluoro-benzoylamino)-1H-pyrazole-3-carboxylic acid phenyl-propyl-amide,
5-(2-Chloro-4,5-difluoro-benzoylamino)-1H-pyrazole-3-carboxylic acid butyl-phenyl-amide,
5-(2-Chloro-4,5-difluoro-benzoylamino)-1H-pyrazole-3-carboxylic acid pentyl-phenyl-amide,
5-(2-Chloro-4,5-difluoro-benzoylamino)-1H-pyrazole-3-carboxylic acid benzhydryl-amide,
5-(2-Chloro-4,5-difluoro-benzoylamino)-1H-pyrazole-3-carboxylic acid (pyridin-2-ylmethyl)-amide hydrochloride,
5-(2-Chloro-4,5-difluoro-benzoylamino)-1H-pyrazole-3-carboxylic acid (pyridin-4-ylmethyl)-amide hydrochloride,
5-(2-Chloro-4,5-difluoro-benzoylamino)-1H-pyrazole-3-carboxylic acid ((R)-2-hydroxy-1-phenyl-ethyl)-amide,
5-(2-Chloro-4,5-difluoro-benzoylamino)-1H-pyrazole-3-carboxylic acid ((S)-2-hydroxy-1-phenyl-ethyl)-amide,
5-(2-Chloro-4,5-difluoro-benzoylamino)-1H-pyrazole-3-carboxylic acid 4-dimethylamino-benzylamide,
5-(2-Chloro-4,5-difluoro-benzoylamino)-1H-pyrazole-3-carboxylic acid (furan-2-ylmethyl)-amide,
5-(2-Chloro-4,5-difluoro-benzoylamino)-1H-pyrazole-3-carboxylic acid (thiophen-2-ylmethyl)-amide,
5-(2-Chloro-4,5-difluoro-benzoylamino)-1H-pyrazole-3-carboxylic acid (2-methoxy-phenyl)-amide,
5-(2-Chloro-4,5-difluoro-benzoylamino)-1H-pyrazole-3-carboxylic acid (2-methoxy-phenyl)-methyl-amide,
5-(2-Chloro-4,5-difluoro-benzoylamino)-1H-pyrazole-3-carboxylic acid 4-dimethylamino-benzylamide hydrochloride,
5-(2-Chloro-4,5-difluoro-benzoylamino)-1H-pyrazole-3-carboxylic acid 3-amino-benzylamide,
5-(2-Chloro-4,5-difluoro-benzoylamino)-1H-pyrazole-3-carboxylic acid 4-amino-benzylamide,
5-(2-Chloro-4,5-difluoro-benzoylamino)-1H-pyrazole-3-carboxylic acid 2-methyl-benzylamide,
5-(2-Chloro-4,5-difluoro-benzoylamino)-1H-pyrazole-3-carboxylic acid 3-methyl-benzylamide,
5-(2-Chloro-4,5-difluoro-benzoylamino)-1H-pyrazole-3-carboxylic acid 3-dimethylamino-benzylamide,
5-(2-Chloro-4,5-difluoro-benzoylamino)-1H-pyrazole-3-carboxylic acid (2-methoxy-pyridin-3-ylmethyl)-amide,
5-(2-Chloro-4,5-difluoro-benzoylamino)-1H-pyrazole-3-carboxylic acid 2-chloro-benzylamide,
5-(2-Chloro-4,5-difluoro-benzoylamino)-1H-pyrazole-3-carboxylic acid 3-chloro-benzylamide,
5-(2-Chloro-4,5-difluoro-benzoylamino)-1H-pyrazole-3-carboxylic acid 3-methoxycarbonyl-benzylamide,
5-(2-Chloro-4,5-difluoro-benzoylamino)-1H-pyrazole-3-carboxylic acid 3-carboxy-benzylamide,
5-(2-Chloro-4,5-difluoro-benzoylamino)-1H-pyrazole-3-carboxylic acid (6-trifluoromethyl-pyridin-3-ylmethyl)-amide,
5-(2-Chloro-4,5-difluoro-benzoylamino)-1H-pyrazole-3-carboxylic acid (2-ethoxy-pyridin-3-ylmethyl)-amide,
5-(2-Chloro-4,5-difluoro-benzoylamino)-1H-pyrazole-3-carboxylic acid (2-dimethylamino-ethyl)-amide,
5-(2-Chloro-4,5-difluoro-benzoylamino)-1H-pyrazole-3-carboxylic acid ethyl-(2-dimethylamino-ethyl)-amide dihydrochloride,
5-(2-Chloro-4,5-difluoro-benzoylamino)-1H-pyrazole-3-carboxylic acid cyclohexyl-(2-dimethylamino-ethyl)-amide,
5-(2-Chloro-4,5-difluoro-benzoylamino)-1H-pyrazole-3-carboxylic acid (4-methoxy-pyridin-2-ylmethyl)-amide,
5-(2-Chloro-4,5-difluoro-benzoylamino)-1H-pyrazole-3-carboxylic acid (2,6-dimethoxy-pyridin-3-ylmethyl)-amide,
5-(2-Chloro-4,5-difluoro-benzoylamino)-1H-pyrazole-3-carboxylic acid 2-methoxycarbonylmethoxy-benzylamide,
5-(2-Chloro-4,5-difluoro-benzoylamino)-1H-pyrazole-3-carboxylic acid 2-carboxymethoy-benzylamide,
5-(4,5-Difluoro-2-methyl-benzoylamino)-1H-pyrazole-3-carboxylic acid (6-methoxy-pyridin-3-ylmethyl)-amide,
5-(2,4-Dichloro-benzoylamino)-1H-pyrazole-3-carboxylic acid (6-methoxy-pyridin-3-ylmethyl)-amide,
5-(2-Chloro-4,5-difluoro-benzoylamino)-1H-pyrazole-3-carboxylic acid (3,5-dimethoxy-pyridin-4-ylmethyl)-amide,
5-(4,5-Difluoro-2-methyl-benzoylamino)-1H-pyrazole-3-carboxylic acid (2,4-dimethyl-oxazol-5-ylmethyl)-amide,
5-(2,4-Dichloro-5-fluoro-benzoylamino)-1H-pyrazole-3-carboxylic acid (2,4-dimethyl-oxazol-5-ylmethyl)-amide,
5-(2-Chloro-4,5-difluorobenzoylamino)-1H-pyrazole-3-carbocylic acid (4-methoxycarbonyl-phenyl)-methyl-amide,
5-(2-Chloro-4,5-difluoro-benzoylamino)-1H-pyrazole-3-carboxylic acid (4-carboxyphenyl)-methyl-amide, 4-{[5-(2-Chloro-4,5-difluoro-benzoylamino)-1H-pyrazole -3-carbonyl]-methyl-amino}-benzoic acid sodium salt,
5-(2-Chloro-4,5-difluoro-benzoylamino)-1H-pyrazole-3-carboxylic acid isopropyl-phenyl-amide,
5-(2-Chloro-4,5-difluoro-benzoylamino)-1H-pyrazole-3-carboxylic acid (ethoxycarbonyl-methyl)-phenyl-amide,
5-(2-Chloro-4,5-difluoro-benzoylamino)-1H-pyrazole-3-carboxylic acid (carboxy-methyl)-phenyl-amide,
5-(2-Chloro-4-fluoro-benzoylamino)-1H-pyrazole-3-carboxylic acid benzylamide,
5-(2,4-Dichloro-benzoylamino)-1H-pyrazole-3-carboxylic acid benzylamide,
5-(2-Chloro-5-fluoro-benzoylamino)-1H-pyrazole-3-carboxylic acid benzylamide,
5-(2-Chloro-4,5-difluoro-benzoylamino)-1H-pyrazole-3-carboxylic acid benzyl-phenyl-amide,
5-(2-Chloro-4,5-difluoro-benzoylamino)-1H-pyrazole-3-carboxylic acid (1-methyl-1-phenyl-ethyl) -amide,
5-(2-Chloro-4,5-difluoro-benzoylamino)-1H-pyrazole-3-carboxylic acid [2-oxo-2-(piperidin-1-yl)-ethyl]-phenyl-amide,
5-(5-Fluoro-2-methyl-benzoylamino)-1H-pyrazole-3-carboxylic acid benzylamide,
5-(5-Chloro-2-methyl-benzoylamino)-1H-pyrazole-3-carboxylic acid benzylamide,
5-(4-Methoxy-2-methyl-benzoylamino)-1H-pyrazole-3-carboxylic acid benzylamide,
5-(4-Chloro-2-methyl-benzoylamino)-1H-pyrazole-3-carboxylic acid benzylamide,
5-(5-Fluoro-2-methoxy-benzoylamino)-1H-pyrazole-3-carboxylic acid benzylamide,
5-(5-Chloro-2-methoxy-benzoylamino)-1H-pyrazole-3-carboxylic acid benzylamide,
5-(2-Chloro-4,5-difluoro-benzoylamino)-1H-pyrazole-3-carboxylic acid (2-ethoxy-ethyl)-phenyl-amide,
5-(2-Fluoro-5-methyl-benzoylamino)-1H-pyrazole-3-carboxylic acid benzylamide,
5-(5-Chloro-2-fluoro-benzoylamino)-1H-pyrazole-3-carboxylic acid benzylamide,
5-(3-Chloro-2,6-difluoro-benzoylamino)-1H-pyrazole-3-carboxylic acid benzylamide,
5-(2-Chloro-3-fluoro-benzoylamino)-1H-pyrazole-3-carboxylic acid benzylamide,
5-(2-Chloro-4,5-difluoro-benzoylamino)-1H-pyrazole-3-carboxylic acid ethylcarbamoylmethyl-phenyl-amide,
5-(2-Chloro-4,5-difluoro-benzoylamino)-1H-pyrazole-3-carboxylic acid isobutyl-phenyl-amide,
5-(2-Chloro-4,5-difluoro-benzoylamino)-1H-pyrazole-3-carboxylic acid (2-methoxycarbonyl-ethyl)-phenyl-amide,
5-(2-Chloro-4,5-difluoro-benzoylamino)-1H-pyrazole-3-carboxylic acid (3-ethoxycarbonyl-propyl)-phenyl-amide,
5-(2-Chloro-4,5-difluoro-benzoylamino)-1H-pyrazole-3-carboxylic acid (2-carboxy-ethyl)-phenyl-amide,
5-(2-Chloro-4,5-difluoro-benzoylamino)-1H-pyrazole-3-carboxylic acid (3-carboxy-propyl)-phenyl-amide,
5-(2-Hydroxy-4-methoxy-benzoylamino)-1H-pyrazole-3-carboxylic acid benzylamide,
5-(2-Hydroxy-5-methoxy-benzoylamino)-1H-pyrazole-3-carboxylic acid benzylamide,
5-(5-Fluoro-2-hydroxy-benzoylamino)-1H-pyrazole-3-carboxylic acid benzylamide,
5-(4-Fluoro-2-trifluoromethyl-benzoylamino)-1H-pyrazole-3-carboxylic acid benzylamide,
5-(5-Fluoro-2-trifluoromethyl-benzoylamino)-1H-pyrazole-3-carboxylic acid benzylamide,
5-(2-Chloro-4,5-difluoro-benzoylamino)-1H-pyrazole-3-carboxylic acid (2-methoxy-ethyl)-phenyl-amide,
5-(2,5-Dimethyl-benzoylamino)-1H-pyrazole-3-carboxylic acid benzylamide,
5-(2-Chloro-5-methyl-benzoylamino)-1H-pyrazole-3-carboxylic acid benzylamide,
5-(2-Chloro-pyridine-3-carbonylamino)-1H-pyrazole-3-carboxylic acid benzylamide,
5-(4-Chloro-pyridine-2-carbonylamino)-1H-pyrazole-3-carboxylic acid benzylamide,
5-(2,6-Dichloro-pyridine-3-carbonylamino)-1H-pyrazole-3-carboxylic acid benzylamide,
5-(2-Chloro-4,5-difluoro-benzoylamino)-1H-pyrazole-3-carboxylic acid butyl-(4-ethoxycarbonyl-phenyl)-amide,
5-(2-Chloro-4,5-difluoro-benzoylamino)-1H-pyrazole-3-carboxylic acid butyl-(4-carboxy-phenyl)-amide,
5-(2-Methyl-pyridine-3-carbonylamino)-1H-pyrazole-3-carboxylic acid benzylamide,
5- (2-Chloro-6-methyl-pyridine-3-carbonylamino) -1H-pyrazole-3-carboxylic acid benzylamide,
5-(3-Chloro-pyridine-4-carbonylamino)-1H-pyrazole-3-carboxylic acid benzylamide,
5-(2-Chloro-4,5-difluoro-benzoylamino)-1H-pyrazole-3-carboxylic acid butyl-(4-ethoxycarbonylmethyl-phenyl)-amide,
5-(2-Chloro-4,5-difluoro-benzoylamino)-1H-pyrazole-3-carboxylic acid butyl-(4-carboxymethyl-phenyl)-amide,
5-(2-Chloro-4,5-difluoro-benzoylamino)-1H-pyrazole-3-carboxylic acid (4-methoxycarbonyl-phenyl)-(2-methoxy-ethyl)-amide,
5-(2-Chloro-4,5-difluoro-benzoylamino)-1H-pyrazole-3-carboxylic acid (4-carboxy-phenyl)-(2-methoxy-ethyl)-amide,
5-(2-Chloro-4,5-difluoro-benzoylamino)-1H-pyrazole-3-carboxylic acid (6-methoxy-pyridin-3-ylmethyl)-amide dihydrochloride,
5-(2-Methyl-4,5-difluoro-benzoylamino)-1H-pyrazole-3-carboxylic acid (6-methoxy-pyridin-3-ylmethyl)-amide dihydrochloride,
5-(2-Chloro-4,5-difluoro-benzoylamino)-1H-pyrazole-3-carboxylic acid (thiazol-4-ylmethyl)-amide dihydrochloride,
5- (2-Chloro-4,5-difluoro-benzoylamino) -1H-pyrazole-3-carboxylic acid 4-[2-(morpholin-4-yl)-ethylcarbamoyl]-benzylamide dihydrochloride,
5-(2-Chloro-4,5-difluoro-benzoylamino)-1H-pyrazole-3-carboxylic acid 4-(morpholin-4-ylcarbamoyl)-benzylamide dihydrochloride, [4-({[5-(2-Chloro-4,5-difluoro-benzoylamino)-1H-pyrazole-3-carbonyl]-amino}-methyl)-benzoylamino]-acetic acid sodium salt, 3-[4-({[5-(2-Chloro-4,5-difluoro-benzoylamino)-1H-pyrazole-3-carbonyl]-amino}-methyl)-benzoylamino]-propionic acid sodium salt,
5-(2-Chloro-4,5-difluoro-benzoylamino)-1H-pyrazole-3-carboxylic acid (3,5-dimethyl-isoxazol-4-ylmethyl)-amide hydrochloride,
5-(2-Chloro-4,5-difluoro-benzoylamino)-1H-pyrazole-3-carboxylic acid (1,3,5-trimethyl-1H-pyrazol-4-ylmethyl)-amide dihydrochloride,
5-(2-Chloro-4,5-difluoro-benzoylamino)-1H-pyrazole-3-carboxylic acid (3,5-dimethyl-isoxazol-4-ylmethyl)-amide methanesulfonate,
5-(2-Chloro-4,5-difluoro-benzoylamino)-1H-pyrazole-3-carboxylic acid 3-[2-(morpholin-4-yl)-ethylcarbamoyl]-benzylamide dihydrochloride,
5-(2-Chloro-4,5-difluoro-benzoylamino)-1H-pyrazole-3-carboxylic acid 3-(morpholin-4-ylcarbamoyl)-benzylamide dihydrochloride,
5-(2-Chloro-4,5-difluoro-benzoylamino)-1H-pyrazole-3-carboxylic acid {2-[2-(morpholin-4-yl)-ethylcarbamoyl]-pyridin-4-ylmethyl}-amide trihydrochloride,
5-(2-Chloro-4,5-difluoro-benzoylamino)-1H-pyrazole-3-carboxylic acid [2-(morpholin-4-ylcarbamoyl)-pyridin-4-ylmethyl]-amide trihydrochloride,
5-(2-Chloro-4,5-difluoro-benzoylamino)-1H-pyrazole-3-carboxylic acid (6-methoxy-pyridin-3-ylmethyl)-amide methanesulfonate,
5-(2-Chloro-4,5-difluoro-benzoylamino)-1H-pyrazole-3-carboxylic acid (6-methoxy-pyridin-3-ylmethyl)-amide hemisulfate
5-(2-Chloro-4,5-difluoro-benzoylamino)-1H-pyrazole-3-carboxylic acid (3,5-dimethoxy-pyridin-4-ylmethyl)-amide dihydrochloride,
5-(2-Chloro-4,5-difluoro-benzoylamino)-1H-pyrazole-3-carboxylic acid (4-aminomethyl-pyridin-2-ylmethyl)-amide trihydrochloride,
5-(2-Chloro-4,5-difluoro-benzoylamino)-1H-pyrazole-3-carboxylic acid (6-chloro-pyridin-3-ylmethyl)-amide methanesulfonate,
5-(2-Chloro-4,5-difluoro-benzoylamino)-1H-pyrazole-3-carboxylic acid (6-chloro-pyridin-3-ylmethyl)-amide dimethanesulfonate,
5-(2-Chloro-4,5-difluoro-benzoylamino)-1H-pyrazole-3-carboxylic acid (2,4-dimethyl-pyridin-3-ylmethyl)-amide hydrochloride,
5-(2-Chloro-4,5-difluoro-benzoylamino)-1H-pyrazole-3-carboxylic acid (pyridin-3-ylmethyl) -amide hydrochloride,
5-(2-Chloro-4,5-difluoro-benzoylamino)-1H-pyrazole-3-carboxylic acid (6-methoxy-pyridin-3-ylmethyl)-propyl-amide dihydrochloride,
5-(2-Chloro-4,5-difluoro-benzoylamino)-1H-pyrazole-3-carboxylic acid [6-(piperidin-1-yl)-pyridin-3-ylmethyl]-amide dihydrochloride,
5-(2-Chloro-4,5-difluoro-benzoylamino)-1H-pyrazole-3-carboxylic acid [6-(morpholin-4-yl)-pyridin-3-ylmethyl]-amide dihydrochloride,
5- (2-Chloro-4,5-difluoro-benzoylamino) -1H-pyrazole-3-carboxylic acid (3,5-dichloro-pyridin-4-ylmethyl)-amide hydrochloride,
5-(2-Chloro-4,5-difluoro-benzoylamino)-1H-pyrazole-3-carboxylic acid (6-dimethylamino-pyridin-3-ylmethyl)-amide dihydrochloride,
5-(2-Chloro-4,5-difluoro-benzoylamino)-1H-pyrazole-3-carboxylic acid (6-dimethylamino-pyridin-3-ylmethyl)-propyl-amide dihydrochloride,
5-(2-Chloro-4,5-difluoro-benzoylamino)-1H-pyrazole-3-carboxylic acid (6-diethylamino-pyridin-3-ylmethyl)-amide dihydrochloride,
5- (2-Chloro-4,5-difluoro-benzoylamino) -1H-pyrazole-3-carboxylic acid (6-chloro-2-dimethylamino-pyridin-3-ylmethyl)-amide dihydrochloride,
5-(2-Chloro-4,5-difluoro-benzoylamino)-1H-pyrazole-3-carboxylic acid (4-[1,2,3]thiadiazol-4-yl-benzyl)-amide hydrochloride,
5-(2-Chloro-4,5-difluoro-benzoylamino)-1H-pyrazole-3-carboxylic acid (4-methyl-thiazol-5-ylmethyl)-amide hydrochloride,
5-(2-Chloro-4,5-difluoro-benzoylamino)-1H-pyrazole-3-carboxylic acid (2,4-dimethyl-thiazol-5-ylmethyl)-amide hydrochloride,
5-(2-Chloro-4,5-difluoro-benzoylamino)-1H-pyrazole-3-carboxylic acid [6-(4-hydroxy-piperidin-1-yl)-pyridin-3-ylmethyl]-amid e dihydrochloride,
5-(2-Chloro-4,5-difluoro-benzoylamino)-1H-pyrazole-3-carboxylic acid 5-methyl-pyrazin-2-ylamide dihydrochloride,
5-(2-Chloro-4,5-difluoro-benzoylamino)-1H-pyrazole-3-carboxylic acid quinolin-5-ylamide dihydrochloride,
5-(2-Chloro-4,5-difluoro-benzoylamino)-1H-pyrazole-3-carboxylic acid quinolin-8-ylamide dihydrochloride,
5-(2-Chloro-4,5-difluoro-benzoylamino)-1H-pyrazole-3-carboxylic acid isoquinolin-5-ylamide dihydrochloride,
5-(2-Chloro-4,5-difluoro-benzoylamino)-1H-pyrazole-3-carboxylic acid [6-(4-methylpiperazin-1-yl)-pyridin-3-ylmethyl]-amide dihydrochloride,
5-(2-Chloro-4,5-difluoro-benzoylamino)-1H-pyrazole-3-carboxylic acid [6-(4-dimethylamino-piperidin-1-yl)-pyridin-3-ylmethyl]-amide dihydrochloride,
5-(2-Chloro-4,5-difluoro-benzoylamino)-1H-pyrazole-3-carboxylic acid [6-(pyrrolidin-1-yl)-pyridin-3-ylmethyl] -amide dihydrochloride,
5-(2-Chloro-4,5-difluoro-benzoylamino)-1H-pyrazole-3-carboxylic acid (pyrazin-2-ylmethyl)-amide dihydrochloride,
5-(2-Chloro-4,5-difluoro-benzoylamino)-1H-pyrazole-3-carboxylic acid [6-(thiomorpholin-4-yl)-pyridin-3-ylmethyl]-amide dihydrochloride,
5-(2-Chloro-4,5-difluoro-benzoylamino)-1H-pyrazole-3-carboxylic acid [6-(1,1-dioxo-116-thiomorpholin-4-yl)-pyridin-3-ylmethyl]-amide dihydrochloride,
5-(2-Chloro-4,5-difluoro-benzoylamino)-1H-pyrazole-3-carboxylic acid carbamoylmethyl-amide,
5-(2,4-Dichloro-5-fluoro-benzoylamino)-1H-pyrazole-3-carboxylic acid (4-carbamoyl-phenyl)-methyl-amide,
5-(2,4-Dichloro-5-fluoro-benzoylamino)-1H-pyrazole-3-carboxylic acid methyl-(4-methylcarbamoyl-phenyl)-amide,
5- (2,4-Dichloro-5-fluoro-benzoylamino) -1H-pyrazole-3-carboxylic acid (4-dimethylcarbamoyl-phenyl)-methyl-amide,
5-(2-Chloro-4,5-difluoro-benzoylamino)-1H-pyrazole-3-carboxylic acid (4-methanesulfonylaminocarbonyl-phenyl)-methyl-amide,
5-(2-Chloro-4,5-difluoro-benzoylamino)-1H-pyrazole-3-carboxylic acid (4-methanesulfonylaminocarbonyl-phenyl)-methyl-amide sodium salt,
5-(2-Chloro-4,5-difluoro-benzoylamino)-1H-pyrazole-3-carboxylic acid (4-cyano-phenyl)-methyl-amide,
5-(2-Chloro-4,5-difluoro-benzoylamino)-1H-pyrazole-3-carboxylic acid methyl-[4-(4H-[1,2,4]triazol-3-yl)-phenyl]-amide hydrochloride,
5-(4-Azido-2-chloro-5-fluoro-benzoylamino)-1H-pyrazole-3-carboxylic acid (4-cyano-phenyl)-methyl-amide, 5-(4-Azido-2-chloro-5-fluoro-benzoylamino)-1H-pyrazole -3-carboxylic acid methyl-[4-(1H-tetrazol-5-yl)-phenyl]-amide,
5-(2-Chloro-4,5-difluoro-benzoylamino)-1H-pyrazole-3-carboxylic acid [4-(N-hydroxycarbamimidoyl)-phenyl]-methyl-amide,
5-(2-Chloro-4,5-difluoro-benzoylamino)-1H-pyrazole-3-carboxylic acid methyl-[4-(5-oxo-2,5-dihydro-[1,2,4]oxadiazol-3-yl)-phenyl]-amide,
5-(2-Chloro-4,5-difluoro-benzoylamino)-1H-pyrazole-3-carboxylic acid {4-[(2,2-dimethyl-propionyloxy)-methoxycarbonyl]-phenyl}-methyl-amide,
5-(2-Chloro-4,5-difluoro-benzoylamino)-1H-pyrazole-3-c arboxylic acid 4-[N-(Methoxy-thiocarbonyloxy)-carbamimidoyl]-methyl-amide,
5-(2-Chloro-4,5-difluoro-benzoylamino)-1H-pyrazole-3-carboxylic acid methyl-[4-(5-thioxo-4,5-dihydro-[1,2,4]oxadiazol-3-yl) -phenyl] -amide,
5-(2-Chloro-4,5-difluoro-benzoylamino)-1H-pyrazole-3-carboxylic acid methyl-[4-(5-oxo-4,5-dihydro-[1,2,4]thiadiazol-3-yl)-phenyl]-amide,
5-(2-Chloro-4,5-difluoro-benzoylamino)-1H-pyrazole-3-carboxylic acid {4-[1-(cyclohexyloxy-carbonyloxy)-ethoxycarbonyl]-phenyl}-methyl-amide,
5-(2-Chloro-4,5-difluoro-benzoylamino)-1H-pyrazole-3-carboxylic acid (4-ethoxycarbonyl-thiazol-2-yl)-ethyl-amide,
5-(2-Chloro-4,5-difluoro-benzoylamino)-1H-pyrazole-3-carboxylic acid 2-methanesulfonylamino-benzylamide,
5-(2-Chloro-4,5-difluoro-benzoylamino)-1H-pyrazole-3-carboxylic acid 2-acetylamino-benzylamide,
5-(2-Chloro-4,5-difluoro-benzoylamino)-1H-pyrazole-3-carboxylic acid 2-(dimethylamino-methyleneamino)-benzylamide hydrochloride,
5-(2-Chloro-4,5-difluoro-benzoylamino)-1H-pyrazole-3-carboxylic acid 3-(dimethylamino-methyleneamino)-benzylamide hydrochloride,
5-(2-Chloro-4,5-difluoro-benzoylamino)-1H-pyrazole-3-carboxylic acid 4-(dimethylamino-methyleneamino)-benzylamide hydrochloride,
5-(2-Chloro-4,5-difluoro-benzoylamino)-1H-pyrazole-3-carboxylic acid 2-[1-(dimethylamino)ethylideneamino]-benzylamide dihydrochloride,
5-(2-Chloro-4,5-difluoro-benzoylamino)-1H-pyrazole-3-carboxylic acid 3-[1-(dimethylamino)ethylideneamino]-benzylamide dihydrochloride,
5-(2-Chloro-4,5-difluoro-benzoylamino)-1H-pyrazole-3-carboxylic acid 4-[1-(dimethylamino)ethylideneamino]-benzylamide dihydrochloride,
5-(2-Chloro-4,5-difluoro-benzoylamino)-1H-pyrazole-3-carboxylic acid 2-(diethylamino-methyleneamino)-benzylamide dihydrochloride,
5-(2-Chloro-4,5-difluoro-benzoylamino)-1H-pyrazole-3-carboxylic acid 3-(diethylamino=methyleneamino)-benzylamide dihydrochloride,
5-(2-Chloro-4,5-difluoro-benzoylamino)-1H-pyrazole-3-carboxylic acid 4-(diethylamino-methyleneamino)-benzylamide dihydrochloride,
5-(2-Chloro-4,5-difluoro-benzoylamino)-1H-pyrazole-3-carboxylic acid 2-acetimidoylamino-benzylamide dihydrochloride,
5-(2-Chloro-4,5-difluoro-benzoylamino)-1H-pyrazole-3-carboxylic acid 3-acetimidoylamino-benzylamide dihydrochloride,
5-(2-Chloro-4,5-difluoro-benzoylamino)-1H-pyrazole-3-carboxylic acid 4-acetimidoylamino-benzylamide dihydrochloride,
5-(2-Chloro-4,5-difluoro-benzoylamino)-1H-pyrazole-3-carboxylic acid 4-cyano-benzylamide,
5-(2-Chloro-4,5-difluoro-benzoylamino)-1H-pyrazole-3-carboxylic acid 3-[(2-fluoro-benzimidoyl)-amino]-benzylamide hyrdoiodide,
5-(2-Chloro-4,5-difluoro-benzoylamino)-1H-pyrazole-3-carboxylic acid 4-[(2-fluoro-benzimidoyl)-amino]-benzylamide hyrdoiodide,
5-(2-Chloro-4,5-difluoro-benzoylamino)-1H-pyrazole-3-carboxylic acid 3-(3,3-dimethyl-ureido)-benzylamide,
5-(2-Chloro-4,5-difluoro-benzoylamino)-1H-pyrazole-3-carboxylic acid 4-(3,3-dimethyl-ureido)-benzylamide,
5-(2-Chloro-4,5-difluoro-benzoylamino)-1H-pyrazole-3-carboxylic acid 2-(toluene-4-sulfonylamino)-benzylamide,
5-(2-Chloro-4,5-difluoro-benzoylamino)-1H-pyrazole-3-carboxylic acid 2-amino-6-fluoro-benzylamide hydrochloride,
5-(2-Chloro-4,5-difluoro-benzoylamino)-1H-pyrazole-3-carboxylic acid 2-amino-4,5-difluoro-benzylamide hydrochloride,
5-(2-Chloro-4,5-difluoro-benzoylamino)-1H-pyrazole-3-carboxylic acid (indan-1-yl)-amide,
5-(2-Chloro-4,5-difluoro-benzoylamino)-1H-pyrazole-3-carboxylic acid (indan-2-yl)-amide,
5-(2-Chloro-4,5-difluoro-benzoylamino)-1H-pyrazole-3-carboxylic (4H-quinazoline-3-yl)-amide,
5-(2-Chloro-4,5-difluoro-benzoylamino)-1H-pyrazole-3-carboxylic (4H-quinazoline-3-yl)-amide dihydrochloride,
5-(2-Chloro-4,5-difluoro-benzoylamino)-1H-pyrazole-3-carboxylic acid (7-fluoro-4H-quinazoline-3-yl)-amide dihydrochloride,
5-(2-Chloro-4,5-difluoro-benzoylamino)-1H-pyrazole-3-carboxylic acid (6-fluoro-4H-quinazoline-3-yl)-amide hydrochloride,
5-(2-Chloro-4,5-difluoro-benzoylamino)-1H-pyrazole-3-carboxylic acid (2-ethoxy-2-methyl-1,4-dihydro-2H-quinazoline-3-yl)-amide,
5-(2-Chloro-4,5-difluoro-benzoylamino)-1H-pyrazole-3-carboxylic acid (2-cyano-pyridin-4-ylmethyl)-amide,
5-(2-Chloro-4,5-difluoro-benzoylamino)-1H-pyrazole-3-carboxylic acid [2-(N-hydroxycarbamimidoyl)-pyridin-4-ylmethyl]-amide,
5-(2-Chloro-4,5-difluoro-benzoylamino)-1H-pyrazole-3-carboxylic acid [2-(5-oxo-4,5-dihydro-[1,2,4]oxadiazol-3-yl)-pyridin-4 -ylmethyl]-amide, 4-{[5-(4,5-difluoro-2-methyl-benzoylamino)-1H-pyrazole -3-carbonyl]-methyl-amino}-benzoic acid sodium salt,
5-(4,5-Difluoro-2-methyl-benzoylamino)-1H-pyrazole-3-carboxylic acid methyl-[4-(1H-tetrazol-5-yl)-phenyl]-amide,
5-(2,4-Dichloro-benzoylamino)-1H-pyrazole-3-carboxylic acid (pyridin-3-ylmethyl)-amide,
5-(2,4-Dichloro-5-fluoro-benzoylamino)-1H-pyrazole-3-carboxylic acid (pyridin-3-ylmethyl)-amide,
5-(2,6-Dichloro-5-fluoro-pyridine-3-carbonylamino)-1H-pyrazole-3-carboxylic acid (pyridin-3-ylmethyl)-amide,
5-[(3-Chloro-benzo[b]thiophene-2-carbonyl)-amino]-1H-pyrazole-3-carboxylic acid (pyridin-3-ylmethyl)-amide,
5-[(3-Chloro-thiophene-2-carbonyl)-amino]-1H-pyrazole-3-carboxylic acid (pyridin-3-ylmethyl)-amide,
5-Benzoylamino-1H-pyrazole-3-carboxylic acid (pyridin-3-ylmethyl)-amide,
5-Phenylacetylamino-1H-pyrazole-3-carboxylic acid (pyridin-3-ylmethyl)-amide,
5-(2-Methyl-4,5-difluoro-benzoylamino)-1H-pyrazole-3-carboxylic acid (pyridin-3-ylmethyl)-amide dihydrochloride,
5-(2-Amino-4,5-difluoro-benzoylamino)-1H-pyrazole-3-carboxylic acid (pyridin-3-ylmethyl)-amide,
5-(4,5-Difluoro-2-methanesulfonylamino-benzoylamino)-1 H-pyrazole-3-carboxylic acid (pyridin-3-ylmethyl)-amide,
5-(2-Acetylamino-4,5-difluoro-benzoylamino)-1H-pyrazole-3-carboxylic acid (pyridin-3-ylmethyl)-amide,
5-[(3,4,5-Trichloro-thiophene-2-carbonyl)-amino]-1H-pyrazole-3-carboxylic acid (pyridin-3-ylmethyl)-amide,
5-(4,5-Difluoro-2-methyl-benzoylamino)-1H-pyrazole-3-carboxylic acid (2-cyano-pyridin-4-ylmethyl)-amide,
5-(4,5-Difluoro-2-methyl-benzoylamino)-1H-pyrazole-3-carboxylic acid [2-(1H-tetrazol-5-yl)-pyridin-4-ylmethyl]-amide, 1H-Pyrazole-3,5-dicarboxylic acid 3-benzylamide
5-[(2,4-dichloro-phenyl)-amide].

A "pharmacologically acceptable salt thereof" may be any salt the pyrazole compound of the present invention having the general formula (I). Examples thereof include:
hydrohalide , such as hydrochloride, hydrobromide and hydriodide,inorganic acidsalts,such asnitrate,perchlorate, sulfate and phosphate; lower alkanesulfonates, such as methanesulfonate, trifluoromethanesulfonate and ethanesulfonate, arylsulfonates, such as benzenesulfonate and p-toluene sulfonate, carboxylic acid salts, such as acetate, malate, fumarate, succinate, citrate, ascorbate, tartrate, oxalate and maleate; and amino acid salts, such as glycine, lysine, arginine, ornithine, glutamic acid and aspartic acid.

Further examples thereof include alkali metal salts, such as sodium, potassium and lithium salts, alkaline earth metal salts, such as calcium and magnesium salts, metal salts, such as aluminum salt; inorganic salts, such as ammonium salt, amine salts or the like, such as tert-octyl amine, dibenzylamine, morpholine, glucosamine, phenylglycine alkylester, ethylenediamine, N-methylglucamine, guanidine, diethylamine, triethylamine, dicyclohexylamine, N,N'-dibenzylethylenediamine, chloroprocaine, procaine, diethanolamine, N-benzylphenethylamine, piperazine, tetramethylammonium and tris(hydroxymethyl)aminomethane salts;

The compounds of the present invention represented by the general formula (I) may be present in the form of various isomers, for example, optical isomers, geometic isomers, and tautomers. All these isomers and mixtures thereof are within the scope of the present invention.

From pyrazole derivatives having the general formula (I) of the present invention, esters or other derivatives thereof, prodrugs and metabolites thereof can be derived.

In addition, the compounds of the invention and a pharmacologically acceptable salts thereof may be present in the form of various solvates (for example, hydrates) . These solvates are within the scope of the present invention.

A "prodrug" as used in the present invention is a derivative of a compound (I) of the present invention that has a chemically or metabolically degradable group and can present pharmaceutical activity by hydrolysis and solvolysis, or by degradation under physiological conditions. Examples include those compounds in which the hydroxyl group is substituted with -CO-alkyl, -COO-alkyl, -CONH-alkyl, -CO-alkenyl, -COO-alkenyl, -CONH-alkenyl, -CO-aryl, -COO-aryl, -CONH-aryl, -CO-heterocycle, -COO-heterocycle, -CONH-heterocycle (the alkyl, alkenyl, aryl and heterocyclic ring may be substituted with a halogen atom, an alkyl group, a hydroxyl group, an alkoxy group, a carboxy group, an amino group, an amino acid, -PO₃H₂, -SO₃H -OPO₃H₂, -OSO₃H, etc.), -CO-polyethylene glycol residue, -COO-polyethylene glycol residue, -CO-polyethylene glycol monoalkyl ether residue, -COO-polyethylene glycol monoalkyl ether residue, -PO₃H₂, etc., those compounds in which the amino group is substituted with -CO-alkyl,-COO-alkyl,-CO-alkenyl,-COO-alkenyl,-COO-aryl, -CO-aryl, -CO-heterocycle, -COO-heterocycle (the alkyl, alkenyl, aryl and heterocyclic ring may be substituted with a halogen atom, an alkyl group, a hydroxyl group, an alkoxy group, a carboxy group, an amino group, an amino acid, -PO₃H₂, -SO₃H, -OPO₃H₂, -OSO₃H, etc.), -CO-polyethylene glycol residue, -COO-polyethylene glycol residue,-CO-polyethylene glycol monoalkyl ether residue, -COO-polyethylene glycol monoalkyl ether residue, -PO₃H₂, etc., or those compounds in which the carboxy group is substituted with an alkoxy group, aryloxy group (the alkoxy and aryloxy groups may be substituted with a halogen atom, an alkyl group, a hydroxyl group, an alkoxy group, a carboxy group, an amino group, an amino acid, -PO₃H₂, -S0₃H, -OPO₃H₂, -OSO₃H, etc.), polyethylene glycol residue, polyethylene glycol monoalkyl ether residue, etc.

The pyrazole compounds of the present invention or salts thereof, esters or other derivatives thereof, prodrugs and metabolites thereof, or hydrates and solvates thereof may be combined with a pharmaceutically acceptable carrier, and administered orally or parenterally in solid form, such as tablets, capsules, granules and powder, or in liquid form, such as syrup and injection.

A "pharmaceutical composition" as used herein refers to a homogeneous mixture of a pyrazole compound or a pharmacologically acceptable salt thereof, an ester or other derivative thereof, a prodrug or metabolite thereof or a hydrate or solvate thereof, as an active ingredient, with an excipient, lubricant.and binder used for preparing solid formulations, or a third ingredient such as a solvent, solubilizer, suspending agent, isotonizing agent and a buffer used for preparing liquid formulations.

Administration maybe by oral administration with tablet, pill, capsule, granule, powder or liquid form, or by parenteral administration with injections, such as intravenous and intramuscular administration, suppositories or transdermal formulations. Parenteral administration includes intravenous, intramuscular, subcutaneous, interstitial, nasal, intracutaneous, drip infusion, intracerebral, rectal, intravaginal and intraperitoneal administration.

"Solid compositions for oral administration" according to the present invention include tablets, powders and granules. In these solid compositions, one or more active substances are mixed with at least one inactive excipient, such as lactose, mannitol, glucose, hydroxypropylcellulose, microcrystalline cellulose, starch, polyvinylpyrrolidone and magnesium aluminometasilicate. The composition may, according to conventional method, also contain additives other than the inactive excipient, for example, a lubricant, a disintegrating agent, a stabilizer such as antioxidant, and a solubilizer. Tablets or pills may be coated as required with sugar coating or a film dissolvable in the gastrointestinal tract, such as sucrose, gelatin, hydroxypropylcellulose, hydroxypropyl methylcellulose, hydroxypropyl methylcellulose phthalate, macrogol, titanium dioxide and talc.

"Liquid compositions for oral administration" include pharmaceutically acceptable emulsions, solutions, suspensions, syrups and elixirs, and also include commonly used inert solvent, such as purified water and ethanol. The composition may contain an auxiliary agent other than the inert solvent, such as a solubilizer, wetting agent and suspending agent, a sweetening agent, a flavoring substance, an aromatic and a preservative.

An "injection for parenteral administration" may be prepared by dissolving, suspending or emulsifying a given amount of an active ingredient in an aqueous solvent (for example, distilled water for injection, physiological saline, ringer solution, etc.) or an oil-based solvent (for example, vegetable oils such as olive oil, sesame oil, cottonseed oil and corn oil, propylene glycol, etc.) together with a dispersant (for example, polysorbate 80, polyoxyethylene hydrogenated castor oil 60, polyethylene glycol, carboxymethylcellulose, sodium alginate, etc.), a preservative (for example, methylparaben, propylparaben, benzyl alcohol, chlorobutanol, phenol, etc.), an isotonizing agent (for example, sodium chloride, glycerin, D-mannitol, D-sorbitol, glucose, etc.), etc. During this process, additivessuchasasolubilizer (forexample, sodiumsalicylate and sodium acetate), a stabilizing agent (for example, human serum albumin) and a soothing agent (for example, benzyl alcohol) may be used as desired. An antioxidant, a coloring agent and other additives may also be added if necessary.

"Pharmaceutically acceptable carriers" include various organic or inorganic carrier materials commonly used as formulation raw materials; for example, excipients, lubricants, binders and disintegrating agents are used as appropriate for solid formulation, and solvents, solubilizers, suspending agents, isotonizing agents, buffers and soothing agents for liquid formulation. If necessary, formulation additives may also be used according to conventional methods, such as a preservative, an antioxidant, a coloring agent, a sweetener, an adsorbent and a gelling agent.

Preferred examples of "excipients" may include lactose, corn starch, saccharose, D-mannitol, D-sorbitol, starch, dextrin, crystalline cellulose, low-substituted hydroxypropylcellulose, sodium carboxymethylcellulose, arabic gum, glucose and silicon dioxide.

Preferred examples of "antioxidants" may include sulfite and ascorbic acid.

"Disintegrating agents" suitable for use include carboxymethylcellulose, carboxymethylcellulose calcium, carboxymethyl starch sodium, crosscarmellose sodium, crosspovidone, low-substituted hydroxypropylcellulose and hydroxypropyl starch.

"Binders" include, for example, hydroxypropylcellulose, hydroxypropyl methylcellulose, polyvinylpyrrolidone, crystalline cellulose, saccharose and powdered acacia. The binder is preferably hydroxypropylcellulose or polyvinylpyrrolidone.

Preferred examples of " lubricants" may include magnesium stearate, calcium stearate, talc and colloidal silica.

Preferred examples of "isotonizing agents" include glucose, D-sorbitol, sodium chloride, glycerin and D-mannitol.

"pH adjusters" include citrate, phosphate, carbonate, tartrate, fumarate, acetate and amino acid salts.

Preferred examples of "solubilizers" include polyethylene glycol, propylene glycol, D-mannitol, benzyl benzoate, ethanol, tris-aminomethane, cholesterol, triethanolamine, sodium carbonate and sodium citrate.

Preferred examples of "solvents" include water for injection, alcohol, propylene glycol, macrogol, sesame oil, corn oil and olive oil.

Preferred examples of "suspending agents" include, hydrophilic macromolecules, such as polyvinyl alcohol, polyvinylpyrrolidone, sodium carboxymethylcellulose, methylcellulose, hydroxymethylcellulose, hydroxyethylcellulose and hydroxypropylcellulose.

Preferred examples of "surfactants" may include sodium lauryl sulfate, lauryl aminopropionic acid, lecithin, benzalkonium chloride, benzethonium chloride and glyceryl monostearate.

Preferred examples of "soothing agents" may include benzyl alcohol.

Preferred examples of "buffers" include phosphate, acetate, carbonate and citrate buffers.

Preferred examples of "preservatives" include paraoxybenzoic acid ester, chlorobutanol, benzyl alcohol, phenethyl alcohol, dehydroacetic acid and sorbic acid.

When the present invention is used as an antidiabetic, it is administered systemically or locally via oral or parenteral route. Dosage may vary depending on age, body weight, symptom and therapeutic effect, and may be generally administered once to several times a day at a dose of 10 mg to 1 g for an adult.

The compound (I) of the present invention may be mixed with an appropriate diluent, a dispersant, an adsorbent, a solubilizing agent, etc., to prepare solid and liquid compositions for oral administration or formulations for parenteral administration such as injection.

The compound (I) of the present invention may also be used for the treatment and prevention of diabetes in animals other than humans, such as mammals.

The compound (I) of the present invention may be used in combination with one ormore other agents in a manner commonly practiced in medicine. The compound (I) of the present invention may be combined with various drugs, including preferably antilipemics and antidiabetics. The "use in combination" herein means use of an agent containing the compound of the present invention (I) in combination with one or more other agents, and is not particularly limited. The other agents may have efficacy and action mechanism the same as or different from those of the agent containing the compound of the present invention (I). These agents may be administered as individual formulations or as a mixture. These formulations may be combined to prepare a kit. In addition, the time of administration is not particularly limited. Both formulations may be administered at the same time, or each formulation may be administered at different times.

The compound, pharmaceutical composition or drug of the present invention may be combined with other pharmaceutical compositions or drugs (hereinafter, also referred to as concomitant drugs).

The timing of dosing the pharmaceutical composition or drug of the present invention and its concomitant drug is not limited, and they may be administered to a subject concurrently or at an interval. The dosage of the concomitant drug may be according to the clinical standard, and may be selected as appropriate according to the subject, age and body weight of the subject, symptom, time of administration, dosage form, administration method and combination. The form of administration of the concomitant drug is not limited, providing that the concomitant drug is combined in any way with the pharmaceutical composition or drug of the present invention.

Concomitant drugs include,
(1) therapeutic or prophylactic agents for hyperlipidemia,
(2) therapeutic or prophylactic agents for obesity,
(3) therapeutic or prophylactic agents for diabetes,
(4) therapeutic or prophylactic agents for diabetic complications, and
(5) therapeutic or prophylactic agents for hypertension, and 1 to 3 of these agents may be combined with the compound of the present invention.

"Therapeutic or prophylactic agents for hyperlipidemia" include, for example,
(1) fibrates (PPARα receptor agonist),
(2) PPARδ receptor agonist,
(3) microsome triglyceride transfer protein (MTP) inhibitor,
(4) cholesteryl ester transfer protein (CETP) inhibitor,
(5) statins (HMG-CoA reductase inhibitor),
(6) anion exchange resin,
(7) probucol,
(8) nicotinic acid,
(9) plant sterol,
(10) apolipoprotein-A1 (Apo-A1) inducer,
(11) lipoprotein lipase (LPL) activator,
(12) endothelial lipase inhibitor,
(13) ezetimibu,
(14) IBAT inhibitor,
(15) squalene synthase inhibitor,
(16) ACAT inhibitor,
(17) LXR receptor agonist,
(18) FXR receptor agonist,
(19) FXR receptor antagonist, and
(20) adenosine A1 agonist,
   and specific examples include clofibrate, bezafibrate, fenofibrate, lovastatin, simvastatin, pravastatin, fluvastatin, atorvastatin, pitavastatin, rosuvastatin, cerivastatin, cholestyramine, colestimide, tocopherol nicotinate, nicomol, niceritrol, soysterol and gamma orizanol.

"Therapeutic or prophylactic agents for obesity" include, for example,
(1) leptin formulation,
(2) pancreatic lipase inhibitor,
(3) noradrenaline serotonin reuptake inhibitor,
(4) cannabinoid receptor antagonist,
(5) monoamine reuptake inhibitor,
(6) diacylglycerol acyltransferase (DGAT) inhibitor,
(7) glucose-dependent insulinotropic polypeptide (GIP) receptor antagonist,
(8) leptin receptor agonist,
(9) bombesin receptor subtype 3 (BRS-3) agonist,
(10) perilipin inhibitor,
(11) acetyl-CoA carboxylase 1 (ACC 1) inhibitor,
(12) acetyl-CoA carboxylase 2 (ACC 2) inhibitor,
(13) fatty acid synthase inhibitor,
(14) sn-1-acyl-glycerol-3-phosphateacyltransferase (AGPAT) inhibitor,
(15) pancreatic phospholipase A2 (pPLA2) inhibitor,
(16) melanocortin (MC) receptor agonist,
(17) neuropeptide Y5 (NPY5) receptor antagonist,
(18) uncoupling protein (UCP) inducer or activator,
(19) carnitine palmitoyltransferase 1 (CPT-1) activator,
(20) CCK1 (CCKA) agonist,
(21) ciliary neurotrophic factor (CNTF),
(22) CRF2 agonist,
(23) neuropeptide Y2 (NPY2) receptor antagonist,
(24) neuropeptide Y4 (NPY4) receptor antagonist,
(25) thyroid hormone receptor β agonist,
(26) growth hormone,
(27) ATP citrate lyase inhibitor,
(28) 5-HT6 antagonist, and
(29) 5-HT2C agonist,
   and specific examples include leptin, orlistat, sibutramine, rimonabant and mazindol.

"Therapeutic or prophylactic agents for diabetes" include, for example,
(1) insulin preparation (injection),
(2) low molecular insulin oral agent,
(3) sulfonylurea receptor agonist (SU agent),
(4) non-sulfonylurea insulinotropic agent,
(5) potassium-dependent ATP (KATP) channel opening agent,
(6) α glucosidase inhibitor,
(7) α amylase inhibitor,
(8) insulin sensitizer,
(9) low molecular tGLP-1 receptor agonist,
(10) tGLP-1 peptide analogue,
(11) dipeptidyl peptidase IV (DPP-IV) inhibitor,
(12) glucagon receptor antagonist,
(13) glucocorticoid receptor antagonist,
(14) biguanide,
(15) SGLUT inhibitor,
(16) fructose-1,6-bisphosphatase (FBPase) inhibitor,
(17) glycogen synthase kinase 3 (GSK-3) inhibitor,
(18) phosphoenolpyruvate carboxykinase (PEPCK) inhibitor,
(19) protein tyrosine phosphatase 1B (PTPase1B) inhibitor,
(20) SH2 domain containing inositol phosphatase (SHIP2) inhibitor,
(21) glycogen phosphorylase (GP) inhibitor,
(22) glucokinase activator,
(23) GPR40 receptor agonist,
(24) pyruvate dehydrogenase kinase (PDHK) inhibitor,
(25) glutamine:fructose-6-phosphate aminotransferase (GFAT) inhibitor,
(26) antioxidant; nitric monoxide scavenger,
(27) carnitine palmitoyltransferase 1 (CPT-1) inhibitor,
(28) growth hormone release factor (GHRF),
(29) triacylglycerol lipase (hormone-sensitive lipase) inhibitor,
(30) PPAR γ receptor agonist,
(31) PPAR γ receptor antagonist,
(32) PPAR α/γ receptor agonist,
(33) AMP activation protein kinase (AM PK) activator,
(34) adiponectin receptor agonist, and
(35) β₃ adrenoceptor agonist,
   and specific examples include insulin, tolbutamide, glyclopyramide, acetohexamide, chlorpropamide, glybuzole, glibenclamide, gliclazide, glimepiride, mitiglinide, repaglinide, nateglinide, voglibose, acarbose, miglitol, rosiglitazone maleate, metformin hydrochloride, pioglitazone hydrochloride and buformin hydrochloride.

"Therapeutic or prophylactic agents for diabetic complications" include, for example,
(1) protein kinase β (PKC β) inhibitor,
(2) angiotensin II receptor antagonist,
(3) aldose reductase inhibitor,
(4) angiotensin converting enzyme (ACE) inhibitor,
(5) advancedglycationendproduct (AGE) production inhibitor,
(6) neuropathy therapeutic agent, and
(7) diabetic nephropathy therapeutic agent, and specific examples include epalrestat (Kinedak), mexiletine hydrochloride and imidapril hydrochloride.

"Therapeutic or prophylactic agents for hypertension" include, for example,
(1) thiazide diuretic,
(2) similar thiazide diuretic,
(3) loop diuretic,
(4) K retaining diuretic,
(5) β blocker,
(6) α, β blocker,
(7) α blocker,
(8) central sympathetic nerve depressant,
(9) peripheral sympathetic nerve depressant (rauwolfia preparation),
(10) Ca antagonist (benzothiazepin),
(11) Ca antagonist (dihydropyridine),
(12) vasodilator,
(13) angiotensin converting enzyme (ACE) inhibitor,
(14) angiotensin II receptor antagonist,
(15) nitric acid,
(16) endothelin ETA receptor antagonist,
(17) endothelin converting enzyme inhibitor; neprilysin inhibitor,
(18) prostaglandin; prostanoid FP agonist,
(19) renin inhibitor,
(20) NOS3 expression enhancer; prostacyclin analogue,
(21) phosphodiesterase V (PDE5A) inhibitor,
(22) prostacyclin analogue, and
(23) aldosterone antagonist, and specific examples include hydrochlorothiazide, trichlormethiazide, bentylhydrochlorothiazide, meticrane, indapamide, tripamide, chlortalidone, mefruside, furosemide, spironolactone, triamteren, atenolol, bisoprolol fumarate, betaxolol hydrochloride, bevantolol hydrochloride, metoprolol tartrate, acebutolol hydrochloride, celiprolol hydrochloride, nipradilol, tilisololhydrochloride, nadolol, propranolol hydrochloride, indenolol hydrochloride, carteolol hydrochloride, pindolol, pindolol sustained-release tablet, bunitrolol hydrochloride, penbutolol sulfate, bopindolol malonate, amosulalol hydrochloride, arotinolol hydrochloride, carvedilol, labetalol hydrochloride, urapidil, terazosin hydrochloride, doxazosin mesilate, bunazosin hydrochloride, prazosin hydrochloride, phentolamine mesilate, clonidine hydrochloride, guanfacine hydrochloride, guanabenz acetate, methyldopa, reserpine, rescinnamine, amlodipine besylate, aranidipine, efonidipine hydrochloride, cilnidipine, nicardipine hydrochloride, nisoldipine, nitrendipine, nifedipine, sustained-release nifedipine, nilvadipine, barnidipine hydrochloride, felodipine, benidipine hydrochloride, manidipine hydrochloride, azelnidipine, diltiazem hydrochloride, hydrazine hydrochloride, todoralazine hydrochloride, budralazine, cadralazine, captopril, enalapril maleate, alacepril, delapril hydrochloride, cilazapril, lisinopril, benazepril hydrochloride, imidapril hydrochloride, tamocapril hydrochloride, quinapril hydrochloride, trandolapril, perindopril erbumine, candesartan cilexetil, valsartan, telmisartan, olmesartan medoxomil, sodium nitroprusside and nitroglycerine.
   The following describes a method for preparing the pyrazole compound represented by the compound (I), but the preparation method of the present invention is not limited thereto. The amount of solvent used in each step is not limited as long as the reaction mixture can be stirred well. Furthermore, in each step, the reaction may be carried out in a conventional manner, and isolation/purification may be performed by employing any of or combining routine methods such as recrystallization or reprecipitation, or methods conventionally used for isolation and purification of organic compounds such as adsorption column chromatography, partition column chromatography, ion-exchange chromatography and gel filtration chromatography.

The following describes in detail methods for preparing the compounds represented by the general formula according to the invention, but the present invention should not be construed as being limited thereto. Therefore, the compounds of the present invention may be prepared by the following Preparation Method A (A-1 to A-3), method B or method C (C-1 to C-6), or according to subsequent examples, or by reference to these methods . In preparing the compounds of the present invention, reaction sequence may be changed as appropriate. Reactionmaybe begun with the step that is considered rational. If necessary, protection and deprotection may be performed as appropriate. Reaction other than the above steps may be performed as appropriate. Reagents other than those exemplified may be used as appropriate to promote reaction.

The scheme shown below is an example of a representative preparation method, but the preparation of the compounds of the present invention should not be construed as being limited thereto. Any compound from each step may be isolated and purified by conventional methods, or the isolation/purification step may be optionally skipped.

### Preparation Method A (Preparation Methods A-1 through A-3):

Preparation Method A is shown as follows. Each reference symbol in the reaction formulas is defined in the same manner as that described above. This also applies to the following description.

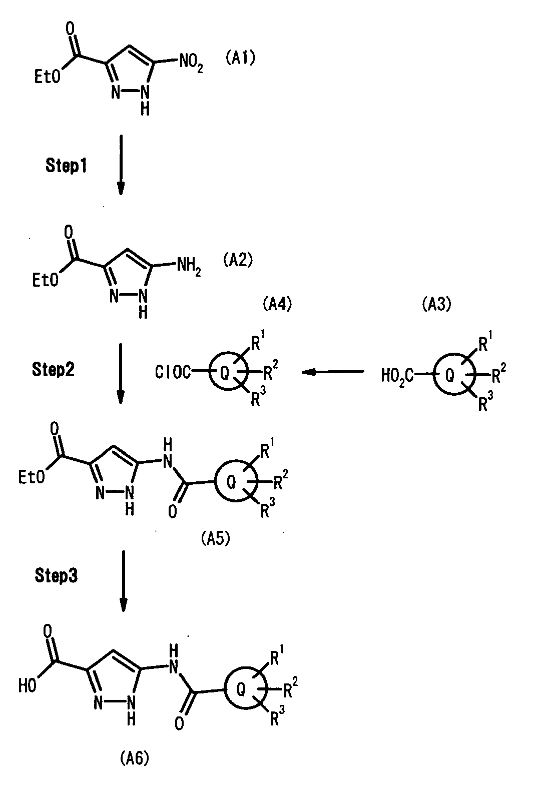

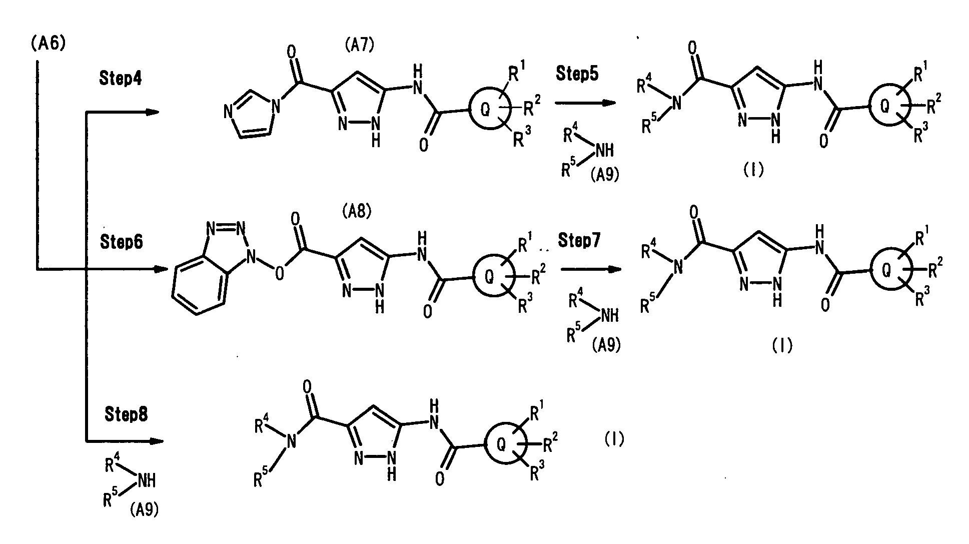

### Step 1: Preparation of Compound (A2);

Compound (A2) can be obtained by hydrogenation of nitro group of compound (A1) to amino group in a solvent in the presence of a catalyst.

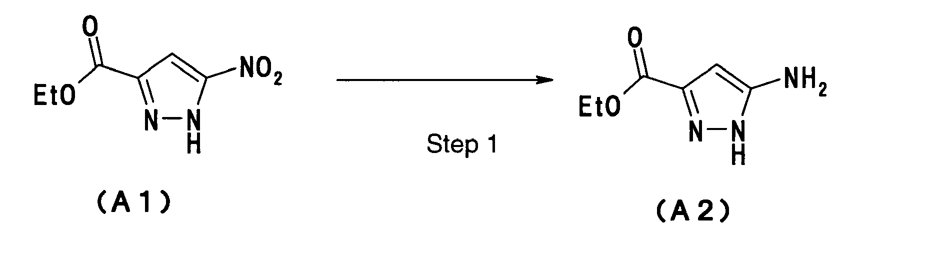

Examples of the solvent used in the reaction include alcohols such as methanol, ethanol and the like; ethers such as dioxane, tetrahydrofuran and the like; esters such as ethyl acetate and the like; polar solvents such as N,N-dimethylformamide and the like; and water or the like. These may be used alone or in combination.

Examples of the catalyst used in the reaction include palladium catalysts such aspalladium black,palladium/carbon and the like; nickel catalysts such as Raney nickel; and platinum catalysts such as platinum oxide, platinum oxide-carbon and the like (Reference: M. Hudlicky, Reductions in Organic Chemistry, Wiley-Interscience, New York, 1984).

The reaction temperature is from approximately 0°C to 100°C, and is preferably from approximately 0°C to room temperature. The reaction time is from approximately 10 minutes to 48 hours, and is preferably from approximately 30 minutes to 24 hours.

Other hydrogen donors than hydrogen such as formic acid, ammonium formate, cyclohexene, hydrazine and the like may also be used (Reference: M. Hudlicky, Reductions in Organic Chemistry, Wiley-Interscience, New York, 1984).

Further, other nitro-group reduction methods include methods wherein zinc/hydrochloric acid, tin(II) chloride, sodium hydrosulfite, titanium(III) chloride or the like may also be used (Reference: M. Hudlicky, Reductions in Organic Chemistry, Wiley-Interscience, New York, 1984).

### Step 2: Preparation of Compound (A5)

Compound (A5) can be obtained by preparing Compound (A4) from Compound (A3) by a conventional acyl chloride synthesis method, and acylating the amino group in Compound (A2) using the Compound (A4) in a solvent in the presence of a base.

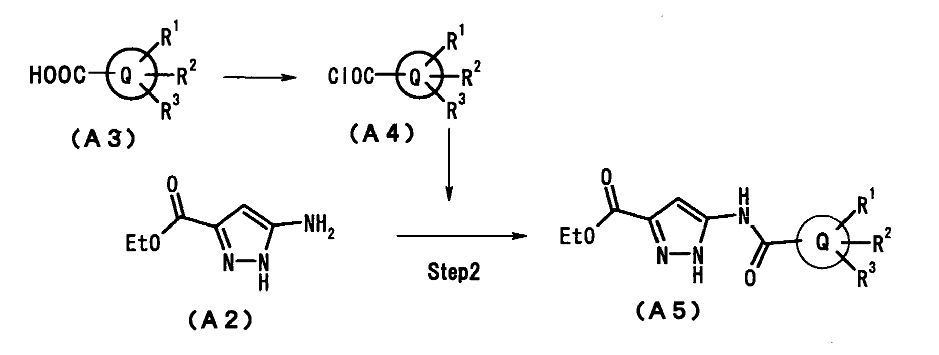

Examples of reagents used in acyl chloride formation include oxalyl chloride, thionyl chloride, phosphorous pentachloride and the like.

N,N-dimethylformamide may be used to promote reaction as necessary.

In addition, examples of the solvents used in the reaction include ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; and these may be used alone or in combination.

The reaction temperature is from approximately -20°C to 120°C, and is preferably from approximately 0°C to 80°C.

The reaction time is from approximately 10 minutes to 48 hours, and is preferably from approximately 30 minutes to 24 hours.

Examples of the solvent which can be used in the acylation reaction include ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethaneandthe like; esters such as ethyl acetate, methyl acetate, butyl acetate and the like; polar solvents such as acetone, N,N-dimethylacetamide, dimethylsulfoxide and the like; and water or the like. These may be used alone or in combination.

Examples of the base used in this reaction include organic bases such as triethylamine, pyridine, 4-dimethylaminopyridine, N-methylmorpholine and the like; inorganic bases such as lithium hydroxide, sodium hydroxide, potassium hydroxide, sodium carbonate, potassium carbonate, sodium bicarbonate, potassium bicarbonate and the like.

The reaction temperature is from approximately 0°C to 120°C, and is preferably from approximately 0°C to 95°C.

The reaction time is from approximately 10 minutes to 48 hours, and is preferably from approximately 30 minutes to 24 hours.

Compound (A5) can also be obtained by direct coupling of compound (A3) with compound (A4) using other methods for amide bond formation reaction.

Examples of methods which can be used include liquid phase synthesis methods by a mixed acid anhydride method, acid azide method, DCC method, EDC method, EDC-HOBt method, EDC-HOSu method, other active ester method, CDI method as well as a peptide solid phase synthesis method.
(DCC; 1,3-dicyclohexylcarbodiimide)
(EDC; 1-ethyl-3-(3'-dimethylaminopropyl)carbodiimide)
(HOBt: 1-hydroxybenzotriazole)
(HOSu; 1-hydroxysuccinimide)
(CDI; 1,1'-carbonyldiimidazole)

### Step 3: Preparation of Compound (A6)

Compound (A6) can be obtained by hydrolysis of ester group of compound (A5) in a solvent.

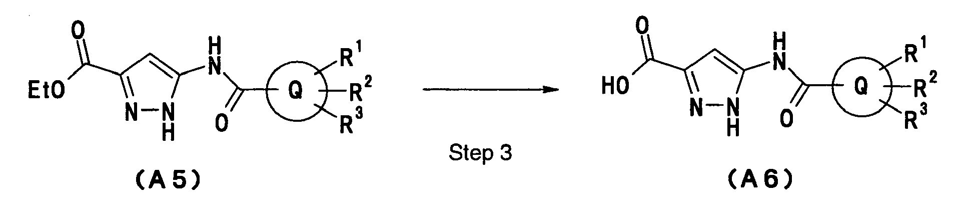

Examples of the solvent used in the reaction include alcohols such as methanol, ethanol and the like; ethers such as dioxane, tetrahydrofuran and the like; ketones such as acetone and the like; polar solvents such as N,N-dimethylformamide, dimethylsulfoxide and the like; and water or the like. These may be used alone or in combination.

Examples of the base used in this reaction include alkali metal hydroxides such as sodium hydroxide, potassium hydroxide; alkali earth metal such as barium hydroxide and the like.

The reaction temperature is from approximately 0°C to 100°C, and is preferably from approximately 0°C to room temperature. The reaction time is from approximately 10 minutes to 48 hours, and is preferably from approximately 30 minutes to 24 hours.

### Step 4 to Step 8: Preparation of Compound (I)

A summary of from Step 4 to Step 8 will now be described.

The compound represented by the general formula (I) can be prepared by isolating compound (A6), in which the carboxyl group is activated, as active amide such as imidazolide (compound (A7); Steps 4 and 5 of Preparation Method A-1), or active ester such as HOBt ester (compound (A8); Steps 6 and 7 of Preparation Method A-2) or HOSu ester, for example; followed by coupling the isolated active carbonyl compound (A6) with amine (A9) through an amide bond.

The compound represented by the general formula (I) can also be prepared directly from compound (A6) by a conventional amide bond formation reaction, for example, a liquid phase synthesis methods such as a mixed acid anhydride method, acyl azide method, DCCmethod, EDCmethod, EDC-HOBtmethod, EDC-HOSu method, other active ester method or CDI method; or a peptide sol id phase synthesis method to form an amide bond with compound (A9) as amine, without isolating the above-described active carbonyl compound (Step 8 of Preparation Method A-3 described below).
(Reference: E.Gross., The Peptides, Academic Press, 1981.)

### Preparation Method A-1: preparation method of Compound (I) via Compound (A7);

### Step 4: Preparation of Compound (A7)

Compound (A7) can be obtained by reacting compound (A6) in a solvent with CDI.

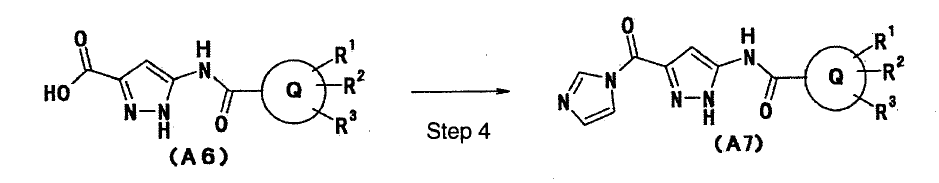

Examples of the solvent used in the reaction include ethers such as tetrahydrofuran and the like; halogenated hydrocarbons such as chloroform and the like; hydrocarbons such as toluene and the like; and polar solvents such as N,N-dimethylformamide and the like. These may be used alone or in combination.

The reaction temperature is from approximately 0°C to 100°C, and is preferably from room temperature to approximately 80°C.

The reaction time is from approximately 10 minutes to 48 hours, and is preferably from approximately 30 minutes to 24 hours.

When using, in place of CDI , an analogue compound thereof, for example, 1,1'-carbonylbis(2-methylimidazole), a corresponding active amide can be obtained.

Compound (A7) or a corresponding active amide can be obtained using other active amide synthesis reaction.

### Step 5: Preparation of Compound (I) from Compound (A7)

The compound represented by the general formula (I) can be obtained by reacting compound (A7) in a solvent with the amine compound (A9).

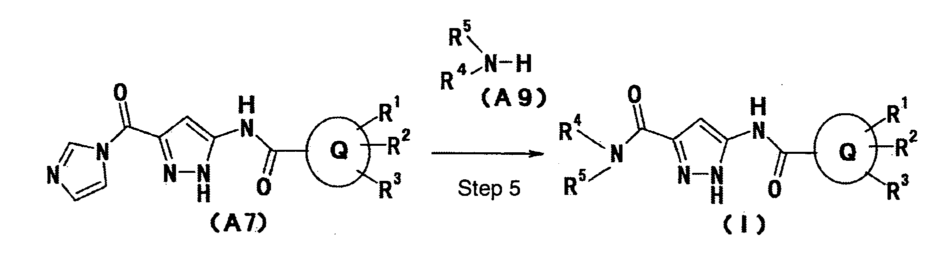

Examples of the solvent used in the reaction include ethers such as tetrahydrofuran and the like; esters such as ethyl acetate and the like; halogenated hydrocarbons such as chloroform and the like; hydrocarbons such as toluene and the like; and polar solvents such as N,N-dimethylformamide and the like. These may be used alone or in combination.

The reaction temperature is from approximately 0°C to 100°C, and is preferably from approximately 0°C to room temperature. The reaction time is from approximately 10 minutes to 48 hours, and is preferably from approximately 30 minutes to 24 hours.

### Preparation Method A-2: preparation method of Compound (I) via Compound (A8)

### Step 6: Preparation of Compound (A8) from Compound (A6)

The active ester compound (A8) can be obtained by coupling compound (A6) in a solvent with HOBt in the presence of coupling reagent.

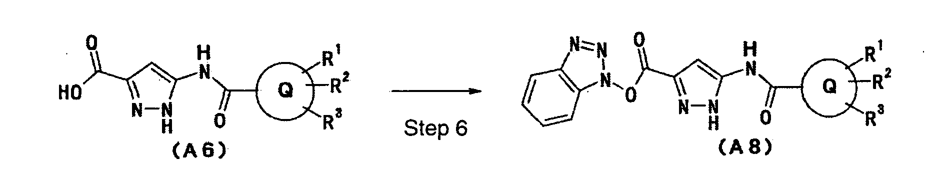

Examples of the coupling reagent include carbodiimides such as EDC, DCC and the like; and haloformate such as ethyl chloroformate and the like.

Examples of the solvent used in the reaction include ethers such as tetrahydrofuran and the like; esters such as ethyl acetate and the like; halogenated hydrocarbons such as chloroform and the like; hydrocarbons such as toluene and the like; and polar solvents such as N,N-dimethylformamide and the like. These may be used alone or in combination.

The reaction temperature is from approximately 0°C to 100°C, and is preferably from approximately 0°C to room temperature.

The reaction time is from approximately 10 minutes to 48 hours, and is preferably from approximately 30 minutes to 24 hours.

When a N-hydroxyimides such as HOSu or the like is used in place of HOBt, the corresponding active esters can be obtained.

### Step 7: Preparation of Compound (I) from Compound (A8)

The compound represented by the general formula (I) can be obtained by reacting compound (A8) in a solvent with amine compound (A9).

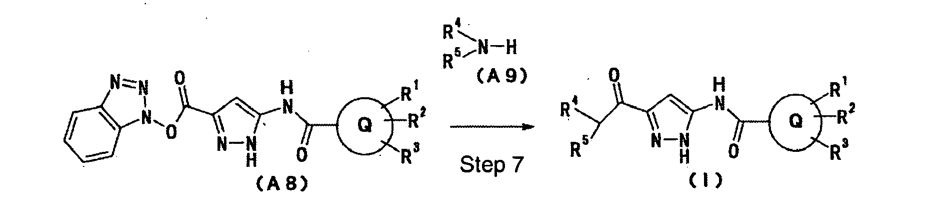

Examples of the solvent used in the reaction include ethers such as tetrahydrofuran and the like; esterts such as ethyl acetate and the like; halogenated hydrocarbons such as chloroform and the like; hydrocarbons such as toluene and the like; polar solvents such as N,N-dimethylformamide and the like; and water or the like. These may be used alone or in combination.

The reaction temperature is from approximately 0°C to 100°C, and is preferably from approximately 0°C to room temperature.

The reaction time is from approximately 10 minutes to 48 hours, and is preferably from approximately 30 minutes to 24 hours.

### Preparation MethodA- 3: Method for Directly Preparing Compound (I) from Compound (A6)

### Step 8: Preparation of Compound (I) from Compound (A6)

The compound represented by the general formula (I) is prepared from compound (A6) by a conventional amide bond formation reaction, for example, a liquid phase synthesis method such as mixed acid anhydride method, acyl azide method, DCC method, EDC method, EDC-HOB method, other active ester method or CDI method; or a peptide solidphase synthesis method.

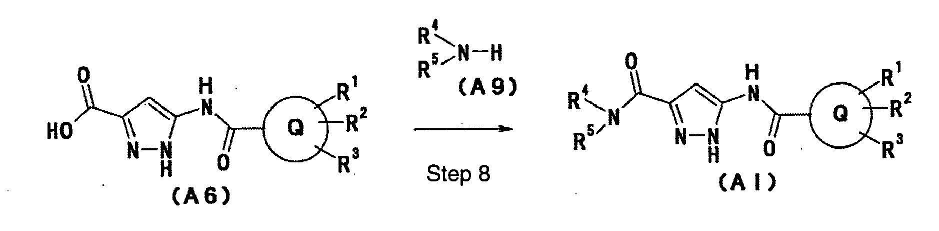

Examples of the solvent used in the reaction include ethers such as tetrahydrofuran and the like; esters such as ethyl acetate and the like; halogenated hydrocarbons such as chloroform and the like; hydrocarbons such as toluene and the like; polar solvents such as N,N-dimethylformamide and the like; and water or the like. These may be used alone or in combination.

The reaction temperature is from approximately 0°C to 100°C, and is preferably from approximately 0°C to room temperature.

The reaction time is from approximately 10 minutes to 48 hours, and is preferably from approximately 30 minutes to 24 hours.

### Preparation Method B:

Preparation Method B is shown as follows. Each reference symbol in the formulas is defined in the same manner as that described.above. This also applies to the following description.

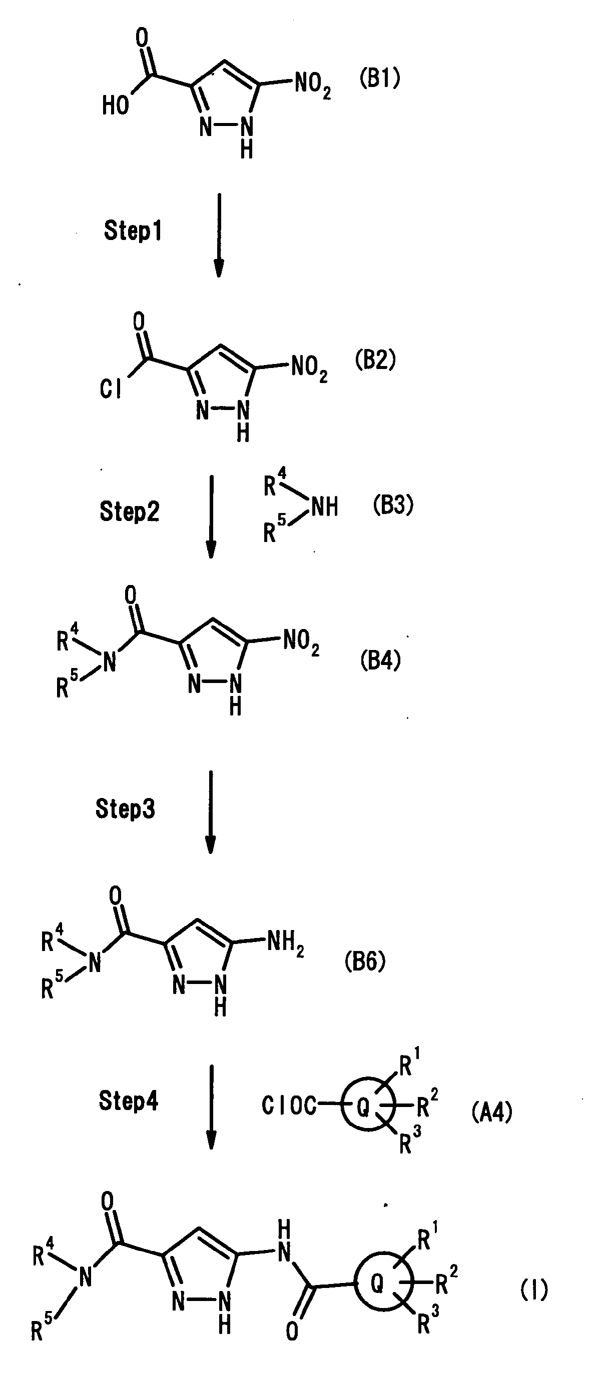

### Step 1: Preparation of Compound (B2)

Compound (B2) can be obtained from compound (B1) in the same manner as in the acyl chloride formation in Step 2 of Preparation Method A.

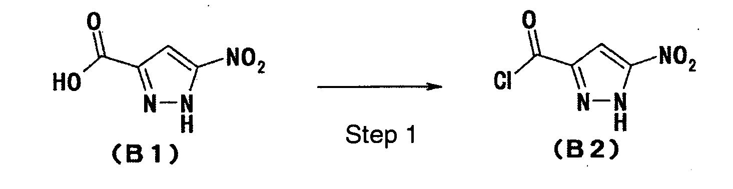

### Step 2: Preparation of Compound (B4)

Compound (B4) can be obtained by reacting the compound (B2) in a solvent with compound (B3) in the presence of a base as necessary.

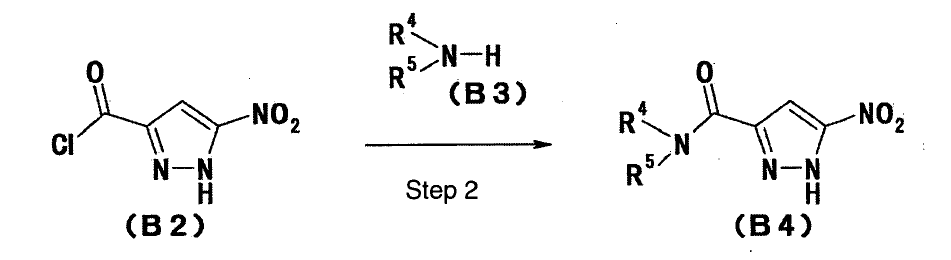

Examples of the solvent used in the reaction include ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide and the like; and water. These may be used alone or in combination.

Examples of the base used include organic bases such as triethylamine, pyridine, 4-dimethylaminopyridine, N-methylmorpholine and the like; inorganic bases such as lithium hydroxide, sodium hydroxide, potassium hydroxide, sodium carbonate, potassium carbonate, sodium bicarbonate, potassium bicarbonate and the like.

The reaction temperature is from approximately 0°C to 120°C, and is preferably from approximately 0°C to 60°C.

The reaction time is from approximately 10 minutes to 48 hours, and is preferably from approximately 30 minutes to 24 hours.

### Step 3: Preparation of Compound (B6)

Compound (B6) can be obtained by hydrogenation of nitro group of compound (B4) in a solvent in the presence of a catalyst in the same manner as in Step 1 of Preparation Method A.

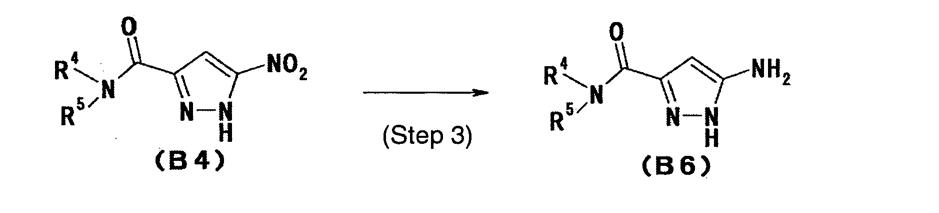

### Step 4: Preparation of Compound (I) from Compound (B6)

The compound represented by the general formula (I) can be obtained in the same manner as in Step 2 of Preparation Method A, by acylating amino group of Compound (B6) using Compound (A4) in a solvent in the presence of a base.

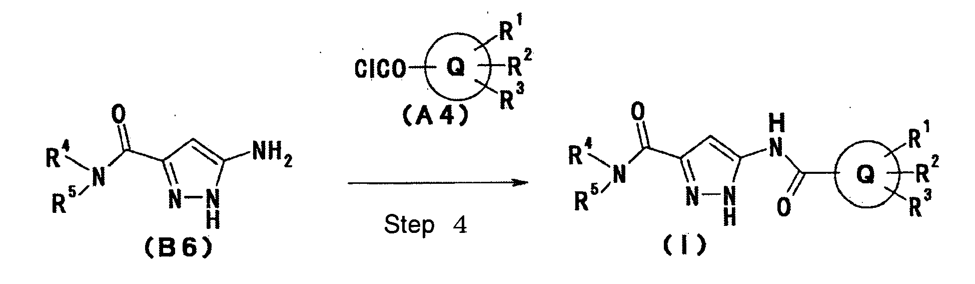

### Preparation Method C: preparation method of the Salt of a Pyrazole Compound

### Preparation Method C-1

The compound represented by the general formula (I) is dissolved in a solvent A, and an acid or a base (itself or in solution) is added thereto. The resulting solution is allowed to stand, and the formed solid is filtered, whereby a salt of the compound represented by the general formula (I) can be obtained.

### Preparation Method C-2

The compound represented by the general formula (I) is dissolved in a solvent A, and an acid or a base (itself or in solution) is added thereto. The resulting solution is concentrated and allowed to stand. The formed solid is filtered, whereby a salt of the compound represented by the general formula (I) can be obtained.

### Preparation Method C-3

The compound represented by the general formula (I) is dissolved in a solvent A, and an acid or a base (itself or in solution) is added thereto. A solvent B is added to the resulting solution, and then the mixture is allowed to stand. The formed solid is filtered, whereby a salt of the compound represented by the general formula (I) can be obtained.

### Preparation Method C-4

The compound represented by the general formula (I) is suspended in a solvent A, and an acid or a base (itself or in solution) is added thereto. Theresulting solution is allowed to stand, and the formed solid is filtered, whereby a salt of the compound represented by the general formula (I) can be obtained.

### Preparation Method C-5

The compound represented by the general formula (I) is suspended in a solvent A, and an acid or a base (itself or in solution) is added thereto. The resulting solution is concentrated and allowed to stand, and the formed solid is filtered, whereby a salt of the compound represented by the general formula (I) can be obtained.

### Preparation Method C-6

The compound represented by the general formula (I) is suspended in a solvent A, and an acid or a base (itself or in solution) is added thereto. A solvent B is added to the resulting solution and the mixture is allowed to stand. The formed solid is filtered, whereby a salt of the compound represented by the general formula (I) can be obtained.

Further, a monoacid addition salt can be obtained by dissolving, or suspending, a diacid addition salt prerared in accordance with the above-described Preparation Method C (C-1 to C-6) in the above-described solvent A, and then letting stand. Examples of the diacid addition salt include dihydrochloride, dimethanesulfonate and the like. Examples of the monoacid addition salt include monohydrochloride and monomethanesulfonate.

Examples of the "solvent A" which is used in the Preparation Method C-1 to C-6 include alcohols such as methanol, ethanol, propanol and the like; ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1, 2-dichloroethane and the like; esters such as ethyl acetate, methyl acetate, butyl acetate and the like; and polar solvents such as acetone, N,N-dimethylformamide, dimethylsulfoxide; water and the like.

Examples of the "solvent B" which is used include ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; and esters such as ethyl acetate, methyl acetate, butyl acetate and the like.

Examples of the "acid" which is used include inorganic acids such as hydrochloric acid, hydrobromic acid, sulfuric acid and the like; carboxylic acids such as malic acid, citric acid, oxalic acid and the like; and sulfonic acids such as methanesulfonic acid, benzenesulfonic acid, p-toluenesulfonic acid and the like. Preferable is hydrochloric acid and p-toluenesulfonic acid.

Examples of the "base" which is used include inorganic bases such as sodium hydroxide, sodium hydride, potassium hydroxide, calcium carbonate and the like, and solutions thereof; and organic amines such as ethanol amine, triethylamine and the like. Preferable is aqueous sodium hydroxide.

### Examples

The present invention will now be explained in further detail with reference to examples, although the present invention is not limited to these.

### Reference Example 1:

### Preparation of 5-(2-chloro-4,5-difluoro-benzoylamino)-1H-pyrazole-3-carb oxylic acid imidazolide and benzotriazol-1-yl-5-(2-chloro-4,5-difluoro-benzoylamino)-1H-pyrazole-3-carboxylate;

### Step 1: Preparation of 2-chloro-4,5-difluorobenzoyl chloride;

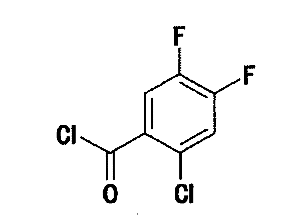

To a solution of 2-chloro-4,5-difluorobenzoic acid (508.20g) in toluene (250 mL) was addedN,N-dimethylformamide (0.2mL), and the resulting mixture was heated to 65°C, followed by addition of thionyl chloride (230 mL) dropwise. After stirring for 3 hours at 120°C , the reaction solution was concentrated under reduced pressure, and the resulting residue was distilled under reduced pressure (bp of 51 to 64°C (130 to 140 Pa)) to obtain the title compound (534.45 g) as an oil.

### Step 2: Preparation of Ethyl 5-nitro-3-pyrazolecarboxylate;

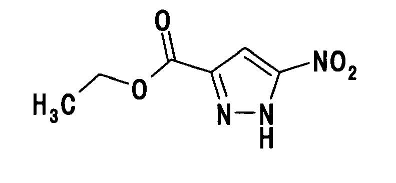

To a solution of 5-nitro-3-pyrazole carboxylic acid (310.24 g) in ethanol (3 L) was added methanesulfonic acid (143 mL), and the resulting mixture was stirred overnight at 94°C. After the resulting reaction solution was concentrated under reducedpressure, water (1.5 L) was added to the resulting residue followed by addition of aqueous potassium carbonate (prepared by dissolving 153.07 g of potassium carbonate in 750 mL of water) with ice-bath cooling. The formed solid was filtered to obtain the title compound (343.09 g) as white crystals.

### Step 3: Preparation of Ethyl 5-amino-3-pyrazolecarboxylate;

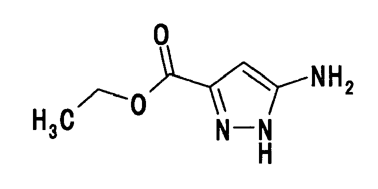

To a solution of ethyl 5-nitro-3-pyrazolecarboxylate (331.48g) in tetrahydrofuran (1.5 L) and ethyl acetate (1.5 L) was added 7.5% palladium-carbon (30.036 g), and the mixture was stirred overnight at room temperature under a hydrogen atmosphere. After the catalyst was removed by filtration, the filtrate was concentrated under reduced pressure and the residual solid was recrystallized from ethyl acetate-hexane to obtain the title compound (265.38 g) as white crystals.

### Step 4: Preparation of Ethyl 5-(2-chloro-4,5-difluoro-benzoylamino)-1H-pyrazole-3-carboxylate;

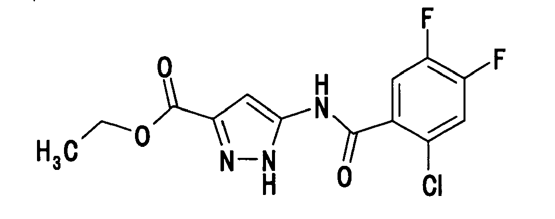

To a solution of ethyl 5-amino-3-pyrazolecarboxylate (265.38 g) in tetrahydrofuran (1.33 L) was added pyridine (153 mL). 2-chloro-4,5-difluorobenzyl chloride (362.00 g) obtained in Step 1 of Reference Example 1 was added dropwise to the resulting solution with ice-bath cooling and the resulting mixture was stirred for 2 hours at room temperature.The resulting reaction mixture was concentrated under reduced pressure, and then recrystallized from 2-propanol-water to obtain the title compound (538.85 g) as white crystals.

### Step 5: Preparation of 5-(2-chloro-4,5-difluoro-benzoylamino)-1H-pyrazole-3-carboxylic acid;

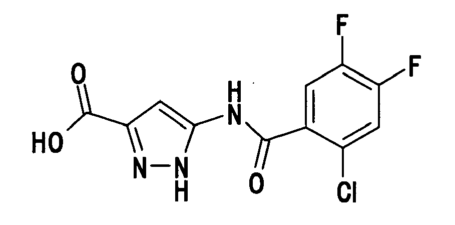

To a suspension of ethyl 5-(2-chloro-4,5-difluoro-benzoylamino)-1H-pyrazole-3-carb oxylate (568.22 g) in ethanol (1 L), water (1L) was added followed by addition of 4 N aqueous sodium hydroxide (500 mL) with ice-bath cooling. After stirring for 3 days at room temperature, the reaction mixture was added dropwise to 3 N hydrochloric acid (1.2 L) with ice-bath cooling. To the resulting suspension was added water (2 L), and the mixture was stirred at room temperature. The formed crystals were filtered, and dried under reduced pressure at 110°C to obtain the title compound (504.71 g) as white crystals.

### Step 6: Preparation of benzotriazol-1-yl 5-(2-chloro-4,5-difluoro-benzoylamino)-1H-pyrazole-3-carboxylate;

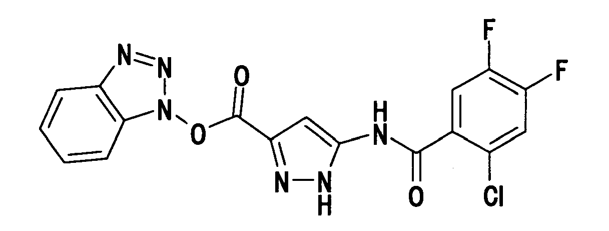

To a solution of 5-(2-chloro-4,5-difluoro-benzoylamino)-1H-pyrazole-3-carb oxylicacid (4.88 g) in N,N-dimethylformamide (70mL)was added 1-hydroxybenzotriazole monohydrate (2.73 g) and then EDC hydrochloride (3.42 g). After stirring at room temperature for 24 hours, 120 mL of water was added to the reaction mixture in several portions over about 2 hours. The formed solid was filtered to obtain the title compound (5.84 g) as a yellow solid.
H NMR(400MHz, DMSO-d₆)
δ: 13.52(3H, br s), 11.25(2H, s), 8.01-7.82(4H, m), 7.75-7.71(1H, m), 7.60-7.54(1H, m), 7.45-7.41(1H, m), 7.04(1H, s).

### Step 7: Preparation of 5(2-chloro-4,5-difluoro-benzoylamino-1H-pyrazole-3-carboxylic acid imidazolide;

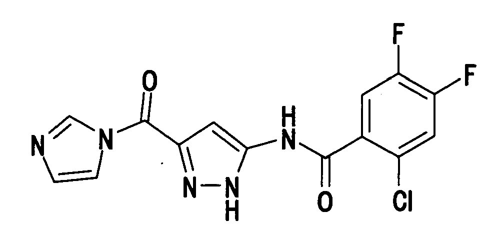

To a suspension of 1,1'-carbonyldiimidazole (64.65 g) in tetrahydrofuran (1.3 L) was added dropwise a solution of 5-(2-chloro-4,5-difluorobenzoylamino)-1H-pyrazole-3-carbo xylic acid (100.05 g) in tetrahydrofuran (700 mL) at 55°C. After stirring at 73°C for 1 hour, ethyl acetate (500 mL) was added and then concentrated under reduced pressure. To the residue were added ethyl acetate (1 L) and hexane (2 L), and to the resulting suspension was added ice water (1 L) with ice-bath cooling. After stirring for 10 minutes, the formed solid was filtered to obtain the title compound (110.42 g) as white crystals.
¹H NMR (400MHz, DMSO-d₆)
δ: 13.97 (1H, br s), 11.66 (1H, br s), 8.76 (1H, s), 8.05-7.87 (3H, m), 7.18 (1H, s), 6.90 (1H, s).

### Reference Example 2: Preparation of 3-aminomethyl-2-isopropoxy-pyridine Step 1: Preparation of 2-isopropoxy-nicotinamide;

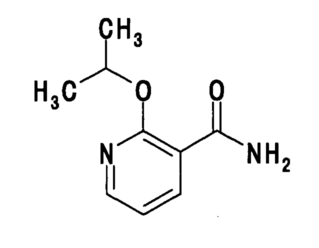

To a solution of 2-chloronicotinamide (1.50 g) in 2-propanol (45m) was added 60% sodiumhydride (640 mg) . After stirring for 2 days at 100°C , the resulting mixture was added to water (50 mL), and extracted with ethyl acetate (80 mL). The organic layer was washed with 1 N hydrochloric acid, dried over anhydrous sodium sulfate, and then concentrated. The resulting residue was purified by silica gel column chromatography (n-hexane-ethyl acetate, 5:1□2:1) to obtain the title compound (1.64 g) as colorless crystals.

### Step 2: Preparation of 2-isopropoxy-nicotinonitrile;

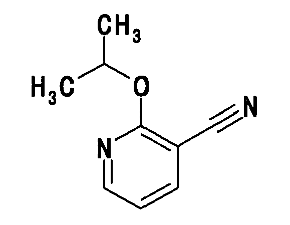

To a solution of 2-isopropoxy-nicotinamide (1.64 g) in chloroform (16 mL) was added triethylamine (2.8 mL). The resulting solution was added trichloroacetyl chloride (1.1 mL) dropwise with ice-bath cooling. After stirring overnight at room temperature, saturated aqueous sodium hydrogencarbonate was added to the resulting reaction solution and the resulting mixture was stirred for 30 minutes. The organic layer was washed with brine, dried over anhydrous sodium sulfate, and then concentrated under reduced pressure to obtain the title compound (1.37 g) as a brown oil.

### Step 3: Preparation of 3-aminomethyl-2-isopropoxy-pyridine;

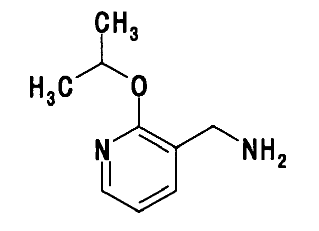

To a solution of 2-isopropoxy-nicotinonitrile (1.37 g) in methanol (25 mL) were added concentrated hydrochloric acid (2.1mL) and 7.5% palladium-carbon (780mg), and this resulting mixture was stirred under a hydrogen atmosphere for 18 hours at room temperature. After the catalyst was removed by filtration, the filtrate was concentrated under reduced pressure, and the residual oil was dissolved in water and washed with ethyl acetate. Then the aqueous layer was basified and extracted with chloroform.

The residue obtained by concentration of the organic layer was purified by silica gel column chromatography (chloroform-methanol, 8:1) to obtain the title compound (545 mg) as a brown oil.

Next, the following compounds were prepared in accordance with the above-described Preparation Method A-1.

### [Example 1-1]

### Preparation of 5-(2-chloro-4,5-difluoro-benzoylamino)-1H-pyrazole-3-carboxylic acid (pyridin-3-ylmethyl)-amide;

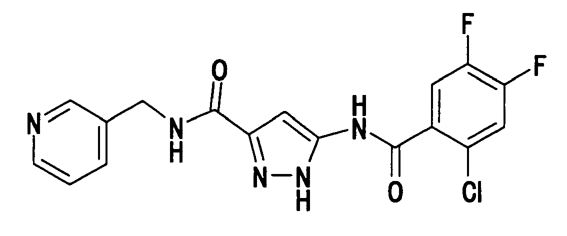

To a suspension of 5-(2-chloro-4,5-difluoro-benzoylamino)-1H-pyrazole-3-carb oxylic acid imidazolide (6.00g) obtained in Step 7 of Reference Example 1 in N,N-dimethylformamide (50 mL) was added 3-picolylamine (1.72 mL) with ice-bath cooling. After stirring overnight at room temperature, water (25 mL) and saturated aqueous sodium hydrogencarbonate (50 mL) were added to the resulting reaction mixture. The formed solid was filtered to obtain the title compound (4.49 g) as a white solid.

### [Example 1-1-1]

### Preparation of 5-(2-chloro-4,5-difluoro-benzoylamino)-1H-pyrazole-3-carboxylic acid (pyridin-3-ylmethyl)-amide dihydrochloride;

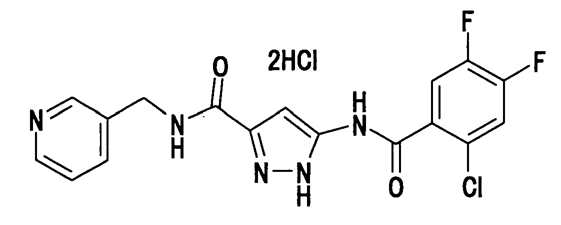

To a suspension of 5-(2-chloro-4,5-difluoro-benzoylamino)-1H-pyrazole-3-carb oxylic acid (pyridin-3-ylmethyl)-amide (1.05 g) obtained in Example 1-1 in methanol (10 mL) was added 4 N hydrochloric acid/ethyl acetate (1.8 mL). The resulting reaction mixture was concentrated under reduced pressure until the remaining residue was about 5 mL, after which the formed solid was filtered to obtain the title compound.(1.13 g) as white crystals.

### [Example 1-1-2]

### Preparation of 5-(2-chloro-4,5-difluoro-benzoylamino)-1H-pyrazole-3-carboxylic acid (pyridin-3-ylmethyl)-amide hydrochloride;

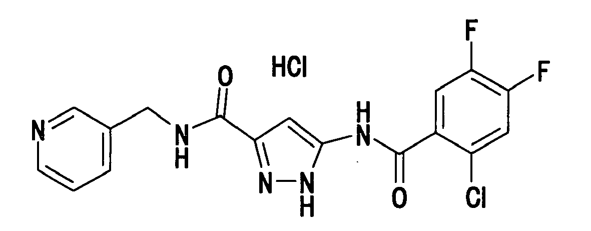

To a suspension of 5-(2-chloro-4,5-difluoro-benzoylamino)-1H-pyrazole-3-carb oxylic acid (pyridin-3-ylmethyl)-amide (700 mg) obtained in Example 1-1 in methanol (15 mL) was added 4 N hydrochloric acid/ethyl acetate (1.5 mL). This reaction mixture was concentrated under reduced pressure, the residue was added methanol (7.5mL) and stirred for 8 hours at 80°C . The reaction mixture was cooled to room temperature and the formed solid was filtered to obtain the title compound (638 mg) as white crystals.

### [Example 1-2]

### Preparation of 5-(2-chloro-4,5-difluoro-benzoylamino)-1H-pyrazole-3-carboxylic acid (4-methyl-oxazol-5-ylmethyl)-amide

### Step 1: Preparation of 5-chloromethyl-4-methyl-oxazole;

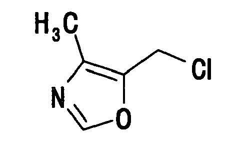

To a solution of 5-hydoxymethyl-4-methy-oxazole (1.01 g) in chloroform (10 mL) was added triethylamine (1.4 mL). The reaction mixture was added methanesulfonyl chloride (0.83 mL) with ice-bath cooling and stirred overnight at room temperature. Saturated aqueous sodium hydrogencarbonate was added to the resulting mixture, stirred for 30 minutes. After which the organic layer was separated, washed with brine, dried over anhydrous sodium sulfate, and then concentrated under reduced pressure to obtain the title compound (955 mg) as a brown oil.

### Step 2: Preparation of 5-aminomethyl-4-methyl-oxazole;

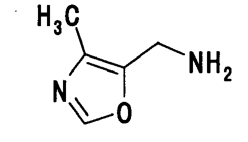

To a solution of 5-chloromethyl-4-methyl-oxazole (955 mg) in dioxane (20 mL) was added 28% aqueous ammonia (100 mL), and the resulting mixture was stirred overnight at room temperature. The resulting reaction mixture was concentrated under reduced pressure, added 8 N aqueous potassium hydroxide (20 mL), and extracted with tetrahydrofuran (20 mL). The organic layer was dried over anhydrous sodium sulfate, concentrated under reduced pressure to obtain the title compound (502 mg) as a brown oil.
Reference: Synth. Commun. 22 (13), 1939-1948, 1992

### Step 3: Preparation of 5-(2-chloro-4,5-difluoro-benzoylamino)-1H-pyrazole-3-carboxylic acid (4-methyl-oxazol-5-ylmethyl)-amide;

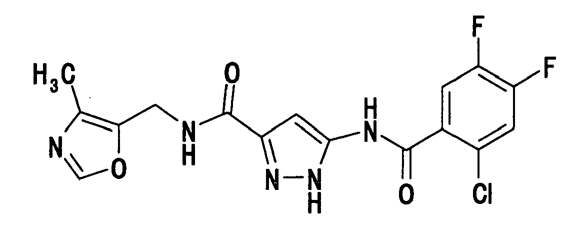

To a suspension of 5-(2-chloro-4,5-difluoro-benzoylamino)-1H-pyrazole-3-carb oxylic acid imidazolide (2.00g) obtained in step 7 of Reference Example 1 in N,N-dimethylformamide (6 mL) was added 5-aminomethyl-4-methyl-oxazole(502 mg) obtained in the above Step 2, and the resulting mixture was stirred overnight at room temperature. Saturated aqueous sodium hydrogencarbonate (30 mL) and then water (30 mL) were added to the resulting reaction mixture. The formed solid was filtered to obtain the title compound (1.11g) as a white solid.

### [Example 1-2-1a]

### Preparation of 5-(2-chloro-4,5-difluoro-benzoylamino)-1H-pyrazole-3-carboxylic acid (4-methyl-oxazol-5-ylmethyl)-amide sesquihydrochloride;

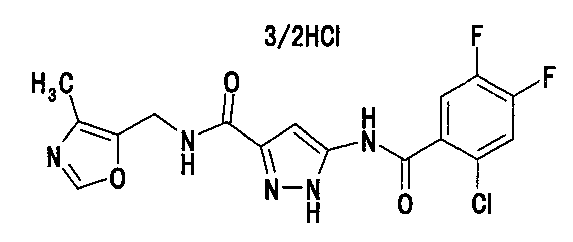

To a suspension of 5- (2-chloro-4,5-difluoro-benzoylamino) -1H-pyrazole-3-carb oxylic acid (4-methyl-oxazol-5-ylmethyl)-amide (200 mg) obtained in Example 1-2 in methanol (2 mL) was added 4 N hydrochloric acid/ethyl acetate (0.15 mL). Ethyl acetate was added to the resulting mixture and the formed solid was filtered to obtain the title compound (53 mg) as a white solid.

### [Example 1-2-1b]

### Preparation of 5-(2-chloro-4,5-difluoro-benzoylamino)-1H-pyrazole-3-carb oxylic acid (4-methyl-oxazol-5-ylmethyl)-amide p-toluenesulfonate;

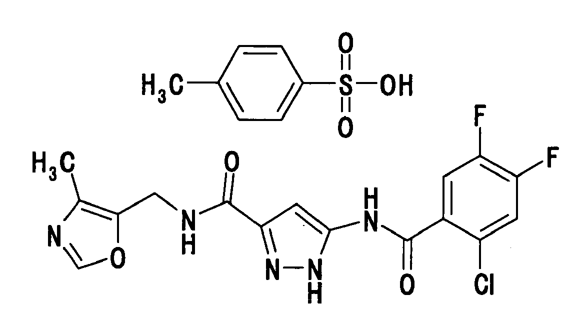

To a solution of 5-(2-Chloro-4,5-difluoro-benzoylamino)-1H-pyrazole-3-carb oxylic acid (4-methyl-oxazol-5-ylmethyl)-amide (828 mg) in acetone (8 mL) was added a solution of p-toluenesulfonic acid monohydrate salt (380 mg) in ethanol (1.9 ml) at 55°C. After 2 hours, the reaction mixture was cooled to room temperature and stirred for 2 hours, and a formed solid was filtered to obtain the title compound (1.052 g) as a white solid.

### [Example 1-2-1c]

### Preparation of 5-(2-chloro-4,5-difluoro-benzoylamino)-1H-pyrazole-3-carboxylic acid (4-methyl-oxazol-5-ylmethyl)-amide L-(+)-tartrate;

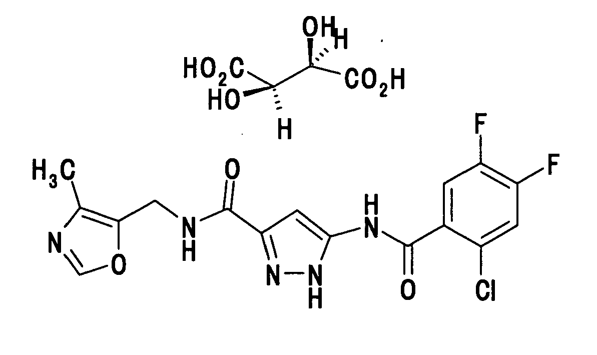

To a solution of 5-(2-Chloro-4,5-difluoro-benzoylamino)-1H-pyrazole-3-carb oxylic acid (4-methyl-oxazol-5-ylmethyl)-amide (100 mg) in acetone (1.1 mL) was added L- (+) -tartrate (45 mg), and this reaction mixture was stirred at room temperature for one day. The formed solid was filtered to obtain the title compound (70 mg) as a white solid.

### [Example 1-2-2]

### Preparation of 5-(2-chloro-4,5-difluoro-benzoylamino)-1H-pyrazole-3-carboxylic acid (2,4-dimethyl-oxazol-5-ylmethyl)-amide p-toluenesulfonate;

### Step 1: Preparation of Ethyl 2,4-dimethyl-oxazole-5-carboxylate;

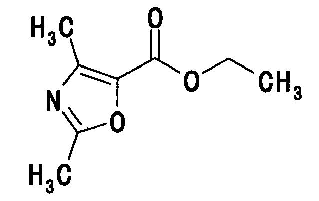

Acetamide (17.01 g) was added to ethyl 2-chloroacetoacetate (22.91 g), and stirred for 28 hours at 120°C. Water (75 mL) and ethanol (7.5 mL) were added to the resulting reaction mixture and the mixture was cooled with ice-bath, after which the formed solid was filtered to obtain the title compound (10.821 g) as a brown solid.

### Step2:Preparation of2,4-dimethyl-5-hydroxymethyl-oxazole;

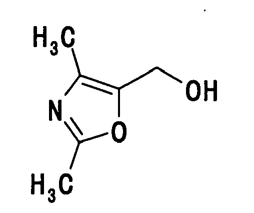

To a suspension of lithium aluminum hydride (1.82 g) in tetrahydrofuran (30 mL) was added a solution of ethyl 2,4-dimethyl-oxazole-5-carboxylate (7.845 g) in tetrahydrofuran (30 mL) dropwise with ice-bath cooling. After stirring for 1 hour 30 minutes, water (1.8 mL), 15% aqueous sodium hydroxide (1.8mL) and water (5.4 mL) were successively added to the resulting reaction mixture, and the mixture was stirred at room temperature. The mixture was dried over anhydrous sodium sulfate, and the dried solution was concentrated under reduced pressure to obtain the title compound (5.539 g) as a yellow oil.

### Step 3: Preparation of 5-chloromethyl-2,4-dimethyl-oxazole hydrochloride;

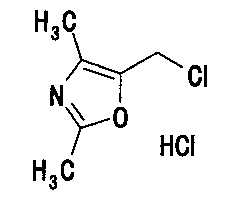

To a solution of 2,4-dimethyl-5-hydroxymethyl-oxazole (5.539 g) in chloroform (50 mL), thionyl chloride (4.2 mL) was added dropwise with ice-bath cooling. After stirring for 3 hours at room temperature, the resulting reaction mixture was concentrated under reduced pressure to obtain the title compound (6.626 g) as a brown solid.

### Step 4: Preparation of 5-azidomethyl-2,4-dimethyl-oxazole;

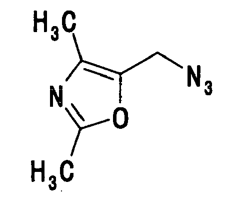

To a solution of 5-chloromethyl-2,4-dimethyl-oxazole hydrochloride (6.626g) inN,N-dimethylformamide (18 mL) were added triethylamine (6 mL) and then 20% aqueous lithium azide (10 mL), and the resulting mixture was stirred for 6 hours at 60°C . Ethyl acetate (100 mL) was added to the resulting reaction mixture, and the resulting mixture was successively washed with water and brine, dried over anhydrous sodium sulfate, and then concentrated under reduced pressure to obtain the title compound (4.31 g) as a brown oil.

### Step 5: Preparation of 5-aminomethyl-2,4-dimethyl-oxazole dihydrochloride;

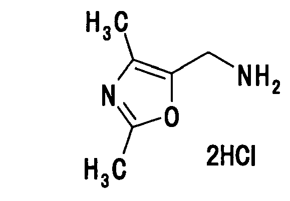

To a solution of 5-azidomethyl-2,4-dimethyl-oxazole (4.31 g) in tetrahydrofuran (45mL) was added a solution of triphenylphosphine (8.27 g) in tetrahydrofuran (25mL), followed by water (1.2 mL) at 50°C, and stirred overnight. The resulting reaction mixture was concentrated under reduced pressure, after which diethyl ether (50 mL) was added to the resulting residue, and the formed solid was filtered off. The filtrate was concentrated under reduced pressure and the residue was dissolved in ethyl acetate (20 mL), added 4 N hydrochloric acid/ethyl acetate (15 mL), and the formed solid was filtered to obtain the title compound (5.30 g) as a yellow solid.

### Step 6: Preparation of 5-(2-chloro-4,5-difluoro-benzoylamino)-1H-pyrazole-3-carboxylic acid (2,4-dimethyl-oxazol-5-ylmethyl)-amide;

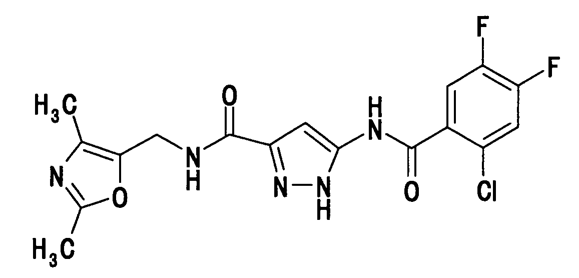

To a solution of 5-aminomethyl-2,4-dimethyl-oxazole dihydrochloride (772 mg) inN,N-dimethylformamide (3 mL) were added triethylamine (1.14 mL) and 5- (2-chloro-4,5-difluoro-benzoylamino) -1H-pyrazole-3-carb oxylic acid imidazolide (1.37 g), and stirred overnight at room temperature. The reactionmixture was added water (24 mL), and the formed solid was filtered to obtain the title compound (1.47 g) as a white solid.

### Step 7: Preparation of 5-(2-chloro-4,5-difluoro-benzoylamino)-1H-pyrazole-3-carboxylic acid (2,4-dimethyl-oxazol-5-ylmethyl)-amide p-tolyenesulfonate;

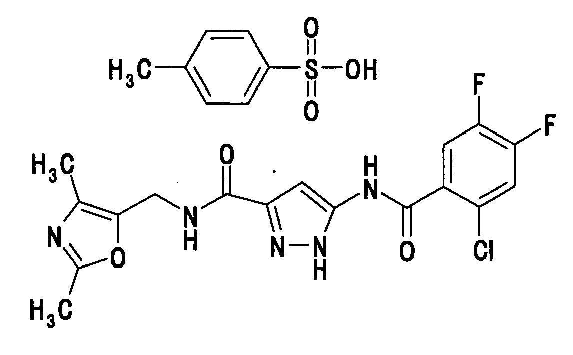

To 5-(2-chloro-4,5-difluoro-benzoylamino)-1H-pyrazole-3-carb oxylic acid (2,4-dimethyl-oxazol-5-ylmethyl)-amide (1.00 g) was added 1-propanol (6mL), and heated at reflux. The resulting solution was added a solution of p-toluenesulfonic acid (466 mg) in 1-propanol (0.5 mL), and the mixture was stirred under reflux for 30 minutes, and then cooled to room temperature and stirred overnight. The formed crystals were filtered to obtain the title compound (1.36 g) as colorless crystals.

### [Example 1-3]

### Preparation of 5-(2-chloro-4,5-difluoro-benzoylamino)-1H-pyrazole-3-carboxylic acid (2,4-dimethyl-pyridine-3-ylmethyl)-amide

### Step 1: Preparation of 3-hydroxymethyl-2,4-dimethylpyridine;

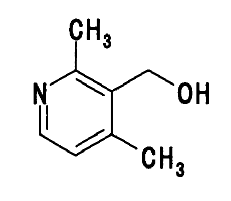

To a solution of ethyl 2,4-dimethylpyridine-3-carboxylate (3.00 g) in tetrahydrofuran (30 mL), lithium aluminum hydride (1.21 g) was added portionwise with ice-bath cooling. After stirring for 2 hours, water (1.2 mL), 15% aqueous sodium hydroxide (1.2 mL) and water (3.6 mL) were successively added to the resulting reaction mixture and the mixture was stirred at room temperature. The organic layer was obtained, dried over anhydrous sodium sulfate, and concentrated under reduced pressure to obtain the title compound (2.26 g) as white crystals.

### Step 2: Preparation of 3-chloromethyl-2,4-dimethylpyridine;

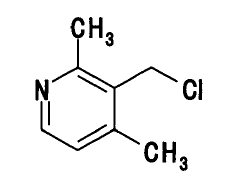

To a solution of 3-hydroxymethyl-2,4-dimethylpyridine (3.00 g) in chloroform (30 mL), thionyl chloride (2.8 mL) was added dropwise with ice-bath cooling. After stirring for 1 hour 30 minutes at room temperature, saturated aqueous sodium hydrogencarbonate (60 mL) was added to the resulting reaction mixture with ice-bath cooling. The organic layer was washed with brine, dried over anhydrous sodium sulfate and then concentrated under reduced pressure to obtain the title compound (3.45 g) as a brown oil.

### Step 3: Preparation of 3-azidomethyl-2,4-dimethylpyridine;

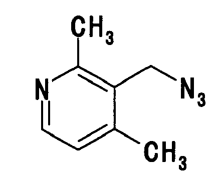

To a solution of 3-chloromethyl-2,4-dimethylpyridine (3.45g) inN,N-dimethylformamide (15mL) was added 20% aqueous lithium azide (5.5 mL) and resulting mixture was stirred overnight at 60°C. Ethyl acetate (100 mL) was added to the resulting reaction mixture, and the mixture was successively washed with water and brine, dried over anhydrous sodium sulfate and then concentrated under reduced pressure to obtain the title compound (3.18 g) as a brown oil.

### Step 4: Preparation of 3-aminomethyl 2,4-dimethylpyridine;

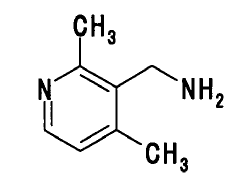

To a solution of 3-azidomethyl-2,4-dimethylpyridine (3.18 g) in ethanol (45 mL) was added to platinum oxide-carbon (613 mg), and the mixture was stirred for 30 minutes at 4 atm hydrogen atmosphere at room temperature. The catalyst was removed by filtration, and the filtrate was concentrated under reduced pressure to obtain the title compound (2.53 g) as a brown oil.

### Step 5: Preparation of 5-(2-chloro-4,5-difluoro-benzoylamino)-1H-pyrazole-3-carboxylic acid (2,4-dimethyl-pyridin-3-ylmethyl) -amide;

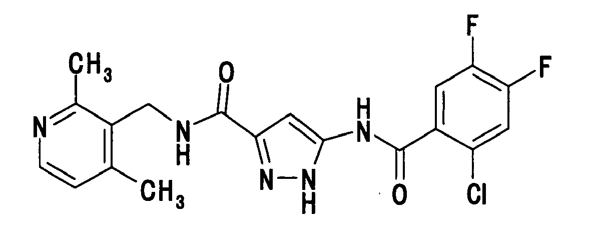

To a solution of 3-aminomethyl-2,4-dimethylpyridine (2.53 g) obtained in Step 4 in N,N-dimethylformamide (15 mL) was added 5-(2-chloro-4,5-difluorobenzoylamino)-1H-pyrazole-3-carbo xylic acid imidazolide (6.21 g) obtained in Step 7 of Reference Example 1, and the resulting mixture was stirred overnight at room temperature. Water (50 mL) was added to the resulting reaction mixture followed by addition of 10% aqueous potassium hydroxide (15 mL), and the formed solid was filtered to obtain the title compound (7.02 g) as a white solid.

### [Example 1-3-1]

### Preparation of 5-(2-chloro-4,5-difluoro-benzoylamino)-1H-pyrazole-3-carboxylic acid (2,4-dimethyl-pyridin-3-ylmethyl)-amide dihydrochloride;

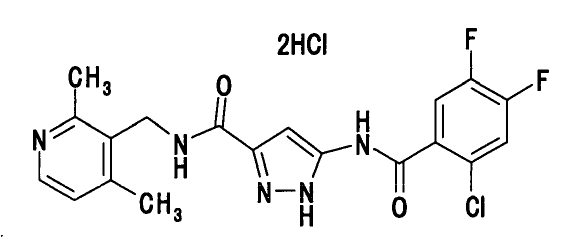

To a solution of the 5-(2-chloro-4,5-difluoro-benzoylamino)-1H-pyrazole-3-carb oxylic acid (2,4-dimethyl-pyridin-3-ylmethyl)-amide obtained in Example 1-3 (120 mg) in methanol (1 mL) was added 4 N hydrochloric acid/ethyl acetate (0.2 mL). Ethyl acetate was added to the resulting reaction mixture and the formed solid was filtered to obtain title compound (135 mg) as a white solid.

The compounds of Example 1-4 to Example 1-167 were prepared in the same manner as in the above Example 1-1 to Example 1-3-1. The following Tables 1 to 24 show structure and the NMR data for these compounds.

**Table 1**

| **Example** | **Molecular Structure** | **NMR** |
|---|---|---|
| 1-1 | 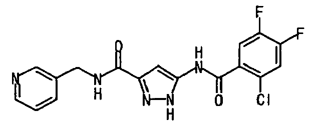 | ¹H-NMR (300 MHz, DMSO-*d*₆) δ:13.26 (1H, s), 11.41 (0.1H, s), 11.15 (0.9H, s), 9.18 (0.9H, br s), 8.78 (0.1H, br s), 8.56 (1 H, d, J = 1.5 Hz), 8.47 (1 H, dd, J = 4.9, 1.5 Hz), 7.96-7.66 (2.9H, m), 7.41-7.30 (2H, m), 6.43 (0.1H, s), 4.47 (2H, d, J = 6.0 Hz). |
| 1-1-1 | 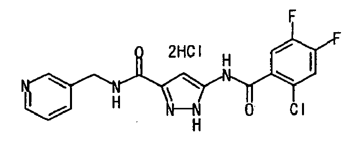 | ¹H-NMR(400 MHz, DMSO-*d*₆) δ: 11.22 (1H, s), 9.34 (1H, m), 8.90 (1H, s), 8.84 (1H, m), 8.50 (1H, m), 8.04 (1H, m), 7.87-7.80 (2H, m), 7.24 (1H, br s), 4.65 (2H, d, J = 6.0 Hz). |
| 1-1-2 | 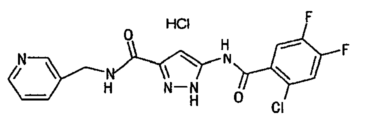 | ¹H-NMR (400 MHz, DMSO-*d*₆) δ: 11.23 (1 H, br s), 9.25 (1H, br s), 8.76 (1H, br s), 8.69 (1H, br s), 8.17 (1 H, br s), 7.90-7.80 (2H, m), 7.76 (1 H, br s), 7.30 (1H, br s), 4.57 (2H, br s). |
| 1-2 | 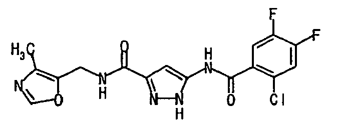 | ¹H-NMR(400 MHz, DMSO-*d*₆) δ: 13.27 (1H, s), 11.52 (1H, s), 9.09 (1H, t, J = 5.6 Hz), 8.19 (1H, s), 7.89-7.79 (2H, m), 7.32 (1H, s), 4.45 (2H, d, J = 5.6 Hz), 2.14 (3H, s). |
| 1-2-1a | 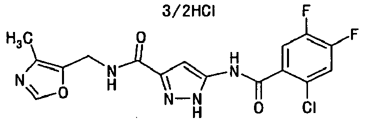 | ¹H-NMR(400 MHz, DMSO-*d*₆) δ: 11.18 (1H, s), 9.07 (1H, br s), 8.19 (1H, s), 7.87-7.80 (2H, m), 7.24(1H,s), 4.45 (2H, d, J = 5.6 Hz), 2.14 (3H, s). |
| 1-2-1b | 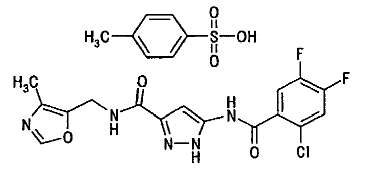 | ¹H-NMR(400 MHz, DMSO-*d*₆) δ: 11.17(IH,s), 9.05(1H,m), 8.19(1H,s), 7.86-7.80(2H,m), 7.49(1 H,d,J=8.0Hz), 7.24(1 H,s), 7.12(!H,d,J=8.4Hz), 4.45(2H,d,J=5.6Hz), 2.29(3H,s), 2.14(3H,s). |
| 1-2-1c | 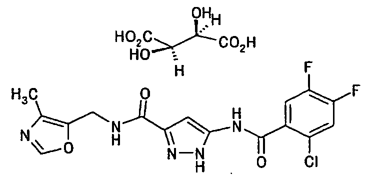 | ¹H-NMR(400 MHz, DMSO-*d*₆) δ: 11.17(1H,s), 9.05(1H,s), 8.72(1H,s), 7.85-7.80(2H,m), 7.25(1H,s), 4.45(2H,d,J=5.6Hz), 4.31(2H,s), 2.14(3H,s). |

**Table 2**

| **Example** | **Molecular Structure** | **NMR** |
|---|---|---|
| 1-2-2 | 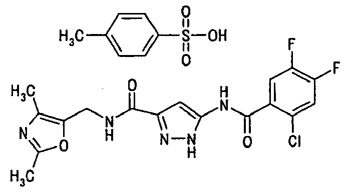 | ¹H-NMR(400 MHz, DMSO-*d*₆) δ: 11.18 (1H, s), 9.04 (1 H, t, J = 5.2 Hz), 7.87-7.80 (2H, m), 7.48 (2H, d, J = 7.8 Hz), 7.25 (1 H, s), 7.12 (2H, d, J = 7.8 Hz), 4.40 (2H, d, J = 5.2 Hz), 2.35 (3H, s), 2.29 (3H, s), 2.09 (3H, s). |
| 1-3 | 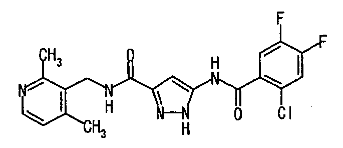 | ¹H-NMR(400 MHz, DMSO-*d*₆) δ: 13.23 (1H, br s), 11.13 (1H, br s), 9.00 (1H, br s), 8.22 (1H, d, J = 4.8 Hz), 7.85-7.78 (2H, m), 7.34 (1 H, br s), 7.06 (1H, d, J = 4.8 Hz), 4.49 (2H, d, J = 4.8 Hz), 2.55 (3H, s), 2.36 (3H, s). |
| 1-3-1 | 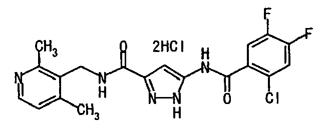 | ¹H-NMR(400 MHz, DMSO-*d*₆) δ: 11.21 (1H, s), 9.00 (1H, br s), 8.59 (1H, d, J = 6.0 Hz), 7.86-7.80 (2H, m), 7.79 (1H, d, J = 6.0Hz), 7.21 (1H, br s), 4.58 (2H, d, J = 4.8 Hz), 2.87 (3H, s), 2.67 (3H, s). |
| 1-4 | 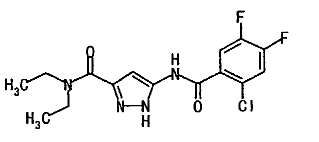 | ¹H-NMR(400 MHz, DMSO-*d*₆) δ: 13.12 (1H, br s), 11.17 (1H, brs), 7.87-7.80 (2H, m), 6.87 (1H, br s), 3.60-3.40 (4H, m), 1.26-1.10 (6H, m). |
| 1-5 | 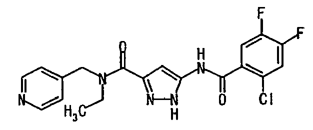 | ¹H-NMR(400 MHz, DMSO-*d*₆) δ: 13.29 (1H, br s), 11.20 (1H, m), 8.60-8.50 (2H, m), 7.89-7.70 (2H, m), 7.31-7.30 (2H, m), 7.01 (1H, br s), 4.90-4.68 (2H, m), 3.75-3.35 (2H, m), 1.18 (3H, m). |
| 1-6 | 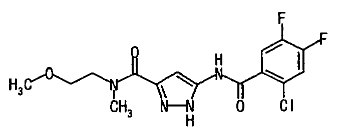 | ¹H-NMR(400 MHz, DMSO-*d*₆) δ: 13.12 (1H, M), 11.15 (1H, br s), 7.86-7.79 (2H, m), 6.93 (1H, br s), 3.71-3.63 (2H, m), 3.54 (2H, t, J = 5.2 Hz), 3.28 (6H, m). |
| 1-7 | 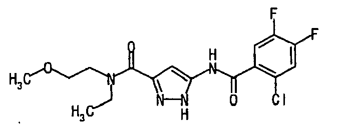 | ¹H-NMR(400 MHz, DMSO-*d*₆) δ: 13.11 (1H, m), 11.17 (1H, br s), 7.87-7.80 (2H, m), 6.86 (1H, br s), 3.71-3.49 (6H, m), 3.28 (3H, s), 1.15 (3H, m). |
| 1-8 | 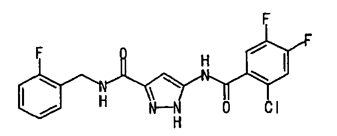 | ¹H-NMR (300 MHz, DMSO-*d*₆) δ: 13.23 (1H, s), 11.13 (1 H, s), 9.10 (1 H, m), 7.85-7.77 (2H, m), 7.41-7.30 (3H, m), 7.22-7.16 (2H, m), 4.49 (2H, d, J = 5.7 Hz). |

**Table 3**

| **Example** | **Molecular Structure** | **NMR** |
|---|---|---|
| 1-9 | 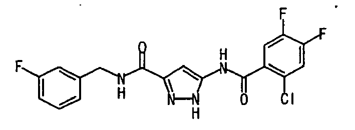 | ¹H-NMR(300 MHz, DMSO-*d*₆) δ: 13.24 (1H, s), 11.14 (1H, s), 9.16 (1H, m), 7.83-7.77 (2H, m), 7.42-7.36 (2H, m), 7.17-7.06 (3H, m), 4.46 (2H, d, J = 5.7 Hz). |
| 1-10 | 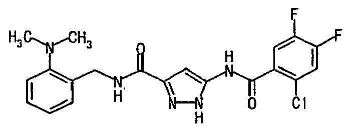 | ¹H-NMR(300 MHz, DMSO-*d*₆) δ: 13.21 (1H, s), 11.11 (1 H, s), 9.02 (1 H, m), 7.86-7.77 (2H, m), 7.37 (1H, s), 7.25-7.20 (2H, m), 7.13 (1H, m), 7.03 (1H, m), 4.54 (2H, d, J = 5.7 Hz), 2.66 (6H, s). |
| 1-11 | 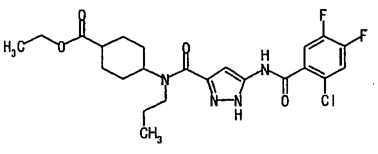 | ¹H-NMR(400 MHz, DMSO-*d*₆) δ: 13.11 (1H, br s), 11.16 (1H, br s), 7.86-7.79 (2H, m), 6.84 (1H, br s), 4.13 (2H, q, J = 7.2 Hz), 4.06 (1H, m), 3.29 (1H, m), 2.67-0.85 (15H, m), 1.22 (3H, t, J = 7.2 Hz), |
| 1-12 | 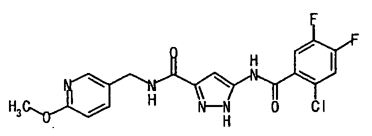 | ¹H-NMR(400 MHz, DMSO-*d*₆) δ: 13.24 (1H, s), 11.15 (1H, br s), 9.10 (1H, t, J = 6.4 Hz), 8.12 (1H,d, J = 2.4 Hz), 7.86-7.79 (2H, m), 7.66 (1H. dd, J = 8.4, 2.4 Hz), 7.31 (1H. d, J = 2.4 Hz), 6.80 (1H. d, J = 8.4 HZ), 4.37 (2H, d, J = 6.4 Hz), 3,83 (3H, s). |
| 1-13 | 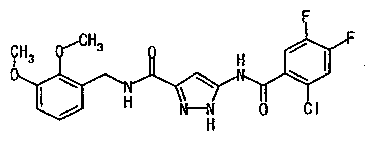 | ¹H-NMR(400 MHz, DMSO-*d*₆) δ: 13.22 (1H, br s), 11.14 (1H, br s), 9.01 (1H, br s), 7.86-7.80 (2H, m), 7.37 (1 H, br s), 7.03 (1 H, dd, J = 8.4, 7.2 Hz), 6.96 (1 H, dd, J = 8.4, 1.2 Hz), 6.86 (1 H, dd, J = 7.2, 1.2 Hz), 4.45 (2H, d, J = 6.0 Hz), 3.80 (3H, s), 3.77 (3H, s). |
| 1-14 | 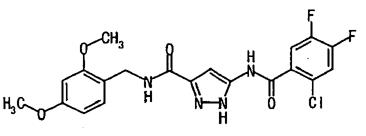 | ¹H-NMR(400 MHz, DMSO-d₆) δ: 13.18 (1H, br s), 11.13 (1H, br s), 8.85 (1H, br s), 7.86-7.80 (2H, m), 7.35 (IH.brs). 7.03 (1 H, d, J = 8.4 Hz), 6.56 (1H, d, J = 2.4 Hz), 6.49 (1H, dd, J = 8.4, 2.4 Hz), 4.33 (2H, d, J = 5.6 Hz), 3.81 (3H, s), 3.74 (3H, s). |
| 1-15 | 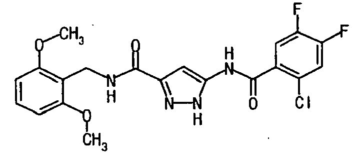 | ¹H-NMR(400 MHz, DMSO-*d*₆) δ: 8.19 (1H, br s), 7.83-7.76 (2H.m). 7.27 (1 H, t, J = 8.4 Hz), 7.17 (1H, brs). 6.68 (2H, d, J = 8.4 Hz), 4.43 (2H, d, J = 4.4 Hz), 3.79 (6H, s). |

**Table 4**

| **Example** | **Molecular Structure** | **NMR** |
|---|---|---|
| 1-16 | 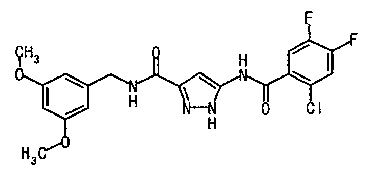 | ¹H-NMR(400 MHz, DMSO-*d*₆) δ: 13.24 (1H, br s), 11.15 (1H, br s), 9.10 (1H, m), 7.86-7.79(2H, m), 7.36 (1H, br s), 6.48 (2H, d, J = 2.4 Hz), 6.39 (1H, t, J = 2.4 Hz), 4.37 (2H, d, J =6.4 Hz), 3.72 (6H, s). |
| 1-17 | 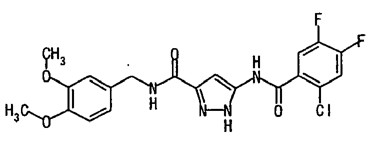 | ¹H-NMR(400 MHz, DMSO-*d*₆) δ: 13.23 (1H, br s), 11.15 (1H, br s), 9.06 (1H, br s), 7.86-7.80 (2H, m), 7.34 (1 H, br s), 6.94 (1 H, d, J = 2.0 Hz), 6.90 (1H, d, J = 8.0 Hz), 6.83 (1H, dd, J = 8.0, 2.0 Hz),4.37 (2H, d, J = 6.0 Hz), 3.74 (3H, s), 3.72 (3H, s). |
| 1-18 | 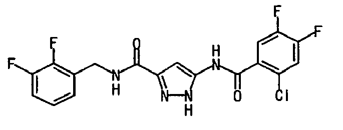 | ¹H-NMR(400 MHz, DMSO-*d*₆) δ: 13.27 (1H, s), 11.16 (1H, m), 9.18 (1H, m), 7.86-7.79 (2H, m), 7.37-7.31 (2H, m), 7.23-7.16 (2H, m), 4.52 (2H, d, J = 5.6 Hz). |
| 1-19 | 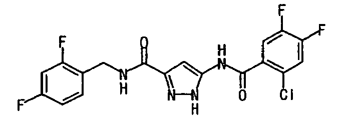 | ¹H-NMR(400 MHz, DMSO-*d*₆) δ: 13.25 (1H, br s), 11.16 (1H, br s), 9.10 (1H, br s), 7.86-7.80 (2H, m), 7.43 (1H, dd, J = 15.6, 8.4 Hz), 7.35 (1H, br s), 7.23 (1 H, m), 7.08 (1H, m), 4,45 (2H, d, J = 5.6 Hz). |
| 1-20 | 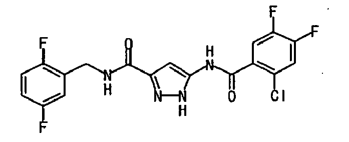 | ¹H-NMR(400 MHz, DMSO-*d*₆) δ: 13.28 (1H, br s), 11.17 (1H, br s), 9.15 (1H, br s), 7.87-7.80 (2H, m), 7.38 (1H, br s), 7.27 (1H, m), 7.20-7.15 (2H, m), 4.47 (2H, d, J = 5.6 Hz). |
| 1-21 | 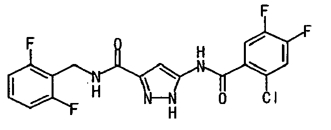 | ¹H-NMR(400 MHz, DMSO-*d*₆) δ: 13.22 (1H, br s), 11.13 (1H, br s), 8.98 (1H, br s), 7.85-7.78 (2H, m), 7.41 (1H.m),7.31 (1H, br s), 7.14-7.05 (2H, m), 4.45 (2H, d, J = 5.2 Hz). |
| 1-22 | 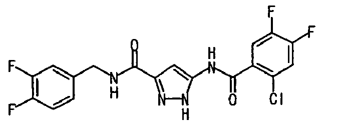 | ¹H-NMR(400 MHz, DMSO-*d*₆) δ:13.22 (1H, br s), 11.21 (1H, brs), 9.13 (1H, brs), 7.86-7.80 (2H, m), 7.43-7.34 (2H, m), 7.28 (1H, br s), 7.17 (1 H, m), 4.42 (2H, d, J = 5.6 Hz). |
| 1-23 | 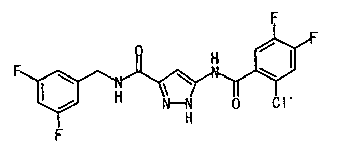 | ¹H-NMR(400 MHz, DMSO-*d*₆) δ: 13.28 (1H, br s), 11.19 (1 H, br s), 9.19 (1 H, brs). 7.87-7.80 (2H, m), 7.36 (1 H, brs). 7.12 (1H, m), 7.09-7.02 (2H, m), 4.46 (2H, d, J = 6.0 Hz). |

**Table 5**

| **Example** | **Molecular Structure** | **NMR** |
|---|---|---|
| 1-24 | 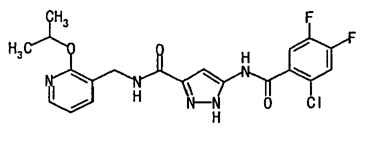 | ¹H-NMR(400 MHz, DMSO-*d*₆) δ:13.26 (1H, s), 11.17 (1H, br s), 8.95 (1H, br s), 8.04 (1H, dd, J =5.2, 2.0 Hz), 7.87-7.80 (2H, m), 7.51 (1H. d, J = 6.8 Hz), 7.37 (1H.m). 6.92 (1H,dd.J=6.8, 5.2 Hz), 5.32 (1H, m), 4.35 (2H, d, J = 5.6 Hz), 1.31 (6H, d, J = 6.4 Hz). |
| 1-25 | 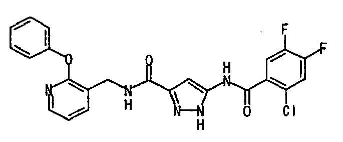 | ¹H-NMR(400 MHz, DMSO-*d*₆) δ:13.29 (1H, br s), 11.20 (1 H, s), 9.13 (1 H, br s), 8.01 (1 H, dd, J = 5.2, 1.6 Hz), 7.87-7.81 (2H, m), 7.75 (1H, dd, J = 7.2, 1.6 Hz), 7.44-7.40 (2H, m), 7.26 (1H, m), 7.20 (1 H, m), 7.16-7.11 (3H, m), 4.55 (2H, d, J = 5.6 Hz). |
| 1-26 | 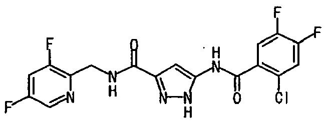 | ¹H-NMR(400 MHz, DMSO-*d*₆) δ: 13.23 (1H, s), 11.14 (1H, s), 9.11 (1H, d, J = 5.6 Hz), 8.48 (1H, m), 7.94 (1H, m), 7.86-7.78 (2H, m), 7.35 (1H, d, J = 1.6 Hz), 4.59 (2H, d, J = 5.6 Hz). |
| 1-27 | 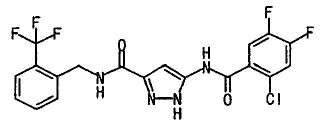 | ¹H-NMR(400 MHz, DMSO-*d*₆) δ: 13.30 (1H, br s), 11.19 (1 H, br s), 9.22 (1H, br s), 7.87-7.81 (2H, m), 7.74 (1H, d, J = 8.0 Hz), 7.68 (1H, dd, J =7.2, 7.2Hz),7.55-7.48(2H, m), 7.42(1H,br s), 4.63 (2H, d, J = 5.2 Hz). |
| 1-28 | 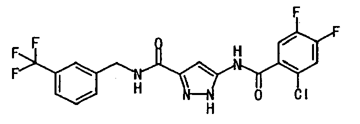 | ¹H-NMR(400 MHz, DMSO-*d*₆) δ: 13.28 (1H, br s), 11.18 (1 H, br s), 9.23 (1H, br s), 7.87-7.80 (2H, m), 7.67-7.57 (4H, m), 7.36 (1 H, br s), 4.53 (2H, d, J = 6.0Hz), |
| 1-29 | 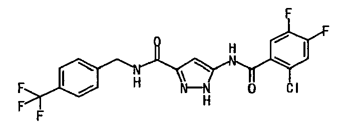 | ¹H-NMR(400 MHz, DMSO-*d*₆) δ: 13.29 (1H, s), 11.18 (1H, s), 9.25 (1H, t, J = 5.6 Hz), 7.87-7.80 (2H, m), 7.71 (2H, d, J = 8.0 Hz), 7.54 (2H, d, J = 8.0 Hz), 7.38 (1H, m), 4.53 (2H, d, J=5.6 Hz). |
| 1-30 | 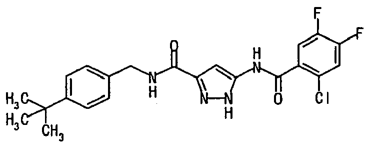 | ¹H-NMR(400 MHz, DMSO-*d*₆) δ: 13.21 (1H, br s), 11.15(1H. br s), 9.07 (1H, brs). 7.86-7.80 (2H, m), 7.35 (2H, d, J=8.4 Hz), 7.34 (1H, br s), 7.24 (2H, d, J=8.4 Hz), 4.40 (2H, d, J=6.0 Hz), 1.26(9H.s). |
| 1-31 | 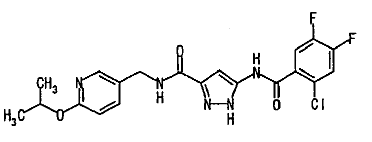 | ¹H-NMR(400 MHz, DMSO-*d*₆) δ: 13.24 (1H, s), 11.15 (1H, brs). 9.08 (1H, t, J = 5.2 Hz), 8.09 (1H, d, J = 2.4 Hz), 7.86-7.79 (2H, m), 7.63 (1H. dd, J = 8.4, 2.4 Hz), 7.32 (1H, s), 6.71 (1H, d, J = 8.4Hz). 5.21 (1H.m), 4.35 (2H, d, J = 5.2 Hz), 1.27 (6H, d, J = 6.0 Hz). |

**Table 6**

| **Example** | **Molecular Structure** | **NMR** |
|---|---|---|
| 1-32 | 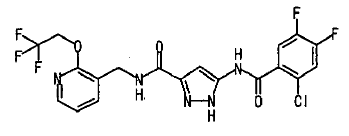 | ¹H-NMR(400 MHz, DMSO-d₆) δ: 13.27 (1H, s), 11.17 (1H, s), 9.06 (1H, t, J = 5.6 Hz), 8.11 (1H, dd, J = 5.2, 2.0 Hz), 7.86-7.79 (2H, m), 7.66 (1 H, d, J = 6.4 Hz), 7.38 (1H. d, J = 2.0 Hz), 7.12 (1 H, dd, J = 6.4, 5.2 Hz), 5.05 (2H, q, J = 9.2 Hz), 4.42 (2H, d, J = 5.6 Hz). |
| 1-33 | 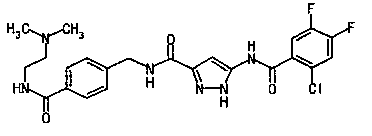 | ¹H-NMR(300 MHz, DMSO-d₆) δ: 11.19 (1H, br s), 11.13 (1H, br s), 9.16 (1H, br s), 8.55 (1H, br s), 7.92-7.71 (4H, m), 7.40 (2H, d, J = 8.1 Hz), 7.28 (1H, br s), 4.49 (2H, d, J = 5.5 Hz), 3.48 (2H, d. J = 5.9 Hz), 2.82 (2H, d, J = 6.6 Hz), 2.49 (6H, s). |
| 1-34 | 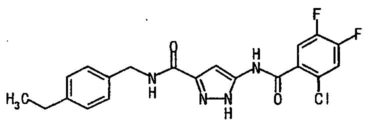 | ¹H-NMR(400 MHz, DMSO-*d*₆) δ: 13.22 (1H, br s), 11.15 (1H, br s), 9.08 (1H, br s), 7.86-7.80 (2H, m), 7.33 (1H, br s), 7.22 (2H, d, J = 7.6 Hz), 7.17 (2H, d, J = 7.6 Hz), 4.40 (2H, d, J = 6.0 Hz), 2.57 (2H, q, J = 7.6 Hz), 1.16 (3H, t, J = 7.6 Hz). |
| 1-35 | 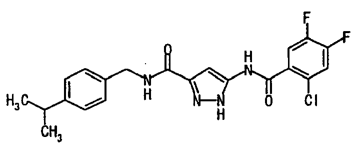 | ¹H-NMR(400 MHz, DMSO-*d*₆) δ: 13.22 (1H, br s), 11.14 (1H, br s), 9.09 (1H, br s), 7.86-7.80 (2H, m), 7.34 (1H, br s), 7.24 (2H, d, J = 8.4 Hz), 7.20 (2H, d, J = 8.4 Hz), 4.40 (2H, d, J =6.0 Hz), 2.86 (1 H, m), 1.18 (6H, d, J = 6.8 Hz). |
| 1-36 | 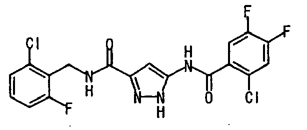 | ¹H-NMR(400 MHz, DMSO-*d*₆) δ: 13.22 (1H, br s), 11.13 (1H, br s), 8.89 (1 H, br s), 7.85-7.78 (2H, m), 7.44-7.34 (3H, m), 7.25 (1 H, m), 4.57 (2H, d, J = 4.0 Hz). |
| 1-37 | 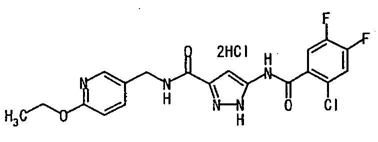 | ¹H-NMR(400 MHz, DMSO-*d*₆) δ: 11.19 (1H, s), 9.13 (1H, br s), 8.14 (1H, s), 7.87-7.79 (2H, m), 7.74 (1H, m), 7.23 (1H, s), 6.86 (1H, m), 4.38 (2H, d, J = 5.6 Hz), 4.30 (2H, m), 1.31 (3H, t, J = 7.2 Hz). |
| 1-38 | 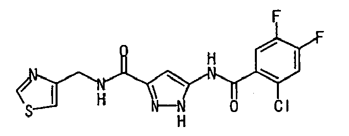 | ¹H-NMR(400 MHz, DMSO-*d*₆) δ:13.23 (1H, br s), 11.16 (1H, s), 9.14 (1H, br s), 9.06 (1H, d, J = 2.0 Hz), 7.87-7.80 (2H, m), 7.47 (1H, s), 7.34 (1H, br s), 4.58 (2H, d, J = 6.0 Hz). |
| 1-39 | 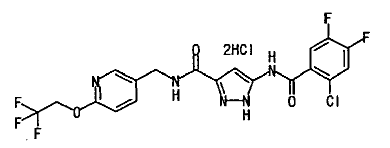 | ¹H-NMR(400 MHz, DMSO-*d*₆) δ: 11.18 (1H, s), 9.13 (1H, br s), 8.16 (1H, d, J = 2.4 Hz), 7.87-7.80 (2H, m), 7.77 (1H. dd, J = 8.4, 2.4 Hz), 7.24 (1H, s), 6.97 (1 H, d, J = 8.4 Hz), 4.98 (2H, q, J = 8.8 Hz), 4.41 (2H, d, J = 6.0 Hz). |

**Table 7**

| **Example** | **Molecular Structure** | **NMR** |
|---|---|---|
| 1-40 | 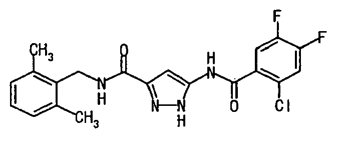 | ¹H-NMR(400 MHz, DMSO-*d*₆) δ: 13.15 (1H, br s), 11.12 (1H, br s), 8.61 (1H, br s), 7.85-7.77 (2H, m), 7.32 (1H, br s), 7.10 (1 H, m), 7.03 (2H, d, J = 7.2 Hz), 4.47 (2H, d, J = 4.4 Hz), 2.35 (6H, s). |
| 1-41 | 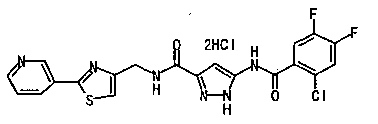 | ¹H-NMR(400 MHz, DMSO-*d*₆) δ: 11.21 (1H, br s), 9.30 (2H, br s), 8.83 (1 H, m), 8.70 (1 H, m), 7.94 (1H, m), 7.87-7.81 (2H, m), 7.71 (1H, m), 7.31 (1H, s), 4.65 (2H, d, J = 6.0 Hz). |
| 1-42 | 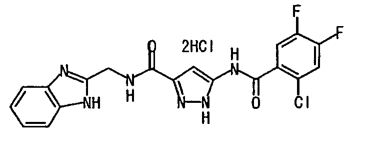 | ¹H-NMR(400 MHz, DMSO-d₆) δ:11.28 (1H, br s), 9.55 (1H, br s), 7.89-7.82 (2H, m), 7.79-7.77 (2H, m), 7.55-7.53 (2H, m), 7.34 (1 H, br s), 4.94 (2H, m). |
| 1-43 | 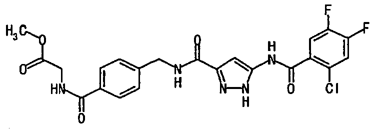 | ¹H-NMR(300 MHz, DMSO-d₆) δ: 13.25 (1H, br s), 11.16 (1H, br s), 9.18 (1 H, br s), 8.90 (1H, t, J = 5.9 Hz), 7.94-7.75 (4H, m), 7.38 (2H, d, J = 8.4 Hz), 7.27 (1 H, br s), 4.50 (2H, d, J = 5.9 Hz), 4.02 (2H, d, J = 5.9 Hz), 3.65 (3H, s). |
| 1-44 | 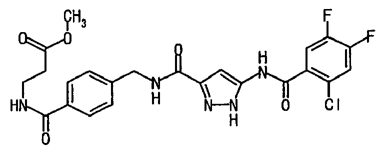 | ¹H-NMR(300 MHz, DMSO-d₆) δ: 13.19 (1H, br s), 11,10 (1H, br s), 9.13 (1H, br s), 8.46 (1H, t, J = 5.5 Hz), 8.22 (1H, d, J = 0.7 Hz), 7.80 (2H, d, J = 8.1 Hz), 7.74-7.64 (2H, m), 7.38 (2H, d, J = 8.1 Hz), 4.50 (2H, d, J = 5.9 Hz), 3.62 (3H, s), 3.51 (2H, q, J = 6.5 Hz), 2.59 (2H, t, J = 7.0 Hz). |
| 1-45 | 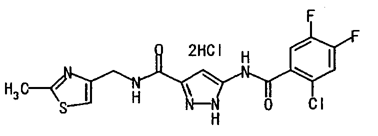 | ¹H-NMR (400 MHz, DMSO-*d*₆) δ: 11.18 (1H, s), 9.12 (1H, br s), 7.87-7.80 (2H, m), 7.29 (1H, s), 7.27 (1 H, br s), 4.49 (2H, d, J = 4.0 Hz), 2.67 (3H, br s). |
| 1-46 | 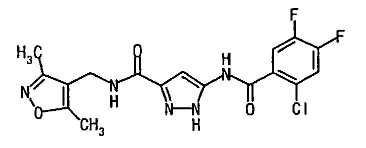 | ¹H-NMR(400 MHz, DMSO-*d*₆) δ: 13.25 (1H, s), 11.15(1H, s), 8.91 (1H, t, J = 5.2 Hz), 7.86-7.79 (2H, m), 7.31 (1H, m), 4.19-4.18 (2H, m), 2.40 (3H, s), 2.22 (3H, s). |
| 1-47 | 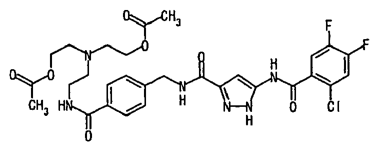 | ¹H-NMR(300 MHz, CD₃OD)) δ: 7.78 (2H, d, J = 8.4 Hz), 7.55 (4H, m), 7.45 (2H, d, J = 8.4 Hz), 4.60 (2H, br s), 4.13 (4H, t, J = 4.2Hz), 3.45 (2H, t, J = 9.6 Hz), 2.84 (4H, t, J = 4.2 Hz), 2.79 (2H, t, J = 4.8 Hz), 1.94 (6H, s). |

**Table 8**

| **Example** | **Molecular Structure** | **NMR** |
|---|---|---|
| 1-48 | 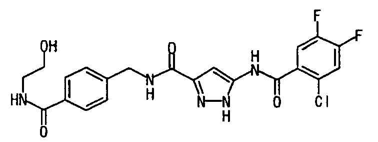 | ¹H-NMR(300 MHz, DMSO-d₆) δ: 13.27 (1H, br s), 11.17 (1 H, br s), 9.20 (1 H, br s), 8.40 (1 H, br s), 7.87 (3H, m), 7.35 (2H, d, J = 9.9 Hz), 4.73 (1H, t, J = 8.1 Hz), 4.50 (2H, d, J = 6.2 Hz), 3.55-3.47 (2H, m), 3.34-3.30 (2H, m). |
| 1-49 | 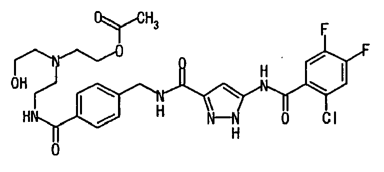 | ¹H-NMR(300 MHz, DMSO-*d*₆) δ: 9.02 (1H, br s), 8.29 (1H, br s), 7.85-7.64 (4H, m), 7.38(2H, d, J = 8.4 Hz), 7.09 (1 H, br s), 4.46 (2H, d, J = 6.2 Hz), 4.04 (2H, t, J = 4.2Hz), 3.32 (2H, m), 2.76-2.55 (6H, m), 1.96 (3H, s). |
| 1-50 | 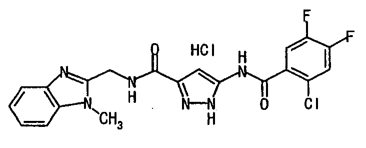 | ¹H-NMR(400 MHz, DMSO-*d*₆) δ: 11.29 (1H, br s), 9.53 (1H, br s), 7.95 (1 H, m), 7.89-7.78 (3H, m), 7.63-7.57 (2H, m), 7.34 (1H. br s), 5.00 (2H, m), 4.04 (3H, s). |
| 1-51 | 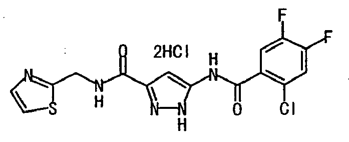 | ¹H-NMR(400 MHz, DMSO-*d*₆) δ: 11.23 (1H, s), 9.49 (1H, br s), 7.88-7.82 (2H, m), 7.76 (1H, m), 7.66 (1H, m), 7.29 (1H, s), 4.73 (2H, d, J = 5.6 Hz). |
| 1-52 | 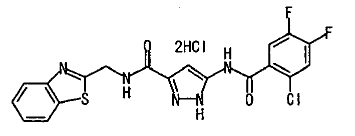 | ¹HNMR(400 MHz, DMSO-*d*₆) δ:11.29 (1H, br s), 9.53 (1H, br s), 8.06 (1H, d, J = 7.2 Hz), 7.97 (1 H, d, J = 7.2 Hz), 7.87-7.83 (2H, m), 7.51 (1H, t, J = 7.2 Hz), 7.42 (1 H, t, J = 7.2 Hz), 7.31 (1 H, s), 4.85 (2H, d, J = 6.0 Hz). |
| 1-53 | 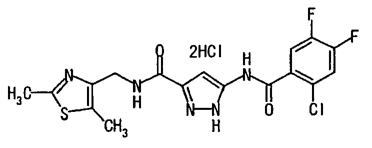 | ¹H-NMR(400 MHz, DMSO-*d*₆) δ: 11.15 (1H, s), 9.02 (1H, br s), 7.87-7.80 (2H, m), 7.27 (1H, br s), 4.40 (2H, m), 2.59 (3H, br s), 2.43 (3H, br s). |
| 1-54 | 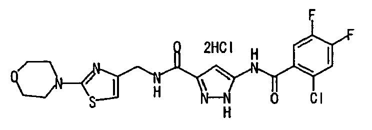 | ¹H-NMR(400 MHz, DMSO-*d*₆) δ: 11.21 (1H, br s), 9.09 (1H, br s), 7.88-7.81 (2H, m), 7.27 (1H, m), 6.71 (1H, br s), 4.38 (2H, m), 3.74 (4H, m), 3.53 (4H, m). |

**Table 9**

| **Example** | **Molecular Structure** | **NMR** |
|---|---|---|
| 1-55 | 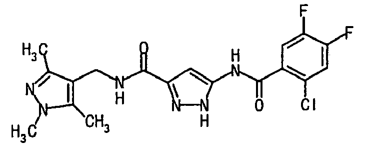 | ¹H-NMR(400 MHz, DMSO-*d*₆) δ: 13.18 (1H, s), 11.12 (1H, s), 8.72 (1H, m), 7.86-7.78 (2H, m). 7.30 (1H, s), 4.16 (2H, d, J = 5.6 Hz), 3.61 (3H, s), 2.22 (3H, s), 2.11 (3H.s). |
| 1-56 | 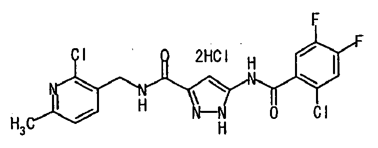 | ¹H-NMR(300 MHz. DMSO-*d*₆) δ: 11.22 (1H, s), 9.16 (1H, t, J = 5.8 Hz), 7.88-7.80 (2H, m), 7.67 (1 H, d, J = 7.9 Hz), 7.31-7.26 (2H, m), 4.45 (2H, d, J = 5.6 Hz), 2.44 (3H, s). |
| 1-57 | 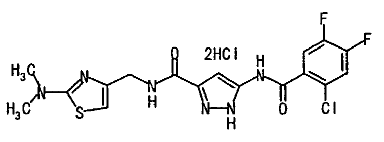 | ¹H-NMR(400 MHz, DMSO-*d*₆) δ: 11.22 (1H, s), 9.12 (1H, m), 7.88-7.81 (2H, m), 7.26 (1H. s), 6.69 (1H, br s), 4.40 (2H, m), 3.20 (6H, s). |
| 1-58 | 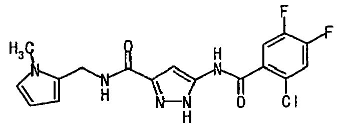 | ¹H-NMR(400 MHz, DMSO-*d*₆) δ: 13.21 (1H, br s), 11.13 (1H, br s), 8.90 (1H, br s), 7.86-7.79 (2H, m), 7.35 (1 H, br s), 6.67 (1H, m), 5.97 (1H, m), 5.90 (1 H, m), 4.40 (2H, d, J =5.2 Hz), 3.58 (3H, s). |
| 1-59 | 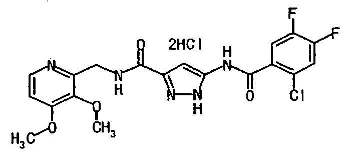 | ¹H-NMR(400 MHz, DMSO-*d*₆) δ: 11.25 (1H, s), 9.28 (1H, br s), 8.59 (1 H, d, J = 6.8 Hz), 7.88-7.81 (2H, m), 7.65 (1H. d, J = 6.8 Hz), 7.24 (1H, br s), 4.73 (2H, d, J = 4.4 Hz), 4.15 (3H, s), 3.91 (3H, s). |
| 1-60 | 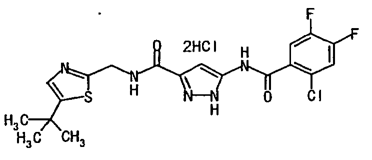 | ¹H-NMR(400 MHz, DMSO-*d*₆) δ: 11.25 (1H, s), 9.47 (1 H, br s), 7.88-7.81 (2H, m), 7.59 (1H, m), 7.27 (1H, s), 4.66 (2H, m), 1.32 (9H, s). |
| 1-61 | 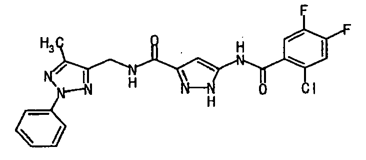 | ¹H-NMR(400 MHz, DMSO-*d*₆) δ: 13.29 (1H, s), 11.16 (1H, s), 9.18 (1H, br s), 7.93 (2H, d, J =7.2 Hz), 7.86-7.80 (2H, m), 7.56-7.51 (2H, m), 7.39-7.35 (2H, m), 4.57 (2H, d, J = 5.6 Hz), 2.37 (3H, s). |

**Table 10**

| **Example** | **Molecular Structure** | **NMR** |
|---|---|---|
| 1-62 | 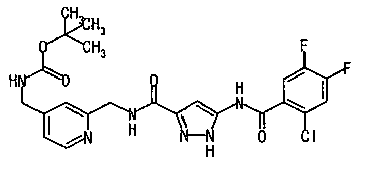 | ¹H-NMR(300 MHz, DMSO-*d*₆) δ: 9.05 (1H, br s), 8.43 (1 H, d, J = 4.8 Hz), 7.86-7.70 (2H, m), 7.47 (1H, t, J = 6.1 Hz), 7.23-7.05 (3H, m), 4.51 (2H, d, J = 5.9 Hz), 4.12 (2H, d, J = 6.2 Hz), 1.34 (9H, s). |
| 1-63 | 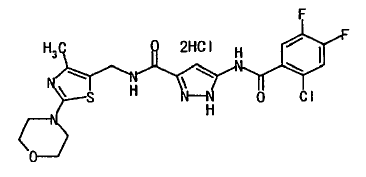 | ¹H-NMR(400 MHz, DMSO-d₆) δ: 11.22 (1H, s), 9.23 (1H, br s), 7.87-7.81 (2H, m), 7.18 (1H, br s), 4.38 (2H, d, J = 6.0 Hz), 3.72 (4H, m), 3.53 (4H, m), 2.30 (3H, s). |
| 1-64 | 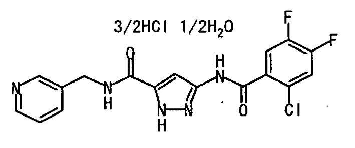 | ¹H-NMR (400 MHz, DMSO-*d*₆) δ: 11.26 (1H, br s), 9.37 (1H.t,J=5.8 Hz), 8.92 (1H, br s), 8.86 (1H, d, J = 5.6 Hz), 8.54 (1H, d, J = 8.3 Hz), 8.07 (1 H, dd, J = 8.1, 5.8 Hz), 7.90-7.81 (2H, m), 7.26 (1H, br s), 4.66 (2H, d, J = 5.6 Hz). |
| 1-65 | 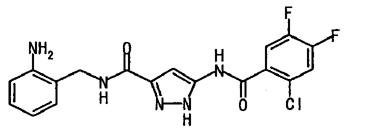 | ¹H-NMR (300 MHz, DMSO-*d*₆) 6:13.22 (1H, s), 11.12 (1H, s), 9.00 (1H, br t, J = 4.9 Hz), 7.85-7.75 (2H, m), 7.32 (1H.s), 7.01 (1H,d.J=7.2 Hz), 6.95 (1H. t, J = 7.5 Hz), 6.61 (1H, d, J = 7.9 Hz), 6.50 (1 H, t, J = 7.3 Hz), 5.10 (2H, s), 4.26 (2H, d, J = 6.0 Hz). |
| 1-66 | 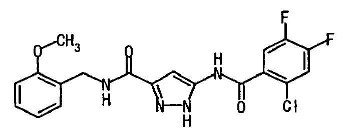 | ¹H-NMR (300 MHz, DMSO-*d*₆) δ:13.21 (1H. s), 11.13 (1H, s), 8.95 (1H, t, J = 5.8 Hz), 7.88-7.76 (2H, m), 7.39 (1H, d, J = 1.5 Hz), 7.29-7.15 (2H, m), 7.00(1H,d,J = 7.9 Hz), 6.92(1H,t,J = 7.2 Hz), 4.42 (2H, d, J = 5.7 Hz), 3.84 (3H, d, J = 6.4 Hz). |
| 1-67 | 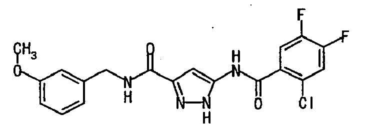 | ¹H-NMR (300 MHz, DMSO-*d*₆) δ: 13.22 (1H, br s), 11.14 (1H, br s), 9.11 (1H, br s), 7.87-7.77 (2H, m), 7.35 (1H, br s), 7.25 (1H, dd, J = 10.9, 5.3 Hz), 6.91-6.87 (2H, m), 6.85-6.79 (1H, m), 4.42 (2H, d, J = 6.0 Hz), 3.74 (3H, s). |
| 1-68 | 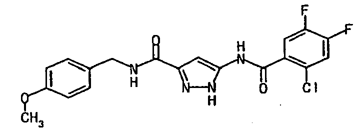 | ¹H-NMR (300 MHz, DMSO-*d*₆) δ: 13.20 (1H, s), 11.12 (1H, s), 9.06 (1H, t, J=6.0 Hz), 7.86-7.77 (2H, m), 7.33 (1H, d,J = 1.9 Hz), 7.25 (2H, d, J = 9.0 Hz), 6.90 (2H, d, J = 9.4 Hz), 4.37 (2H, d,J = 9.0 Hz), 3.73 (3H, s). |
| 1-69 | 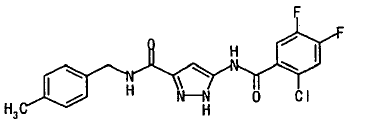 | ¹H-NMR (300 MHz, DMSO-*d*₆) δ: 12.43 (1H, br s), 11.20 (1 H, br s), 9.04 (1 H, t, J = 5.8 Hz), 7.87-7.78 (2H, m). 7.26 (1H, s), 7.20 (2H, d, J = 7.9 Hz), 7.14 (2H, d, J = 7.5 Hz), 4.39 (2H, d, J = 6.0 Hz), 2.27 (3H, s). |

**Table 11**

| **Example** | **Molecular Structure** | **NMR** |
|---|---|---|
| 1-70 | 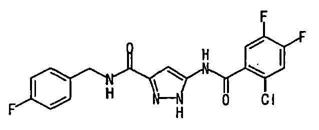 | ¹H-NMR (300 MHz, DMSO-*d*₆) δ: 13.23 (1H, s), 11.14 (1H, s), 9.13 (1H, s), 7.86-7.78 (2H, m), 7.39-7.33 (3H, m), 7.17-7.14 (2H, m), 4.42 (2H, d, J = 6.0 Hz). |
| 1-71 | 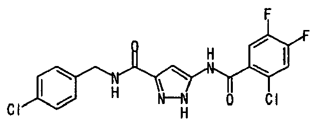 | ¹H-NMR (300 MHz, DMSO-*d*₆) δ: 13.23 (1H, br s), 11.15 (1 H, br s), 9.14 (1 H, br s), 7.86-7.79 (2H, m), 7.40 (2H, d, J = 7.2 Hz), 7.34 (2H, d, J = 8.7 Hz), 4.43 (3H, d, J = 6.0 Hz). |
| 1-72 | 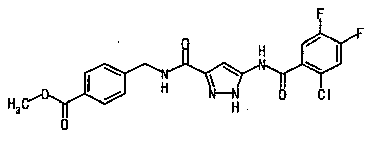 | ¹H-NMR (300 MHz, DMSO-*d*₆) δ:13.26 (1H, s), 11.15 (1H, s), 9.22 (1H, t, J = 5.8 Hz), 7.94 (2H, d, J = 8.3 Hz), 7.86-7.78 (2H, m), 7.46 (2H, d, J = 8.3 Hz), 7.37 (1H, s), 4.53 (2H, d, J = 5.7 Hz), 3.85 (3H, s). |
| 1-73 | 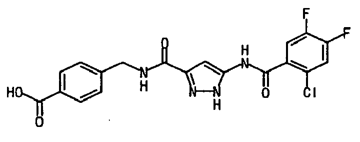 | ¹H-NMR (300 MHz, DMSO-*d*₆) δ: 13.05 (2H, br s), 11.16 (1H, s), 9.16 (1H, t, J = 5.7 Hz), 7.90 (2H, d, J = 8.3 Hz), 7.84-7.77 (2H, m), 7.41 (2H, d, J = 8.3 Hz), 7.27 (1H, s), 4.50 (2H, d, J = 6.0 Hz). |
| 1-74 | 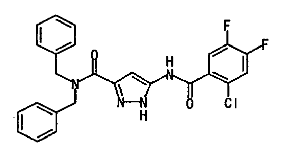 | ¹H-NMR (300 MHz, DMSO-d₆) δ: 13.38 (0.8H, br s), 13.11 (0.2H, br s), 11.43 (0.2H, br s), 11.11 (0.8H, s), 7.92-7.66 (2H, m), 7.45-7.17 (10H, m), 6.80 (0.8H, br s), 6.51 (0.2H, br s), 5.05-4.42 (4H, m). |
| 1-75 | 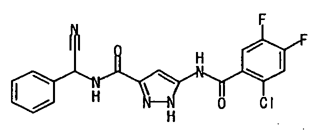 | ¹H-NMR (300 MHz, DMSO-*d*₆) δ: 13.44 (1H, s), 11.20 (1H, s), 9.91 (1H, d, J = 7.9 Hz), 7.93-7.76 (2H, m), 7.57-7.38 (6H, m), 6.40 (1 H, d, J = 7.9 Hz). |
| 1-76 | 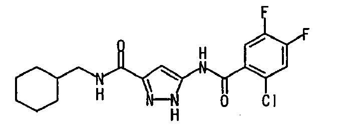 | ¹H-NMR (300 MHz, DMSO-*d*₆)δ: 13.13 (1H, s), 11.10 (1H. s), 8.51 (1H. t, J = 5.8 Hz), 7:86-7.77 (2H, m), 7.31 (1 H, d, J = 2.3 Hz), 3.08 (2H, t, J = 6.4 Hz), 1.78-1.43 (6H, m), 1.29-1.05 (3H, m), 1.02-0.81 (2H, m). |
| 1-77 | 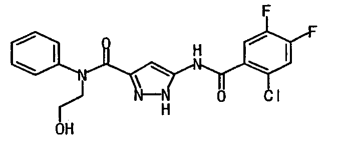 | ¹H-NMR (300 MHz, DMSO-*d*₆) δ: 13.70 (1H, s), 11.26 (1H, s), 7.87-7.79 (2H, m), 7.16 (1H, s), 7.09 (2H, t, J = 8.5 Hz), 6.65 (2H, d, J = 7.9 Hz), 6.55 (1 H, t, J = 7.2 Hz), 5.79 (1 H, t, J = 6.0 Hz), 4.39 (2H, t, J = 5.7 Hz), 3.42 (2H, dd, J = 5.7, 2.8 Hz). |

**Table 12**

| **Example** | **Molecular Structure** | **NMR** |
|---|---|---|
| 1-78 | 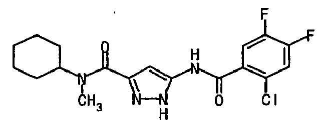 | ¹H-NMR (300 MHz, DMSO-*d*₆) δ: 13.10 (1H, s), 11.13 (1H, s), 7.84-7.76 (2H, m), 6.86 (1H, s), 4.13-4.10 (1H, m), 3.12-2.76 (3H, m), 1.87-1.00 (10H, m). |
| 1-79 | 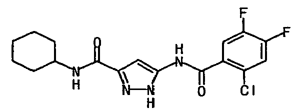 | ¹H-NMR (300 MHz, DMSO-*d*₆) δ: 13.12 (1H, s), 11.09 (1H, s), 8.34 (1H. d, J = 7.9 Hz), 7.86-7.77 (2H, m), 7.34 (1H, d, J = 2.3 Hz), 3.80-3.64 (1H, m), 1.88-1.53 (5H, m), 1.40-1.03 (5H, m). |
| 1-80 | 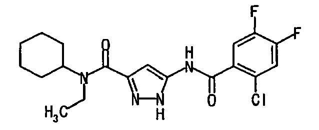 | ¹H-NMR (300 MHz, DMSO-*d*₆) δ: 13.10 (1H, s), 11.14 (1H, s), 7.88-7.78 (2H, m), 6.84 (1H, s), 4.02 (1H, s), 1.86-1.47 (8H, m), 1.38-1.02 (7H, m). |
| 1-81 | 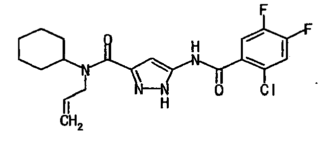 | ¹H-NMR (300 MHz, DMSO-*d*₆) δ: 13.13 (1H, s), 11.11 (1H, s), 7.93-7.76 (2H, m), 6.88 (1H, s), 5.98-5.79 (1 H, m), 5.26-5.00 (2H, m), 4.25-3.86 (3H, m), 1.80-1.58 (7H, m), 1.38-1.03 (3H, m). |
| 1-82 | 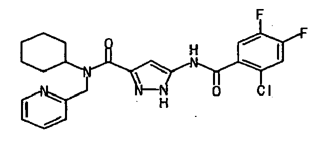 | ¹H-NMR (300 MHz, DMSO-*d*₆) δ: 13.23 (1H, br s), 11.19 (1H, br s), 8.51 (1H, br s), 7.95-7.64 (3H, m), 7.45-7.12 (2H, m), 6.96 (0.4H, br s), 6.66 (0.3H, br s), 6.40 (0.2H, br s), 5.18 (0.1 H, br s), 4.91-4.51 (2H, m), 4.39-4.07 (1H, m), 1.90-0.89 (10H, m). |
| 1-83 | 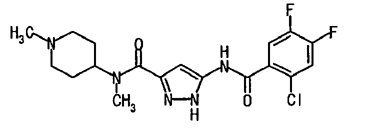 | ¹H-NMR (300 MHz, DMSO-*d*₆) δ: 13.12 (1H, br s), 11.19 (1 H, br s), 7.88-7.76 (2H, m), 6.81 (1H, br s), 4.35-3.83 (1 H, m), 3.16-2.72 (7H, m), 2.20 (3H, br s), 2.06-1.45 (4H, m). |
| 1-84 | 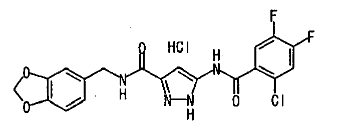 | ¹H-NMR (300 MHz, DMSO-*d*₆) δ: 11.15 (1H, s), 9.02 (1H, t, J = 5.7 Hz), 7.87-7.78 (2H, m), 7.26 (1H, s), 6.89-6.77 (4H, m), 5.98 (2H, s), 4.34 (2H, d, J = 6.0 Hz). |
| 1-85 | 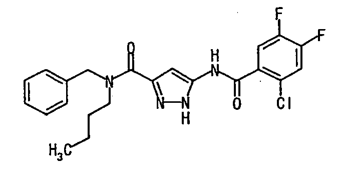 | ¹H-NMR (300 MHz, DMSO-*d*₆) δ: 13.26 (1H, s), 11.18 (1H, s), 7.97-7.68 (2H, m), 7.50-7.17 (5H, m), 6.97 (0.4H, br s), 6.76 (0.25H, br s), 6.43 (0.15H, br s), 5.10 (0.1H, s), 4.93-4.59 (2H, m), 3.72-3.17 (2H, m), 1.68-1.42 (2H, m), 1.39-1.13 (2H, m), 0.94-0.75 (3H, m). |

**Table 13**

| **Example** | **Molecular Structure** | **NMR** |
|---|---|---|
| 1-86 | 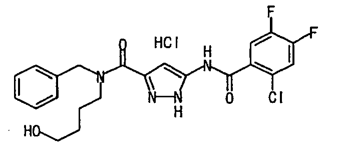 | ¹H-NMR (300 MHz, DMSO-*d*₆) δ: 11.33 (1H, s), 9.37 (2H, br s), 7.89-7.78 (2H, m), 7.65-7.51 (2H, m), 7.47-7.41 (3H, m), 7.07 (1H, s), 4.30 (2H, t, J = 5.3 Hz), 4.12 (2H, t, J = 5.5 Hz), 2.95 (2H, br s), 1.92-1.71 (4H, m). |
| 1-87 | 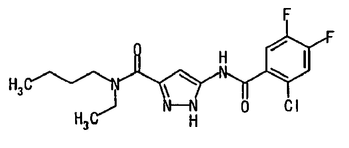 | ¹H-NMR (300 MHz, DMSO-*d*₆) δ: 13.12 (1H, s), 11.15 (1H, s), 7.97-7.73 (2H, m), 6.88 (1H, s), 3.76-3.15 (4H, m), 1.69-1.44 (2H, m), 1.41-1.05 (5H, m), 0.90 (3H, t, J = 8.0 Hz). |
| 1-88 | 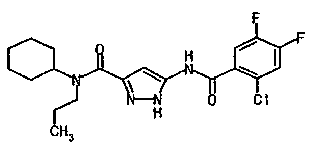 | ¹H-NMR (300 MHz, DMSO-*d*₆) δ: 13.09 (1H, s), 11.14 (1H, s), 7.93-7.74 (2H, m), 6.83 (1H, s), 4.39-3.85 (1H, m), 3.42-3.13 (2H, m), 1.86-1.45 (9H, m), 1.39-1.04 (3H, m), 0.87 (3H, t, J = 6.9 Hz). |
| 1-89 | 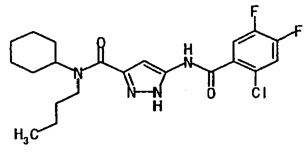 | ¹H-NMR (300 MHz, DMSO-*d*₆) δ: 13.08 (1H, s), 11.14 (1 H, s), 7.95-7.75 (2H, m), 6.84 (1H, s), 4.23-3.85 (1H, m), 3.48-3.19 (2H, m), 1.86-1.46 (9H, m), 1.41-1.04 (5H, m), 0.91 (3H, t, J = 7.2 Hz). |
| 1-90 | 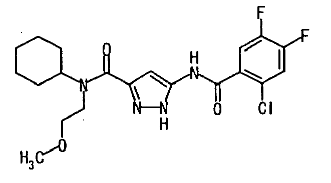 | ¹H-NMR (300 MHz, DMSO-d₆) δ: 13.05 (1H, br s), 11.39 (0H, s), 11.14 (1 H, s), 7.96-7.74 (2H, m), 6.88 (1 H, br s), 6.32 (0H, br s), 4.47-4.29 (0H, m), 4.10-3.91 (1H, m), 3.67-3.40 (4H, m), 3.32-3.24 (3H, m), 1.89-1.43 (7H, m), 1.41-0.99 (3H, m). |
| 1-91 | 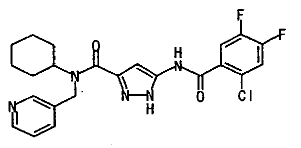 | ¹H-NMR (300 MHz, DMSO-d₆) δ:13.26(1H, s), 8.59 (1H, s), 8.52-8.47 (2H, m), 8.02-7.57 (3H, m), 7.46-7.24 (1H, m), 6.93 (0.5H. br s), 6.41 (0.2H, br s), 5.13 (0.1 H, br s), 4.99-4.45 (2H, m), 4.38-4.01 (1H, m), 1.88-1.39 (7H, m), 1.34-0.95 (3H, m). |
| 1-92 | 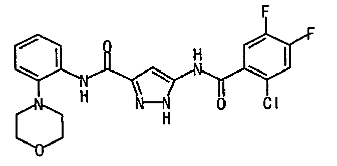 | ¹H-NMR (300 MHz, DMSO-d₆) δ: 13.49 (1H, br s), 11.55 (br s), 11.26 (br s), 9.98 (br s), 9.60 (br s), 8.31 (br s),7.90 (2H, s), 7.48-7.04 (4H, m), 6.56 (br s), 3.94-3.70 (4H, m), 2.92-2.82 (4H, m). |

**Table 14**

| **Example** | **Molecular Structure** | **NMR** |
|---|---|---|
| 1-93 | 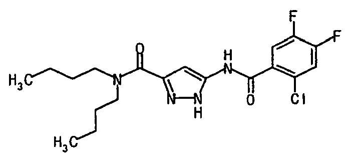 | ¹H-NMR (300 MHz, DMSO-d₆) δ: 13.11 (0.8H, br s), 12.97 (0.2H, br s), 11.37 (0.2H, s), 11.15 (0.8H, s), 7.95-7.76 (2H, m), 6.91-6.85 (0.8H, m), 6.34-6.31 (0.2H, m), 3.75-3.33 (4H, m), 1.68-1.46 (4H, m), 1.40-1.17 (4H, m), 0.93 (6H, t, J = 6.8 Hz). |
| 1-94 | 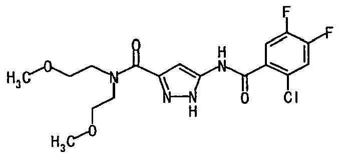 | ¹H-NMR (300 MHz, DMSO-*d*₆) δ: 13.11 (1H, s), 11.39 (0.1H, s), 11.13 (0.9H, s), 7.96-7.68 (2H, m), 6.94 (0.9H, br s), 6.38 (0.1H, br s), 3.84-3.58 (4H, m), 3.59-3.48 (4H, m), 3.27 (6H, s). |
| 1-95 | 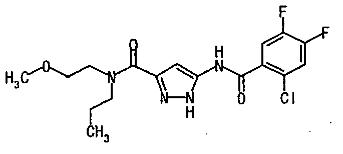 | ¹H-NMR (300 MHz, DMSO-*d*₆) δ: 13.09 (1H, br s), 11.38 (0.2H, br s), 11.14 (0.8H, br s), 7.98-7.73 (2H, m), 6.90 (0.8H, br s), 6.36 (0.2H, br s), 3.98-3.35 (6H, m), 3.29 (3H, s), 1.72-1.51 (2H, m), 0.88 (3H, t, J = 7.3 Hz). |
| 1-96 | 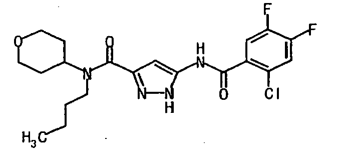 | ¹H-NMR (300 MHz, DMSO-*d*₆) δ: 13.12 (0.8H, br s), 12.98 (0.2H, br s), 11.38 (0.2H, br s), 11.15 5 (0.8H, br s), 7.97-7.74 (2H, m), 6.86 (0.8H, br s), 6.33 (0.2H, br s), 4.64 (0H, br s), 4.24 (1 H, br s), 4.02-3.84 (2H, m), 3.66-3.12 (4H, m), 2.05-1.79 (2H, m), 1.72-1.47 (4H, m), 1.42-1.18 (2H, m), 0.91 (3H, t, J = 7.3 Hz). |
| 1-97 | 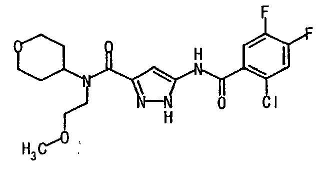 | ¹H-NMR (300 MHz, DMSO-*d*₆) δ: 13.12 (1H, br s), 11.40 (0.2H, s), 11.14 (0.8H, br s), 7.99-7.72 (2H, m), 6.89 (0.8H, br s), 6.36 (0.2H, br s), 4.71 (0.2H, m), 4.20 (0.8H, m), 4.01-3.84 (2H, m), 3.71-3.10 (9H, m), 2.09-1.72 (2H, m), 1.71-1.54 (2H, m). |
| 1-98 | 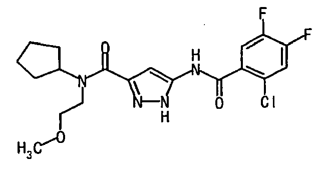 | ¹H-NMR (300 MHz, DMSO-*d*₆) δ: 13.11 (1H, br s), 11.15 (1 H, br s), 7.86-7.82 (2H, m), 6.87 (0.8H, br s), 6.33 (0.2H, br s), 4.43-4.39 (1H, m), 3.52-3.49 (4H, m), 3.27 (3H, s), 1.75-1.57 (8H, m). |
| 1-99 | 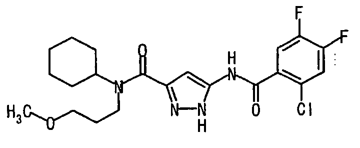 | ¹H-NMR (300 MHz, DMSO-*d*₆) δ: 13.10 (1H, s), 11.14 (1H, s), 7.95-7.72 (2H, m), 6.85 (1 H, br s), 4.43-3.84 (1H, m), 3.64-3.28 (4H, m), 3.22 (3H, s), 1.90-1.45 (9H, m), 1.40-1.00 (3H, m). |

**Table 15**

| **Example** | **Molecular Structure** | **NMR** |
|---|---|---|
| 1-100 | 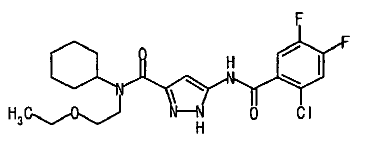 | ¹H-NMR (300 MHz, DMSO-*d*₆) δ:13.09 (1H, br s), 11.12 (1H, s), 7.93-7.73 (2H, m), 6.85 (1H, br s),4.09-3.86 (1 H, m), 3.67-3.29 (6H, m), 1.83-1.48 (7H, m), 1.38-0.97 (6H, m). |
| 1-101 | 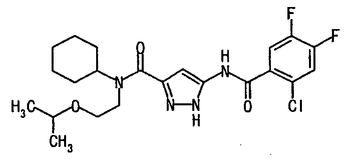 | ¹H-NMR (300 MHz, DMSO-*d*₆) δ: 13.11 (1H, br s), 11.14 (1H, s), 7.94-7.75 (2H, m), 6.86 (1H, br s), 4.01-3.98 (1H, m), 3.74-3.34 (5H, m), 1.87-1.45 (7H.m). 1.36-1.01 (9H.m). |
| 1-102 | 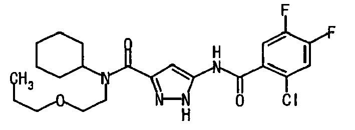 | ¹H-NMR (300 MHz, DMSO-*d*₆) δ: 13.09 (1H, br s), 11.12 (1H, s), 7.87-7.80 (2H, m), 6.85 (1H. br s), 4.09-3.88 (1 H, m), 3.65-3.41 (4H, m), 3.35 (2H, t, J = 6.6 Hz), 1.84-1.43 (9H, m), 1.36-1.02 (3H, m), 0.85 (3H, t, J = 7.3 Hz). |
| 1-103 | 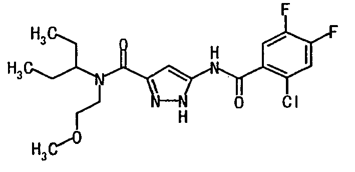 | ¹H-NMR (300 MHz, DMSO-*d*₆) δ: 13.10 (1H, s), 11.16 (1H, s), 7.99-7.68 (2H, m), 6.81 (1H, s), 3.97-3.83 (1H, m), 3.58-3.15 (7H, m), 1.77-1.37 (4H, m), 0.98-0.67 (6H, m). |
| 1-104 | 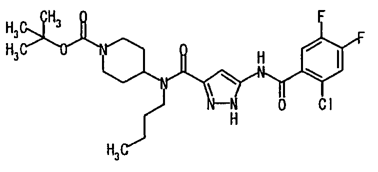 | ¹H-NMR (300 MHz, DMSO-*d*₆) δ: 13.11 (0.8H, s), 12.98 (0.2H, s), 11.37 (0.2H, s), 11.15 (0.8H, s), 7.94-7.75 (2H, m), 6.87 (0.8H, s), 6.32 (0.2H, s), 4.66-4.50 (0.2H, m), 4.33-3.87 (2.8H, m), 3.63-3.11 (3H, m), 2.98-2.55 (2H, m), 1.91-1.12 (16H, m), 0.89 (3H, t, J = 7.3 Hz). |
| 1-105 | 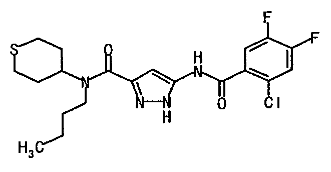 | ¹H-NMR (300 MHz, DMSO-*d*₆) δ: 13.11 (1H, s), 11.15 (1H, s), 7.94-7.75 (2H, m), 6.84 (2H, br s), 4.08-3.87 (1H, m), 3.49-3.24 (2H, m), 2.87-2.57 (3H, m), 2.17-1.88 (4H, m), 1.66-1.45 (2H, m), 1.38-1.23 (2H, m), 0.89 (3H, t, J = 6.7 Hz). |
| 1-106 | 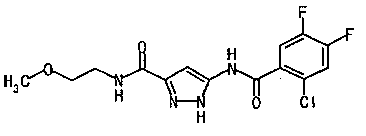 | ¹H-NMR (300 MHz, DMSO*-d*₆) δ: 13.15 (1H, s), 11.09 (1H, s), 8.60 (1H, t, J = 5.3 Hz), 7.98-7.73 (2H, m), 7.29 (1H, s), 3.47-3.33 (4H, m), 3.26 (3H, s). |

**Table 16**

| **Example** | **Molecular Structure** | **NMR** |
|---|---|---|
| 1-107 | 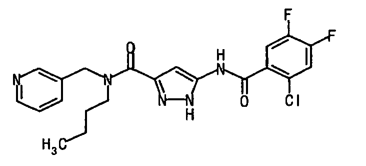 | ¹H-NMR (300 MHz, DMSO-*d*₆) δ: 13.27 (0.8H, br s), 13.09 (0.2H, br s), 11.42 (0.2H, br s), 11.18 (0.8H, br s), 8.62-8.39 (2H, m), 7.97-7.60 (3H, m), 7.46-7.28 (1H, m), 7.07-6.64 (0.8H, m), 6.45 (0.2H, br s), 5.14 (0.2H, br s), 4.74-4.71 (1.8H, m), 3.82-3.16 (2H, m), 1.60-1.18 (4H, m), 0.93-0.74 (3H, m). |
| 1-108 | 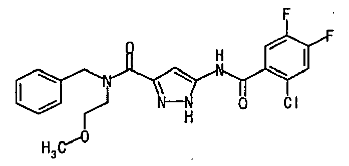 | ¹H-NMR (300 MHz, DMSO-*d*₆) δ: 13.20 (1H, br s), 11.50-11.00(1H, br s), 7.97-7.69 (2H, m), 7.51-6.27 (6H, m), 5.28-4.58 (2H, m), 4.00-3.36 (4H, m), 3.23 (3H, s). |
| 1-109 | 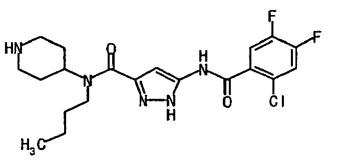 | ¹H-NMR (300 MHz, DMSO-*d*₆) δ: 12.79 (1H, br s), 11.09 (1H, br s), 7.88-7.78 (2H, m), 6.76 (1 H, br s), 4.28-3.95 (1H, m), 3.53-3.10 (4H, m), 3.00 (1 H, d, J = 12.4 Hz), 2.59-2.32 (2H, m), 1.78-1.49 (6H, m), 1.38-1.22 (2H, m), 0.90 (3H, t, J = 7.3 Hz). |
| 1-110 | 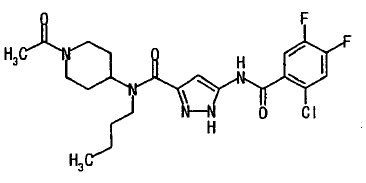 | ¹H-NMR (300MHz, DMSO-*d*₆) δ: 13.12 (1 H, s), 11.16 (1H, s), 7.99-7.73 (2H, m), 6.88 (1H, s), 4.55 (1H, br d, J = 12.8 Hz), 4.23 (1H, br s), 3.95 (1 H, br d, J = 12.8 Hz), 3.65-2.85 (3H, m), 2.63-2.27 (1H, m), 2.08-1.41 (9H, m), 1.40-1.21 (2H, m), 0.90 (3H, t, J = 7.2 Hz). |
| 1-111 | 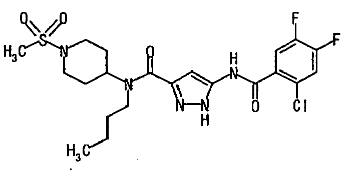 | ¹H-NMR (300 MHz, Dmso-*d*₆) δ: 13.12 (1H, br s), 11.18 (1H, s), 7.89-7.76 (2H, m), 6.84 (1H, br s), 4.31-3.99 (1 H, m), 3.67 (2H, d, J = 11.7 Hz), 3.56-3.18 (2H, m), 2.98-2.59 (5H, m), 2.10-1.73 (4H, m), 1.65-1.46 (2H, m), 1.40-1.22 (2H, m), 0.90 (3H, t, J = 7.2 Hz). |
| 1-112 | 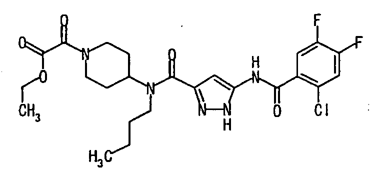 | ¹H-NMR (300 MHz, DMSO-*d*₆) δ: 13.12 (1H, s), 11.15 (1H, s), 7.93-7.74 (2H, m), 6.88 (1H, br s), 4.41-4.18 (4H, m), 3.62 (1H, d, J = 13.6 Hz), 3.52-3.01 (3H, m), 2.91-2.64 (1H, m), 2.01-1.67 (4H, m), 1.64-1.44 (2H, m), 1.37-1.14 (5H, m), 0.93-0.78 (3H, m). |

**Table 17**

| **Example** | **Molecular Structure** | **NMR** |
|---|---|---|
| 1-113 | 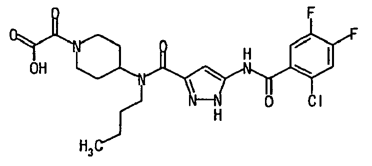 | ¹H-NMR (300 MHz, DMSO-*d*₆) δ: 13.53 (1H, br s), 11.21 (1H, s), 7.88-7.78 (2H, m), 6.75 (1 H, br s), 4.39-4.20 (2H, m), 3.63 (1H, br d, J = 12.4 Hz), 3.57-3.01 (4H, m), 2.84-2.61 (1H, m), 1.96-1.46 (6H, m), 1.38-1.21 (2H, m), 0.89 (3H, t, J = 7.3 Hz). |
| 1-114 | 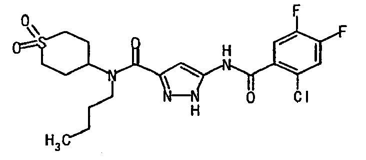 | ¹H-NMR (300 MHz, DMSO-*d*₆) δ: 13.13 (1H, s), 11.16(1H,s), 7.96-7.72 (2H, m), 6.89 (1H, br s), 4.34-4.11 (1H, m), 3.69-3.24 (4H, m), 3.19-3.02 (2H, m), 2.65-2.36 (2H, m), 2.15-1.93 (2H, m), 1.68-1.50 (2H, m), 1.39-1.21 (2H, m), 0.87 (3H, t, J = 7.2 Hz). |
| 1-115 | 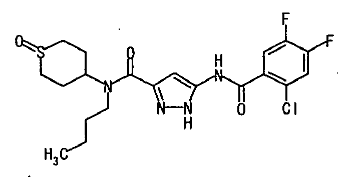 | ¹H-NMR (300 MHz, DMSO-*d*₆) δ: 13.12 (1H, s), 11.16 (1H.s). 7.98-7.67 (2H,m). 6.88 (1H, br s), 4.60-3.98 (1H, m), 3.70-3.18 (4H, m). 3.06-2.12 (4H, m), 2.06-1.44 (4H. m), 1.42-1.15 (2H, m), 1.00-0.78 (3H, m). |
| 1-116 | 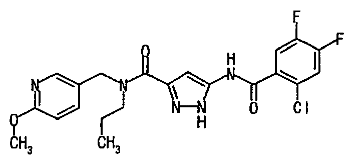 | ¹H-NMR (300 MHz, DMSO-*d*₆) δ: 13.21 (1H, br s), 11.27 (1H,brs),8.24-7.53 (4H, m), 7.00-6.30 (2H, m), 5.09-4.45 (2H, m), 3.84 (3H, s), 3.70-3.10 (2H, m), 1.74-1.43 (2H, m), 0.92-0.77 (3H, m). |
| 1-117 | 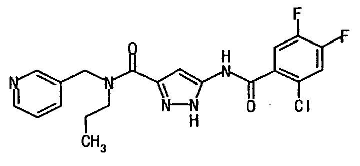 | ¹H-NMR (300 MHz, DMSO-*d*₆) δ: 13.34 (1H, br s), 11.31 (1 H, br s), 8.65-8.43 (2H, m), 7.93-7.63 (3H, m), 7.47-7.34 (1H, m), 7.09-6.37 (1H, m), 5.26-4.55 (2H, m), 3.78-3.14 (2H, m), 1.77-1.41 (2H, m), 0.93-0.73 (3H, m). |
| 1-118 | 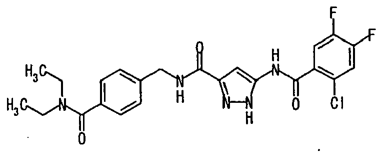 | ¹H-NMR(300 MHz, DMSO-d6) δ:13.25 (1H, br s), 11.14 (1H, br s), 9.17 (1H, br s), 7.90-7.75 (2H, m), 7.45-7.25 (5H, m), 4.48 (2H, d, J = 5.5 Hz), 3.55-3.05 (4H, br d), 1.09 (6H, br s). |
| 1-119 | 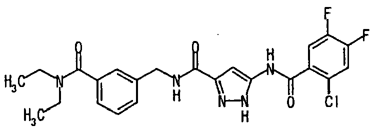 | ¹H-NMR (300 MHz, DMSO-d₆) δ: 13.24 (1H, br s), 11.14 (1H, br s), 9.17 (1H, br s), 7.90-7.75 (2H, m), 7.45-7.15 (5H, m), 4.47 (2H, d, J = 5.9 Hz), 3.50-3.00 (4H, br d), 1.05 (6H, br s). |

**Table 18**

| **Example** | **Molecular Structure** | **NMR** |
|---|---|---|
| 1-120 | 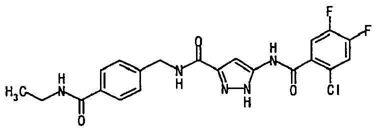 | ¹H-NMR (300 MHz, DMSO-d₆) δ: 13.24 (1H, br s), 11.14 (1 H, br s), 9.17 (1H, br s), 8.39 (1H, br t, J = 5.1 Hz), 7.95-7.60 (4H, m), 7.38 (3H, d, J = 8.1 Hz), 4.49 (2H, d, J = 5.9 Hz), 3.27 (2H, q, J = 7.2 Hz), 1.11 (3H, t, J = 7.2 Hz). |
| 1-121 | 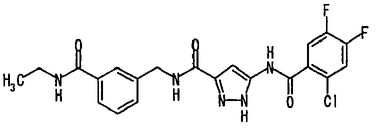 | ¹H-NMR (300 MHz, DMSO-d₆) δ: 13.24 (1H. br s), 11.14 (1H, br s), 9.18 (1H, br s), 8.44 (1H, br s), 7.86-7.70 (4H, m), 7.45-7.38 (3H, m), 4.49 (2H, d, J = 5.5 Hz), 3.28 (2H, dt, J = 14.4, 6.2 Hz), 1.11 (3H, t, J = 7.2 Hz). |
| 1-122 | 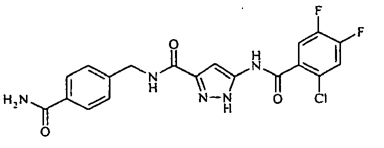 | ¹H-NMR (300 MHz, DMSO-d₆) δ: 9.14 (1 H, br s), 7.95-7.75 (5H, m), 7.42-7.25 (4H, m), 4.49 (2H, d, J = 5.9 Hz). |
| 1-123 | 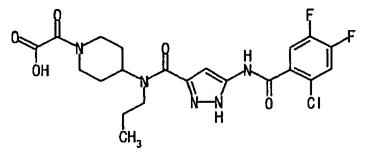 | ¹H-NMR (300 MHz, DMSO-*d*₆) δ: 11.24 (1H, s), 7.90-7.78 (2H, m), 6.78 (1 H, br s), 4.44-4.25 (2H, m), 3.76-2.56 (6H, m), 2.02-1.45 (6H, m), 0.87 (3H, t, J = 7.2 Hz). |
| 1-124 | 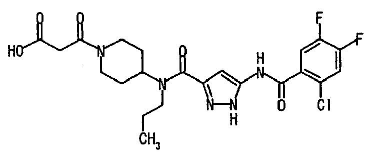 | ¹H-NMR (300 MHz, DMSO-*d*₆) δ: 12.87 (1H, br s), 11.22 (1H, s), 7.90-7.77 (2H, m), 6.79 (1H, br s), 4.51 (1H, br d, J = 12.8 Hz), 4.32 (1 H, s), 3.89 (1H, br d, J = 12.8 Hz), 3.65-2.91 (6H, m), 2.68-2.41 (1H, m), 2.00-1.46 (6H, m), 0.87 (3H, t, J = 7.3 Hz). |
| 1-125 | 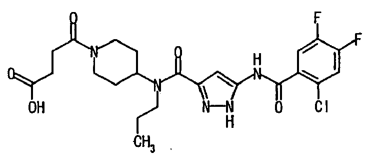 | ¹H-NMR (300 MHz,DMSO-*d*₆) δ: 13.39-11.87 (1H, br s), 11.22 (1H, br s), 7.89-7.79 (2H, m), 6.83 (1H, br s), 4.51 (1H, d, J = 13.2 Hz), 4.40-4.18 (1H, m), 3.99 (1H, d, J = 13.2 Hz), 3.54-2.84 (4H,m), 2.70-2.31 (4H,m), 1.96-1.46 (6H,m), 0.86 (3H, t, J = 7.2 Hz). |
| 1-126 | 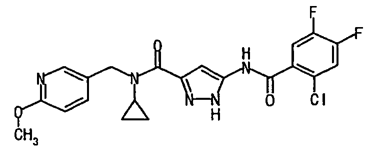 | ¹H-NMR (300 MHz, DMSO-*d*₆) δ: 13.20 (1H, s), 11.14 (1H, s), 8.13 (1H, d, J = 1.9 Hz), 7.87-7.77 (2H, m), 7.63 (1 H, dd, J = 8.5, 2.4 Hz), 7.16 (1 H, s), 6.82 (1H, d, J = 8.3 Hz), 4.65 (2H, s), 3.83 (3H, s), 3.02-2.90 (1H, m), 0.87-0.67 (4H, m). |

**Table 19**

| **Example** | **Molecular Structure** | **NMR** |
|---|---|---|
| 1-127 | 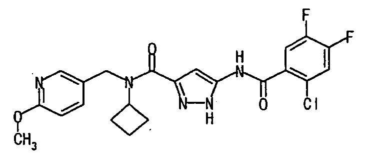 | ¹H-NMR (300 MHz. DMSO-d₆) δ: 13.24 (1H, br s), 11.21 (1H, br s), 8.07 (1H, s), 7.89-7.78 (2H, m), 7.58 (1 H, d, J = 6.8 Hz), 6.94-6.71 (2H, m), 4.94-4.57 (3H, m), 3.83 (3H, s), 2.32-1.97 (4H, m), 1.70-1.44 (2H, m). |
| 1-128 | 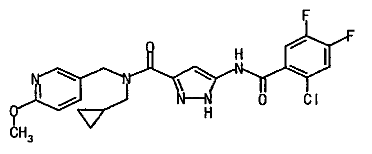 | ¹H-NMR (300 MHz, DMSO-*d*₆) δ: 13.24 (1H, br s), 11.23 (1H, br s), 8.14 (1 H, br s), 7.89-7.79 (2H, m), 7.72-7.59 (1H, m), 7.05-6.59 (2H, m), 5.06-4.52 (2H, m), 3.84 (3H, s), 3.58-3.13 (2H, m), 1.09-0.94 (1H, m), 0.56-0.34 (2H, m), 0.21-0.19 (2H, m). |
| 1-129 | 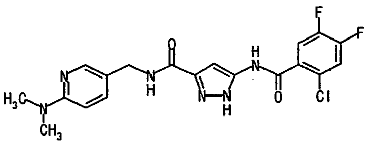 | ¹H-NMR (300 MHz, DMSO-*d*₆) δ: 13.21 (1H, s), 11.13 (1H, s), 9.01 (1H, s), 8.05 (1H, d, J = 2.3 Hz), 7.88-7.77 (2H, m), 7.48 (1H,dd, J = 8.7, 2.6 Hz), 7.31 (1H, s), 6.62 (1H, d, J = 9.0 Hz), 4.29 (2H, d, J = 5.7 Hz), 3.00 (6H, s). |
| 1-130 | 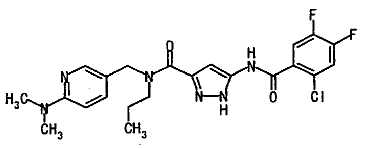 | ¹H-NMR (300 MHz, DMSO-*d*₆) δ: 13.24 (1H, br s), 11.18 (1H, br s), 8.16-7.73 (3H, m), 7.57-7.27 (1 H, m), 6.89 (0.8H, br s), 6.63 (1 H, d, J = 8.3 Hz), 6.41 (0.2H, br s), 4.99-4.30 (2H, m), 3.66-3.15 (2H, m), 3.00 (6H, s), 1.73-1.44 (2H, m), 0.83 (3H, t, J = 6.8 Hz). |
| 1-131 | 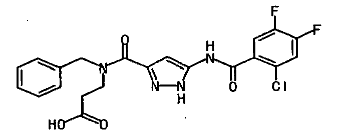 | ¹H-NMR (300 MHz, DMSO-*d*₆) δ: 13.77-11.97 (1 H, m), 11.22 (1 H, br s), 7.83 (2H, br s), 7.41-7.24 (5H, m), 6.92-6.58 (1H, m), 5.04-4.60 (2H, m), 3.85-3.63 (1H, m), 3.62-3.42 (1H, m), 2.70-2.52 (2H, m). |
| 1-132 | 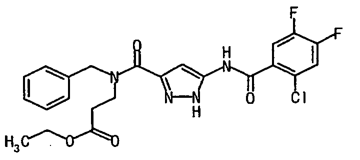 | ¹H-NMR (300 MHz, DMSO-*d*₆) δ: 13.29 (1H, br s), 11.15 (1H, br s), 7.98-7.67 (2H, m), 7.46-7.19 (5H, m), 7.13-6.35 (1H, m), 5.24-4.62 (2H, m), 4.12-3.38 (4H, m), 2.76-2.55 (2H, m), 1.16 (3H, t, J = 6.8 Hz). |
| 1-133 | 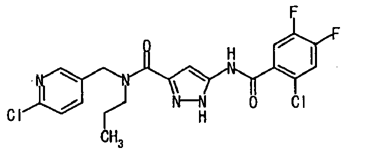 | ¹H-NMR (300 MHz, DMSO-*d*₆) δ: 13.25 (1H, br s), 11.26 (1 H, br s), 8.45-8.32 (1H, m), 7.90-7.78 (3H, m), 7.57-7.43 (1H, m), 7.01-6.51 (1H, m), 5.08-4.56 (2H, m), 3.64-3.19 (2H, m), 1.76-1.50 (2H, m), 0.85 (3H, t, J = 7.3 Hz). |

**Table 20**

| **Example** | **Molecular Structure** | **NMR** |
|---|---|---|
| 1-134 | 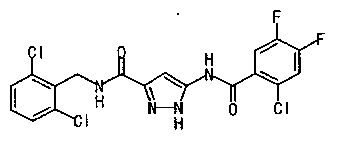 | ¹H-NMR (300 MHz, DMSO-*d*₆) δ: 13.23 (1H. br s), 11.13 (1H, br s), 8.80 (1H, br t), 7.87-7.76 (2H, m), 7.55-7.49 (2H, m), 7.45-7.32 (2H, m), 4.70 (2H, d, J = 4.5 Hz). |
| 1-135 | 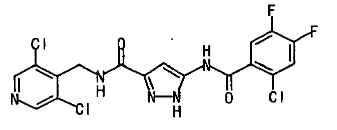 | ¹H-NMR (300 MHz, DMSO-*d*₆) δ: 13.28 (1H, s), 11.15 (1H, s), 8.94 (1H, t, J = 4.5 Hz), 8.66 (2H, s), 7.90-7.74 (2H, m), 7.33 (1H, s), 4.67 (2H, d, J = 4.5 Hz). |
| 1-136 | 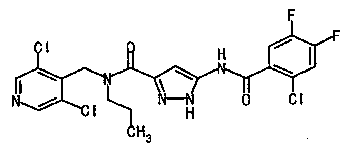 | ¹H-NMR (300 MHz, DMSO-*d*₆) δ: 13.37-13.01 (1H, m), 11.53-11.03 (1H, m), 8.65 (2H, s), 7.98-7.70 (2.5H, m), 6.93 (0.5H, s), 5.61-4.68 (2H, m), 3.80-3.00 (2H, m), 1.71-1.36 (2H, m), 0.85 (3H, t, J = 6.4 Hz). |
| 1-137 | 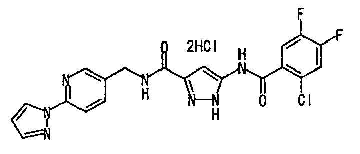 | ¹H-NMR (300 MHz, DMSO-*d*₆) δ: 11.20 (1H, s), 9.24 (1 H, t, J = 5.8 Hz), 8.61 (1H, d, J = 2.6 Hz), 8.60 (br s), 8.44 (1H, s), 7.97-7.79 (5H, m), 7.26 (1H, s), 6.56 (1 H, dd, J = 1.9, 2.6 Hz), 4.51 (2H, d, J = 5.7 Hz). |
| 1-138 | 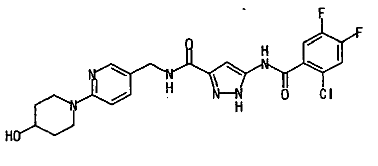 | ¹H-NMR (300 MHz, DMSO-*d*₆) δ: 13.20 (1H, br s), 11.16 (1H, br s), 9.00 (1H, br s), 8.06 (1H, d, J = 2.3 Hz), 7.89-7.79 (2H, m), 7.48 (1H, dd, J = 8.9, 2.4 Hz), 7.30 (1H, br s), 6.82 (1H, d, J = 8.7 Hz), 4.67 (1H, d, J = 4.1 Hz), 4.29 (2H, d, J = 5.7 Hz), 4.03-3.94 (2H, m), 3.73-3.62 (1H, m), 3.10-3.00 (2H, m), 1.80-1.71 (2H, m), 1.40-1.26 (2H, m). |
| 1-139 | 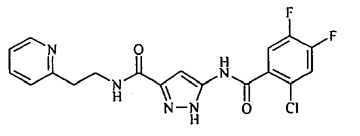 | ¹H-NMR (400 MHz, DMSO-*d*₆) δ: 13.17 (1H, br s), 11.13 (1H, br s), 8.67 (1H, br t, J = 5.6 Hz), 8.50 (1H, br d, J = 3.0 Hz), 7.87-7.77 (2H, m), 7.70 (1H, td, J = 7.4, 1.9 Hz), 7.27 (2H, d, J = 7.9 Hz), 7.24-7.19 (1H. m), 3.59 (2H, q, J=6.8 Hz), 2.98 (2H, t, J=7.4 Hz). |
| 1-140 | 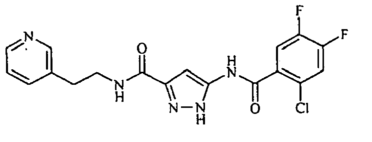 | ¹H-NMR (400 MHz, DMSO-*d*₆) δ: 13.17 (1H, br s), 11.14 (1H. s), 8.66 (1H. t, J=5.6 Hz), 8.44 (1H, d, J = 1.9 Hz), 8.40 (1H, dd, J = 4.6,1.4 Hz), 7.87-7.77 (2H, m), 7.65 (1H. dt, J = 7.9, 1.9 Hz), 7.31 (1H, q, J=4.2 Hz), 7.26 (1H, d, J=1.9 Hz), 3.49 (2H, q, J=6.6 Hz), 2.86 (2H, t, J=6.7 Hz). |

**Table 21**

| **Example** | **Molecular Structure** | **NMR** |
|---|---|---|
| 1-141 | 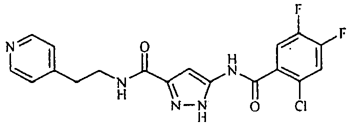 | ¹H-NMR (400 MHz, DMSO-*d*₆) δ: 13.18 (1H, s), 11.14 (1H, s), 8.67 (1H, t, J = 5.6 Hz), 8.46 (2H, d, J = 5.6 Hz), 7.87-7.77 (2H, m), 7.26 (3H, q, J = 2.0 Hz), 3.51 (2H, q, J = 6.5 Hz), 2.86 (2H, t, J = 7.0 Hz). |
| 1-142 | 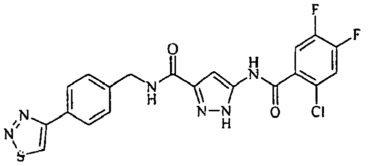 | ¹H-NMR (400 MHz, DMSO-*d*₆) δ: 13.27 (1H, br s), 11.17 (1H, s), 9.59 (1H, s), 9.23 (1H, t, J = 6.0 Hz), 8.11 (2H, d, J = 8.3 Hz), 7.87-7.78 (2H, m), 7.49 (2H, d, J = 8.3 Hz), 7.38 (1 H, d, J = 2.3 Hz), 4.52 (2H, d, J = 6.0 Hz). |
| 1-143 | 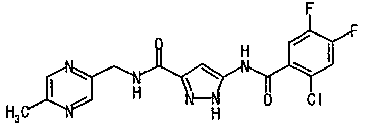 | ¹H-NMR (400 MHz, DMSO-*d*₆) δ: 13.25 (1 H, br s), 11.15 (1H, br s), 9.22 (1H, br s), 8.48 (2H, br s), 7.89-7.77 (2H, m), 7.35 (1H, br s), 4.53 (2H, d, J = 6.0 Hz), 2.46 (3H, s). |
| 1-144 | 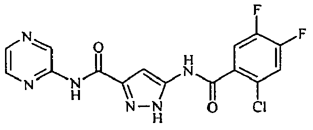 | ¹H-NMR (400 MHz, DMSO-d₆) δ: 13.54 (1 H, s), 11.33 (1H, s), 11.21 (1H, s), 9.40 (1H, d, J = 1.4 Hz), 8.49 (1H, br s), 8.43 (1H, d, J = 2.8 Hz), 7.91-7.80 (2H, m), 7.77 (1H, s). |
| 1-145 | 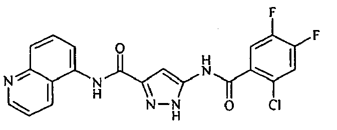 | ¹H-NMR (400 MHz, DMSO-*d*₆) δ: 13.48 (1H, s), 11.27 (1H, s), 10.65 (1H, s), 8.95 (1H, q, J = 1.9 Hz), 8.40 (1H, d, J = 8.3 Hz), 7.97 (1H, d, J = 8.3 Hz), 7.92-7.78 (3H, m), 7.70 (2H, d, J = 7.4 Hz), 7.59 (1 H, q, J = 4.2 Hz). |
| 1-146 | 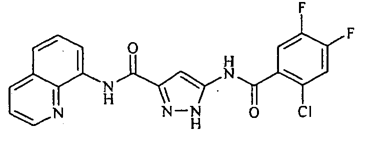 | ¹H-NMR (400 MHz, DMSO-*d*₆) δ: 13.71 (0.24H, s), 13.47 (0.75H, s), 11.61 (0.74H, s), 11.34 (0.26H, s), 11.21 (0.80H, d, J = 15.8 Hz), 10.48 (0.23H, s), 9.02 (0.16H, s), 8.98 (0.68H, q, J = 1.9 Hz), 8.79 (0.74H, t, J = 3.7 Hz), 8.62 (0.22H, d, J = 7.4 Hz), 8.47-8.43 (1.06H, m), 8.00-7.82 (1.90H, m), 7.77 (0.19H, d, J = 7.4 Hz), 7.73-7.59 (2.75H, m), 7.42 (0.26H, s), 6.59 (0.71 H, d, J = 1.4 Hz). |
| 1-147 | 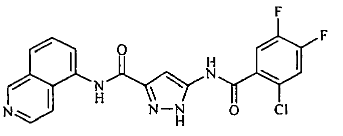 | ¹H-NMR (400 MHz, DMSO-d₆) 6: 13.48 (1H, br s), 11.25 (1H, s), 10.63 (1H, s), 9.36 (1H, s), 8.54 (1H, d, J = 6.0 Hz), 8.07 (1 H, d, J = 8.3 Hz), 7.91-7.80 (3H, m), 7.77-7.66 (2H, m). |
| 1-148 | 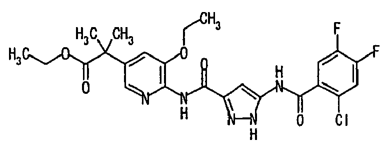 | ¹H-NMR (400 MHz, DMSO-*d*₆) δ: 13.37 (1H, br s), 11.20 (1H, br s), 10.30 (1H, br s), 7.96 (1H, d, J=1.9 Hz), 7.87-7.84 (2H, m), 7.47 (1H, br s), 7.37 (1H, d, J = 1.9 Hz), 4.13-4.09 (4H, m), 1.57 (6H, s), 1.28 (3H, t, J = 6.7 Hz), 1.15 (3H, t, J = 7.2 Hz). |

**Table 22**

| **Example** | **Molecular Structure** | **NMR** |
|---|---|---|
| 1-149 | 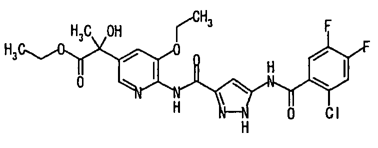 | ¹H-NMR (400 MHz, DMSO-*d*₆) δ: 13.37 (1H, br s),11.19 (1H, br s), 10.33 (1H, br s), 8.10 (1H, br s), 7.84 (2H, br s),7.54 (1 H, br s), 7.50 (1 H, br s),6.25 (1H, br s), 4.13-4.04 (4H, m), 1.70 (3H, s), 1.29 (3H, t, J = 6.7 Hz), 1.17 (3H, t, J = 7.0 Hz). |
| 1-150 | 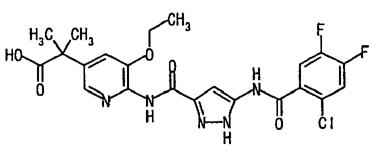 | ¹H-NMR (400 MHz, DMSO-de) δ: 13.36 (1H, br s), 11.18 (1H, br s), 10.27 (1 H, br s), 7.99 (1H, d, J = 1.9 Hz), 7.90-7.80 (2H, m), 7.39 (1 H, d, J = 1.9 Hz), 4.12 (2H, q, J = 7.0 Hz), 3.32 (2H, s), 1.54 (6H, s), 1.28 (3H, t, J = 7.0 Hz). |
| 1-151 | 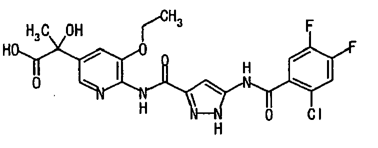 | ¹H-NMR (400 MHz, DMSO-*d*₆) δ: 13.35 (1H, br s), 11.23 (1 H, br s), 10.30 (1H. br s), 8.13 (1H, d, J = 1.9 Hz), 7.91-7.79 (2H, m), 7.56 (1 H, d, J = 1.9 Hz), 4.10 (2H, q, J = 7.1 Hz), 3.32 (2H, s), 1.69 (3H, s), 1.29 (3H, t, J = 7.0 Hz). |
| 1-152 | 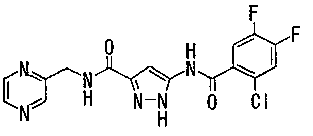 | ¹H-NMR (400 MHz, DMSO-*d*₆) δ: 13.30 (1H, br s), 11.19 (1H, br s), 9.29 (1H, br s), 8.65 (1H, br s), 8.62 (1H, t, J = 2.1 Hz), 8.57 (1H, d, J = 2.3 Hz), 7.92-7.79 (2H, m), 7.39 (1H, br s), 4.61 (2H, d, J = 6.0 Hz). |
| 1-153 | 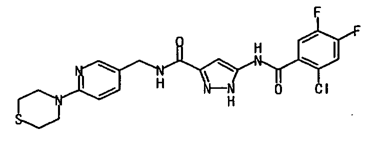 | ¹H-NMR (300 MHz, DMSO-*d*₆) δ: 13.24 (1H, br s), 11.17 (1H, br s), 9.03 (1H, br s), 8.09 (1H, d, J =1.9 Hz), 7.87-7.81 (2H, m), 7.52 (1H, dd, J = 8.8, 2.3 Hz), 7.32 (1 H, br s), 6.84 (1H, d, J = 8.3 Hz), 4.31 (2H, d, J = 6.0 Hz), 3.90-3.87 (4H, m), 2.60-2.56 (4H, m). |
| 1-154 | 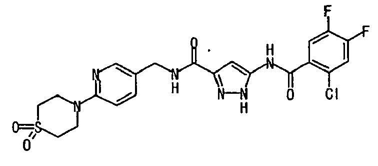 | ¹H-NMR (300 MHz, DMSO-*d*₆) δ: 13.24 (1H, br s), 11.19 (1H, br s), 9.05 (1 H, br s), 8.15 (1H, d, J = 2.3 Hz), 7.88-7.81 (2H, m), 7.59 (1 H, dd, J = 2.8, 8.8 Hz), 7.30 (1 H, br s), 7.03 (1H, d, J = 8.8 Hz), 4.34 (2H, d, J = 5.6 Hz), 4.07-4.03 (4H, m), 3.10-3.06 (4H, m). |
| 1-155 | 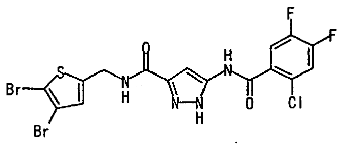 | ¹H-NMR (400 MHz, DMSO-*d*₆) δ: 13.33 (1H, br s), 11.21 (1H, br s), 9.29 (1H, br s), 7.87-7.81 (2H, m), 7.29 (1H, br s), 7.05 (1H, s), 4.52 (2H, d, J = 5.80 Hz). |

**Table 23**

| **Example** | **Molecular Structure** | **NMR** |
|---|---|---|
| 1-156 | 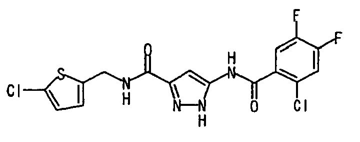 | ¹H-NMR (400 MHz, DMSO-*d*₆) δ: 13.31 (1H, br s), 11.19 (1H, br s), 9.26 (1H, br s), 7.87-7.81 (2H, m), 7.29 (1 H, br s), 6.97 (1 H, d, J = 3.8 Hz), 6.90 (1H, d, J = 3.8 Hz), 4.51 (2H, d, J = 5.8 Hz). |
| 1-157 | 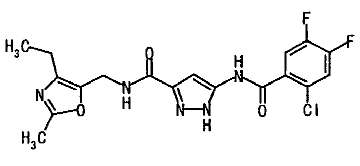 | ¹H-NMR (400 MHz, DMSO-*d*₆) δ: 13.26 (1H, s), 11.15 (1H, s), 9.06 (1H, s), 7.86-7.80 (2H, m), 7.32 (1H, s), 4.39 (2H, d, J = 5.3 Hz), 2.47 (2H, q, J = 7.5 Hz), 2.33 (3H, s), 1.12 (3H, t, J = 7.5 Hz). |
| 1-158 | 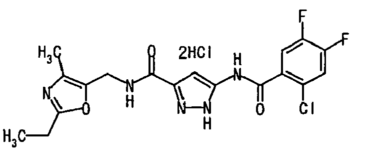 | ¹H-NMR (400 MHz, DMSO-*d*₆) δ: 11.19 (1H, s), 9.07 (1H, t, J = 5.6 Hz), 7.87-7.80 (2H, m), 7.25 (1H, s), 4.41 (2H, d, J=5.6 Hz), 2.70 (2H, q, J= 7.6 Hz), 2.11 (3H, s), 1.21 (3H, t, J=7.6 Hz). |
| 1-159 | 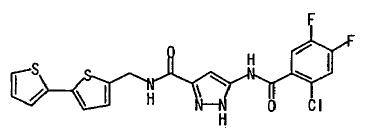 | ¹H-NMR (400 MHz, DMSO-*d*₆) δ: 13.30 (1H. br s), 11.19 (1H, br s), 9.27 (1H, br s), 7.85-7.82 (2H, m), 7.48 (1H, dd, J = 5.1, 1.2 Hz), 7.32 (1H, br s), 7.25 (1 H, dd, J = 3.7, 1.2 Hz), 7.13 (1 H, d, J = 3.7 Hz), 7.06 (1H, dd, J = 5.1, 3.7 Hz), 6.97 (1 H, d, J = 3.7 Hz), 4.58 (2H, d, J = 5.8 Hz). |
| 1-160 | 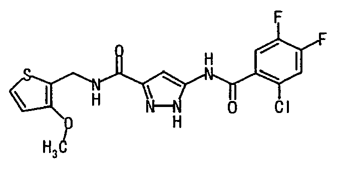 | ¹H-NMR (400 MHz, DMSO-*d*₆) 6:13.24 (1H, br s), 11.14 (1H, br s), 9.09 (1H, br s), 7.84-7.81 (2H, m), 7.33 (1 H, d, J = 5.5 Hz), 7.30 (1 H, br s), 6.97 (1H, d, J = 5.5 Hz), 4.44 (2H, d, J = 5.7 Hz), 3.82 (3H, s). |
| 1-161 | 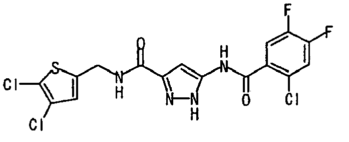 | ¹H-NMR (400 MHz, DMSO-*d*₆) δ: 13.34 (1H, s), 11.19 (1H, s), 9.34 (1H, t, J = 5.6 Hz), 7.87-7.80 (2H, m), 7.33 (1H, s), 7.08 (1H, s), 4.52 (2H, d, J = 5.6 Hz). |
| 1-162 | 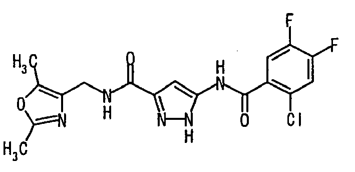 | ¹H-NMR(400 MHz, DMSO-*d*₆) δ: 13.21 (1H, s), 11.13 (1H, s), 8.96 (1H, br s), 7.86-7.80 (2H, m), 7.32 (1H, s), 4.19 (2H, d, J = 5.6 Hz), 2.31 (3H, s).2.29(3H,s). |

**Table 24**

| **Example** | **Molecular Structure** | **NMR** |
|---|---|---|
| 1-163 | 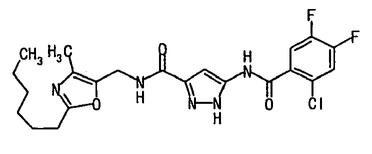 | ¹H-NMR(400 MHz, DMSO-*d*₆) δ: 13.26 (1H, s), 11.15 (1H, s), 9.05 (1 H, br s), 7.86-7.79 (2H, m), 7.32 (1H, s), 4.39 (2H,d,J=5.2 Hz), 2.63 (2H, t, J = 7.2 Hz), 2.08 (3H, s), 1.64-1.59 (2H, m), 1.31-1.24 (6H,m), 0.84 (3H,t,J=7.2 Hz). |
| 1-164 | 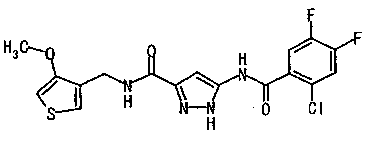 | ¹H-NMR(400 MHz, DMSO-*d*₆) δ: 13.23 (1H, s), 11.15 (1H, s), 8.98 (1 H, t, J = 5.6 Hz), 7.86-7.80 (2H, m), 7.37 (1H, s), 7.19 (1H, d, J = 3.6 Hz), 6.57 (1 H, d, J = 3.6 Hz), 4.26 (2H, d, J = 5.6 Hz), 3.79 (3H, s). |
| 1-165 | 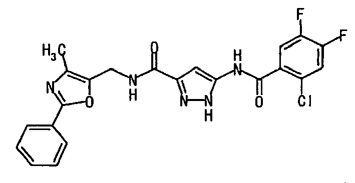 | ¹H-NMR(400 MHz, DMSO-*d*₆) δ: 13.30 (1H, s), 11.17 (1H, s), 9.16 (1H, t, J = 5.2 Hz), 7.94-7.92 (2H, m), 7.87-7.80 (2H, m), 7.55-7.49 (3H, m), 7.35 (1H.s). 4.52 (2H, d, J = 5.2 Hz), 2.22 (3H, s). |
| 1-166 | 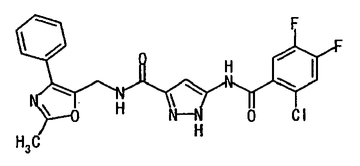 | ¹H-NMR(400 MHz, DMSO-*d*₆) δ: 13.31 (1H, s), 11.16(1H,s), 9.24(1H, t, J = 5.2 Hz), 7.88-7.81 (4H, m), 7.49-7.45 (2H, m), 7.38-7.35 (2H, m), 4.67 (2H,d.J=5.2 Hz), 2.46 (3H, s). |
| 1-167 | 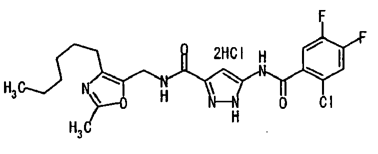 | ¹H-NMR(400 MHz, DMSO-*d*₆) δ: 11.18 (1H, s), 9.07 (1H, br s), 7.87-7.79 (2H, m), 7.25 (1H, s), 4.41 (2H, d, J = 4.0 Hz), 2.47-2.44 (2H, m), 2.38 (3H, s), 1.56-1.49 (2H, m), 1.30-1.16 (6H, m), 0.84 (3H, t, J = 6.6 Hz). |

Next, the following compounds were prepared in accordance with the above-described Preparation Method A-2.

### [Example 2-1]

### Preparation of 5-(2-chloro-4,5-difluoro-benzoylamino)-1H-pyrazole-3-carboxylic acid (6-chloro-pyridin-3-ylmethyl)-amide;

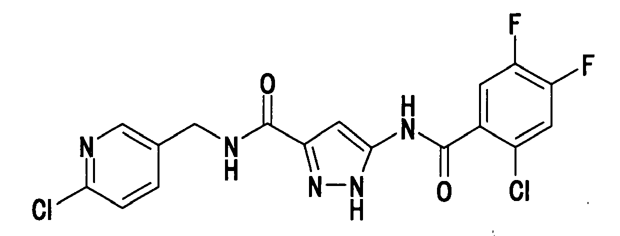

To a solution of the benzotriazol-1-yl 5-(2-chloro-4,5-difluoro-benzoylamino)-1H-pyrazole-3-carb oxylate (2.15 g) obtained in Step 6 of Reference Example 1 in N,N-dimethylformamide (11 mL) was added commercially available 3-aminomethyl-6-chloro-pyridine (0.77 g) and stirred for 3 hours at room temperature. The resulting mixture was added water (10ml) dropwise,and the formed solid was filtered to obtain the title compound (2.10 g).

### [Example 2-1-1]

### Preparation of 5-(2-chloro-4,5-difluoro-benzoylamino)-1H-pyrazole-3-carboxylic acid (6-chloro-pyridin-3-ylmethyl)-amide dihydrochloride;

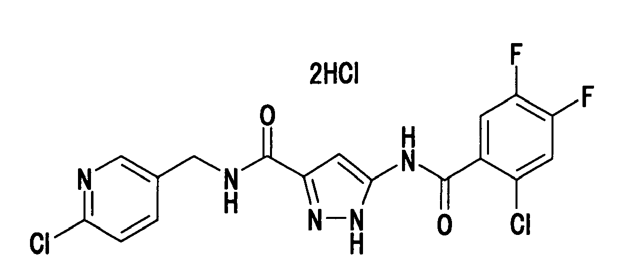

To a solution of 5-(2-chloro-4,5-difluoro-benzoylamino)-1H-pyrazole-3-carb oxylic acid (6-chloro-pyridin-3-ylmethyl)-amide (1.16 g) obtained in Example 2-1 in a methanol (10 mL) -tetrahydrofuran (10 mL) was added 4 N hydrochloric acid-ethyl acetate (1.7 ml), and stand. The formed solid was filtered to obtain the title compound (1.07 g).

### [Example 2-2]

### Preparation of 5-(2-chloro-4,5-difluoro-benzoylamino)-1H-pyrazole-3-carboxylic acid (4H-[1,2,4]triazol-3-ylmethyl)-amide;

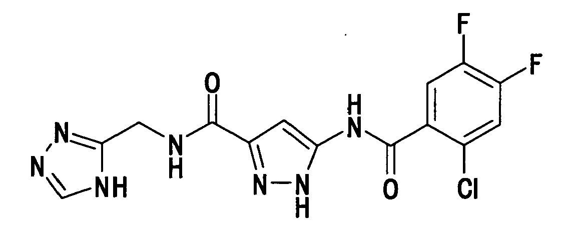

In the same manner as in Example 2-1, the title compound (50 mg) was obtained from benzotriazol-1-yl 5-(2-chloro-4,5-difluoro-benzoylamino)-1H-pyrazole-3-carb oxylate (160 mg) and (4H-[1,2,4]triazol-3-ylmethyl)-amine (42 mg) prepared in accordance with Bioorganic Med. Chem. Lett . , 4, 2441, 1994.

### [Example 2-2-1]

### Preparation of 5-(2-chloro-4,5-difluoro-benzoylamino)-1H-pyrazole-3-carboxylic acid (4H-[1,2,4]triazol-3-ylmethyl)-amide dihydrochloride;

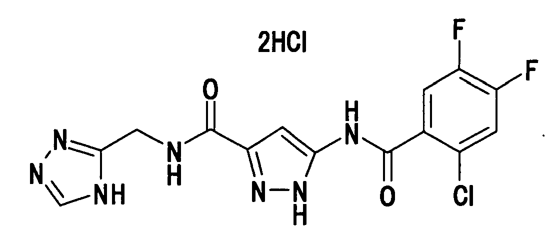

In the same manner as in Example 2-1-1, the title compound (30 mg) was obtained from 5-(2-chloro-4,5-difluoro-benzoylamino)-1H-pyrazole-3-carb oxylic acid (4H-[1,2,4]triazol-3-ylmethyl)-amide (37 mg).

### [Example 2-3]

### Preparation of 5-(2-chloro-4,5-difluoro-benzoylamino)-1H-pyrazole-3-carboxylic acid(5-fluoro-4H-quinazoline-3-yl)-amide;

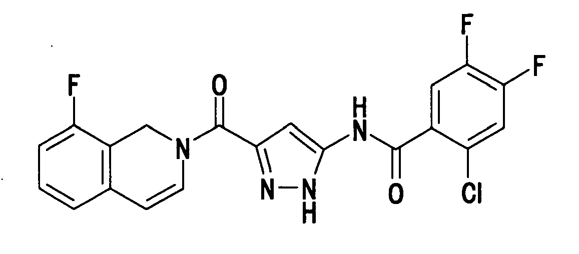

In the same manner as in Example 2-1, the title compound (155 mg) was obtained from benzotriazol-1-yl 5-(2-chloro-4,5-difluorobenzoylamino)-1H-pyrazole-3-carbo xylate (210mg) and 5-fluoro-3,4-dihydro-quinazoline (150mg) prepared in accordance with J. Heterocyclic Chem., 12, 883 1975.

### [Example 2-3-1]

### Preparation of 5-(2-chloro-4,5-difluoro-benzoylamino)-1H-pyrazole-3-carboxylic acid (5-fluoro-4H-quinazoline-3-yl)-amide dihydrochloride;

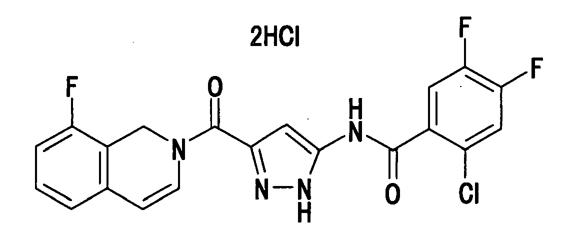

In the same manner as in Example 2-1-1, the title compound (23 mg) was obtained from 5-(2-chloro-4,5-difluoro-benzoylamino)-1H-pyrazole-3-carb oxylic acid (5-fluoro-4H-quinazoline-3-yl)-amide (48 mg).

### [Example 2-4]

### Preparation of 5-(2-chloro-4,5-difluoro-benzoylamino)-1H-pyrazole-3-carboxylic acid (4,6-dimethyl-pyridin-3-ylmethyl)-amide Step 1: Preparation of 3-aminomethyl-4,6-dimethyl-pyridine;

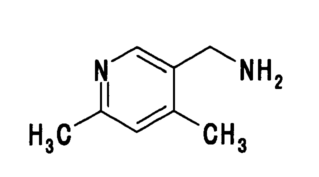

3-cyano-4,6-dimethylpyridine (1.23 g) was prepared from 2-chloro-3-cyano-4,6-dimethylpyridine(2.00g)in accordance with Tetrahedron, 22, 3417, 1996.

The title compound (436 mg) was prepared from 3-cyano-4,6-dimethylpyridine with reference to J. Heterocyclic Chem., 30, 473, 1993.

### Step 2: Preparation of

### 5-(2-chloro-4,5-difluoro-benzoylamino)-1H-pyrazole-3-carboxylic acid (4,6-dimethyl-pyridin-3-ylmethyl)-amide;

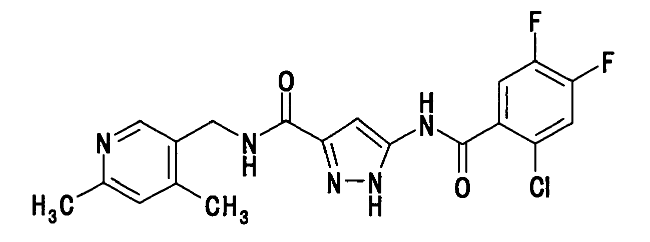

In the same manner as in Example 2-1, the title compound (0.110 g) was obtained from benzotriazol-1-yl 5-(2-chloro-4,5-difluoro-benzoylamino)-1H-pyrazole-3-carb oxylate (140mg) and 3-aminomethyl-4,6-dimethyl-pyridine (70 mg).

### [Example 2-4-1]

### Preparation of 5-(2-chloro-4,5-difluoro-benzoylamino)-1H-pyrazole-3-carboxylic acid (4,6-dimethyl-pyridin-3-ylmethyl)-amide dihydrochloride;

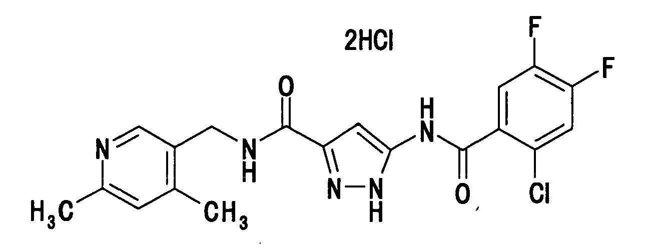

To a suspension of 5-(2-chloro-4,5-difluoro-benzoylamino)-1H-pyrazole-3-carb oxylicacid(4,6-dimethyl-pyridin-3-ylmethyl)-amide(430mg) in methanol (0.5 mL)-tetrahydrofuran (25 mL) was added 4 N hydrochloric acid/ethyl acetate (0.64 mL). The resulting solution was concentrated to about 1/6 under reduced pressure, allowed to stand and the formed solid was filtered to obtain the title compound (444 mg).

### [Example 2-5]

### Preparation of 5-(2-chloro-4,5-difluoro-benzoylamino) -1H-pyrazole-3-carboxylic acid (2-methoxy-6-methyl-pyridin-3-ylmethyl) -amide; Step 1: Preparation of 3-aminomethyl-2-methoxy-6-methyl-pyridine;

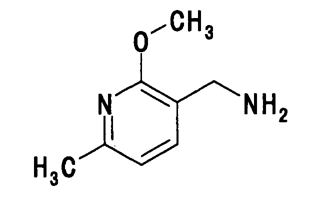

3-cyano-2-methoxy-6-methylpyridine (2.74 g) was prepared from 2-chloro-3-cyano-6-methylpyridine (3.00 g) in accordance with J. Heterocyclic Chem., 36, 653, 1999. Subsequently, the title compound (608 mg) was prepared from 3-cyano-2-methoxy-6-methylpyridine (739 mg) with reference to J. Heterocyclic Chem., 30, 473, 1993.

### Step 2: Preparation of 5-(2-chloro-4,5-difluoro-benzoylamino)-1H-pyrazole-3-carboxylic acid (2-methoxy-6-methyl-pyridin-3-ylmethyl)-amide;

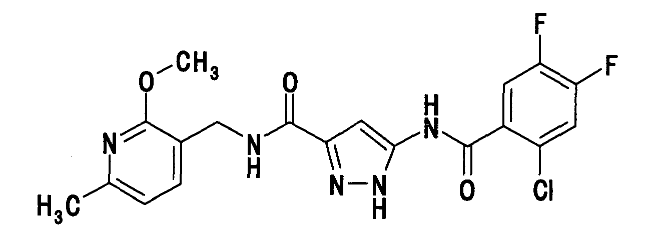

In the same manner as in Example 2-1, the title compound (501 mg) was obtained from benzotriazol-1-yl 5-(2-chloro-4,5-difluoro-benzoylamino)-1H-pyrazole-3-carb oxylate (1.66 g) and 3-aminomethyl 2-methoxy-6-methyl-pyridine (603 mg).

### [Example 2-6]

### Preparation of 5-(2-chloro-4,5-difluoro-benzoylamino)-1H-pyrazole-3-carboxylic acid (4-methoxy-6-methyl-pyridin-3-ylmethyl)-amide Step 1: Preparation of ethyl 4-methoxy-6-methylnicotinate;

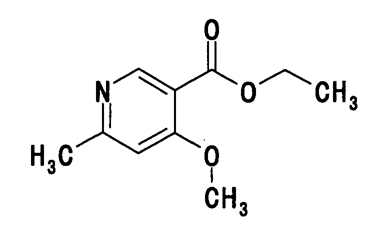

Ethyl 2-chloro-4-methoxy-6-methylnicotinate) (0.387g) was prepared from ethyl 2,4-dichloro-6-methylnicotinate) (1.00 g) in accordance with Synthesis, 6, 479, 1988. Subsequently, the title compound (217 mg) was prepared in accordance with the same document.

### Step 2: Preparation of 3-hydroxymethyl-4-methoxy-6-methylpyridine;

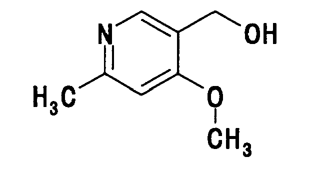

The title compound (61 mg) was prepared from 4-methoxy-6-methyl nicotinate (212 mg) with reference to Synthesis, 26, 2257, 1996.

### Step 3: Preparation of 3-aminomethyl-4-methoxy-6-methyl-pyridine;

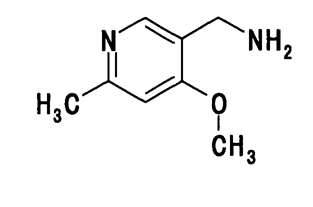

3-chloromethyl-4-methoxy-6-methylpyridine was prepared from 3-hydroxymethyl-4-methoxy-6-methylpyridine (553 mg) with reference to J. Med. Chem., 46, 453, 2003. Without being purified, this product was reacted with sodium azide to prepare 3-azidomethyl-4-methoxy-6-methylpyridine (403 mg) and the title compound (331 mg) was further prepared in accordance with the same document.

### Step 4: Preparation of 5-(2-chloro-4,5-difluoro-benzoylamino)-1H-pyrazole-3-carboxylic acid (4-methoxy-6-methyl-pyridin-3-ylmethyl)-amide;

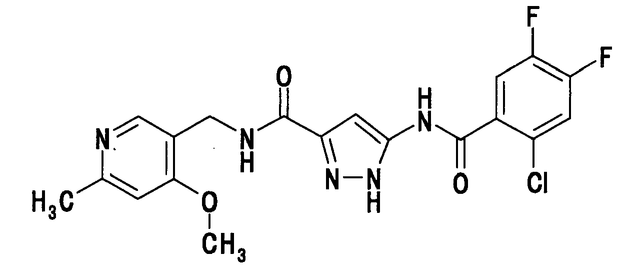

In the same manner as in Example 2-1, the title compound (368 mg) was obtained from benzotriazol-1-yl 5-(2-chloro-4,5-difluoro-benzoylamino)-1H-pyrazole-3-carb oxylate (413 mg) and 3-aminomethyl-4-methoxy-6-methyl-pyridine (150 mg).

### [Example 2-6-1]

### Preparation of 5-(2-chloro-4,5-difluoro-benzoylamino)-1H-pyrazole-3-carboxylic acid (4-methoxy-6-methyl-pyridin-3-ylmethyl)-amide dihydrochloride;

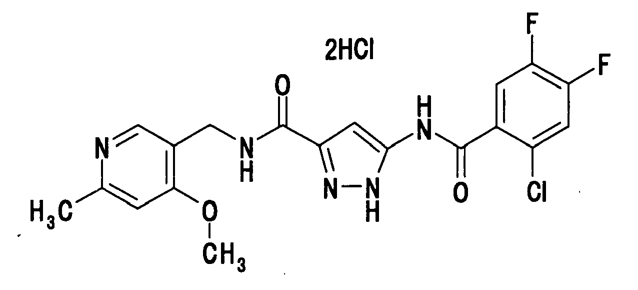

In the same manner as in Example 2-4-1, the title compound (124 mg) was prepared from 5-(2-chloro-4,5-difluoro-benzoylamino)-1H-pyrazole-3-carb oxylic acid (4-methoxy-6-methyl-pyridin-3-ylmethyl)-amide (363 mg).

### [Example 2-7]

### Preparation of 5-(2-chloro-4,5-difluoro-benzoylamino)-1H-pyrazole-3-carboxylic acid (2-methoxy-4,6-dimethyl-pyridin-3-ylmethyl)-amide;

### Step 1: Preparation of 3-aminomethyl-2-methoxy-4,6-dimethyl-pyridine;

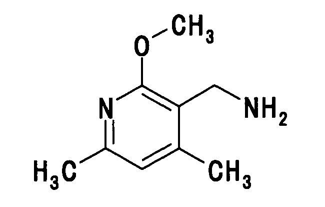

3-cyano-2-methoxy-4,6-dimethylpyridine (1.31 g) was prepared from 3-cyano-2-chloro-4,6-dimethylpyridine (1.50 g) in accordance with J. Heterocyclic Chem., 36, 653, 1999. The title compound (826 mg) was further prepared from 3-cyano-2-methoxy-4,6-dimethylpyridine (809 mg) with reference to J. Heterocyclic Chem., 30, 473, 1993.

### Step 2: Preparation of

### 5-(2-chloro-4,5-difluoro-benzoylamino)-1H-pyrazole-3-carboxylic acid (2-methoxy-4,6-dimethyl-pyridin-3-ylmethyl)-amide;

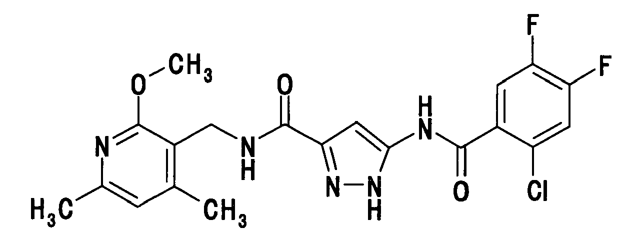

In the same manner as in Example 2-1, the title compound (1.23 g) was obtained from benzotriazol-1-yl 5-(2-chloro-4,5-difluorobenzoylamino)-1H-pyrazole-3-carbo xylate (1.26 g) and 3-aminomethyl 2-methoxy-4, 6-dimethyl-pyridine (500mg) obtained in Step 1.

### [Example 2-7-1]

### Preparation of 5-(2-chloro-4,5-difluoro-benzoylamino)-1H-pyrazole-3-carboxylic acid (2-methoxy-4,6-dimethyl-pyridin-3-ylmethyl)-amide dihydrochloride;

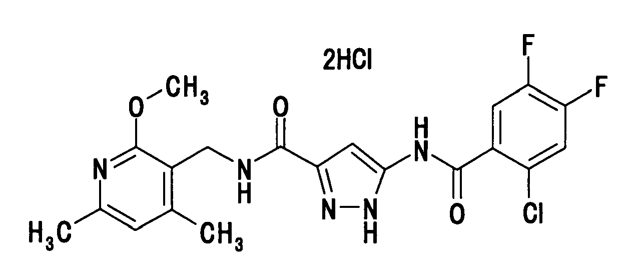

In the same manner as in Example 2-1-1, the title compound (288 mg) was prepared from 5-(2-chloro-4,5-difluoro-benzoylamino)-1H-pyrazole-3-carb oxylic acid (2-methoxy-4,6-dimethyl-pyridin-3-ylmethyl)-amide (261 mg).

Next, the compounds Example 2-8 to Example 2-33 illustrated in the following tables were prepared in the same manner as in the above-described Example 2-1 to Example 2-7-1.

**Table 25**

| **Example** | **Molecular Structure** | **NMR** |
|---|---|---|
| 2-1 | 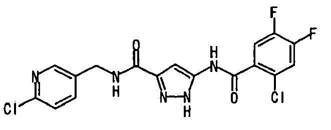 | ¹H-NMR(400 MHz, DMSO-*d*₆) δ: 4.46 (2H, d, J = 5.5 Hz), 7.34 (1H, s), 7.50 (1H, d, J= 8.1 Hz), 7.79-7.81 (3H, m), 8.38 (1H, s), 9.18 (1H, s), 11.17 (1H, s), 13.27 (1H, s). |
| 2-1-1 | 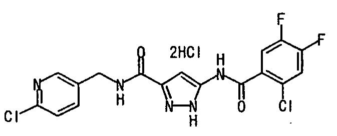 | ¹H-NMR(400 MHz, DMSO-*d*₆) δ: 4.46 (2H, d, J = 5.9 Hz), 7.24 (1H, s), 7.50 (1H.d.J=8.1 Hz), 7.81-7.84 (3H, m), 8.38 (1H, d, J = 2.2 Hz), 9.19 (1H, s), 11.19 (1H, s). |
| 2-2 | 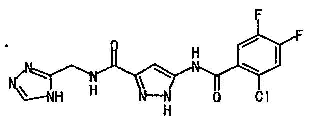 | ¹H-NMR (400 MHz, DMSO-*d*₆) δ:4.52(2H,d,J=5.6Hz), 7,34(1H,brs), 7.83-7.87(2H,m), 9.14(1H,s),11.18(1H,s),13.26(1H,s). |
| 2-2-1 | 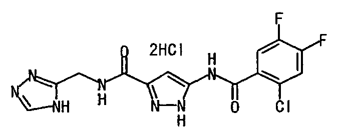 | 1H-NMR (400 MHz, DMSO-*d*₆) δ: 11.24 (1H, s), 9.26 (1H, d, J = 5.8 Hz), 8.89 (1H, s), 7.89-7.83 (2H, m), 7.28 (1H, s), 4.65 (2H, d, J = 5.6 Hz). |
| 2-3 | 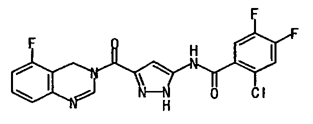 | H-NMR (400 MHz, DMSO-*d*₆) δ:4.92(2H,s),7.06-7.14(2H,m),7.33(1H,m),7.90(1H,m), 11.52(1H,m),13.71(1H,brs). |
| 2-3-1 | 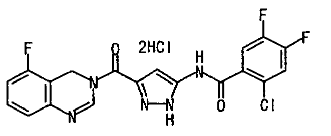 | ¹H-NMR(400 MHz, DMSO-*d*₆) δ: 11.64(1H.s), 8.99(1H,brs), 7.92(2H,m), 7.43(1H,m), 7.19(2H,m), 6.89(1H,s), 5.01(2H,s). |
| 2-4 | 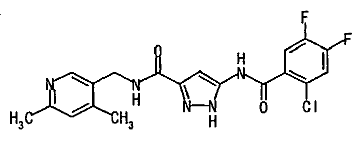 | ¹H-NMR (400 MHz, DMSO-d₆) δ:2.30 (3H, s), 2.39 (3H, s), 4.42 (2H, d, J = 5.5 Hz), 7.06 (1H, s), 7.34 (1H, s), 7.78-7.86 (2H, m), 8.29 (1H, s), 8.97 (1H, s), 11.14 (1H, s), 13.23 (1H, s). |
| 2-4-1 | 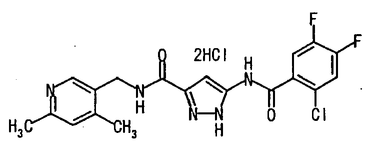 | ¹H-NMR(400 MHz, DMSO-d₆) δ:2.58 (3H, s), 2.68 (3H, s), 4.56 (2H, d, J = 5.5 Hz), 7.25 (1H, s), 7.79-7.85 (3H, m), 8.54 (1H, s), 9.19 (1H. s), 11.22 (1H, s). |

**Table 26**

| **Example** | **Molecular Structure** | **NMR** |
|---|---|---|
| 2-5 | 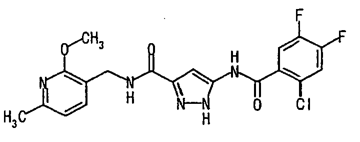 | ¹H-NMR(400 MHz, DMSO-*d*₆)) δ: 2.37 (3H, s), 3.89 (3H, s), 4.33 (2H, d, J=5.5 Hz), 6.81 (1H, d, J = 7.7 Hz), 7.36 (1 H, s), 7.43 (1 H, d, J = 7.3 Hz), 7.79-7.87 (2H, m), 8.96 (1H, s), 11.16 (1H, s), 13.22 (1H, s). |
| 2-6 | 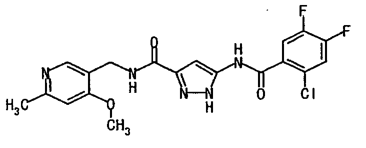 | ¹H-NMR (400 MHz, DMSO-*d*₆) δ: 2.42 (3H, s), 3.86 (3H, s), 4.36 (2H, d, J = 5.5 Hz), 6.93 (1H, s), 7.35 (1H, s), 7.82 (2H, dt, J = 10.0, 5.0 Hz), 8.14 (1H, s), 8.91 (1H, t, J = 10.0 Hz), 11.13 (1H, s), 13.22 (1H, s). |
| 2-6-1 | 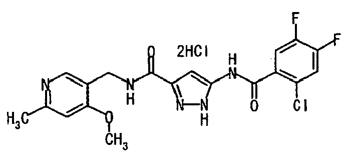 | ¹H-NMR(400 MHz, DMSO-*d*₆) δ: 2.70 (3H, s), 4.12 (3H, s), 4.45 (2H, d, J = 5.6 Hz), 7.26 (1H, s), 7.60 (1H, s), 7.81-7.88 (2H, m), 8.40 (1H, s), 9.11 (1H, t, J = 5.6 Hz), 11.23 (1H, s), 15.43 (1H, s). |
| 2-7 | 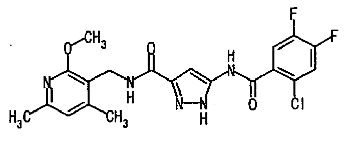 | H-NMR (400 MHz, DMSO-*d*₆) δ: 2.29 (3H, s), 2.33 (3H, s), 3.85 (3H, s), 4.40 (2H, d, J = 4.8 Hz), 6.70 (1H, s), 7.30 (1H, s), 7.76-7.85 (2H, m), 8.54 (1H, s), 11.09 (1H,s), 13.15 (1H,s). |
| 2-7-1 | 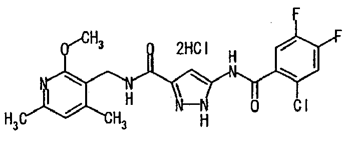 | ¹H-NMR(400 MHz, DMSO-*d*₆) δ:2.30 (3H, s), 2.34 (3H, s), 3.87 (3H, s), 4.40 (2H, d, J = 4.8 Hz), 6.72 (1H, s), 7.24 (1H, s), 7.76-7.85 (2H, m), 8.53 (1H, t, J = 4.8 Hz), 11.11 (1H, s). |
| 2-8 | 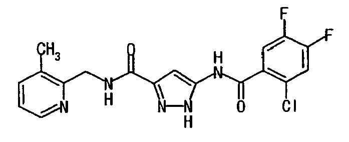 | ¹H-NMR(300 MHz, DMSO-*d*₆) δ:13.21 (1H, s), 11.14 (1 H, s), 8.92 (1 H, s), 8.37 (1 H, d, J = 4.2 Hz), 7.84 (2H, dd, J = 10.0, 7.2 Hz), 7.59 (1H, d, J = 7.4 Hz), 7.35 (1H, s), 7.23 (1H, dd, J = 7.4, 4.6 Hz), 4.56 (2H, d, J = 5.1 Hz), 2.34 (3H, s). |
| 2-9 | 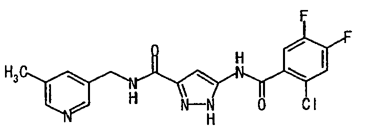 | ¹H-NMR(300 MHz, DMSO-*d*₆) δ: 13.27 (1H, s), 11.16 (1H, s), 9.14 (1H, s), 8.33 (2H, d, J=15.0 Hz), 7.86-7.79 (2H, m), 7.53 (1H, s), 7.33 (1H, s), 4.43 (2H, d, J = 6.0 Hz), 2.29 (3H, s). |

**Table 27**

| **Example** | **Molecular Structure** | **NMR** |
|---|---|---|
| 2-10 | 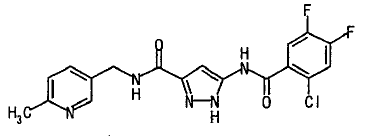 | ¹H-NMR(300 MHz, DMSO-*d*₆) δ: 13.27 (1H, s), 11.17 (1H, s), 9.14 (1H, s), 8.41 (1H, s), 7.86-7.80 (2H, m), 7.60 (1 H, dd, J = 7.9, 2.3 Hz), 7.32 (1 H, s), 7.22 (1H, d, J = 7.9 Hz), 4.41 (2H, d, J = 5.6 Hz), 2.44 (3H, s). |
| 2-11 | 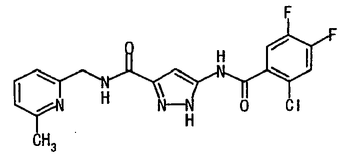 | ¹H-NMR(300 MHz, DMSO-*d*₆) δ:13.27 (1H, s), 11.17 (1H, s), 9.16 (1H, s), 7.87-7.79 (3H, m), 7.64 (1 H, t, J = 7.7 Hz), 7.12 (2H, t, J = 6.4 Hz), 4.48 (2H, d, J = 5.9 Hz), 2.46 (3H, s). |
| 2-12 | 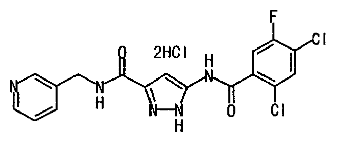 | ¹H-NMR(300 MHz, DMSO-*d*₆) δ: 11.28 (1H, s), 9.36 (1H, t, J = 5.8 Hz), 8.90 (1H, s), 8.85 (1 H, d, J = 5.6 Hz), 8.52 (1H, d, J = 8.3 Hz), 8.05 (1H, dd, J = 7.9, 5.6 Hz), 7.95 (1 H, d, J = 6.5 Hz), 7.78 (1 H, d, J = 9.3 Hz), 7.25 (1H, s), 4.65 (2H, d, J = 5.6 Hz). |
| 2-13 | 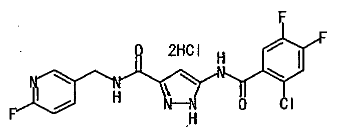 | ¹H-NMR(300 MHz, DMSO-*d*₆)δ: 11.19 (1H, s), 8.20-8.11 (1H, m), 7.94-7.68 (2H, m), 7.24 (1H, s), 7.18 (1 H, m), 4.33 (2H, d, J = 5.7 Hz). |
| 2-14 | 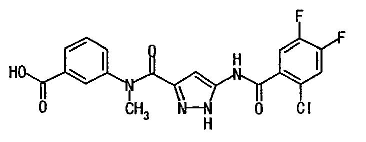 | ¹H-NMR(300 MHz, DMSO-*d*₆) δ: 13.24 (2H, s), 10.98 (1H, s), 7.97-7.49 (5H, m), 5.57 (2H, d, J = 5.2 Hz), 3.38 (3H, s). |
| 2-15 | 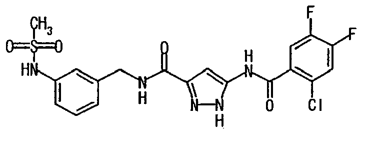 | ¹H-NMR(300 MHz, DMSO-*d*₆) δ: 13.23 (1H, s), 11.14 (1H. s), 9.73 (1H. s), 9.14 (1 H, s), 7.83 (2H, q, J = 8.8 Hz), 7.34-7.28 (2H, m), 7.17-7.04 (3H, m), 4.42 (2H, d, J = 5.5 Hz), 2.97 (3H, s). |
| 2-16 | 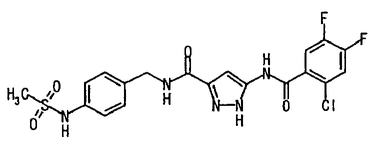 | ¹H-NMR(300 MHz, DMSO-*d*₆) δ:13.22 (1H, s), 11.13 (1H, s), 9.65 (1H, s), 9.10 (1H, s), 7.87-7.77 (2H, m), 7.32-7.29 (5H, m), 4.40 (2H, d, J = 5.9 Hz), 2.95 (3H, s). |

**Table 28**

| **Example** | **Molecular Structure** | **NMR** |
|---|---|---|
| 2-17 | 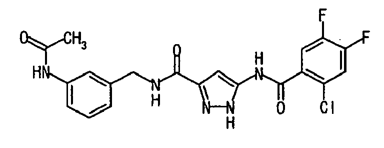 | ¹H-NMR(300 MHz, DMSO-*d*₆) δ: 13.22 (1H, s), 11.13 (1H, s), 9.91 (1H, s), 9.14 (1H, s), 7.86-7.78 (2H, m), 7.54-7.44 (1H, m), 7.36 (1H, s), 7.25-7.23 (1 H, m), 6.98 (1 H, d, J = 7.0 Hz), 6.50-6.43 (1 H, m), 4.41 (2H, d, J = 5.5 Hz), 2.01 (3H, s). |
| 2-18 | 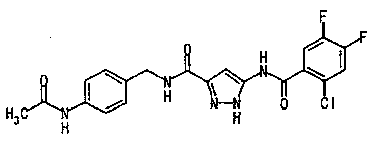 | ¹H-NMR(300 MHz, DMSO-*d*₆) 6: 13.21 (1H, s), 11.12 (1H, s), 9.89 (1H, s), 9.07 (1H, s), 7.88-7.76 (2H, m), 7.52 (2H, d, J = 8.4 Hz), 7.34 (1H, s), 7.23 (2H, d, J = 8.4 Hz), 4.38 (2H, d, J = 5.1 Hz), 2.02 (3H, s). |
| 2-19 | 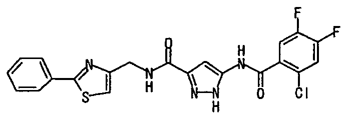 | ¹H-NMR(300 MHz, DMSO-*d*₆) δ: 13.28 (1H, s), 11.17 (1H, s), 9.23 (1H, s), 7.93-7.82 (4H, m), 7.49-7.40 (5H, m), 4.60 (2H, d, J = 5.6 Hz). |
| 2-20 | 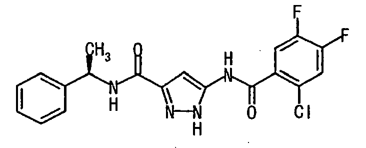 | ¹H-NMR(300 MHz, DMSO-*d*₆) δ: 13.15 (1H, s), 11.13 (1 H, s), 8.93 (1H, s), 7.95-7.81 (1H, m), 7.39-7.28 (7H, m), 5.14 (1 H, t, J = 6.8 Hz), 1.47 (3H, d, J =6.6 Hz). |
| 2-21 | 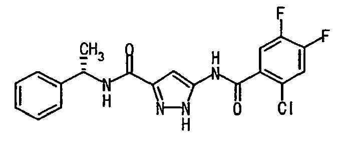 | ¹H-NMR(300 MHz, DMSO-*d*₆) δ: 13.15 (1H, s), 11.12 (1H, s), 8.93 (1H, s), 7.84-7.81 (2H, m), 7.53-7.17 (6H, m), 5.14 (1H, t, J = 6.8 Hz), 1.47 (3H, d, J = 6.2 Hz). |
| 2-22 | 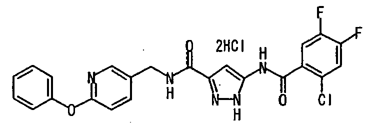 | ¹H-NMR(300 MHz, DMSO-*d*₆) δ: 11.18 (1H, s), 9.13 (1H, t, J = 6.0 Hz), 8.12 (1H, d, J = 2.3 Hz), 7.87-7.80 (3H, m), 7.41 (2H, td, J = 6.8, 1.7 Hz), 7.25-7.18 (2H, m), 7.12-7.09 (2H, m), 7.01 (1H, d, J = 8.3 Hz), 4.41 (2H, d, J = 5.6 Hz). |
| 2-23 | 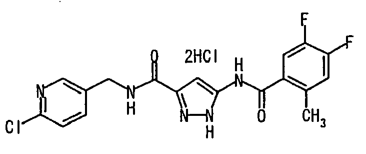 | ¹H-NMR(300 MHz, DMSO-*d*₆) δ: 10.99 (1H, s), 9.15 (1H, t, J=6.2 Hz), 8.38 (1H, d, J = 2.6 Hz), 7.80 (1H, dd, J = 8.1, 2.6 Hz), 7.59 (1H, dd, J = 10.8, 8.3 Hz), 7.50 (1H, d, J = 8.1 Hz), 7.40 (1 H, dd, J = 11.6, 7.5 Hz), 7.19 (1H, s), 4.45 (2H, d, J = 5.9 Hz), 2.36 (3H, s). |
| 2-24 | 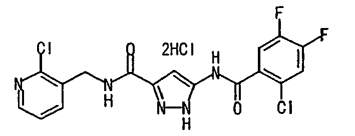 | ¹H-NMR(300 MHz, DMSO-*d*₆) δ: 11.20 (1H, s), 9.18 (1H, t, J = 6.1 Hz), 8.34 (1 H, q, J = 2.2 Hz), 7.88-7.77 (3H, m), 7.44 (1 H, dd, J = 7.7, 4.8 Hz), 7.30 (1H, s), 4.50 (2H, d, J = 5.5 Hz). |

**Table 29**

| **Example** | **Molecular Structure** | **NMR** |
|---|---|---|
| 2-25 | 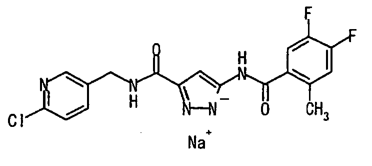 | ¹H-NMR(300 MHz, DMSO-*d*₆) δ: 8.69 (1H, br s), 8.35 (1H, d, J = 2.2 Hz), 7.81-7.76 (1H, m), 7.59-7.44 (2H, m), 7.25-7.19 (1H, m), 6.52 (1H, s), 4.41 (2H, s).2.41 (3H, s). |
| 2-26 | 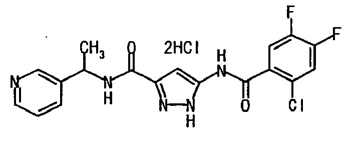 | ¹H-NMR(300 MHz, DMSO-*d*₆) δ: 11.22 (1H, s), 9.22-9.12 (1H,m), 8.91 (1H, s), 8.82-8.72 (1H, m), 8.50-8.40 (1H, m), 7.94-7.80 (3H, m), 7.36 (1H, s), 5.31-5.28 (1H, m), 1.51 (3H, s). |
| 2-27 | 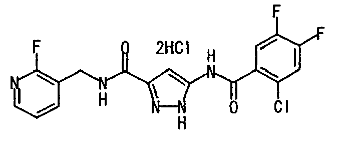 | ¹H-NMR(300 MHz, DMSO-*d*₆) δ:11.20 (1H, s), 9.16 (1H, t, J = 5.8 Hz), 8.15 (1H, d, J = 4.6 Hz), 7.91-7.80 (3H, m), 7.37-7.34 (1H, m), 7.29 (1H, s), 4.47 (2H, d, J = 6.0 Hz). |
| 2-28 | 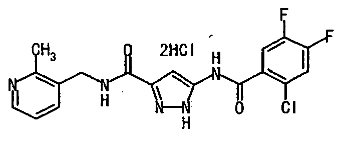 | ¹H-NMR(300 MHz, DMSO-*d*₆) δ:11.24 (1H, s), 9.29 (1H. s), 8.68 (1H, d, J = 4.6 Hz), 8.35 (1H, d, J = 7.9 Hz), 7.89-7.79 (3H, m), 7.29 (1 H, s), 4.58 (2H, d, J = 5.6 Hz), 2.80 (3H, d, J = 7.4 Hz). |
| 2-29 | 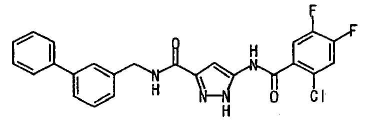 | ¹H-NMR(300 MHz, DMSO-*d*₆) δ: 13.29 (1H, s), 11.15 (1H. s), 9.16 (1H, s), 7.86-7.31 (12H, m), 4.52 (2H, d, J = 5.9 Hz). |
| 2-30 | 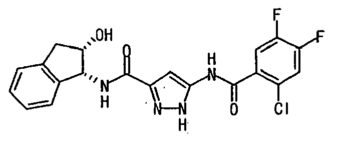 | ¹H-NMR(300 MHz, DMSO-*d*₆) δ: 8.37 (1H, s), 7.87-7.77 (2H, m), 7.28-7.17 (5H, m), 5.40 (1H, dd, J = 8.4, 5.1 Hz), 5.16 (1 H, s), 4.51 (1 H, s), 3.10 (1H, dd, J = 16.3, 5.0 Hz), 2.93-2.85 (2H, m), 2.74-2.71 (1H, m). |
| 2-31 | 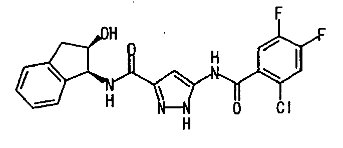 | ¹H-NMR(300 MHz, DMSO-*d*₆) δ: 13.24 (1H, s), 11.11 (1H, s), 8.48 (1H, s), 7.95-7.73 (2H, m), 7.49-7.15 (3H, m), 5.40 (1 H, t, J = 6.8 Hz), 5.10 (1 H, s), 4.51 (1H, d, J = 2.6 Hz), 3.10 (1H, dd, J = 16.1, 5.1 Hz), 2.93-2.85 (2H, m), 2.73 (1H, s). |

**Table 30**

| **Example** | **Molecular Structure** | **NMR** |
|---|---|---|
| 2-32 | 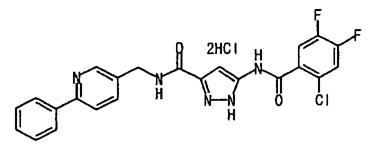 | ¹H-NMR(300 MHz, DMSO-*d*₆) δ: 11.22 (1H, s), 9.28 (1H, t, J = 5.8 Hz), 8.73 (1 H, s), 8.16-8.06 (4H, m), 7.88-7.80 (3H, m), 7.59-7.52 (3H, m), 7.27 (1H, s), 4.58 (2H, d, J = 6.0 Hz). |
| 2-33 | 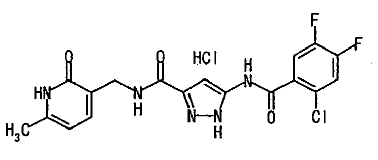 | ¹H-NMR(300 MHz, DMSO-*d*₆) δ: 11.69 (1H, s), 11.17 (1H. s), 8.84 (1H, t, J = 5.9 Hz). 7.88-7.78 (2H, m), 7.27 (1H. s), 7.18 (1H. d, J = 7.0 Hz), 5.98 (1 H, d, J = 7.3 Hz), 4.17 (2H, d, J = 5.5 Hz), 2.16 (3H.s). |

The following compounds were prepared in accordance with the above-described Preparation Method A-3.

### [Example 3-1]

### Preparation of 5-(2-chloro-4,5-difluoro-benzoylamino)-1H-pyrazole-3-carboxylic acid benzyl-methyl-amide;

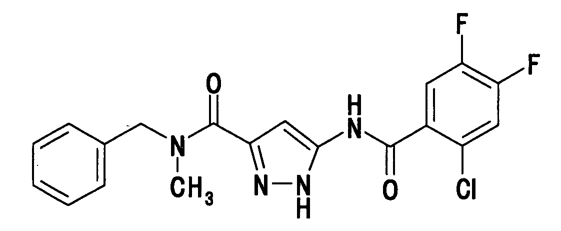

To a solution of 5-(2-chloro-4,5-difluoro-benzoylamino)-1H-pyrazole-3-carb oxylic acid (70 mg) prepared according to Step 5 of Reference Example 1, N-methylbenzylamine (0.033 mL) and 1-hydroxybenzotriazole hydrate (43 mg) in N,N-dimethylformamide (1 mL) was added EDC hydrochloride (50 mg), and the resulting mixture was stirred overnight at room temperature. Saturated aqueous sodium bicarbonate (2 mL) and water (5 mL) were added to the resulting reaction mixture, and the formed solid was filtered to obtain the title compound (76 mg) as a white solid.

### [Example 3-2]

### Preparation of 5-(2-chloro-4,5-difluoro-benzoylamino)-1H-pyrazole-3-carboxylic acid benzylamide;

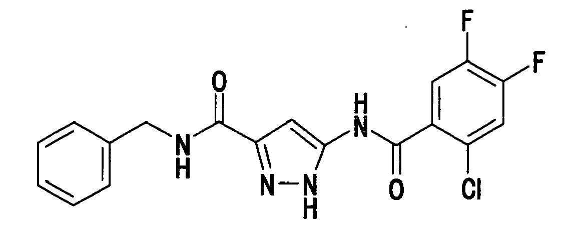

To a solution of 5-(2-chloro-4,5-difluoro-benzoylamino)-1H-pyrazole-3-carb oxylic acid (70 mg) obtained according to Step 5 of Reference Example 1, benzylamine (0.028 mL) and 1-hydroxybenzotriazole hydrate (45 mg) in N,N-dimethylformamide (1 mL) was added EDC hydrochloride (50 mg), and the resulting mixture was stirred overnight at room temperature. 0.1 N aqueous sodium hydroxide (1 mL) and then water (3 mL) were added to the resulting reaction mixture, and the formed solid was filtered to obtain the title compound (66 mg) as a white solid.

The compounds Example 3-3 to Example 3-53 were prepared in the same manner as in the above-described Example 3-1 and Example 3-2.

**Table 31**

| **Example** | **Molecular Structure** | **NMR** |
|---|---|---|
| 3-1 | 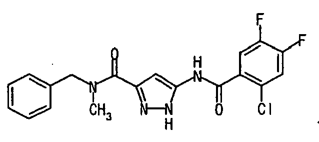 | ¹H-NMR(400 MHz, DMSO-*d*₆) δ: 13.29 (1H, s), 11.44 (0.2H.brs). 11.19 (0.5H, br s), 11.14 (0.3H, br s), 7.95-7.75 (2H, m), 7.45-7.23 (5H, m), 7.00 (0.5H, m), 6.77 (0.3H, m), 6.44 (0.2H, m), 5.11 (0.3H, m), 4,82 (0.6H, m), 4.70 (1.1H, m), 3.17 (1.7H, s), 2,94 (0.9 H, s), 2.86 (0.4 H, s). |
| 3-2 | 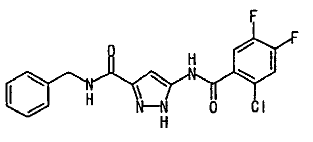 | ¹H-NMR(400 MHz, DMSO-*d*₆) δ: 13.24 (1H, s), 11.15 5 (1H, s), 9.14 (1H, t, J = 6.0 Hz), 7.86-7.79 (2H, m), 7.38-7.31 (5H, m), 7.24 (1H, m), 4.45 (2H, d, J = 6.0 Hz). |
| 3-3 | 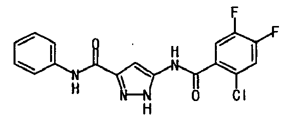 | ¹H-NMR(400 MHz, DMSO-*d*₆) δ: 13.41 (1H, s), 11.23 (1H. s), 10.30 (1H, s), 7.89-7.79 (4H, m), 7.62 (1H, s), 7.40-7.32 (2H, m), 7.12 (1H, m). |
| 3-4 | 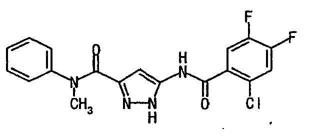 | ¹H-NMR(400 MHz, DMSO-*d*₆) δ: 13.20 (1H, s), 10.91 (1H. s), 7.81-7.67 (2H, m), 7.51-7.43 (3H, m), 7.40-7.36 (2H, m), 5.48 (1H, s), 3.32 (3H, s). |
| 3-5 | 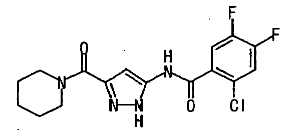 | ¹H-NMR(400 MHz, DMSO-*d*₆) δ: 13.14 (1H, s), 11.17 (1H, s), 7.87-7.79 (2H, m), 6.81 (1H, s), 3.64-3.57 (4H, m), 1.68-1.52 (6H, m) |
| 3-6 | 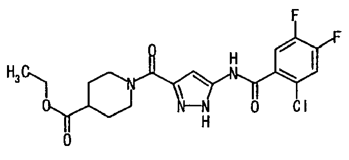 | ¹H-NMR(400 MHz, DMSO-*d*₆) δ:13.16 (1H, s), 11.18 (1H, s), 7.86-7.78 (2H; m), 6.83 (1H, d, J=1.6 Hz), 4.31 (1H, m), 4.09 (2H, q, J = 6.8 Hz), 3.10-2.90 (2H, m), 2.70-2.65 (2H, m), 1.94-1.91 (2H, m), 1.58-1.52 (2H, m), 1.19 (3H, t, J =6.8 Hz). |
| 3-7 | 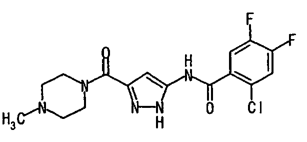 | ¹H-NMR(400 MHz, DMSO-*d*₆) δ: 13.17 (1H, br s), 11.19 (1H, br s), 7.86-7.83 (2H, m),6.83(1H, br s), 3.70-3.65 (4H, m), 2.36-2.34 (4H, m), 2.20 (3H, s). |
| 3-8 | 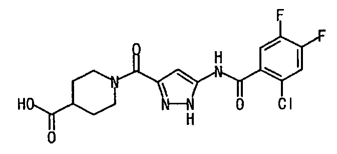 | ¹H-NMR(400 MHz, DMSO-*d*₆) δ: 11.21 (1H, br s), 7.87-7.80 (2H, m), 6.76 (1H, br s), 4,36-4.05 (2H, m), 3.32-2.98 (2H, m), 2.57 (1H, m), 1.93-1.89 (2H, m), 1.55-1.48 (2H, m). |

**Table 32**

| **Example** | **Molecular Structure** | **NMR** |
|---|---|---|
| 3-9 | 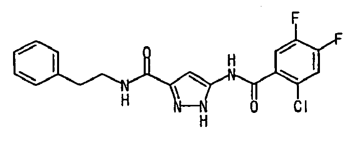 | ¹H-NMR(400 MHz, DMSO-*d*₆) δ: 13.17 (1H, s), 11.13 (1H, s). 8.67 (1H, m), 7.86-7.79 (2H, m), 7.36-7.18 (6H, m), 3.47 (2H, m), 2.85 (2H, t, J = 7.2 Hz). |
| 3-10 | 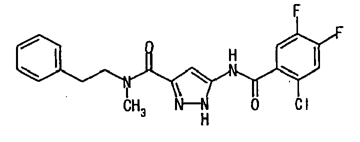 | ¹H-NMR(400 MHz, DMSO-*d*₆) δ: 13.17 (1H, m), 11.17 (1H. br s), 7.92-7.84 (2H, m), 7.29-7.23 (5H, m), 6.93 (1H, m). 3.92 (1H, m), 3.69 (2H, m), 3.25-2.89 (4H, m). |
| 3-11 | 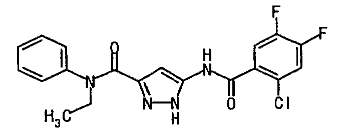 | ¹H-NMR(400 MHz, DMSO-d₆) δ: 13.18 (1H, s), 10.89 (1H, s). 7.77 (1H, dd, J = 10.4, 7.2 Hz), 7.69 (1H, dd, J = 10.8, 8.4 Hz), 7.52-7.44 (3H, m), 7.34-7.32 (2H, m), 5.40 (1H, br s), 3.81 (2H, q, J = 7.2 Hz), 1.12 (3H, t, J = 7.2 Hz). |
| 3-12 | 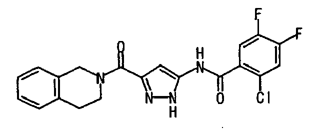 | ¹H-NMR(400 MHz, DMSO-*d*₆) δ:13.21 (1H, s), 11.20 (1H, s), 7.87-7.80 (2H, m), 7.26-7.15 (4H, m), 6.97 (1H, s), 4.77 (2H, m), 3.88 (2H, m), 2.93 (2H, m). |
| 3-13 | 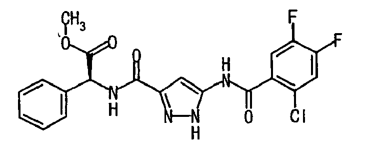 | ¹H-NMR(400 MHz, DMSO-*d*₆) δ: 13.28 (1H, s), 11.15 (1 H, s), 9.40 (1H, d, J = 7.2 Hz), 7.86-7.79 (2H, m), 7.52-7.35 (6H, m), 5.67 (1H, d, J = 7.2 Hz), 3.67 (3H, s). |
| 3-14 | 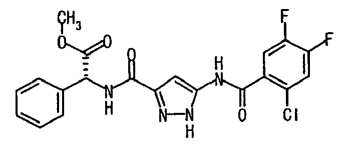 | ¹H-NMR(400 MHz, DMSO-*d*₆) δ: 13.28 (1H, s), 11.15 (1H, s), 9.40 (1H, d, J = 7.2 Hz), 7.86-7.79 (2H, m), 7.52-7.37 (6H, m), 5.67 (1H, d, J = 7.2 Hz), 3.67 (3H, s). |
| 3-15 | 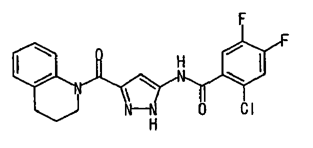 | ¹H-NMR(400 MHz, DMSO-*d*₆) δ: 13.25 (1H, br s), 11.09 (1H, s), 7.83-7.74 (2H, m), 7.25 (1H, d, J = 6.4 Hz), 7.18-7.04 (3H, m), 6.45 (1H, s), 3.85 (2H, t, J = 6.4 Hz), 2.80 (2H, t, J = 6.4 Hz), 1.95 (2H, m). |

**Table 33**

| **Example** | **Molecular Structure** | **NMR** |
|---|---|---|
| 3-16 | 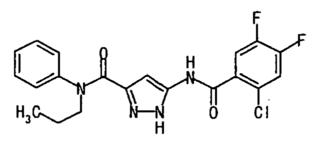 | ¹H-NMR(400 MHz, DMSO-*d*₆) δ:13.16 (1H, s), 10.88 (1H, s), 7.77 (1H, dd, J = 10.4, 7.2 Hz), 7.69 (1H, dd, J = 10.0, 8.4 Hz), 7.51-7.43 (3H, m), 7.34-7.32 (2H, m), 5.39 (1 H, br s), 3.75 (2H, t, J = 7.2 Hz), 1.53 (2H, m), 0.89 (3H, J = 7.2 Hz). |
| 3-17 | 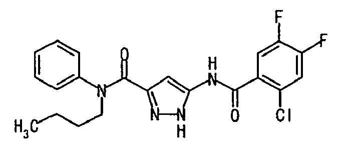 | ¹H-NMR(400 MHz, DMSO-*d*₆ δ: 13.16 (1H, s), 10.88 (1H, s), 7.77 (1H, dd, J = 10.4, 7.2), 7.69 (1H, dd, J = 10.0, 8.4), 7.51-7.43 (3H, m), 7.33-7.31 (2H, m), 5.39 (1H, br s), 3.79 (2H, t, J = 7.2 Hz), 1.50 (2H, m), 1.34 (2H, m), 0.88 (3H, t, J = 7.2 Hz). |
| 3-18 | 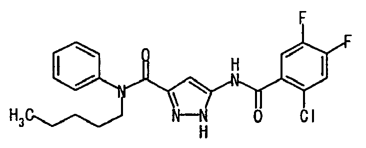 | ¹H-NMR(400 MHz, DMSO-*d*₆ δ: 13.16 (1 H, br s), 10.88 (1H, s), 7.77 (1H, m), 7.69 (1H, m), 7.51-7.45 (3H, m), 7.33-7.31 (2H, m), 5.38 (1 H, br s), 3.78 (2H, t, J = 7.6 Hz), 1.56-1.47 (2H, m), 1.34-1.28 (4H, m), 0.85 (3H, m). |
| 3-19 | 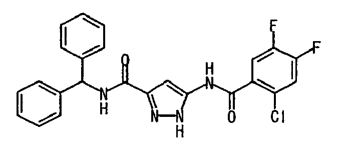 | ¹H-NMR(400 MHz, DMSO-*d*₆) δ: 13.23 (1H, s), 11.14 (1 H, s), 9.43 (1 H, d, J = 8.8 Hz), 7.85-7.78 (2H, m), 7.55 (1H, m), 7.39-7.35 (8H, m), 7.31-7.26 (2H, m), 6.39 (1 H, d, J = 8.8 Hz). |
| 3-20 | 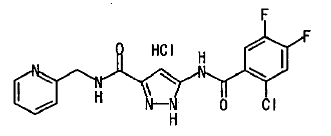 | ¹H-NMR(400 MHz, DMSO-*d*₆) δ: 11.25 (1H, s), 9.41 (1 H, br s), 8.79 (1 H, m), 8.40 (1H, m), 7.88-7.79 (4H, m), 7.29 (1H, s), 4.73 (2H, m). |
| 3-21 | 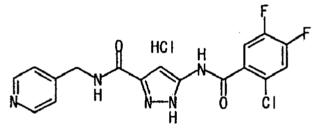 | ¹H-NMR(400 MHz, DMSO-*d*₆) δ: 11.25 (1H, s), 9.45 (1 H, br s), 8.85 (2H, d, J = 9.6 Hz), 7.92 (2H, d, J = 9.6 Hz), 7.88-7.80 (2H, m), 7.27 (1 H, s), 4.79 (2H, m). |
| 3-22 | 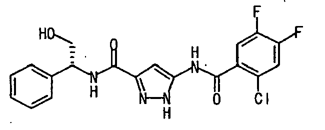 | ¹H-NMR(400 MHz, DMSO-d₆) δ: 13.15 (1H, m), 11.14 (1H, s), 8.85 (1H, d, J = 8.0 Hz), 7.86-7.79 (2H, m), 7.48 (1H, m), 7.39-7.31 (4H, m), 7.24 (1H, m), 5.04 (1H, m), 4.95 (1 H, t, J = 5.6 Hz), 3.74-3.62 (2H, m). |
| 3-23 | 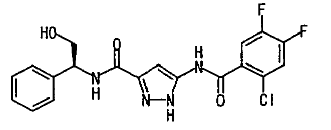 | ¹H-NMR(400 MHz, DMSO-*d*₆) δ: 13.16(1H, m), 11.14 (1H, s), 8.85 (1H, d, J = 8.0 Hz), 7.86-7.79 (2H, m), 7.48 (1H, m), 7.39-7.31 (4H, m), 7.24 (1H, m), 5.05 (1H, m), 4.95 (1H, t, J = 5.6 Hz), 3.74-3.62 (2H, m). |

**Table 34**

| **Example** | **Molecular Structure** | **NMR** |
|---|---|---|
| 3-24 | 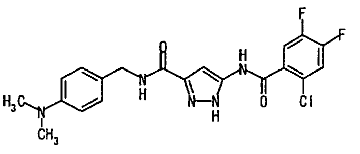 | ¹H-NMR(400 MHz, DMSO-*d*₆) δ: 13.18 (1H, s), 11.11 (1H, s), 9.14 (1H, t, J = 6.0 Hz), 7.85-7.78 (2H, m), 7.31 (1H, d, J = 1.6 Hz), 7.14 (2H, d, J = 8.4 Hz), 6.69 (2H, d, J = 8.4 Hz), 4.31 (2H, d, J = 6.0 Hz), 2.86 (6H, s). |
| 3-25 | 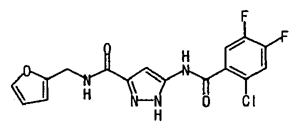 | ¹H-NMR(400 MHz, DMSO-*d*₆) δ: 13.24 (1H, s), 11.15 (1H, s), 9.08 (1H, t, J = 5.6 Hz), 7.86-7.79 (2H, m), 7.58 (1H, m), 7.34 (1H, d, J = 2.8 Hz), 6.41 (1H, m), 6.29 (1H, d, J = 2.8 Hz), 4.43 (2H, d, J = 5.6 Hz). |
| 3-26 | 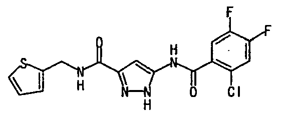 | ¹H-NMR(400 MHz, DMSO-*d*₆) δ: 13.26 (1H, s), 11.14 (1H, s), 9.24 (1H, br s), 7.77-7.75 (2H, m), 7.40 (1H, m), 7.33(1H, m), 7.02 (1H, m), 6.97 (1H, m),4.60 (2H, m). |
| 3-27 | 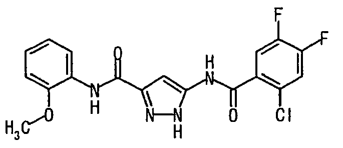 | ¹H-NMR(400 MHz, DMSO-*d*₆) δ: 13.38 (1H, br s), 11.56 (0.45H, br s), 11.18 (0.55 br s), 9.63 (0.55H, br s), 9.35 (0.45H, br s), 8.34 (0.45H, m), 7.94-7.83 (1.55H, m), 7.63 (0.45H, m), 7.48 (0.55H, m), 7.21-7.10 (2.55H, m), 6.98 (1H, m), 6.54 (0.45H, m), 3.92 (1.35H, br s), 3.85 (1.65H,br s). |
| 3-28 | 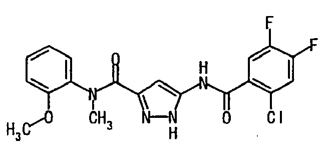 | ¹H-NMR(400 MHz, DMSO-*d*₆) δ: 13.13 (1H, s), 10.86 (1 H, s), 7.78 (1H, dd, J = 10.0,6.8 Hz), 7.70 (1H, m), 7.44 (1H. m), 7.33 (1H. dd, J = 7.6. 1.2 Hz), 7.18 (1H, d, J = 8.4 Hz), 7.05 (1 H, m), 5.51 (1H, br s), 3.74 (3H, s), 3.23 (3H, s). |
| 3-29 | 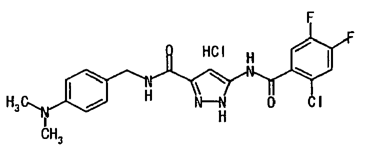 | ¹H-NMR(400 MHz, DMSO-*d*₆) δ: 11.16 (1H, s), 9.14 (1H. m), 7.86-7.81 (2H, m), 7.55-7.41 (4H, m). 7.25 (1H, s), 4.43 (2H, m). 3.05 (6H. m). |
| 3-30 | 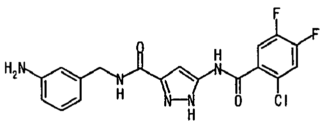 | ¹H-NMR (400 MHz, DMSO-*d*₆) δ: 13.21 (1H, br s), 11.12(1H. br s), 9.04 (1H, br s), 7.76-7.90 (2H, m), 7.35 (1H, br s), 6.97 (1H, m), 6.52-6.46 (3H, m), 5.23 (2H, br s), 4.61 (2H, m). |
| 3-31 | 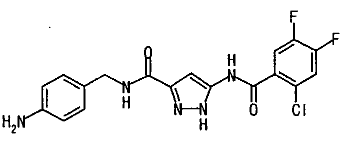 | ¹H-NMR(400 MHz, DMSO-*d*₆) δ: 13.19 (1H, s), 11.11 (1H, s), 8.96 (1H, br s), 7.85-7.78 (2H, m), 7.32 (1H, br s), 6.99 (2H, d, J = 7.6 Hz), 6.55 (2H, d, J = 7.6 Hz), 5.28 (2H, br s), 4.27 (2H, d, J = 5.6 Hz). |

**Table 35**

| **Example** | **Molecular Structure** | **NMR** |
|---|---|---|
| 3-32 | 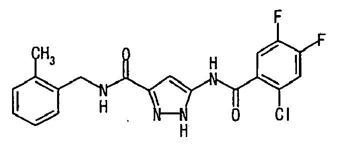 | ¹H-NMR(400 MHz, DMSO-*d*₆) δ: 13.22 (1 H, br s), 11.14(1H, br s), 9.00 (1H, br s), 7.86-7.79 (2H, m), 7.38 (1H, br s), 7.25 (1H, m), 7.18-7.13 (3H, m), 4.43 (2H, d, J = 6.0 Hz), 2.32 (3H, s). |
| 3-33 | 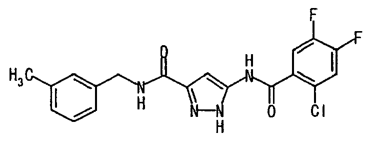 | ¹H-NMR(400 MHz, DMSO-*d*₆) δ: 13.22 (1H, br s), 11.14(1H, br s), 9.10 (1H, br s), 7.86-7.79 (2H, m), 7.35 (1H, br s), 7.22 (1H, m), 7.13-7.06 (3H, m), 4.41 (2H, d, J = 6.0 Hz), 2.29 (3H, s). |
| 3-34 | 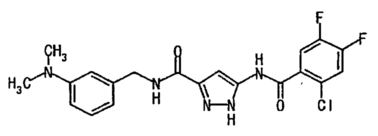 | ¹H-NMR(400 MHz, DMSO-*d*₆) δ: 13.21 (1H, s), 11.13 3 (1H, s), 9.07 (1H, m), 7.85-7.79 (2H, m), 7.35 (1H, s), 7.13 (1H, m), 6.70 (1H, m), 6.62-6.60 (2H, m), 4.38 (2H, d, J = 5.6 Hz), 2.88 (6H, s). |
| 3-35 | 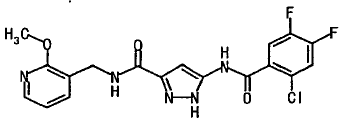 | ¹H-NMR(400 MHz, DMSO-*d*₆) δ: 13.25 (1H, s), 11.16 (1 H, br s), 9.02 (1 H, br s), 8.07 (1H, d, J = 5.2 Hz), 7.86-7.80 (2H, m), 7.55 (1H, d, J = 7.2 Hz), 7.38 (1H, br s), 6.97 (1H, dd, J = 7.2, 5.2 Hz), 4.38 (2H, d, J = 5.6 Hz), 3.92 (3H, s). |
| 3-36 | 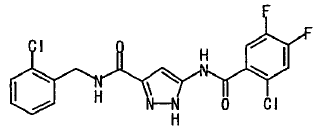 | ¹H-NMR(400 MHz, DMSO-*d*₆) δ: 13.27 (1H, s), 11.16 (1H, br s), 9.13 (1H, br s), 7.86-7.80 (2H, m), 7.53-7.29 (5H, m), 4.52 (2H, d, J=5.6 Hz). |
| 3-37 | 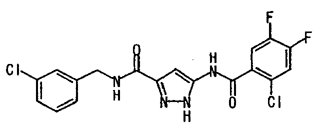 | ¹H-NMR(400 MHz, DMSO-*d*₆) δ: 13.26 (1H, s), 11.16 (1 H, br s), 9.17 (1 H, br s), 7.86-7.79 (2H, m), 7.40-7.28 (5H, m), 4.45 (2H, d, J = 6.0 Hz). |
| 3-38 | 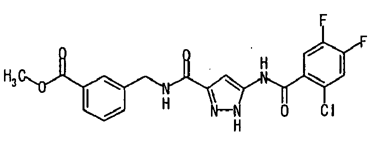 | ¹H-NMR(300 MHz, DMSO-*d*₆) δ: 13.24 (1H, s), 11.14 (1H, s), 9.19 (1H, m), 7.94 (1H, s), 7.86-7.69 (3H, m), 7.60 (1H, m), 7.50 (1H, m), 7.28 (1H, m), 4,50 (2H, d, J = 5.7 Hz), 3.85 (3H, s). |
| 3-39 | 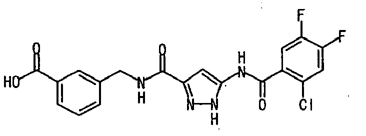 | ¹H-NMR(400 MHz, DMSO-*d*₆) δ:13.09 (2H, br s), 11.18 (1H, s), 9.18 (1H, br s), 7.92 (1H, s), 7.86-7.80 (3H, m), 7.56 (1H, d, J = 8.0 Hz), 7.47 (1H, m), 7.31 (1 H, br s), 4.50 (2H, d, J = 6.0 Hz). |

**Table 36**

| **Example** | **Molecular Structure** | **NMR** |
|---|---|---|
| 3-40 | 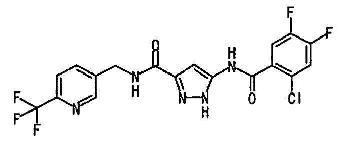 | ¹H-NMR(300 MHz, DMSO-*d*₆) δ: 13.29 (1H, s), 11.17 (1H, s), 9.26 (1 H, s), 8.74 (1 H, s), 8.00 (1 H, d, J = 7.4 Hz), 7.90 (1 H, d, J = 7.9 Hz), 7.83 (2H, q, J = 7.9 Hz), 7.35 (1H, s), 4.57 (2H, d, J = 5.6 Hz). |
| 3-41 | 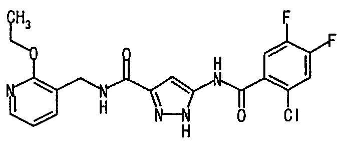 | ¹H-NMR(400 MHz, DMSO-*d*₆) δ: 13.25 (1H, br s), 11.16 (1H, br s), 9.00 (1H, br s), 8.05 (1H, dd, J = 5.2, 2.0 Hz), 7.86-7.80 (2H, m), 7.53 (1H, d, J = 6.8 Hz), 7.38 (1H, br s), 6.95 (1H, dd, J = 6.8,5.2 Hz), 4.40-4.34 (4H, m), 1.34 (3H, t, J = 7.2 Hz). |
| 3-42 | 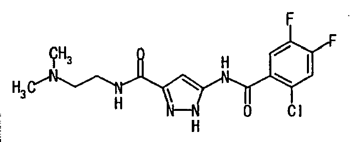 | ¹H-NMR(400 MHz, DMSO-*d*₆) δ: 13.16 (1H, br s), 11.15(1H, br s), 8.44 (1H, br s), 7.86-7.80 (2H,m), 7.23 (1H, br s),3.33 (2H, t, J = 6.8 Hz), 2.39 (2H, t, J = 6.8 Hz), 2.17 (6H, s). |
| 3-43 | 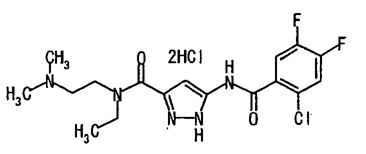 | ¹H-NMR(400 MHz, DMSO-*d*₆) δ:13.24 (1H, br s), 11.22 (1H, br s), 10.24 (2H, br s), 7.89-7.79 (2H, m), 6.94 (1H, br s),3.80-3.33 (6H, m), 2.83 (6H, m), 1.20 (3H,m). |
| 3-44 | 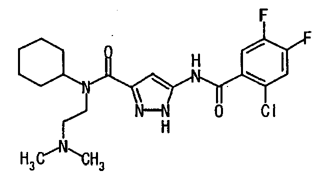 | ¹H-NMR(400 MHz, DMSO-d₆) δ:13.15 (1H, br s), 11.17 (1H, br s), 7.86-7.81 (2H, m),6.81 (1H, br s), 4.04 (1H. m), 3.55-3.48 (2H, m), 2.42 (2H, t, J = 7.2 Hz), 2.20 (6H, s), 1.81-1.12 (10H, m). |
| 3-45 | 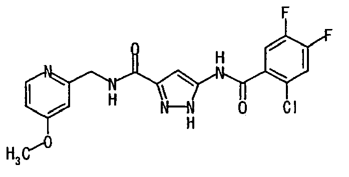 | ¹H-NMR(400 MHz, DMSO-*d*₆) δ:13.26 (1H, s), 11.16 (1H, s), 9.16 (1H, t, J=6.0 Hz), 8.34 (1H, d, J=6.4 Hz), 7.86-7.80 (2H, m), 7.38 (1H,s), 6.88 (1H, s), 6.87 (1H, d, J=6.4 Hz), 4.48 (2H,d,J = 6.0 Hz), 3.81 (3H, s). |
| 3-46 | 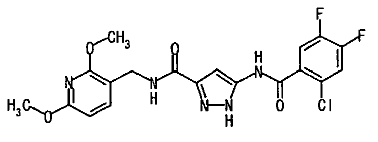 | ¹H-NMR(400 MHz, DMSO-*d*₆) δ:13.21 (1H, br s), 11.15 (1H, br s), 8.91 (1H, br s), 7.86-7.80 (2H, m), 7.52 (1H, d, J = 8.4 Hz), 7.35 (1H, br s), 6.36 (1H, d, J = 8.4 Hz), 4.30 (2H, d, J = 5.6 Hz), 3.92 (3H, s), 3.85 (3H, s). |

**Table 37**

| **Example** | **Molecular Structure** | **NMR** |
|---|---|---|
| 3-47 | 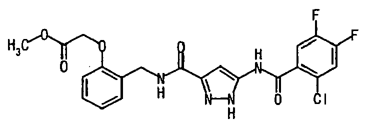 | ¹H-NMR(400 MHz, DMSO-*d*₆) δ: 13.25 (1H, s) 11.15 (1H, s), 8.95 (1H, m), 7.86-7.80 (2H, m), 7.39 (1H, br s), 7.24-7.20 (2H, m), 6.92-6.98 (2H, m), 4.89 (2H, s), 4.49 (2H, d, J = 6.0 Hz), 3.72 (3H, s). |
| 3-48 | 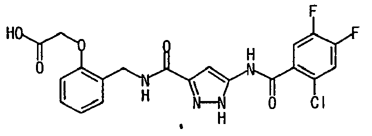 | ¹H-NMR(400 MHz, DMSO-*d*₆) δ: 13.15 (2H, br s) 11.17(1H, s), 8.93 (1H, brs). 7.87-7.80 (2H, m), 7.36 (1H, br s), 7.24-7.17 (2H, m), 6.96-6.88 (2H, m), 4.76 (2H, s), 4.48 (2H, d, J = 6.0 Hz). |
| 3-49 | 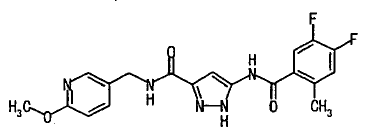 | ¹H-NMR(400 MHz, DMSO-*d*6) δ: 13.19 (1H, s), 10.94 (1H. s), 9.07 (1H. t, J = 5.6 Hz), 8.12 (1H. d, J = 2.0 Hz), 7.66 (1H. dd, J = 8.8, 2.0 Hz), 7.56 (1H, dd, J = 10.4,8.8 Hz), 7.38 (1H, dd, J = 11.6, 7.6 Hz), 7.32 (1H, s), 6.80 (1 H, d, J = 8.8 Hz), 4.37 (2H, d, J = 5.6 Hz), 3.83 (3H, s), 2.34 (3H, s). |
| 3-50 | 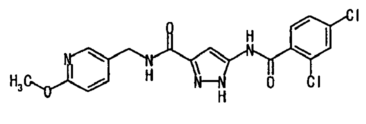 | ¹H-NMR(400 MHz, DMSO-*d*₆) δ: 13.23 (1H, s), 11.12 (1H, s), 9.09 (1H, t, J = 5.6 Hz). 8.12 (1H, d, J = 2.4 Hz), 7.72 (1 H, d, J = 2.4 Hz), 7.66 (1H, dd, J = 8.4, 2.4 Hz), 7.57 (1H. d, J = 8.4 Hz), 7.51 (1H. dd, J = 8.4, 2.4 Hz), 7.32 (1H, m). 6.80 (1H. d, J = 8.4 Hz). 4.37 (2H, d, J = 5.6 Hz). 3.83 (3H, s). |
| 3-51 | 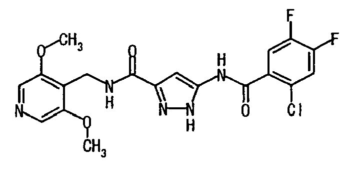 | ¹H-NMR(400 MHz, DMSO-*d*₆) δ: 13.16 (1H, s), 11.19 9 (1H, s), 8.51 (1H, m), 8.11 (2H, s), 7.89-7.77 (2H, m), 7.30 (1H, br s), 4.41 (2H, m), 3.90 (6H, s). |
| 3-52 | 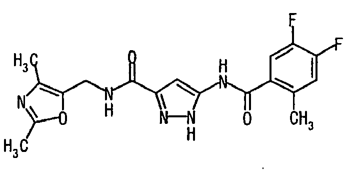 | ¹H-NMR(400 MHz, DMSO-*d*₆) δ: 13.22 (1H, s), 10.95 (1H, s), 9.04 (1H, t, J = 5.6 Hz), 7.57 (1H, m), 7.39 (1H. m), 7.33 (1H, s), 4.39 (2H, d, J = 5.6 Hz), 2.39 (3H, s), 2.32 (3H, s), 2.08 (3H, s). |
| 3-53 | 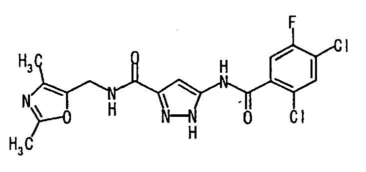 | ¹H-NMR(400 MHz, DMSO-*d*₆) δ: 13.28 (1H, s), 11.19 (1H. s), 9.06 (1H. t, J = 5.6 Hz), 7.93 (1H, d, J = 6.4 Hz), 7.77 (1H, d, J = 8.8 Hz), 7.33 (1H, s), 4.39 (2H, d, J = 5.6 Hz), 2.32 (3H, s), 2.07 (3H, s). |

The following compounds were prepared in accordance with the above-described Preparation Method B.

### [Example 4-1]

### Preparation of methyl 5-(2-chloro-4,5-difluorobenzoylamino)-1H-pyrazole-3-carbocylic acid (4-methoxycarbonyl-phenyl)-methyl-amide;

### Step 1: Preparation of methyl 4-[methyl-(5-nitro-1H-pyrazole-3-carbonyl)-amino]-benzoate;

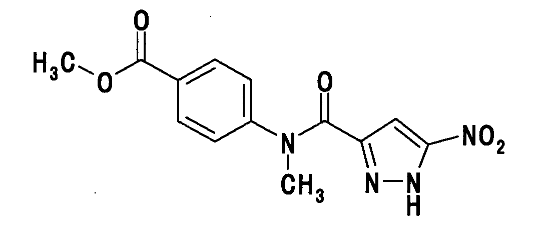

To a solution of 5-nitro-3-pyrazolecarboxylic acid (400 mg) in chloroform (10 mL) were added oxalyl chloride (0.27 mL) andN,N-dimethylformamide (1 drop) with ice-bath cooling. After stirring overnight at room temperature, the resulting reaction mixture was concentrated under reduced pressure to obtain 5-nitro-3-pyrazolecarbonyl chloride.

Then, to a suspension of 5-nitro-3-pyrazolecarbonyl chloride and methyl 4-methylaminobenzoate (410 mg) in chloroform (25 mL) was added triethylamine (1.2 mL), and the resulting mixture was stirred overnight at room temperature. The resulting reaction mixture was successively washed with saturated aqueous sodium bicarbonate, 1 N hydrochloric acid and brine, dried over anhydrous sodium sulfate, and then concentrated under reduced pressure. The residue was purified by silica gel column chromatography (chloroform-ethyl acetate, 5:1 → 3:1 → 2:1) to obtain the title compound (394 mg) as white crystals.

### Step 2: Preparation of methyl 4-[(5-amino-1H-pyrazole-3-carbonyl)-methyl-amino]-benzoate;

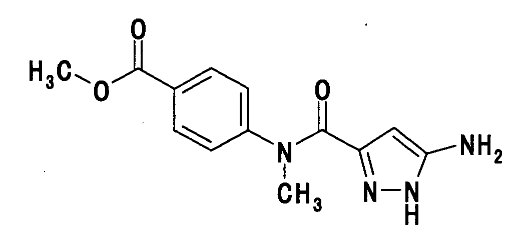

To a solution of methyl 4-[methyl-(5-nitro-1H-pyrazole-3-carbonyl)-amino]-benzoat e (394 mg) in tetrahydrofuran (10 mL) -ethyl acetate (10 mL) was added 7.5% palladium-carbon (60 mg). The resulting mixture was stirred overnight at room temperature under hydrogen atmosphere. The catalyst was removed by filtration, and the filtrate was concentrated under reduced pressure to obtain the title compound (244 mg) as a white solid.

### Step 3: Preparation of methyl 5-(2-chloro-4,5-difluoro-benzoylamino)-1H-pyrazole-3-carboxylic acid (4-methoxycarbonyl-phenyl)-methyl-amide;

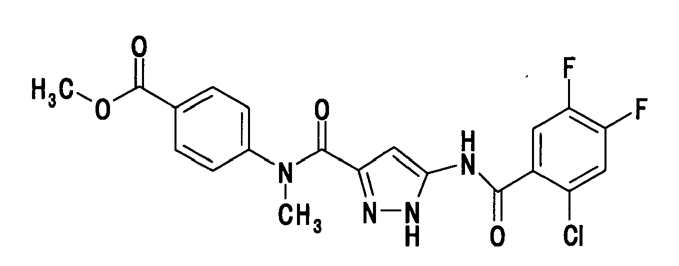

To a solution of methyl 4-[(5-amino-1H-pyrazole-3-carbonyl)-methyl-amino]-benzoat e (244 mg) in tetrahydrofuran (5 mL) was added pyridine (0.1 mL), and 2-chloro-4,5-difluorobenzoyl chloride (0.135 mL) obtained in Step 1 of Reference Example 1 was added dropwise thereto with ice-bath cooling. After stirring overnight at room temperature, the reaction mixture was concentrated under reduced pressure, and the resulting residue was purified using silica gel column chromatography (chloroform-ethyl acetate, 5:1 □ 1:1) to obtain the title compound (228 mg) as white crystals.

### [Example 4-2]

### Preparation of 5-(2-chloro-4,5-difluoro-benzoylamino)-1H-pyrazole-3-carboxylic acid (4-carboxyphenyl)-methyl-amide;

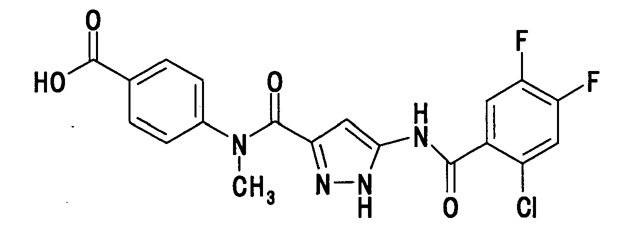

To a solution of methyl 5-(2-chloro-4,5-difluorobenzoylamino)-1H-pyrazole-3-carbo xylic acid (4-methoxycarbonyl-phenyl)-methyl-amide (100 mg) prepared according to Example 4-1 in methanol (2 mL) was added 1 N aqueous sodium hydroxide (0.7 mL), and this solution was stirred overnight at room temperature. To the resulting reaction mixture was added 1 N hydrochloric acid (1.5 mL), and the formed solid was filtered to obtain the title compound (78 mg) as white solids.

### [Example 4-2-1]

### Preparation of 4-{[5-(2-chloro-4,5-difluoro-benzoylamino)-1H-pyrazole-3-carbonyl]-methyl-amino}-benzoic acid sodium salt;

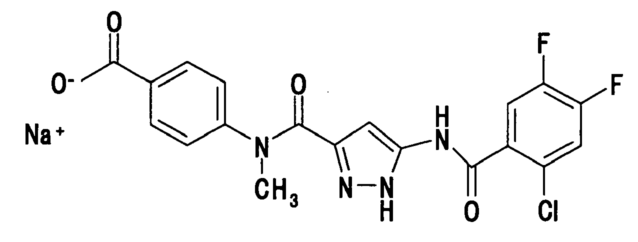

To a suspension of 5-(2-chloro-4,5-difluoro-benzoylamino)-1H-pyrazole-3-carb oxylic acid (4-carboxyphenyl)-methyl-amino (49.4 mg) prepared according to Example 4-2 in ethanol (0.81 mL) was added 4 N aqueous sodium hydroxide solution (0.025 ml). This solution was allowed to stand at room temperature, and the solids which formed were filtered to obtain the title compound (36 mg).

The compounds Example 4-3 to Example 4-48 illustrated in the following tables were produced in the same manner as in the above-described Example 4-1 and Example 4-2-1.

**Table 38**

| **Example** | **Molecular Structure** | **NMR** |
|---|---|---|
| 4-1 | 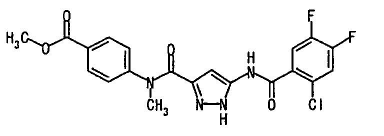 | ¹H-NMR(400 MHz, DMSO-*d*₆) δ: 13.28 (1H, s), 10.96 (1H, s), 8.03 (2H, d, J = 8.4 Hz), 7.80-7.69 (2H, m), 7.52 (2H, d, J = 8.4 Hz), 5.69 (1H, s), 3.87 (3H, s), 3.39 (3H, s). |
| 4-2 | 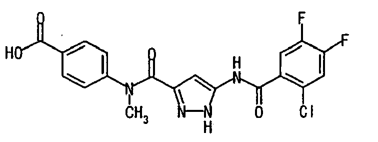 | ¹H-NMR(400 MHz, DMSO-*d*₆) δ: 13.17 (2H, br s), 10,98 (1H, s), 8.00 (2H, d, J = 8.4 Hz), 7.80-7.70 (2H, m), 7.47 (2H, d, J = 8.4 Hz), 5.70 (1H, s), 3.38 (3H, s). |
| 4-2-1 | 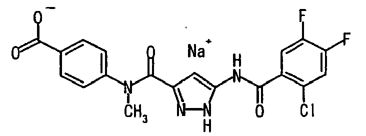 | NMR(σ 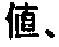 , 400MHz, DMSO-*d*₆) 3.30(3H,s),5.73(1H.s).7.15(2H,d.J=8.4Hz),7.67(2H,m).7.8 4(2H,d.J=8.4Hz). |
| 4-3 | 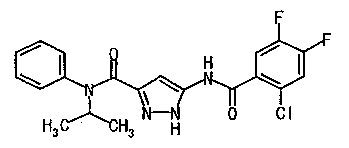 | ¹H-NMR(400 MHz, DMSO-*d*₆) δ: 13.11 (1H, s), 10.84 (1H, s), 7.77 (1H, m), 7.68 (1H, s), 7.52-7.50 (3H, m), 7.30-7.29 (2H, m), 5.19 (1H, br s), 4.97 (1H, m), 1.10 (6H, d, J = 6.4 Hz). |
| 4-4 | 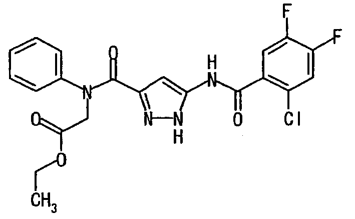 | ¹H-NMR(400 MHz, DMSO-*d*₆) δ: 13.32 (1H, s), 10.95 (1H, s), 7.78 (1H, dd, J = 10.0, 6.8 Hz), 7.71 (1H, m), 7.49-7.45 (3H, m), 7.42-7.39 (2H, m), 5.47 (1H. br s), 4.52 (2H, s), 4.15 (2H, q, J = 7.2 Hz), 1.21 (3H, t, J = 7.2 Hz). |
| 4-5 | 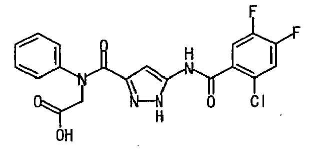 | ¹H-NMR(400 MHz, DMSO-*d*₆) δ: 13.27 (2H, br s), 10.97 (1H, br s), 7.80-7.69 (2H, m), 7.48-7.35 (5H, m), 5.51 (1H, br s), 4.39 (2H, s). |
| 4-6 | 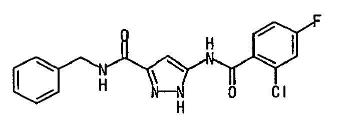 | ¹H-NMR(300 MHz, DMSO-*d*₆) δ: 13.18 (1H, br s), 11.05 (1 H, br s), 9.11 (1 H, br s), 7.62 (1 H, t, J=8.9 Hz), 7.52 (1H, d, J = 9.0 Hz), 7.39-7.24 (7H, m), 4.44 (2H, d, J = 6.6 Hz). |

**Table 39**

| **Example** | **Molecular Structure** | **NMR** |
|---|---|---|
| 4-7 | 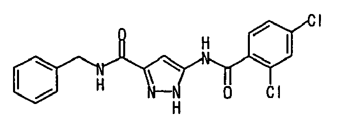 | ¹H-NMR(300 MHz, DMSO-d₆) δ: 13.19 (1H, br s), 11.09 (1H, br s), 9.11 (1H, br s), 7.72 (1H, br s), 7.58 (1H, d, J = 7.7 Hz), 7.51 (1H, d, J =7.7 Hz), 7.34-7.20 (6H, m), 4.44 (2H, d, J = 5.9 Hz). |
| 4-8 | 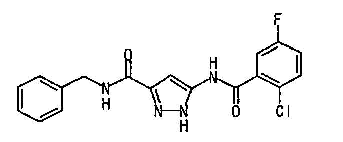 | ¹H-NMR(300 MHz, DMSO-*d*₆) δ: 13.20 (1H, br s), 11.11 (1H, br s), 9.12 (1H, t, J = 5.7 Hz), 7.58 (1 H, q, J = 4.5 Hz), 7.48 (1H, dd, J = 8.6, 3.1 Hz), 7.41-7.19 (7H, m), 4.45 (2H, d, J = 5.9 Hz). |
| 4-9 | 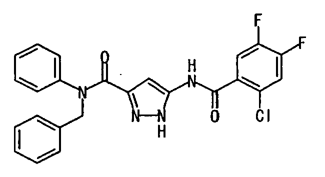 | ¹H-NMR(400 MHz, DMSO-*d*₆) δ: 13.35 (1H, br s), 10.93 (1H, s), 7.92-7.68 (2H, m), 7.42-7.17 (10H, m), 5.47 (1H, br s), 5.04 (2H, s). |
| 4-10 | 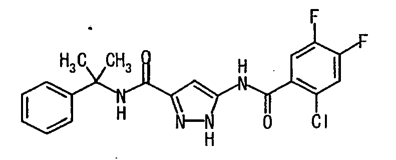 | ¹H-NMR(400 MHz, DMSO-*d*₆) δ: 13.00 (1H, br s), 11.11 (1 H, br s), 8.58 (1H, br s), 7.86-7.79 (2H, m), 7.47 (1H, br s), 7.39-7.37 (2H, m), 7.33-7.23 (2H, m), 7.14 (1H, t, J = 7.2 Hz), 1.68 (6H, s). |
| 4-11 | 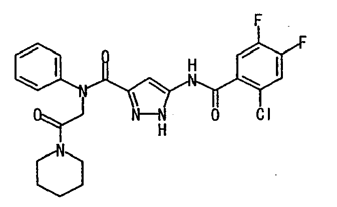 | ¹H-NMR(400 MHz, DMSO-*d*₆) δ: 13.30 (1H, br s), 10.92 (1H, br s), 7.78 (1H, m), 7.71,(1H, m), 7.47-7.39 (5H, m), 5.44 (1H, br s), 4.64 (2H, s), 3.45-3.39 (4H, m), 1.58-1.44 (6H, m). |
| 4-12 | 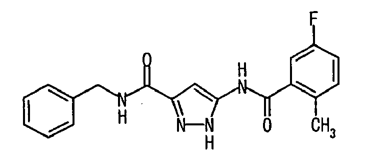 | ¹H-NMR(300 MHz, DMSO-*d*₆) δ: 13.16 (1H, br s), 10.89 (1H, br s), 9.10 (1H, br s), 7.39-7.17 (9H, m), 4.44 (2H, d, J = 5.5 Hz), 2.34 (3H, s). |

**Table 40**

| **Example** | **Molecular Structure** | **NMR** |
|---|---|---|
| 4-13 | 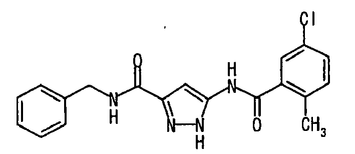 | ¹H-NMR(300 MHz, DMSO-*d*₆) δ: 13.16 (1H, br s), 10.93 (1H, br s), 9.10 (1H, br s), 7.55-7.18 (9H, m), 4.45 (2H, d, J = 5.9 Hz), 2.35 (3H, s). |
| 4-14 | 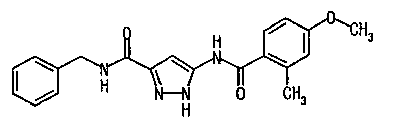 | ¹H-NMR(300 MHz, DMSO-d₆) δ: 13.09 (1H. br s), 10.63 (1H, br s), 9.07 (1H, br s), 7.52-7.18 (7H, m), 6.83 (2H, br s), 4.44 (2H, d, J = 5.9 Hz), 3.79 (3H, s), 2.39 (3H, s). |
| 4-15 | 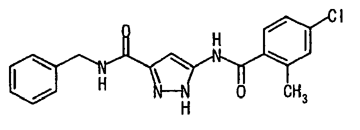 | ¹H-NMR(300 MHz, DMSO-*d*₆) δ: 13.16 (1H, br s), 10.88 (1H, br s), 9.10 (1H, br s), 7.59-7.16 (9H, m), 4.45 (2H, d, J = 6.2 Hz), 2.38 (3H, s). |
| 4-16 | 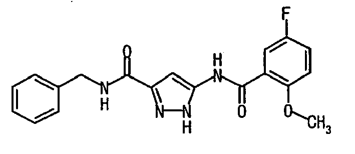 | ¹H-NMR(300 MHz, DMSO-*d*₆) δ: 13.22 (1H. br s), 10.47 (1H, br s), 9.11 (1H, br s), 7.53 (1H, d, J = 9.5 Hz), 7.40-7.20 (8H, m), 4.43 (2H, d, J = 6.2 Hz), 3.93 (3H, s). |
| 4-17 | 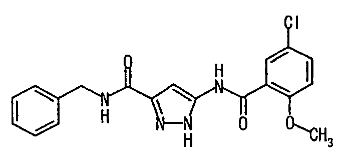 | ¹H-NMR(300 MHz, DMSO-*d*₆) δ: 13.22 (1H, br s), 10.49 (1H, br s), 9.12 (1H, t, J = 5.7 Hz), 7.69 (1 H, d, J = 2.6 Hz), 7.56 (1H, dd, J = 8.8, 2.9 Hz), 7.40-7.18 (7H, m), 4.45 (2H, d, J = 5.9 Hz), 3.93 (3H, s). |
| 4-18 | 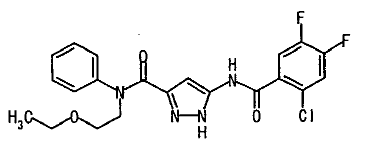 | ¹H-NMR(400 MHz, DMSO-*d*₆) δ: 13.19 (1H, d, J= 1.6 Hz), 10.88 (1H.s), 7.77 (1H**,** dd, J = 10.4, 7.2 Hz), 7.69 (1H, dd. J = 10.4, 8.4 Hz), 7.49-7.44 (3H, m), 7.36-7.34 (2H, m), 5.38 (1H, br s), 3.93 (2H, t, J = 6.0 Hz), 3.54 (2H, t, J = 6.0 Hz), 3.40 (2H, q, J = 6.8 Hz), 1.07 (3H, t, J = 6.8 Hz). |
| 4-19 | 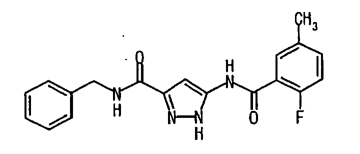 | ¹H-NMR(300 MHz, DMSO-*d*₆) δ: 13.19 (1H, br s), 10.82 (1H, br s), 9.13(1H, t, J = 4.5 Hz), 7.46 (1H, d.J=4.6 Hz), 7.40-7.14 (8H, m), 4.45 (2H, d, J = 5.9 Hz), 3.31 (3H, s). |

**Table 41**

| **Example** | **Molecular Structure** | **NMR** |
|---|---|---|
| 4-20 | 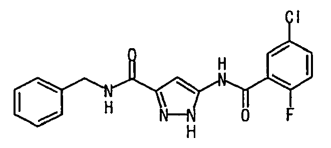 | ¹H-NMR(300 MHz, DMSO-d₆) δ: 13.24 (1H, br s), 11.07 (1H, br s), 9.13 (1H, t, J = 4.5 Hz), 7.70 (1H, t, J = 2.7 Hz), 7.60(1H, dd, J = 4.7, 2.7 Hz), 7.41-7.24 (7H, m), 4.46 (2H, d, J = 4.5 Hz). |
| 4-21 | 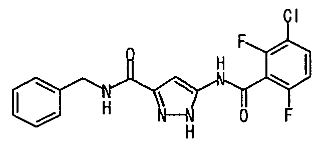 | ¹H-NMR(300 MHz, DMSO-*d*₆) δ: 13.29 (1 H, br s), 11.42 (1H, br s), 9.15 (1H, br s), 7.77 (1 H, br s), 7.34-7.24 (7H, m), 4.45 (2H, d, J = 6.2 Hz). |
| 4-22 | 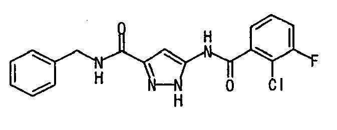 | ¹H-NMR(300 MHz, DMSO-d₆) δ: 13.24 (1H, br s), 11.15 (1H, br s), 9.14 (1H, br s), 7.57-7.19 (9H, m), 4.43 (2H, d, J = 5.5 Hz). |
| 4-23 | 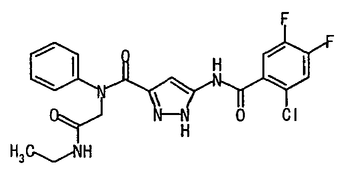 | ¹H-NMR(400 MHz, DMSO-*d*₆) δ:13.22 (1H. br s), 10.91 (1 H, s), 8.00 (1 H, t, J = 5.2 Hz). 7.78 (1H. dd, J = 10.0. 7.2 Hz). 7.70 (1H, dd. J = 10.8, 8.4 Hz), 7.50-7.42 (5H, m), 5.45 (1H, br s), 4.34 (2H, s), 3.10 (2H, qt, J = 7.2, 5.2 Hz), 1.01 (3H, t, J = 7.2 Hz). |
| 4-24 | 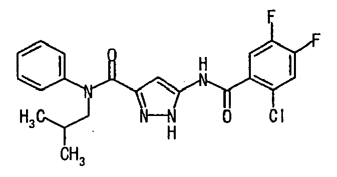 | ¹H-NMR(400 MHz, DMSO-d₆) δ:13.17 (1H, s), 10.87 (1H, s), 7.77 (1H, dd, J = 10.4, 7.2 Hz), 7.68 (1H, dd, J = 10.4, 8.4 Hz), 7.51-7.42 (3H, m), 7.35-7.34 (2H, m), 5.37 (1H, br s), 3.68 (2H, d, J = 7.6 Hz), 1.78 (1H, qt, J = 7.6, 7.6 Hz), 0.91 (6H, d, J = 7.6 Hz). |
| 4-25 | 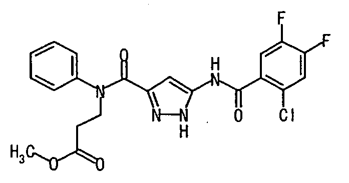 | ¹H-NMR(400 MHz, DMSO-*d*₆) δ: 13.22 (1H, s), 10.89 (1H, s), 7.77 (1H, dd, J = 10.0, 6.8 Hz), 7.69 (1H, dd, J = 10.0, 8.4 Hz), 7.52-7.44 (3H, m), 7.36-7.35 (2H, m), 5.38 (1H, s), 4.03 (2H, t, J = 7.2 Hz), 3.55 (3H, s), 2.62 (2H, t, J = 7.2 Hz). |

**Table 42**

| **Example** | **Molecular Structure** | **NMR** |
|---|---|---|
| 4-26 | 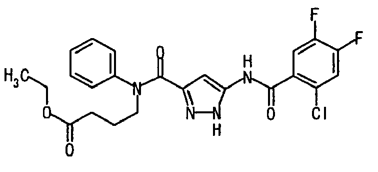 | ¹H-NMR(400 MHz, DMSO-*d*₆) δ: 13.17 (1H, s), 10.89 (1H, s), 7.77 (1H, dd, J = 10.0, 6.8 Hz), 7.69 (1H, dd, J = 10.4, 8.4 Hz), 7.51-7.43 (3H, m), 7.35-7.34 (2H, m), 5.40 (1H. s), 4.03 (2H,q, J = 7.2 Hz), 3.82 (2H, t, J = 7.2 Hz), 2.37 (2H, t, J = 7.2 Hz), 1.77 (2H, tt, J = 7.2, 7.2 Hz), 1.16 (3H, t, J = 7.2 Hz). |
| 4-27 | 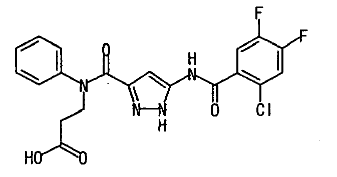 | ¹H-NMR(400 MHz, DMSO-d₆) δ: 13.19 (1H, br s), 12.34 (1H, br s), 10.90 (1H, s), 7.77 (1H, dd, J = 10.0, 7.2 Hz), 7.69 (1H, dd, J = 10.0, 8.4 Hz), 7.51-7.44 (3H, m), 7.37-7.35 (2H, m), 5.40 (1H, br s), 4.00 (2H, t, J = 7.2 Hz), 2.55 (2H, t, J = 7.2 Hz). |
| 4-28 | 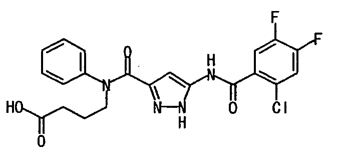 | ¹H-NMR(400 MHz, DMSO-d₆) δ: 13.17 (1H, br s), 12.08 (1H, br s), 10.88 (1H, s), 7.77 (1H, dd, J = 10.4, 7.2 Hz), 7.69 (1H, dd, J = 10.4, 8.8 Hz), 7.51-7.43 (3H, m), 7.35-7.33 (2H, m), 5.40 (1H, br s), 3.82 (2H,t , J = 7.2 Hz), 2.29 (2H, t, J = 7.2 Hz), 1.75 (2H, tt, J = 7.2, 7.2 Hz). |
| 4-29 | 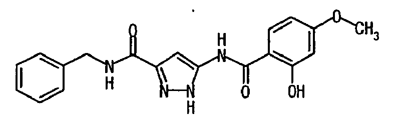 | ¹H-NMR(300 MHz, DMSO-*d*₆) δ: 12.51 (1H, br s), 8.42 (1H. t. J = 4.5 Hz), 8.05 (1H, s), 7.30-7.19 (5H, m), 6.17 (1H. s), 4.39 (2H, d, J = 4.8 Hz), 3.31 (3H, s). |
| 4-30 | 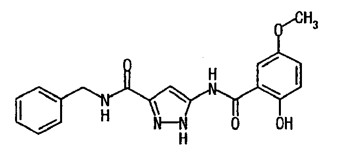 | ¹H-NMR(300 MHz, DMSO-*d*₆)) δ: 13.30 (1H, br s), 11.58 (1H, br s), 11.01 (1H, br s), 9.12 (1H, br s), 7.60 (1H, br s), 7.39-7.19 (6H, m), 7.05 (1H, q, J = 4.5 Hz), 6.93 (1H, d, J = 6.6 Hz),4.46 (2H, d, J = 6.2 Hz), 3.76 (3H, s). |
| 4-31 | 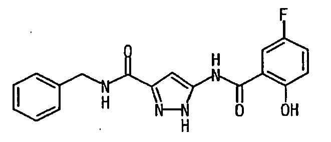 | ¹H-NMR(300 MHz, DMSO-*d*₆) δ: 13.29 (1H, br s), 11.84 (1H, br s), 10.92 (1H, br s), 9.12 (1H, br s), 7.81 (1H, d, J = 9.2 Hz), 7.39-7.19 (7H, m), 7.02 (1H, q, J = 4.5 Hz), 4.45 (2H, d, J = 6.2 Hz). |
| 4-32 | 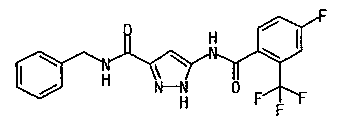 | ¹H-NMR(300 MHz, DMSO-*d*₆) δ: 13.20 (1H,s), 11.14 (1H, s), 9.13 (1H, d, J = 5.5 Hz), 7.79-7.70 (2H, m), 7.64 (1H, t, J = 6.2 Hz), 7.39-7.19 (6H, m), 4.44 (2H, d, J = 5.9 Hz). |

**Table 43**

| **Example** | **Molecular Structure** | **NMR** |
|---|---|---|
| 4-33 | 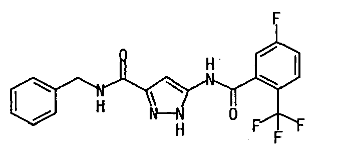 | ¹H-NMR (300 MHz, DMSO-*d*₆) δ: 13.20 (1 H, br s), 11.18 (1H, br s), 9.12 (1H, br s), 7.94-7.86 (1H, m), 7.66-7.47 (2H, m), 7.39-7.20 (6H, m), 4.44 (2H, d, J = 6.2 Hz). |
| 4-34 | 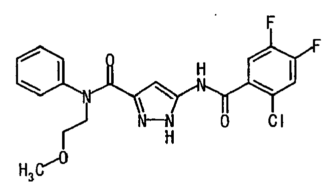 | ¹H-NMR(400 MHz, DMSO-*d*₆) δ: 13.20 (1H, s), 10.88 (1H, s), 7.77 (1H, dd, J = 10.4, 7.2 Hz), 7.69 (1H, dd, J = 10.4, 8.4 Hz), 7.52-7.45 (3H, m), 7.35-7.33 (2H, m), 5.38 (1 H, br s), 3.95 (2H, t, J = 5.6 Hz), 3.49 (2H, t, J = 5.6 Hz), 3.31 (3H, s). |
| 4-35 | 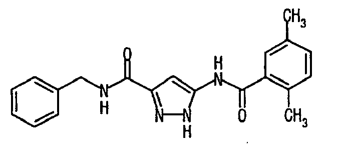 | ¹H-NMR(300 MHz, DMSO-*d*₆) δ: 13.11 (1H, br s), 10.72 (1H, br s), 9.12 (1H, br s), 7.40-7.10 (9H, m), 4.44 (2H, d, J = 6.2 Hz), 2.33 (3H, s), 2.31 (3H, s). |
| 4-36 | 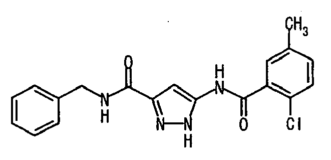 | ¹H-NMR(300 MHz, DMSO-*d*₆) δ: 7.40-7.15 (6H, m), 6.92(1H, br s), 5.99 (1H, s), 5.66 (1H, br s), 4.46 (2H, d, J = 6.6 Hz),1.55(3H, s). |
| 4-37 | 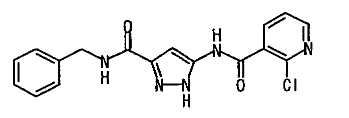 | ¹H-NMR(300 MHz, DMSO-*d*₆)) δ: 8.58 (1H, d, J = 1.8 Hz), 8.44 (1H, t, J = 5.9 Hz), 8.18 (1H, dd, J = 7.3, 1.8Hz), 7.59 (1H, dd, J = 7.7, 4.8 Hz), 7.32-7.18 (5H, m), 6.94(1H, br s), 5.75 (1H, br s), 4.46 (2H, d, J = 6.6 Hz). |
| 4-38 | 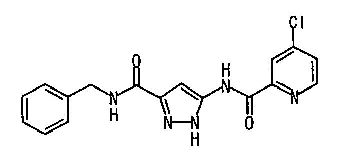 | ¹H-NMR(300 MHz, DMSO-*d*₆) δ: 13.32 (1H, br s), 10.58 (1H, br s), 9.15 (1H, br s), 8.72 (1H, br s), 8.14 (1H, s), 7.85 (1H, s), 7.42-7.18 (6H, m), 4.46 (2H, d, J = 6.6 Hz). |
| 4-39 | 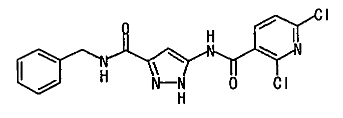 | ¹H-NMR(300 MHz, DMSO-d₆) δ:13.26 (1H, br s), 11.25 (1H, br s), 9.14 (1H, br s), 8.13 (1H, d, J = 7.7 Hz), 7.69 (1H. d, J = 8.1 Hz), 7.41-7.19 (6H, m), 4.45 (2H, d, J = 5.9 Hz). |

**Table 44**

| | | |
|---|---|---|
| 4-40 | 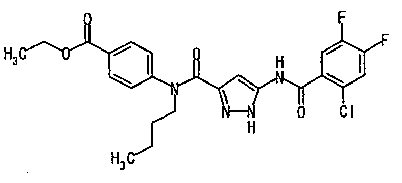 | ¹H-NMR(400 MHz, DMSO-*d*₆) δ: 13.26 (1H, m), 10.95 (1H, s), 8.04 (2H, d, J = 8.4 Hz), 7.78 (1H, d, J = 10.4, 7.2 Hz), 7.71 (1 H, dd, J = 9.6, 8.4 Hz), 7.48 (2H, d, J = 8.4 Hz), 5.57 (1H, br s), 4.32 (2H, q, J = 7.2 Hz), 3.84 (2H, t, J = 7.2 Hz), 1.52-1.45 (2H, m), 1.35-1.26 (2H, m), 1.32 (3H, t, J = 7.2 Hz), 0.87 (3H, t, J = 7.2 Hz). |
| 4-41 | 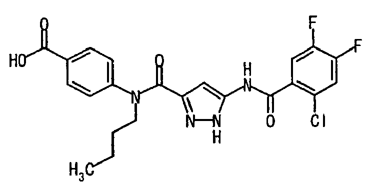 | ¹H-NMR(400 MHz, DMSO-*d*₆) δ: 13.17 (2H, br s), 10.95 (1H, s), 8.01 (2H, d, J = 8.0 Hz), 7.80-7.72 (2H, m), 7.44 (2H, d, J = 8.0 Hz), 5.57 (1H, s), 3.84 (2H, t, J = 7.2 Hz), 1.51-1.48 (2H, m), 1.34-1.30 (2H, m), 0.88 (3H, t, J = 7.2 Hz). |
| 4-42 | 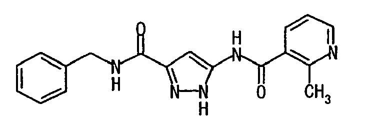 | ¹H-NMR(300 MHz, DMSO-*d*₆) δ: 13.19 (1H, br s), 11.01 (1H, br s), 9.12 (1H, br s), 8.52 (1H, br s), 7.82 (1H, br s), 7.41-7.22 (5H, m), 4.44 (2H, d, J = 6.6 Hz), 2.55 (3H, s). |
| 4-43 | 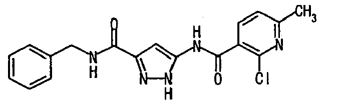 | ¹H-NMR (300 MHz, DMSO-*d*₆) δ: 13.22 (1H, br s), 11.12 (1H, br s), 9.13 (1H, t, J = 6.2 Hz), 7.89 (1H, d, J = 7.7 Hz), 7.41-7.20 (7H, m), 4.45 (2H, d, J = 5.9 Hz), 2.49 (3H, s). |
| 4-44 | 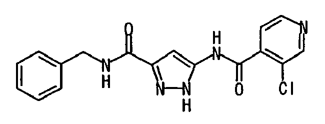 | ¹H-NMR(300 MHz, DMSO-*d*₆) δ: 13.28 (1H, br s), 11.31 (1H, br s), 9.16 (1H, br s),8.75 (1H, s), 8.62 (1H, br s), 7.61 (1H, br s), 7.41-7.22 (6H, m), 4.44 (2H, d, J = 6.6 Hz). |
| 4-45 | 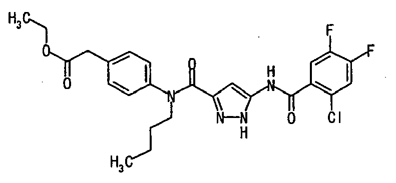 | ¹H-NMR(400 MHz, DMSO-*d*₆) δ: 13.16 (1H, s), 10.87 (1H, s), 7.78 (1H, dd, J = 10.4, 7.2 Hz), 7.69 (1 H, dd, J = 10.4, 8.8 Hz), 7.38 (2H, d, J = 8.4 Hz), 7.26 (2H, d, J = 8.4 Hz), 5.44 (1H, br s), 4.47 (2H, q, J = 7.2 Hz), 3.77 (2H, t, J = 7.2 Hz), 3.71 (2H, s), 1.52-1.46 (2H, m), 1.34-1.29 (2H, m), 1.15 (3H, t, J = 7.2 Hz), 0.88 (3H, t, J = 7.2 Hz). |
| 4-46 | 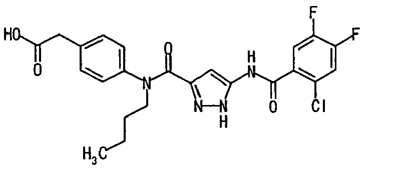 | ¹H-NMR(400 MHz, DMSO-*d*₆) δ: 13.11 (1H, br s), 12.49 (1H, br s), 10.89 (1H, s), 7.80-7.68 (2H, m), 7.37 (2H, d, J = 8.4 Hz), 7.25 (2H, d, J = 8.4 Hz), 5.50 (1H, br s), 3.78 (2H, t, J = 7.2 Hz), 3.62 (2H, s), 1.51-1.48 (2H, m), 1.34-1.30 (2H, m), 0.89 (3H, t, J = 7.2 Hz). |

**Table 45**

| **Example** | **Molecular Structure** | **NMR** |
|---|---|---|
| 4-47 | 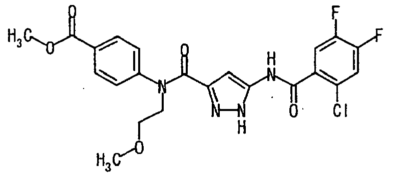 | ¹H-NMR(400 MHz, DMSO-d₆) δ: 13.30 (1H, m), 10.95 (1H, s) 8.04 (2H, d, J = 8.0 Hz), 7.78 (1H, dd, J = 10.4, 7.2 Hz), 7.71 (1H, dd, J = 10.4, 8.4 Hz), 7.49 (2H, d, J = 8.0 Hz), 5.55 (1 H, br s), 3.99 (2H, t, J = 5.6 Hz), 3.87 (3H, s), 3.50 (2H, t, J = 5.6 Hz), 3.32 (3H, s). |
| 4-48 | 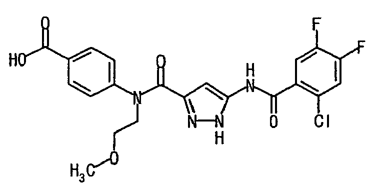 | ¹H-NMR(400 MHz, DMSO-*d*₆) δ: 13.18 (2H, br s), 10.97 (1H. s) 8.01 (2H,d, J = 8.4 Hz), 7.81-7.70 (2H, m), 7.45 (2H, d, J = 8.4 Hz), 5.56 (1 H, br s), 3.99 (2H, t, J = 5.6 Hz), 3.50 (2H, t, J = 5.6 Hz), 3.22 (3H, s). |

The compounds Example 5-1 to Example 5-44 illustrated in the following tables were prepared in the same manner as in the above-described Preparation Methods A, B, C or the above-described respective Examples.

**Table 46**

| **Example** | **Molecular Structure** | **NMR** |
|---|---|---|
| 5-1 | 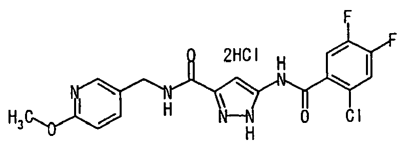 | ¹H-NMR(400 MHz, DMSO-*d*₆) δ:11.18 (1H, s), 9.11 (1H, m), 8.14 (1H, s), 7.86-7.80 (2H, m), 7.72 (1H, m), 7.23 (1H, s), 6.86 (1H, m), 4.38 (2H, d, J = 5.2 Hz), 3.86 (3H, s). |
| 5-2 | 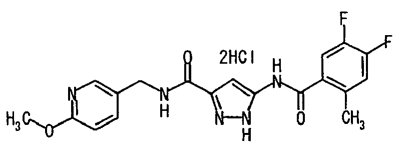 | ¹H-NMR(400 MHz, DMSO-*d*₆) δ: 10.98 (1H, s), 9.07 (1H, br s), 8.14 (1H, s), 7.72 (1H, m), 7.58 (1H, dd, J = 11.2, 8.4 Hz), 7.40 (1H, dd, J = 11.6, 8.0 Hz), 7.19 (1H, s), 6.85 (1H, m), 4.37 (2H, d, J = 5.6 Hz), 3.85 (3H, s), 2.35 (3H, s). |
| 5-3 | 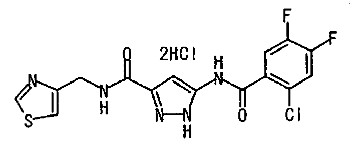 | ¹H-NMR(400 MHz, DMSO-*d*₆) δ: 11.18 (1H, s), 9.16(1H, m), 9.10 (1H, m), 7.87-7.81 (2H ,m), 7.49 (1H, br s), 7.29 (1H, s), 4.59 (2H, d, J = 5.6 Hz). |
| 5-4 | 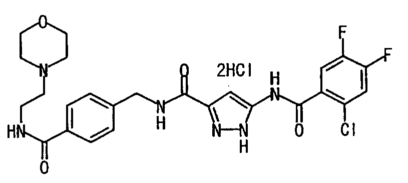 | ¹H-NMR(300 MHz,DMSO-*d*₆) δ: 11.20(1H,br s), 10.49 (1H, br s), 9.19 (1H, br s), 8.83(1H, t, J = 4.2 Hz), 7.92-7.75 (4H, m), 7.43 (2H, d, J = 8.1 Hz), 7.29 (1H, br s), 4.49 (2H, d, J = 5.5 Hz), 4.03-3.42 (10H, m). 3.30 (2H, d, J = 5.4 Hz), 2.49 (2H, br d, J = 6.1 Hz). |
| 5-5 | 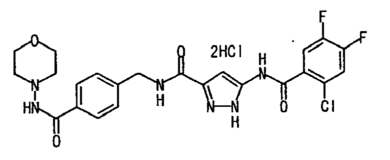 | ¹H-NMR(300 MHz, DMSO-*d*₆) δ: 11.19 (1H, br s), 9.57 (1H, br s), 9.17 (1H, t, J = 4.5 Hz), 7.84-7.73 (2H, m), 7.74 (2H, d, J = 6.7 Hz), 7.40 (2H, d, J = 6.7 Hz), 7.28 (1 H, br s), 4.49 (2H, d, J = 5.5 Hz), 3.90 (2H, brs), 3.60(4H, br s), 2.91 (4H, br s). |
| 5-6 | 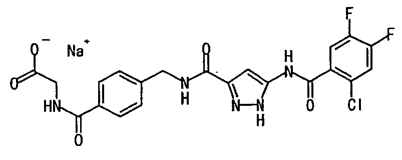 | ¹H-NMR(300 MHz, DMSO-*d*₆) δ: 8.88 (1H, br s), 7.90-7.60 (5H, m), 7.38 (2H, d, J = 8.1 Hz), 4.47 (2H, d, J = 4.2 Hz), 3.46 (2H, s). |

**Table 47**

| **Example** | **Molecular Structure** | **NMR** |
|---|---|---|
| 5-7 | 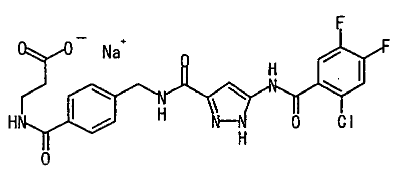 | ¹H-NMR(300 MHz, DMSO-d₆) δ: 8.84 (1H, br s), 8.52 (1H, br s), 7.72 (2H, d, J = 8.4 Hz), 7.57 (2H, t, J = 10.6 Hz), 7.35 (2H, d, J = 8.4 Hz), 6.34 (1 H, br s), 4.44 (2H, d, J = 6.2 Hz), 3.35 (2H, m), 2.12 (2H, t, J = 7.0 Hz). |
| 5-8 | 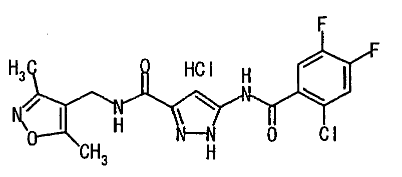 | ¹H-NMR(400 MHz, DMSO-*d*₆) δ: 11.18 (1H, s), 8.91 (1H, m), 7.87-7.80 (2H, m), 7.25 (1H, s), 4.18 (2H, d, J = 5.6 Hz), 2.40 (3H, s), 2.22 (3H, s). |
| 5-9 | 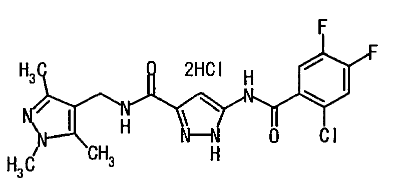 | ¹H-NMR(400 MHz, DMSO-*d*₆) δ: 11.17 (1H, br s), 8.90 (1H, m), 7.87-7.79 (2H, m), 7.23 (1H, s), 4.21 (2H, m), 3.78 (3H, m), 2.32 (6H, m). |
| 5-10 | 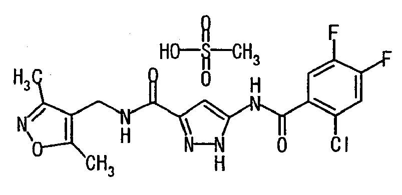 | ¹H-NMR(400 MHz, DMSO-*d*₆) δ: 11.17 (1H, s), 8.89 (1H, t, J = 5.6 Hz), 7.86-7.80 (2H, m), 7.25 (1H, s), 4.18 (2H, d, J = 5.6 Hz), 2.40 (3H, s), 2.22 (3H, s). |
| 5-11 | 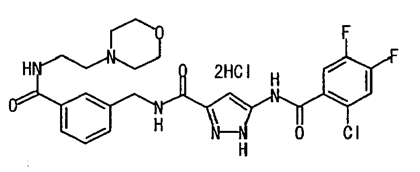 | ¹H-NMR(300 MHz, DMSO-*d*₆) δ: 11.21 (1H, s), 10.79 (1H, br s), 9.21 (1H, br s), 8.90 (1H, br s), 7.89-7.78 (4H, m), 7.57-7.43 (3H, m), 4.49 (2H, d, J = 5.9 Hz), 3.98-3.09 (14H, m). |
| 5-12 | 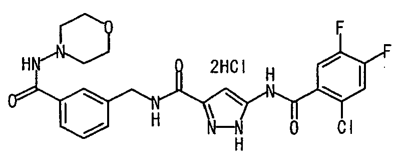 | ¹H-NMR(300 MHz, DMSO-d₆) δ: 11.18 (1H, br s), 10.04 (1H, br s), 9.19 (1H, t, J = 6.1 Hz), 7.90-7.74 (3H, m), 7.70 (1H, d, J =7.3 Hz), 7.54-7.38 (2H, m), 7.28 (1 H, s), 4.49 (2H, d, J = 4.9 Hz), 3.70 (4H, br s), 2.99 (4H, br s). |

**Table 48**

| **Example** | **Molecular Structure** | **NMR** |
|---|---|---|
| 5-13 | 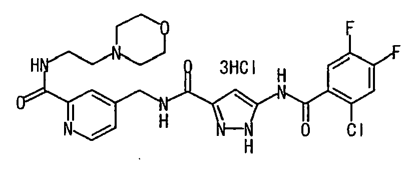 | ¹H-NMR(300 MHz,DMSO-*d*₆) δ: 11.25(1H,br s), 10.27 (1 H, br s), 9.34 (1 H, t, J = 4.5 Hz), 9.18 (1H, t, J = 4.5 Hz), 8.61 (1H, d, J = 4.3 Hz), 8.00 (1H, s), 7.89-7.80 (2H, m), 7.57 (1H, d, J = 3.6 Hz), 7.30 (1H, br s), 4.55 (2H, d, J = 4.5 Hz), 4.01-3.97 (2H, m), 3.77-3.05 (10H, m). |
| 5-14 | 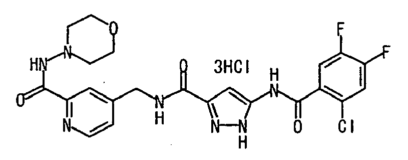 | H-NMR (300 MHz, DMSO-d6) δ: 11.24 (1H, br s), 9.97 (1H, br s), 9.30 (1H, t, J = 4.5 Hz), 8.58 (1H, d, J = 3.6 Hz), 7.97 (1H, s), 7.89-7.80 (2H, m), 7.55 (1H, d, J = 3.6 Hz), 7.30 (1H, s), 4.55 (2H, d, J = 4.5 Hz), 3.67 (4H, d, J = 8.1 Hz), 2.90 (4H, d, J = 7.8 Hz). |
| 5-15 | 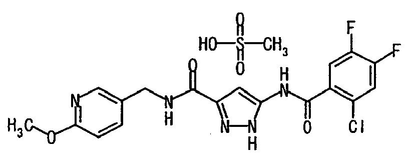 | ¹H-NMR(400 MHz, DMSO-*d*₆) δ: 11.18 (1H, s), 9.08 (1 H, t, J = 6.0 Hz), 8.14 (1H, s), 7.87-7.80 (2H, m), 7.71 (1H, m), 7.24 (1H, s), 6.85 (1H, m), 4.38 (2H, d, J = 6.0 Hz), 3.85 (3H, s), 2.39 (3H, s). |
| 5-16 | 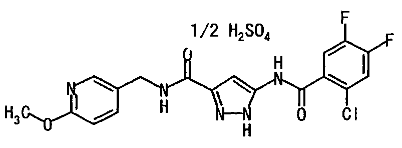 | ¹H-NMR(400 MHz, DMSO-*d*₆) δ: 11.18 (1H, s), 9.08 (1H, t, J = 6.0 Hz), 8.14 (1H, s), 7.87-7.80 (2H, m), 7.70 (1H, m), 7.25 (1H, s), 6.83 (1H, m), 4.38 (2H, d, J = 6.0 Hz), 3.84 (3H, s). |
| 5-17 | 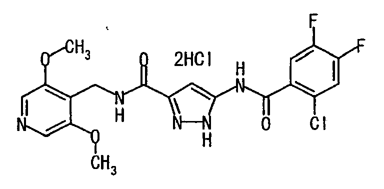 | ¹H-NMR(400 MHz, DMSO-d₆) δ: 11.17 (1H, s), 8.67 (1H, br s), 8.41 (2H, s), 7.86-7.78 (2H, m), 7.20 (1H, s), 4.52 (2H,d,J=5.2 Hz), 4.02 (6H, s). |
| 5-18 | 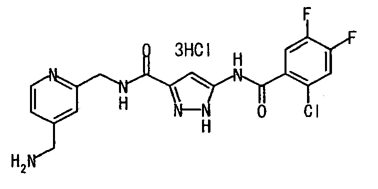 | ¹H-NMR(300 MHz, DMSO-d₆) δ: 11.23 (1H, s), 9.33 (1H, t, J = 5.5 Hz), 8.84-8.57 (3H, m), 7.91-7.77 (2H, m), 7.76-7.65 (2H, m), 7.29 (1H, s), 4.68 (2H, d, J = 5.5 Hz), 4.20 (2H, d, J = 5.5 Hz). |

**Table 49**

| **Example** | **Molecular Structure** | **NMR** |
|---|---|---|
| 5-19 | 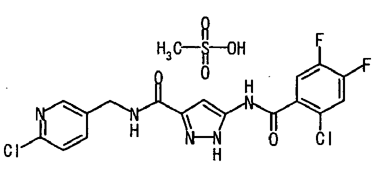 | ¹H-NMR(300 MHz, DMSO-*d*₆) δ: 11.19 (1H, s), 9.15 (1H, t, J=5.7 Hz), 8.38 (1H, d, J=2.2 Hz), 7.88-7.77 (3H, m), 7.50 (1H, d, J=8.1 Hz), 7.24 (1H, s), 4.46 (2H, d, J = 5.9 Hz), 2.37 (3H, s). |
| 5-20 | 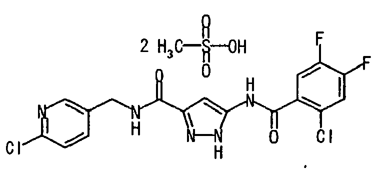 | ¹H-NMR(300 MHz, DMSO-*d*₆) δ: 11.19 (1H, s), 9.15 (1H, t, J = 5.9 Hz), 8.38 (1H, d, J = 2.2 Hz), 7.88-7.77 (3H, m), 7.50 (1H, d, J = 8.4 Hz), 7.24 (1H, s), 4.46 (2H, d, J=5.9 Hz), 2.44 (6H, s). |
| 5-21 | 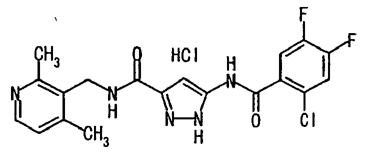 | ¹H-NMR(400 MHz, DMSO-*d*₆) δ: 13.33 (1H, br s), 11.21 (1H, br s), 9.00 (1H, br s), 8.58 (1H, d, J = 6.4 Hz), 7.87-7.81 (2H, m), 7.78 (1H, d, J = 6.4 Hz), 7.24 (1H, br s), 4.58 (2H, d, J = 4.8 Hz), 2.86 (3H, s), 2.66 (3H, s). |
| 5-22 | 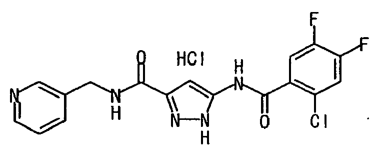 | ¹H-NMR(400 MHz, DMSO-*d*₆) δ: 11.25 (1H, s), 9.34 (1H, m), 8.87 (1H, m), 8.81 (1H, m), 8.45 (1H, m), 7.98 (1H, m), 7.88-7.81 (2H, m), 7.25 (1H, m), 4.63 (2H, m). |
| 5-23 | 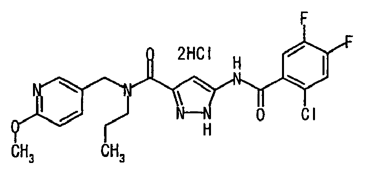 | ¹H-NMR (300 MHz, DMSO-*d*₆) δ: 11.25 (1H, s), 9.37 (br s), 8.25-7.98 (1H, m), 7.90-7.53 (3H, m), 6.91-6.58 (2H, m), 4.94-4.43 (2H, m), 3.86 (3H, s), 3.58-3.14 (2H, m), 1.64-1.60 (2H, m), 0.84 (3H, t, J = 7.5 Hz). |
| 5-24 | 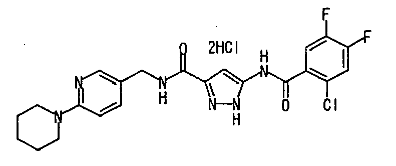 | ¹H-NMR (300 MHz, DMSO-*d*₆) δ: 11.22 (1H, s), 9.19 (1H, t, J = 5.7 Hz), 7.99-7.77 (4H, m), 7.43 (1H, d, J = 9.4 Hz), 7.21 (1 H, s), 4.37 (2H, d, J = 5.7 Hz), 3.83-3.62 (4H, m), 1.72-1.50 (6H, m). |

**Table 50**

| **Example** | **Molecular Structure** | **NMR** |
|---|---|---|
| 5-25 | 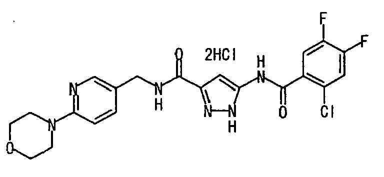 | ¹H-NMR (300 MHz, DMSO-*d*₆)δ: 11.22 (1H, s), 9.21 (1 H, t, J = 5.8 Hz), 8.04-7.99 (2H, m), 7.88-7.80 (2H, m), 7.41 (1H, d, J = 10.2 Hz), 7.22 (1H, s), 4.40 (2H, d, J = 5.7 Hz), 3.81-3.59 (8H, m). |
| 5-26 | 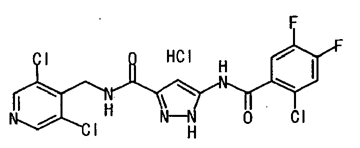 | ¹H-NMR (300 MHz, DMSO-*d*₆) δ: 11.18 (1H, s), 8.91 (1H, s), 8.65 (2H, d, J = 5.7 Hz), 7.87-7.78 (2H, m), 7.27 (1H, s), 4.67 (2H, d, J = 4.5 Hz). |
| 5-27 | 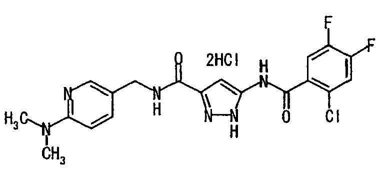 | ¹H-NMR (300 MHz, DMSO-*d*₆) δ: 11.23 (1H, s), 9.24 (1 H, t, J = 5.8 Hz), 8.02-7.79 (4H, m), 7.30-7.18 (2H, m), 5.68 (br s), 4.38 (2H, d, J = 5.7 Hz), 3.24 (6H, s). |
| 5-28 | 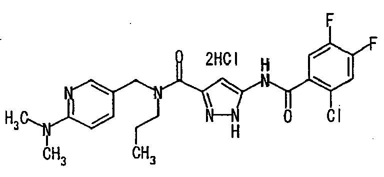 | ¹H-NMR (300 MHz, DMSO-d₆) δ: 13.85 (1H, br s), 11.31 (1H, s), 8.05-7.80 (4H, m), 7.25 (1H, d, J = 9.4 Hz), 6.83 (1H, br s), 5.01-4.49 (2H, m), 3.63-3.42 (1H, m), 3.38 (br s), 3.25 (7H, m), 1.75-1.51 (2H, m), 0.85 (3H, t, J = 7.3 Hz). |
| 5-29 | 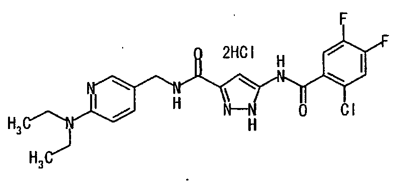 | ¹H-NMR (300 MHz, DMSO-d₆) δ: 13.81 (br s), 11.23(1H,s), 9.20 (1H, br t), 8.01-7.78 (4H,m), 7.30-7.13 (2H, m), 4.37 (2H, d, J = 5.7 Hz), 3.65 (4H, q, J = 7.0 Hz), 1.18 (6H, t, J = 7.0 Hz). |
| 5-30 | 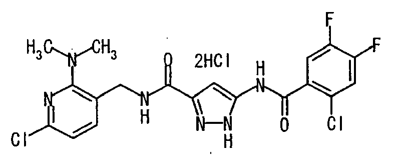 | ¹H-NMR (300 MHz, DMSO-*d*₆) δ: 11.18 (1H, s), 10.08 (br s), 9.01 (1H, t, J = 5.7 Hz), 7.88-7.79 (2H, m), 7.53 (1H, d, J = 8.7 Hz), 7.28 (1H, s), 6.62 (1H, d, J = 8.3 Hz), 4.36 (2H, d, J = 5.7 Hz), 3.00 (6H, s). |

**Table 51**

| **Example** | **Molecular Structure** | **NMR** |
|---|---|---|
| 5-31 | 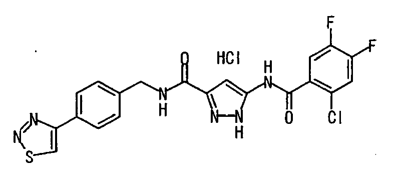 | ¹H-NMR (400 MHz, DMSO-*d*₆) δ: 11.18 (1H, s), 9.59 (1H, s), 9.19 (1H, t, J = 5.8 Hz), 8.10 (2H, d, J=7.9 Hz), 7.84-7.81 (2H, m), 7.48 (2H, d, J= 8.3 Hz), 7.30 (1 H, br s), 4.51 (2H, d, J = 6.0 Hz). |
| 5-32 | 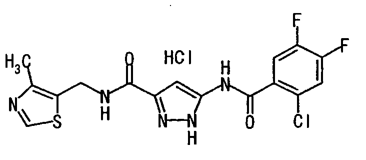 | ¹H-NMR (300MHz, DMSO-*d*₆) δ: 11.59 (br s), 9.49-9.32 (2H, m), 7.88-7.81 (2H, m), 7.21 (1H, s), 4.60 (2H, d, J = 5.7 Hz), 2.48 (3H, s). |
| 5-33 | 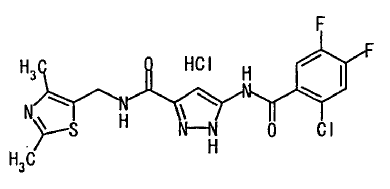 | ¹H-NMR (300 MHz, DMSO-*d*₆) δ: 11.25(1H, s), 9.90 (br s), 9.45 (1H, t, J = 5.7 Hz), 7.88-7.79 (2H, m), 7.18 (1H, s), 4.54 (2H, d, J = 5.7 Hz), 2.82 (3H, s), 2.48 (3H, s). |
| 5-34 | 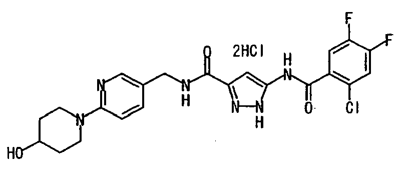 | ¹H-NMR (300 MHz, DMSO-*d*₆) δ: 11.23(1H, s), 9.20 (1H, t, J=5.8Hz), 8.00-7.80 (4H, m), 7.45 (1H,d, J = 9.4 Hz), 7.22 (1 H, s), 4.37 (2H, d, J = 5.7 Hz), 3.94-4.04 (2H, m), 3.79-3.87 (1H, m), 3.48-3.60 (2H, m), 1.79-1.90 (2H, m), 1.42-1.54 (2H,m). |
| 5-35 | 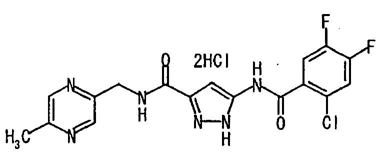 | ¹H-NMR (400 MHz, DMSO-*d*₆) δ: 11.19(1H, s), 9.21 (1H, t, J = 5.8 Hz), 8.49 (2H, s), 7.88-7.78 (2H, m), 7.26 (1 H, s), 4.53 (2H, d, J = 5.6 Hz), 2.47 (3H, s). |
| 5-36 | 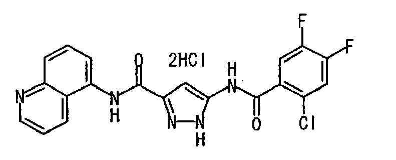 | ¹H-NMR (400 MHz, DMSO-*d*₆) δ: 11.33 (1H, s), 10.95 (1H, s), 9.26 (1H, d, J =4.2 Hz), 9.02 (1H, br d, J = 8.3 Hz), 8.23 (1H, d, J = 8.3 Hz), 8.11 (1H, t, J = 7.9 Hz), 7.99 (2H, d, J = 7.4 Hz), 7.91-7.83 (2H, m), 7.59 (1 H, br s). |

**Table 52**

| **Example** | **Molecular Structure** | **NMR** |
|---|---|---|
| 5-37 | 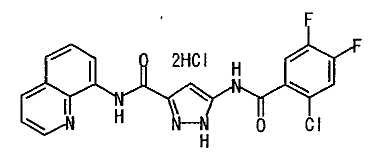 | ¹H-NMR (400 MHz, DMSO-*d*₆) δ: 11.59 (1H, br s), 11.02 (1H, br s), 9.01 (1H, dd, J = 4.2, 1.4 Hz), 8.73 (1H, d, J = 7.4 Hz), 8.50 (1H, dd, J = 8.3, 1.4 Hz), 7.96-7.89 (2H, m), 7.80-7.62 (3H, m), 6.84 (1H, br s), 5.81-5.79 (2H, br s). |
| 5-38 | 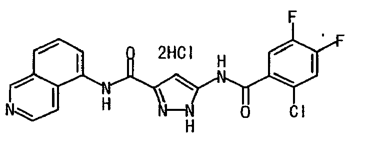 | ¹H-NMR (400 MHz, DMSO-*d*₆) δ: 11.33 (1H, s), 10.92 (1H, s), 9.87 (1H, s), 8.68 (1H, d, J = 6.5 Hz), 8.40 (1H, d, J = 8.3 Hz), 8.34 (1H, d, J = 7.0 Hz), 8.23 (1H, d, J = 7.0 Hz), 8.02 (1 H, t, J = 7.9 Hz), 7.91-7.83 (2H, m), 7.59 (1H, s). |
| 5-39 | 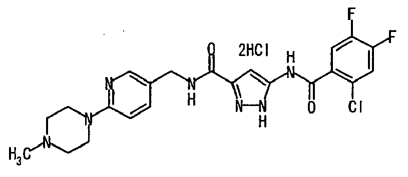 | ¹H-NMR (300 MHz, DMSO-*d*₆) δ: 11.50 (1H, s), 11.22 (1H, br s), 9.21 (1H, s), 8.09 (1H, s), 8.00-7.76 (3H, m), 7.38-7.26 (1H, m), 7.22 (1H, s), 4.56-4.29 (4H, m), 3.63-3.40 (4H, m), 3.26-3.06 (2H, m), 2.79-2.76 (3H, m). |
| 5-40 | 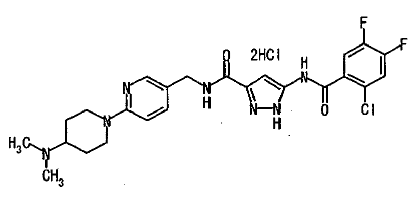 | ¹H-NMR (300 MHz, DMSO-*d*₆) δ: 11.22 (1H, s), 11.06 (1H, br s), 9.20 (1H, br t, J = 5.3 Hz), 8.01-7.91 (2H, m), 7.88-7.80 (2H, m), 7.40 (1 H, br d, J = 9.8 Hz), 7.22 (1H, s), 4.52 (2H, br d, J = 13.2 Hz), 4.37 (2H, br d, J = 6.0 Hz), 3.54-3.40 (1 H, m), 3.14 (2H, br t, J = 8.3 Hz), 2.70 (3H, s), 2.69 (3H, s), 2.17 (2H, br d, J = 12.4 Hz), 1.79-1.65 (2H,m). |
| 5-41 | 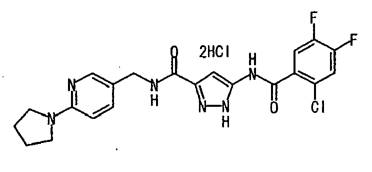 | ¹H-NMR (300 MHz, DMSO-*d*₆) δ: 13.80 (br s), 11.23 (1H, s), 9.23 (1 H, t, J = 5.8 Hz), 8.01-7.79 (4H, m), 7.22 (1H, s), 7.12 (1 H, d, J = 9.4 Hz), 4.38 (2H, d, J = 5.7 Hz), 3.62-3.48 (4H, m), 2.10-1.95 (4H, m). |
| 5-42 | 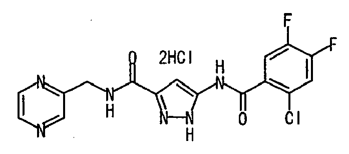 | ¹H-NMR (400 MHz, DMSO-*d*₆) δ: 11.22 (1H, s), 9.28 (1H, t, J = 5.8 Hz), 8.66 (1H, d, J = 1.4 Hz), 8.62 (1H. dd, J = 2.8, 1.4 Hz), 8.57 (1H, d, J = 2.8 Hz), 7.90-7.81 (2H, m), 7.29 (1H, s), 4.61 (2H, d, J = 6.0 Hz). |

**Table 53**

| **Example** | **Molecular Structure** | **NMR** |
|---|---|---|
| 5-43 | 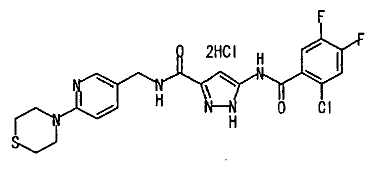 | ¹H-NMR (300 MHz, DMSO-*d*₆) δ: 11.21 (1H, s), 9.21 (1 H, t, J = 5.7 Hz), 8.01-7.95 (2H, m), 7.86-7.76 (2H, m), 7.42 (1H, d, J = 9.4 Hz), 7.20 (1H, s), 4.36 (2H, d, J = 6.0 Hz), 4.10-4.02 (4H, m), 2.75-2.70 (4H, m). |
| 5-44 | 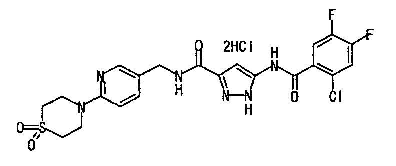 | ¹H-NMR (300 MHz, DMSO-*d*₆)δ: 11.23 (1H, s), 9.21 (1H, br t, J =6.0 Hz), 8.09 (1H, s), 7.94 (1H, d, J = 8.3 Hz), 7.89-7.81 (2H, m), 7.40 (1H, br d, J = 8.8 Hz), 7.24 (1 H, s), 4.40 (2H, d, J = 6.0 Hz), 4.18 (4H, br s), 3.28 (4H, br s). |

Further, the compounds of Example 6-1 to Example 6-50 and the compounds of Example 7-1 to Example 7-17 illustrated in the following tables were prepared in the same manner.

**Table 54**

| **Example** | **Molecular Structure** | **NMR** |
|---|---|---|
| **6-1** | 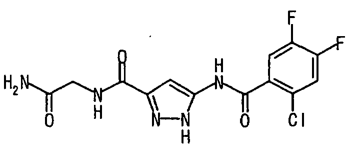 | ¹H-NMR (300 MHz, DMSO-*d*₆) δ: 13.24(1H,s), 11.14(1H,brs), 8.80(1H,brs), 7.94-7.80(2H,m), 7.69(1H,s), 7.40-7.32(1H,m), 7.06(1H,s), 3.79(2H,d,J=5.6Hz). |
| **6-2** | 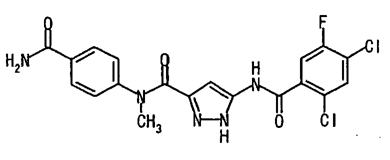 | ¹H-NMR (400MHz,DMSO-*d*₆) δ: 13.26 (1H, s), 10.96 (1H, s), 7.98(2H, d, J = 8.3 Hz), 7.81-7.80(2H,m), 7.45 2H, d, J = 8.1 Hz), 5.67 (1H, s), 3.36 (3H,s). |
| **6-3** | 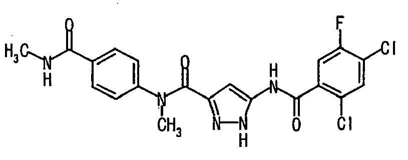 | ¹H-NMR (400MHz,DMSO-*d*₆) δ: 13.27 (1H, s), 10.96 (1H, s), 8.55(1 H,d,J=4.0Hz), 7.95 (2H, d, J = 8.3 Hz), 7.82-7.70(2H,m), 7.46 (2H, d, J = 8.1 Hz), 3.38 (3H, s), 2.82 (3H, d, J = 4.6Hz). |
| **6-4** | 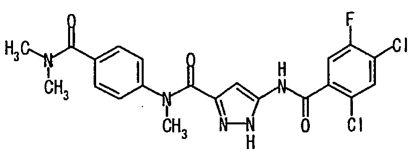 | ¹H-NMR (400 MHz, DMSO-*d*₆) δ: 13.27(1H,s), 10.98(1H,s), 7.79(1H,m), 7.69(1H,m), 7.52-7.43(4H,m), 5.56(1H,s), 3.38(3H,s), 2.98(6H,m). |
| **6-5** | 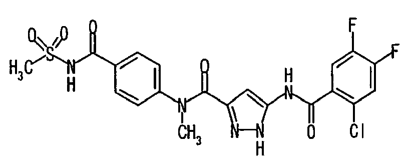 | ¹H-NMR (400 MHz, DMSO-*d*₆) δ: 13.29(1H,brs), 12.21(1H,m), 11.00(1H,brs), 8.05(2H,d,J=7.2Hz), 7.82-7.74(2H,m), 7.51(2H,d,J=6.5Hz), 5.81(1H,m), 3.39(3h,s). |
| **6-6** | 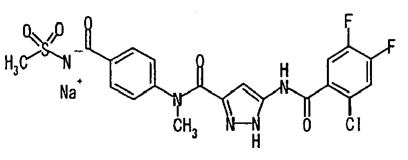 | ¹H-NMR (400 MHz, DMSO-*d*₆) δ: 13.20(1H,s), 10.89(1H,s), 7.98(2H,d,J=8.0Hz), 7.78(2H,m), 7.27(2H,d,J=8.0Hz), 5.69(1H,s), 4.34(1h,d,J=4.4Hz), 3.35(3H,s), 2.84(3H,s). |
| **6-7** | 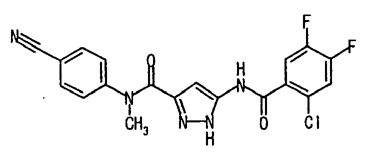 | ¹H-NMR (400 MHz, DMSO-*d*₆) δ: 13.30(1H,s), 11.01(1H,s), 7.96(2H,d,J=8.0Hz), 7.85-7.74(2H,m), 7.62(2H,d,J=7.8Hz), 5.70(1H,s), 3.39(3H,s). |

**Table 55**

| **Example** | **Molecular Structure** | **NMR** |
|---|---|---|
| **6-8** | 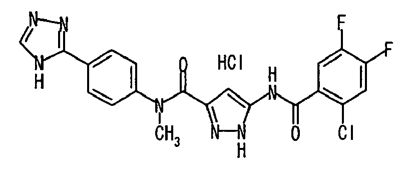 | ¹H-NMR (400 MHz, DMSO-*d*₆) δ: 10.98 (1H, s), 8.60 (1H, s), 8.12 (2H, d, J = 8.6 Hz), 7.78-7.68 (2H,m), 7.48(2H,d,J=8.6Hz), 5.75 (1H, s), 3.39 (3H, s). |
| **6-9** | 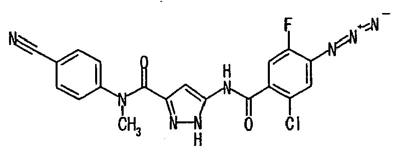 | ¹H-NMR (300 MHz, DMSO-d₆) δ:13.28(3H,s), 10.97(1H,s), 7.96(2H,d,J=8.1 Hz), 7.60(2H,d,J=8.3Hz), 7.56-7.42(3H,m), 5,72(1,s), 3.39(3H,s): |
| **6-10** | 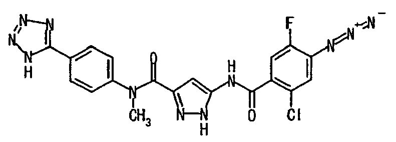 | ¹H-NMR (400 MHz, DMSO-*d*₆) δ: 13.30(1h,s), 10.94(1H,s), 8.12(2H,d,J=8.2Hz), 7.63(2H,d,J=8.2Hz), 7.52-7.44(2H,m), 5.73(1H,s), 3.41(3H,s). |
| **6-11** | 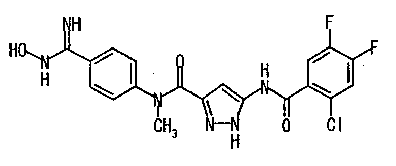 | ¹H-NMR (400 MHz, DMSO-*d*₆) δ: 13.23 (1H, s), 10.94 (1H, s), 9.74 (1H, s), 7.80-7.70 (4H,m), 7.37 (2H.d,J=8.6 Hz).5.87 (2H.s). 5.68(1H,s), 3.36 (3H, s). |
| **6-12** | 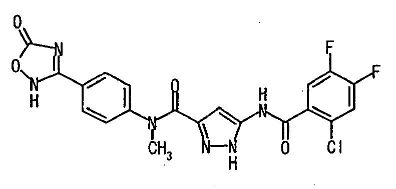 | ¹H-NMR (400 MHz, DMSO-*d*₆) δ: 13.28 (1H, s), 13.06(1H,s), 10.98 (1H, s), 7.89 (2H, d, J = 8.6 Hz), 7.78(1H,m), 7.70(1H,m), 7.59 (2H, d, J = 8.3 Hz), 5.73 (1H, s), 3.38 (3H, s). |
| **6-13** | 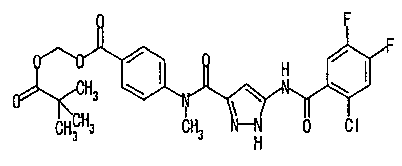 | ¹H-NMR (400 MHz, DMSO-*d*₆) δ: 13.31 (1H, s), 10.99 (1H, s), 8.06 (2H, d, J = 8.3 Hz), 7.56 (2.1H, d, J = 8.3 Hz), 5.97 (2H, s), 3.40 (3H,s),1.12 (9H, s). |
| **6-14** | 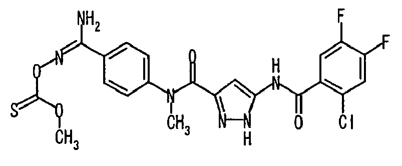 | ¹H-NMR (400 MHz, DMSO-d₆) δ:13.27(1H,s), 10.97(1H,s), 7.83-7.72(4H,m), 7.47(2H,d,J=8.1 Hz), 7.05(2H,m), 5.77(1 H,s), 4.07(3H,s), 3.42(3H,s). |

**Table 56**

| **Example** | **Molecular Structure** | **NMR** |
|---|---|---|
| **6-15** | 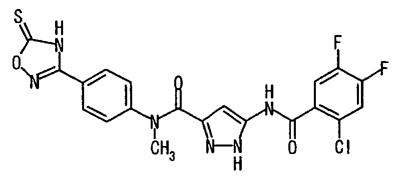 | ¹H-NMR (400 MHz, DMSO-*d*₆) δ: 13.25(1H,s), 10.94(1H,s), 7.95 (2H, d, J = 7.7 Hz), 7.80-7.70(2H,m),, 7.47 (2H, d, J = 7.7 Hz), 5.73(1H,s), 3.38 (3H, s). |
| **6-16** | 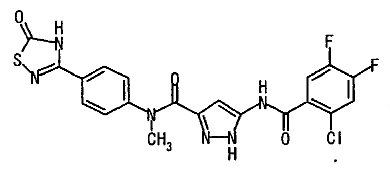 | ¹H-NMR (400 MHz, DMSO-*d*₆) δ: 13.52((1H,s), 13.29(1H,s), 10.98(1H,s), 8.05 (2H, d, J = 8.6 Hz), 7.81-7.69(2H,m), 7.56 (2H, d, J = 8.3 Hz), 5.74(1H,s), 3.39 (3H, s). |
| **6-17** | 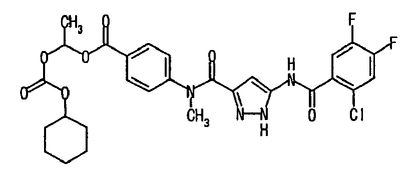 | ¹H-NMR (400MHz, CDCl3) δ: 13.43(1H,m), 10.59(1H,brs), 8.15 (1H, d, J=8.6 Hz), 7.56(1H,m), 7.33-7.24 (3H, m), 7.04(1H,m), 6.00(1H,m), 4.65(1H,m), 3.20 (3H, s), 1.91(1H,m), 1.73-1.23(12H, m). |
| **6-18** | 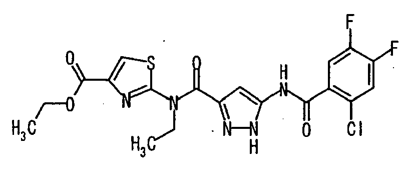 | ¹H-NMR (400 MHz, DMSO-*d*₆) δ:13.73(0.6H,s), 13.56(0.4H,s), 11.60(0.4H,s), 11.36(0.6H,s), 8.35(0.6H,s) 8.17(0.4H,s), 7.97-7.84(2H,m), 7.23(0.6H,s), 6.66(0.4H,s), 4.76(1H,m), 4.46(2H,m), 4.30(2H,m), 1.37(3H,m), 1.34(3H,t,J= |
| **6-19** | 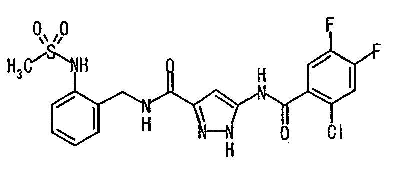 | ¹H-NMR (400 MHz, DMSO-*d*₆) δ:13.32(1H,s), 11.20(1H,s), 9.43(1H,s), 9.20(1H,s), 7.83(2H,m), 7.35-7.25(4H,m), 4.50(2H,d,J=8.4Hz), 3.05(3H,s). |
| **6-20** | 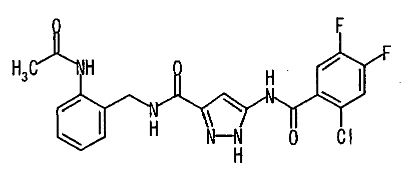 | ¹H-NMR (400 MHz, DMSO-*d*₆) δ:13.31(1H,s), 11.17-11.01(1h,m), 9.69(1H,m), 9.14(1H,m), 7.93(1H,m), 7.82(1H,m), 7.53(1H,m), 7.36-7.15(4H,m), 4.42(2H,m), 2.11(3H,s). |

**Table 57**

| **Example** | **Molecular Structure** | **NMR** |
|---|---|---|
| **6-21** | 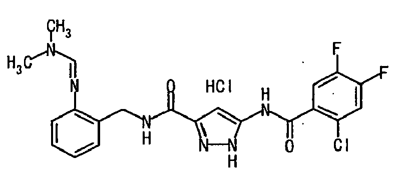 | ¹H-NMR (400 MHz, DMSO-d₆) δ: 13.36 (1H, s), 11.60(1H, s), 11.20(1H, s), 9.52 (0.4H, s), 8.52 (1H, s), 7.83 (2H, m), 7.41-7.34 (5H, m), 4.56 (2H, s), 3.33(6H, s). |
| **6-22** | 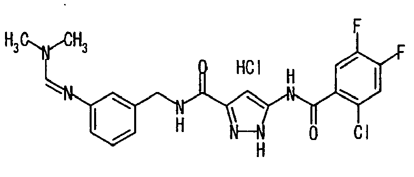 | ¹H-NMR (400 MHz, DMSO-d₆) δ: 13.31 (1H.s). 11.32(1H,s), 11.17 (1H, s), 9.26 (1 H, s), 8.70 (1 H, s), 7.86 (2H, m), 7.41-7.35 (3H, m), 7.22(1H, d, J=7.0Hz), 4.47 (2H, d, J = 6.0 Hz), 3.24 (3H, s). |
| **6-23** | 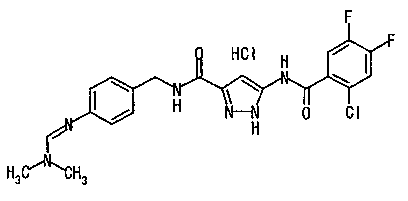 | ¹H-NMR (400 MHz, DMSO-*d*₆) δ: 13.28 (1H, s), 11.37(1H, s), 11.16(1H, s), 9.22 (1H, m), 8.68 (1H, s), 7.94-7.78 (2H, m), 7.3645-7.37 (5H, m), 4.44(2H, d, J=5.6Hz), 3.26(3H,s). |
| **6-24** | 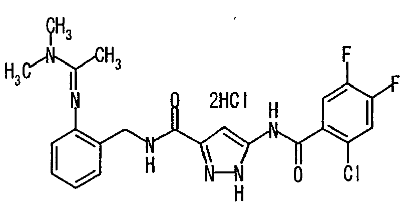 | ¹H-NMR (300 MHz, DMSO-*d*₆) δ:11.21 (1 H,s),10.42(1 H,s),9.18(1H,s),7.88-7.78(2H,m),7.50-7.45(2H,m),7.32-7.24(3H,m),4.47(2H,d,J=5.5Hz), 3.45(3H,m),3.21 (3H, s), 2.03 (3H, s). |
| **6-25** | 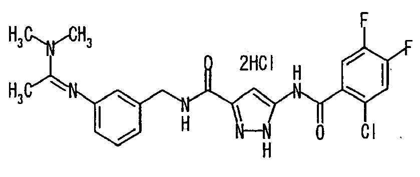 | ¹H-NMR (300 MHz, DMSO-*d*₆) δ: 11.20 (1H, s), 10.46 (1H, s), 9.23 (1H, s), 7.88-7.69 (2H, m), 7.50-7.09(5H,m), 4.50 (2H, d, J = 6.2 Hz), 3.27-3.17 (6H, m), 2.33-2.29 (3H, m). |
| **6-26** | 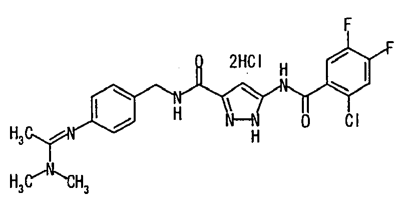 | ¹H-NMR (300 MHz, DMSO-*d*₆) δ: 11.19 (1H, s), 10.52(1H,s),9.21(1H,m),7.83 (2H, dd, J = 18.2, 8.6 Hz), 7.49-7.25 (4H, m), 7.11(1H,m),4.49 (2H, d, J=5.9 Hz), 3.28 (6H, m), 2.16 (3H,s). |

**Table 58**

| **Example** | **Molecular Structure** | **NMR** |
|---|---|---|
| **6-27** | 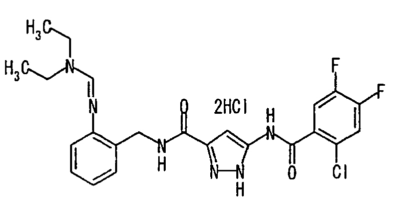 | ¹H-NMR (300 MHz, DMSO-*d*₆) δ:11.47(1H,d,J=12,0Hz),9.47(1H,s), 8.49 (1H, d, J = 12.8 Hz), 7.88-7.79 (2H, m), 7.45-7.35 (4H, m), 7.25(1H,m),4.55 (2H, d, J = 5.9 Hz), 3.78-3.63 (4H, m), 1.34-1.26 (6H, m),. |
| **6-28** | 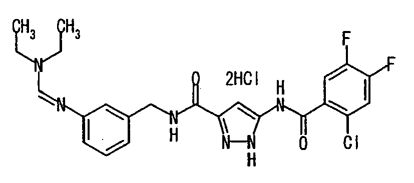 | ¹H-NMR (300 MHz, DMSO-*d*₆) δ: 11.34(1 H,d,J=1 3.2Hz), 11.20 (1H, s),9.21(1H,m),8.66(1H,d,J=12.8Hz), 7.88-7.79 (2H, m), 7.47-7.42(2H,m),7.34-7.26 (2H, m), 4.47 (2H, d, J = 5.5 Hz), 3.74-3.64 (4H, m), 1.31-1.18 (6H, m). |
| **6-29** | 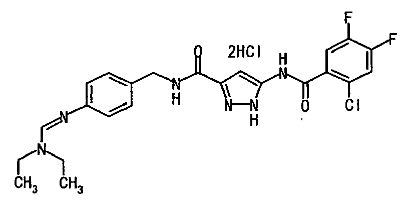 | ¹H-NMR (300 MHz, DMSO-d₆) 11.41(1H,d,J=15Hz),11.19 (1H, s),9.17(1H,m), 8.63 (1H, d, J = 13.2 Hz), 7.87-7.78 (2H, m), 7.48(2H,d,J=8.4Hz),7.40 (2H, dd, J = 8.4 Hz), 7.26(1 H,m),4.44 (2H, d, J = 5.9 Hz), 3.69 (4H, dd, J = 16.0, 7.2 Hz),, 1.30-1.20 (6H, m). |
| **6-30** | 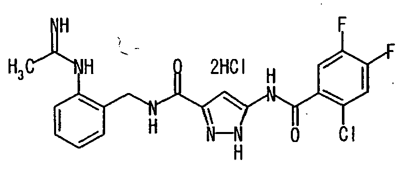 | ¹H-NMR (400 MHz, DMSO-*d*₆) δ: 11.26(1H,s), 11.21(1H,s), 9.45(1H,s), 9.10(1H,m), 8.29(1H,s), 7.88-7.79(2H,m), 7.51-7.43(3H,m), 7.32-7.23(2H,m), 4.42(2H,d,J=8.8Hz). |
| **6-31** | 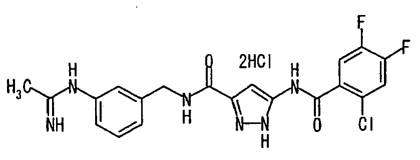 | ¹H-NMR (400 MHz, DMSO-*d*₆) δ: 11.39(1H,s), 11.21(1H,s), 9.55(1H,s), 9.21(1H,s), 7.88-7.80(2H,m), 7.48-7.21(5H,m), 4.49(2H,d,J=6.OHz), 2.34(3H,s). |
| **6-32** | 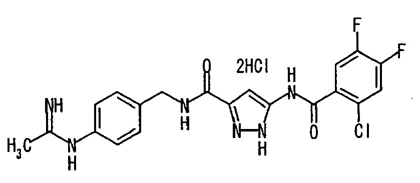 | ¹H-NMR (400 MHz, DMSO-*d*₆) δ: 11.34(1H,s), 11.21 (1H,s), 9.48(1H,s), 9.24(1H,brs), 8.52(1H,s). 7.88-7.82(2H,m), 7.46(2H,d,J=9.2Hz), 7.30(2H,d,J=9.2Hz), 4.47(2H,m), 2.33(3H,s). |

**Table 59**

| **Example** | **Molecular Structure** | **NMR** |
|---|---|---|
| **6-33** | 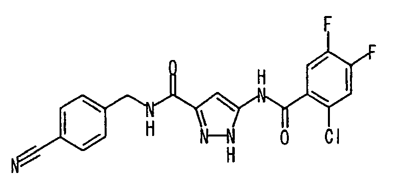 | ¹H-NMR (400 MHz, DMSO-*d*₆) δ: 13.28 (1H, s), 11.17 (1H, s), 9.24 (1H, m), 7.94-7.80 (4H, m), 7.50(2H ,d, J=8.1Hz), 7.36(1 h, s), 4.52 (2H, d, J = 5.8 Hz). |
| **6-34** | 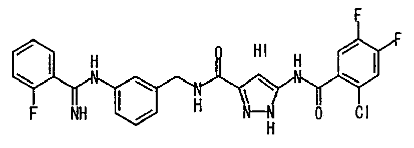 | ¹H-NMR (400 MHz, DMSO-*d*₆) δ:11.69 (1H, s), 11.20 (1H, s), 10.02 (1H, s), 9.36 (1H, s), 9.17(1H, s), 8.31 (1H, d, J = 2.6 Hz), 7.88-7.79 (4H, m), 7.58-7.31(8H, m), 4.53 (2H, d, J = 6.0 Hz). |
| **6-35** | 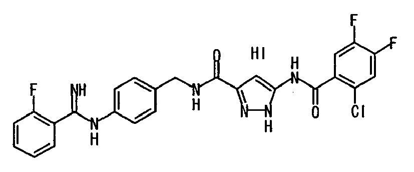 | ¹H-NMR (400 MHz, DMSO-*d*₆) δ: 11.63 (1H, s), 11.19 (1H, s), 9.98 (1H, s), 9.33 (1H, s), 9.27(1H, s), 7.87-7.79 (5H, m), 7.56-7.42(7H,), 4.51 (2H, d, J = 6.0 Hz). |
| **6-36** | 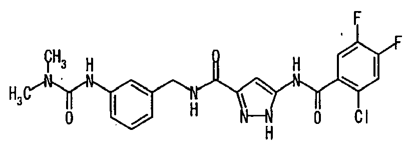 | ¹H-NMR (400 MHz, DMSO-*d*₆ δ: 13.24(1 H,s),11.15(1H,s),9.12(1 H,m),8.26(1 H,s),7.84-7.80(2H,m),7.41(1 H,s),7.36(2H,brs),7.16(1 H,t,J=8.0Hz),6,89(1H,m),4.39(2H,d,J=6.0H z),,2.91(6H,s). |
| **6-37** | 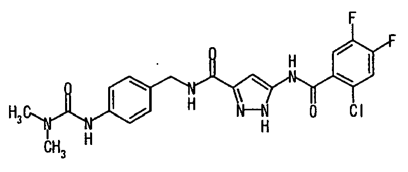 | ¹H-NMR (400 MHz, DMSO-*d*₆) δ: 1H-NMR (DMSO-d6) δ:13.22 (1H, s), 11.13 (1 H, d, J = 7.9 Hz), 9.08(1H,m), 8.24 (1H, s), 7.85-7.78 (2H, m), 7.42-7.15 (5H, m). 4.36 (2H, d, J = 6.0 Hz), 2.96 (6H, s). |
| **6-38** | 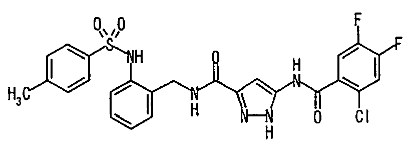 | ¹H-NMR (300 MHz, DMSO-d₆) δ:13.35(1H,brs),11.19(1 H,s),9.84(1H,s),9.1 4(1H,s), 7.79-7.87(2H,m),7.59 (2H, d, J = 8.4 Hz), 7.0-7.35 (7H,m),4.25(2H,d,J=6.0Hz), 2.36 (3H, m), |
| **6-39** | 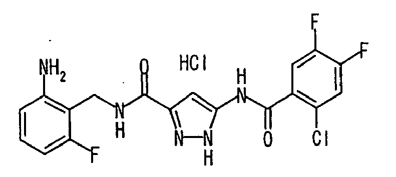 | ¹H-NMR (400 MHz, DMSO-*d*₆) δ: 11.20 (1H, s), 9.22(1H,s),7.84 (2H, m), 7.30(1 H,s), 7.18(1 H,m), 6.78(1H,m).6.6.9(1H,m),4.42 (2H, d, J = 5.6 Hz),. |

**Table 60**

| **Example** | **Molecular Structure** | **NMR** |
|---|---|---|
| **6-40** | 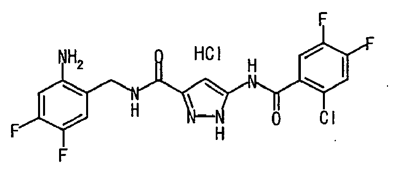 | ¹H-NMR (300 MHz, DMSO-*d*₆) δ: 11.20 ¹(1H, s), 9.08 (1H, t, J = 5.7 Hz), 7.83 (2H, ddd, J = 11.6, 6.7, 3.9 Hz), 7.23(1H,s),7.05 (1.0H, t,J=12Hz), 6.72 (1H. dd, J = 12.7, 7.2 Hz), 4.26 (2H, d,J=6.OHz) |
| **6-41** | 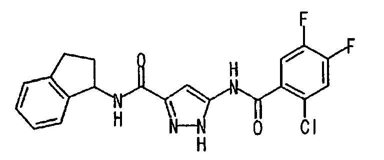 | ¹H-NMR (400 MHz, DMSO-*d*₆) δ: 13.24 (1H, s), 11.12 (1H, s), 7.84-7.77(2H, m), 7.37(1H, s), 7.28-7.18(4H, m), 5.52 (1H, q, J = 8.0 Hz), 2.99 (1H. m), 2.87(1H. m), 2.43 (1H, m), 2.00-1.95 (1H, m). |
| **6-42** | 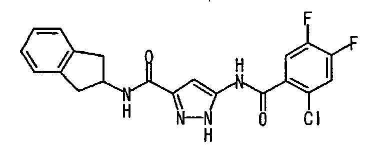 | ¹H-NMR (400 MHz, DMSO-d₆) δ: 13.19 (1H, d, J = 1.6 Hz), 11.12 (1H, s), 8.79 (1H, d, J = 7.0 Hz), 7.84-7.77 (2H, m), 7.35 (1H, d, J=2.1Hz), 7.25-7.22(2H, m), 7.17-7.14(2H, m), 4.67 (1H, q, J = 7.2 Hz), 3.30-3.20 (2H, m), 2.97-2.92(2H, m). |
| **6-43** | 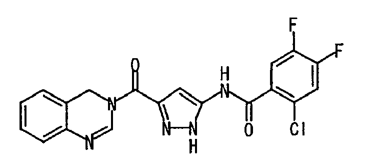 | ¹H-NMR (400 MHz, DMSO-*d*₆) δ: 13.74(1H,m), 11.46(1H,m), 7.95-7.89(2H,m), 7.28-7.22(4H,m), 6.86(1H,m), 4.92(2H,s). |
| **6-44** | 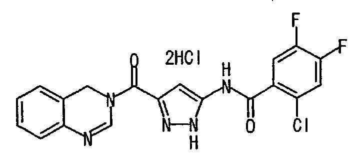 | ¹H-NMR (400 MHz, CDCI3) δ: 11.69(1H,s), 7.92(2H,m), 7.40(4H,m), 6.92(1H,brs), 5.08(2H,s). |
| **6-45** | 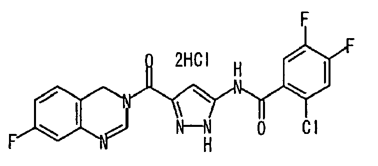 | ¹H-NMR (400 MHz, DMSO-*d*₆) δ: 12.09(1H,S9, 11.61(1H,2H,s), 7.91-7.83(3H,m), 7.24(1H,m), 7.24-7.16(3H,m), 6.88(1H,brs), 5.10(2H,s). |
| **6-46** | 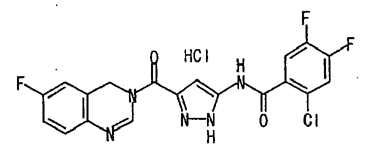 | ¹H-NMR (400 MHz, DMSO-*d*₆) δ: 11.66(1H,s),9.10(1H,m),7.93 (2H, dd, J = 10.6, 7.1 Hz), 7.42(1H,m),7.34 (1H, m), 7.25(1H,m),6.91(1H,brs),5.05 (2H, s). |

**Table 61**

| **Example** | **Molecular Structure** | **NMR** |
|---|---|---|
| **6-47** | 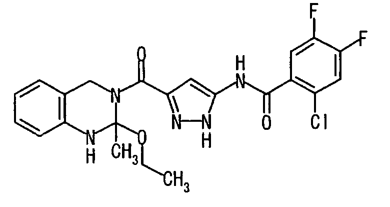 | ¹H-NMR (400 MHz, DMSO-*d*₆) δ: 13.23 (1H.s). 11.16 (H, s), 8.91 (1 H, s), 7.84 (2H, dd, J = 18.6, 8.3 Hz), 7.37-7.01 (5H, m), 6.70(1H.d.J=7.7Hz).4.30(2H.d,J= 5.6Hz),4.23(2H,d,J=7.1 Hz), 1.77 (3H, s), 1.29 (4.6H, t, J = 7.1 Hz).. |
| **6-48** | 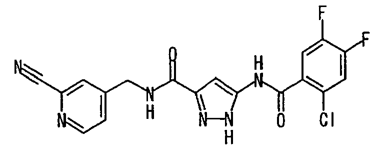 | ¹H-NMR (400 MHz, DMSO-*d*₆) δ:13.31(1H,s), 11.20(1H,s), 9.27(1H,t,J=6.0Hz), 8.71(1H,d,J=5.2Hz), 7.87(1H,m), 7.82(2H,m). 7.65(1H,m), 7.39(1H,s), 4.55(2H,d,J=6.0Hz). |
| **6-49** | 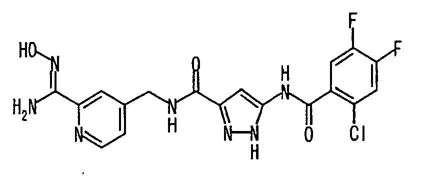 | ¹H-NMR (400 MHz, DMSO-*d*₆) δ:13.34(1H,s), 11.21(1H,s), 9.88(1H,s), 9.29(1H,t,J=6.0Hz), 8.49(1H,m), 7.88(2H.m), 7.79(1H,s), 7.40(1H,s), 7.34(1h,m), 4.50(2H,m). |
| **6-50** | 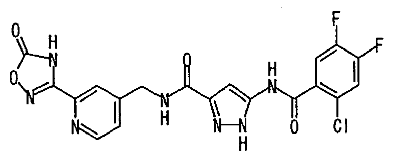 | ¹H-NMR (400 MHz, DMSO-*d*₆) δ:13.26(1H,s), 11.18(1H,s), 9.32(1H,m), 8.69(1H,m), 7.94-7.83(4H,m), 7.57(1H,s), 7.40(1H,s), 4.56(2H,m), |

**Table 62**

| **Example** | **Molecurar Structure** | **NMR** |
|---|---|---|
| **7-1** | 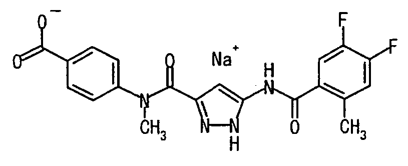 | ¹H-NMR (400 MHz, DMSO-*d*₆) δ: 7.78 (2H, d, J = 8.6 Hz), 7.49-7.43(2H,m), 7.12-7.05 (3H, m), 5.54(1H,m), 3.37 (3H, s). |
| **7-2** | 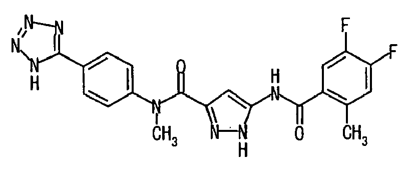 | ¹H-NMR (400 MHz, DMSO-*d*₆) δ: 13.26(1H,brs), 10.77(1H,brs), 8.13 (2H, d, J = 7.0 Hz), 7.62 (2H, s), 7.46(1H,m), 7.33(1 H,m), 3.43 (3H, s). |
| **7-3** | 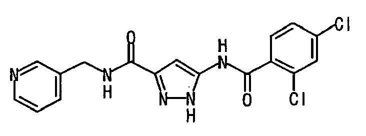 | ¹H-NMR(400 MHz, DMSO-*d*₆)δ: 13.26 (1H, s), 11.13 (1 H, s), 9.19 (1 H, t, J = 5.9 Hz), 8.57(1H,s), 8.49 (1 H, d, J = 3.5 Hz), 7.75(2H,m), 7.60-7.51 (2H, m), 7.40-7.35(2H,m), 4.48 (1.1H, d, J = 6.0 Hz).. |
| **7-4** | 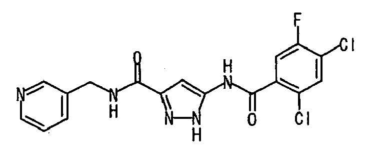 | ¹H-NMR(400 MHz, DMSO-*d*₆) δ: 13.28 (1H, s), 11.20 (1 H, s), 9.20 (1 H, t, J = 5.9 Hz), 8.55(1H,s), 8.48 (1H, d, J = 3.5 Hz), 7.93 (1 H, d, J = 6.5Hz), 7.79-7.73(2H,m), 7.40-7.35 (2H, m), 4.48 (2H, d, J = 5.8 Hz). |
| **7-5** | 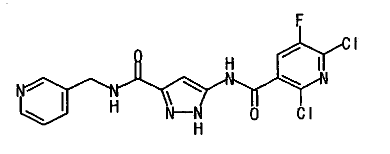 | ¹H-NMR(300 MHz, Dmso-*d*₆) δ: 13.32(1H,m), 11.32-11.27(1 H,m), 9.19(1H,m), 8.60-8.40(3H,m), 7.58(1H,m), 7.35(2H,m), 4.46(2H,s). |
| **7-6** | 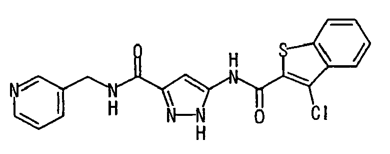 | ¹H-NMR(300 MHz, DMSO-*d*₆) δ: 9.10(1H,m), 8.55(1H,s), 8.47(1H,d,J=3.7Hz), 8.11(1H,m), 7.91(1H,m), 7.73(1H,d,J=8.1Hz), 7.60-7.57(2H,m), 7.39-7.35(2H,m), 4.47(2H,d,J=6.2Hz). |
| **7-7** | 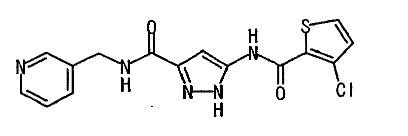 | ¹H-NMR(400 MHz, DMSO-*d*₆) δ: 13.30(1H,s), 10.62(1H,s), 9.14(1H,t,J=5.4Hz), 8.55(1H,s), 8.47(1H,d,J=4.5Hz), 7.89(1 H,d,J=5.5Hz)m 7.72(1 H,d,J=7.3Hz), 7.37(1H,dd,J=7.5,4.9Hz), 7.29(1H,s), |

**Table 63**

| **Example** | **Molecurar Structure** | **NMR** |
|---|---|---|
| **7-8** | 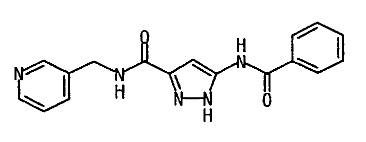 | ¹H-NMR(400 MHz, DMSO-*d*₆) δ: 13.33(1H,s), 11.06(1H.s). 9.19(1H,brs), 8.57(1h.s), 8.48 (1H, m), 8.02 (2H, d, J = 7.4 Hz), 7.74 (1 H, d, J = 7.7 Hz), 7.59-7.52 (3H, m), 7.37(2H,m), 4.48 (1H, s). |
| **7-9** | 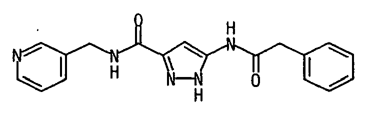 | ¹H-NMR(400 MHz, DMSO-*d*₆) δ: 13.12 (1H, s), 10.74 (1H, s), 9.08(1H.s). 8.52 (1H, s), 8.46(1H,s), 7.70 (1H, d, J = 7.4 Hz), 7.32-7.12 (7H, m), 4.44 (2H, d, J = 4.9 Hz), 3.63 (2H, s). |
| **7-10** | 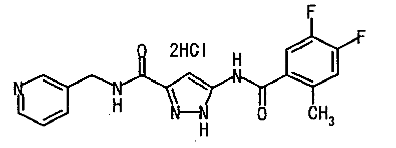 | ¹H-NMR(400 MHz, DMSO-d₆) δ: 11.06 (1H, s), 9.32(1.0H,s), 8.88 (2H, t, J= 11.0 Hz), 8.53 (1H, d, J = 8.1 Hz), 8.06 (1H. dd, J = 8.0, 5.7 Hz), 7.63 (1H, m), 7.40(1H,m),7.20 (1H, s), 4.66 (2H, d, J = 6.0 Hz),, 2.37 (3H, s). |
| **7-11** | 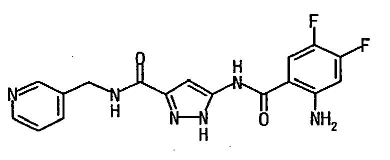 | ¹H-NMR(300 MHz, DMSO-*d*₆) δ:13.24(1H,s),10.69(1H,s), 9.15(1H,s), 8.56(1H,s), 8.48(1H,s), 7.79(1H,m), 7.72(1H, m), 7.38(1H,s), 7.27(1H,s), 6.67(3H,m),4.46(2h,s). |
| **7-12** | 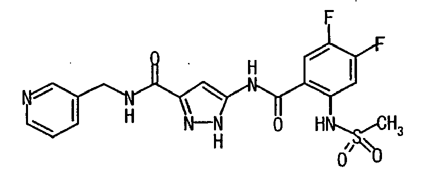 | ¹H-NMR(400 MHz, DMSO-*d*₆) δ: 11.31 (1H, s), 9.25(1H,brs), 8.77(1H,s), 8.72 (1H, d, J= 4.6 Hz), 8.25(1H, d, J=7.8Hz), 8.14(1H, dd, J=11.4, 8.8Hz), 7.83(1 H,dd, J=7.9, 5.3Hz), 7,52(1 H,dd, J=12.3, 7.9Hz), 7.26(1 H,brs),4.58 (2H, d, J = 5.8 Hz) 3.19(3H,s). |
| **7-13** | 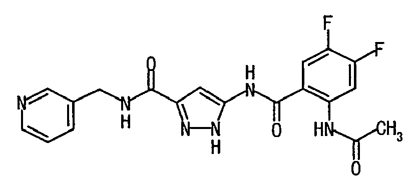 | ¹H-NMR(400 MHz, DMSO-*d*₆) δ: 13.34 (1H, s), 11.22 (1H, s), 10.83 (1 H, s), 9.19 (1H, t, J = 5.9 Hz), 8.56(1H,s), 8.46(1H,m), 8.30(1H,m), 7.72 (1H, d, J = 7.9 Hz), 7.38-7.32 (2H, m), 4.458 (2H, d, J = 5.8 Hz), 2.09(3H,s). |
| **7-14** | 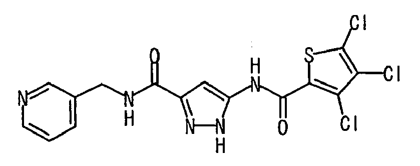 | ¹H-NMR(400 MHz, DMSO-*d*₆) δ: 13.48(1H,brs). 10.99 (1H, s), 9.17(1H,s), 8.57 (1H, d, J=l.6Hz), 8.49(1H,m), 7.74 (H, dt, J = 7.8,1.8 Hz), 7.39 (1H,dd, J = 7.8, 4.8 Hz), 7.29(1H,m), 4.50 (2H, t, J = 8.9 Hz). |

**Table 64**

| **Example** | **Molecurar Structure** | **NMR** |
|---|---|---|
| **7-15** | 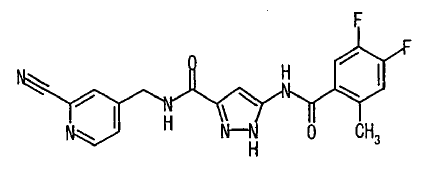 | ¹H-NMR(400 MHz, DMSO-d₆) δ:13.27(1H,s), 10.99(1 H,s), 9.25(1 1 H,t,J=6.OHz), 8.71(1H,d,J=5.2Hz), 7.90(1H,m), 7.66-7.54(2H,m), 7.40(2H,m), 4.45(2H,d,J=5.6Hz). |
| **7-16** | 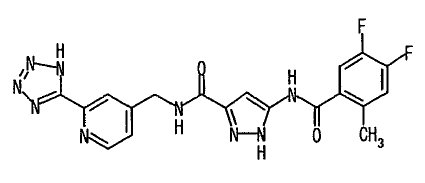 | ¹H-NMR(400 MHz, DMSO-*d*₆) δ: 13.28(1H,s), 10.98(1H,s), 9.31(1H,s), 8.54(1H,s), 8.00(1h,s), 7.59(1H,m), 7.38(2H,m), 7.27(1 H,m), 4.53(2H,m). |
| **7-17** | 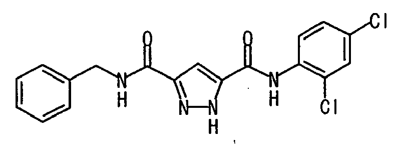 | ¹H-NMR(400 MHz, DMSO-*d*₆) δ: 9.70(1 H,s),9.23(1 H,s),8.12(1 H,d,J=8.6H z), 7.76(1 H,s), 751-7.27(7H,m)4.51 (2H,d,J=5.3Hz). |

### [Pharmacological test]

### 1. Exemplary test (1) Method for measuring liver glycogen phosphorylase activity

The measurement of glycogen phosphorylase activity was carried out by a method using the forward reaction system.

The measurement of glycogen phosphorylase activity using the forward reaction system was carried out as follows. Glucose-1-phosphate prepared from glycogen by glycogen phosphorylase was converted into glucono-δ-lactone 6-phosphatethrough transphosphorylation and dehydrogenation using phosphoglucomutase and glucose-6-phosphate dehydrogenase (G6PDH) . NADPH generated from NADP during the dehydrogenation by G6PDH was detected.

A homogenate of the Sf 9 cells which expressed exogenously recombinant human liver glycogen phosphorylase was diluted with 100 mmol/LBES buffer solution (pH 6.8, containing 2 mmol/L EDTA) and the dilution was used as the enzyme solution of human liver glycogen phosphorylase. As the substrate, a phosphate buffer (containing 16 mmol/L KH₂PO₄, 24 mmol/L Na₂HPO₄) was used. A mixture of 8U/mL phosphoglucomutase and 60U/mL G6PDH was prepared in BES buffer solution. Buffer solution (containing 1.4 mmol/L NADP, 30 mmol/L MgCl₂, 8 µ mol/L glucose-1,6-diphosphate,8mg/mL glycogen, 40mmo1/L BES, 0.8 mmol/L EDTA) for reaction and a glucose solution (containing 75 mmol/L glucose, 100 mmol/L BES, 2 mmol/L EDTA) were prepared individually. The test compound was dissolved in 1% DMSO solution.

The enzymatic reaction was begun by adding 20 µL of the recombinant human liver glycogen phosphorylase solution and 20 µL of the mixture of phosphoglucomutase and G6PDH to the mixture of 20 µL of the glucose solution, 20 µL of the substrate, 100 µL of the buffer solution for reaction and 20 µL of the test compound solution. As control, 1% DMSO solution was added instead of the test compound. The mixture without the substrate was used as blank. The absorbance at 340 nm was measured immediately after the reaction started. The absorbance at 340 nm was measured again after reacting at room temperature for 75 minutes . The enzymatic activity is a value obtained by subtracting the blank variation in absorbance over 75 minutes from the variation in absorbance over 75 minutes.

### 2. Exemplary test (2) Method for measuring blood plasma glucose concentration

Using db/db mice representing an obesity type of diabetic model were used to examine the effect of compound (1) of the present invention on glucose concentrations in plasma. Plasma glucose concentrations of the db/db mice (9-18 weeks old) were measured, and the mice were divided into groups each of 5 or 8 mice such that there would be no difference in the average value of plasma glucose concentrations. After fasting for four hours, the example compound or the solvent (0.5% methylcellulose) was administered orally to the db/db mice, and plasma glucose concentrations were measured 1 and 3 hours after administration. The analysis of the hypoglycemic effect of the example compound was performed through a statistical test at every hour between the group receiving the solvent and the groups of example compound (Dunnett test).

Test results of the exemplary test described above are shown in the following tables.

The inhibition rate (%) of the test compound was calculated by the expression " (1 - enzymatic activity of test compound /enzymatic activity of control) x 100."

A linear equation was determined from the two concentration points with the 50% inhibition rate therebetween, and the IC₅₀ value was calculated from the concentration at the intersection of the line with the 50% inhibition rate.

"IC₅₀" in the tables indicates the above-described enzyme inhibitory activity with respect to liver glycogen phosphorylase, and the activity to inhibit liver glycogen phosphorylase was represented as ++ when the IC₅₀ value (nmol/L) was less than 100 nmol/L, as + when the IC₅₀ value was between 100 nmol/L and 300 nmol/L. When IC₅₀ is 300 nmol/L or more, an inhibition rate (%) at a certain concentration of the compound (I) of the present invention is shown in Tables. For example, when the inhibition rate is 20% at a concentration of 300 nmol, the rate is described as 20%(300).

Further, "vivo" indicates hypoglycemic effect in db/db mice and compounds exhibiting statistically significant hypoglycemic effect at a dose of 10 mg/kg or less are indicated with the symbol "++" , and compounds exhibiting not significant but obvious hypoglycemic effect at a dose of 10 mg/kg are indicated with the symbol "+".

**Table 65**

| **Example** | **IC₅₀** | **vivo** |
|---|---|---|
| 1-1 | ++ | |
| 1-1-1 | ++ | ++ |
| 1-1-2 | ++ | |
| 1-2 | ++ | ++ |
| 1-2-1a | ++ | ++ |
| 1-2-2 | ++ | |
| 1-3-1 | ++ | ++ |
| 1-4 | ++ | |
| 1-5 | ++ | |
| 1-6 | + | |
| 1-7 | + | |
| 1-8 | ++ | |
| 1-9 | ++ | |
| 1-10 | + | |
| 1-11 | + | |
| 1-12 | ++ | ++ |
| 1-13 | ++ | |
| 1-14 | ++ | |
| 1-15 | ++ | + |
| 1-16 | ++ | |
| 1-17 | ++ | |
| 1-18 | ++ | + |
| 1-19 | ++ | + |
| 1-20 | ++ | |
| 1-21 | ++ | + |
| 1-22 | ++ | |
| 1-23 | ++ | |
| 1-24 | ++ | + |
| 1-25 | ++ | + |
| 1-26 | ++ | + |
| 1-27 | ++ | |
| 1-28 | ++ | |
| 1-29 | ++ | |
| 1-30 | ++ | |
| 1-31 | ++ | + |
| 1-32 | ++ | |
| 1-33 | ++ | |
| 1-34 | ++ | |
| 1-35 | ++ | |

**Table 66**

| **Example** | **IC₅₀** | **vivo** |
|---|---|---|
| 1-36 | ++ | |
| 1-37 | ++ | + |
| 1-38 | ++ | + |
| 1-39 | ++ | + |
| 1 -40 | ++ | + |
| 1-41 | ++ | + |
| 1-42 | ++ | + |
| 1-43 | ++ | |
| 1-44 | ++ | |
| 1-45 | ++ | |
| 1-46 | ++ | ++ |
| 1-47 | ++ | |
| 1-48 | ++ | |
| 1-49 | ++ | |
| 1-50 | ++ | + |
| 1-51 | ++ | |
| 1-52 | ++ | + |
| 1-53 | ++ | |
| 1-54 | ++ | |
| 1-55 | ++ | |
| 1-56 | ++ | + |
| 1-57 | ++ | |
| 1-58 | ++ | |
| 1-59 | ++ | ++ |
| 1-60 | ++ | |
| 1-61 | ++ | |
| 1-62 | ++ | |
| 1-63 | ++ | + |
| 1-64 | ++ | ++ |
| 1-65 | ++ | |
| 1-66 | ++ | + |
| 1-67 | ++ | + |
| 1-68 | ++ | + |
| 1-69 | ++ | + |
| 1-70 | ++ | ++ |
| 1-71 | ++ | + |
| 1-72 | ++ | + |
| 1-73 | ++ | + |
| 1-74 | + | |
| 1-75 | ++ | |

**Table 67**

| Example | IC₅₀ | vivo |
|---|---|---|
| 1-76 | ++ | |
| 1-77 | 43.8%(1000) | |
| 1-78 | ++ | + |
| 1-79 | 3.3%(300) | |
| 1-80 | ++ | + |
| 1-81 | ++ | + |
| 1-82 | ++ | + |
| 1-83 | 6.1%(300) | |
| 1-84 | ++ | ++ |
| 1-85 | ++ | |
| 1-86 | 17.0%(300) | |
| 1-87 | ++ | + |
| 1-88 | ++ | + |
| 1-89 | ++ | |
| 1-90 | ++ | + |
| 1-91 | ++ | + |
| 1-92 | 11.7%(300) | |
| 1-93 | ++ | |
| 1 -94 | 36.5%(300) | |
| 1 -95 | ++ | + |
| 1-96 | ++ | + |
| 1 -97 | ++ | |
| 1 -98 | ++ | + |
| 1 -99 | ++ | + |
| 1 -100 | ++ | + |
| 1 -101 | ++ | |
| 1-102 | ++ | |
| 1-103 | 21.6%(300) | |
| 1-104 | 32.3%(300) | |
| 1-105 | + | |
| 1-106 | + | |
| 1-107 | ++ | ++ |
| 1-108 | 45.3%(300) | |
| 1-109 | 33.3%(300) | |
| 1-110 | ++ | |
| 1-111 | + | |
| 1-112 | 49.9%(300) | |
| 1-113 | ++ | ++ |
| 1-114 | 38.9%(300) | |
| 1-115 | 39.7%(300) | |

**Table 68**

| **Example** | **IC₅₀** | **vivo** |
|---|---|---|
| 1-116 | ++ | ++ |
| 1-117 | ++ | + |
| 1-118 | ++ | |
| 1-119 | + | |
| 1-120 | ++ | |
| 1-121 | ++ | |
| 1-122 | ++ | |
| 1-123 | ++ | + |
| 1-124 | ++ | |
| 1-125 | ++ | |
| 1-126 | ++ | + |
| 1-127 | ++ | + |
| 1-128 | ++ | + |
| 1-129 | ++ | + |
| 1-130 | ++ | + |
| 1-131 | ++ | |
| 1-132 | ++ | |
| 1-133 | ++ | + |
| 1-134 | ++ | + |
| 1-135 | ++ | + |
| 1-136 | ++ | + |
| 1-137 | ++ | + |
| 1-138 | ++ | + |
| 1-139 | 22.9%(100) | |
| 1-140 | 30.8%(100) | |
| 1-141 | 31.4%(100) | |
| 1-142 | ++ | |
| 1-143 | ++ | |
| 1-144 | 5%(100) | |
| 1-145 | 12.8%(100) | |
| 1-146 | 11.3%(100) | |
| 1-147 | 9.6%(100) | |
| 1-148 | 14.1%(100) | |
| 1-149 | 20.8%(100) | |
| 1-150 | 13.8%(100) | |
| 1-151 | 13.3%(100) | |
| 1-152 | ++ | |
| 1-153 | ++ | |
| 1-154 | ++ | |
| 1-155 | ++ | |

**Table 69**

| **Example** | **IC₅₀** | **vivo** |
|---|---|---|
| 1-156 | ++ | |
| 1-157 | ++ | |
| 1-158 | ++ | |
| 1-159 | + | |
| 1-160 | ++ | |
| 1-161 | ++ | |
| 1-162 | ++ | |
| 1-163 | ++ | |
| 1-164 | ++ | |
| 1-165 | ++ | |
| 1-166 | ++ | |
| 1-167 | ++ | |

**Table 70**

| **Example** | **IC₅₀** | **vivo** |
|---|---|---|
| 2-1 | ++ | |
| 2-1-1 | ++ | + |
| 2-2-1 | ++ | |
| 2-3-1 | ++ | |
| 2-4-1 | ++ | ++ |
| 2-5 | ++ | ++ |
| 2-6 | ++ | ++ |
| 2-6-1 | ++ | ++ |
| 2-7-1 | ++ | ++ |
| 2-8 | ++ | + |
| 2-9 | ++ | + |
| 2-10 | ++ | + |
| 2-11 | ++ | + |
| 2-12 | ++ | ++ |
| 2-13 | ++ | + |
| 2-14 | 37.5%(300) | |
| 2-15 | ++ | + |
| 2-16 | ++ | |
| 2-17 | ++ | |
| 2-18 | ++ | |
| 2-19 | ++ | |
| 2-20 | 43.1%(100) | |
| 2-21 | 5.7%(100) | |
| 2-22 | ++ | |
| 2-23 | ++ | + |
| 2-24 | ++ | + |
| 2-25 | ++ | + |
| 2-26 | 36.4%(100) | |
| 2-27 | ++ | + |
| 2-28 | ++ | + |
| 2-29 | 45.8%(100) | |
| 2-30 | 1.8%(100) | |
| 2-31 | 5.5%(100) | |
| 2-32 | ++ | |
| 2-33 | ++ | |

**Table 71**

| **Example** | **IC₅₀** | **vivo** |
|---|---|---|
| 3-1 | 50%(490) | |
| 3-2 | + | + |
| 3-3 | 50%(640) | |
| 3-4 | + | + |
| 3-5 | 41.1%(1000) | |
| 3-6 | 42.5%(1000) | |
| 3-8 | 11.7%(1000) | |
| 3-9 | 50%(480) | |
| 3-10 | 23.2%(1000) | |
| 3-11 | ++ | + |
| 3-12 | 50%(400) | |
| 3-13 | 25.5%(1000) | |
| 3-14 | 41.7%(1000) | |
| 3-15 | + | |
| 3-16 | ++ | |
| 3-17 | ++ | |
| 3-18 | + | |
| 3-19 | 9.2%(1000) | |
| 3-20 | ++ | + |
| 3-21 | ++ | ++ |
| 3-22 | 50%(640) | |
| 3-23 | 26.1%(1000) | |
| 3-24 | ++ | + |
| 3-25 | ++ | + |
| 3-26 | ++ | + |
| 3-27 | 41.5%(300) | |
| 3-28 | + | |
| 3-29 | ++ | + |
| 3-30 | ++ | |
| 3-31 | ++ | + |
| 3-32 | ++ | + |
| 3-33 | ++ | + |
| 3-34 | ++ | |
| 3-35 | ++ | ++ |
| 3-36 | ++ | |
| 3-37 | ++ | |
| 3-38 | ++ | + |
| 3-39 | ++ | + |
| 3-40 | ++ | ++ |

**Table 72**

| **Example** | **IC₅₀** | **vivo** |
|---|---|---|
| 3-41 | ++ | ++ |
| 3-42 | 1.1%(300) | |
| 3-43 | 13.4%(300) | |
| 3-44 | + | |
| 3-45 | ++ | + |
| 3-46 | ++ | + |
| 3-47 | ++ | |
| 3-48 | ++ | |
| 3-49 | ++ | |
| 3-50 | ++ | |
| 3-51 | ++ | |
| 3-52 | ++ | |
| 3-53 | ++ | |

**Table 73**

| **Example** | **IC₅₀** | **vivo** |
|---|---|---|
| 4-1 | 13.8%(1000) | |
| 4-2 | ++ | ++ |
| 4-2-1 | ++ | ++ |
| 4-3 | + | |
| 4-4 | ++ | + |
| 4-5 | + | |
| 4-6 | + | |
| 4-7 | + | |
| 4-8 | + | |
| 4-9 | ++ | + |
| 4-10 | 50%(590) | |
| 4-11 | ++ | |
| 4-12 | + | |
| 4-13 | 38.0%(300) | |
| 4-14 | 10.6%(300) | |
| 4-15 | + | |
| 4-16 | 5.1%(300) | |
| 4-17 | 1.1%(300) | |
| 4-18 | ++ | + |
| 4-19 | 3.3%(300) | |
| 4-20 | 3.3%(300) | |
| 4-21 | 24.2%(300) | |
| 4-23 | ++ | + |
| 4-24 | + | |
| 4-25 | ++ | |
| 4-26 | ++ | |
| 4-27 | + | |
| 4-28 | + | |
| 4-32 | 7.8%(300) | |
| 4-33 | 15.2%(300) | |
| 4-34 | ++ | + |
| 4-35 | 9.7%(300) | |
| 4-39 | 5.3%(300) | |
| 4-40 | 3.6%(300) | |
| 4-41 | ++ | + |
| 4-42 | 8.5%(300) | |
| 4-43 | 3.8%(300) | |
| 4-44 | 5.7%(100) | |

**Table 74**

| **Example** | **IC₅₀** | **vivo** |
|---|---|---|
| 4-45 | 5.9%(100) | |
| 4-46 | ++ | + |
| 4-47 | 1.2%(100) | |
| 4-48 | ++ | ++ |

**Table 75**

| **Example** | **IC₅₀** | **vivo** |
|---|---|---|
| 5-1 | ++ | + |
| 5-2 | ++ | + |
| 5-3 | ++ | |
| 5-4 | ++ | |
| 5-5 | ++ | |
| 5-6 | ++ | |
| 5-7 | ++ | |
| 5-8 | ++ | + |
| 5-9 | ++ | + |
| 5-10 | ++ | + |
| 5-11 | ++ | |
| 5-12 | ++ | + |
| 5-13 | ++ | + |
| 5-14 | ++ | + |
| 5-15 | ++ | + |
| 5-16 | ++ | + |
| 5-17 | ++ | + |
| 5-18 | ++ | |
| 5-19 | ++ | |
| 5-20 | ++ | |
| 5-21 | ++ | |
| 5-22 | ++ | |
| 5-23 | ++ | ++ |
| 5-24 | ++ | ++ |
| 5-25 | ++ | ++ |
| 5-26 | ++ | |
| 5-27 | ++ | ++ |
| 5-28 | ++ | + |
| 5-29 | ++ | ++ |
| 5-30 | ++ | + |
| 5-31 | ++ | + |
| 5-32 | ++ | + |
| 5-33 | ++ | + |
| 5-34 | ++ | |

**Table 76**

| **Example** | **IC₅₀** | **vivo** |
|---|---|---|
| 5-35 | ++ | + |
| 5-36 | 17.8%(100) | |
| 5-37 | 22.3%(100) | |
| 5-38 | 15.2%(100) | |
| 5-39 | ++ | |
| 5-40 | ++ | |
| 5-41 | ++ | + |
| 5-42 | ++ | + |
| 5-43 | ++ | + |
| 5-44 | ++ | + |

**Table 77**

| **Example** | **IC₅₀** | **vivo** |
|---|---|---|
| 6-1 | 15.5%(100) | |
| 6-2 | 12.3%(300) | |
| 6-3 | 9.7%(300) | |
| 6-4 | 46.8%(300) | |
| 6-5 | ++ | |
| 6-6 | ++ | |
| 6-7 | 12.9%(300) | |
| 6-8 | 11.7%(300) | |
| 6-9 | 5.5%(300) | |
| 6-10 | + | |
| 6-11 | 2.1%(100) | |
| 6-12 | ++ | + |
| 6-13 | 17.5%(100) | |
| 6-14 | 20.1%(100) | |
| 6-15 | ++ | |
| 6-16 | ++ | + |
| 6-17 | 9.8%(100) | + |
| 6-18 | 6.8%(100) | |
| 6-19 | ++ | |
| 6-20 | ++ | |
| 6-21 | ++ | ++ |
| 6-22 | ++ | + |
| 6-23 | ++ | + |
| 6-24 | ++ | |
| 6-25 | ++ | |
| 6-26 | ++ | |
| 6-27 | ++ | |
| 6-28 | ++ | |
| 6-29 | ++ | |
| 6-30 | ++ | |
| 6-31 | ++ | |
| 6-32 | ++ | |
| 6-33 | ++ | + |
| 6-34 | ++ | |
| 6-35 | ++ | |
| 6-36 | ++ | |

**Table 78**

| **Example** | **IC₅₀** | **vivo** |
|---|---|---|
| 6-37 | ++ | |
| 6-38 | ++ | |
| 6-39 | ++ | + |
| 6-40 | ++ | |
| 6-41 | 12.3%(100) | |
| 6-42 | 21.7%(100) | |
| 6-43 | ++ | |
| 6-44 | ++ | |
| 6-45 | ++ | |
| 6-46 | ++ | |
| 6-47 | 41.1%(100) | |
| 6-48 | ++ | ++ |
| 6-49 | ++ | |
| 6-50 | ++ | |

**Table 79**

| **Example** | **IC₅₀** | **In vivo** |
|---|---|---|
| 7-1 | ++ | + |
| 7-2 | ++ | |
| 7-3 | ++ | |
| 7-4 | ++ | ++ |
| 7-5 | 8.5%(300) | |
| 7-7 | 2.9%(300) | |
| 7-8 | 6.8%(300) | |
| 7-10 | ++ | ++ |
| 7-11 | 47.3%(100) | |
| 7-12 | 8.0%(100) | |
| 7-13 | 1.9%(100) | |
| 7-14 | 4.6%(100) | |
| 7-15 | ++ | |
| 7-16 | ++ | |

### Industrial Applicability

As is clear from the test results described above, the compounds of the present invention and pharmacologically acceptable salts thereof had potent inhibitory effects on human liver glycogen phosphorylase. Therefore, the compounds of the present invention are useful as a novel antidiabetic based on the new mode of action of GP inhibitory effect. As well as having the potential as a combination therapy with other antidiabetic or antilipidemic drugs, the compound of the present invention has the potential as a therapeutic agent for insulin resistance, diabetic neuropathy, diabetic nephropathy, diabetic retinopathy, cataract, hypercholesterolemia, hypertension, hyperinsulinemia, hyperlipidemia, atherosclerosis, tissue ischemia and myocardial ischemia, a therapeutic agent for appetite control and obesity, and a therapeutic agent for infections such as bacterial, fungal, parasitic or viral infection.

## Claims

1. A pharmaceutical composition for treating or preventing diabetes, comprising a pyrazole compound represented by the following general formula (I): 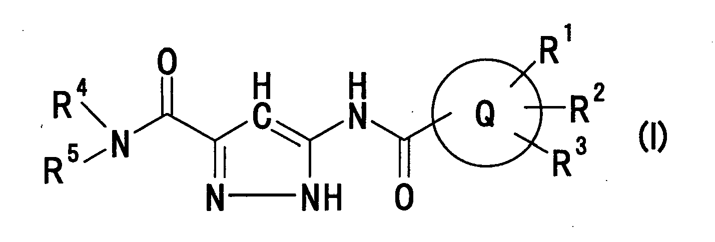 wherein Ring Q represents an aryl group or a heteroaromatic group;
R¹ represents a hydrogen atom, a halogen atom, a C₁₋₆ alkyl group or a C₁₋₆ alkoxy group;
R² represents a halogen atom, a C₁₋₆ alkyl group, a C₁₋₆ alkoxy group or an azido group;
R³ represents a halogen atom, a hydroxyl group, a C₁₋₆ alkyl group, a halo C₁₋₆ alkyl group, a C₁₋₆ alkoxy group, an azido group, an amino group, an acylamino group or a C₁₋₆ alkylsulfonylamino group;
R⁴ and R⁵ are identical with or different from each other, and represent
(1) a hydrogen atom;
(2) a C₁₋₆ alkyl group that may be substituted with one or more substituents selected from Group A below:
[Group A]
A1. a hydroxyl group,
A2. a C₁₋₆ alkoxy group,
A3. -N (R⁴¹) (R^{41'})
wherein R⁴¹ and R^{41'} are identical with or different from each other and represent a hydrogen atom or a C₁₋₆ alkyl group, or R⁴¹ and R⁴¹' may, together with the adjacent nitrogen atom, form a saturated 5- or 6-memberedmonocyclic heterocyclic ring,
A4. an aryl group,
A5. a heteroaromatic group,
A6. -CO-N (R⁴¹¹) (R^{411'})
wherein R⁴¹¹ and R^{411'} are identical with or different from each other and represent a hydrogen atom or a C₁₋₆ alkyl group, or R⁴¹¹ and R^{411'} may, together with the adjacent nitrogen atom, forma saturated 5- or 6-memberedmonocyclic heterocyclic ring,
A7. a carboxy group,
and,
A8. a C₁₋₆ alkoxycarbonyl group,
(3) a C₂₋₆ alkenyl group;
(4) a C₂₋₆ alkynyl group;
(5) a C₃₋₈ cycloalkyl group;
wherein the cycloalkyl group may be substituted with a hydroxyl group, a carboxy group or a C₁₋₆ alkoxycarbonyl group or may be condensed with a pyridine ring,
(6) a C₃₋₈ cycloalkyl C₁₋₆ alkyl group;
wherein the cycloalkyl group of the C₃₋₈ cycloalkyl C₁₋₆ alkyl group may be substituted with a hydroxyl group, a carboxy group or a C₁₋₆ alkoxycarbonyl group or may be condensed with a pyridine ring,
(7) a saturated 5- or 6-memberedmonocyclic heterocyclic group that may be substituted with one or more substituents selected from Group C below:
[Group C]
C1. a C₁₋₆ alkyl group,
C2. an acyl group,
C3. a C₁₋₆ alkylsulfonyl group,
C4. a carboxy group,
C5. a C₁₋₆ alkoxycarbonyl group,
and,
C6 . -CO-(Alk)n-COOR⁵²,
wherein R⁵² is a hydrogen atom or a C₁₋₆ alkyl group, Alk is a C₁₋₄ alkylene group, and n is 0 or an integer from 1 to 3,
(8) an aryl group that may be substituted with one or more substituents selected from Group D below:
[Group D]
D1. a hydroxyl group,
D2. a C₁₋₆ alkoxy group,
D3. a cyano group,
D4. a C₁₋₆ alkyl group,
wherein the C₁₋₆ alkyl group may be substituted with one or more substituents selected from the group consisting of a hydroxyl group, a carboxy group and a C₁₋₆ alkoxycarbonyl group,
D5. -N (R⁵³) (R^{53'}),
wherein R⁵³ and R^{53'} are identical with or different from each other and represent a hydrogen atom, a C₁₋₆ alkyl group or a C₁₋₆ alkylsulfonyl group,
D6. -CO-N(R⁵³¹) (R^{531'})
wherein R⁵³¹ and R^{531'} are identical with or different from each other and represent a hydrogen atom, a C₁₋₆ alkyl group or a C₁₋₆ alkylsulfonyl group,
D7. -COOR⁵⁴,
wherein R⁵⁴ is a hydrogen atom, a C₁₋₆ alkyl group, a C₁₋₆ alkylcarbonyloxy C₁₋₆ alkyl group or a C₃₋₈ cycloalkyloxycarbonyloxy C₁₋₆ alkyl group,
D8. -C(NH(OH))=N-R⁵⁵,
wherein R⁵⁵ is a hydrogen atom or a C₁₋₆ alkylsulfonyl group,
D9. a saturated 5- or 6-membered monocyclic heterocyclic group,
and,
D10. a 5- or 6-membered monocyclic heteroaromatic group that may be substituted with an oxo or thioxo group,
(9) a 5- or 6-membered monocyclic heteroaromatic group or a 5- or 6-membered condensed heteroaromatic group with a benzene ring, wherein the 5- or 6-membered heteroaromatic groups may be substituted with one or more substituents selected from the group consisting of a carboxy group and a C₁₋₆ alkoxycarbonyl group;
(10) a C₇₋₁₄ aralkyl group;
wherein the alkyl moiety of the C₇₋₁₄ aralkyl group may be substituted with one or two substituents selected from Group E below, and the aryl moiety may be substituted with one or more substituents selected from Group F below:
[Group E]
E1. a C₁₋₆ alkyl group that may be substituted with a hydroxyl group,
E2. a cyano group,
E3. a carboxy group,
E4. a C₁₋₆ alkoxycarbonyl group,
and,
E5. a phenyl group,
[Group F]
F1. a C₁₋₆ alkyl group,
F2. a halogen atom,
F3. a cyano group,
F4. a hydroxyl group,
F5. a C₁₋₆ alkoxy group that may be substituted with one or more substituents selected from the group consisting of a carboxy group and a C₁₋₆ alkoxycarbonyl group,
F6. a halo C₁₋₆ alkyl group,
F7. a carboxy group,
F8. a C₁₋₆ alkoxycarbonyl group,
F9. -CO-N(R^{56a}) (R^{56a'})
wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f below:
[Group f]
f1. an amino group,
f2. a mono C₁₋₆ alkylamino group,
f3. a di C₁₋₆ alkylamino group,
f4. a carboxy group,
f5. a C₁₋₆ alkoxycarbonyl group,
f6. a hydroxyl group,
and,
f7. a saturated 5- or 6-membered monocyclic heterocyclic group having at least one nitrogen atom,
F10 . -N (R^{56b}) (R^{56b'}),
wherein R^{56b} and R^{56b}' are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
F11. -N=CR⁵⁷(-N(R⁵⁸) (R^{58'})),
wherein R⁵⁷ is a hydrogen atom or a C₁₋₆ alkyl group, R⁵⁸ and R^{58'} are identical with or different from each other and represent a hydrogen atom or a C₁₋₆ alkyl group,
F12. a 5- or 6-membered monocyclic heteroaromatic group, and,
F13. a methylenedioxy group or an ethylenedioxy group, or,
(11) a C₁₋₆ alkyl group substituted with one or more identical or different substituents selected from the group consisting of a 5- or 6-membered monocyclic heteroaromatic group and a 5- or 6-membered condensed heteroaromatic group with a benzene ring;
wherein the heteroaromatic group may be substituted with one or more substituents selected from Group G below:
[Group G]
G1. a C₁₋₆ alkyl group that may be substituted with a one or more substituents selected from Group g below:
[Group g]
g1. a halogen atom,
g2. an amino group,
g3. a mono C₁₋₆ alkylamino group,
g4. a di C₁₋₆ alkylamino group,
g5. a C₁₋₆ alkoxycarbonylamino group,
and,
g6. a hydroxyimino group,
G2. a halogen atom,
G3. a C₁₋₆ alkoxy group that may be substituted with a halogen atom,
G4. an aryloxy group,
G5. a cyano group,
G6. -N (R^{59a}) (R^{59a'}),
wherein R^{59a} and R^{59a'} are identical with or different from each other and represent a hydrogen atom or a C₁₋₆ alkyl group, or R^{59a} and R^{59a'} may, together with the adjacent nitrogen atom, forma saturated 5- or 6-memberedmonocyclic heterocyclic ring, and the saturated heterocyclic ring may be substituted with one or more identical or different substituents selected from the group consisting of a hydroxyl group, an amino group, a mono C₁₋₆ alkylamino group and a di C₁₋₆ alkylamino group,
G7. -CO-N(R^{59b}) (R^{59b'})
wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
G8. an aryl group,
and,
G9. a 5- or 6-membered monocyclic heteroaromatic group that may be substituted with an oxo or thioxo group, or
R⁴ and R⁵ may, together with the adjacent nitrogen atom, forma saturated 5- or 6-memberedmonocyclic heterocyclic ring, wherein a part of the saturated heterocyclic ring may have a double bond, and the saturated heterocyclic ring may be condensed with a benzene ring to form a condensed ring, the saturated heterocyclic ring which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof, and a pharmaceutically acceptable carrier.

2. The pharmaceutical composition for treating or preventing diabetes according to claim 1, comprisingapyrazole compound represented by the following general formula (I): 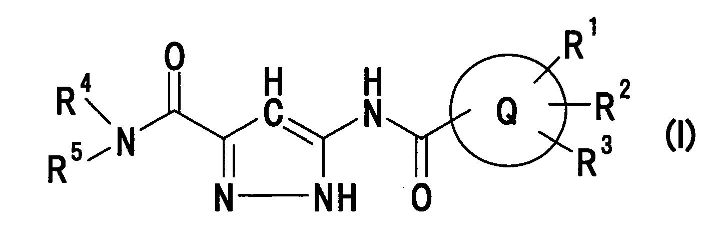 wherein Ring Q represents an aryl group or a heteroaromatic group;
R¹ represents a hydrogen atom, a halogen atom, a C₁₋₆ alkyl group or a C₁₋₆ alkoxy group;
R² represents a halogen atom, a C₁₋₆ alkyl group, a C₁₋₆ alkoxy group or an azido group;
R³ represents a halogen atom, a hydroxyl group, a C₁₋₄ alkyl group, a halo C₁₋₆ alkyl group, a C₁₋₆ alkoxy group, an azido group, an amino group, an acylamino group or a C₁₋₆ alkylsulfonylamino group;
R⁴ represents
(1) a hydrogen atom;
(2) a C₁₋₆ alkyl group that may be substituted with one or more substituents selected from Group A below:
[Group A]
A1. a hydroxyl group,
A2. a C₁₋₆ alkoxy group,
A3. -N(R⁴¹) (R^{41'})
wherein R⁴¹ and R^{41'} are identical with or different from each other and represent a hydrogen atom or a C₁₋₆ alkyl group, or R⁴¹ and R^{41'} may, together with the adjacent nitrogen atom, forma saturated 5- or 6-memberedmonocyclic heterocyclic ring,
A4. an aryl group,
A5. a heteroaromatic group,
A6. -CO-N(R⁴¹¹) (R^{411'})
wherein R⁴¹¹ and R^{411'} are identical with or different from each other and represent a hydrogen atom or a C₁₋₆ alkyl group, or R⁴¹¹ and R⁴¹¹' may, together with the adjacent nitrogen atom, forma saturated 5- or 6-memberedmonocyclic heterocyclic ring,
A7. a carboxy group,
and,
A8. a C₁₋₆ alkoxycarbonyl group,
(3) a C₂₋₆ alkenyl group;
(4) a C₂₋₆ alkynyl group;
(5) a C₃₋₈ cycloalkyl group;
(6) a C₃₋₈ cycloalkyl C₁₋₆ alkyl group;
or,
(7) an aryl group; and
R⁵ represents
(1) a C₁₋₆ alkyl group that may be substituted with one or more substituents selected from Group B below:
[Group B]
B1. a hydroxyl group,
B2. a C₁₋₆ alkoxy group,
B3. -N(R⁵¹) (R^{51'})
wherein R⁵¹ and R^{51'} are identical with or different from each other and represent a hydrogen atom or a C₁₋₆ alkyl group, or R⁵¹ and R^{51'} may, together with the adjacent nitrogen atom, forma saturated 5- or 6-memberedmonocyclic heterocyclic ring, and,
B4 . -CO-N (R⁵¹¹) (R^{511'})
wherein R⁵¹¹ and R^{511'} are identical with or different from each other and represent a hydrogen atom or a C₁₋₆ alkyl group, or R⁵¹¹ and R^{511'} may, together with the adjacent nitrogen atom, forma saturated 5- or 6-memberedmonocyclicheterocyclic ring,
(2) a C₃₋₈ cycloalkyl group;
wherein the cycloalkyl group may be substituted with a hydroxyl group, a carboxy group or a C₁₋₆ alkoxycarbonyl group or may be condensed with a pyridine ring,
(3) a C₃₋₈ cycloalkyl C₁₋₆ alkyl group;
wherein the cycloalkyl group of the C₃₋₈ cycloalkyl C₁₋₆ alkyl group may be substituted with a hydroxyl group, a carboxy group or a C₁₋₆ alkoxycarbonyl group or may be condensed with a pyridine ring,
(4) a saturated 5- or 6-memberedmonocyclic heterocyclic group that may be substituted with one or more substituents selected from Group C below:
[Group C]
C1. a C₁₋₆ alkyl group,
C2. an acyl group,
C3. a C₁₋₆ alkylsulfonyl group,
C4. a carboxy group,
C5. a C₁₋₆ alkoxycarbonyl group,
and,
C6. -CO-(Alk)n-COOR⁵²,
wherein R⁵² is a hydrogen atom or a C₁₋₆ alkyl group, Alk is a C₁₋₄ alkylene group, and n is 0 or an integer from 1 to 3,
(5) an aryl group that may be substituted with one or more substituents selected from Group D below:
[Group D]
D1. a hydroxyl group,
D2. a C₁₋₆ alkoxy group,
D3. a cyano group,
D4. a C₁₋₆ alkyl group,
wherein the C₁₋₆ alkyl group may be substituted with one or more substituents selected from the group consisting of a hydroxyl group, a carboxy group and a C₁₋₆ alkoxycarbonyl group,
D5. -N(R⁵³) (R^{53'}),
wherein R⁵³ and R⁵³' are identical with or different from each other and represent a hydrogen atom, a C₁₋₆ alkyl group or a C₁₋₆ alkylsulfonyl group,
D6. -CO-N (R⁵³¹) (R^{531'})
wherein R⁵³¹ and R^{531'} are identical with or different from each other and represent a hydrogen atom, a C₁₋₆ alkyl group or a C₁₋₆ alkylsulfonyl group,
D7. -COOR⁵⁴,
wherein R⁵⁴ is a hydrogen atom, a C₁₋₆ alkyl group, a C₁₋₆ alkylcarbonyloxy C₁₋₆ alkyl group or a C₃₋₈ cycloalkyloxycarbonyloxy C₁₋₆ alkyl group,
D8. -C(NH(OH))=N-R⁵⁵,
wherein R⁵⁵ is a hydrogen atom or a C₁₋₆ alkylsulfonyl group,
D9. a saturated 5- or 6-membered monocyclic heterocyclic group, and
D10. a 5- or 6-membered monocyclic heteroaromatic group that may be substituted with an oxo or thioxo group,
(6) a 5- or 6-membered monocyclic heteroaromatic group or a 5- or 6-membered condensed heteroaromatic group with a benzene ring, wherein the 5- or 6-membered heteroaromatic groups may be substituted with one or more substituents selected from the group consisting of a carboxygroup and a C₁₋₆ alkoxycarbonyl group;
(7) a C₇₋₁₄ aralkyl group;
wherein the alkyl moiety of the C₇₋₁₄ aralkyl group may be substituted with one or two substituents selected from Group E below, and the aryl moiety may be substituted with one or more substituents selected from Group F below:
[Group E]
E1. a C₁₋₆ alkyl group that may be substituted with a hydroxyl group,
E2. a cyano group,
E3. a carboxy group,
E4. a C₁₋₆ alkoxycarbonyl group,
and,
E5. a phenyl group,
[Group F]
F1. a C₁₋₆ alkyl group,
F2. a halogen atom,
F3. a cyano group,
F4. a hydroxyl group,
F5. a C₁₋₆ alkoxy group that may be substituted with one or more substituents selected from the group consisting of a carboxy group and a C₁₋₆ alkoxycarbonyl group,
F6. a halo C₁₋₆ alkyl group,
F7. a carboxy group,
F8. a C₁₋₆ alkoxycarbonyl group,
F9. -CO-N(R^{56a}) (R^{56a'})
wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f below:
[Group f]
f1. an amino group,
f2. a mono C₁₋₆ alkylamino group,
f3. a di C₁₋₆ alkylamino group,
f4. a carboxy group,
f5. a C₁₋₆ alkoxycarbonyl group,
f6. a hydroxyl group,
and,
f7. a saturated 5- or 6-membered monocyclic heterocyclic group having at least one nitrogen atom,
F10. -N (R^{56b}) (R^{56b'}),
wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
F11. -N=CR⁵⁷(-N(R⁵⁸) (R^{58'})),
wherein R⁵⁷ is a hydrogen atom or a C₁₋₆ alkyl group, R⁵⁸ and R^{58'} are identical with or different from each other and represent a hydrogen atom or a C₁₋₆ alkyl group,
F12. a 5- or 6-membered monocyclic heteroaromatic group, and,
F13. a methylenedioxy group or an ethylenedioxy group, or,
(8) a C₁₋₆ alkyl group substituted with a 5- or 6-membered monocyclic heteroaromatic group or a C₁₋₆ alkyl group substituted with a 5- or 6-membered condensed heteroaromatic group with a benzene ring;
wherein the heteroaromatic group may be substituted with one or more substituents selected from Group G below:
[Group G]
G1. a C₁₋₆ alkyl group that may be substituted with one or more substituents selected from Group g below:
[Group g]
g1. a halogen atom,
g2. an amino group,
g3. a mono C₁₋₆ alkylamino group,
g4. a di C₁₋₆ alkylamino group,
g5. a C₁₋₆ alkoxycarbonylamino group,
and
g6. a hydroxyimino group,
G2. a halogen atom,
G3. a C₁₋₆ alkoxy group that may be substituted with a halogen atom,
G4. an aryloxy group,
G5. a cyano group,
G6 . -N(R^{59a}) (R^{59a'}),
wherein R^{59a} and R^{59a'} are identical with or different from each other and represent a hydrogen atom or a C₁₋₆ alkyl group, or R^{59a} and R^{59a'} may, together with the adjacent nitrogen atom, form a saturated 5- or 6-membered monocyclic heterocyclic ring, and the saturated heterocyclic ring may be substituted with one or more identical or different substituents selected from the group consisting of a hydroxyl group, an amino group, a mono C₁₋₆ alkylamino group and a di C₁₋₆ alkylamino group,
G7. -CO-N (R^{59b}) (R^{59b'})
wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
G8. an aryl group,
and,
G9. a 5- or 6-membered monocyclic heteroaromatic group that may be substituted with an oxo or thioxo group, or
R⁴ and R⁵ may, together with the adjacent nitrogen atom, forma saturated 5- or 6-memberedmonocyclic heterocyclic ring, wherein a part of the saturated heterocyclic ring may have a double bond, and the saturated heterocyclic ring may be condensed with a benzene ring to form a condensed ring, and the saturated heterocyclic ring which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof, and a pharmaceutically acceptable carrier.

3. The pharmaceutical composition for treating or preventing diabetes according to claim 1, wherein the pyrazole compound is a pyrazole compound represented by the following general formula (II'): 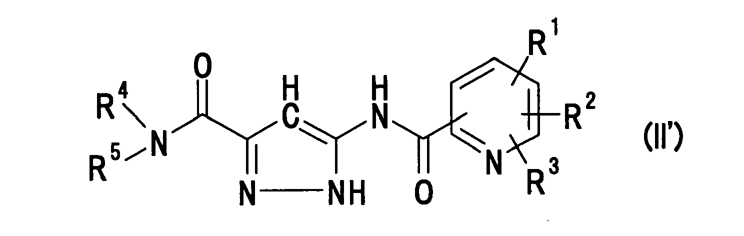 wherein R¹, R², R³, R⁴ and R⁵ are as defined in claim 1.

4. The pharmaceutical composition for treating or preventing diabetes according to claim 1, wherein the pyrazole compound is a pyrazole compound represented by the following general formula (II): 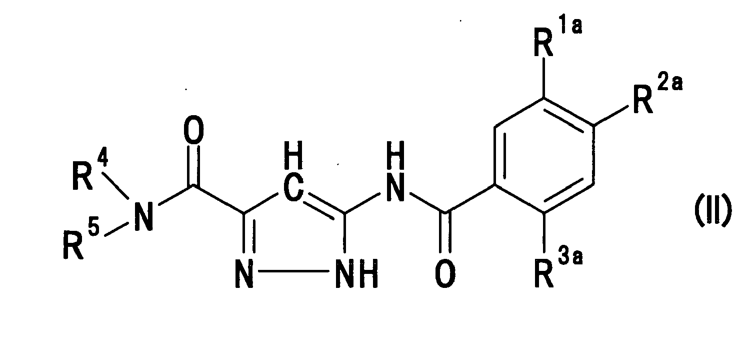 wherein R⁴ and R⁵ are as defined in claim 1, R^{1a} represents a halogen atom or a hydrogen atom, R^{2a} a halogen atom, and R^{3a} a halogen atom or a C₁₋₆ alkyl group .

5. The pharmaceutical composition for treating or preventing diabetes according to claim 4, wherein R^{1a} is a hydrogen or fluorine atom, R^{2a} is a fluorine or chlorine atom, and R^{3a} is a chlorine atom or a C₁₋₆ alkyl group.

6. The pharmaceutical composition for treating or preventing diabetes according to claim 5, wherein the pyrazole compound is a pyrazole compound represented by the following general formula (III): 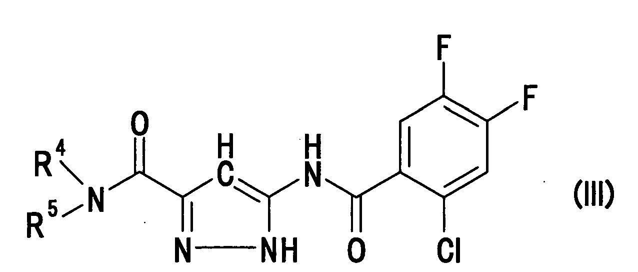 wherein R⁴ and R⁵ are as defined above.

7. The pharmaceutical composition for treating or preventing diabetes according to any of claims 2 to 6, wherein R⁴ is a group selected from the following group:
(1) a hydrogen atom;
(2) a C₁₋₆ alkyl group that may be substituted with one or more substituents selected from Group A' below:
[Group A']
A'1. a hydroxyl group,
A'2. a C₁₋₄ alkoxy group,
A'3. -N(R⁴¹) (R^{41'})
wherein R⁴¹ and R^{41'} are identical with or different from each other and represent a hydrogen atom or a C₁₋₄ alkyl group, or R⁴¹ and R^{41'} may, together with the adjacent nitrogen atom, forma saturated 5- or 6-memberedmonocyclic heterocyclic ring,
A'4. a phenyl group,
A'5. a 5- or 6-membered monocyclic heteroaromatic group,
A'6. -CO-N(R⁴¹¹) (R^{411'})
wherein R⁴¹¹ and R^{411'} are identical with or different from each other and represent a hydrogen atom or a C₁₋₄ alkyl group, or R⁴¹¹ and R^{411'} may, together with the adjacent nitrogen atom, forma saturated 5- or 6-memberedmonocyclic heterocyclic ring,
A'7. a carboxy group, and
A'8. a C₁₋₄ alkoxycarbonyl group,
(3) a C₂₋₆ alkenyl group;
(4) a C₂₋₆ alkynyl group;
(5) a C₃₋₈ cycloalkyl group;
and,
(6) a C₃₋₈ cycloalkyl C₁₋₄ alkyl group .

8. The pharmaceutical composition for treating or preventing diabetes according to any of claims 2 to 7, wherein R⁵ is a C₁₋₆ alkyl group.

9. The pharmaceutical composition for treating or preventing diabetes according to any of claims 2 to 7, wherein R⁵ is a C₃₋₈ cycloalkyl group that may be substituted with one or more identical or different substituents selected from the group consisting of a hydroxyl group, a carboxy group and a C₁₋₆ alkoxycarbonyl group.

10. The pharmaceutical composition for treating or preventing diabetes according to any of claims 2 to 7, wherein R⁵ is a C₃₋₈ cycloalkyl C₁₋₆ alkyl group.

11. The pharmaceutical composition for treating or preventing diabetes according to any of claims 2 to 7, wherein R⁵ is a saturated 5- or 6-membered monocyclic heterocyclic group that may be substituted with one or more substituents selected from the group consisting of an acyl group and -CO- (Alk) n-COOR⁵², wherein R⁵² is a hydrogen atom or a C₁₋₆ alkyl group, Alk is a C₁₋₄ alkylene group, and n is 0 or an integer from 1 to 3.

12. The pharmaceutical composition for treating or preventing diabetes according to any of claims 2 to 7, wherein R⁵ is a phenyl group that may be substituted with one or more substituents selected from the following Group D':
[Group D']
D'1. a C₁₋₄ alkyl group that may be substituted with a carboxy group,
D'2. -CO-N(R⁵³) (R^{53'}),
wherein R⁵³ and _{R}⁵³' are identical with or different from each other and represent a hydrogen atom, a C₁₋₄ alkyl group or a C₁₋₄ alkylsulfonyl group,
D'3. COOR⁵⁴,
wherein R⁵⁴ is a hydrogen atom, a C₁₋₄ alkyl group, a C₁₋₄ alkylcarbonyloxy C₁₋₄ alkyl group or a C₃₋₈ cycloalkyloxycarbonyloxy C₁₋₄ alkyl group,
and,
D'4. a 5- or 6-membered monocyclic heteroaromatic group that may be substituted with an oxo or thioxo group.

13. The pharmaceutical composition for treating or preventing diabetes according to any of claims 2 to 7, wherein R⁵ is a C₇₋₁₄ aralkyl group and the aryl moiety of the C₇₋₁₄ aralkyl group may be substituted with one ormore substituents selected from the following Group F':
[Group F']
F'1. a C₁₋₆ alkyl group,
F'2. a halogen atom,
F'3. a cyano group,
F'4. a hydroxyl group,
F'5. a C₁₋₆ alkoxy group that may be substituted with one or more substituents selected from the group consisting of a carboxy group and a C₁₋₆ alkoxycarbonyl group,
F'6. a halo C₁₋₆ alkyl group,
F'7. a carboxy group,
F'8. a C₁₋₆ alkoxycarbonyl group,
F'9. -CO-N(R^{56a}) (R^{56a'}),
wherein R^{56a} and R^{56a}' are identical with or different from each other and represent a hydrogen atom, a saturated 5- or 6-membered monocyclic heterocyclic group having at least one nitrogen atom, or a C₁₋₆ alkyl group that may be substituted with one or more substituents selected from the following Group f':
[Group f']
f'1. an amino group,
f'2. a mono C₁₋₆ alkylamino group,
f'3. a di C₁₋₆ alkylamino group,
f'4. a carboxy group,
f'5. a C₁₋₆ alkoxycarbonyl group,
f'6. a hydroxyl group,
and,
f'7. a saturated 5- or 6-membered monocyclic heterocyclic group having at least one nitrogen atom,
F'10. -N(R^{56b}) (R^{56b'})
wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
F'11. -N=CR⁵⁷(-N(R⁵⁸) (R^{58'})),
wherein R⁵⁷ is a hydrogen atom or a C₁₋₄ alkyl group, R⁵⁸ and R^{58'} are identical with or different from each other and represent a hydrogen atom or a C₁₋₄ alkyl group,
F'12. a 5- or 6-memberedmonocyclic heteroaromatic group, and,
F'13. a methylenedioxy group or an ethylenedioxy group .

14. The pharmaceutical composition for treating or preventing diabetes according to any of claims 2 to 7, wherein R⁵ is a C₁₋₆ alkyl group substituted with a 5- or 6-membered monocyclic heteroaromatic group and the heteroaromatic group may be substituted with one or more substituents selected from the following Group G':
[Group G']
G'1. a C₁₋₆ alkyl group that may be substituted with one or more substituents selected from Group g' below:
[Group g']
g'1. a halogen atom,
g'2. an amino group,
g'3. a mono C₁₋₆ alkylamino group,
g'4. a di C₁₋₆ alkylamino group,
g'5. a C₁₋₆ alkoxycarbonylamino group,
and,
g'6. a hydroxyimino group,
G'2. a halogen atom,
G'3. a C₁₋₆ alkoxy group that may be substituted with a halogen atom,
G'4. an aryloxy group,
G'5. a cyano group,
G'6. -N(R^{59a}) (R^{59a'}),
wherein R^{59a}and R^{59a'} are identical with or different from each other and represent a hydrogen atom or a C₁₋₆ alkyl group, or R^{59a} or R^{59a'} may, together with the adjacent nitrogen atom, form a saturated 5- or 6-membered monocyclic heterocyclic ring and the saturated heterocyclic ring may be substituted with one or more substituents selected from the group consisting of a hydroxyl group, an amino group, a mono C₁₋₆ alkylamino group and a di C₁₋₆ alkylamino group,
G'7. -CO-N (R^{59b}) (R^{59b'})
wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
G'8. an aryl group,
and,
G'9. a 5- or 6-membered monocyclic heteroaromatic group that may be substituted with an oxo or thioxo group.

15. The pharmaceutical composition for treating or preventing diabetes according to any of claims 2 to 6, wherein R⁵ and R⁴, together with the adjacent nitrogen atom, form a saturated 5- or 6-membered monocyclic heterocyclic group, wherein a part of the saturated heterocyclic group may have a double bond, and the saturated heterocyclic group may be condensed with a benzene ring to form a condensed ring, and the saturated heterocyclic group which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group.

16. The pharmaceutical composition for treating or preventing diabetes according to claim 2, wherein the pyrazole compound is a pyrazole compound represented by the following general formula (IV): 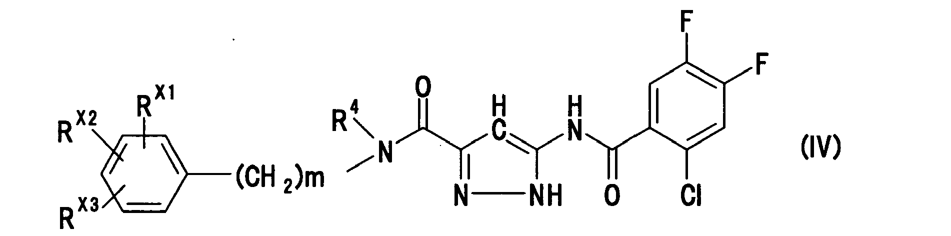 wherein R⁴ is as described in claim 2, R^{X1}, R^{X2} and R^{X3} are identical with or different from each other and represent a hydrogen atom or a substituent selected from the following Group F", and m is 0 or an integer from 1 to 2,
[Group F"]
F''1. a C₁₋₆ alkyl group,
F''2. a halogen atom,
F''3. a cyano group,
F''4. a hydroxyl group,
F''5. a C₁₋₄ alkoxy group that may be substituted with one or more substituents selected from the group consisting of a carboxy group and a C₁₋₄ alkoxycarbonyl group,
F''6. a halo C₁₋₆ alkyl group,
F''7. a carboxy group,
F''8. a C₁₋₆ alkoxycarbonyl group,
F''9. -CO-N (R^{56a}) (R^{56a'})
wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f'' below:
[Group f'']
f''1. an amino group,
f''2. a mono C₁₋₆ alkylamino group,
f''3. a di C₁₋₆ alkylamino group,
f''4. a carboxy group,
f''5. a C₁₋₆ alkoxycarbonyl group,
f''6. a hydroxyl group,
and,
f''7. a saturated 5- or 6-membered monocyclic heterocyclic group having at least one nitrogen atom,
F''10. -N (R^{56b}) (R^{56b'})
wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
F''11. -N=CR⁵⁷(-N(R⁵⁸) (R^{58'})),
wherein R⁵⁷ is a hydrogen atom or a C₁₋₆ alkyl group, R⁵⁸ and R^{58'} are identical with or different from each other and represent a hydrogen atom or a C₁₋₆ alkyl group,
F''12. a 5- or 6-membered monocyclic heteroaromatic group,
and,
F''13. a methylenedioxy group or an ethylenedioxy group.

17. The pharmaceutical composition for treating or preventing diabetes according to claim 2, wherein the pyrazole compound is a pyrazole compound represented by the following general formula (V): 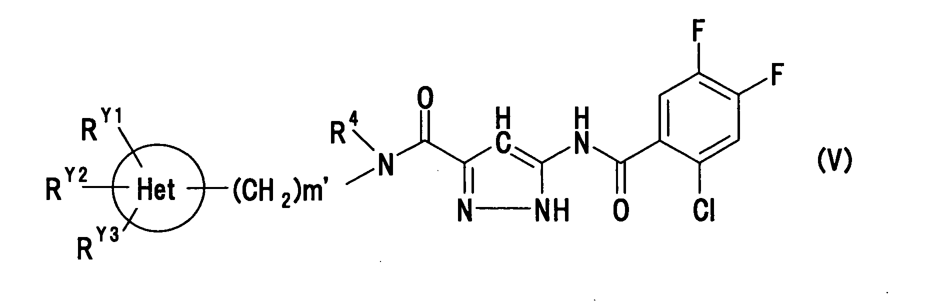 wherein R⁴ is as defined in claim 2, the ring Het represents a heteroaromatic group of a 5- or 6-membered monocyclic ring, R^{Y1}, R^{Y2} and R^{Y3} are identical with or different from each other and represent a hydrogen atom or a substituent selected from the following Group G', and m' is 0 or an integer from 1 to 2,
[Group G']
G'1. a C₁₋₆ alkyl group that may be substituted with one or more substituents selected from Group g' below:
[Group g']
g'1. a halogen atom,
g'2. an amino group,
g'3. a mono C₁₋₆ alkylamino group,
g'4. a di C₁₋₆ alkylamino group,
g'5. a C₁₋₆ alkoxycarbonylamino group,
and,
g'6. a hydroxyimino group,
G'2. a halogen atom,
G'3. a C₁₋₆ alkoxy group that may be substituted with a halogen atom,
G'4. an aryloxy group,
G'5. a cyano group,
G'6. -N (R^{59a}) (R^{59a'}),
wherein R^{59a} and R^{59a'} are identical with or different from each other and represent a hydrogen atom or a C₁₋₆ alkyl group, or R^{59a} and R^{59a'} may, together with the adjacent nitrogen atom, forma saturated 5- or 6-memberedmonocyclicheterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
G'7. -CO-N (R^{59b}) (R^{59b'})
wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
G'8. an aryl group,
and,
G'9. a 5- or 6-membered monocyclic heteroaromatic group that may be substituted with an oxo or thioxo group.

18. The pharmaceutical composition for treating or preventing diabetes according to claim 17, wherein the ring Het is a pyridine ring, a triazole ring or an oxazole ring.

19. The pharmaceutical composition for treating or preventing diabetes according to claim 18, wherein the pyrazole compound is a pyrazole compound represented by the following general formula (VI): 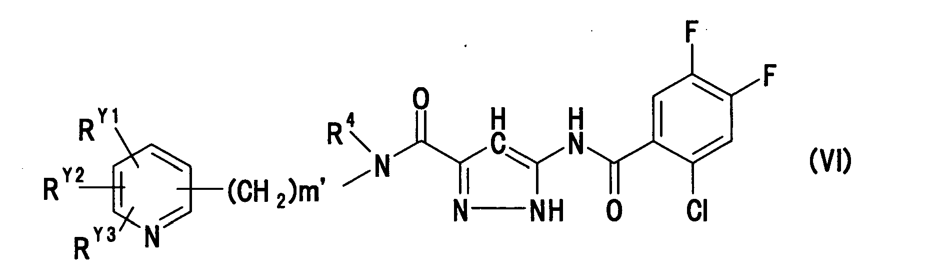 wherein R⁴, R^{Y1}, R^{Y2}, R^{Y3} and m' are as described above.

20. The pharmaceutical composition for treating or preventing diabetes according to claim 2, wherein the pyrazole compound or a pharmacologically acceptable salt thereof is selected from the following group:
5-(2-Chloro-4,5-difluoro-benzoylamino)-1H-pyrazole-3-carboxylic acid (pyridin-3-ylmethyl)-amide,
5-(2-Chloro-4,5-difluoro-benzoylamino)-1H-pyrazole-3-carboxylic acid (pyridin-3-ylmethyl)-amide dihydrochloride,
5-(2-Chloro-4,5-difluoro-benzoylamino)-1H-pyrazole-3-carboxylic acid (pyridin-3-ylmethyl) -amide hydrochloride,
5-(2-Chloro-4,5-difluoro-benzoylamino)-1H-pyrazole-3-carboxylic acid (4-methyl-oxazol-5-ylmethyl)-amide,
5-(2-Chloro-4,5-difluoro-benzoylamino)-1H-pyrazole-3-carboxylic acid (4-methyl-oxazol-5-ylmethyl)-amide,
5-(2-Chloro-4,5-difluoro-benzoylamino)-1H-pyrazole-3-carboxylic acid (4-methyl-oxazol-5-ylmethyl)amide-p-toluenesulfonate,
5-(2-Chloro-4,5-difluoro-benzoylamino)-1H-pyrazole-3-carboxylic acid (4-methyl-oxazol-5-ylmethyl)amide-L-(+)-tartrate,
5-(2-Chloro-4,5-difluoro-benzoylamino)-1H-pyrazole-3-carboxylic acid (2,4-dimethyl-oxazol-5-ylmethyl)-amide p-toluenesulfonate,
5-(2-Chloro-4,5-difluoro-benzoylamino)-1H-pyrazole-3-carboxylic acid (2,4-dimethyl-oxazol-5-ylmethyl)-amide,
5-(2-Chloro-4,5-difluoro-benzoylamino)-1H-pyrazole-3-carboxylic acid (2,4-dimethyl-pyridin-3-ylmethyl)amide,
5-(2-Chloro-4,5-difluoro-benzoylamino)-1H-pyrazole-3-carboxylic acid diethylamide,
5-(2-Chloro-4,5-difluoro-benzoylamino)-1H-pyrazole-3-carboxylic acid ethyl-(pyridin-4-ylmethyl)-amide,
5-(2-Chloro-4,5-difluoro-benzoylamino)-1H-pyrazole-3-carboxylic acid (2-methoxy-ethyl)-methyl-amide,
5-(2-Chloro-4,5-difluoro-benzoylamino)-1H-pyrazole-3-carboxylic acid ethyl-(2-methoxy-ethyl)-amide,
5-(2-Chloro-4,5-difluoro-benzoylamino)-1H-pyrazole-3-carboxylic acid 2-fluoro-benzylamide,
5-(2-Chloro-4,5-difluoro-benzoylamino)-1H-pyrazole-3-carboxylic acid 3-fluoro-benzylamide,
5-(2-Chloro-4,5-difluoro-benzoylamino)-1H-pyrazole-3-carboxylic acid 2-dimethylamino-benzylamide,
5-(2-Chloro-4,5-difluoro-benzoylamino)-1H-pyrazole-3-carboxylic acid (4-ethoxycarbonyl-cyclohexyl)-propyl-amide,
5-(2-Chloro-4,5-difluoro-benzoylamino)-1H-pyrazole-3-carboxylic acid (6-methoxy-pyridin-3-ylmethyl)-amide,
5-(2-Chloro-4,5-difluoro-benzoylamino)-1H-pyrazole-3-carboxylic acid 2,3-dimethoxy-benzylamide,
5-(2-Chloro-4,5-difluoro-benzoylamino)-1H-pyrazole-3-carboxylic acid 2,4-dimethoxy-benzylamide,
5-(2-Chloro-4,5-difluoro-benzoylamino)-1H-pyrazole-3-carboxylic acid 2,6-dimethoxy-benzylamide,
5-(2-Chloro-4,5-difluoro-benzoylamino)-1H-pyrazole-3-carboxylic acid 3,5-dimethoxy-benzylamide,
5-(2-Chloro-4,5-difluoro-benzoylamino)-1H-pyrazole-3-carboxylic acid 3,4-dimethoxy-benzylamide,
5-(2-Chloro-4,5-difluoro-benzoylamino)-1H-pyrazole-3-carboxylic acid 2,3-difluoro-benzylamide,
5-(2-Chloro-4,5-difluoro-benzoylamino)-1H-pyrazole-3-carboxylic acid 2,4-difluoro-benzylamide,
5-(2-Chloro-4,5-difluoro-benzoylamino)-1H-pyrazole-3-carboxylic acid 2,5-difluoro-benzylamide,
5-(2-Chloro-4,5-difluoro-benzoylamino)-1H-pyrazole-3-carboxylic acid 2,6-difluoro-benzylamide,
5-(2-Chloro-4,5-difluoro-benzoylamino)-1H-pyrazole-3-carboxylic acid 3,4-difluoro-benzylamide,
5-(2-Chloro-4,5-difluoro-benzoylamino)-1H-pyrazole-3-carboxylic acid 3,5-difluoro-benzylamide,
5-(2-Chloro-4,5-difluoro-benzoylamino)-1H-pyrazole-3-carboxylic acid (2-isopropoxy-pyridin-3-ylmethyl)-amide,
5-(2-Chloro-4,5-difluoro-benzoylamino)-1H-pyrazole-3-carboxylic acid (2-phenoxy-pyridin-3-ylmethyl)-amide,
5-(2-Chloro-4,5-difluoro-benzoylamino)-1H-pyrazole-3-carboxylic acid (3,5-difluoro-pyridin-2-ylmethyl)-amide,
5-(2-Chloro-4,5-difluoro-benzoylamino)-1H-pyrazole-3-carboxylic acid 2-trifluoromethyl-benzylamide,
5-(2-Chloro-4,5-difluoro-benzoylamino)-1H-pyrazole-3-carboxylic acid 3-trifluoromethyl-benzylamide
5-(2-Chloro-4,5-difluoro-benzoylamino)-1H-pyrazole-3-carboxylic acid 4-trifluoromethyl-benzylamide,
5-(2-Chloro-4,5-difluoro-benzoylamino)-1H-pyrazole-3-carboxylic acid 4-tert-butyl-benzylamide,
5-(2-Chloro-4,5-difluoro-benzoylamino)-1H-pyrazole-3-carboxylic acid (6-isopropoxy-pyridin-3-ylmethyl)-amide,
5-(2-Chloro-4,5-difluoro-benzoylamino)-1H-pyrazole-3-carboxylic acid [2-(2,2,2-trifluoro-ethoxy)-pyridin-3-ylmethyl]-amide,
5-(2-Chloro-4,5-difluoro-benzoylamino)-1H-pyrazole-3-carboxylic acid 4-(2-dimethylamino-ethylcarbamoyl)-benzylamide,
5-(2-Chloro-4,5-difluoro-benzoylamino)-1H-pyrazole-3-carboxylic acid 4-ethyl-benzylamide,
5-(2-Chloro-4,5-difluoro-benzoylamino)-1H-pyrazole-3-carboxylic acid 4-isopropyl-benzylamide,
5-(2-Chloro-4,5-difluoro-benzoylamino)-1H-pyrazole-3-carboxylic acid 2-chloro-6-fluoro-benzylamide,
5-(2-Chloro-4,5-difluoro-benzoylamino)-1H-pyrazole-3-carboxylic acid (6-ethoxy-pyridin-3-ylmethyl)-amide dihydrochloride,
5-(2-Chloro-4,5-difluoro-benzoylamino)-1H-pyrazole-3-carboxylic acid (thiazol-4-ylmethyl)-amide,
5- (2-Chloro-4,5-difluoro-benzoylamino) -1H-pyrazole-3-carboxylic acid [6-(2,2,2-trifluoro-ethoxy)-pyridin-3-ylmethyl]-amide dihydrochloride,
5-(2-Chloro-4,5-difluoro-benzoylamino)-1H-pyrazole-3-carboxylic acid 2,6-dimethyl-benzylamide,
5-(2-Chloro-4,5-difluoro-benzoylamino)-1H-pyrazole-3-carboxylic acid [2-(pyridin-3-yl)-thiazol-4-ylmethyl]-amide dihydrochloride,
5-(2-Chloro-4,5-difluoro-benzoylamino)-1H-pyrazole-3-carboxylic acid (1H-benzoimidazol-2-ylmethyl)-amide dihydrochloride, [4-({[5-(2-Chloro-4,5-difluoro-benzoylamino)-1H-pyrazole-3-carbonyl]-amino}-methyl)-benzoylamino]-acetic acid methyl ester, 3-[4-({[5-(2-Chloro-4,5-difluoro-benzoylamino)-1H-pyrazole-3-carbonyl]-amino}-methyl)-benzoylamino]-propionic acid methyl ester
5-(2-Chloro-4,5-difluoro-benzoylamino)-1H-pyrazole-3-carboxylic acid (2-methyl-thiazol-4-ylmethyl)-amide dihydrochloride,
5-(2-Chloro-4,5-difluoro-benzoylamino)-1H-pyrazole-3-carboxylic acid (3,5-dimethyl-isoxazol-4-ylmethyl)-amide,
5-(2-Chloro-4,5-difluoro-benzoylamino)-1H-pyrazole-3-carboxylic acid 4-[2-bis-(2-acetoxyethyl)-amino-ethylcarbamoyl]-benzylamide,
5-(2-Chloro-4,5-difluoro-benzoylamino)-1H-pyrazole-3-carboxylic acid 4-(2-hydroxy-ethylcarbamoyl)-benzylamide,
5-(2-Chloro-4,5-difluoro-benzoylamino)-1H-pyrazole-3-carboxylic acid 4-{2-[(2-acetoxyethyl)-(2-hyroxyethyl)-amino]-ethylcarbamoyl}-benzylamide,
5-(2-Chloro-4,5-difluoro-benzoylamino)-1H-pyrazole-3-carboxylic acid (1-methyl-1H-benzoimidazol-2-ylmethyl)-amide hydrochloride,
5-(2-Chloro-4,5-difluoro-benzoylamino)-1H-pyrazole-3-carboxylic acid (thiazol-2-ylmethyl)-amide dihydrochloride,
5-(2-Chloro-4,5-difluoro-benzoylamino)-1H-pyrazole-3-carboxylic acid (benzothiazol-2-ylmethyl)-amide dihydrochloride,
5-(2-Chloro-4,5-difluoro-benzoylamino)-1H-pyrazole-3-carboxylic acid (2,5-dimethyl-thiazol-4-ylmethyl)-amide dihydrochloride,
5-(2-Chloro-4,5-difluoro-benzoylamino)-1H-pyrazole-3-carboxylic acid [2-(morpholin-4-yl)-thiazol-4-ylmethyl]-amide dihydrochloride,
5-(2-Chloro-4,5-difluoro-benzoylamino)-1H-pyrazole-3-carboxylic acid (1,3,5-trimethyl-1H-pyrazol-4-ylmethyl)-amide,
5-(2-Chloro-4,5-difluoro-benzoylamino)-1H-pyrazole-3-carboxylic acid (2-chloro-6-methyl-pyridin-3-ylmethyl)-amide dihydrochloride,
5-(2-Chloro-4,5-difluoro-benzoylamino)-1H-pyrazole-3-carboxylic acid (2-dimethylamino-thiazol-4-ylmethyl)-amide dihydrochloride,
5-(2-Chloro-4,5-difluoro-benzoylamino)-1H-pyrazole-3-carboxylic acid (1-methyl-1H-pyrrol-2-ylmethyl)-amide,
5-(2-Chloro-4,5-difluoro-benzoylamino)-1H-pyrazole-3-carboxylic acid (3,4-dimethoxy-pyridin-2-ylmethyl)-amide dihydrochloride,
5-(2-Chloro-4,5-difluoro-benzoylamino)-1H-pyrazole-3-carboxylic acid (5-tert-butyl-thiazol-2-ylmethyl)-amide dihydrochloride,
5-(2-Chloro-4,5-difluoro-benzoylamino)-1H-pyrazole-3-carboxylic acid
(5-methyl-2-phenyl-2H-[1,2,3]triazol-4-ylmethyl)-amide,
5-(2-Chloro-4,5-difluoro-benzoylamino)-1H-pyrazole-3-carboxylic acid [4-(tert-butoxycarbonyl-amino)-methyl-pyridin-2-ylmethyl]-amide,
5-(2-Chloro-4,5-difluoro-benzoylamino)-1H-pyrazole-3-carboxylic acid [4-methyl-2-(morpholin-4-yl)-thiazol-5-ylmethyl]-amide dihydrochloride,
5-(2-Chloro-4,5-difluoro-benzoylamino)-1H-pyrazole-3-carboxylic acid (pyridin-3-ylmethyl)-amide 3/2hydrochloride hemihydrate,
5-(2-Chloro-4,5-difluoro-benzoylamino)-1H-pyrazole-3-carboxylic acid 2-amino-benzylamide,
5-(2-Chloro-4,5-difluoro-benzoylamino)-1H-pyrazole-3-carboxylic acid 2-methoxy-benzylamide,
5-(2-Chloro-4,5-difluoro-benzoylamino)-1H-pyrazole-3-carboxylic acid 3-methoxy-benzylamide,
5-(2-Chloro-4,5-difluoro-benzoylamino)-1H-pyrazole-3-carboxylic acid 4-methoxy-benzylamide,
5-(2-Chloro-4,5-difluoro-benzoylamino)-1H-pyrazole-3-carboxylic acid 4-methyl-benzylamide,
5-(2-Chloro-4,5-difluoro-benzoylamino)-1H-pyrazole-3-carboxylic acid 4-fluoro-benzylamide,
5-(2-Chloro-4,5-difluoro-benzoylamino)-1H-pyrazole-3-carboxylic acid 4-chloro-benzylamide,
5-(2-Chloro-4,5-difluoro-benzoylamino)-1H-pyrazole-3-carboxylic acid 4-ethoxycarbonyl-benzylamide,
5-(2-Chloro-4,5-difluoro-benzoylamino)-1H-pyrazole-3-carboxylic acid 4-carboxy-benzylamide,
5-(2-Chloro-4,5-difluoro-benzoylamino)-1H-pyrazole-3-carboxylic acid dibenzylamide,
5-(2-Chloro-4,5-difluoro-benzoylamino)-1H-pyrazole-3-carboxylic acid (cyano-phenyl-methyl)-amide,
5-(2-Chloro-4,5-difluoro-benzoylamino)-1H-pyrazole-3-carboxylic acid cyclohexylmethyl-amide,
5-(2-Chloro-4,5-difluoro-benzoylamino)-1H-pyrazole-3-carboxylic acid (2-hydroxy-ethyl)-phenyl-amide,
5-(2-Chloro-4,5-difluoro-benzoylamino)-1H-pyrazole-3-carboxylic acid cyclohexyl-methyl-amide,
5-(2-Chloro-4,5-difluoro-benzoylamino)-1H-pyrazole-3-carboxylic acid cyclohexylamide,
5-(2-Chloro-4,5-difluoro-benzoylamino)-1H-pyrazole-3-carboxylic acid cyclohexyl-ethyl-amide,
5-(2-Chloro-4,5-difluoro-benzoylamino)-1H-pyrazole-3-carboxylic acid allyl-cyclohexyl-amide,
5-(2-Chloro-4,5-difluoro-benzoylamino)-1H-pyrazole-3-carboxylic acid cyclohexyl-(pyridin-2-ylmethyl)-amide,
5-(2-Chloro-4,5-difluoro-benzoylamino)-1H-pyrazole-3-carboxylic acid methyl-(1-methyl-piperidin-4-yl)-amide,
5-(2-Chloro-4,5-difluoro-benzoylamino)-1H-pyrazole-3-carboxylic acid [(3,4-methylenedioxyphenyl)-methyl]-amide hydrochloride,
5-(2-Chloro-4,5-difluoro-benzoylamino)-1H-pyrazole-3-carboxylic acid benzyl-butyl-amide,
5-(2-Chloro-4,5-difluoro-benzoylamino)-1H-pyrazole-3-carboxylic acid benzyl-(4-hydroxy-butyl)-amide hydrochloride,
5-(2-Chloro-4,5-difluoro-benzoylamino)-1H-pyrazole-3-carboxylic acid butyl-ethyl-amide,
5-(2-Chloro-4,5-difluoro-benzoylamino)-1H-pyrazole-3-carboxylic acid cyclohexyl-propyl-amide,
5-(2-Chloro-4,5-difluoro-benzoylamino)-1H-pyrazole-3-carboxylic acid butyl-cyclohexyl-amide,
5-(2-Chloro-4,5-difluoro-benzoylamino)-1H-pyrazole-3-carboxylic acid cyclohexyl-(2-methoxy-ethyl)-amide,
5-(2-Chloro-4,5-difluoro-benzoylamino)-1H-pyrazole-3-carboxylic acid cyclohexyl-(pyridin-3-ylmethyl)-amide,
5-(2-Chloro-4,5-difluoro-benzoylamino)-1H-pyrazole-3-carboxylic acid [2-(morpholin-4-yl)-phenyl]-amide,
5-(2-Chloro-4,5-difluoro-benzoylamino)-1H-pyrazole-3-carboxylic acid dibutylamide,
5-(2-Chloro-4,5-difluoro-benzoylamino)-1H-pyrazole-3-carboxylic acid bis-(2-methoxy-ethyl)-amide,
5-(2-Chloro-4,5-difluoro-benzoylamino)-1H-pyrazole-3-carboxylic acid (2-methoxy-ethyl)-propyl-amide,
5-(2-Chloro-4,5-difluoro-benzoylamino)-1H-pyrazole-3-carboxylic acid butyl-(tetrahydro-pyran-4-yl)-amide,
5-(2-Chloro-4,5-difluoro-benzoylamino)-1H-pyrazole-3-carboxylic acid (2-methoxy-ethyl)-(tetrahydro-pyran-4-yl)-amide,
5-(2-Chloro-4,5-difluoro-benzoylamino)-1H-pyrazole-3-carboxylic acid cyclopentyl-(2-methoxy-ethyl)-amide,
5-(2-Chloro-4,5-difluoro-benzoylamino)-1H-pyrazole-3-carboxylic acid cyclohexyl-(3-methoxy-propyl)-amide,
5-(2-Chloro-4,5-difluoro-benzoylamino)-1H-pyrazole-3-carboxylic acid cyclohexyl-(2-ethoxy-ethyl)-amide,
5-(2-Chloro-4,5-difluoro-benzoylamino)-1H-pyrazole-3-carboxylic acid cyclohexyl-(2-isopropoxy-ethyl)-amide,
5-(2-Chloro-4,5-difluoro-benzoylamino)-1H-pyrazole-3-carboxylic acid cyclohexyl-(2-propoxy-ethyl)-amide,
5-(2-Chloro-4,5-difluoro-benzoylamino)-1H-pyrazole-3-carboxylic acid (1-ethyl-propyl)-(2-methoxy-ethyl)-amide,
5-(2-Chloro-4,5-difluoro-benzoylamino)-1H-pyrazole-3-carboxylic acid butyl-(1-tert-butoxycarbonyl-piperidin-4-yl)-amide,
5-(2-Chloro-4,5-difluoro-benzoylamino)-1H-pyrazole-3-carboxylic acid butyl-(tetrahydro-thiopyran-4-yl)-amide,
5-(2-Chloro-4,5-difluoro-benzoylamino)-1H-pyrazole-3-carboxylic acid (2-methoxy-ethyl)-amide,
5-(2-Chloro-4,5-difluoro-benzoylamino)-1H-pyrazole-3-carboxylic acid butyl-(pyridin-3-ylmethyl)-amide,
5-(2-Chloro-4,5-difluoro-benzoylamino)-1H-pyrazole-3-carboxylic acid benzyl-(2-methoxy-ethyl)-amide,
5-(2-Chloro-4,5-difluoro-benzoylamino)-1H-pyrazole-3-carboxylic acid butyl-(piperidin-4-yl)-amide,
5-(2-Chloro-4,5-difluoro-benzoylamino)-1H-pyrazole-3-carboxylic acid (1-acetyl-piperidin-4-yl)-butyl-amide,
5-(2-Chloro-4,5-difluoro-benzoylamino)-1H-pyrazole-3-carboxylic acid butyl-(1-methanesulfonyl-piperidin-4-yl)-amide,
5-(2-Chloro-4,5-difluoro-benzoylamino)-1H-pyrazole-3-carboxylic acid butyl-(1-ethoxalyl-piperidin-4-yl)-amide,
5-(2-Chloro-4,5-difluoro-benzoylamino)-1H-pyrazole-3-carboxylic acid butyl-(1-oxalo-piperidin-4-yl)-amide,
5-(2-Chloro-4,5-difluoro-benzoylamino)-1H-pyrazole-3-carboxylic acid butyl-(1,1-dioxo-hexahydro-1λ⁶ -thiopyran-4-yl)-amide,
5-(2-Chloro-4,5-difluoro-benzoylamino)-1H-pyrazole-3-carboxylic acid butyl-(1-oxo-hexahydro-1λ⁴-thiopyran-4-yl)-amide,
5-(2-Chloro-4,5-difluoro-benzoylamino)-1H-pyrazole-3-carboxylic acid (6-methoxy-pyridin-3-ylmethyl)-propyl-amide,
5-(2-Chloro-4,5-difluoro-benzoylamino)-1H-pyrazole-3-carboxylic acid propyl-(pyridin-3-ylmethyl)-amide,
5-(2-Chloro-4,5-difluoro-benzoylamino)-1H-pyrazole-3-carboxylic acid 4-diethylcarbamoyl-benzylamide,
5-(2-Chloro-4,5-difluoro-benzoylamino)-1H-pyrazole-3-carboxylic acid 3-diethylcarbamoyl-benzylamide,
5-(2-Chloro-4,5-difluoro-benzoylamino)-1H-pyrazole-3-carboxylic acid 4-ethylcarbamoyl-benzylamide,
5-(2-Chloro-4,5-difluoro-benzoylamino)-1H-pyrazole-3-carboxylic acid 3-ethylcarbamoyl-benzylamide,
5-(2-Chloro-4,5-difluoro-benzoylamino)-1H-pyrazole-3-carboxylic acid 4-carbamoyl-benzylamide,
5-(2-Chloro-4,5-difluoro-benzoylamino)-1H-pyrazole-3-carboxylic acid (1-oxalo-piperidin-4-yl)-propyl-amide,
5-(2-Chloro-4,5-difluoro-benzoylamino)-1H-pyrazole-3-carboxylic acid (1-carboxyacetyl-piperidin-4-yl)-propyl-amide,
5-(2-Chloro-4,5-difluoro-benzoylamino)-1H-pyrazole-3-carboxylic acid [1-(2-carboxypropionyl)-piperidin-4-yl]-propyl-amide,
5-(2-Chloro-4,5-difluoro-benzoylamino)-1H-pyrazole-3-carboxylic acid cyclopropyl-(6-methoxy-pyridin-3-ylmethyl)-amide
5-(2-Chloro-4,5-difluoro-benzoylamino)-1H-pyrazole-3-carboxylic acid cyclobutyl-(6-methoxy-pyridin-3-ylmethyl)-amide,
5-(2-Chloro-4,5-difluoro-benzoylamino)-1H-pyrazole-3-carboxylic acid cyclopropylmethyl-(6-methoxy-pyridin-3-ylmethyl)-amide,
5-(2-Chloro-4,5-difluoro-benzoylamino)-1H-pyrazole-3-carboxylic acid (6-dimethylamino-pyridin-3-ylmethyl)-amide,
5-(2-Chloro-4,5-difluoro-benzoylamino)-1H-pyrazole-3-carboxylic acid (6-dimethylamino-pyridin-3-ylmethyl)-propyl-amide,
5-(2-Chloro-4,5-difluoro-benzoylamino)-1H-pyrazole-3-carboxylic acid benzyl-(2-carboxy-ethyl)-amide,
5-(2-Chloro-4,5-difluoro-benzoylamino)-1H-pyrazole-3-carboxylic acid benzyl-(2-ethoxycarbonyl-ethyl)-amide,
5-(2-Chloro-4,5-difluoro-benzoylamino)-1H-pyrazole-3-carboxylic acid (6-chloro-pyridin-3-ylmethyl)-propyl-amide,
5- (2-Chloro-4,5-difluoro-benzoylamino) -1H-pyrazole-3-carboxylic acid 2,6-dichloro-benzylamide,
5-(2-Chloro-4,5-difluoro-benzoylamino)-1H-pyrazole-3-carboxylic acid (3,5-dichloro-pyridin-4-ylmethyl)-amide,
5-(2-Chloro-4,5-difluoro-benzoylamino)-1H-pyrazole-3-carboxylic acid (3,5-dichloro-pyridin-4-ylmethyl)-propyl-amide,
5-(2-Chloro-4,5-difluoro-benzoylamino)-1H-pyrazole-3-carboxylic acid [6-(1H-pyrazol-1-yl)-pyridin-3-ylmethyl]-amide dihydrochloride,
5-(2-Chloro-4,5-difluoro-benzoylamino)-1H-pyrazole-3-carboxylic acid [6-(4-hydroxy-piperidin-1-yl)-pyridin-3-ylmethyl]-amide dihydrochloride,
5-(2-Chloro-4,5-difluoro-benzoylamino)-1H-pyrazole-3-carboxylic acid (2-pyridin-2-yl-ethyl)-amide,
5-(2-Chloro-4,5-difluoro-benzoylamino)-1H-pyrazole-3-carboxylic acid (2-pyridin-3-yl-ethyl)-amide,
5-(2-Chloro-4,5-difluoro-benzoylamino)-1H-pyrazole-3-carboxylic acid (2-pyridin-4-yl-ethyl)-amide,
5-(2-Chloro-4,5-difluoro-benzoylamino)-1H-pyrazole-3-carboxylic acid (4-[1,2,3]thiadiazol-4-yl-benzyl)-amide,
5-(2-Chloro-4,5-difluoro-benzoylamino)-1H-pyrazole-3-carboxylic acid (5-methyl-pyrazin-2-ylmethyl)-amide,
5-(2-Chloro-4,5-difluoro-benzoylamino)-1H-pyrazole-3-carboxylic acid pyrazin-2-ylamide,
5-(2-Chloro-4,5-difluoro-benzoylamino)-1H-pyrazole-3-carboxylic acid quinolin-5-ylamide,
5-(2-Chloro-4,5-difluoro-benzoylamino)-1H-pyrazole-3-carboxylic acid quinolin-8-ylamide,
5-(2-Chloro-4,5-difluoro-benzoylamino)-1H-pyrazole-3-carboxylic acid isoquinolin-5-ylamide,
5-(2-Chloro-4,5-difluoro-benzoylamino)-1H-pyrazole-3-carboxylic acid [3-ethoxy-5-(1-ethoxycarbonyl-1-methyl-ethyl)-pyridin-2-yl]-amide,
5-(2-Chloro-4,5-difluoro-benzoylamino)-1H-pyrazole-3-carboxylic acid [3-ethoxy-5-(1-ethoxycarbonyl-1-hydroxy-ethyl)-pyridin-2-yl]-amide,
5-(2-Chloro-4,5-difluoro-benzoylamino)-1H-pyrazole-3-carboxylic acid
[5-(1-carboxy-1-methyl-ethyl)-3-ethoxy-pyridin-2-yl]-amide,
5-(2-Chloro-4,5-difluoro-benzoylamino)-1H-pyrazole-3-carboxylic acid
[5-(1-carboxy-1-hydroxy-ethyl)-3-ethoxy-pyridin-2-yl]-amide,
5-(2-Chloro-4,5-difluoro-benzoylamino)-1H-pyrazole-3-carboxylic acid (pyrazin-2-ylmethyl)-amide,
5-(2-Chloro-4,5-difluoro-benzoylamino)-1H-pyrazole-3-carboxylic acid [6-(thiomorpholin-4-yl)-pyridin-3-ylmethyl]-amide,
5-(2-Chloro-4,5-difluoro-benzoylamino)-1H-pyrazole-3-carboxylic acid [6-(1,1-dioxo-1λ⁶-thiomorpholin-4-yl)-pyridin-3-ylmethyl]-amide,
5-(2-Chloro-4,5-difluoro-benzoylamino)-1H-pyrazole-3-carboxylic acid (4,5-dibromo-thiophen-2-ylmethyl)-amide,
5-(2-Chloro-4,5-difluoro-benzoylamino)-1H-pyrazole-3-carboxylic acid (5-chloro-thiophen-2-ylmethyl)-amide,
5-(2-Chloro-4,5-difluoro-benzoylamino)-1H-pyrazole-3-carboxylic acid (4-e thyl-2-methyl-oxazol-5-ylmethyl)-amide,
5-(2-Chloro-4,5-difluoro-benzoylamino)-1H-pyrazole-3-carboxylic acid (2-e thyl-4-methyl-oxazol-5-ylmethyl)-amide dihydrochloride,
5-(2-Chloro-4,5-difluoro-benzoylamino)-1H-pyrazole-3-carboxylic acid ([2,2']bithiophenyl-5-ylmethyl)-amide,
5-(2-Chloro-4,5-difluoro-benzoylamino)-1H-pyrazole-3-carboxylic acid (3-methoxy-thiophen-2-ylmethyl)-amide,
5-(2-Chloro-4,5-difluoro-benzoylamino)-1H-pyrazole-3-carboxylic acid (4,5-Dichloro-thiophen-2-ylmethyl)-amide,
5-(2-Chloro-4,5-difluoro-benzoylamino)-1H-pyrazole-3-carboxylic acid (2,5-dimethyl-oxazol-4-ylmethyl)-amide,
5-(2-Chloro-4,5-difluoro-benzoylamino)-1H-pyrazole-3-carboxylic acid (2-hexyl-4-methyl-oxazol-5-ylmethyl)-amide,
5-(2-Chloro-4,5-difluoro-benzoylamino)-1H-pyrazole-3-carboxylic acid (4-methoxy-thiophen-3-ylmethyl)-amide,
5-(2-Chloro-4,5-difluoro-benzoylamino)-1H-pyrazole-3-carboxylic acid (4-methyl-2-phenyl-oxazol-5-ylmethyl)-amide,
5-(2-Chloro-4,5-difluoro-benzoylamino)-1H-pyrazole-3-carboxylic acid (2-methyl-4-phenyl-oxazol-5-ylmethyl)-amide,
5-(2-Chloro-4,5-difluoro-benzoylamino)-1H-pyrazole-3-carboxylic acid (4-hexyl-2-methyl-oxazol-5-ylmethyl)-amide dihydrochloride,
5-(2-Chloro-4,5-difluoro-benzoylamino)-1H-pyrazole-3-carboxylic acid (6-chloro-pyridin-3-ylmethyl)-amide,
5-(2-Chloro-4,5-difluoro-benzoylamino)-1H-pyrazole-3-carboxylic acid (6-chloro-pyridin-3-ylmethyl)-amide dihydrochloride,
5-(2-Chloro-4,5-difluoro-benzoylamino)-1H-pyrazole-3-carboxylic acid (4H-[1,2,4]triazol-3-ylmethyl)-amide,
5-(2-Chloro-4,5-difluoro-benzoylamino)-1H-pyrazole-3-carboxylic acid (4H-[1,2,4]triazol-3-ylmethyl)-amide dihydrochloride,
5-(2-Chloro-4,5-difluoro-benzoylamino)-1H-pyrazole-3-carboxylic acid(5-fluoro-4H-quinazoline-3-yl)-amide,
5-(2-Chloro-4,5-difluoro-benzoylamino)-1H-pyrazole-3-carboxylic acid (5-fluoro-4H-quinazoline-3-yl)-amide dihydrochloride,
5-(2-Chloro-4,5-difluoro-benzoylamino)-1H-pyrazole-3-carboxylic acid (4,6-dimethyl-pyridin-3-ylmethyl)-amide,
5-(2-Chloro-4,5-difluoro-benzoylamino)-1H-pyrazole-3-carboxylic acid (4,6-dimethyl-pyridin-3-ylmethyl)-amide dihydrochloride,
5-(2-Chloro-4,5-difluoro-benzoylamino)-1H-pyrazole-3-carboxylic acid (2-methoxy-6-methyl-pyridin-3-ylmethyl)-amide,
5-(2-Chloro-4,5-difluoro-benzoylamino)-1H-pyrazole-3-carboxylic acid (4-methoxy-6-methyl-pyridin-3-ylmethyl)-amide,
5-(2-Chloro-4,5-difluoro-benzoylamino)-1H-pyrazole-3-carboxylic acid (4-methoxy-6-methyl-pyridin-3-ylmethyl)-amide dihydrochloride,
5-(2-Chloro-4,5-difluoro-benzoylamino)-1H-pyrazole-3-carboxylic acid (2-methoxy-4,6-dimethyl-pyridin-3-ylmethyl)-amide,
5-(2-Chloro-4,5-difluoro-benzoylamino)-1H-pyrazole-3-carboxylic acid (2-methoxy-4,6-dimethyl-pyridin-3-ylmethyl)-amide dihydrochloride,
5-(2-Chloro-4,5-difluoro-benzoylamino)-1H-pyrazole-3-carboxylic acid (3-methyl-pyridin-2-ylmethyl)-amide,
5-(2-Chloro-4,5-difluoro-benzoylamino)-1H-pyrazole-3-carboxylic acid (5-methyl-pyridin-3-ylmethyl)-amide,
5-(2-Chloro-4,5-difluoro-benzoylamino)-1H-pyrazole-3-carboxylic acid (6-methyl-pyridin-3-ylmethyl)-amide,
5-(2-Chloro-4,5-difluoro-benzoylamino)-1H-pyrazole-3-carboxylic acid (6-methyl-pyridin-2-ylmethyl)-amide,
5-(2,4-Dichloro-5-fluoro-benzoylamino)-1H-pyrazole-3-carboxylic acid (pyridin-3-ylmethyl)-amide dihydrochloride,
5-(2-Chloro-4,5-difluoro-benzoylamino)-1H-pyrazole-3-carboxylic acid (6-fluoro-pyridin-3-ylmethyl)-amide dihydrochloride,
5-(2-Chloro-4,5-difluoro-benzoylamino)-1H-pyrazole-3-carboxylic acid (3-carboxy-phenyl)-methyl-amide,
5-(2-Chloro-4,5-difluoro-benzoylamino)-1H-pyrazole-3-carboxylic acid 3-methanesulfonylamino-benzylamide,
5-(2-Chloro-4,5-difluoro-benzoylamino)-1H-pyrazole-3-carboxylic acid 4-methanesulfonylamino-benzylamide,
5-(2-Chloro-4,5-difluoro-benzoylamino)-1H-pyrazole-3-carboxylic acid 3-acetylamino-benzylamide,
5-(2-Chloro-4,5-difluoro-benzoylamino)-1H-pyrazole-3-carboxylic acid 4-acetylamino-benzylamide,
5-(2-Chloro-4,5-difluoro-benzoylamino)-1H-pyrazole-3-carboxylic acid (2-phenyl-thiazol-4-ylmethyl)-amide,
5-(2-Chloro-4,5-difluoro-benzoylamino)-1H-pyrazole-3-carboxylic acid ((R)-1-phenyl-ethyl)-amide,
5- (2-Chloro-4,5-difluoro-benzoylamino) -1H-pyrazole-3-carboxylic acid ((S)-1-phenyl-ethyl)-amide,
5- (2-Chloro-4,5-difluoro-benzoylamino) -1H-pyrazole-3-carboxylic acid (6-phenoxy-pyridin-3-ylmethyl)-amide dihydrochloride,
5-(2-Methyl-4,5-difluoro-benzoylamino)-1H-pyrazole-3-carboxylic acid (6-chloro-pyridin-3-ylmethyl)-amide dihydrochloride,
5-(2-Chloro-4,5-difluoro-benzoylamino)-1H-pyrazole-3-carboxylic acid (2-chloro-pyridin-3-ylmethyl)-amide dihydrochloride,
5-(2-Methyl-4,5-difluoro-benzoylamino)-1H-pyrazole-3-carboxylic acid (6-chloro-pyridin-3-ylmethyl)-amide sodium salt,
5-(2-Chloro-4,5-difluoro-benzoylamino)-1H-pyrazole-3-carboxylic acid [1-(pyridin-3-yl)-ethyl]-amide dihydrochloride ,
5-(2-Chloro-4,5-difluoro-benzoylamino)-1H-pyrazole-3-carboxylic acid (2-fluoro-pyridin-3-ylmethyl)-amide dihydrochloride,
5-(2-Chloro-4,5-difluoro-benzoylamino)-1H-pyrazole-3-carboxylic acid (2-methyl-pyridin-3-ylmethyl)-amide dihydrochloride,
5-(2-Chloro-4,5-difluoro-benzoylamino)-1H-pyrazole-3-carboxylic acid (biphenyl-3-ylmethyl)-amide,
5-(2-Chloro-4,5-difluoro-benzoylamino)-1H-pyrazole-3-carboxylic acid ((1R,2S)-2-hydroxy-indan-1-yl)-amide,
5-(2-Chloro-4,5-difluoro-benzoylamino)-1H-pyrazole-3-carboxylic acid ((1S,2R)-2-hydroxy-indan-1-yl)-amide,
5-(2-Chloro-4,5-difluoro-benzoylamino)-1H-pyrazole-3-carboxylic acid (6-phenyl-pyridin-3-ylmethyl)-amide dihydrochloride,
5-(2-Chloro-4,5-difluoro-benzoylamino)-1H-pyrazole-3-carboxylic acid (6-methyl-2-pyridon-3-ylmethyl)-amide hydrochloride,
5-(2-Chloro-4,5-difluoro-benzoylamino)-1H-pyrazole-3-carboxylic acid benzyl-methyl-amide,
5-(2-Chloro-4,5-difluoro-benzoylamino)-1H-pyrazole-3-carboxylic acid benzylamide ,
5-(2-Chloro-4,5-difluoro-benzoylamino)-1H-pyrazole-3-carboxylic acid phenylamide,
5-(2-Chloro-4,5-difluoro-benzoylamino)-1H-pyrazole-3-carboxylic acid methyl-phenyl-amide,
5-(2-Chloro-4,5-difluoro-benzoylamino)-1H-pyrazole-3-carboxylic acid (piperidin-1-yl)-amide,
5-(2-Chloro-4,5-difluoro-benzoylamino)-1H-pyrazole-3-carboxylic acid (4-ethoxycarbonyl-piperidin-1-yl)-amide,
5-(2-Chloro-4,5-difluoro-berizoylamino)-1H-pyrazole-3-carboxylic acid (4-methyl-piperazin-1-yl)-amide,
5-(2-Chloro-4,5-difluoro-benzoylamino)-1H-pyrazole-3-carboxylic acid (4-carboxy-piperidin-1-yl)-amide,
5-(2-Chloro-4,5-difluoro-benzoylamino)-1H-pyrazole-3-carboxylic acid phenethylamide,
5-(2-Chloro-4,5-difluoro-benzoylamino)-1H-pyrazole-3-carboxylic acid methyl-phenethyl-amide,
5-(2-Chloro-4,5-difluoro-benzoylamino)-1H-pyrazole-3-carboxylic acid ethyl-phenyl-amide,
5-(2-Chloro-4,5-difluoro-benzoylamino)-1H-pyrazole-3-carboxylic acid (1,2,3,4-tetrahydroisoquinolin-2-yl)-amide,
5-(2-Chloro-4,5-difluoro-benzoylamino)-1H-pyrazole-3-carboxylic acid ((S)-α-methoxycarbonyl-benzyl)-amide,
5-(2-Chloro-4,5-difluoro-benzoylamino)-1H-pyrazole-3-carboxylic acid ((R)-α-mefhoxycarbonyl-benzyl)-amide,
5-(2-Chloro-4,5-difluoro-benzoylamino)-1H-pyrazole-3-carboxylic acid (1,2,3,4-tetrahydroquinoline-1-yl)-amide,
5-(2-Chloro-4,5-difluoro-benzoylamino)-1H-pyrazole-3-carboxylic acid phenyl-propyl-amide,
5-(2-Chloro-4,5-difluoro-benzoylamino)-1H-pyrazole-3-carboxylic acid butyl-phenyl-amide,
5-(2-Chloro-4,5-difluoro-benzoylamino)-1H-pyrazole-3-carboxylic acid pentyl-phenyl-amide,
5-(2-Chloro-4,5-difluoro-benzoylamino)-1H-pyrazole-3-carboxylic acid benzhydryl-amide,
5-(2-Chloro-4,5-difluoro-benzoylamino)-1H-pyrazole-3-carboxylic acid (pyridin-2-ylmethyl) -amide hydrochloride,
5- (2-Chloro-4,5-difluoro-benzoylamino) -1H-pyrazole-3-carboxylic acid (pyridin-4-ylmethyl) -amide hydrochloride,
5-(2-Chloro-4,5-difluoro-benzoylamino)-1H-pyrazole-3-carboxylic acid ((R)-2-hydroxy-1-phenyl-ethyl)-amide,
5-(2-Chloro-4,5-difluoro-benzoylamino)-1H-pyrazole-3-carboxylic acid ((S) -2-hydroxy-1-phenyl-e'thyl) -amide,
5-(2-Chloro-4,5-difluoro-benzoylamino)-1H-pyrazole-3-carboxylic acid 4-dimethylamino-benzylamide,
5-(2-Chloro-4,5-difluoro-benzoylamino)-1H-pyrazole-3-carboxylic acid (furan-2-ylmethyl)-amide,
5-(2-Chloro-4,5-difluoro-benzoylamino)-1H-pyrazole-3-carboxylic acid (thiophen-2-ylmethyl)-amide,
5-(2-Chloro-4,5-difluoro-benzoylamino)-1H-pyrazole-3-carboxylic acid (2-methoxy-phenyl)-amide,
5-(2-Chloro-4,5-difluoro-benzoylamino)-1H-pyrazole-3-carboxylic acid (2-methoxy-phenyl)-methyl-amide,
5-(2-Chloro-4,5-difluoro-benzoylamino)-1H-pyrazole-3-carboxylic acid 4-dimethylamino-benzylamide hydrochloride,
5-(2-Chloro-4,5-difluoro-benzoylamino)-1H-pyrazole-3-carboxylic acid 3-amino-benzylamide,
5-(2-Chloro-4,5-difluoro-benzoylamino)-1H-pyrazole-3-carboxylic acid 4-amino-benzylamide,
5-(2-Chloro-4,5-difluoro-benzoylamino)-1H-pyrazole-3-carboxylic acid 2-methyl-benzylamide,
5-(2-Chloro-4,5-difluoro-benzoylamino)-1H-pyrazole-3-carboxylic acid 3-methyl-benzylamide,
5-(2-Chloro-4,5-difluoro-benzoylamino)-1H-pyrazole-3-carboxylic acid 3-dimethylamino-benzylamide,
5-(2-Chloro-4,5-difluoro-benzoylamino)-1H-pyrazole-3-carboxylic acid (2-methoxy-pyridin-3-ylmethyl)-amide,
5-(2-Chloro-4,5-difluoro-benzoylamino)-1H-pyrazole-3-carboxylic acid 2-chloro-benzylamide,
5-(2-Chloro-4,5-difluoro-benzoylamino)-1H-pyrazole-3-carboxylic acid 3-chloro-benzylamide,
5-(2-Chloro-4,5-difluoro-benzoylamino)-1H-pyrazole-3-carboxylic acid 3-methoxycarbonyl-benzylamide,
5-(2-Chloro-4,5-difluoro-benzoylamino)-1H-pyrazole-3-carboxylic acid 3-carboxy-benzylamide,
5-(2-Chloro-4,5-difluoro-benzoylamino)-1H-pyrazole-3-carboxylic acid (6-trifluoromethyl-pyridin-3-ylmethyl)-amide,
5-(2-Chloro-4,5-difluoro-benzoylamino)-1H-pyrazole-3-carboxylic acid (2-ethoxy-pyridin-3-ylmethyl)-amide,
5-(2-Chloro-4,5-difluoro-benzoylamino)-1H-pyrazole-3-carboxylic acid (2-dimethylamino-ethyl)-amide,
5-(2-Chloro-4,5-difluoro-benzoylamino)-1H-pyrazole-3-carboxylic acid ethyl-(2-dimethylamino-ethyl)-amide dihydrochloride,
5-(2-Chloro-4,5-difluoro-benzoylamino)-1H-pyrazole-3-carboxylic acid cyclohexyl-(2-dimethylamino-ethyl)-amide,
5-(2-Chloro-4,5-difluoro-benzoylamino)-1H-pyrazole-3-carboxylic acid (4-methoxy-pyridin-2-ylmethyl)-amide,
5-(2-Chloro-4,5-difluoro-benzoylamino)-1H-pyrazole-3-carboxylic acid (2,6-dimethoxy-pyridin-3-ylmethyl)-amide,
5-(2-Chloro-4,5-difluoro-benzoylamino)-1H-pyrazole-3-carboxylic acid 2-methoxycarbonylmethoxy-benzylamide,
5-(2-Chloro-4,5-difluoro-benzoylamino)-1H-pyrazole-3-carboxylic acid 2-carboxymethoy-benzylamide,
5-(4,5-Difluoro-2-methyl-benzoylamino)-1H-pyrazole-3-carboxylic acid (6-methoxy-pyridin-3-ylmethyl)-amide,
5-(2,4-Dichloro-benzoylamino)-1H-pyrazole-3-carboxylic acid (6-methoxy-pyridin-3-ylmethyl)-amide,
5-(2-Chloro-4,5-difluoro-benzoylamino)-1H-pyrazole-3-carboxylic acid (3,5-dimethoxy-pyridin-4-ylmethyl)-amide,
5-(4,5-Difluoro-2-methyl-benzoylamino)-1H-pyrazole-3-carboxylic acid (2,4-dimethyl-oxazol-5-ylmethyl)-amide,
5-(2,4-Dichloro-5-fluoro-benzoylamino)-1H-pyrazole-3-carboxylic acid (2,4-dimethyl-oxazol-5-ylmethyl) -amide,
5-(2-Chloro-4,5-difluorobenzoylamino)-1H-pyrazole-3-carbocylic acid (4-methoxycarbonyl-phenyl)-methyl-amide,
5-(2-Chloro-4,5-difluoro-benzoylamino)-1H-pyrazole-3-carboxylic acid (4-carboxyphenyl)-methyl-amide, 4-{[5-(2-Chloro-4,5-difluoro-benzoylamino)-1H-pyrazole -3-carbonyl]-methyl-amino}-benzoic acid sodium salt,
5-(2-Chloro-4,5-difluoro-benzoylamino)-1H-pyrazole-3-carboxylic acid isopropyl-phenyl-amide,
5-(2-Chloro-4,5-difluoro-benzoylamino)-1H-pyrazole-3-carboxylic acid (ethoxycarbonyl-methyl)-phenyl-amide,
5-(2-Chloro-4,5-difluoro-benzoylamino)-1H-pyrazole-3-carboxylic acid (carboxy-methyl)-phenyl-amide,
5-(2-Chloro-4-fluoro-benzoylamino)-1H-pyrazole-3-carboxylic acid benzylamide,
5-(2,4-Dichloro-benzoylamino)-1H-pyrazole-3-carboxylic acid benzylamide,
5-(2-Chloro-5-fluoro-benzoylamino)-1H-pyrazole-3-carboxylic acid benzylamide,
5-(2-Chloro-4,5-difluoro-benzoylamino)-1H-pyrazole-3-carboxylic acid benzyl-phenyl-amide,
5-(2-Chloro-4,5-difluoro-benzoylamino)-1H-pyrazole-3-carboxylic acid (1-methyl-1-phenyl-ethyl)-amide,
5-(2-Chloro-4,5-difluoro-benzoylamino)-1H-pyrazole-3-carboxylic acid [2-oxo-2-(piperidin-1-yl)-ethyl]-phenyl-amide,
5-(5-Fluoro-2-methyl-benzoylamino)-1H-pyrazole-3-carboxylic acid benzylamide,
5-(5-Chloro-2-methyl-benzoylamino)-1H-pyrazole-3-carboxylic acid benzylamide,
5-(4-Methoxy-2-methyl-benzoylamino)-1H-pyrazole-3-carboxylic acid benzylamide,
5-(4-Chloro-2-methyl-benzoylamino)-1H-pyrazole-3-carboxylic acid benzylamide,
5-(5-Fluoro-2-methoxy-benzoylamino)-1H-pyrazole-3-carboxylic acid benzylamide,
5-(5-Chloro-2-methoxy-benzoylamino)-1H-pyrazole-3-carboxylic acid benzylamide,
5-(2-Chloro-4,5-difluoro-benzoylamino)-1H-pyrazole-3-carboxylic acid (2-ethoxy-ethyl)-phenyl-amide,
5-(2-Fluoro-5-methyl-benzoylamino)-1H-pyrazole-3-carboxylic acid benzylamide,
5-(5-Chloro-2-fluoro-benzoylamino)-1H-pyrazole-3-carboxylic acid benzylamide,
5-(3-Chloro-2,6-difluoro-benzoylamino)-1H-pyrazole-3-carboxylic acid benzylamide,
5-(2-Chloro-3-fluoro-benzoylamino)-1H-pyrazole-3-carboxylic acid benzylamide,
5-(2-Chloro-4,5-difluoro-benzoylamino)-1H-pyrazole-3-carboxylic acid ethylcarbamoylmethyl-phenyl-amide,
5-(2-Chloro-4,5-difluoro-benzoylamino)-1H-pyrazole-3-carboxylic acid isobutyl-phenyl-amide,
5-(2-Chloro-4,5-difluoro-benzoylamino)-1H-pyrazole-3-carboxylic acid (2-methoxycarbonyl-ethyl)-phenyl-amide,
5-(2-Chloro-4,5-difluoro-benzoylamino)-1H-pyrazole-3-carboxylic acid (3-ethoxycarbonyl-propyl)-phenyl-amide,
5-(2-Chloro-4,5-difluoro-benzoylamino)-1H-pyrazole-3-carboxylic acid (2-carboxy-ethyl)-phenyl-amide,
5-(2-Chloro-4,5-difluoro-benzoylamino)-1H-pyrazole-3-carboxylic acid (3-carboxy-propyl)-phenyl-amide,
5- (2-Hydroxy-4-methoxy-benzoylamino) -1H-pyrazole-3-carboxylic acid benzylamide,
5-(2-Hydroxy-5-methoxy-benzoylamino)-1H-pyrazole-3-carboxylic acid benzylamide,
5-(5-Fluoro-2-hydroxy-benzoylamino)-1H-pyrazole-3-carboxylic acid benzylamide,
5-(4-Fluoro-2-trifluoromethyl-benzoylamino)-1H-pyrazole-3-carboxylic acid benzylamide,
5-(5-Fluoro-2-trifluoromethyl-benzoylamino)-1H-pyrazole-3-carboxylic acid benzylamide,
5-(2-Chloro-4,5-difluoro-benzoylamino)-1H-pyrazole-3-carboxylic acid (2-methoxy-ethyl)-phenyl-amide,
5- (2,5-Dimethyl-benzoylamino) -1H-pyrazole-3-carboxylic acid benzylamide,
5-(2-Chloro-5-methyl-benzoylamino)-1H-pyrazole-3-carboxylic acid benzylamide,
5-(2-Chloro-pyridine-3-carbonylamino)-1H-pyrazole-3-carboxylic acid benzylamide,
5-(4-Chloro-pyridine-2-carbonylamino)-1H-pyrazole-3-carboxylic acid benzylamide,
5-(2,6-Dichloro-pyridine-3-carbonylamino)-1H-pyrazole-3-carboxylic acid benzylamide,
5-(2-Chloro-4,5-difluoro-benzoylamino)-1H-pyrazole-3-carboxylic acid butyl-(4-ethoxycarbonyl-phenyl)-amide,
5-(2-Chloro-4,5-difluoro-benzoylamino)-1H-pyrazole-3-carboxylic acid butyl-(4-carboxy-phenyl)-amide,
5-(2-Methyl-pyridine-3-carbonylamino)-1H-pyrazole-3-carboxylic acid benzylamide,
5- (2-Chloro-6-methyl-pyridine-3-carbonylamino) -1H-pyrazole-3-carboxylic acid benzylamide,
5- (3-Chloro-pyridine-4-carbonylamino) -1H-pyrazole-3-carboxylic acid benzylamide,
5- (2-Chloro-4,5-difluoro-benzoylamino) -1H-pyrazole-3-carboxylic acid butyl-(4-ethoxycarbonylmethyl-phenyl)-amide,
5-(2-Chloro-4,5-difluoro-benzoylamino)-1H-pyrazole-3-carboxylic acid butyl-(4-carboxymethyl-phenyl)-amide,
5-(2-Chloro-4,5-difluoro-benzoylamino)-1H-pyrazole-3-carboxylic acid (4-methoxycarbonyl-phenyl)-(2-methoxy-ethyl)-amide,
5-(2-Chloro-4,5-difluoro-benzoylamino)-1H-pyrazole-3-carboxylic acid (4-carboxy-phenyl)-(2-methoxy-ethyl)-amide,
5-(2-Chloro-4,5-difluoro-benzoylamino)-1H-pyrazole-3-carboxylic acid (6-methoxy-pyridin-3-ylmethyl)-amide dihydrochloride,
5-(2-Methyl-4,5-difluoro-benzoylamino)-1H-pyrazole-3-carboxylic acid (6-methoxy-pyridin-3-ylmethyl)-amide dihydrochloride,
5-(2-Chloro-4,5-difluoro-benzoylamino)-1H-pyrazole-3-carboxylic acid (thiazol-4-ylmethyl)-amide dihydrochloride,
5-(2-Chloro-4,5-difluoro-benzoylamino)-1H-pyrazole-3-carboxylic acid 4-[2-(morpholin-4-yl)-ethylcarbamoyl]-benzylamide dihydrochloride,
5-(2-Chloro-4,5-difluoro-benzoylamino)-1H-pyrazole-3-carboxylic acid 4-(morpholin-4-ylcarbamoyl)-benzylamide dihydrochloride, [4-({[5-(2-Chloro-4,5-difluoro-benzoylamino)-1H-pyrazole-3-carbonyl]-amino}-methyl)-benzoylamino]-acetic acid sodium salt, 3-[4-({[5-(2-Chloro-4,5-difluoro-benzoylamino)-1H-pyrazole-3-carbonyl]-amino}-methyl)-benzoylamino]-propionic acid sodium salt,
5-(2-Chloro-4,5-difluoro-benzoylamino)-1H-pyrazole-3-carboxylic acid (3,5-dimethyl-isoxazol-4-ylmethyl)-amide hydrochloride,
5-(2-Chloro-4,5-difluoro-benzoylamino)-1H-pyrazole-3-carboxylic acid (1,3,5-trimethyl-1H-pyrazol-4-ylmethyl)-amide dihydrochloride,
5-(2-Chloro-4,5-difluoro-benzoylamino)-1H-pyrazole-3-carboxylic acid (3,5-dimethyl-isoxazol-4-ylmethyl)-amide methanesulfonate,
5-(2-Chloro-4,5-difluoro-benzoylamino)-1H-pyrazole-3-carboxylic acid 3-[2-(morpholin-4-yl)-ethylcarbamoyl]-benzylamide dihydrochloride,
5-(2-Chloro-4,5-difluoro-benzoylamino)-1H-pyrazole-3-carboxylic acid 3-(morpholin-4-ylcarbamoyl)-benzylamide dihydrochloride,
5-(2-Chloro-4,5-difluoro-benzoylamino)-1H-pyrazole-3-carboxylic acid {2-[2-(morpholin-4-yl)-ethylcarbamoyl]-pyridin-4-ylmethyl}-amide trihydrochloride,
5-(2-Chloro-4,5-difluoro-benzoylamino)-1H-pyrazole-3-carboxylic acid [2-(morpholin-4-ylcarbamoyl)-pyridin-4-ylmethyl]-amide trihydrochloride,
5-(2-Chloro-4,5-difluoro-benzoylamino)-1H-pyrazole-3-carboxylic acid (6-methoxy-pyridin-3-ylmethyl)-amide methanesulfonate,
5-(2-Chloro-4,5-difluoro-benzoylamino)-1H-pyrazole-3-carboxylic acid (6-methoxy-pyridin-3-ylmethyl)-amide hemisulfate
5-(2-Chloro-4,5-difluoro-benzoylamino)-1H-pyrazole-3-carboxylic acid (3,5-dimethoxy-pyridin-4-ylmethyl)-amide dihydrochloride,
5-(2-Chloro-4,5-difluoro-benzoylamino)-1H-pyrazole-3-carboxylic acid (4-aminomethyl-pyridin-2-ylmethyl)-amide trihydrochloride,
5-(2-Chloro-4,5-difluoro-benzoylamino)-1H-pyrazole-3-carboxylic acid (6-chloro-pyridin-3-ylmethyl)-amide methanesulfonate,
5-(2-Chloro-4,5-difluoro-benzoylamino)-1H-pyrazole-3-carboxylic acid (6-chloro-pyridin-3-ylmethyl)-amide dimethanesulfonate,
5-(2-Chloro-4,5-difluoro-benzoylamino)-1H-pyrazole-3-carboxylic acid (2,4-dimethyl-pyridin-3-ylmethyl)-amide hydrochloride,
5-(2-Chloro-4,5-difluoro-benzoylamino)-1H-pyrazole-3-carboxylic acid (pyridin-3-ylmethyl)-amide hydrochloride,
5-(2-Chloro-4,5-difluoro-benzoylamino)-1H-pyrazole-3-carboxylic acid (6-methoxy-pyridin-3-ylmethyl)-propyl-amide dihydrochloride,
5-(2-Chloro-4,5-difluoro-benzoylamino)-1H-pyrazole-3-carboxylic acid [6-(piperidin-1-yl)-pyridin-3-ylmethyl]-amide dihydrochloride,
5-(2-Chloro-4,5-difluoro-benzoylamino)-1H-pyrazole-3-carboxylic acid [6-(morpholin-4-yl)-pyridin-3-ylmethyl]-amide dihydrochloride,
5-(2-Chloro-4,5-difluoro-benzoylamino)-1H-pyrazole-3-carboxylic acid (3,5-dichloro-pyridin-4-ylmethyl)-amide hydrochloride,
5-(2-Chloro-4,5-difluoro-benzoylamino)-1H-pyrazole-3-carboxylic acid (6-dimethylamino-pyridin-3-ylmethyl)-amide dihydrochloride,
5-(2-Chloro-4,5-difluoro-benzoylamino)-1H-pyrazole-3-carboxylic acid (6-dimethylamino-pyridin-3-ylmethyl)-propyl-amide dihydrochloride,
5-(2-Chloro-4,5-difluoro-benzoylamino)-1H-pyrazole-3-carboxylic acid (6-diethylamino-pyridin-3-ylmethyl)-amide dihydrochloride,
5-(2-Chloro-4,5-difluoro-benzoylamino)-1H-pyrazole-3-carboxylic acid (6-chloro-2-dimethylamino-pyridin-3-ylmethyl)-amide dihydrochloride,
5- (2-Chloro-4,5-difluoro-benzoylamino) -1H-pyrazole-3-carboxylic acid (4- [1,2,3] thiadiazol-4-yl-benzyl) -amide hydrochloride,
5-(2-Chloro-4,5-difluoro-benzoylamino)-1H-pyrazole-,3-carboxylic acid (4-methyl-thiazol-5-ylmethyl)-amide hydrochloride,
5-(2-Chloro-4,5-difluoro-benzoylamino)-1H-pyrazole-3-carboxylic acid (2,4-dimethyl-thiazol-5-ylmethyl)-amide hydrochloride,
5-(2-Chloro-4,5-difluoro-benzoylamino)-1H-pyrazole-3-carboxylic acid [6-(4-hydroxy-piperidin-1-yl)-pyridin-3-ylmethyl]-amide dihydrochloride,
5-(2-Chloro-4,5-difluoro-benzoylamino)-1H-pyrazole-3-carboxylic acid 5-methyl-pyrazin-2-ylamide dihydrochloride,
5-(2-Chloro-4,5-difluoro-benzoylamino)-1H-pyrazole-3-carboxylic acid quinolin-5-ylamide dihydrochloride,
5-(2-Chloro-4,5-difluoro-benzoylamino)-1H-pyrazole-3-carboxylic acid quinolin-8-ylamide dihydrochloride,
5-(2-Chloro-4,5-difluoro-benzoylamino)-1H-pyrazole-3-carboxylic acid isoquinolin-5-ylamide dihydrochloride,
5-(2-Chloro-4,5-difluoro-benzoylamino)-1H-pyrazole-3-carboxylic acid [6-(4-methylpiperazin-1-yl)-pyridin-3-ylmethyl]-amide dihydrochloride,
5-(2-Chloro-4,5-difluoro-benzoylamino)-1H-pyrazole-3-carboxylic acid [6-(4-dimethylamino-piperidin-1-yl)-pyridin-3-ylmethyl]-amide dihydrochloride,
5-(2-Chloro-4,5-difluoro-benzoylamino)-1H-pyrazole-3-carboxylic acid [6-(pyrrolidin-1-yl)-pyridin-3-ylmethyl] -amide dihydrochloride,
5-(2-Chloro-4,5-difluoro-benzoylamino)-1H-pyrazole-3-carboxylic acid (pyrazin-2-ylmethyl)-amide dihydrochloride,
5-(2-Chloro-4,5-difluoro-benzoylamino)-1H-pyrazole-3-carboxylic acid [6-(thiomorpholin-4-yl)-pyridin-3-ylmethyl]-amide dihydrochloride,
5- (2-Chloro-4,5-difluoro-benzoylamino) -1H-pyrazole-3-carboxylic acid [6-(1,1-dioxo-116-thiomorpholin-4-yl)-pyridin-3-ylmethyl]-amide dihydrochloride,
5-(2-Chloro-4,5-difluoro-benzoylamino)-1H-pyrazole-3-carboxylic acid carbamoylmethyl-amide,
5-(2,4-Dichloro-5-fluoro-benzoylamino)-1H-pyrazole-3-carboxylic acid (4-carbamoyl-phenyl)-methyl-amide,
5-(2,4-Dichloro-5-fluoro-benzoylamino)-1H-pyrazole-3-carboxylic acid methyl-(4-methylcarbamoyl-phenyl)-amide,
5-(2,4-Dichloro-5-fluoro-benzoylamino)-1H-pyrazole-3-carboxylic acid (4-dimethylcarbamoyl-phenyl)-methyl-amide,
5-(2-Chloro-4,5-difluoro-benzoylamino)-1H-pyrazole-3-carboxylic acid (4-methanesulfonylaminocarbonyl-phenyl)-methyl-amide,
5-(2-Chloro-4,5-difluoro-benzoylamino)-1H-pyrazole-3-carboxylic acid (4-methanesulfonylaminocarbonyl-phenyl)-methyl-amide sodium salt,
5-(2-Chloro-4,5-difluoro-benzoylamino)-1H-pyrazole-3-carboxylic acid (4-cyano-phenyl)-methyl-amide,
5-(2-Chloro-4,5-difluoro-benzoylamino)-1H-pyrazole-3-carboxylic acid methyl-[4-(4H-[1,2,4]triazol-3-yl)-phenyl]-amide hydrochloride,
5- (4-Azido-2-chloro-5-fluoro-benzoylamino) -1H-pyrazole-3-carboxylic acid (4-cyano-phenyl)-methyl-amide,
5- (4-Azido-2-chloro-5-fluoro-benzoylamino) -1H-pyrazole-3-carboxylic acid methyl- [4- (1H-tetrazol-5-yl) -phenyl] -amide,
5-(2-Chloro-4,5-difluoro-benzoylamino)-1H-pyrazole-3-carboxylic acid [4-(N-hydroxycarbamimidoyl)-phenyl]-methyl-amide,
5-(2-Chloro-4,5-difluoro-benzoylamino)-1H-pyrazole-3-carboxylic acid methyl-[4-(5-oxo-2,5-dihydro-[1,2,4]oxadiazol-3-yl)-phenyl]-amide,
5-(2-Chloro-4,5-difluoro-benzoylamino)-1H-pyrazole-3-carboxylic acid {4-[(2,2-dimethyl-propionyloxy)-methoxycarbonyl]-phenyl}-methyl-amide,
5-(2-Chloro-4,5-difluoro-benzoylamino)-1H-pyrazole-3-carboxylic acid 4-[N-(methoxy-thiocarbonyloxy)-carbamimidoyl]-methyl-amide,
5-(2-Chloro-4,5-difluoro-benzoylamino)-1H-pyrazole-3-carboxylic acid methyl-[4-(5-thioxo-4,5-dihydro-[1,2,4]oxadiazol-3-yl) -phenyl]-amide,
5-(2-Chloro-4,5-difluoro-benzoylamino)-1H-pyrazole-3-carboxylic acid methyl-[4-(5-oxo-4,5-dihydro-[1,2,4]thiadiazol-3-yl)-phenyl] -amide,
5-(2-Chloro-4,5-difluoro-benzoylamino)-1H-pyrazole-3-carboxylic acid {4-[1-(cyclohexyloxy-carbonyloxy)-ethoxycarbonyl]-phenyl}-methyl-amide,
5-(2-Chloro-4,5-difluoro-benzoylamino)-1H-pyrazole-3-carboxylic acid (4-ethoxycarbonyl-thiazol-2-yl)-ethyl-amide,
5-(2-Chloro-4,5-difluoro-benzoylamino)-1H-pyrazole-3-carboxylic acid 2-methanesulfonylamino-benzylamide,
5-(2-Chloro-4,5-difluoro-benzoylamino)-1H-pyrazole-3-carboxylic acid 2-acetylamino-benzylamide,
5-(2-Chloro-4,5-difluoro-benzoylamino)-1H-pyrazole-3-carboxylic acid 2-(dimethylamino-methyleneamino)-benzylamide hydrochloride,
5-(2-Chloro-4,5-difluoro-benzoylamino)-1H-pyrazole-3-carboxylic acid 3-(dimethylamino-methyleneamino)-benzylamide hydrochloride,
5-(2-Chloro-4,5-difluoro-benzoylamino)-1H-pyrazole-3-carboxylic acid 4-(dimethylamino-methyleneamino)-benzylamide hydrochloride,
5-(2-Chloro-4,5-difluoro-benzoylamino)-1H-pyrazole-3-carboxylic acid 2-[1-(dimethylamino)ethylideneamino]-benzylamide dihydrochloride,
5-(2-Chloro-4,5-difluoro-benzoylamino)-1H-pyrazole-3-carboxylic acid 3-[1-(dimethylamino)ethylideneamino]-benzylamide dihydrochloride,
5-(2-Chloro-4,5-difluoro-benzoylamino)-1H-pyrazole-3-carboxylic acid 4-[1-(dimethylamino)ethylideneamino]-benzylamide dihydrochloride,
5-(2-Chloro-4,5-difluoro-benzoylamino)-1H-pyrazole-3-carboxylic acid 2-(diethylamino-methyleneamino)-benzylamide dihydrochloride,
5-(2-Chloro-4,5-difluoro-benzoylamino)-1H-pyrazole-3-carboxylic acid 3-(diethylamino-methyleneamino)-benzylamide dihydrochloride,
5-(2-Chloro-4,5-difluoro-benzoylamino)-1H-pyrazole-3-carboxylic acid 4-(diethylamino-methyleneamino)-benzylamide dihydrochloride,
5-(2-Chloro-4,5-difluoro-benzoylamino)-1H-pyrazole-3-carboxylic acid 2-acetimidoylamino-benzylamide dihydrochloride,
5-(2-Chloro-4,5-difluoro-benzoylamino)-1H-pyrazole-3-carboxylic acid 3-acetimidoylamino-benzylamide dihydrochloride,
5-(2-Chloro-4,5-difluoro-benzoylamino)-1H-pyrazole-3-carboxylic acid 4-acetimidoylamino-benzylamide dihydrochloride,
5-(2-Chloro-4,5-difluoro-benzoylamino)-1H-pyrazole-3-carboxylic acid 4-cyano-benzylamide,
5-(2-Chloro-4,5-difluoro-benzoylamino)-1H-pyrazole-3-carboxylic acid 3-[(2-fluoro-benzimidoyl)-amino]-benzylamide hyrdoiodide,
5-(2-Chloro-4,5-difluoro-benzoylamino)-1H-pyrazole-3-carboxylic acid 4-[(2-fluoro-benzimidoyl)-amino]-benzylamide hyrdoiodide,
5-(2-Chloro-4,5-difluoro-benzoylamino)-1H-pyrazole-3-carboxylic acid 3-(3,3-dimethyl-ureido)-benzylamide,
5-(2-Chloro-4,5-difluoro-benzoylamino)-1H-pyrazole-3-carboxylic acid 4-(3,3-dimethyl-ureido)-benzylamide,
5- (2-Chloro-4,5-difluoro-benzoylamino) -1H-pyrazole-3-carboxylic acid 2-(toluene-4-sulfonylamino)-benzylamide,
5-(2-Chloro-4,5-difluoro-benzoylamino)-1H-pyrazole-3-carboxylic acid 2-amino-6-fluoro-benzylamide hydrochloride,
5-(2-Chloro-4,5-difluoro-benzoylamino)-1H-pyrazole-3-carboxylic acid 2-amino-4,5-difluoro-benzylamide hydrochloride,
5-(2-Chloro-4,5-difluoro-benzoylamino)-1H-pyrazole-3-carboxylic acid (indan-1-yl)-amide,
5-(2-Chloro-4,5-difluoro-benzoylamino)-1H-pyrazole-3-carboxylic acid (indan-2-yl)-amide,
5-(2-Chloro-4,5-difluoro-benzoylamino)-1H-pyrazole-3-carboxylic (4H-quinazoline-3-yl)-amide,
5-(2-Chloro-4,5-difluoro-benzoylamino)-1H-pyrazole-3-carboxylic (4H-quinazoline-3-yl)-amide dihydrochloride,
5-(2-Chloro-4,5-difluoro-benzoylamino)-1H-pyrazole-3-carboxylic acid (7-fluoro-4H-quinazoline-3-yl)-amide dihydrochloride,
5-(2-Chloro-4,5-difluoro-benzoylamino)-1H-pyrazole-3-carboxylic acid (6-fluoro-4H-quinazoline-3-yl)-amide hydrochloride, 5-(2-Chloro-4,5-difluoro-benzoylamino)-1H-pyrazole-3-carboxylic acid (2-ethoxy-2-methyl-1,4-dihydro-2H-quinazoline-3-yl)-amide,
5-(2-Chloro-4,5-difluoro-benzoylamino)-1H-pyrazole-3-carboxylic acid (2-cyano-pyridin-4-ylmethyl)-amide,
5-(2-Chloro-4,5-difluoro-benzoylamino)-1H-pyrazole-3-carboxylic acid [2-(N-hydroxycarbamimidoyl)-pyridin-4-ylmethyl]-amide,
5-(2-Chloro-4,5-difluoro-benzoylamino)-1H-pyrazole-3-carboxylic acid [2-(5-oxo-4,5-dihydro-[1,2,4]oxadiazol-3-yl)-pyridin-4 -ylmethyl]-amide, 4-{[5-(4,5-difluoro-2-methyl-benzoylamino)-1H-pyrazole -3-carbonyl]-methyl-aminol-benzoic acid sodium salt,
5-(4,5-Difluoro-2-methyl-benzoylamino)-1H-pyrazole-3-carboxylic acid methyl-[4-(1H-tetrazol-5-yl)-phenyl]-amide,
5-(2,4-Dichloro-benzoylamino)-1H-pyrazole-3-carboxylic acid (pyridin-3-ylmethyl)-amide,
5-(2,4-Dichloro-5-fluoro-benzoylamino)-1H-pyrazole-3-carboxylic acid (pyridin-3-ylmethyl)-amide,
5-(2,6-Dichloro-5-fluoro-pyridine-3-carbonylamino)-1H-pyrazole-3-carboxylic acid (pyridin-3-ylmethyl)-amide,
5-[(3-Chloro-benzo[b]thiophene-2-carbonyl)-amino]-1H-p yrazole-3-carboxylic acid (pyridin-3-ylmethyl)-amide,
5-[(3-Chloro-thiophene-2-carbonyl)-amino]-1H-pyrazole-3-carboxylic acid (pyridin-3-ylmethyl)-amide,
5-Benzoylamino-1H-pyrazole-3-carboxylic acid (pyridin-3-ylmethyl)-amide,
5-Phenylacetylamino-1H-pyrazole-3-carboxylic acid (pyridin-3-ylmethyl)-amide,
5-(2-Methyl-4,5-difluoro-benzoylamino)-1H-pyrazole-3-carboxylic acid (pyridin-3-ylmethyl)-amide dihydrochloride,
5-(2-Amino-4,5-difluoro-benzoylamino)-1H-pyrazole-3-carboxylic acid (pyridin-3-ylmethyl)-amide,
5-(4,5-Difluoro-2-methanesulfonylamino-benzoylamino)-1H-pyrazole-3-carboxylic acid (pyridin-3-ylmethyl)-amide,
5-(2-Acetylamino-4,5-difluoro-benzoylamino)-1H-pyrazole-3-carboxylic acid (pyridin-3-ylmethyl)-amide,
5-[(3,4,5-Trichloro-thiophene-2-carbonyl)-amino]-1H-pyrazole-3-carboxylic acid (pyridin-3-ylmethyl)-amide,
5-(4,5-Difluoro-2-methyl-benzoylamino)-1H-pyrazole-3-carboxylic acid (2-cyano-pyridin-4-ylmethyl)-amide,
5- (4,5-Difluoro-2-methyl-benzoylamino) -1H-pyrazole-3-carboxylic acid [2-(1H-tetrazol-5-yl) -pyridin-4-ylmethyl]-amide, and 1H-Pyrazole-3,5-dicarboxylic acid 3-benzylamide
5-[(2,4-dichloro-phenyl)-amide].

21. A medicine comprising a combination of a pharmaceutical composition for treating or preventing diabetes according to any of claims 1 to 20 and another therapeutic or prophylactic agent for diabetes.

22. The medicine according to claim 21, wherein the other therapeutic or prophylactic agent for diabetes is an insulin preparation (injection), a low molecular insulin oral agent, a sulfonylurea receptor agonist (SU agent), anon-sulfonylurea insulinotropic agent, a potassium-dependent ATP (KATP) channel opening agent, an α glucosidase inhibitor, an α amylase inhibitor, an insulin sensitizer, a low molecular tGLP-1 receptor agonist, a tGLP-1 peptide analogue, a dipeptidyl peptidase IV (DPP-IV) inhibitor, a glucagon receptor antagonist, a glucocorticoid receptor antagonist, abiguanide, an SGLUT inhibitor, a fructose-1,6- bisphosphatase (FBPase) inhibitor, a glycogen synthase kinase 3 (GSK-3) inhibitor, a phosphoenolpyruvate carboxykinase (PEPCK) inhibitor, a protein tyrosine phosphatase 1B (PTPase1B) inhibitor, an SH2 domain containing inositol phosphatase (SHIP2) inhibitor, a glycogen phosphorylase (GP) inhibitor, a glucokinase activator, a GPR40 receptor agonist, a pyruvate dehydrogenase kinase (PDHK) inhibitor, a glutamine: fructose-6-phosphate aminotransferase (GFAT) inhibitor, an antioxidant; a nitric monoxide scavenger, a carnitine palmitoyltransferase 1 (CPT-1) inhibitor, a growth hormone-release factor (GHRF), a triacylglycerol lipase (hormone-sensitive lipase) inhibitor, a PPAR γ receptor agonist, a PPAR γ receptor antagonist, a PPAR α/γ receptor agonist, an AMP activation protein kinase (AMPK) activator, an adiponectin receptor agonist or a β3 adrenoceptor agonist.

23. The medicine according to claim 21 or 22, wherein the other therapeutic or prophylactic agent for diabetes is insulin, tolbutamide, glyclopyramide, acetohexamide, chlorpropamide, glybuzole, glibenclamide, gliclazide, glimepiride, mitiglinide, repaglinide, nateglinide, voglibose, acarbose, miglitol, rosiglitazone maleate, metformin hydrochloride, pioglitazone hydrochloride or buformin hydrochloride.

24. A medicine comprising a combination of a pharmaceutical composition for treating or preventing diabetes according to any of claims 1 to 20 and another therapeutic or prophylactic agent for diabetic complications.

25. The medicine according to claim 24, wherein the other therapeutic or prophylactic agent for diabetic complications is a protein kinase β (PKC β) inhibitor, an angiotensin II receptor antagonist, an aldose reductase inhibitor, an angiotensin converting enzyme (ACE) inhibitor, an advanced glycation end product(AGE)productioninhibitor,a neuropathy therapeutic agent or a diabetic nephropathy therapeutic agent.

26. The medicine according to claim 24 or 25, wherein the other therapeutic or prophylactic agent for diabetic complications is epalrestat, mexiletine hydrochloride or imidapril hydrochloride.

27. A medicine comprising a combination of a pharmaceutical composition for treating or preventing diabetes according to any of claims 1 to 20 and another therapeutic or prophylactic agent for hyperlipidemia.

28. The medicine according to claim 27, wherein the other therapeutic or prophylactic agent for hyperlipidemia is a fibrate (PPARα receptor agonist), a PPARδ receptor agonist, a microsome triglyceride transfer protein (MTP) inhibitor, a cholesteryl ester transfer protein (CETP) inhibitor, astatin (HMG-CoA reductase inhibitor), an anion exchanger, probucol, a nicotinic drug, a plant sterol, an apolipoprotein-A1 (Apo-A1) inducer, a lipoprotein lipase (LPL) activator, an endodermis lipase inhibitor, ezetimibu, an IBAT inhibitor, a squalene synthesis enzyme inhibitor, an ACAT inhibitor, an LXR receptor agonist, an FXR receptor agonist, an FXR receptor antagonist or an adenosine A1 agonist.

29. The medicine according to claim 27 or 28, wherein the other therapeutic or prophylactic agent for hyperlipidemia is clofibrate, bezafibrate, fenofibrate, lovastatin, simvastatin, pravastatin, fluvastatin, atorvastatin, pitavastatin, rosuvastatin, cerivastatin, cholestyramine, colestyramine, tocopherol nicotinate, nicomol, niceritrol, soysterol or gamma orizanol.

30. A medicine comprising a combination of a pharmaceutical composition for treating or preventing diabetes according to any of claims 1 to 20 and another therapeutic or prophylactic agent for obesity.

31. The medicine according to claim 30, wherein the other therapeutic or prophylactic agent for obesity is a leptin preparation, a pancreatic lipase inhibitor, a noradrenaline-serotonin reuptake inhibitor, a cannabinoid receptor antagonist, a monoamine reuptake inhibitor, a diacylglycerol acyltransferase (DGAT) inhibitor, a glucose-dependent insulinotropic polypeptide (GIP) receptor antagonist, a leptin receptor agonist, a bombesin receptor subtype 3 (BRS-3) agonist, aperilipin inhibitor, anacetyl-CoA carboxylase 1 (ACC1) inhibitor, an acetyl-CoA carboxylase 2 (ACC2) inhibitor, a fatty acid synthase inhibitor, an sn-1-acyl-glycerol-3-phosphate acyltransferase (AGPAT) inhibitor, a pancreas phospholipase A2 (pPLA2) inhibitor, a melanocortin (MC) receptor agonist, a neuropeptide Y5 (NPY5) receptor antagonist, an uncoupling protein (UCP) inducer or activator, a carnitine palmitoyltransferase 1 (CPT-1) activator, a CCK1 (CCKA) agonist, a ciliaryneurotrophic factor (CNTF), a CRF2 agonist, a neuropeptide Y2 (NPY2) receptor antagonist, a neuropeptide Y4 (NPY4) receptor antagonist, a thyroid hormone receptor β agonist, a growth hormone, an ATP citrate lyase inhibitor, a 5-HT6 antagonist or a 5-HT2C agonist.

32. The medicine according to claim 30 or 31, wherein the other therapeutic or prophylactic agent for obesity is leptin, orlistat, sibutramine, rimonabant or mazindol.

33. A medicine comprising a combination of a pharmaceutical composition for treating or preventing diabetes according to any of claims 1 to 20 with another therapeutic or prophylactic agent for hypertension.

34. The medicine according to claim 33, wherein the other therapeutic or prophylactic agent for hypertension is a thiazide diuretic, a similar thiazide diuretic, a loop diuretic, a K retaining diuretic, a β blocker, an α, β blocker, an α blocker, a central sympathetic nerve depressant, a peripheral sympathetic nerve depressant (rauwolfia preparation), a Ca antagonist (benzothiazepin), a Ca antagonist (dihydropyridine), a vasodilator, an angiotensin converting enzyme (ACE) inhibitor, an angiotensin II receptor antagonist, a nitric acid preparation, an endothelin ETA receptor antagonist, an endothelin-converting enzyme inhibitor; a neprilysin inhibitor, a prostaglandin; a prostanoid FP agonist, a renin inhibitor, an NOS3 expression enhancer; a prostacyclin analogue, a phosphodiesterase V (PDE5A) inhibitor, a prostacyclin analogue or an aldosterone antagonist.

35. The medicine according to claim 33 or 34, wherein the other therapeutic or prophylactic agent for hypertension is hydrochlorothiazide, trichlormethiazide, bentylhydrochlorothiazide, meticrane, indapamide, tripamide, chlortalidone, mefruside, furosemide, spironolactone, triamteren, atenolol, bisoprolol fumarate, betaxolol hydrochloride, bevantololhydrochloride, metoprololtartrate, acebutolol hydrochloride, celiprolol hydrochloride, nipradilol, tilisolol hydrochloride, nadolol, propranolol hydrochloride, indenolol hydrochloride, carteolol hydrochloride, pindolol, pindolol sustained-release tablet, bunitrolol hydrochloride, penbutolol sulfate, bopindolol malonate, amosulalol hydrochloride, arotinolol hydrochloride, carvedilol, labetalol hydrochloride, urapidil, terazosin hydrochloride, doxazosin mesilate, bunazosin hydrochloride, prazosin hydrochloride, phentolamine mesilate, clonidine hydrochloride, guanfacine hydrochloride, guanabenz acetate, methyldopa, reserpine, rescinnamine, amlodipine besilate, aranidipine, efonidipine hydrochloride, cilnidipine, nicardipine hydrochloride, nisoldipine, nitrendipine, nifedipine, sustained-release nifedipine, nilvadipine, barnidipine hydrochloride, felodipine, benidipine hydrochloride, manidipine hydrochloride, azelnidipine, diltiazem hydrochloride, hydrazine monohydrochloride, todoralazine hydrochloride, budralazine, cadralazine, captopril, enalapril maleate, alacepril, delapril hydrochloride, cilazapril, lisinopril, benazepril hydrochloride, imidapril hydrochloride, tamocapril hydrochloride, quinapril hydrochloride, trandolapril, perindopril erbumine, candesartan cilexetil, valsartan, telmisartan, olmesartan medoxomil, sodium nitroprusside or nitroglycerine.

36. A pyrazole compound represented by the following general formula (I): 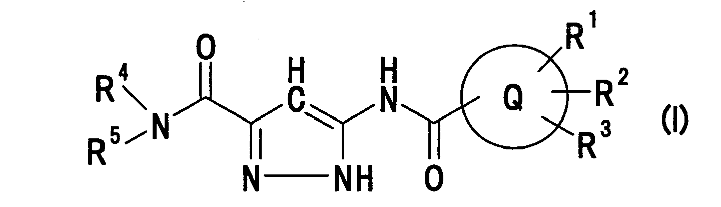 wherein,
Ring Q represents an aryl group or a heteroaromatic group;
R¹ represents a hydrogen atom, a halogen atom, a C₁₋₆ alkyl group or a C₁₋₆ alkoxy group;
R² represents a halogen atom, a C₁₋₆ alkyl group, a C₁₋₆ alkoxy group or an azido group;
R³ represents a halogen atom, a hydroxyl group, a C₁₋₆ alkyl group, a halo C₁₋₆ alkyl group, a C₁₋₆ alkoxy group, an azido group, an amino group, an acylamino group or a C₁₋₆ alkylsulfonylamino group; and
R⁴ and R⁵ are identical with or different from each other, and represent
(1) a hydrogen atom;
(2) a C₁₋₆ alkyl group that may be substituted with one or more substituents selected from Group A below:
[Group A]
A1. a hydroxyl group,
A2. a C₁₋₆ alkoxy group,
A3. -N(R⁴¹) (R^{41'})
wherein R⁴¹ and R^{41'} are identical with or different from each other and represent a hydrogen atom or a C₁₋₆ alkyl group, or R⁴¹ and R^{41'} may, together with the adjacent nitrogen atom, forma saturated 5- or 6-memberedmonocyclic heterocyclic ring,
A4. an aryl group,
A5. a heteroaromatic group,
A6. -CO-N(R⁴¹¹) (R^{411'})
wherein R⁴¹¹ and R^{411'} are identical with or different from each other and represent a hydrogen atom or a C₁₋₆ alkyl group, or R⁴¹¹ and R^{411'} may, together with the adjacent nitrogen atom, forma saturated 5- or 6-memberedmonocyclic heterocyclic ring,
A7. a carboxy group,
and,
A8. a C₁₋₆ alkoxycarbonyl group,
(3) a C₂₋₆ alkenyl group;
(4) a C₂₋₆ alkynyl group;
(5) a C₃₋₈ cycloalkyl group;
wherein the cycloalkyl group may be substituted with a hydroxyl group, a carboxy group or a C₁₋₆ alkoxycarbonyl group or may be condensed with a pyridine ring,
(6) a C₃₋₈ cycloalkyl C₁₋₆ alkyl group;
wherein the cycloalkyl group of the C₃₋₈ cycloalkyl C₁₋₆ alkyl group may be substituted with a hydroxyl group, a carboxy group or a C₁₋₆ alkoxycarbonyl group or may be condensed with a pyridine ring,
(7) a saturated 5- or 6-memberedmonocyclic heterocyclic group that may be substituted with one or more substituents selected from Group C below:
[Group C]
C1. a C₁₋₆ alkyl group,
C2. an acyl group,
C3. a C₁₋₆ alkylsulfonyl group,
C4. a carboxy group,
C5. a C₁₋₆ alkoxycarbonyl group,
and,
C6. -CO-(Alk)n-COOR⁵²,
wherein R⁵² is a hydrogen atom or a C₁₋₆ alkyl group, Alk is a C₁₋₄ alkylene group, and n is 0 or an integer from 1 to 3,
(8) an aryl group that may be substituted with one or more substituents selected from Group D below:
[Group D]
D1. a hydroxyl group,
D2. a C₁₋₆ alkoxy group,
D3. a cyano group,
D4. a C₁₋₆ alkyl group,
wherein the C₁₋₆ alkyl group may be substituted with one or more substituents selected from the group consisting of a hydroxyl group, a carboxy group and a C₁₋₆ alkoxycarbonyl group,
D5. -N(R⁵³) (R^{53'}),
wherein R⁵³ and R^{53'} are identical with or different from each other and represent a hydrogen atom, a C₁₋₆ alkyl group or a C₁₋₆ alkylsulfonyl group,
D6. -CO-N(R⁵³¹) (R^{531'})
wherein R⁵³¹ and R^{531'} are identical with or different from each other and represent a hydrogen atom, a C₁₋₆ alkyl group or a C₁₋₆ alkylsulfonyl group,
D7. -COOR⁵⁴,
wherein _{R}⁵⁴ is a hydrogen atom, a C₁₋₆ alkyl group, a C₁₋₆ alkylcarbonyloxy C₁₋₆ alkyl group or a C₃₋₈ cycloalkyloxycarbonyloxy C₁₋₆ alkyl group,
D8. -C(NH(OH))=N-R⁵⁵,
wherein R⁵⁵ is a hydrogen atom or a C₁₋₆ alkylsulfonyl group,
D9. a saturated 5- or 6-membered monocyclic heterocyclic group,
and,
D10. a 5- or 6-membered monocyclic heteroaromatic group that may be substituted with an oxo or thioxo group,
(9) a 5- or 6-membered monocyclic heteroaromatic group or a 5- or 6-membered condensed heteroaromatic group with a benzene ring, wherein the 5- or 6-membered heteroaromatic groups may be substituted with one or more substituents selected from the group consisting of a carboxy group and a C₁₋₆ alkoxycarbonyl group;
(10) a C₇₋₁₄ aralkyl group;
wherein the alkyl moiety of the C₇₋₁₄ aralkyl group may be substituted with one or two substituents selected from Group E below, and the aryl moiety may be substituted with one or more substituents selected from Group F below:
[Group E]
E1. a C₁₋₆ alkyl group that may be substituted with a hydroxyl group,
E2. a cyano group,
E3. a carboxy group,
E4. a C₁₋₆ alkoxycarbonyl group,
and,
E5. a phenyl group,
[Group F]
F1. a C₁₋₆ alkyl group,
F2. a halogen atom,
F3. a cyano group,
F4. a hydroxyl group,
F5. a C₁₋₆ alkoxy group that may be substituted with one or more substituents selected from the group consisting of a carboxy group and a C₁₋₆ alkoxycarbonyl group,
F6. a halo C₁₋₆ alkyl group,
F7. a carboxy group,
F8. a C₁₋₆ alkoxycarbonyl group,
F9. -CO-N(R ^{56a}) (R^{56a'}),
wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f below:
[Group f]
f1. an amino group,
f2. a mono C₁₋₆ alkylamino group,
f3. a di C₁₋₆ alkylamino group,
f4. a carboxy group,
f5. a C₁₋₆ alkoxycarbonyl group,
f6. a.hydroxyl group,
and,
f7. a saturated 5- or 6-membered monocyclic heterocyclic group having at least one nitrogen atom,
F10. -N (R^{56b}) (R^{56b'})
wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
F11. -N=CR⁵⁷(-N(R⁵⁸) (R^{58'})),
wherein R⁵⁷ is a hydrogen atom or a C₁₋₆ alkyl group, R⁵⁸ and R^{58'} are identical with or different from each other and represent a hydrogen atom or a C₁₋₆ alkyl group,
F12. a 5- or 6-membered monocyclic heteroaromatic group,
and,
F13. a methylenedioxy group or an ethylenedioxy group, or,
(11) a C₁₋₆ alkyl group substituted with one or more substituents selected from the group consisting of a 5- or 6-membered monocyclic heteroaromatic group and a 5- or 6-membered condensed heteroaromatic group with a benzene ring;
wherein the heteroaromatic groups may be substituted with one or more substituents selected from Group G below: [Group G]
G1. a C₁₋₆ alkyl group that may be substituted with one or more substituents selected from Group g below:
[Group g]
g1. a halogen atom,
g2. an amino group,
g3. a mono C₁₋₆ alkylamino group,
g4. a di C₁₋₆ alkylamino group,
g5. a C₁₋₆ alkoxycarbonylamino group,
and,
g6. a hydroxyimino group,
G2. a halogen atom,
G3. a C₁₋₆ alkoxy group that may be substituted with a halogen atom,
G4. an aryloxy group,
G5. a cyano group,
G6. -N(R^{59a}) (R^{59a'}),
wherein R^{59a} and R^{59a'} are identical with or different from each other and represent a hydrogen atom or a C₁₋₆ alkyl group, or R^{59a} and R^{59a'} may, together with the adjacent nitrogen atom, forma saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
G7. -CO-N(R^{59b}) (R^{59b'})
wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
G8. an aryl group,
and,
G9. a 5- or 6-membered monocyclic heteroaromatic group that may be substituted with an oxo or thioxo group,
R⁴ and R⁵ may, together with the adjacent nitrogen atom, form a saturated 5- or 6-membered monocyclic heterocyclic ring, wherein a part of the saturated heterocyclic ring may have a double bond, and the saturated heterocyclic ring may be condensed with a benzene ring to form a condensed ring, the saturated heterocyclic ring which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof.

37. The pyrazole compound according to claim 36 represented by the following general formula (I): 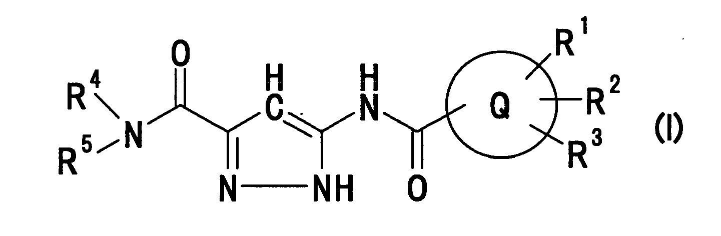 wherein Ring Q represents an aryl group or a heteroaromatic group;
R¹ represents a hydrogen atom, a halogen atom, a C₁₋₆ alkyl group or a C₁₋₆ alkoxy group;
R² represents a halogen atom, a C₁₋₆ alkyl group, a C₁₋₆ alkoxy group or an azido group;
R³ represents a halogen atom, a hydroxyl group, a C₁₋₄ alkyl group, a halo C₁₋₆ alkyl group, a C₁₋₆ alkoxy group, an azido group, an amino group, an acylamino group or a C₁₋₆ alkylsulfonylamino group;
R⁴ represents
(1) a hydrogen atom;
(2) a C₁₋₆ alkyl group that may be substituted with one or more substituents selected from Group A below:
[Group A]
A1. a hydroxyl group,
A2. a C₁₋₆ alkoxy group,
A3. -N(R⁴¹) (R^{41'}),
wherein R⁴¹ and R^{41'} are identical with or different from each other and represent a hydrogen atom or a C₁₋₆ alkyl group, or R⁴¹ and R^{41'} may, together with the adjacent nitrogen atom, form a saturated 5- or 6-memberedmonocyclic heterocyclic ring,
A4. an aryl group,
A5. a heteroaromatic group,
A6. -CO-N (R⁴¹¹) (R^{411'}),
wherein R⁴¹¹ and R^{411'} are identical with or different from each other and represent a hydrogen atom or a C₁₋₆ alkyl group, or R⁴¹¹ and R^{411'} may, together with the adjacent nitrogen atom, form a saturated 5- or 6-memberedmonocyclic heterocyclic ring,
A7. a carboxy group,
and,
A8. a C₁₋₆ alkoxycarbonyl group,
(3) a C₂₋₆ alkenyl group;
(4) a C₂₋₆ alkynyl group;
(5) a C₃₋₈ cycloalkyl group;
(6) a C₃₋₈ cycloalkyl C₁₋₆ alkyl group;
or,
(7) an aryl group; and
R⁵ represents
(1) a C₁₋₆ alkyl group that may be substituted with one or more substituents selected from Group B below:
[Group B]
B1. a hydroxyl group,
B2. a C₁₋₆ alkoxy group,
B3 . -N (R⁵¹) (R⁵¹),
wherein _{R}⁵¹ and R⁵¹ ' are identical with or different from each other and represent a hydrogen atom or a C₁₋₆ alkyl group, or R⁵¹ and R⁵¹ may, together with the adjacent nitrogen atom, forma saturated 5- or 6-memberedmonocyclic heterocyclic ring, and,
B4. -CO-N(R⁵¹¹) (R^{511')},
wherein R⁵¹¹ and R^{511'} are identical with or different from each other and represent a hydrogen atom or a C₁₋₆ alkyl group, or _{R}⁵¹¹ and R^{511'} may, together with the adjacent nitrogen atom, form a saturated 5- or 6-memberedmonocyclic heterocyclic ring,
(2) a C₃₋₈ cycloalkyl group;
wherein the cycloalkyl group may be substituted with a hydroxyl group, a carboxy group or a C₁₋₆ alkoxycarbonyl group or may be condensed with a pyridine ring,
(3) a C₃₋₈ cycloalkyl C₁₋₆ alkyl group;
wherein the cycloalkyl group of the C₃₋₈ cycloalkyl C₁₋₆ alkyl group may be substituted with a hydroxyl group, a carboxy group or a C₁₋₆ alkoxycarbonyl group or may be condensed with a pyridine ring,
(4) a saturated 5- or 6-memberedmonocyclic heterocyclic group that may be substituted with one or more substituents selected from Group C below:
[Group C]
C1. a C₁₋₆ alkyl group,
C2. an acyl group,
C3. a C₁₋₆ alkylsulfonyl group,
C4. a carboxy group,
C5. a C₁₋₆ alkoxycarbonyl group,
and,
C6. -CO-(Alk)n-COOR⁵²,
wherein R⁵² is a hydrogen atom or a C₁₋₆ alkyl group, Alk is a C₁₋₄ alkylene group, and n is 0 or an integer from 1 to 3,
(5) an aryl group that may be substituted with one or more substituents selected from Group D below:
[Group D]
D1. a hydroxyl group,
D2. a C₁₋₆ alkoxy group,
D3. a cyano group,
D4. a C₁₋₆ alkyl group,
wherein the C₁₋₆ alkyl group may be substituted with one or more substituents selected from the group consisting of a hydroxyl group, a carboxy group and a C₁₋₆ alkoxycarbonyl group,
D5. -N(R⁵³) (R^{53'}),
wherein R⁵³ and R^{53'} are identical with or different from each other and represent a hydrogen atom, a C₁₋₆ alkyl group or a C₁₋₆ alkylsulfonyl group,
D6 . -CO-N(R⁵³¹) (R^{531'}),
wherein R⁵³¹ and R^{531'} are identical with or different from each other and represent a hydrogen atom, a C₁₋₆ alkyl group or a C₁₋₆ alkylsulfonyl group,
D7. -COOR⁵⁴,
wherein R⁵⁴ is a hydrogen atom, a C₁₋₆ alkyl group, a C₁₋₆ alkylcarbonyloxy C₁₋₆ alkyl group or a C₃₋₈ cycloalkyloxycarbonyloxy C₁₋₆ alkyl group,
D8. -C(NH(OH))=N-R⁵⁵,
wherein R⁵⁵ is a hydrogen atom or a C₁₋₆ alkylsulfonyl group,
D9. a saturated 5- or 6-membered monocyclic heterocyclic group,
and,
D10. a 5- or 6-membered monocyclic heteroaromatic group that may be substituted with an oxo or thioxo group,
(6) a 5- or 6-membered monocyclic heteroaromatic group or a 5- or 6-membered condensed heteroaromatic group with a benzene ring, wherein the 5- or 6-membered heteroaromatic groups may be substituted with one or more substituents selected from the group consisting of a carboxy group and a C₁₋₆ alkoxycarbonyl group;
(7) a C₇₋₁₄ aralkyl group;
wherein the alkyl moiety of the C₇₋₁₄ aralkyl group may be substituted with one or two substituents selected from Group E below, and the aryl moiety may be substituted with one or more substituents selected from Group F below:
[Group E]
E1. a C₁₋₆ alkyl group that may be substituted with a hydroxyl group,
E2. a cyano group,
E3. a carboxy group,
E4. a C₁₋₆ alkoxycarbonyl group,
and,
E5. a phenyl group,
[Group F]
F1. a C₁₋₆ alkyl group,
F2. a halogen atom,
F3. a cyano group,
F4. a hydroxyl group,
F5. a C₁₋₆ alkoxy group that may be substituted with one or more substituents selected from the group consisting of a carboxy group and a C₁₋₆ alkoxycarbonyl group,
F6. a halo C₁₋₆ alkyl group,
F7. a carboxy group,
F8. a C₁₋₆ alkoxycarbonyl group,
F9. -CO-N (R^{56a}) (R^{56a'}),
wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents a substituent selected from Group f below:
[Group f]
f1. an amino group,
f2. a mono C₁₋₆ alkylamino group,
f3. a di C₁₋₆ alkylamino group,
f4. a carboxy group,
f5. a C₁₋₆ alkoxycarbonyl group,
f6. a hydroxyl group,
and,
f7. a saturated 5- or 6-membered monocyclic heterocyclic group having at least one nitrogen atom,
F10. -N (R^{56b}) (R^{56b'}),
wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
F11. -N=CR⁵⁷(-N(R⁵⁸) (R^{58'})),
wherein R⁵⁷ is a hydrogen atom or a C₁₋₆ alkyl group, R⁵⁸ and R⁵⁸' are identical with or different from each other and represent a hydrogen atom or a C₁₋₆ alkyl group,
F12. a 5- or 6-membered monocyclic heteroaromatic group, and,
F13. a methylenedioxy group or an ethylenedioxy group, or,
(8) a C₁₋₆ alkyl group substituted with a 5- or 6-membered monocyclic heteroaromatic group or a C₁₋₆ alkyl group substituted with a 5- or 6-membered condensed heteroaromatic group with a benzene ring;
wherein the heteroaromatic group may be substituted with one or more substituents selected from Group G below:
[Group G]
G1. a C₁₋₆ alkyl group that may be substituted with one or more substituents selected from Group g below:
[Group g]
g1. a halogen atom,
g2. an amino group,
g3. a mono C₁₋₆ alkylamino group,
g4. a di C₁₋₆ alkylamino group,
g5. a C₁₋₆ alkoxycarbonylamino group,
and,
g6. a hydroxyimino group,
G2. a halogen atom,
G3. a C₁₋₆ alkoxy group that may be substituted with a halogen atom,
G4. an aryloxy group,
G5. a cyano group,
G6. -N(R^{59a}) (R^{59a'}),
wherein R^{59a} and R^{59a'} are identical with or different from each other and represent a hydrogen atom or a C₁₋₆ alkyl group, or R^{59a} and R^{59a'} may, together with the adjacent nitrogen atom, forma saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
G7. -CO-N (R^{59b}) (R^{59b'})
wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
G8. an aryl group,
and,
G9. a 5- or 6-membered monocyclic heteroaromatic group that may be substituted with an oxo or thioxo group,
R⁴ and R⁵ may, together with the adjacent nitrogen atom, form a saturated 5- or 6-memberedmonocyclic heterocyclic ring, wherein a part of the saturated heterocyclic ring may have a double bond, and the saturated heterocyclic ring may be condensed with a benzene ring to form a condensed ring, or the saturated heterocyclic ring which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof.

38. The pyrazole compound according to claim 36 represented by the following general formula (II'): 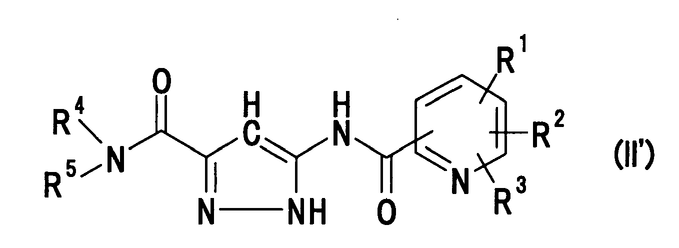 wherein R¹, R², R³, R⁴ and R⁵ are as defined in claim 36, or a pharmacologically acceptable salt thereof.

39. The pyrazole compound according to claim 36 represented by the following general formula (II): 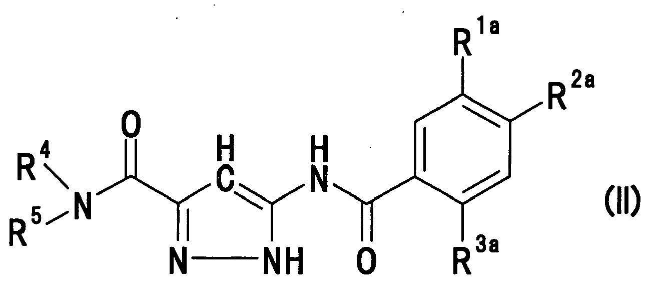 wherein R⁴ and R⁵ are as defined in claim 36, R^{1a} represents a halogen atom or a hydrogen atom, R^{2a} a halogen atom, and R^{3a} a halogen atom or a C₁₋₆ alkyl group, or a pharmacologically acceptable salt thereof.

40. The pyrazole compound according to claim 39, wherein R^{1a} is a hydrogen or fluorine atom, R^{2a} is a fluorine or chlorine atom, and R^{3a} is a chlorine atom or a C₁₋₆ alkyl group, or a pharmacologically acceptable salt thereof.

41. The pyrazole compound according to claim 40, wherein the pyrazole compound is a pyrazole compound represented by the following general formula (III): 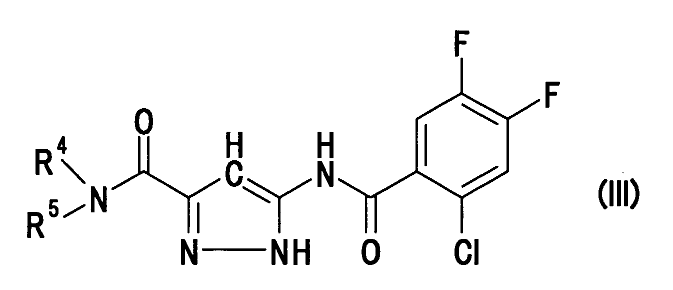 wherein R⁴ and R⁵ are as defined in claim 37, or a pharmacologically acceptable salt thereof.

42. The pyrazole compound according to any of claims 37 to 41, wherein R⁴ is a group selected from the following group:
(1) a hydrogen atom;
(2) a C₁₋₆ alkyl group that may be substituted with one or more substituents selected from Group A' below:
[Group A']
A'1. a hydroxyl group,
A'2. a C₁₋₄ alkoxy group,
A'3. -N (R⁴¹) (R^{41'}),
wherein R⁴¹ and _{R}⁴¹' are identical with or different from each other and represent a hydrogen atom or a C₁₋₄ alkyl group, or R⁴¹ and R^{41'} may, together with the adjacent nitrogen atom, forma saturated 5- or 6-memberedmonocyclicheterocyclic ring,
A'4. a phenyl group,
A'5. a 5- or 6-membered monocyclic heteroaromatic group,
A'6. -CO-N (R⁴¹¹) (R^{411'}),
wherein R⁴¹¹ and R^{411'} are identical with or different from each other and represent a hydrogen atom or a C₁₋₄ alkyl group, or R⁴¹¹ and R^{411'} may, together with the adjacent nitrogen atom, forma saturated5- or 6-memberedmonocyclic heterocyclic ring,
A'7. a carboxy group,
and,
A'8. a C₁₋₄ alkoxycarbonyl group,
(3) a C₂₋₆ alkenyl group;
(4) a C₂₋₆ alkynyl group;
(5) a C₃₋₈ cycloalkyl group;
and,
(6) a C₃₋₈ cycloalkyl C₁₋₄ alkyl group;
or a pharmacologically acceptable salt thereof.

43. The pyrazole compound according to any of claims 37 to 42, wherein R⁵ is a C₁₋₆ alkyl group, or a pharmacologically acceptable salt thereof.

44. The pyrazole compound according to any of claims 37 to 42, wherein R⁵ is a C₃₋₈ cycloalkyl group that may be substituted with one or more substituents selected from the group consisting of a hydroxyl group, a carboxy group or a C₁₋₆ alkoxycarbonyl group, or a pharmacologically acceptable salt thereof .

45. The pyrazole compound according to any of claims 37 to 42, wherein R⁵ is a C₃₋₈ cycloalkyl C₁₋₆ alkyl group, or a pharmacologically acceptable salt thereof.

46. The pyrazole compound according to any of claims 37 to 42, wherein R⁵ is a saturated 5- or 6-membered monocyclic heterocyclic group that may be substituted with one or more substituents selected from the group consisting of an acyl group and -CO-(Alk)n-COOR⁵², wherein R⁵² is a hydrogen atom or a C₁₋₆ alkyl group, Alk is a C₁₋₄ alkylene group, and n is 0 or an integer from 1 to 3, or a pharmacologically acceptable salt thereof.

47. The pyrazole compound according to any of claims 37 to 42, wherein R⁵ is a phenyl group that may be substituted with one or more substituents selected from the following Group D':
[Group D']
D'1. a C₁₋₄ alkyl group that may be substituted with a carboxy group,
D'2. -CO-N(R⁵³) (R^{53'}),
wherein R⁵³ and R^{53'} are identical with or different from each other and represent a hydrogen atom, a C₁₋₄ alkyl group or a C₁₋₄ alkylsulfonyl group,
D'3. -COOR⁵⁴,
wherein R⁵⁴ is a hydrogen atom, a C₁₋₄ alkyl group, a C₁₋₄ alkylcarbonyloxy C₁₋₄ alkyl group or a C₃₋₈ cycloalkyloxycarbonyloxy C₁₋₄ alkyl group,
and,
D'4. a 5- or 6-membered monocyclic heteroaromatic group that may be substituted with an oxo or thioxo group, or a pharmacologically acceptable salt thereof.

48. The pyrazole compound according to any of claims 37 to 42, wherein R⁵ is a C₇₋₁₄ aralkyl group and the aryl moiety of the C₇₋₁₄ aralkyl group may be substituted with one or more substituents selected from the following Group F':
[Group F']
F'1. a C₁₋₆ alkyl group,
F'2. a halogen atom,
F'3. a cyano group,
F'4. a hydroxyl group,
F'5. a C₁₋₆ alkoxy group that may be substituted with one or more substituents selected from the group consisting of a carboxy group and a C₁₋₆ alkoxycarbonyl group,
F'6. a halo C₁₋₆ alkyl group,
F'7. a carboxy group,
F'8. a C₁₋₆ alkoxycarbonyl group,
F'9. -CO-N (R^{56a}) (R^{56a'})
wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f' below:
[Group f']
f'1. an amino group,
f'2. a mono C₁₋₆ alkylamino group,
f'3. a di C₁₋₆ alkylamino group,
f'4. a carboxy group,
f'5. a C₁₋₆ alkoxycarbonyl group,
f'6. a hydroxyl group,
and,
f'7. a saturated 5- or 6-membered monocyclic heterocyclic group having at least one nitrogen atom,
F'10. -N (R^{56b}) (R^{56b'}),
wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an aryl.sulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
F'11. -N=CR⁵⁷(-N (R⁵⁸) (R^{58'})),
wherein R⁵⁷ is a hydrogen atom or a C₁₋₄ alkyl group, R⁵⁸ and R^{58'} are identical with or different from each other and represent a hydrogen atom or a C₁₋₄ alkyl group,
F'12. a 5- or 6-memberedmonocyclic heteroaromatic group, and,
F'13. a methylenedioxy group or an ethylenedioxy group, or a pharmacologically acceptable salt thereof.

49. The pyrazole compound according to any of claims 37 to 42, wherein R⁵ is a C₁₋₆ alkyl group substituted with a 5- or 6-membered monocyclic heteroaromatic group and the heteroaromatic group may be substituted with one or more substituents selected from the following Group G':
[Group G']
G'1. a C₁₋₆ alkyl group that may be substituted with one or more substituents selected from Group g' below:
[Group g']
g'1. a halogen atom,
g'2. an amino group,
g'3. a mono C₁₋₆ alkylamino group,
g'4. a di C₁₋₆ alkylamino group,
g'5. a C₁₋₆ alkoxycarbonylamino group,
and,
g'6. a hydroxyimino group,
G'2. a halogen atom,
G'3. a C₁₋₆ alkoxy group that may be substituted with a halogen atom,
G'4. an aryloxy group,
G'5. a cyano group,
G'6. -N(R^{59a}) (R^{59a'}),
wherein R^{59a} and R^{59a'} are identical with or different from each other and represent a hydrogen atom or a C₁₋₆ alkyl group, or _{R}^{59a} and _{R}^{59a}' may, together with the adjacent nitrogen atom, form a saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
G'7. -CO-N(R^{59b}) (R^{59b'})
wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
G'8. an aryl group,
and,
G'9. a 5- or 6-membered monocyclic heteroaromatic group that may be substituted with an oxo or thioxo group, or a pharmacologically acceptable salt thereof.

50. The pyrazole compound according to any of claims 37 to 41, wherein R⁵ and R⁴, together with the adjacent nitrogen atom, formasaturated5-or6-memberedmonocyclicheterocyclic ring, and the saturated heterocyclic group may have a double bond in part and may be condensed with a benzene ring to form a condensed ring, and the saturated heterocyclic group which may be condensed with a benzene ring to form a condensed ring may be substituted with one or more substituents selected from the group consisting of a halogen atom, a C₁₋₆ alkyl group, a C₁₋₆ alkoxy group, a carboxy group and a C₁₋₆ alkoxycarbonyl group, or a pharmacologically acceptable salt thereof.

51. The pyrazole compound according to claim 37, wherein the pyrazole compound is a pyrazole compound represented by the following general formula (IV): 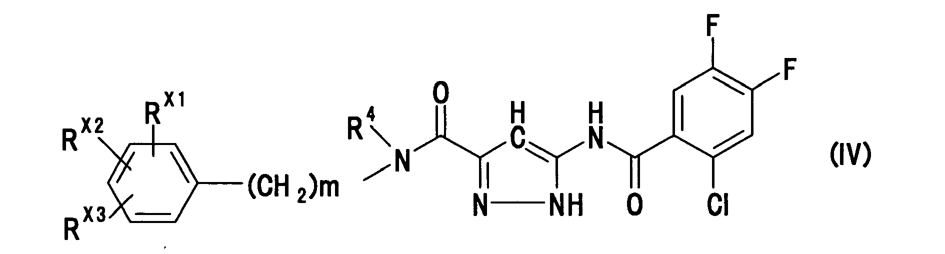 wherein R⁴ is as described in claim 37, R^{X1}, R^{x2} and _{R}X3 are identical with or different from each other and represent a hydrogen atom or a substituent selected from the following Group F'', and m is 0 or an integer from 1 to 2,
[Group F'']
F''1. a C₁₋₆ alkyl group,
F''2. a halogen atom,
F''3. a cyano group,
F''4. a hydroxyl group,
F''5. a C₁₋₄ alkoxy group that may be substituted with one or more substituents selected from the group consisting of a carboxy group and a C₁₋₄ alkoxycarbonyl group,
F''6. a halo C₁₋₆ alkyl group,
F''7. a carboxy group,
F''8. a C₁₋₆ alkoxycarbonyl group,
F''9. -CO-N (R^{56a}) (R^{56a'})
wherein R^{56a} and R^{56a'} are identical with or different from each other and represent a hydrogen atom, a saturated 5- or 6-membered monocyclic heterocyclic group having at least one nitrogen atom, or a C₁₋₆ alkyl group that may be substituted with one or more substituents selected from Group f'' below:
[Group f'']
f''1. an amino group,
f''2. a mono C₁₋₆ alkylamino group,
f''3. a di C₁₋₆ alkylamino group,
f''4. a carboxy group,
f''5. a C₁₋₆ alkoxycarbonyl group,
f''6. a hydroxyl group,
and,
f''7. a saturated 5- or 6-membered monocyclic heterocyclic group having at least one nitrogen atom,
F''10. -N (R^{56b}) (R^{56b'}),
wherein R^{56b} and R^{56b'} are identical with or different from each other and represent a hydrogen atom, a C₁₋₆ alkyl group that may be substituted with an imino group, an aralkyl group that may be substituted with one or more identical or different substituents selected from the group consisting of an imino group and a halogen atom, an arylsulfonyl group that may be substituted with a C₁₋₆ alkyl group, a C₁₋₆ alkylsulfonyl group, an acyl group, a carbamoyl group, a mono C₁₋₆ alkylcarbamoyl group or a di C₁₋₆ alkylcarbamoyl group,
F''11. -N=CR⁵⁷(-N(R⁵⁸) (R^{58'})),
wherein R⁵⁷ is a hydrogen atom or a C₁₋₆ alkyl group, R⁵⁸ and R⁵⁸' are identical with or different from each other and represent a hydrogen atom or a C₁₋₆ alkyl group,
F''12. a 5- or 6-membered monocyclic heteroaromatic group,
and,
F"13. a methylenedioxy group or an ethylenedioxy group, or a pharmacologically acceptable salt thereof.

52. The pyrazole compound according to claim 37, wherein the pyrazole compound is a pyrazole compound represented by the following general formula (V): 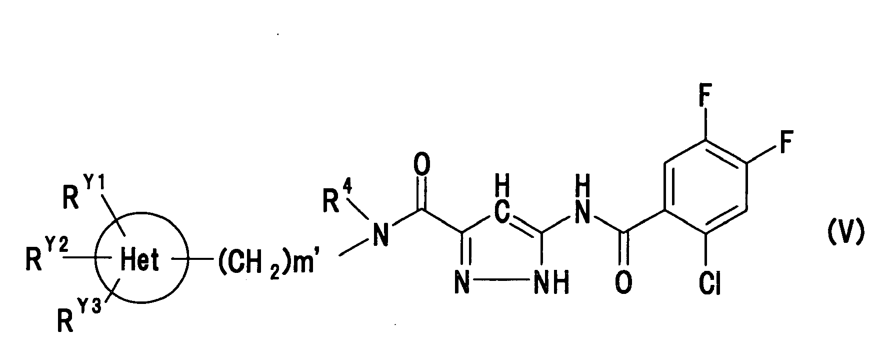 wherein R⁴ is as defined above, the ring Het represents a 5- or 6-membered monocyclic heteroaromatic group, R^{Y1}, R^{Y2} and R^{Y3} are identical with or different from each other and represent a hydrogen atom or a substituent selected from the following Group G', and m' is 0 or an integer from 1 to 2,
[Group G']
G'1. a C₁₋₆ alkyl group that may be substituted with one or more substituents selected from Group g' below:
[Group g']
g'1. a halogen atom,
g'2. an amino group,
g'3. a mono C₁₋₆ alkylamino group,
g'4. a di C₁₋₆ alkylamino group,
g'5. a C₁₋₆ alkoxycarbonylamino group,
and,
g'6. a hydroxyimino group,
G'2. a halogen atom,
G'3. a C₁₋₆ alkoxy group that may be substituted with a halogen atom,
G'4. an aryloxy group,
G'5. a cyano group,
G'6. -N (R^{59a}) (R^{59a'}),
wherein R^{59a} and R^{59a'} are identical with or different from each other and represent a hydrogen atom or a C₁₋₆ alkyl group, or R^{59a} and R^{59a'} may, together with the adjacent nitrogen atom, forma saturated 5- or 6-memberedmonocyclic heterocyclic ring, and the saturated heterocyclic ring may be substituted with one or more substituents selected from the group consisting of a hydroxyl group, an amino group, a mono C₁₋₆ alkylamino group and a di C₁₋₆ alkylamino group,
G'7. -CO-N(R^{59b}) (R^{59b'})
wherein R^{59b} and R^{59b'} are identical with or different from each other and represent a hydrogen atom, a saturated 5- or 6-membered monocyclic heterocyclic group or a C₁₋₆ alkyl group that may be substituted with the heterocyclic group,
G'8. an aryl group,
and,
G'9. a 5- or 6-membered monocyclic heteroaromatic group that may be substituted with an oxo or thioxo group, or a pharmacologically acceptable salt thereof.

53. The pyrazole compound according to claim 52, wherein the ring Het is a pyridine ring, a triazole ring or an oxazole ring, or a pharmacologically acceptable salt thereof.

54. The pyrazole compound according to claim 53, wherein the pyrazole compound is a pyrazole compound represented by the following general formula (VI), 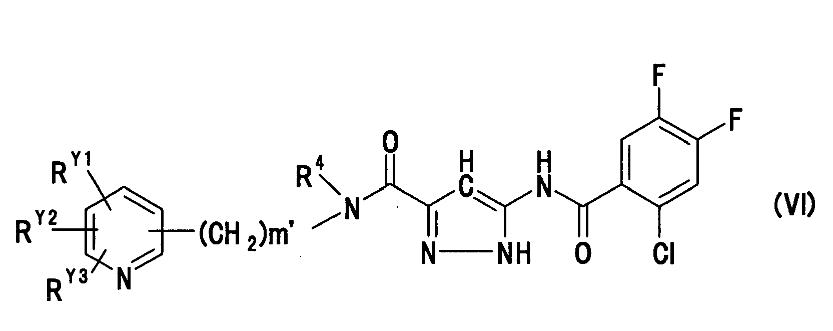 wherein R⁴, R^{Y1}, R^{Y2}, R^{Y3} and m' are as defined above, or a pharmacologically acceptable salt thereof.

55. The pyrazole compound or a pharmacologically acceptable salt thereof according to claim 37, wherein the pyrazole compound or a pharmacologically acceptable salt is selected from the following group:
5-(2-Chloro-4,5-difluoro-benzoylamino)-1H-pyrazole-3-carboxylic acid (pyridin-3-ylmethyl)-amide,
5-(2-Chloro-4,5-difluoro-benzoylamino)-1H-pyrazole-3-carboxylic acid (pyridin-3-ylmethyl)-amide dihydrochloride,
5-(2-Chloro-4,5-difluoro-benzoylamino)-1H-pyrazole-3-carboxylic acid (pyridin-3-ylmethyl) -amide hydrochloride,
5-(2-Chloro-4,5-difluoro-benzoylamino)-1H-pyrazole-3-carboxylic acid (4-methyl-oxazol-5-ylmethyl)-amide,
5-(2-Chloro-4,5-difluoro-benzoylamino)-1H-pyrazole-3-carboxylic acid (4-methyl-oxazol-5-ylmethyl)-amide,
5-(2-Chloro-4,5-difluoro-benzoylamino)-1H-pyrazole-3-carboxylic acid (4-methyl-oxazol-5-ylmethyl)amide-p-toluenesulfonate,
5-(2-Chloro-4,5-difluoro-benzoylamino)-1H-pyrazole-3-carboxylic acid (4-methyl-oxazol-5-ylmethyl)amide-L-(+)-tartrate,
5-(2-Chloro-4,5-difluoro-benzoylamino)-1H-pyrazole-3-carboxylic acid (2,4-dimethyl-oxazol-5-ylmethyl)-amide p-toluenesulfonate,
5-(2-Chloro-4,5-difluoro-benzoylamino)-1H-pyrazole-3-carboxylic acid (2,4-dimethyl-oxazol-5-ylmethyl)-amide,
5-(2-Chloro-4,5-difluoro-benzoylamino)-1H-pyrazole-3-carboxylic acid (2,4-dimethyl-pyridin-3-ylmethyl)amide,
5-(2-Chloro-4,5-difluoro-benzoylamino)-1H-pyrazole-3-carboxylic acid diethylamide,
5-(2-Chloro-4,5-difluoro-benzoylamino)-1H-pyrazole-3-carboxylic acid ethyl-(pyridin-4-ylmethyl)-amide,
5-(2-Chloro-4,5-difluoro-benzoylamino)-1H-pyrazole-3-carboxylic acid (2-methoxy-ethyl)-methyl-amide,
5-(2-Chloro-4,5-difluoro-benzoylamino)-1H-pyrazole-3-carboxylic acid ethyl-(2-methoxy-ethyl)-amide,
5-(2-Chloro-4,5-difluoro-benzoylamino)-1H-pyrazole-3-carboxylic acid 2-fluoro-benzylamide,
5-(2-Chloro-4,5-difluoro-benzoylamino)-1H-pyrazole-3-carboxylic acid 3-fluoro-benzylamide,
5-(2-Chloro-4,5-difluoro-benzoylamino)-1H-pyrazole-3-carboxylic acid 2-dimethylamino-benzylamide,
5-(2-Chloro-4,5-difluoro-benzoylamino)-1H-pyrazole-3-carboxylic acid (4-ethoxycarbonyl-cyclohexyl)-propyl-amide,
5- (2-Chloro-4,5-difluoro-benzoylamino) -1H-pyrazole-3-carboxylic acid (6-methoxy-pyridin-3-ylmethyl)-amide,
5-(2-Chloro-4,5-difluoro-benzoylamino)-1H-pyrazole-3-carboxylic acid 2,3-dimethoxy-benzylamide,
5-(2-Chloro-4,5-difluoro-benzoylamino)-1H-pyrazole-3-carboxylic acid 2,4-dimethoxy-benzylamide,
5-(2-Chloro-4,5-difluoro-benzoylamino)-1H-pyrazole-3-carboxylic acid 2,6-dimethoxy-benzylamide,
5-(2-Chloro-4,5-difluoro-benzoylamino)-1H-pyrazole-3-carboxylic acid 3,5-dimethoxy-benzylamide,
5-(2-Chloro-4,5-difluoro-benzoylamino)-1H-pyrazole-3-carboxylic acid 3,4-dimethoxy-benzylamide,
5-(2-Chloro-4,5-difluoro-benzoylamino)-1H-pyrazole-3-carboxylic acid 2,3-difluoro-benzylamide,
5-(2-Chloro-4,5-difluoro-benzoylamino)-1H-pyrazole-3-carboxylic acid 2,4-difluoro-benzylamide,
5-(2-Chloro-4,5-difluoro-benzoylamino)-1H-pyrazole-3-carboxylic acid 2,5-difluoro-benzylamide,
5-(2-Chloro-4,5-difluoro-benzoylamino)-1H-pyrazole-3-carboxylic acid 2,6-difluoro-benzylamide,
5-(2-Chloro-4,5-difluoro-benzoylamino)-1H-pyrazole-3-carboxylic acid 3,4-difluoro-benzylamide,
5-(2-Chloro-4,5-difluoro-benzoylamino)-1H-pyrazole-3-carboxylic acid 3,5-difluoro-benzylamide,
5-(2-Chloro-4,5-difluoro-benzoylamino)-1H-pyrazole-3-carboxylic acid (2-isopropoxy-pyridin-3-ylmethyl)-amide,
5-(2-Chloro-4,5-difluoro-benzoylamino)-1H-pyrazole-3-carboxylic acid (2-phenoxy-pyridin-3-ylmethyl)-amide,
5-(2-Chloro-4,5-difluoro-benzoylamino)-1H-pyrazole-3-carboxylic acid (3,5-difluoro-pyridin-2-ylmethyl)-amide,
5-(2-Chloro-4,5-difluoro-benzoylamino)-1H-pyrazole-3-carboxylic acid 2-trifluoromethyl-benzylamide,
5-(2-Chloro-4,5-difluoro-benzoylamino)-1H-pyrazole-3-carboxylic acid 3-trifluoromethyl-benzylamide
5-(2-Chloro-4,5-difluoro-benzoylamino)-1H-pyrazole-3-carboxylic acid 4-trifluoromethyl-benzylamide,
5-(2-Chloro-4,5-difluoro-benzoylamino)-1H-pyrazole-3-carboxylic acid 4-tert-butyl-benzylamide,
5-(2-Chloro-4,5-difluoro-benzoylamino)-1H-pyrazole-3-carboxylic acid (6-isopropoxy-pyridin-3-ylmethyl)-amide,
5-(2-Chloro-4,5-difluoro-benzoylamino)-1H-pyrazole-3-carboxylic acid [2-(2,2,2-trifluoro-ethoxy)-pyridin-3-ylmethyl]-amide,
5-(2-Chloro-4,5-difluoro-benzoylamino)-1H-pyrazole-3-carboxylic acid 4-(2-dimethylamino-ethylcarbamoyl)-benzylamide,
5-(2-Chloro-4,5-difluoro-benzoylamino)-1H-pyrazole-3-carboxylic acid 4-ethyl-benzylamide,
5- (2-Chloro-4,5-difluoro-benzoylamino) -1H-pyrazole-3-carboxylic acid 4-isopropyl-benzylamide,
5-(2-Chloro-4,5-difluoro-benzoylamino)-1H-pyrazole-3-carboxylic acid 2-chloro-6-fluoro-benzylamide,
5-(2-Chloro-4,5-difluoro-benzoylamino)-1H-pyrazole-3-carboxylic acid (6-ethoxy-pyridin-3-ylmethyl)-amide dihydrochloride,
5-(2-Chloro-4,5-difluoro-benzoylamino)-1H-pyrazole-3-carboxylic acid (thiazol-4-ylmethyl)-amide,
5-(2-Chloro-4,5-difluoro-benzoylamino)-1H-pyrazole-3-carboxylic acid [6-(2,2,2-trifluoro-ethoxy)-pyridin-3-ylmethyl]-amide dihydrochloride,
5- (2-Chloro-4,5-difluoro-benzoylamino) -1H-pyrazole-3-carboxylic acid 2,6-dimethyl-benzylamide,
5-(2-Chloro-4,5-difluoro-benzoylamino)-1H-pyrazole-3-carboxylic acid [2-(pyridin-3-yl)-thiazol-4-ylmethyl]-amide dihydrochloride,
5-(2-Chloro-4,5-difluoro-benzoylamino)-1H-pyrazole-3-carboxylic acid (1H-benzoimidazol-2-ylmethyl)-amide dihydrochloride, [4-({[5-(2-Chloro-4,5-difluoro-benzoylamino)-1H-pyrazole-3-carbonyl]-amino}-methyl)-benzoylamino]-acetic acid methyl ester, 3-[4-({[5-(2-Chloro-4,5-difluoro-benzoylamino)-1H-pyrazole-3-carbonyl]-amino}-methyl)-benzoylamino]-propioni c acid methyl ester
5-(2-Chloro-4,5-difluoro-benzoylamino)-1H-pyrazole-3-carboxylic acid (2-methyl-thiazol-4-ylmethyl)-amide dihydrochloride,
5-(2-Chloro-4,5-difluoro-benzoylamino)-1H-pyrazole-3-carboxylic acid (3,5-dimethyl-isoxazol-4-ylmethyl)-amide,
5-(2-Chloro-4,5-difluoro-benzoylamino)-1H-pyrazole-3-carboxylic acid 4-[2-bis-(2-acetoxyethyl)-amino-ethylcarbamoyl]-benzyl amide,
5-(2-Chloro-4,5-difluoro-benzoylamino)-1H-pyrazole-3-carboxylic acid 4-(2-hydroxy-ethylcarbamoyl)-benzylamide,
5-(2-Chloro-4,5-difluoro-benzoylamino)-1H-pyrazole-3-carboxylic acid 4-{2-[(2-acetoxyethyl)-(2-hyroxyethyl)-amino]-ethylcarbamoyl}-benzylamide,
5-(2-Chloro-4,5-difluoro-benzoylamino)-1H-pyrazole-3-carboxylic acid (1-methyl-1H-benzoimidazol-2-ylmethyl)-amide hydrochloride,
5-(2-Chloro-4,5-difluoro-benzoylamino)-1H-pyrazole-3-carboxylic acid (thiazol-2-ylmethyl)-amide dihydrochloride,
5-(2-Chloro-4,5-difluoro-benzoylamino)-1H-pyrazole-3-carboxylic acid (benzothiazol-2-ylmethyl)-amide dihydrochloride,
5-(2-Chloro-4,5-difluoro-benzoylamino)-1H-pyrazole-3-carboxylic acid (2,5-dimethyl-thiazol-4-ylmethyl)-amide dihydrochloride,
5-(2-Chloro-4,5-difluoro-benzoylamino)-1H-pyrazole-3-carboxylic acid [2-(morpholin-4-yl)-thiazol-4-ylmethyl]-amide dihydrochloride,
5-(2-Chloro-4,5-difluoro-benzoylamino)-1H-pyrazole-3-carboxylic acid (1,3,5-trimethyl-1H-pyrazol-4-ylmethyl)-amide,
5-(2-Chloro-4,5-difluoro-benzoylamino)-1H-pyrazole-3-carboxylic acid (2-chloro-6-methyl-pyridin-3-ylmethyl)-amide dihydrochloride,
5-(2-Chloro-4,5-difluoro-benzoylamino)-1H-pyrazole-3-carboxylic acid (2-dimethylamino-thiazol-4-ylmethyl)-amide dihydrochloride,
5-(2-Chloro-4,5-difluoro-benzoylamino)-1H-pyrazole-3-carboxylic acid (1-methyl-1H-pyrrol-2-ylmethyl)-amide,
5-(2-Chloro-4,5-difluoro-benzoylamino)-1H-pyrazole-3-carboxylic acid (3,4-dimethoxy-pyridin-2-ylmethyl)-amide dihydrochloride,
5-(2-Chloro-4,5-difluoro-benzoylamino)-1H-pyrazole-3-carboxylic acid (5-tert-butyl-thiazol-2-ylmethyl)-amide dihydrochloride,
5-(2-Chloro-4,5-difluoro-benzoylamino)-1H-pyrazole-3-carboxylic acid
(5-methyl-2-phenyl-2H-[1,2,3]triazol-4-ylmethyl)-amide
5- (2-Chloro-4,5-difluoro-benzoylamino) -1H-pyrazole-3-carboxylic acid [4-(tert-butoxycarbonyl-amino)-methyl-pyridin-2-ylmethyl]-amide,
5-(2-Chloro-4,5-difluoro-benzoylamino)-1H-pyrazole-3-carboxylic acid [4-methyl-2-(morpholin-4-yl)-thiazol-5-ylmethyl]-amide dihydrochloride,
5-(2-Chloro-4,5-difluoro-benzoylamino)-1H-pyrazole-3-carboxylic acid (pyridin-3-ylmethyl)-amide 3/2hydrochloride hemihydrate,
5- (2-Chloro-4,5-difluoro-benzoylamino) -1H-pyrazole-3-carboxylic acid 2-amino-benzylamide,
5-(2-Chloro-4,5-difluoro-benzoylamino)-1H-pyrazole-3-carboxylic acid 2-methoxy-benzylamide,
5-(2-Chloro-4,5-difluoro-benzoylamino)-1H-pyrazole-3-carboxylic acid 3-methoxy-benzylamide,
5-(2-Chloro-4,5-difluoro-benzoylamino)-1H-pyrazole-3-carboxylic acid 4-methoxy-benzylamide,
5-(2-Chloro-4,5-difluoro-benzoylamino)-1H-pyrazole-3-carboxylic acid 4-methyl-benzylamide,
5-(2-Chloro-4,5-difluoro-benzoylamino)-1H-pyrazole-3-carboxylic acid 4-fluoro-benzylamide,
5-(2-Chloro-4,5-difluoro-benzoylamino)-1H-pyrazole-3-carboxylic acid 4-chloro-benzylamide,
5-(2-Chloro-4,5-difluoro-benzoylamino)-1H-pyrazole-3-carboxylic acid 4-ethoxycarbonyl-benzylamide,
5-(2-Chloro-4,5-difluoro-benzoylamino)-1H-pyrazole-3-carboxylic acid 4-carboxy-benzylamide,
5-(2-Chloro-4,5-difluoro-benzoylamino)-1H-pyrazole-3-carboxylic acid dibenzylamide,
5-(2-Chloro-4,5-difluoro-benzoylamino)-1H-pyrazole-3-carboxylic acid (cyano-phenyl-methyl)-amide,
5-(2-Chloro-4,5-difluoro-benzoylamino)-1H-pyrazole-3-carboxylic acid cyclohexylmethyl-amide,
5-(2-Chloro-4,5-difluoro-benzoylamino)-1H-pyrazole-3-carboxylic acid (2-hydroxy-ethyl)-phenyl-amide,
5-(2-Chloro-4,5-difluoro-benzoylamino)-1H-pyrazole-3-carboxylic acid cyclohexyl-methyl-amide,
5-(2-Chloro-4,5-difluoro-benzoylamino)-1H-pyrazole-3-carboxylic acid cyclohexylamide,
5-(2-Chloro-4,5-difluoro-benzoylamino)-1H-pyrazole-3-carboxylic acid cyclohexyl-ethyl-amide,
5-(2-Chloro-4,5-difluoro-benzoylamino)-1H-pyrazole-3-carboxylic acid allyl-cyclohexyl-amide,
5-(2-Chloro-4,5-difluoro-benzoylamino)-1H-pyrazole-3-carboxylic acid cyclohexyl-(pyridin-2-ylmethyl)-amide,
5-(2-Chloro-4,5-difluoro-benzoylamino)-1H-pyrazole-3-carboxylic acid methyl-(1-methyl-piperidin-4-yl)-amide,
5-(2-Chloro-4,5-difluoro-benzoylamino)-1H-pyrazole-3-carboxylic acid [(3,4-methylenedioxyphenyl)-methyl]-amide hydrochloride,
5-(2-Chloro-4,5-difluoro-benzoylamino)-1H-pyrazole-3-carboxylic acid benzyl-butyl-amide,
5-(2-Chloro-4,5-difluoro-benzoylamino)-1H-pyrazole-3-carboxylic acid benzyl-(4-hydroxy-butyl)-amide hydrochloride,
5-(2-Chloro-4,5-difluoro-benzoylamino)-1H-pyrazole-3-carboxylic acid butyl-ethyl-amide,
5-(2-Chloro-4,5-difluoro-benzoylamino)-1H-pyrazole-3-carboxylic acid cyclohexyl-propyl-amide,
5-(2-Chloro-4,5-difluoro-benzoylamino)-1H-pyrazole-3-carboxylic acid butyl-cyclohexyl-amide,
5-(2-Chloro-4,5-difluoro-benzoylamino)-1H-pyrazole-3-carboxylic acid cyclohexyl-(2-methoxy-ethyl)-amide,
5-(2-Chloro-4,5-difluoro-benzoylamino)-1H-pyrazole-3-carboxylic acid cyclohexyl-(pyridin-3-ylmethyl)-amide,
5-(2-Chloro-4,5-difluoro-benzoylamino)-1H-pyrazole-3-carboxylic acid [2-(morpholin-4-yl)-phenyl]-amide,
5-(2-Chloro-4,5-difluoro-benzoylamino)-1H-pyrazole-3-carboxylic acid dibutylamide,
5-(2-Chloro-4,5-difluoro-benzoylamino)-1H-pyrazole-3-carboxylic acid bis-(2-methoxy-ethyl)-amide,
5-(2-Chloro-4,5-difluoro-benzoylamino)-1H-pyrazole-3-carboxylic acid (2-methoxy-ethyl)-propyl-amide,
5-(2-Chloro-4,5-difluoro-benzoylamino)-1H-pyrazole-3-carboxylic acid butyl-(tetrahydro-pyran-4-yl)-amide,
5-(2-Chloro-4,5-difluoro-benzoylamino)-1H-pyrazole-3-carboxylic acid (2-methoxy-ethyl)-(tetrahydro-pyran-4-yl)-amide,
5-(2-Chloro-4,5-difluoro-benzoylamino)-1H-pyrazole-3-carboxylic acid cyclopentyl-(2-methoxy-ethyl)-amide,
5-(2-Chloro-4,5-difluoro-benzoylamino)-1H-pyrazole-3-carboxylic acid cyclohexyl-(3-methoxy-propyl)-amide,
5-(2-Chloro-4,5-difluoro-benzoylamino)-1H-pyrazole-3-carboxylic acid cyclohexyl-(2-ethoxy-ethyl)-amide,
5-(2-Chloro-4,5-difluoro-benzoylamino)-1H-pyrazole-3-carboxylic acid cyclohexyl-(2-isopropoxy-ethyl)-amide,
5-(2-Chloro-4,5-difluoro-benzoylamino)-1H-pyrazole-3-carboxylic acid cyclohexyl-(2-propoxy-ethyl)-amide,
5-(2-Chloro-4,5-difluoro-benzoylamino)-1H-pyrazole-3-carboxylic acid (1-ethyl-propyl)-(2-methoxy-ethyl)-amide,
5-(2-Chloro-4,5-difluoro-benzoylamino)-1H-pyrazole-3-carboxylic acid butyl-(1-tert-butoxycarbonyl-piperidin-4-yl)-amide,
5-(2-Chloro-4,5-difluoro-benzoylamino)-1H-pyrazole-3-carboxylic acid butyl-(tetrahydro-thiopyran-4-yl)-amide,
5-(2-Chloro-4,5-difluoro-benzoylamino)-1H-pyrazole-3-carboxylic acid (2-methoxy-ethyl)-amide,
5-(2-Chloro-4,5-difluoro-benzoylamino)-1H-pyrazole-3-carboxylic acid butyl-(pyridin-3-ylmethyl)-amide,
5-(2-Chloro-4,5-difluoro-benzoylamino)-1H-pyrazole-3-carboxylic acid benzyl-(2-methoxy-ethyl)-amide,
5-(2-Chloro-4,5-difluoro-benzoylamino)-1H-pyrazole-3-carboxylic acid butyl-(piperidin-4-yl)-amide,
5-(2-Chloro-4,5-difluoro-benzoylamino)-1H-pyrazole-3-carboxylic acid (1-acetyl-piperidin-4-yl)-butyl-amide,
5-(2-Chloro-4,5-difluoro-benzoylamino)-1H-pyrazole-3-carboxylic acid butyl-(1-methanesulfonyl-piperidin-4-yl)-amide,
5-(2-Chloro-4,5-difluoro-benzoylamino)-1H-pyrazole-3-carboxylic acid butyl-(1-ethoxalyl-piperidin-4-yl)-amide,
5-(2-Chloro-4,5-difluoro-benzoylamino)-1H-pyrazole-3-carboxylic acid butyl-(1-oxalo-piperidin-4-yl)-amide,
5-(2-Chloro-4,5-difluoro-benzoylamino)-1H-pyrazole-3-carboxylic acid butyl-(1,1-dioxo-hexahydro-1λ⁶ -thiopyran-4-yl)-amide,
5-(2-Chloro-4,5-difluoro-benzoylamino)-1H-pyrazole-3-carboxylic acid butyl-(l-oxo-hexahydro-1λ⁴ -thiopyran-4-yl)-amide,
5-(2-Chloro-4,5-difluoro-benzoylamino)-1H-pyrazole-3-carboxylic acid (6-methoxy-pyridin-3-ylmethyl)-propyl-amide,
5-(2-Chloro-4,5-difluoro-benzoylamino)-1H-pyrazole-3-carboxylic acid propyl-(pyridin-3-ylmethyl)-amide,
5-(2-Chloro-4,5-difluoro-benzoylamino)-1H-pyrazole-3-carboxylic acid 4-diethylcarbamoyl-benzylamide,
5-(2-Chloro-4,5-difluoro-benzoylamino)-1H-pyrazole-3-carboxylic acid 3-diethylcarbamoyl-benzylamide,
5-(2-Chloro-4,5-difluoro-benzoylamino)-1H-pyrazole-3-carboxylic acid 4-ethylcarbamoyl-benzylamide,
5-(2-Chloro-4,5-difluoro-benzoylamino)-1H-pyrazole-3-carboxylic acid 3-ethylcarbamoyl-benzylamide,
5-(2-Chloro-4,5-difluoro-benzoylamino)-1H-pyrazole-3-carboxylic acid 4-carbamoyl-benzylamide,
5-(2-Chloro-4,5-difluoro-benzoylamino)-1H-pyrazole-3-carboxylic acid (1-oxalo-piperidin-4-yl)-propyl-amide,
5-(2-Chloro-4,5-difluoro-benzoylamino)-1H-pyrazole-3-carboxylic acid (1-carboxyacetyl-piperidin-4-yl)-propyl-amide,
5-(2-Chloro-4,5-difluoro-benzoylamino)-1H-pyrazole-3-carboxylic acid [1-(2-carboxypropionyl)-piperidin-4-yl]-propyl-amide,
5-(2-Chloro-4,5-difluoro-benzoylamino)-1H-pyrazole-3-carboxylic acid cyclopropyl-(6-methoxy-pyridin-3-ylmethyl)-amide
5-(2-Chloro-4,5-difluoro-benzoylamino)-1H-pyrazole-3-carboxylic acid cyclobutyl-(6-methoxy-pyridin-3-ylmethyl)-amide,
5-(2-Chloro-4,5-difluoro-benzoylamino)-1H-pyrazole-3-carboxylic acid cyclopropylmethyl-(6-methoxy-pyridin-3-ylmethyl)-amide,
5-(2-Chloro-4,5-difluoro-benzoylamino)-1H-pyrazole-3-carboxylic acid (6-dimethylamino-pyridin-3-ylmethyl)-amide,
5-(2-Chloro-4,5-difluoro-benzoylamino)-1H-pyrazole-3-carboxylic acid (6-dimethylamino-pyridin-3-ylmethyl)-propyl-amide,
5-(2-Chloro-4,5-difluoro-benzoylamino)-1H-pyrazole-3-carboxylic acid benzyl-(2-carboxy-ethyl)-amide,
5-(2-Chloro-4,5-difluoro-benzoylamino)-1H-pyrazole-3-carboxylic acid benzyl-(2-ethoxycarbonyl-ethyl)-amide,
5-(2-Chloro-4,5-difluoro-benzoylamino)-1H-pyrazole-3-carboxylic acid (6-chloro-pyridin-3-ylmethyl)-propyl-amide,
5-(2-Chloro-4,5-difluoro-benzoylamino)-1H-pyrazole-3-carboxylic acid 2,6-dichloro-benzylamide,
5-(2-Chloro-4,5-difluoro-benzoylamino)-1H-pyrazole-3-carboxylic acid (3,5-dichloro-pyridin-4-ylmethyl)-amide,
5-(2-Chloro-4,5-difluoro-benzoylamino)-1H-pyrazole-3-carboxylic acid (3,5-dichloro-pyridin-4-ylmethyl)-propyl-amide,
5-(2-Chloro-4,5-difluoro-benzoylamino)-1H-pyrazole-3-carboxylic acid [6-(1H-pyrazol-1-yl)-pyridin-3-ylmethyl]-amide dihydrochloride,
5-(2-Chloro-4,5-difluoro-benzoylamino)-1H-pyrazole-3-carboxylic acid [6-(4-hydroxy-piperidin-1-yl)-pyridin-3-ylmethyl]-amide dihydrochloride,
5-(2-Chloro-4,5-difluoro-benzoylamino)-1H-pyrazole-3-carboxylic acid (2-pyridin-2-yl-ethyl)-amide,
5-(2-Chloro-4,5-difluoro-benzoylamino)-1H-pyrazole-3-carboxylic acid (2-pyridin-3-yl-ethyl)-amide,
5-(2-Chloro-4,5-difluoro-benzoylamino)-1H-pyrazole-3-carboxylic acid (2-pyridin-4-yl-ethyl)-amide,
5-(2-Chloro-4,5-difluoro-benzoylamino)-1H-pyrazole-3-carboxylic acid (4- [1, 2, 3] thiadiazol-4-yl-benzyl) -amide,
5-(2-Chloro-4,5-difluoro-benzoylamino)-1H-pyrazole-3-carboxylic acid (5-methyl-pyrazin-2-ylmethyl)-amide,
5-(2-Chloro-4,5-difluoro-benzoylamino)-1H-pyrazole-3-carboxylic acid pyrazin-2-ylamide,
5-(2-Chloro-4,5-difluoro-benzoylamino)-1H-pyrazole-3-carboxylic acid quinolin-5-ylamide,
5-(2-Chloro-4,5-difluoro-benzoylamino)-1H-pyrazole-3-carboxylic acid quinolin-8-ylamide,
5-(2-Chloro-4,5-difluoro-benzoylamino)-1H-pyrazole-3-carboxylic acid isoquinolin-5-ylamide,
5-(2-Chloro-4,5-difluoro-benzoylamino)-1H-pyrazole-3-carboxylic acid [3-ethoxy-5-(1-ethoxycarbonyl-1-methyl-ethyl)-pyridin-2-yl] -amide,
5-(2-Chloro-4,5-difluoro-benzoylamino)-1H-pyrazole-3-carboxylic acid [3-ethoxy-5-(1-ethoxycarbonyl-1-hydroxy-ethyl)-pyridin-2-yl] -amide,
5-(2-Chloro-4,5-difluoro-benzoylamino)-1H-pyrazole-3-carboxylic acid
[5-(1-carboxy-1-methyl-ethyl)-3-ethoxy-pyridin-2-yl]-amide,
5-(2-Chloro-4,5-difluoro-benzoylamino)-1H-pyrazole-3-carboxylic acid
[5-(1-carboxy-1-hydroxy-ethyl)-3-ethoxy-pyridin-2-yl]-amide,
5-(2-Chloro-4,5-difluoro-benzoylamino)-1H-pyrazole-3-carboxylic acid (pyrazin-2-ylmethyl)-amide,
5-(2-Chloro-4,5-difluoro-benzoylamino)-1H-pyrazole-3-carboxylic acid [6-(thiomorpholin-4-yl)-pyridin-3-ylmethyl]-amide,
5-(2-Chloro-4,5-difluoro-benzoylamino)-1H-pyrazole-3-carboxylic acid [6-(1,1-dioxo-1λ⁶-thiomorpholin-4-yl)-pyridin-3-ylmethyl]-amide,
5-(2-Chloro-4,5-difluoro-benzoylamino)-1H-pyrazole-3-carboxylic acid (4,5-dibromo-thiophen-2-ylmethyl)-amide,
5-(2-Chloro-4,5-difluoro-benzoylamino)-1H-pyrazole-3-carboxylic acid (5-chloro-thiophen-2-ylmethyl)-amide,
5-(2-Chloro-4,5-difluoro-benzoylamino)-1H-pyrazole-3-carboxylic acid (4-e thyl-2-methyl-oxazol-5-ylmethyl)-amide,
5-(2-Chloro-4,5-difluoro-benzoylamino)-1H-pyrazole-3-carboxylic acid (2-e thyl-4-methyl-oxazol-5-ylmethyl)-amide dihydrochloride,
5-(2-Chloro-4,5-difluoro-benzoylamino)-1H-pyrazole-3-carboxylic acid ([2,2']bithiophenyl-5-ylmethyl)-amide,
5-(2-Chloro-4,5-difluoro-benzoylamino)-1H-pyrazole-3-carboxylic acid (3-methoxy-thiophen-2-ylmethyl)-amide,
5-(2-Chloro-4,5-difluoro-benzoylamino)-1H-pyrazole-3-carboxylic acid (4,5-Dichloro-thiophen-2-ylmethyl)-amide,
5-(2-Chloro-4,5-difluoro-benzoylamino)-1H-pyrazole-3-carboxylic acid (2,5-dimethyl-oxazol-4-ylmethyl)-amide,
5-(2-Chloro-4,5-difluoro-benzoylamino)-1H-pyrazole-3-carboxylic acid (2-hexyl-4-methyl-oxazol-5-ylmethyl)-amide,
5-(2-Chloro-4,5-difluoro-benzoylamino)-1H-pyrazole-3-carboxylic acid (4-methoxy-thiophen-3-ylmethyl)-amide,
5-(2-Chloro-4,5-difluoro-benzoylamino)-1H-pyrazole-3-carboxylic acid (4-methyl-2-phenyl-oxazol-5-ylmethyl)-amide,
5-(2-Chloro-4,5-difluoro-benzoylamino)-1H-pyrazole-3-carboxylic acid (2-methyl-4-phenyl-oxazol-5-ylmethyl)-amide,
5-(2-Chloro-4,5-difluoro-benzoylamino)-1H-pyrazole-3-carboxylic acid (4-hexyl-2-methyl-oxazol-5-ylmethyl)-amide dihydrochloride,
5-(2-chloro-4,5-difluoro-benzoylamino)-1H-pyrazole-3-carboxylic acid (6-chloro-pyridin-3-ylmethyl)-amide,
5-(2-chloro-4,5-difluoro-benzoylamino)-1H-pyrazole-3-carboxylic acid (6-chloro-pyridin-3-ylmethyl)-amide dihydrochloride,
5-(2-chloro-4,5-difluoro-benzoylamino)-1H-pyrazole-3-carboxylic acid (4H-[1,2,4]triazol-3-ylmethyl)-amide,
5- (2-chloro-4,5-difluoro-benzoylamino) -1H-pyrazole-3-carboxylic acid (4H-[1,2,4]triazol-3-ylmethyl)-amide dihydrochloride,
5-(2-chloro-4,5-difluoro-benzoylamino)-1H-pyrazole-3-carboxylic acid(5-fluoro-4H-quinazoline-3-yl)-amide,
5-(2-chloro-4,5-difluoro-benzoylamino)-1H-pyrazole-3-carboxylic acid (5-fluoro-4H-quinazoline-3-yl)-amide dihydrochloride,
5-(2-chloro-4,5-difluoro-benzoylamino)-1H-pyrazole-3-carboxylic acid (4,6-dimethyl-pyridin-3-ylmethyl)-amide,
5-(2-chloro-4,5-difluoro-benzoylamino)-1H-pyrazole-3-carboxylic acid (4,6-dimethyl-pyridin-3-ylmethyl)-amide dihydrochloride,
5-(2-chloro-4,5-difluoro-benzoylamino)-1H-pyrazole-3-carboxylic acid (2-methoxy-6-methyl-pyridin-3-ylmethyl)-amide,
5-(2-chloro-4,5-difluoro-benzoylamino)-1H-pyrazole-3-carboxylic acid (4-methoxy-6-methyl-pyridin-3-ylmethyl)-amide,
5-(2-chloro-4,5-difluoro-benzoylamino)-1H-pyrazole-3-carboxylic acid (4-methoxy-6-methyl-pyridin-3-ylmethyl)-amide dihydrochloride,
5-(2-chloro-4,5-difluoro-benzoylamino)-1H-pyrazole-3-carboxylic acid (2-methoxy-4,6-dimethyl-pyridin-3-ylmethyl)-amide,
5-(2-chloro-4,5-difluoro-benzoylamino)-1H-pyrazole-3-carboxylic acid (2-methoxy-4,6-dimethyl-pyridin-3-ylmethyl)-amide dihydrochloride,
5-(2-Chloro-4,5-difluoro-benzoylamino)-1H-pyrazole-3-carboxylic acid (3-methyl-pyridin-2-ylmethyl)-amide,
5-(2-Chloro-4,5-difluoro-benzoylamino)-1H-pyrazole-3-carboxylic acid (5-methyl-pyridin-3-ylmethyl)-amide,
5-(2-Chloro-4,5-difluoro-benzoylamino)-1H-pyrazole-3-carboxylic acid (6-methyl-pyridin-3-ylmethyl)-amide,
5-(2-Chloro-4,5-difluoro-benzoylamino)-1H-pyrazole-3-carboxylic acid (6-methyl-pyridin-2-ylmethyl)-amide,
5-(2,4-Dichloro-5-fluoro-benzoylamino)-1H-pyrazole-3-carboxylic acid (pyridin-3-ylmethyl)-amide dihydrochloride,
5-(2-Chloro-4,5-difluoro-benzoylamino)-1H-pyrazole-3-carboxylic acid (6-fluoro-pyridin-3-ylmethyl)-amide dihydrochloride,
5-(2-Chloro-4,5-difluoro-benzoylamino)-1H-pyrazole-3-carboxylic acid (3-carboxy-phenyl)-methyl-amide,
5-(2-Chloro-4,5-difluoro-benzoylamino)-1H-pyrazole-3-carboxylic acid 3-methanesulfonylamino-benzylamide,
5-(2-Chloro-4,5-difluoro-benzoylamino)-1H-pyrazole-3-carboxylic acid 4-methanesulfonylamino-benzylamide,
5-(2-Chloro-4,5-difluoro-benzoylamino)-1H-pyrazole-3-carboxylic acid 3-acetylamino-benzylamide,
5-(2-Chloro-4,5-difluoro-benzoylamino)-1H-pyrazole-3-carboxylic acid 4-acetylamino-benzylamide,
5-(2-Chloro-4,5-difluoro-benzoylamino)-1H-pyrazole-3-carboxylic acid (2-phenyl-thiazol-4-ylmethyl)-amide,
5-(2-Chloro-4,5-difluoro-benzoylamino)-1H-pyrazole-3-carboxylic acid ((R)-1-phenyl-ethyl)-amide,
5-(2-Chloro-4,5-difluoro-benzoylamino)-1H-pyrazole-3-carboxylic acid ((S)-1-phenyl-ethyl)-amide,
5-(2-Chloro-4,5-difluoro-benzoylamino)-1H-pyrazole-3-carboxylic acid (6-phenoxy-pyridin-3-ylmethyl)-amide dihydrochloride,
5-(2-Methyl-4,5-difluoro-benzoylamino)-1H-pyrazole-3-carboxylic acid (6-chloro-pyridin-3-ylmethyl)-amide dihydrochloride,
5-(2-Chloro-4,5-difluoro-benzoylamino)-1H-pyrazole-3-carboxylic acid (2-chloro-pyridin-3-ylmethyl)-amide dihydrochloride,
5-(2-Methyl-4,5-difluoro-benzoylamino)-1H-pyrazole-3-carboxylic acid (6-chloro-pyridin-3-ylmethyl)-amide sodium salt,
5-(2-Chloro-4,5-difluoro-benzoylamino)-1H-pyrazole-3-carboxylic acid [1-(pyridin-3-yl)-ethyl]-amide dihydrochloride,
5-(2-Chloro-4,5-difluoro-benzoylamino)-1H-pyrazole-3-carboxylic acid (2-fluoro-pyridin-3-ylmethyl)-amide dihydrochloride,
5-(2-Chloro-4,5-difluoro-benzoylamino)-1H-pyrazole-3-carboxylic acid (2-methyl-pyridin-3-ylmethyl)-amide dihydrochloride,
5-(2-Chloro-4,5-difluoro-benzoylamino)-1H-pyrazole-3-carboxylic acid (biphenyl-3-ylmethyl)-amide,
5- (2-Chloro-4,5-difluoro-benzoylamino) -1H-pyrazole-3-carboxylic acid ((1R,2S)-2-hydroxy-indan-1-yl)-amide,
5-(2-Chloro-4,5-difluoro-benzoylamino)-1H-pyrazole-3-carboxylic acid ((1S,2R)-2-hydroxy-indan-1-yl)-amide,
5- (2-Chloro-4,5-difluoro-benzoylamino) -1H-pyrazole-3-carboxylic acid (6-phenyl-pyridin-3-ylmethyl)-amide dihydrochloride,
5- (2-Chloro-4,5-difluoro-benzoylamino) -1H-pyrazole-3-carboxylic acid (6-methyl-2-pyridon-3-ylmethyl)-amide hydrochloride,
5-(2-chloro-4,5-difluoro-benzoylamino)-1H-pyrazole-3-carboxylic acid benzyl-methyl-amide,
5-(2-chloro-4,5-difluoro-benzoylamino)-1H-pyrazole-3-carboxylic acid benzylamide,
5-(2-Chloro-4,5-difluoro-benzoylamino)-1H-pyrazole-3-carboxylic acid phenylamide,
5-(2-Chloro-4,5-difluoro-benzoylamino)-1H-pyrazole-3-carboxylic acid methyl-phenyl-amide,
5-(2-Chloro-4,5-difluoro-benzoylamino)-1H-pyrazole-3-carboxylic acid (piperidin-1-yl)-amide,
5-(2-Chloro-4,5-difluoro-benzoylamino)-1H-pyrazole-3-carboxylic acid (4-ethoxycarbonyl-piperidin-1-yl)-amide,
5-(2-Chloro-4,5-difluoro-benzoylamino)-1H-pyrazole-3-carboxylic acid (4-methyl-piperazin-1-yl)-amide,
5-(2-Chloro-4,5-difluoro-benzoylamino)-1H-pyrazole-3-carboxylic acid (4-carboxy-piperidin-1-yl)-amide,
5-(2-Chloro-4,5-difluoro-benzoylamino)-1H-pyrazole-3-carboxylic acid phenethylamide,
5-(2-Chloro-4,5-difluoro-benzoylamino)-1H-pyrazole-3-carboxylic acid methyl-phenethyl-amide,
5-(2-Chloro-4,5-difluoro-benzoylamino)-1H-pyrazole-3-carboxylic acid ethyl-phenyl-amide,
5-(2-Chloro-4,5-difluoro-benzoylamino)-1H-pyrazole-3-carboxylic acid (1,2,3,4-tetrahydroisoquinolin-2-yl)-amide,
5-(2-Chloro-4,5-difluoro-benzoylamino)-1H-pyrazole-3-carboxylic acid ((S)-α-methoxycarbonyl-benzyl)-amide,
5-(2-Chloro-4,5-difluoro-benzoylamino)-1H-pyrazole-3-carboxylic acid ((R)-α-methoxycarbonyl-benzyl)-amide,
5-(2-Chloro-4,5-difluoro-benzoylamino)-1H-pyrazole-3-carboxylic acid (1,2,3,4-tetrahydroquinoline-1-yl)-amide,
5-(2-Chloro-4,5-difluoro-benzoylamino)-1H-pyrazole-3-carboxylic acid phenyl-propyl-amide,
5-(2-Chloro-4,5-difluoro-benzoylamino)-1H-pyrazole-3-carboxylic acid butyl-phenyl-amide,
5-(2-Chloro-4,5-difluoro-benzoylamino)-1H-pyrazole-3-carboxylic acid pentyl-phenyl-amide,
5-(2-Chloro-4,5-difluoro-benzoylamino)-1H-pyrazole-3-carboxylic acid benzhydryl-amide,
5-(2-Chloro-4,5-difluoro-benzoylamino)-1H-pyrazole-3-carboxylic acid (pyridin-2-ylmethyl)-amide hydrochloride,
5-(2-Chloro-4,5-difluoro-benzoylamino)-1H-pyrazole-3-carboxylic acid (pyridin-4-ylmethyl)-amide hydrochloride,
5-(2-Chloro-4,5-difluoro-benzoylamino)-1H-pyrazole-3-carboxylic acid ((R)-2-hydroxy-1-phenyl-ethyl)-amide,
5-(2-Chloro-4,5-difluoro-benzoylamino)-1H-pyrazole-3-carboxylic acid ((S)-2-hydroxy-1-phenyl-ethyl)-amide,
5-(2-Chloro-4,5-difluoro-benzoylamino)-1H-pyrazole-3-carboxylic acid 4-dimethylamino-benzylamide,
5-(2-Chloro-4,5-difluoro-benzoylamino)-1H-pyrazole-3-carboxylic acid (furan-2-ylmethyl)-amide,
5-(2-Chloro-4,5-difluoro-benzoylamino)-1H-pyrazole-3-carboxylic acid (thiophen-2-ylmethyl)-amide,
5-(2-Chloro-4,5-difluoro-benzoylamino)-1H-pyrazole-3-carboxylic acid (2-methoxy-phenyl)-amide,
5-(2-Chloro-4,5-difluoro-benzoylamino)-1H-pyrazole-3-carboxylic acid (2-methoxy-phenyl)-methyl-amide,
5-(2-Chloro-4,5-difluoro-benzoylamino)-1H-pyrazole-3-carboxylic acid 4-dimethylamino-benzylamide hydrochloride,
5-(2-Chloro-4,5-difluoro-benzoylamino)-1H-pyrazole-3-carboxylic acid 3-amino-benzylamide,
5-(2-Chloro-4,5-difluoro-benzoylamino)-1H-pyrazole-3-carboxylic acid 4-amino-benzylamide,
5-(2-Chloro-4,5-difluoro-benzoylamino)-1H-pyrazole-3-carboxylic acid 2-methyl-benzylamide,
5-(2-Chloro-4,5-difluoro-benzoylamino)-1H-pyrazole-3-carboxylic acid 3-methyl-benzylamide,
5-(2-Chloro-4,5-difluoro-benzoylamino)-1H-pyrazole-3-carboxylic acid 3-dimethylamino-benzylamide,
5-(2-Chloro-4,5-difluoro-benzoylamino)-1H-pyrazole-3-carboxylic acid (2-methoxy-pyridin-3-ylmethyl)-amide,
5-(2-Chloro-4,5-difluoro-benzoylamino)-1H-pyrazole-3-carboxylic acid 2-chloro-benzylamide,
5-(2-Chloro-4,5-difluoro-benzoylamino)-1H-pyrazole-3-carboxylic acid 3-chloro-benzylamide,
5-(2-Chloro-4,5-difluoro-benzoylamino)-1H-pyrazole-3-carboxylic acid 3-methoxycarbonyl-benzylamide,
5-(2-Chloro-4,5-difluoro-benzoylamino)-1H-pyrazole-3-carboxylic acid 3-carboxy-benzylamide,
5-(2-Chloro-4,5-difluoro-benzoylamino)-1H-pyrazole-3-carboxylic acid (6-trifluoromethyl-pyridin-3-ylmethyl)-amide,
5-(2-Chloro-4,5-difluoro-benzoylamino)-1H-pyrazole-3-carboxylic acid (2-ethoxy-pyridin-3-ylmethyl)-amide,
5-(2-Chloro-4,5-difluoro-benzoylamino)-1H-pyrazole-3-carboxylic acid (2-dimethylamino-ethyl)-amide,
5-(2-Chloro-4,5-difluoro-benzoylamino)-1H-pyrazole-3-carboxylic acid ethyl-(2-dimethylamino-ethyl)-amide dihydrochloride,
5-(2-Chloro-4,5-difluoro-benzoylamino)-1H-pyrazole-3-carboxylic acid cyclohexyl-(2-dimethylamino-ethyl)-amide,
5-(2-Chloro-4,5-difluoro-benzoylamino)-1H-pyrazole-3-carboxylic acid (4-methoxy-pyridin-2-ylmethyl)-amide,
5-(2-Chloro-4,5-difluoro-benzoylamino)-1H-pyrazole-3-carboxylic acid (2,6-dimethoxy-pyridin-3-ylmethyl)-amide,
5-(2-Chloro-4,5-difluoro-benzoylamino)-1H-pyrazole-3-carboxylic acid 2-methoxycarbonylmethoxy-benzylamide,
5-(2-Chloro-4,5-difluoro-benzoylamino)-1H-pyrazole-3-carboxylic acid 2-carboxymethoy-benzylamide,
5-(4,5-Difluoro-2-methyl-benzoylamino)-1H-pyrazole-3-carboxylic acid (6-methoxy-pyridin-3-ylmethyl)-amide,
5-(2,4-Dichloro-benzoylamino)-1H-pyrazole-3-carboxylic acid (6-methoxy-pyridin-3-ylmethyl)-amide,
5-(2-Chloro-4,5-difluoro-benzoylamino)-1H-pyrazole-3-carboxylic acid (3,5-dimethoxy-pyridin-4-ylmethyl)-amide,
5-(4,5-Difluoro-2-methyl-benzoylamino)-1H-pyrazole-3-carboxylic acid (2,4-dimethyl-oxazol-5-ylmethyl)-amide,
5-(2,4-Dichloro-5-fluoro-benzoylamino)-1H-pyrazole-3-carboxylic acid (2,4-dimethyl-oxazol-5-ylmethyl)-amide, Methyl
(5-(2-chloro-4,5-difluorobenzoylamino)-1H-pyrazole-3-carbocylic acid (4-methoxycarbonyl-phenyl)-methyl-amide,
5-(2-chloro-4,5-difluoro-benzoylamino)-1H-pyrazole-3-carboxylic acid (4-carboxyphenyl)-methyl-amide, Monosodium 4-{[5-(2-chloro-4,5-difluoro-benzoylamino)-1H-pyrazole -3-carbonyl]-methyl-amino}-benzoate,
5-(2-Chloro-4,5-difluoro-benzoylamino)-1H-pyrazole-3-carboxylic acid isopropyl-phenyl-amide,
5-(2-Chloro-4,5-difluoro-benzoylamino)-1H-pyrazole-3-carboxylic acid (ethoxycarbonyl-methyl)-phenyl-amide,
5-(2-Chloro-4,5-difluoro-benzoylamino)-1H-pyrazole-3-carboxylic acid (carboxy-methyl)-phenyl-amide,
5-(2-Chloro-4-fluoro-benzoylamino)-1H-pyrazole-3-carboxylic acid benzylamide,
5-(2,4-Dichloro-benzoylamino)-1H-pyrazole-3-carboxylic acid benzylamide,
5-(2-Chloro-5-fluoro-benzoylamino)-1H-pyrazole-3-carboxylic acid benzylamide,
5-(2-Chloro-4,5-difluoro-benzoylamino)-1H-pyrazole-3-carboxylic acid benzyl-phenyl-amide,
5-(2-Chloro-4,5-difluoro-benzoylamino)-1H-pyrazole-3-carboxylic acid (1-methyl-1-phenyl-ethyl)-amide,
5-(2-Chloro-4,5-difluoro-benzoylamino)-1H-pyrazole-3-carboxylic acid [2-oxo-2-(piperidin-1-yl)-ethyl]-phenyl-amide,
5-(5-Fluoro-2-methyl-benzoylamino)-1H-pyrazole-3-carboxylic acid benzylamide,
5-(5-Chloro-2-methyl-benzoylamino)-1H-pyrazole-3-carboxylic acid benzylamide,
5-(4-Methoxy-2-methyl-benzoylamino)-1H-pyrazole-3-carboxylic acid benzylamide,
5-(4-Chloro-2-methyl-benzoylamino)-1H-pyrazole-3-carboxylic acid benzylamide,
5-(5-Fluoro-2-methoxy-benzoylamino)-1H-pyrazole-3-carboxylic acid benzylamide,
5-(5-Chloro-2-methoxy-benzoylamino)-1H-pyrazole-3-carboxylic acid benzylamide,
5-(2-Chloro-4,5-difluoro-benzoylamino)-1H-pyrazole-3-carboxylic acid (2-ethoxy-ethyl)-phenyl-amide,
5-(2-Fluoro-5-methyl-benzoylamino)-1H-pyrazole-3-carboxylic acid benzylamide,
5-(5-Chloro-2-fluoro-benzoylamino)-1H-pyrazole-3-carboxylic acid benzylamide,
5-(3-Chloro-2,6-difluoro-benzoylamino)-1H-pyrazole-3-carboxylic acid benzylamide,
5-(2-Chloro-3-fluoro-benzoylamino)-1H-pyrazole-3-carboxylic acid benzylamide,
5-(2-Chloro-4,5-difluoro-benzoylamino)-1H-pyrazole-3-carboxylic acid ethylcarbamoylmethyl-phenyl-amide,
5-(2-Chloro-4,5-difluoro-benzoylamino)-1H-pyrazole-3-carboxylic acid isobutyl-phenyl-amide,
5-(2-Chloro-4,5-difluoro-benzoylamino)-1H-pyrazole-3-carboxylic acid (2-methoxycarbonyl-ethyl)-phenyl-amide,
5-(2-Chloro-4,5-difluoro-benzoylamino)-1H-pyrazole-3-carboxylic acid (3-ethoxycarbonyl-propyl)-phenyl-amide,
5-(2-Chloro-4,5-difluoro-benzoylamino)-1H-pyrazole-3-carboxylic acid (2-carboxy-ethyl)-phenyl-amide,
5-(2-Chloro-4,5-difluoro-benzoylamino)-1H-pyrazole-3-carboxylic acid (3-carboxy-propyl)-phenyl-amide,
5-(2-Hydroxy-4-methoxy-benzoylamino)-1H-pyrazole-3-carboxylic acid benzylamide,
5-(2-Hydroxy-5-methoxy-benzoylamino)-1H-pyrazole-3-carboxylic acid benzylamide,
5-(5-Fluoro-2-hydroxy-benzoylamino)-1H-pyrazole-3-carboxylic acid benzylamide,
5-(4-Fluoro-2-trifluoromethyl-benzoylamino)-1H-pyrazole-3-carboxylic acid benzylamide,
5-(5-Fluoro-2-trifluoromethyl-benzoylamino)-1H-pyrazole-3-carboxylic acid benzylamide,
5-(2-Chloro-4,5-difluoro-benzoylamino)-1H-pyrazole-3-carboxylic acid (2-methoxy-ethyl)-phenyl-amide,
5-(2,5-Dimethyl-benzoylamino)-1H-pyrazole-3-carboxylic acid benzylamide,
5-(2-Chloro-5-methyl-benzoylamino)-1H-pyrazole-3-carboxylic acid benzylamide,
5- (2-Chloro-pyridine-3-carbonylamino) -1H-pyrazole-3-carboxylic acid benzylamide,
5- (4-Chloro-pyridine-2-carbonylamino) -1H-pyrazole-3-carboxylic acid benzylamide,
5-(2,6-Dichloro-pyridine-3-carbonylamino)-1H-pyrazole-3-carboxylic acid benzylamide,
5-(2-Chloro-4,5-difluoro-benzoylamino)-1H-pyrazole-3-carboxylic acid butyl-(4-ethoxycarbonyl-phenyl)-amide,
5-(2-Chloro-4,5-difluoro-benzoylamino)-1H-pyrazole-3-carboxylic acid butyl-(4-carboxy-phenyl)-amide,
5-(2-Methyl-pyridine-3-carbonylamino)-1H-pyrazole-3-carboxylic acid benzylamide,
5-(2-Chloro-6-methyl-pyridine-3-carbonylamino)-1H-pyrazole-3-carboxylic acid benzylamide,
5-(3-Chloro-pyridine-4-carbonylamino)-1H-pyrazole-3-carboxylic acid benzylamide,
5-(2-Chloro-4,5-difluoro-benzoylamino)-1H-pyrazole-3-carboxylic acid butyl-(4-ethoxycarbonylmethyl-phenyl)-amide,
5-(2-Chloro-4,5-difluoro-benzoylamino)-1H-pyrazole-3-carboxylic acid butyl-(4-carboxymethyl-phenyl)-amide,
5-(2-Chloro-4,5-difluoro-benzoylamino)-1H-pyrazole-3-carboxylic acid (4-methoxycarbonyl-phenyl)-(2-methoxy-ethyl)-amide,
5-(2-Chloro-4,5-difluoro-benzoylamino)-1H-pyrazole-3-carboxylic acid (4-carboxy-phenyl)-(2-methoxy-ethyl)-amide,
5-(2-Chloro-4,5-difluoro-benzoylamino)-1H-pyrazole-3-carboxylic acid (6-methoxy-pyridin-3-ylmethyl)-amide dihydrochloride,
5-(2-Methyl-4,5-difluoro-benzoylamino)-1H-pyrazole-3-carboxylic acid (6-methoxy-pyridin-3-ylmethyl)-amide dihydrochloride,
5- (2-Chloro-4,5-difluoro-benzoylamino) -1H-pyrazole-3-carboxylic acid (thiazol-4-ylmethyl)-amide dihydrochloride,
5-(2-Chloro-4,5-difluoro-benzoylamino)-1H-pyrazole-3-carboxylic acid 4-[2-(morpholin-4-yl)-ethylcarbamoyl]-benzylamide dihydrochloride,
5-(2-Chloro-4,5-difluoro-benzoylamino)-1H-pyrazole-3-carboxylic acid 4-(morpholin-4-ylcarbamoyl)-benzylamide dihydrochloride, [4-({[5-(2-chloro-4,5-difluoro-benzoylamino)-1H-pyrazole-3-carbonyl]-amino}-methyl)-benzoylamino]-acetic acid sodium salt, 3-[4-({[5-(2-chloro-4,5-difluoro-benzoylamino)-1H-pyrazole-3-carbonyl]-amino}-methyl)-benzoylamino]-propioni c acid sodium salt,
5-(2-Chloro-4,5-difluoro-benzoylamino)-1H-pyrazole-3-carboxylic acid (3,5-dimethyl-isoxazol-4-ylmethyl)-amide hydrochloride,
5-(2-Chloro-4,5-difluoro-benzoylamino)-1H-pyrazole-3-carboxylic acid (1,3,5-trimethyl-1H-pyrazol-4-ylmethyl)-amide dihydrochloride,
5-(2-Chloro-4,5-difluoro-benzoylamino)-1H-pyrazole-3-carboxylic acid (3,5-dimethyl-isoxazol-4-ylmethyl)-amide methanesulfonate,
5-(2-Chloro-4,5-difluoro-benzoylamino)-1H-pyrazole-3-carboxylic acid 3-[2-(morpholin-4-yl)-ethylcarbamoyl]-benzylamide dihydrochloride,
5-(2-Chloro-4,5-difluoro-benzoylamino)-1H-pyrazole-3-carboxylic acid 3-(morpholin-4-ylcarbamoyl)-benzylamide dihydrochloride,
5-(2-Chloro-4,5-difluoro-benzoylamino)-1H-pyrazole-3-carboxylic acid {2-[2-(morpholin-4-yl)-ethylcarbamoyl]-pyridin-4-ylmethyl}-amide trihydrochloride,
5-(2-Chloro-4,5-difluoro-benzoylamino)-1H-pyrazole-3-carboxylic acid [2-(morpholin-4-ylcarbamoyl)-pyridin-4-ylmethyl]-amide trihydrochloride,
5-(2-Chloro-4,5-difluoro-benzoylamino)-1H-pyrazole-3-carboxylic acid (6-methoxy-pyridin-3-ylmethyl)-amide methanesulfonate,
5-(2-Chloro-4,5-difluoro-benzoylamino)-1H-pyrazole-3-carboxylic acid (6-methoxy-pyridin-3-ylmethyl)-amide hemisulfate
5-(2-Chloro-4,5-difluoro-benzoylamino)-1H-pyrazole-3-carboxylic acid (3,5-dimethoxy-pyridin-4-ylmethyl)-amide dihydrochloride,
5-(2-Chloro-4,5-difluoro-benzoylamino)-1H-pyrazole-3-carboxylic acid (4-aminomethyl-pyridin-2-ylmethyl)-amide trihydrochloride,
5-(2-Chloro-4,5-difluoro-benzoylamino)-1H-pyrazole-3-carboxylic acid (6-chloro-pyridin-3-ylmethyl)-amide methanesulfonate,
5-(2-Chloro-4,5-difluoro-benzoylamino)-1H-pyrazole-3-carboxylic acid (6-chloro-pyridin-3-ylmethyl)-amide dimethanesulfonate,
5-(2-Chloro-4,5-difluoro-benzoylamino)-1H-pyrazole-3-carboxylic acid (2,4-dimethyl-pyridin-3-ylmethyl)-amide hydrochloride,
5-(2-Chloro-4,5-difluoro-benzoylamino)-1H-pyrazole-3-carboxylic acid (pyridin-3-ylmethyl)-amide hydrochloride,
5-(2-Chloro-4,5-difluoro-benzoylamino)-1H-pyrazole-3-carboxylic acid (6-methoxy-pyridin-3-ylmethyl)-propyl-amide dihydrochloride,
5-(2-Chloro-4,5-difluoro-benzoylami.no)-1H-pyrazole-3-carboxylic acid [6-(piperidin-1-yl)-pyridin-3-ylmethyl]-amide dihydrochloride,
5-(2-Chloro-4,5-difluoro-benzoylamino)-1H-pyrazole-3-carboxylic acid [6-(morpholin-4-yl)-pyridin-3-ylmethyl]-amide dihydrochloride,
5-(2-Chloro-4,5-difluoro-benzoylamino)-1H-pyrazole-3-carboxylic acid (3,5-dichloro-pyridin-4-ylmethyl)-amide hydrochloride,
5-(2-Chloro-4,5-difluoro-benzoylamino)-1H-pyrazole-3-carboxylic acid (6-dimethylamino-pyridin-3-ylmethyl)-amide dihydrochloride,
5-(2-Chloro-4,5-difluoro-benzoylamino)-1H-pyrazole-3-carboxylic acid (6-dimethylamino-pyridin-3-ylmethyl)-propyl-amide dihydrochloride,
5-(2-Chloro-4,5-difluoro-benzoylamino)-1H-pyrazole-3-carboxylic acid (6-diethylamino-pyridin-3-ylmethyl)-amide dihydrochloride,
5-(2-Chloro-4,5-difluoro-benzoylamino)-1H-pyrazole-3-carboxylic acid (6-chloro-2-dimethylamino-pyridin-3-ylmethyl)-amide dihydrochloride,
5-(2-Chloro-4,5-difluoro-benzoylamino)-1H-pyrazole-3-carboxylic acid (4-[1,2,3]thiadiazol-4-yl-benzyl)-amide hydrochloride,
5-(2-Chloro-4,5-difluoro-benzoylamino)-1H-pyrazole-3-carboxylic acid (4-methyl-thiazol-5-ylmethyl)-amide hydrochloride,
5-(2-Chloro-4,5-difluoro-benzoylamino)-1H-pyrazole-3-carboxylic acid (2,4-dimethyl-thiazol-5-ylmethyl)-amide hydrochloride,
5-(2-Chloro-4,5-difluoro-benzoylamino)-1H-pyrazole-3-carboxylic acid [6-(4-hydroxy-piperidin-1-yl)-pyridin-3-ylmethyl]-amid e dihydrochloride,
5-(2-Chloro-4,5-difluoro-benzoylamino)-1H-pyrazole-3-carboxylic acid 5-methyl-pyrazin-2-ylamide dihydrochloride,
5-(2-Chloro-4,5-difluoro-benzoylamino)-1H-pyrazole-3-carboxylic acid quinolin-5-ylamide dihydrochloride,
5-(2-Chloro-4,5-difluoro-benzoylamino)-1H-pyrazole-3-carboxylic acid quinolin-8-ylamide dihydrochloride,
5-(2-Chloro-4,5-difluoro-benzoylamino)-1H-pyrazole-3-carboxylic acid isoquinolin-5-ylamide dihydrochloride,
5-(2-Chloro-4,5-difluoro-benzoylamino)-1H-pyrazole-3-carboxylic acid [6-(4-methylpiperazin-1-yl)-pyridin-3-ylmethyl]-amide dihydrochloride,
5-(2-Chloro-4,5-difluoro-benzoylamino)-1H-pyrazole-3-carboxylic acid [6-(4-dimethylamino-piperidin-1-yl)-pyridin-3-ylmethyl ]-amide dihydrochloride,
5-(2-Chloro-4,5-difluoro-benzoylamino)-1H-pyrazole-3-carboxylic acid [6-(pyrrolidin-1-yl)-pyridin-3-ylmethyl] -amide dihydrochloride,
5-(2-Chloro-4,5-difluoro-benzoylamino)-1H-pyrazole-3-carboxylic acid (pyrazin-2-ylmethyl)-amide dihydrochloride,
5-(2-Chloro-4,5-difluoro-benzoylamino)-1H-pyrazole-3-carboxylic acid [6-(thiomorpholin-4-yl)-pyridin-3-ylmethyl]-amide dihydrochloride,
5-(2-Chloro-4,5-difluoro-benzoylamino)-1H-pyrazole-3-carboxylic acid [6-(1,1-dioxo-116-thiomorpholin-4-yl)-pyridin-3-ylmethyl]-amide dihydrochloride,
5-(2-Chloro-4,5-difluoro-benzoylamino)-1H-pyrazole-3-carboxylic acid carbamoylmethyl-amide,
5-(2,4-Dichloro-5-fluoro-benzoylamino)-1H-pyrazole-3-carboxylic acid (4-carbamoyl-phenyl)-methyl-amide,
5-(2,4-Dichloro-5-fluoro-benzoylamino)-1H-pyrazole-3-carboxylic acid methyl-(4-methylcarbamoyl-phenyl)-amide,
5-(2,4-Dichloro-5-fluoro-benzoylamino)-1H-pyrazole-3-carboxylic acid (4-dimethylcarbamoyl-phenyl)-methyl-amide,
5-(2-Chloro-4,5-difluoro-benzoylamino)-1H-pyrazole-3-carboxylic acid (4-methanesulfonylaminocarbonyl-phenyl)-methyl-amide,
5-(2-Chloro-4,5-difluoro-benzoylamino)-1H-pyrazole-3-carboxylic acid (4-methanesulfonylaminocarbonyl-phenyl)-methyl-amide sodium salt,
5-(2-Chloro-4,5-difluoro-benzoylamino)-1H-pyrazole-3-carboxylic acid (4-cyano-phenyl)-methyl-amide,
5-(2-Chloro-4,5-difluoro-benzoylamino)-1H-pyrazole-3-carboxylic acid methyl-[4-(4H-[1,2,4]triazol-3-y)-phenyl]-amide hydrochloride,
5-(4-Azido-2-chloro-5-fluoro-benzoylamino)-1H-pyrazole -3-carboxylic acid (4-cyano-phenyl)-methyl-amide,
5-(4-Azido-2-chloro-5-fluoro-benzoylamino)-1H-pyrazole -3-carboxylic acid methyl-[4-(1H-tetrazol-5-yl)-phenyl]-amide,
5-(2-Chloro-4,5-difluoro-benzoylamino)-1H-pyrazole-3-carboxylic acid [4-(N-hydroxycarbamimidoyl)-phenyl]-methyl-amide,
5-(2-Chloro-4,5-difluoro-benzoylamino)-1H-pyrazole-3-carboxylic acid methyl-[4-(5-oxo-2,5-dihydro-[1,2,4]oxadiazol-3-yl)-phenyl]-amide,
5-(2-Chloro-4,5-difluoro-benzoylamino)-1H-pyrazole-3-carboxylic acid {4-[(2,2-dimethyl-propionyloxy)-methoxycarbonyl]-phenyl}-methyl-amide,
5-(2-Chloro-4,5-difluoro-benzoylamino)-1H-pyrazole-3-carboxylic acid 4-[N-(methoxy-thiocarbonyloxy)-carbamimidoyl]-methyl-amide,
5-(2-Chloro-4,5-difluoro-benzoylamino)-1H-pyrazole-3-carboxylic acid methyl-[4-(5-thioxo-4,5-dihydro-[1,2,4]oxadiazol-3-yl) -phenyl] -amide,
5-(2-Chloro-4,5-difluoro-benzoylamino)-1H-pyrazole-3-carboxylic acid methyl-[4-(5-oxo-4,5-dihydro-[1,2,4]thiadiazol-3-yl)-phenyl]-amide,
5-(2-Chloro-4,5-difluoro-benzoylamino)-1H-pyrazole-3-carboxylic acid {4-[1-(cyclohexyloxy-carbonyloxy)-ethoxycarbonyl]-phenyl}-methyl-amide,
5-(2-Chloro-4,5-difluoro-benzoylamino)-1H-pyrazole-3-carboxylic acid (4-ethoxycarbonyl-thiazol-2-yl)-ethyl-amide,
5-(2-Chloro-4,5-difluoro-benzoylamino)-1H-pyrazole-3-carboxylic acid 2-methanesulfonylamino-benzylamide,
5-(2-Chloro-4,5-difluoro-benzoylamino)-1H-pyrazole-3-carboxylic acid 2-acetylamino-benzylamide,
5-(2-Chloro-4,5-difluoro-benzoylamino)-1H-pyrazole-3-carboxylic acid 2-(dimethylamino-methyleneamino)-benzylamide hydrochloride,
5-(2-Chloro-4,5-difluoro-benzoylamino)-1H-pyrazole-3-carboxylic acid 3-(dimethylamino-methyleneamino)-benzylamide hydrochloride,
5-(2-Chloro-4,5-difluoro-benzoylamino)-1H-pyrazole-3-carboxylic acid 4-(dimethylamino-methyleneamino)-benzylamide hydrochloride,
5-(2-Chloro-4,5-difluoro-benzoylamino)-1H-pyrazole-3-carboxylic acid 2-[1-(dimethylamino)ethylideneamino]-benzylamide dihydrochloride,
5-(2-Chloro-4,5-difluoro-benzoylamino)-1H-pyrazole-3-carboxylic acid 3-[1-(dimethylamino)ethylideneamino]-benzylamide dihydrochloride,
5-(2-Chloro-4,5-difluoro-benzoylamino)-1H-pyrazole-3-carboxylic acid 4-[1-(dimethylamino)ethylideneamino]-benzylamide dihydrochloride,
5-(2-Chloro-4,5-difluoro-benzoylamino)-1H-pyrazole-3-carboxylic acid 2-(diethylamino-methyleneamino)-benzylamide dihydrochloride,
5-(2-Chloro-4,5-difluoro-benzoylamino)-1H-pyrazole-3-carboxylic acid 3-(diethylamino-methyleneamino)-benzylamide dihydrochloride,
5-(2-Chloro-4,5-difluoro-benzoylamino)-1H-pyrazole-3-carboxylic acid 4-(diethylamino-methyleneamino)-benzylamide dihydrochloride,
5-(2-Chloro-4,5-difluoro-benzoylamino)-1H-pyrazole-3-carboxylic acid 2-acetimidoylamino-benzylamide dihydrochloride,
5-(2-Chloro-4,5-difluoro-benzoylamino)-1H-pyrazole-3-carboxylic acid 3-acetimidoylamino-benzylamide dihydrochloride,
5- (2-Chloro-4,5-difluoro-benzoylamino) -1H-pyrazole-3-carboxylic acid 4-acetimidoylamino-benzylamide dihydrochloride,
5-(2-Chloro-4,5-difluoro-benzoylamino)-1H-pyrazole-3-carboxylic acid 4-cyano-benzylamide,
5-(2-Chloro-4,5-difluoro-benzoylamino)-1H-pyrazole-3-carboxylic acid 3-[(2-fluoro-benzimidoyl)-amino]-benzylamide hyrdoiodide,
5-(2-Chloro-4,5-difluoro-benzoylamino)-1H-pyrazole-3-carboxylic acid 4-[(2-fluoro-benzimidoyl)-amino]-benzylamide hyrdoiodide,
5-(2-Chloro-4,5-difluoro-benzoylamino)-1H-pyrazole-3-carboxylic acid 3-(3,3-dimethyl-ureido)-benzylamide,
5-(2-Chloro-4,5-difluoro-benzoylamino)-1H-pyrazole-3-carboxylic acid 4-(3,3-dimethyl-ureido)-benzylamide,
5-(2-Chloro-4,5-difluoro-benzoylamino)-1H-pyrazole-3-carboxylic acid 2-(toluene-4-sulfonylamino)-benzylamide,
5-(2-Chloro-4,5-difluoro-benzoylamino)-1H-pyrazole-3-carboxylic acid 2-amino-6-fluoro-benzylamide hydrochloride,
5-(2-Chloro-4,5-difluoro-benzoylamino)-1H-pyrazole-3-carboxylic acid 2-amino-4,5-difluoro-benzylamide hydrochloride,
5-(2-Chloro-4,5-difluoro-benzoylamino)-1H-pyrazole-3-carboxylic acid (indan-1-yl)-amide,
5-(2-Chloro-4,5-difluoro-benzoylamino)-1H-pyrazole-3-carboxylic acid (indan-2-yl)-amide,
5-(2-Chloro-4,5-difluoro-benzoylamino)-1H-pyrazole-3-carboxylic (4H-quinazoline-3-yl)-amide,
5-(2-Chloro-4,5-difluoro-benzoylamino)-1H-pyrazole-3-carboxylic (4H-quinazoline-3-yl)-amide dihydrochloride,
5-(2-Chloro-4,5-difluoro-benzoylamino)-1H-pyrazole-3-carboxylic acid (7-fluoro-4H-quinazoline-3-yl)-amide dihydrochloride,
5-(2-Chloro-4,5-difluoro-benzoylamino)-1H-pyrazole-3-carboxylic acid (6-fluoro-4H-quinazoline-3-yl)-amide hydrochloride,
5-(2-Chloro-4,5-difluoro-benzoylamino)-1H-pyrazole-3-carboxylic acid (2-ethoxy-2-methyl-1,4-dihydro-2H-quinazoline-3-yl)-amide,
5-(2-Chloro-4,5-difluoro-benzoylamino)-1H-pyrazole-3-carboxylic acid (2-cyano-pyridin-4-ylmethyl)-amide,
5-(2-Chloro-4,5-difluoro-benzoylamino)-1H-pyrazole-3-carboxylic acid [2-(N-hydroxycarbamimidoyl)-pyridin-4-ylmethyl]-amide,
5-(2-Chloro-4,5-difluoro-benzoylamino)-1H-pyrazole-3-carboxylic acid [2-(5-oxo-4,5-dihydro-[1,2,4]oxadiazol-3-yl)-pyridin-4 -ylmethyl]-amide, 4-{[5-(4,5-difluoro-2-methyl-benzoylamino)-1H-pyrazole-3-carbonyl]-methyl-amino}-benzoic acid sodium salt,
5-(4,5-Difluoro-2-methyl-benzoylamino)-1H-pyrazole-3-carboxylic acid methyl-[4-(1H-tetrazol-5-yl)-phenyl]-amide,
5-(2,4-Dichloro-benzoylamino)-1H-pyrazole-3-carboxylic acid (pyridin-3-ylmethyl)-amide,
5-(2,4-Dichloro-5-fluoro-benzoylamino)-1H-pyrazole-3-carboxylic acid (pyridin-3-ylmethyl)-amide,
5-(2,6-Dichloro-5-fluoro-pyridine-3-carbonylamino)-1H-pyrazole-3-carboxylic acid (pyridin-3-ylmethyl)-amide,
5-[(3-Chloro-benzo[b]thiophene-2-carbonyl)-amino]-1H-p yrazole-3-carboxylic acid (pyridin-3-ylmethyl)-amide,
5-[(3-Chloro-thiophene-2-carbonyl)-amino]-1H-pyrazole-3-carboxylic acid (pyridin-3-ylmethyl)-amide,
5-Benzoylamino-1H-pyrazole-3-carboxylic acid (pyridin-3-ylmethyl)-amide,
5-Phenylacetylamino-1H-pyrazole-3-carboxylic acid (pyridin-3-ylmethyl)-amide,
5-(2-Methyl-4,5-difluoro-benzoylamino)-1H-pyrazole-3-carboxylic acid (pyridin-3-ylmethyl)-amide dihydrochloride,
5-(2-Amino-4,5-difluoro-benzoylamino)-1H-pyrazole-3-carboxylic acid (pyridin-3-ylmethyl)-amide,
5-(4,5-Difluoro-2-methanesulfonylamino-benzoylamino)-1H-pyrazole-3-carboxylic acid (pyridin-3-ylmethyl)-amide,
5-(2-Acetylamino-4,5-difluoro-benzoylamino)-1H-pyrazole-3-carboxylic acid (pyridin-3-ylmethyl)-amide,
5-[(3,4,5-Trichloro-thiophene-2-carbonyl)-amino]-1H-pyrazole-3-carboxylic acid (pyridin-3-ylmethyl)-amide,
5-(4,5-Difluoro-2-methyl-benzoylamino)-1H-pyrazole-3-carboxylic acid (2-cyano-pyridin-4-ylmethyl)-amide,
5-(4,5-Difluoro-2-methyl-benzoylamino)-1H-pyrazole-3-carboxylic acid [2-(1H-tetrazol-5-yl)-pyridin-4-ylmethyl]-amide, and 1H-Pyrazole-3,5-dicarboxylic acid 3-benzylamide 5-[(2,4-dichloro-phenyl)-amide].

56. A therapeutic or prophylactic agent for insulin resistance, diabetic neuropathy, diabetic nephropathy, diabetic retinopathy, cataract, hypercholesterolemia, hypertension, hyperinsulinemia, hyperlipidemia, atherosclerosis, tissue ischemia, myocardial ischemia, obesity or bacterial, fungal, parasitic or viral infection, comprising a pyrazole compound according to any of claims 36 to 55 or a pharmacologically acceptable salt thereof as an active ingredient.

57. A medicine comprising a combination of a therapeutic or prophylactic agent according to claim 56 and another drug.
